# EUROPEAN PATENT SPECIFICATION

(11) **EP 3 149 206 B1**
(45) Date of publication and mention of the grant of the patent: **02.09.2020**
(21) Application number: 15799243.9
(22) Date of filing: 28.05.2015
(51) Int. Cl.: C12Q 1/6827, C12Q 1/6883

(54) **DNA METHYLATION STATUS AS A BIOMARKER OF ALCOHOL USE AND ABSTINENCE**
DNA-METHYLIERUNGSSTATUS ALS BIOMARKER VON ALKOHOLKONSUM UND ABSTINENZ
ÉTAT DE MÉTHYLATION DE L'ADN UTILISÉ COMME BIOMARQUEUR DE LA CONSOMMATION D'ALCOOL ET DE L'ABSTINENCE

(30) Priority: 30.05.2014 US 201462005408 P
(43) Date of publication of application: 05.04.2017
(73) Proprietor: Philibert, Robert, Iowa City, Iowa 52246 (US)
(72) Inventor: Philibert, Robert, Iowa City, Iowa 52246 (US)
(74) Representative: Kunz, Herbert
(86) International application number: PCT/IB2015/054041
(87) International publication number: WO 2015/181779

(56) References cited:
- US-B2- 8 637 652
- ROBERT A. PHILIBERT ET AL: "The Impact of Recent Alcohol Use on Genome Wide DNA Methylation Signatures", FRONTIERS IN GENETICS, vol. 3, 1 January 2012 (2012-01-01), XP055361007, DOI: 10.3389/fgene.2012.00054
- YUNLONG LIU ET AL: "Alcohol exposure alters DNA methylation profiles in mouse embryos at early neurulation", EPIGENETICS, vol. 4, no. 7, 1 October 2009 (2009-10-01) , pages 500-511, XP055361008, US ISSN: 1559-2294, DOI: 10.4161/epi.4.7.9925
- HUIPING ZHANG ET AL: "Array-Based Profiling of DNA Methylation Changes Associated with Alcohol Dependence", ALCOHOLISM: CLINICAL AND EXPERIMENTAL RESEARCH., vol. 37, 24 August 2012 (2012-08-24), pages E108-E115, XP055411295, US ISSN: 0145-6008, DOI: 10.1111/j.1530-0277.2012.01928.x
- PHILIBERT, RA ET AL.: 'The impact Of Recent Alcohol Use On Genome Wide DNA Methylation Signatures.' FRONTIERS IN GENETICS vol. 3, no. 54, April 2012, pages 1 - 8, XP055361007
- LIU, Y ET AL.: 'Alcohol Exposure Alters DNA Methylation Profiles In Mouse Embryos At Early Neurulation.' EPIGENETICS. vol. 4, no. 7, 2009, pages 500 - 511, XP055361008
- Robert A Philibert ET AL: "A pilot examination of the genome-wide DNA methylation signatures of subjects entering and exiting short-term alcohol dependence treatment programs", EPIGENETICS, vol. 9, no. 9, 11 August 2014 (2014-08-11) , pages 1212-1219, XP055587945, US ISSN: 1559-2294, DOI: 10.4161/epi.32252

## Description

### BACKGROUND

Alcoholism has a profound socio-economic and personal impact on tens of millions of individuals throughout the world. Unfortunately, the ability make the diagnosis is often hindered by the reliance on self-reporting, while the ability to monitor treatment response is challenged by the absence of robust and reliable biomarkers.

Robert A. Philibert et al. describe in Frontiers in Genetics, vol. 3, 1 January 2012 the impact of recent alcohol use on genome wide DNA methylation signatures.

Yunlong Liu et al. describe in Epigentics, vol. 4, no. 7, 1 October 2009, on pages 500-511 that alcohol exposure alters DNA methylation profiles in mouse embryos at early neurulation.

Huiping Zhang et al. describe in Alcoholism: Clinical and Experimental Reasearch, vol. 37, 24 August 2012 on pages E108-E115 an array based profiling of DNA methylation changes associated with alcohol dependence.

### SUMMARY

The present invention is defined by the appended claims. The dependent claims depict other embodiments of the invention.

This disclosure provides for methods and materials that can be used to determine whether or not an individual is using alcohol, and also to determine whether or not the individual has stopped using alcohol.

In one aspect, a method of determining whether or not an individual uses alcohol is provided. Such a method typically includes determining the methylation status of at least one CpG dinucleotide in a biological sample from the individual; and correlating the methylation status of the at least one CpG dinucleotide to determine whether or not the individual uses alcohol.

In some embodiments, the at least one CpG dinucleotide comprises position 71389896 of chromosome 10. For example, demethylation at position 71389896 of chromosome 10 is indicative of previous or current alcohol use; and remethylation at position 71389896 of chromosome 10 is indicative of less or no alcohol use (e.g., abstinence).

In some embodiments, the at least one CpG dinucleotide comprises position 54677008 of chromosome 12. For example, demethylation at position 54677008 of chromosome 12 is indicative of previous or current alcohol use; and remethylation at position 54677008 of chromosome 12 is indicative of less or no alcohol use (e.g., abstinence).

In some embodiments, the at least one CpG dinucleotide comprises position 75262522 of chromosome 8. For example, demethylation at position 75262522 of chromosome 8 is indicative of previous or current alcohol use; and remethylation at position 75262522 of chromosome 8 is indicative of less or no alcohol use (e.g., abstinence).

In some embodiments, the at least one CpG dinucleotide comprises position 92137791 of chromosome 9. For example, demethylation at position 92137791 of chromosome 9 is indicative of previous or current alcohol use; and remethylation at position 92137791 of chromosome 9 is indicative of less or no alcohol use (e.g., abstinence).

In some embodiments, the determining step includes contacting DNA in the biological sample with bisulfite under alkaline conditions to produce bisulfite-treated DNA; optionally, amplifying the bisulfite-treated DNA to produce amplified bisulfite-treated DNA; contacting the bisulfite-treated DNA or the amplified bisulfite-treated DNA with at least one oligonucleotide that is complementary to a sequence comprising the at least one CpG dinucleotide; and detecting the methylation status of the at least one CpG dinucleotide.

In some embodiments, the at least one oligonucleotide detects the CpG dinucleotide in the bisulfite-treated DNA in the unmethylated state. In some embodiments, the at least one oligonucleotide detects the CpG dinucleotide in the bisulfite-treated DNA in the methylated state. Representative biological samples include, without limitation, peripheral blood, lymphocytes, urine, saliva, and buccal cells. In some embodiments, the methylation status of the at least one CpG dinucleotide is determined using bi-sulfite treated DNA. In some embodiments, such a method further includes obtaining self-report data from the individual regarding whether or not the individual is user of alcohol.

In another aspect, a computer implemented method for determining whether or not an individual uses alcohol is provided. Such a method typically includes obtaining measured data associated with the user, the measured data comprising one or more measured CpG methylation status; generating a predictive score based on the obtained measured data; and providing a likelihood of alcohol use by the user based on the predictive score.

In some embodiments, such a method further includes determining the CpG methylation status for the user, wherein a change in methylation status is an indicator of alcohol use. In some embodiments, such a method further includes outputting a predicted level of alcohol use based on the predictive score.

In one aspect, a kit for determining the methylation status of at least one CpG dinucleotide is provided. Such a kit typically includes at least one first nucleic acid primer at least 8 nucleotides in length that is complementary to a bisulfite-converted nucleic acid sequence that includes at least one CpG dinucleotide, where the at least one first nucleic acid primer detects the methylated CpG dinucleotide. In some embodiments, the kit further includes at least one second nucleic acid primer at least 8 nucleotides in length that is complementary to a bisulfite-converted nucleic acid sequence that includes the at least one CpG dinucleotide, where the at least one second nucleic acid primer detects the unmethylated CpG dinucleotide.

In some embodiments, the first nucleic acid primer is complementary to a bisulfite-converted sequence that includes a methylated CpG dinucleotide at position 71389896 of chromosome 10. In some embodiments, the first nucleic acid primer is complementary to a bisulfite-converted sequence that includes an unmethylated CpG dinucleotide at position 71389896 of chromosome 10. In some embodiments, the first nucleic acid primer is complementary to a bisulfite-converted sequence that includes a methylated CpG dinucleotide at position 54677008 of chromosome 12. In some embodiments, the first nucleic acid primer is complementary to a bisulfite-converted sequence that includes an unmethylated CpG dinucleotide at position 54677008 of chromosome 12. In some embodiments, the first nucleic acid primer is complementary to a bisulfite-converted sequence that includes a methylated CpG dinucleotide at position 75262522 of chromosome 8. In some embodiments, the first nucleic acid primer is complementary to a bisulfite-converted sequence that includes an unmethylated CpG dinucleotide at position 75262522 of chromosome 8. In some embodiments, the first nucleic acid primer is complementary to a bisulfite-converted sequence that includes a methylated CpG dinucleotide at position 92137791 of chromosome 9. In some embodiments, the first nucleic acid primer is complementary to a bisulfite-converted sequence that includes an unmethylated CpG dinucleotide at position 92137791 of chromosome 9.

In some embodiments, a kit as described herein can include at least a third nucleic acid primer at least 8 nucleotides in length that is complementary to a nucleic acid sequence upstream of the CpG dinucleotide. In some embodiments, a kit as described herein can include at least a fourth nucleic acid primer at least 8 nucleotides in length that is complementary to a nucleic acid sequence downstream of the CpG dinucleotide. In some embodiments, the at least third nucleic acid primer is complementary to a bisulfite-converted nucleic acid sequence. In some embodiments, the at least fourth nucleic acid primer is complementary to a bisulfite-converted nucleic acid sequence.

In some embodiments, the at least one first nucleic acid primer, the at least one second nucleic acid primer, the at least one third nucleic acid primer, and/or the at least one fourth nucleic acid primer includes one or more nucleotide analogs. In some embodiments, the at least one first nucleic acid primer, the at least one second nucleic acid primer, the at least one third nucleic acid primer, and/or the at least one fourth nucleic acid primer includes one or more synthetic or non-natural nucleotides.

In some embodiments, the kit further includes a solid substrate to which the at least one first nucleic acid primer is bound. Representative solid substrates include, without limitation, polymers, glass, semiconductors, papers, metals, gels and/or hydrogels. In some embodiments, the solid substrate is a microarray or microfluidics card. In some embodiments, the kit further includes a detectable label. In some embodiments, such a kit further includes instructions for correlating a change in methylation status at one or more CpG positions with alcohol use.

Unless otherwise defined, all technical and scientific terms used herein have the same meaning as commonly understood by one of ordinary skill in the art to which the methods and compositions of matter belong. Although methods and materials similar or equivalent to those described herein can be used in the practice or testing of the methods and compositions of matter, suitable methods and materials are described below. In addition, the materials, methods, and examples are illustrative only and not intended to be limiting.

### DESCRIPTION OF DRAWINGS

Figure 1 is a graph showing Quantile-Quantile (QQ) plots of the comparison between the methylation status of DNA from the 32 case subjects (at time T1) and the methylation status of DNA from the 33 abstinent controls.
Figure 2 is a graph showing QQ plots of the comparison between the methylation status of DNA from 25 case subjects at time T1 and the methylation status of DNA from the same 25 case subjects at time T2.

### DETAILED DESCRIPTION

Using a genome wide approach, this disclosure shows that, as compared to abstinent controls, alcohol use is associated with widespread changes in DNA methylation. This disclosure also shows that the changes in DNA methylation have a tendency to revert as a function of abstinence. Thus, DNA methylation can be used clinically to assess alcohol use status and/or to monitor alcohol treatment response.

### Methylation of Nucleic Acids and Determining the Methylation Status of Nucleic Acids

CpG islands are stretches of DNA in which the frequency of the CpG sequence is higher than other regions. The "p" in the term CpG designates the phosphodiester bond that binds the cysteine ("C") nucleotide and the guanine ("G") nucleotide. CpG islands are often located around promoters and are often involved in regulating the expression of a gene (e.g., housekeeping genes). Generally, CpG islands are not methylated when a sequence is expressed, and methylated to suppress expression (or "inactivate" the gene).

The methylation status of one or more CpG dinucleotides in genomic DNA or in a particular nucleic acid sequence (e.g., a CpG island) can be determined using any number of biological samples, such as blood, urine (e.g., cells from the bladder and/or urethra contained within the urine), saliva, or buccal cells. In certain embodiments, a particular cell type, e.g., lymphocytes, basophils, or monocytes, can be obtained (e.g., from a blood sample) and the DNA evaluated for its methylation status.

The methylation status of genomic DNA, of a CpG island, or of one or more specific CpG dinucleotides can be determined by the skilled artisan using any number of methods. The most common method for evaluating the methylation status of DNA begins with a bisulfite-based reaction on the DNA (see, for example, Frommer et al., 1992, PNAS USA, 89(5):1827-31). Commercial kits are available for bisulfite-modifying DNA. See, for example, EpiTect Bisulfite or EpiTect Plus Bisulfite Kits (Qiagen).

Following bisulfite modification, the nucleic acid can be amplified. Since treating DNA with bisulfite deaminates unmethylated cytosine nucleotides to uracil, and since uracil pairs with adenosine, thymidines are incorporated into DNA strands in positions of unmethylated cytosine nucleotides during subsequent PCR amplifications.

In some embodiments, the methylation status of DNA can be determined using one or more nucleic acid-based methods. For example, an amplification product of bisulfite-treated DNA can be cloned and directly sequenced using recombinant molecular biology techniques routine in the art. Software programs are available to assist in determining the original sequence, which includes the methylation status of one or more nucleotides, of a bisulfite-treated DNA (e.g., CpG Viewer (Carr et al., 2007, Nucl. Acids Res., 35:e79)). Also for example, amplification products of bisulfite-treated DNA can be hybridized with one or more oligonucleotides that, for example, are specific for the methylated, bisulfite-treated DNA sequence, or specific for the unmethylated, bisulfite-treated DNA sequence.

In some embodiments, the methylation status of DNA can be determined using a non-nucleic acid-based method. A representative non-nucleic acid-based method relies upon sequence-specific cleavage of bisulfite-treated DNA followed by mass spectrometry (e.g., MALDI-TOF MS) to determine the methylation ratio (methyl CpG/total CpG) (see, for example, Ehrich et al., 2005, PNAS USA, 102:15785-90). Such a method is commercially available (e.g., MassARRAY Quantitative Methylation Analysis (Sequenom, San Diego, CA)).

### Nucleic Acid Sequences in Which the Methylation Status is Associated with Alcohol Use

The methylation status of certain genomic DNA has been shown to be altered in individuals that use alcohol (relative to non-users). See, for example, Philibert et al. (2008, Am. J. Med. Genet. B Neuropsychiatr. Genet., 147B:565-70); Philibert et al. (2008, Am. J. Med. Genet. B Neuropsychiatr. Genet., 147B:543-9); Philibert et al. (2009, Psychiatr. Genet., 19:91-8); Philibert et al. (2012, Front Genet., 3:54); and also in U.S. Patent No. 8,637,652.

The present disclosure describes additional changes in the methylation status of one or more CpG islands and/or particular CpG dinucleotides that are correlated with alcohol use (e.g., heavy alcohol use). See, for example, Table II, which shows the top 30 probes that were most-significantly associated with changes in the methylation status of alcohol users compared to non-drinkers; and Table III, which show the top 30 differentially regulated gene pathways between alcohol users and non-drinkers. Any one or more of the CpG dinucleotides in which methylation status has been associated with alcohol use can be used in the methods herein to determine the predictive value (e.g., representing the likelihood of alcohol use). It would be understood that, particularly for determining alcohol use, the more CpG dinucleotides (i.e., CpG dinucleotides in which methylation status has been associated with alcohol use) are evaluated, the more accurate the predictive value will be.

In addition, it would be appreciated that the methylation status of one or more neighboring CpG dinucleotides can be in linkage disequilibrium with the methylation status of a CpG dinucleotide having significance with alcohol use (see, for example, Philibert et al., 2009, Am. J. Med. Genet. B. Neuropsychiatr. Genet., 153B:619-28) and, therefore, the methylation status of those neighboring CpG dinucleotides (e.g., about 200 nucleotides upstream and/or downstream of a CpG dinucleotide having significance with alcohol use (e.g., about 100 nucleotides upstream and/or downstream; about 50 nucleotides upstream and/or downstream; about 25 nucleotides upstream and/or downstream; about 20 nucleotides upstream and/or downstream; about 10 nucleotides upstream and/or downstream; or about 5 nucleotides upstream and/or downstream)) can be used in the methods described herein. Further, it would be appreciated that, in some instances, the greater (or more significant) the changes in the methylation status, the greater the alcohol use. See, for example, Philibert et al., 2012, Epigenetics, 7:1-8.

### Nucleic Acids and Methods Related Thereto

As used herein, nucleic acids can include DNA and RNA, and includes nucleic acids that contain one or more nucleotide analogs or backbone modifications. A nucleic acid can be single stranded or double stranded, which usually depends upon its intended use.

As used herein, an "isolated" nucleic acid molecule is a nucleic acid molecule that is free of sequences that naturally flank one or both ends of the nucleic acid in the genome of the organism from which the isolated nucleic acid molecule is derived (e.g., a cDNA or genomic DNA fragment produced by PCR or restriction endonuclease digestion). Such an isolated nucleic acid molecule is generally introduced into a vector (e.g., a cloning vector, or an expression vector) for convenience of manipulation or to generate a fusion nucleic acid molecule, discussed in more detail below. In addition, an isolated nucleic acid molecule can include an engineered nucleic acid molecule such as a recombinant or a synthetic nucleic acid molecule.

Nucleic acids can be isolated using techniques routine in the art. For example, nucleic acids can be isolated using any method including, without limitation, recombinant nucleic acid technology, and/or the polymerase chain reaction (PCR). General PCR techniques are described, for example in PCR Primer: A Laboratory Manual, Dieffenbach & Dveksler, Eds., Cold Spring Harbor Laboratory Press, 1995. Recombinant nucleic acid techniques include, for example, restriction enzyme digestion and ligation, which can be used to isolate a nucleic acid. Isolated nucleic acids also can be chemically synthesized, either as a single nucleic acid molecule or as a series of oligonucleotides.

A vector containing a nucleic acid (e.g., a nucleic acid that encodes a polypeptide) also is provided. Vectors, including expression vectors, are commercially available or can be produced by recombinant DNA techniques routine in the art. A vector containing a nucleic acid can have expression elements operably linked to such a nucleic acid, and further can include sequences such as those encoding a selectable marker (e.g., an antibiotic resistance gene). A vector containing a nucleic acid can encode a chimeric or fusion polypeptide (i.e., a polypeptide operatively linked to a heterologous polypeptide, which can be at either the N-terminus or C-terminus of the polypeptide). Representative heterologous polypeptides are those that can be used in purification of the encoded polypeptide (e.g., 6xHis tag, glutathione S-transferase (GST))

Expression elements include nucleic acid sequences that direct and regulate expression of nucleic acid coding sequences. One example of an expression element is a promoter sequence. Expression elements also can include introns, enhancer sequences, response elements, or inducible elements that modulate expression of a nucleic acid. Expression elements can be of bacterial, yeast, insect, mammalian, or viral origin, and vectors can contain a combination of elements from different origins. As used herein, operably linked means that a promoter or other expression element(s) are positioned in a vector relative to a nucleic acid in such a way as to direct or regulate expression of the nucleic acid (e.g., in-frame). Many methods for introducing nucleic acids into host cells, both *in vivo* and *in vitro,* are well known to those skilled in the art and include, without limitation, electroporation, calcium phosphate precipitation, polyethylene glycol (PEG) transformation, heat shock, lipofection, microinjection, and viral-mediated nucleic acid transfer.

Vectors as described herein can be introduced into a host cell. As used herein, "host cell" refers to the particular cell into which the nucleic acid is introduced and also includes the progeny or potential progeny of such a cell. A host cell can be any prokaryotic or eukaryotic cell. For example, nucleic acids can be expressed in bacterial cells such as *E. coli,* or in insect cells, yeast or mammalian cells (such as Chinese hamster ovary cells (CHO) or COS cells). Other suitable host cells are known to those skilled in the art.

Oligonucleotides for amplification or hybridization can be designed using, for example, a computer program such as OLIGO (Molecular Biology Insights, Inc., Cascade, CO). Important features when designing oligonucleotides to be used as amplification primers include, but are not limited to, an appropriate size amplification product to facilitate detection (e.g., by electrophoresis), similar melting temperatures for the members of a pair of primers, and the length of each primer (i.e., the primers need to be long enough to anneal with sequence-specificity and to initiate synthesis but not so long that fidelity is reduced during oligonucleotide synthesis). Typically, oligonucleotide primers are 15 to 30 (e.g., 16, 18, 20, 21, 22, 23, 24, or 25) nucleotides in length. Designing oligonucleotides to be used as hybridization probes can be performed in a manner similar to the design of amplification primers. In some embodiments, hybridization probes can be designed to distinguish between to targets that contain different sequences (e.g., a polymorphism or mutation, e.g., the methylated vs. non-methylated sequence in the bisulfite-treated DNA).

Hybridization between nucleic acids is discussed in detail in Sambrook et al. (1989, Molecular Cloning: A Laboratory Manual, 2nd Ed., Cold Spring Harbor Laboratory Press, Cold Spring Harbor, NY; Sections 7.37-7.57, 9.47-9.57, 11.7-11.8, and 11.45-11.57). Sambrook et al. discloses suitable Southern blot conditions for oligonucleotide probes less than about 100 nucleotides (Sections 11.45-11.46). The Tm between a sequence that is less than 100 nucleotides in length and a second sequence can be calculated using the formula provided in Section 11.46. Sambrook et al. additionally discloses Southern blot conditions for oligonucleotide probes greater than about 100 nucleotides (see Sections 9.47-9.54). The Tm between a sequence greater than 100 nucleotides in length and a second sequence can be calculated using the formula provided in Sections 9.50-9.51 of Sambrook et al.

The conditions under which membranes containing nucleic acids are prehybridized and hybridized, as well as the conditions under which membranes containing nucleic acids are washed to remove excess and non-specifically bound probe, can play a significant role in the stringency of the hybridization. Such hybridizations and washes can be performed, where appropriate, under moderate or high stringency conditions. For example, washing conditions can be made more stringent by decreasing the salt concentration in the wash solutions and/or by increasing the temperature at which the washes are performed. Simply by way of example, high stringency conditions typically include a wash of the membranes in 0.2X SSC at 65°C.

In addition, interpreting the amount of hybridization can be affected, for example, by the specific activity of the labeled oligonucleotide probe, by the number of probe-binding sites on the template nucleic acid to which the probe has hybridized, and by the amount of exposure of an autoradiograph or other detection medium. It will be readily appreciated by those of ordinary skill in the art that although any number of hybridization and washing conditions can be used to examine hybridization of a probe nucleic acid molecule to immobilized target nucleic acids, it is more important to examine hybridization of a probe to target nucleic acids under identical hybridization, washing, and exposure conditions. Preferably, the target nucleic acids are on the same membrane.

A nucleic acid molecule is deemed to hybridize to a nucleic acid but not to another nucleic acid if hybridization to a nucleic acid is at least 5-fold (e.g., at least 6-fold, 7-fold, 8-fold, 9-fold, 10-fold, 20-fold, 50-fold, or 100-fold) greater than hybridization to another nucleic acid. The amount of hybridization can be quantitated directly on a membrane or from an autoradiograph using, for example, a PhosphorImager or a Densitometer (Molecular Dynamics, Sunnyvale, CA).

A nucleic acid sequence, or a polypeptide sequence, can be compared to one or more related nucleic acid sequences or polypeptide sequences, respectively, using percent sequence identity. In calculating percent sequence identity, two sequences are aligned and the number of identical matches of nucleotides or amino acid residues between the two sequences is determined. The number of identical matches is divided by the length of the aligned region (i.e., the number of aligned nucleotides or amino acid residues) and multiplied by 100 to arrive at a percent sequence identity value. It will be appreciated that the length of the aligned region can be a portion of one or both sequences up to the full-length size of the shortest sequence. It also will be appreciated that a single sequence can align with more than one other sequence and hence, can have different percent sequence identity values over each aligned region.

The alignment of two or more sequences to determine percent sequence identity can be performed using the computer program ClustalW and default parameters, which allows alignments of nucleic acid or polypeptide sequences to be carried out across their entire length (global alignment). Chenna et al., 2003, Nucleic Acids Res., 31(13):3497-500. ClustalW calculates the best match between a query and one or more subject sequences, and aligns them so that identities, similarities and differences can be determined. Gaps of one or more residues can be inserted into a query sequence, a subject sequence, or both, to maximize sequence alignments. For fast pairwise alignment of nucleic acid sequences, the default parameters can be used (i.e., word size: 2; window size: 4; scoring method: percentage; number of top diagonals: 4; and gap penalty: 5); for an alignment of multiple nucleic acid sequences, the following parameters can be used: gap opening penalty: 10.0; gap extension penalty: 5.0; and weight transitions: yes. For fast pairwise alignment of polypeptide sequences, the following parameters can be used: word size: 1; window size: 5; scoring method: percentage; number of top diagonals: 5; and gap penalty: 3. For multiple alignment of polypeptide sequences, the following parameters can be used: weight matrix: blosum; gap opening penalty: 10.0; gap extension penalty: 0.05; hydrophilic gaps: on; hydrophilic residues: Gly, Pro, Ser, Asn, Asp, Gln, Glu, Arg, and Lys; and residue-specific gap penalties: on. ClustalW can be run, for example, at the Baylor College of Medicine Search Launcher website or at the European Bioinformatics Institute website on the World Wide Web.

Changes can be introduced into nucleic acid coding sequences using, for example, mutagenesis (e.g., site-directed mutagenesis, PCR-mediated mutagenesis) or by chemically synthesizing a nucleic acid molecule having such changes. Such nucleic acid changes can lead to conservative and/or non-conservative amino acid substitutions at one or more amino acid residues. A "conservative amino acid substitution" is one in which one amino acid residue is replaced with a different amino acid residue having a similar side chain (see, for example, Dayhoff et al. (1978, in Atlas of Protein Sequence and Structure, 5(Suppl. 3):345-352), which provides frequency tables for amino acid substitutions), and a non-conservative substitution is one in which an amino acid residue is replaced with an amino acid residue that does not have a similar side chain.

Nucleic acids can be detected using any number of amplification techniques (see, e.g., PCR Primer: A Laboratory Manual, 1995, Dieffenbach & Dveksler, Eds., Cold Spring Harbor Laboratory Press, Cold Spring Harbor, NY; and U.S. Patent Nos. 4,683,195; 4,683,202; 4,800,159; and 4,965,188) with an appropriate pair of oligonucleotides (e.g., primers). A number of modifications to the original PCR have been developed and can be used to detect a nucleic acid. Detection (e.g., of an amplification product, a hybridization complex, or a polypeptide) is usually accomplished using detectable labels. The term "label" is intended to encompass the use of direct labels as well as indirect labels. Detectable labels include enzymes, prosthetic groups, fluorescent materials, luminescent materials, bioluminescent materials, and radioactive materials.

### Articles of Manufacture or Kits

This disclosure also provides for articles of manufacture that can be used to determine the methylation status of one or more CpG dinucleotides and/or CpG islands. An article of manufacture as provided herein can include one or more oligonucleotides for determining the methylation status of one or more CpG dinucleotides and/or CpG islands, together with suitable packaging materials. The one or more oligonucleotides can detect the CpG dinucleotide in the bisulfite-treated DNA in the unmethylated state or in the methylated state.

Articles of manufacture may additionally include reagents (e.g., buffers, enzymes, cofactors) for carrying out the methods disclosed herein (e.g., bisulfite-treating DNA, amplification, sequencing, hybridization). Articles of manufacture provided herein also can contain a package insert or package label having instructions thereon for using the components within the article of manufacture to determine the methylation status of one or more CpG dinucleotides and/or one or more CpG islands in a biological sample.

In accordance with the present invention, there may be employed conventional molecular biology, microbiology, biochemical, and recombinant DNA techniques within the skill of the art. Such techniques are explained fully in the literature. The invention will be further described in the following examples,

### EXAMPLES

### Example 1-Materials and Methods

All protocols and procedures used in this study were approved by the University of Iowa Institutional Review Board and full details are available from the inventors upon request. The case subjects described herein were recruited from the University of Iowa hospitals and clinics or two alcohol treatment centers in the State of Iowa. In brief, after admission to a hospital or alcohol treatment center and achievement of both sobriety and absence of significant cognitive impairment, potential subjects were approached by facility personnel as to their potential interest in participating in the current study. If interested, this information was relayed to the research team with the potential subjects then being contacted by a member of the research team who provided detailed information and screened the individuals for suitability for the study. Inclusion criteria included the ability to consent, an absence of significant active substance use disorders other than tobacco, an absence of medications hypothesized to affect DNA methylation, the absence of medical disorders including cancer, gastrointestinal disorders, diabetes, chronic obstructive pulmonary disease or severe cardiac disease, and in general, otherwise overall general good health. If not excluded and still willing to participate, subjects were consented and the study procedure initiated.

At the index intake (Time 1, or T1), which could occur up to seven days after admission, all case subjects were interviewed with a modified version of the Semi-Structured Assessment for the Genetics of Alcoholism, Version 2 (SSAGA-II; Bucholz et al., 1994, J. Stud. Alcohol, 55:149-58), which included specific items to assess use of substances over the past six months. In addition, subjects were phlebotomized to provide biomaterial for the current study. Then, approximately 4 weeks later (Time 2, or T2), the same subjects were once again interviewed to assess for changes in medical status and phlebotomized.

Control subjects were recruited from the Iowa City region. Inclusion criteria for the study were similar to those of the case subjects and included good overall general health, a complete abstinence from alcohol for at least six months, an absence of medications hypothesized to influence DNA methylation, and an absence of significant substance use other than tobacco. All control subjects were interviewed with the same instruments as the case subjects and phlebotomized to provide biomaterial for the current study.

Sera and mononuclear cell (i.e. lymphocyte) pellets were prepared as previously described (Philibert et al., 2012, Epigenetics, 7:1331-8). As part of an effort to assess self-report reliability, cotinine and tetrahydrocannabinol levels were assessed in sera using an enzyme linked immunoassay (ELISA) kits supplied by Abnova (Taiwan) according to manufacturer's directions and previous protocols (Philibert et al., 2013, Epigenetics, 5:19-26). DNA was prepared from the lymphocyte cell pellets using a QiaAmp kit (Qiagen, Germany) according to manufacturer's directions.

Genome wide DNA methylation was assessed using the Illumina (San Diego, CA) HumanMethylation450 Beadchip by the University of Minnesota Genome Center (Minneapolis, MN) using the protocol specified by the manufacturer as previously described (Monick et al., 2012, Am. J. Med. Genet., Part B Neuropsychiatric Genet., 159:141-51). This chip contains 485,577 probes recognizing at least 20216 transcripts, potential transcripts or CpG islands (from the Genome Reference Consortium human genome build 37 (GRCh37)).

The resulting data were inspected for complete bisulfite conversion and average beta values were determined for each targeted CpG residue determined using the Illumina Genome Studio Methylation Module, Version 3.2. The resulting data were then cleaned using a PERL-based algorithm to remove those beta values whose detection *p*-values, an index of the likelihood that the observed sequence represents random noise, were greater than 0.05. Then, data from arrays with < 99.5% complete assessment success were removed.

The surviving data was imported into MethLAB (Kilaru et al., 2012, Epigenetics, 7:225-9) and analyzed with respect to alcohol use status using standard general linear models approach controlling for chip and batch variables. The resulting *p*-values were corrected for genome wide comparisons using either False Discovery Rate or Bonferroni correction methods (Benjamini et al., 1995, J. Royal Stat. Soc. Series B, Method., 57:289-300).

Essentially, a genome wide linear regression was used; in this case, a t-test that accounts for batch and slide co-variates. "Batch" is a term in DNA methylation that refers to the grouping with respect to bisulfite conversion (e.g., "batch" takes into account the degree of bisulfite conversion or lack thereof). "Slide" refers to the individual chip(s) or array(s) on which the hybridizations are performed, since, for example, there can be slide to slide variation with respect to the degree of hybridization of the same sample. It would be understood that accounting for batch and slide variation usually results in a greater degree of significance.

### Example 2-Experimental Results

The clinical and demographic characteristics of the case and control subjects included in the study are described in Table 1. In brief, in keeping with the population of Iowa and overall referral characteristics of patients referred for alcohol treatment, the subjects tended to be in their 40's and were primarily white males. The case subjects were extremely heavy alcohol users, with the typical subject describing the average consumption of a fifth of vodka or 12 pack of beer for at least the past week prior to admission. The delay between the time of phlebotomy and their last drink averaged four days and varied between one and eight days. Not surprisingly, the case subjects were enriched for tobacco use disorders, with 85% of them being daily smokers. They also tended to have a higher rate of THC use, with 10 subjects having either marginal or markedly positive hydroxy-THC levels. In each and every case, the results of the serum cotinine and THC ELISA tests were consistent with self-report data. Of the 41 case Time 1 (T1) subjects ascertained during the time period leading up to this study, only 26 completed the Time 2 (T2) assessments. The reasons for the failure varied. In some cases, the subject had already left treatment and had begun to drink, while in other cases, the subject had already left treatment and was either unavailable or unwilling to complete the second stage of assessment.

**Table I. Clinical and demographic characteristics of study subjects**

| | Case Subjects | Control Subjects |
|---|---|---|
| N= | 33 | 33 |
| Age | 45.5 7.8 | 46.7 7.8 |

| Ethnicity | | |
|---|---|---|
| White | 30 | 31 |
| African American | 2 | 1 |
| Hispanic | 0 | 1 |

| Gender | | |
|---|---|---|
| Male | 25 | 25 |
| Female | 8 | 8 |
| Days since last drink | 4.0 1.8 | |

| Average daily drink consumption prior to admission by self report | | |
|---|---|---|
| Past week | | 0 |
| Past month | | 0 |
| Past 6 months | | 0 |

| Smoking Status | | |
|---|---|---|
| Current daily | 27 | 1 |
| Positive cotinine | 28 | 1 |

| Cannabis Use Status | | |
|---|---|---|
| Use in past year by self report | 11 | 0 |
| Positive hydroxyl-THC | 9 | 1 |

In contrast, though similar in age, gender and ethnicity, the 33 control subjects had lower rates of both tobacco and cannabis use. In fact, only one of the control subjects was a current daily smoker and all control subjects denied cannabis use in the past year. In contrast to the results from the case subjects, one discrepancy in the serum ELISA testing was observed (i.e., there was one positive test for THC).

Genome wide methylation data was successfully obtained (measurements for >99.5% of all probes) for 95 samples, including two lymphoblast DNA standards and one internal replicate. This included 33 controls, 33 case subjects at T1 and 26 case subjects at T2. The correlation between the internal replicate was greater than 0.998. The average beta value for the controls, and the subjects at T1 and T2 was 0.4788, 0.4800 and 0.4833, respectively.

The results from these experiments and the CpG residues, regions and gene claimed in this application are given in Appendix A. The data contained in Appendix A provides the Illumina probe ID, the sequences of which are publicly available, and the identity of the CpG in question denoted by sequence information and mapping information. Finally, the p-value for the t-test comparing methylation of the alcoholic subjects to that for the controls is provided. A p < 0.05 is considered significant, and complete annotation files for the probes listed in Appendix A are publically available.

Specifically, Column A, "Probe Name": the Illumina designation for the probe. Column B, "Target Region": the genomic sequence of the region; the center of the region, the CpG residue, is denoted by brackets (e.g. [CG]). Column C, "CHR": the chromosome on which the target region is found. Column D, "Map Info": the base pair at which the CpG residue is found in the GRCH37 assembly; note that the since the cytosine and guanine nucleotides are complementary, the CpG residue is found on both the sense and anti-sense strand. Column E, "UCSC Ref Gene Name": the generally accepted gene names, when present, in which the CpG residue is found. Column F, "p-value": the significance of the t-test comparing methylation of the alcoholic cases to that of the controls.

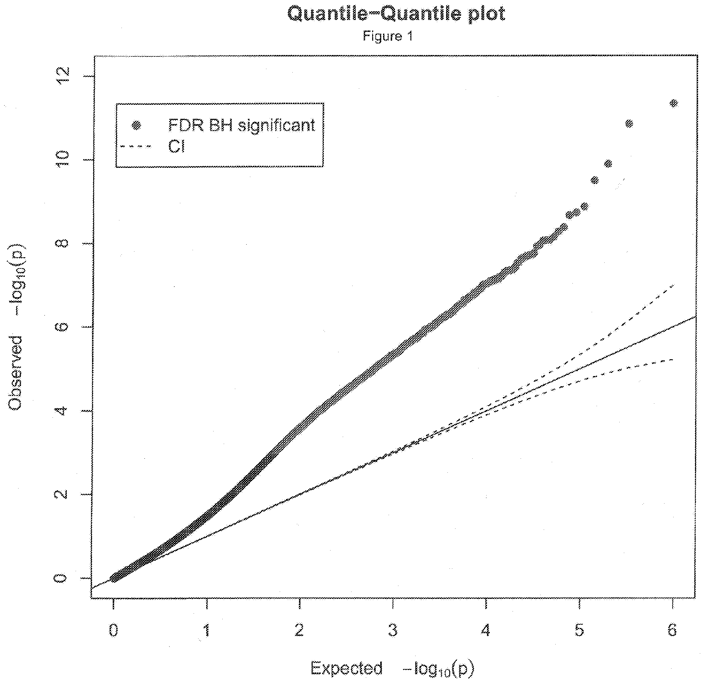

The list of the 30 most significant results from the comparison of the methylation status of the DNA from the 32 T1 subjects and the 33 healthy abstinent controls are listed in Table II. Overall, after genome wide correction using false discovery rate (FDR) method, a total of 8636 probes were differentially methylated while, when using more conservative Bonferroni correction, only 56 comparisons were statistically significant. Examination of the QQ plot for the comparison reveals the basis for these observed differences is significant with positive skewing (greater numbers of more significant p values) being markedly prominent (Figure 1).

**Table II. The 30 Most Significantly Associated Probes in Case and Control Analysis**

| | | | | Average Beta Values | | | Corrected |
|---|---|---|---|---|---|---|---|
| Probe ID | GENE | Placement | Island Status | Case | Control | T-test | P-value |
| cg23193759 | C10orf35 | TSS200 | Island | 0.128 | 0.168 | 4.66E-12 | 2.26E-06 |
| cg02583484 | HNRNPA1 | Body | S Shelf | 0.250 | 0.319 | 1.42E-11 | 3.46E-06 |
| cg23779890 | GDAP1 | TSS200 | Island | 0.194 | 0.243 | 1.30E-10 | 2.10E-05 |
| cg13415831 | | | | 0.073 | 0.098 | 3.18E-10 | 3.87E-05 |
| cg09935388 | GFI1 | Body | Island | 0.647 | 0.799 | 1.35E-09 | 0.0001 |
| cg01432120 | | | Island | 0.660 | 0.720 | 1.86E-09 | 0.0002 |
| cg12655542 | | | | 0.225 | 0.282 | 2.18E-09 | 0.0002 |
| cg11832281 | CUGBP2 | Body | S Shelf | 0.070 | 0.097 | 4.20E-09 | 0.0003 |
| cg06126421 | | | | 0.643 | 0.750 | 5.34E-09 | 0.0003 |
| cg12895631 | C11orf75 | 5'UTR | | 0.161 | 0.192 | 7.07E-09 | 0.0003 |
| cg25998745 | | | | 0.588 | 0.666 | 8.43E-09 | 0.0003 |
| cg08352774 | TMEM181 | Body | S Shelf | 0.110 | 0.149 | 8.46E-09 | 0.0003 |
| cg19939077 | PPIF | Body | S Shore | 0.147 | 0.184 | 8.85E-09 | 0.0003 |
| cg13126206 | | | S Shelf | 0.468 | 0.521 | 1.11E-08 | 0.0004 |
| cg22888484 | SNHG11 | TSS200 | N Shore | 0.043 | 0.056 | 1.21E-08 | 0.0004 |
| cg00159243 | SELPLG | 5'UTR | | 0.397 | 0.474 | 1.76E-08 | 0.0005 |
| cg24046474 | RPL12 | Body | N Shore | 0.221 | 0.296 | 1.89E-08 | 0.0005 |
| cg00957665 | TRIM8 | Body | S Shore | 0.097 | 0.125 | 1.93E-08 | 0.0005 |
| cg06093152 | | | | 0.572 | 0.654 | 2.03E-08 | 0.0005 |
| cg12126344 | | | | 0.795 | 0.835 | 2.13E-08 | 0.0005 |
| cg17485265 | FAM50B | TSS1500 | N Shore | 0.677 | 0.744 | 2.29E-08 | 0.0005 |
| cg16854826 | ZMIZ1 | 5'UTR | | 0.603 | 0.654 | 2.42E-08 | 0.0005 |
| cg23028436 | STK38L | TSS200 | Island | 0.064 | 0.094 | 2.89E-08 | 0.0006 |
| cg00690082 | STAT5A | TSS1500 | N Shore | 0.309 | 0.376 | 3.00E-08 | 0.0006 |
| cg06285727 | ATG16L2 | TSS1500 | N Shore | 0.152 | 0.216 | 3.68E-08 | 0.0007 |
| cg09267773 | | | N Shore | 0.517 | 0.433 | 4.00E-08 | 0.0007 |
| cg21475150 | RPL31 | TSS1500 | Island | 0.794 | 0.849 | 4.30E-08 | 0.0007 |
| cg21416692 | PHC2 | 5'UTR | | 0.742 | 0.792 | 4.41E-08 | 0.0007 |
| cg02348119 | TBC1D16 | 5'UTR | | 0.598 | 0.647 | 4.48E-08 | 0.0007 |
| cg10691866 | TPST1 | Body | | 0.419 | 0.491 | 4.55E-08 | 0.0007 |

| | | | | | | | |
|---|---|---|---|---|---|---|---|
| All average methylation values are non-log transformed beta-values. Island status refers to the position of the probe relative to the island. Classes include: 1) Island, 2) north (N) shore, 3) south (S) shore, 4) north (N) shelf, 5) south (S) shelf and 6) blank denoting that the probe does not map to an island. | | | | | | | |

A frequent concern in high-risk cohorts such as these case subjects is the effect of co-morbid substances. In particular, this is a concern for the current results because smoking has profound effects on DNA methylation (see, for example, Dogan et al., 2014, BMC Genomics, 15:151), and 27 of the 33 case subjects were active smokers. In this regard, cg05575921, which has been shown to correlate strongly with tobacco use (see, for example, Dogan et al., 2014, BMC Genomics, 15:151), was the 31^{st} ranked probe. However, overall, there was no significant overlap between the signal for alcohol consumption and the signal for smoking. Only 22 of the 910 probes that attained genome wide significance with respect to smoking in Dogan et al. (2014, BMC Genomics, 15:151) were ranked among the top 10,000 most highly associated probes in the current study. Conversely, the overall rank of the 10,000 most highly associated probes with respect to smoking in Dogan et al. was 302,264 (of 485,577), with the median of the distribution being 318,258^{th}. Hence, there appears to be little overlap in the methylation signatures between subjects that use alcohol and subjects that use tobacco.

As the next step of the analyses, the differential distribution of the 1000 most significantly associated probes were analyzed using the GoMiner™ algorithm (Zeeberg et al., 2003, Genome Biol., 4:R28). The results, shown in Table III, demonstrated a marked enrichment of the most highly associated probes for pathways involved in programmed cell death (apoptosis) or GTPase signaling.

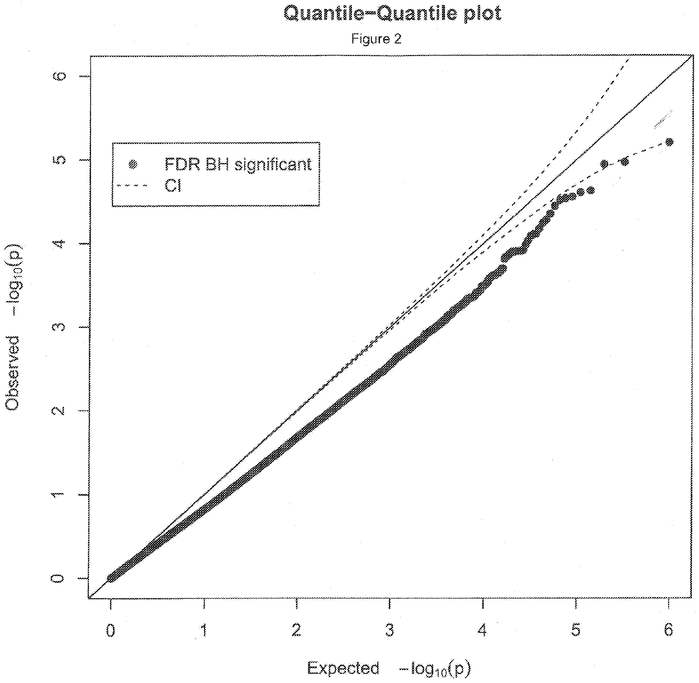

Whereas the primary goal of many biomarker studies is to determine whether a given marker can be used to differentiate pathological states from control states, a secondary goal of the present study was to determine whether DNA methylation could be used to monitor alcohol abstinence. As a first step in that assessment, the genome wide DNA methylation patterns of 25 subjects for whom we successfully obtained both T1 and T2 data were compared. The average length of time between the T1 and T2 draws for these 25 individuals was 25 days. No single probe crossed the threshold of genome-wide significance with the best observed uncorrected p-value being only 5 x 10⁻⁶ with QQ plots of the analysis demonstrating significant negative skewing was prominent (Figure 2).

**Table III. The Top 30 Most Differentially Regulated Gene Ontology Pathways**

| | | Genes | | Log10 | |
|---|---|---|---|---|---|
| GO Category | Category Name | Total | Changed | P-Value | FDR |
| GO:0005515 | protein binding | 6815 | 286 | -7.37 | 0 |
| GO:0005737 | cytoplasm | 7845 | 312 | -5.75 | 0 |
| GO:0008219 | cell death | 1392 | 78 | -5.69 | 0 |
| GO:0016265 | death | 1395 | 78 | -5.66 | 0 |
| GO:0005829 | cytosol | 1884 | 98 | -5.63 | 0 |
| GO:0012501 | programmed cell death | 1278 | 72 | -5.36 | 0 |
| GO:0007264 | small GTPase mediated signal transduction | 566 | 40 | -5.33 | 0 |
| GO:0043067 | regulation of programmed cell death | 981 | 59 | -5.30 | 0 |
| GO:0010941 | regulation of cell death | 989 | 59 | -5.20 | 0 |
| GO:0006915 | apoptosis | 1271 | 71 | -5.16 | 0 |
| GO:0042981 | regulation of apoptosis | 974 | 57 | -4.79 | 0 |
| GO:0023033 | signaling pathway | 2812 | 130 | -4.73 | 0 |
| GO:0019899 | enzyme binding | 671 | 43 | -4.63 | 0 |
| GO:0048523 | negative regulation of cellular process | 2069 | 101 | -4.61 | 0 |
| GO:0002376 | immune system process | 1256 | 68 | -4.52 | 0 |
| GO:0023034 | intracellular signaling pathway | 1707 | 86 | -4.44 | 0.01 |
| GO:0023052 | signaling | 3787 | 164 | -4.36 | 0.01 |
| GO:0065007 | biological regulation | 7226 | 283 | -4.33 | 0.01 |
| GO:0048519 | negative regulation of biological process | 2235 | 106 | -4.31 | 0.01 |
| GO:0035556 | intracellular signal transduction | 1454 | 75 | -4.24 | 0.01 |
| GO:0005622 | intracellular | 11231 | 411 | -4.22 | 0.01 |
| GO:0060548 | negative regulation of cell death | 441 | 31 | -4.21 | 0.01 |
| GO:0044464 | cell part | 14663 | 511 | -4.14 | 0.02 |
| GO:0005623 | cell | 14664 | 511 | -4.13 | 0.01 |
| GO:0035466 | regulation of signaling pathway | 1158 | 62 | -4.02 | 0.02 |
| GO:0009987 | cellular process | 11702 | 424 | -3.98 | 0.02 |
| GO:0043069 | negative regulation of cell death | 434 | 30 | -3.96 | 0.02 |
| GO:0007265 | Ras protein signal transduction | 335 | 25 | -3.91 | 0.02 |
| GO:0051056 | regulation of GTPase signal transduction | 339 | 25 | -3.83 | 0.03 |
| GO:0050794 | regulation of cellular process | 6319 | 249 | -3.81 | 0.03 |

| | | | | | |
|---|---|---|---|---|---|
| FDR=false discovery rate. | | | | | |

Secondary analysis of the T1 and T2 proved highly interesting. Since exposure to ethanol is stressful to cells, and biological systems tend to revert to their homeostatic means after perturbation, we next asked which methylation assessment for the ethanol-imbibing subjects, T1 or T2, was more similar to that of the controls for the 8636 FDR-significant probes identified. Remarkably, the average methylation of these CpG residues for all 25 subjects was more similar to the control subjects at the T2 draw time than it was at the T1 draw time at 7360 of 8636 probes (Chi Square *p*<0.0001) including all 30 of those listed in Table II. Unfortunately, the average version to the mean of the controls was rather modest at each locus, with the overall change in the beta value being approximately 0.005 (i.e. 0.5%).

### Example 3-Summary

The experiments herein demonstrate that alcohol use is associated with significant and widespread changes in DNA methylation as compared to controls that do not use alcohol. The experiments herein also demonstrate that the degree of the changes in methylation tends to diminish after approximately one month of abstinence. Thus, the DNA methylation signature can be used to infer recent alcohol use status. The results reported herein likely will impact the choice of settings in which alcohol treatment is conducted and monitored.

As would be expected with alcohol use, the magnitude of the changes in DNA methylation due to alcohol use are not as strong as those observed with, for example, tobacco use. In contrast to that of smoking, for example, where the differences of methylation at cg05575921 between chronic smokers (∼60%) and non-smokers (92%) can exceed 30%, the average differences at any one point in the current alcohol use study tend to be around 5% to 10%.

It is to be understood that, while the methods and compositions of matter have been described herein in conjunction with a number of different aspects, the foregoing description of the various aspects is intended to illustrate and not limit the scope of the methods and compositions of matter. Other aspects, advantages, and modifications are within the scope of the following claims.

Disclosed are methods and compositions that can be used for, can be used in conjunction with, can be used in preparation for, or are products of the disclosed methods and compositions. These and other materials are disclosed herein, and it is understood that combinations, subsets, interactions, groups, etc. of these methods and compositions are disclosed. That is, while specific reference to each various individual and collective combinations and permutations of these compositions and methods may not be explicitly disclosed, each is specifically contemplated and described herein. For example, if a particular composition of matter or a particular method is disclosed and discussed and a number of compositions or methods are discussed, each and every combination and permutation of the compositions and the methods are specifically contemplated unless specifically indicated to the contrary. Likewise, any subset or combination of these is also specifically contemplated and disclosed.

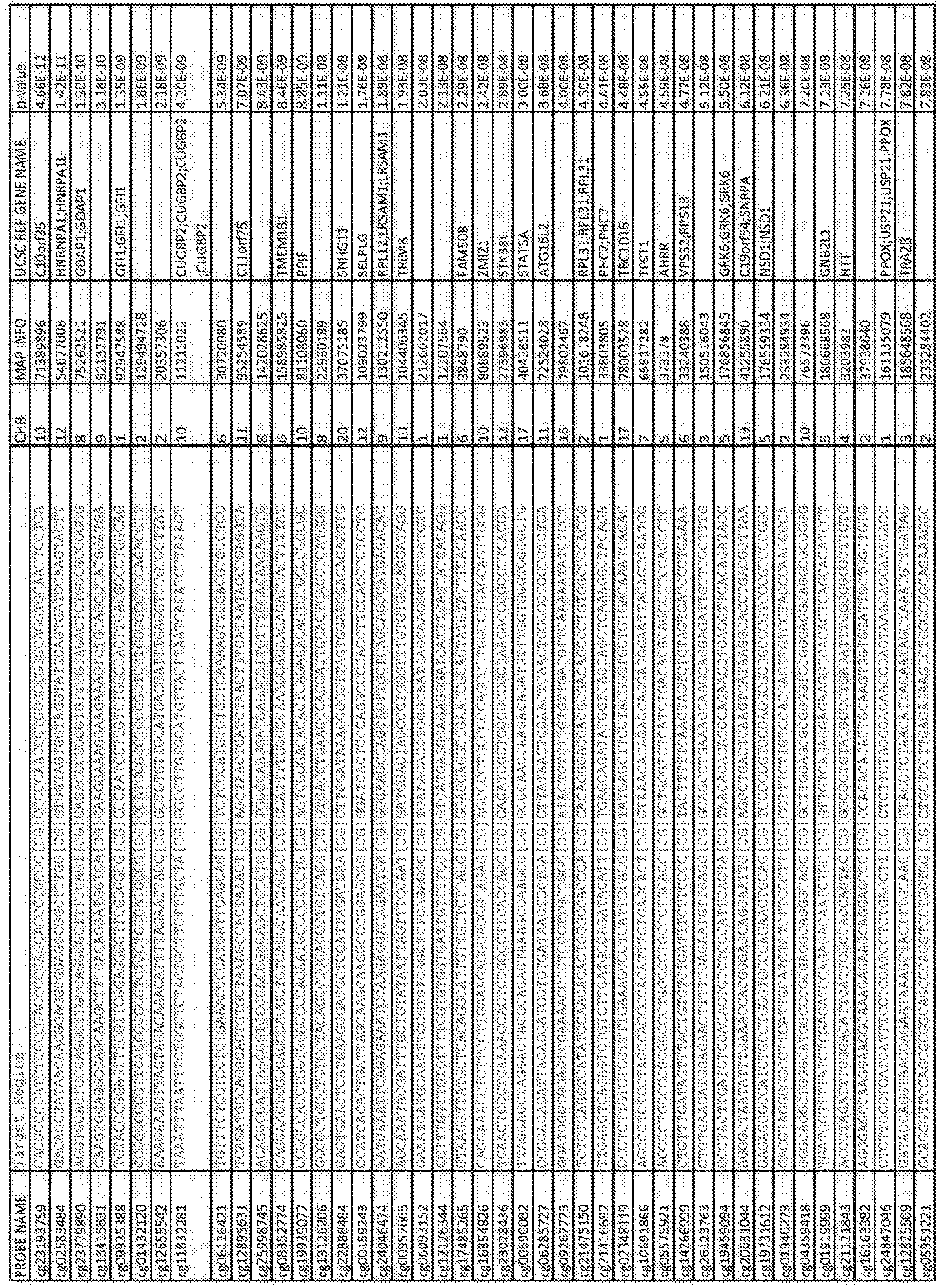

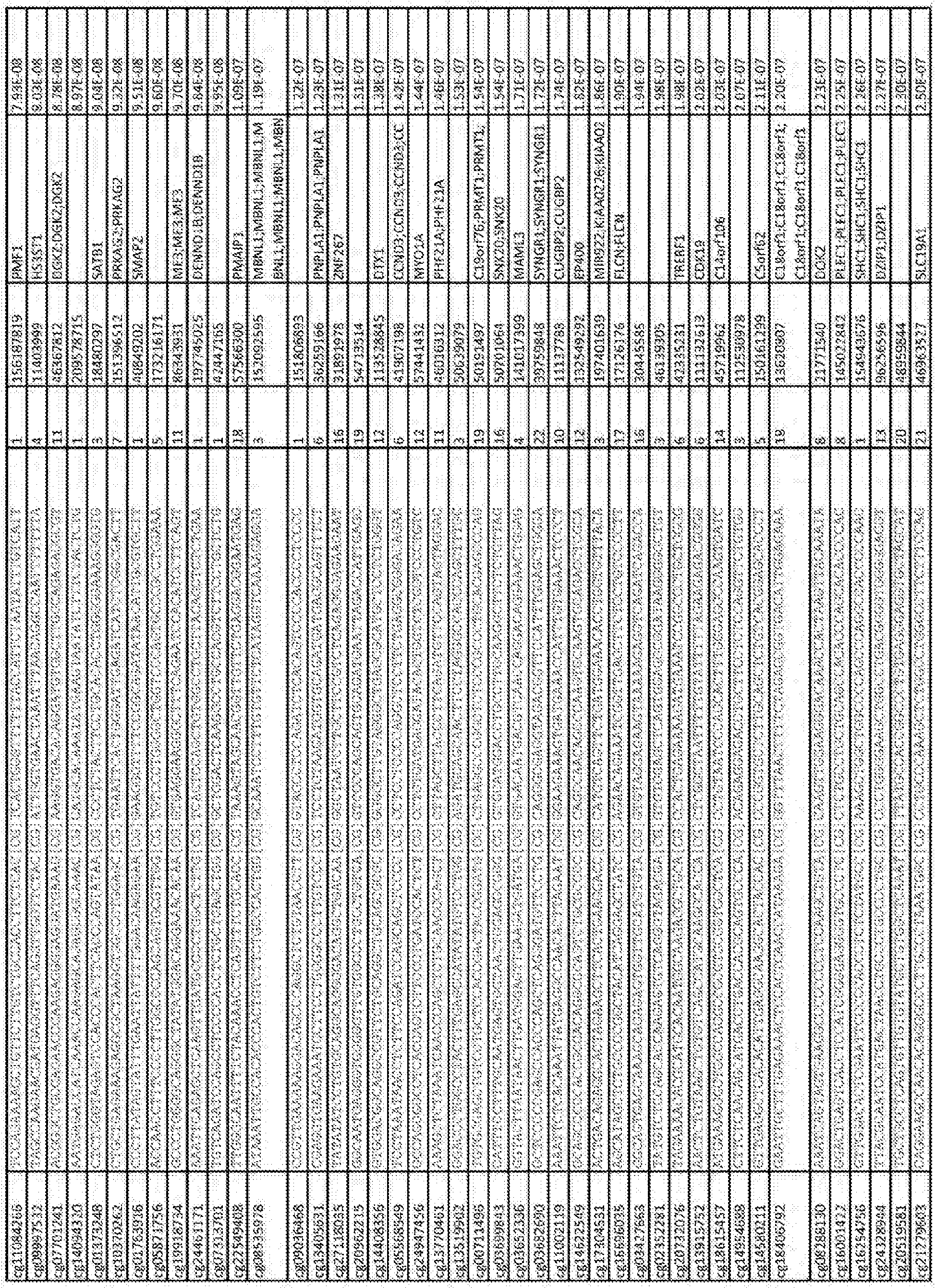

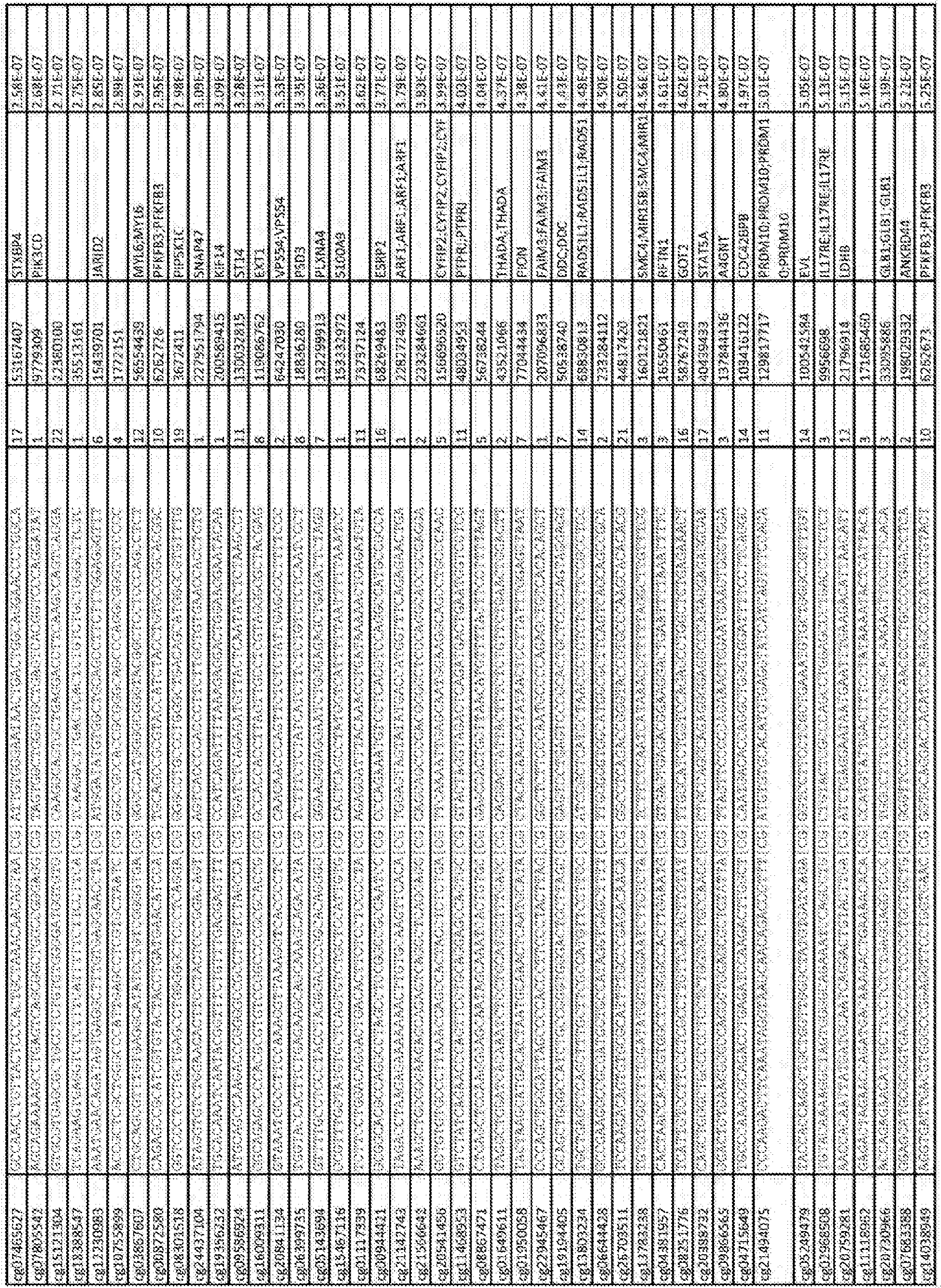

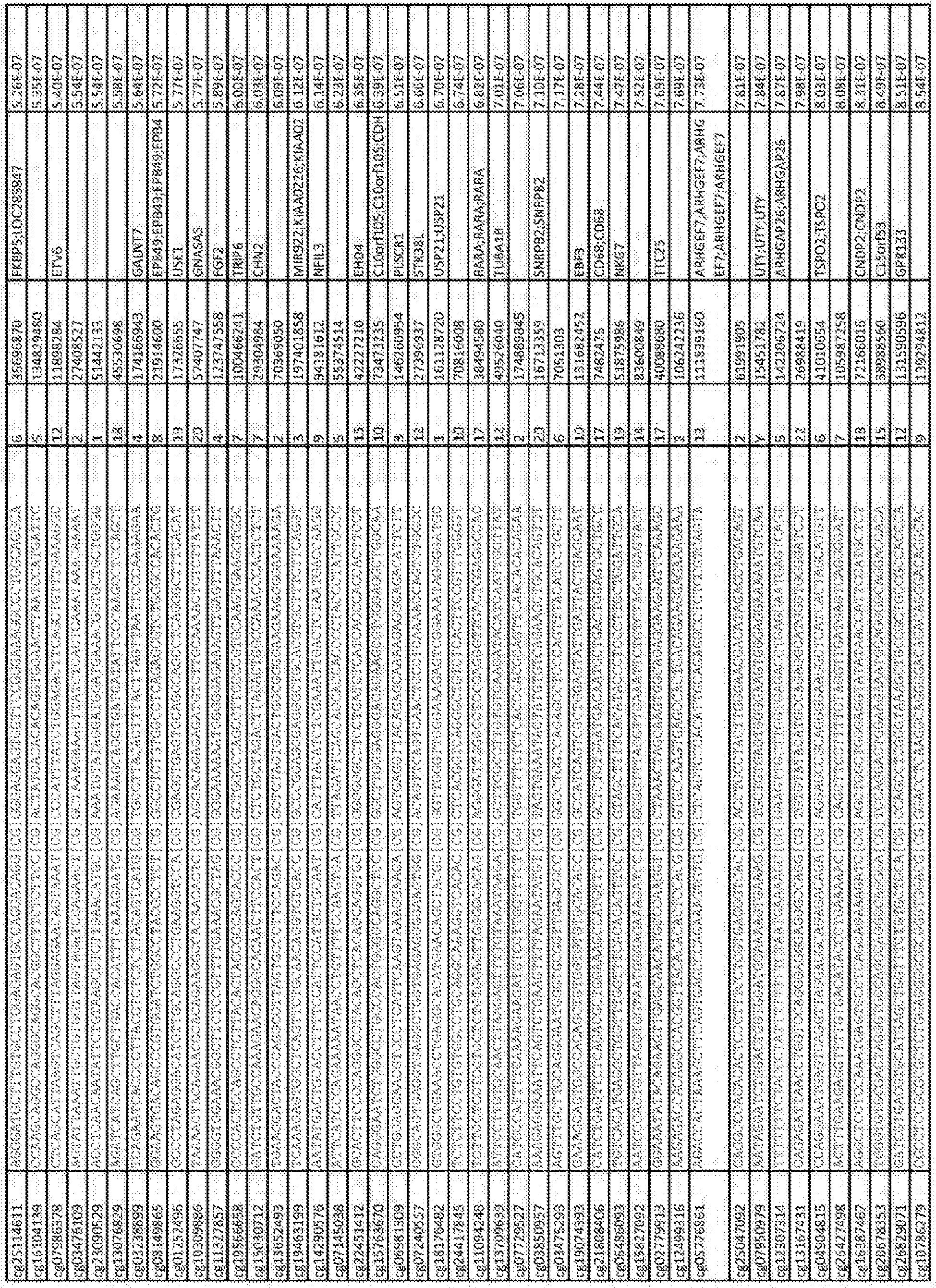

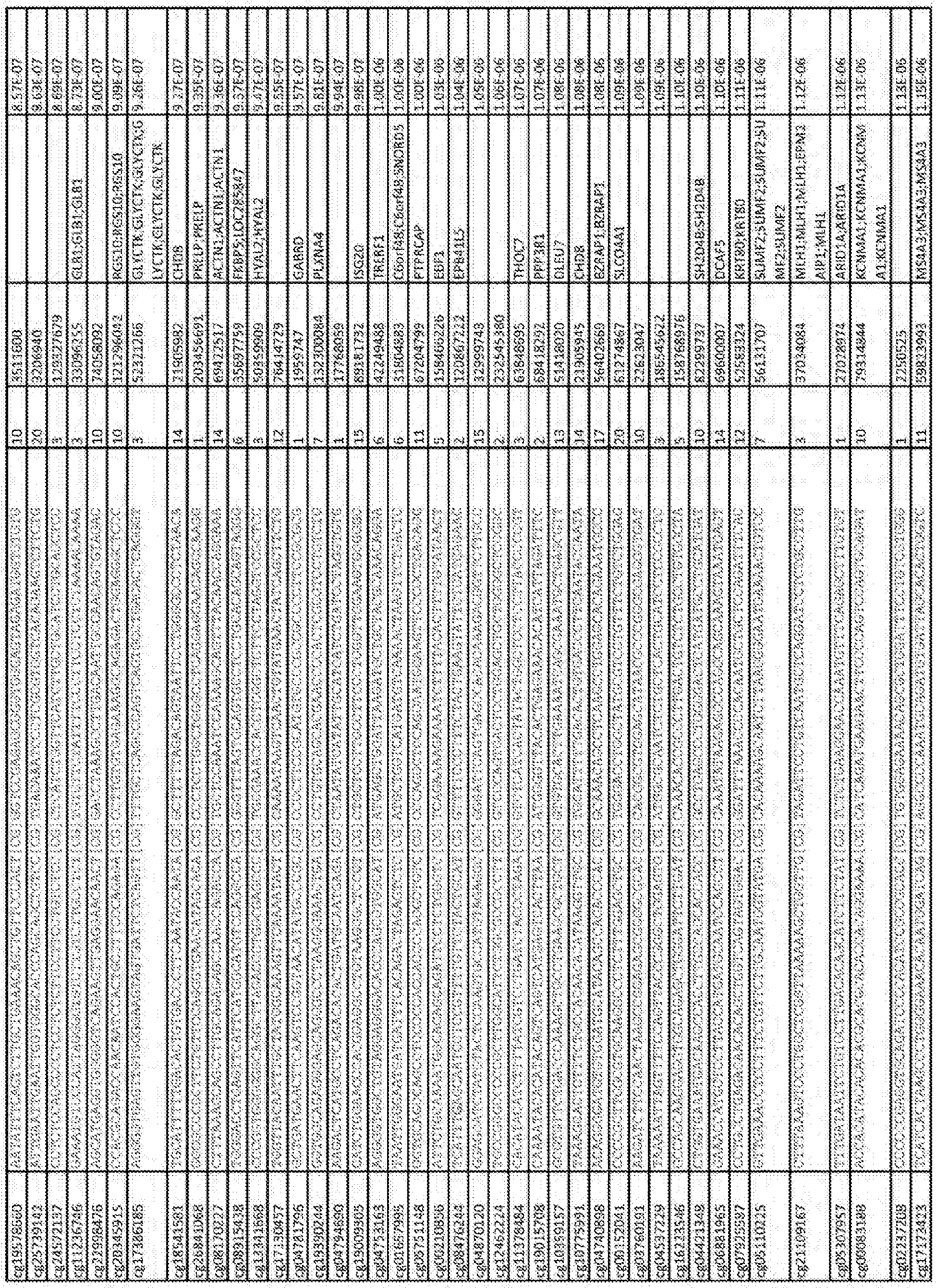

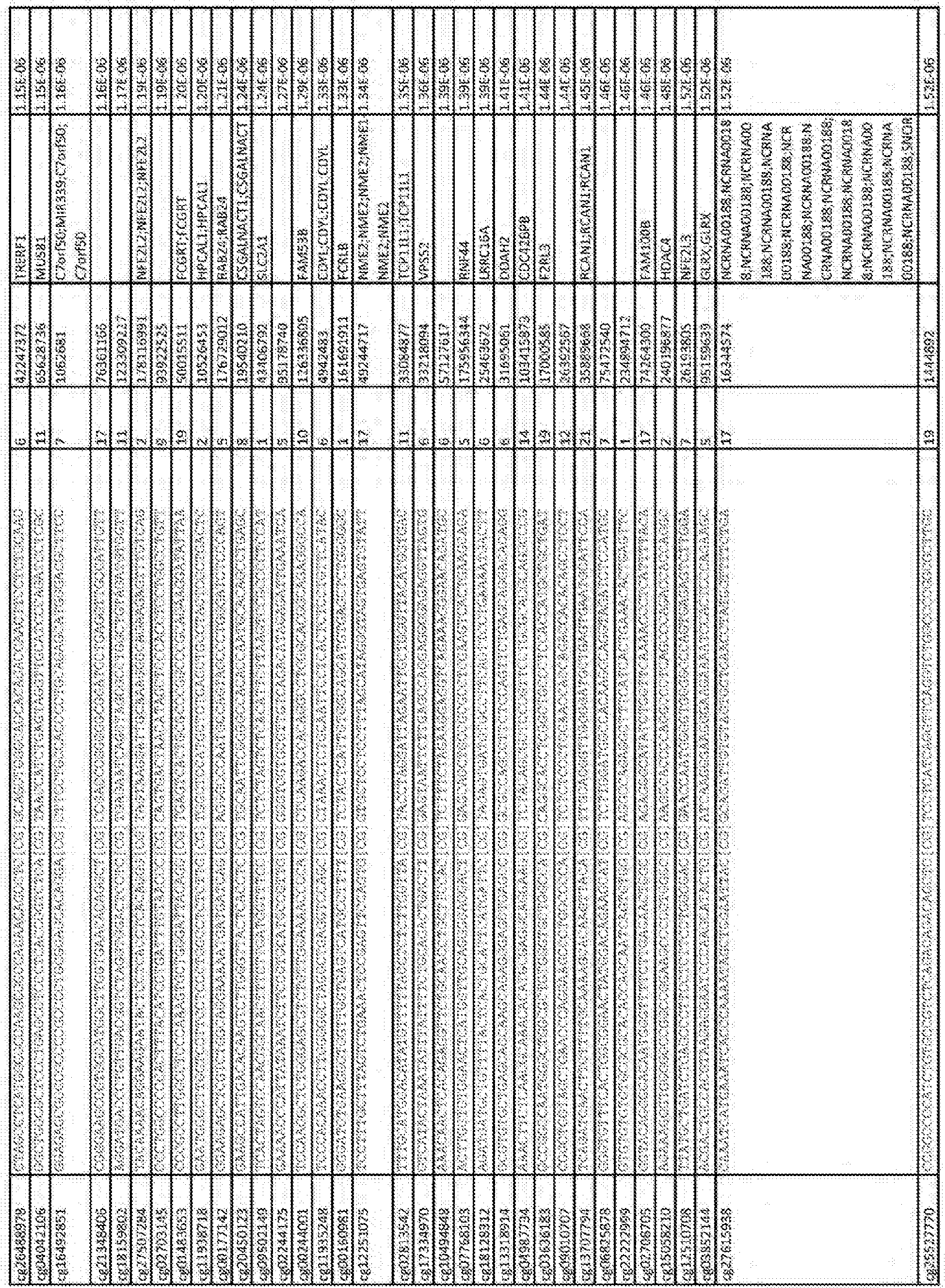

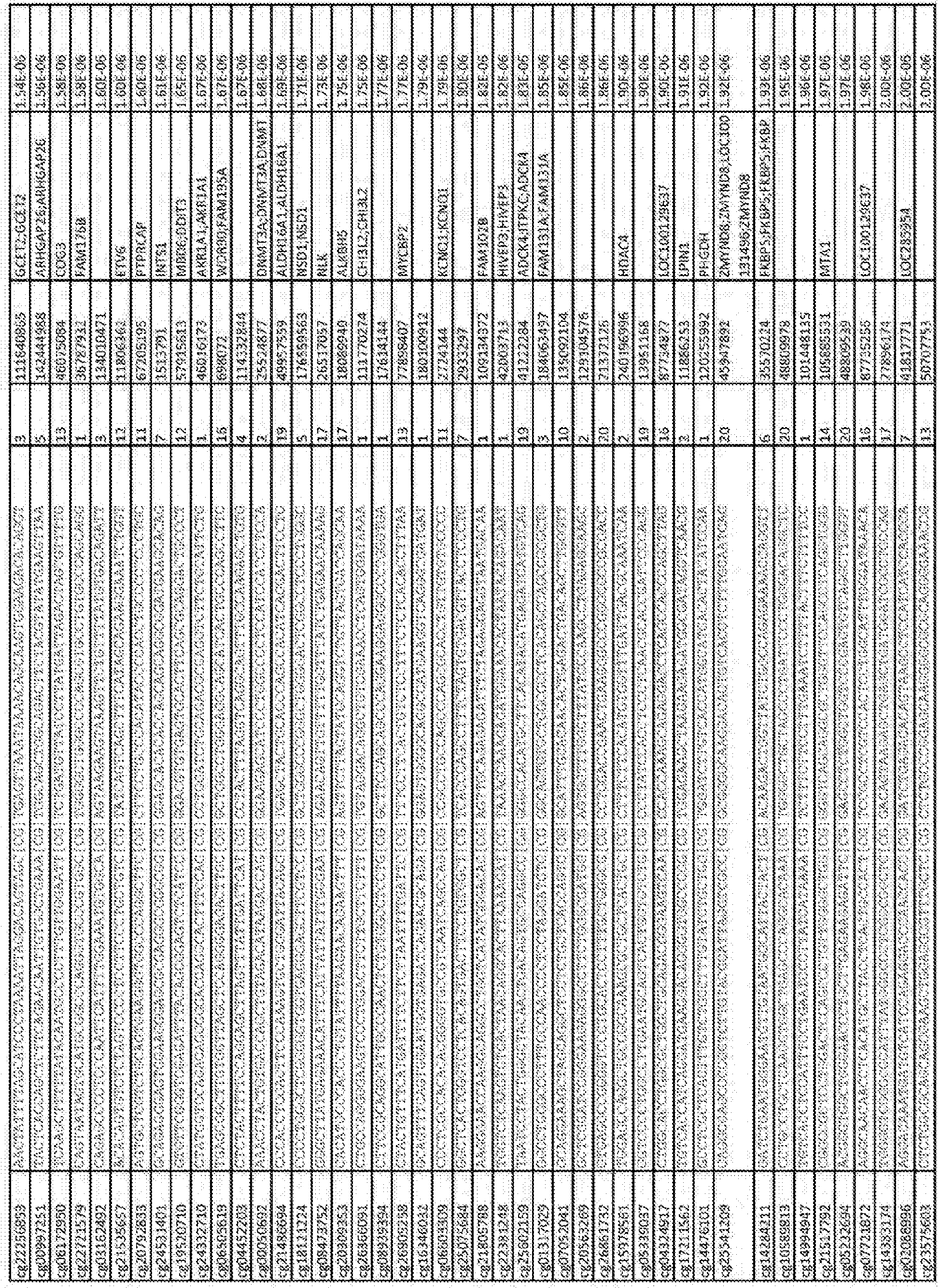

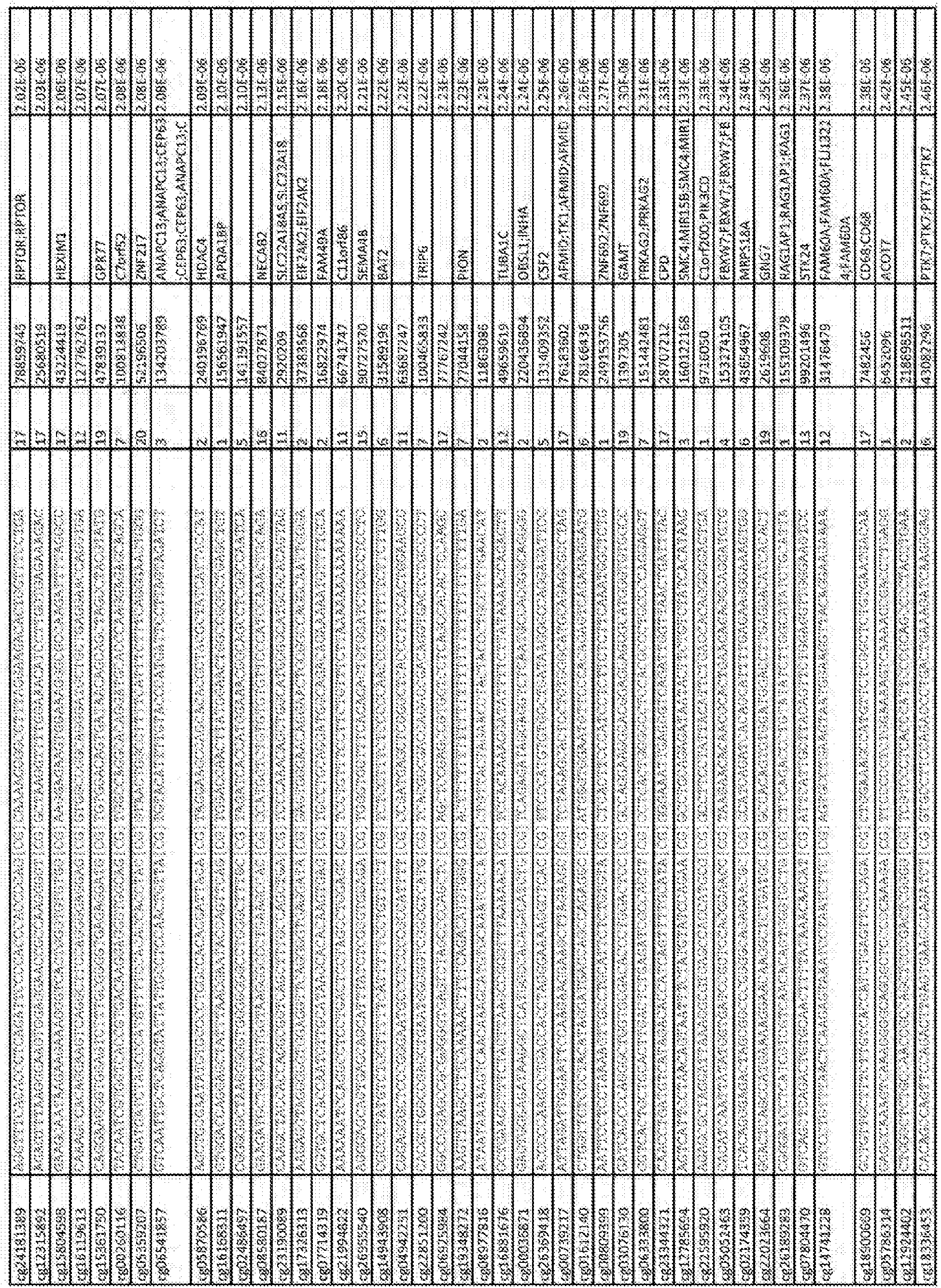

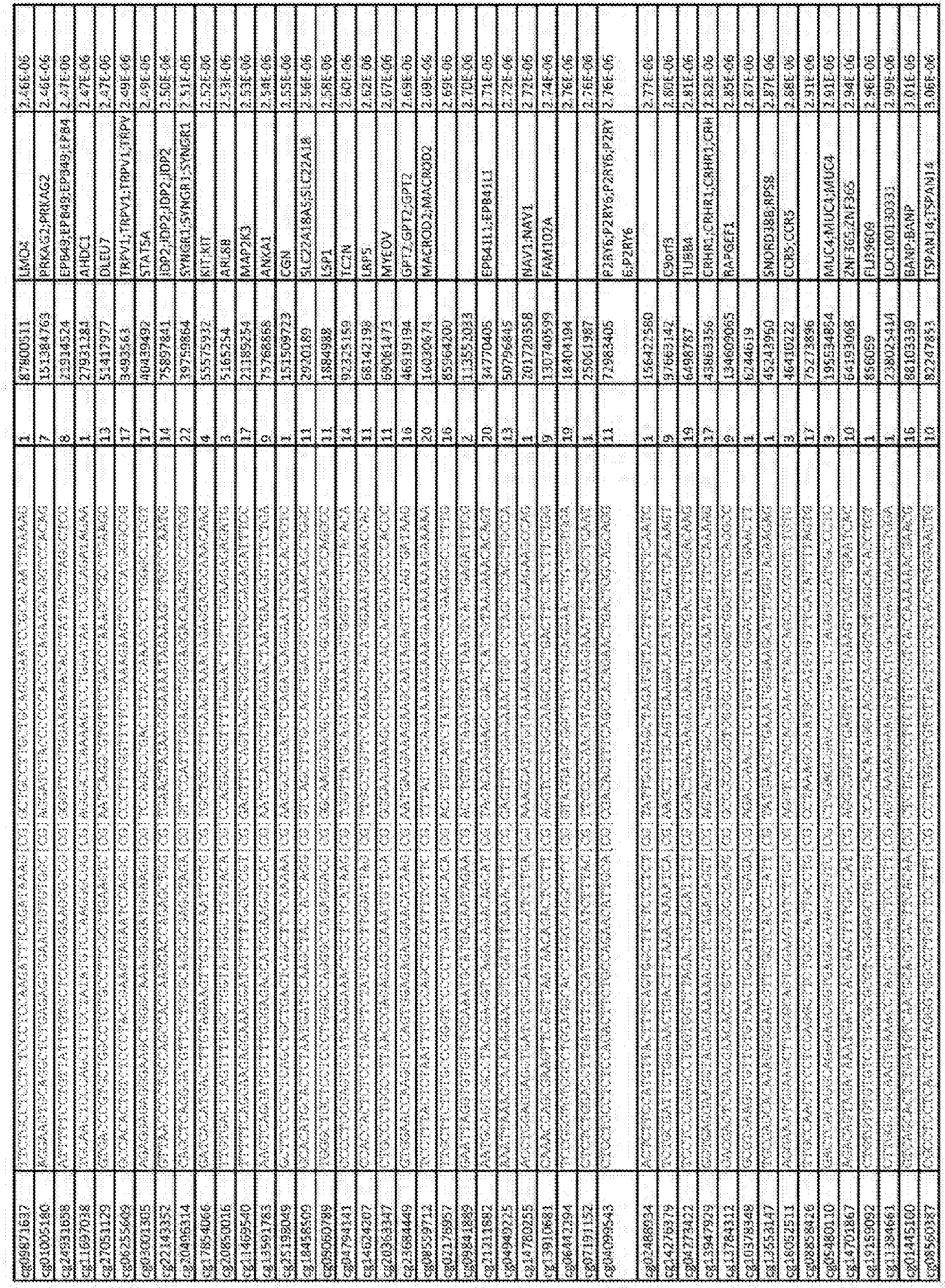

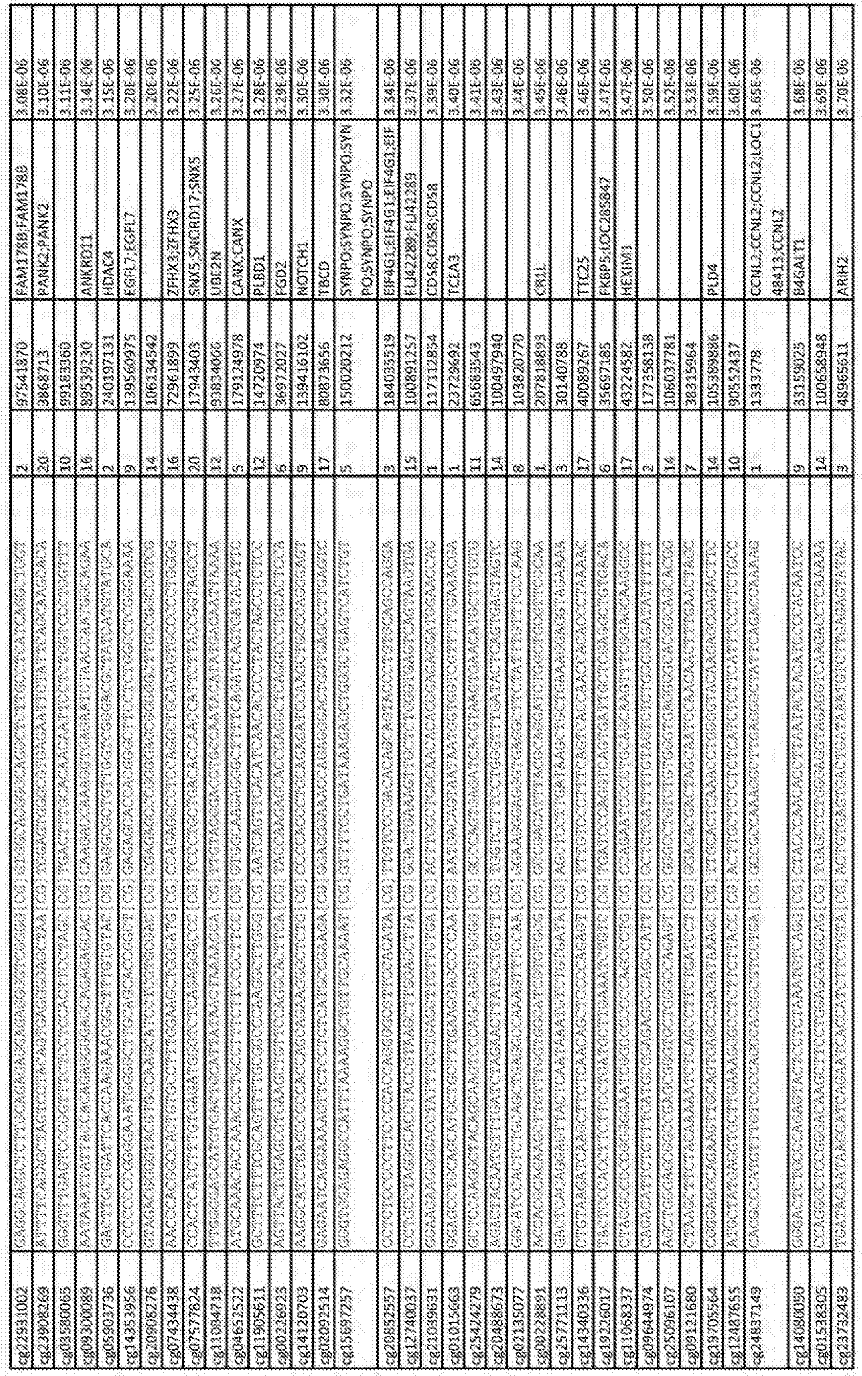

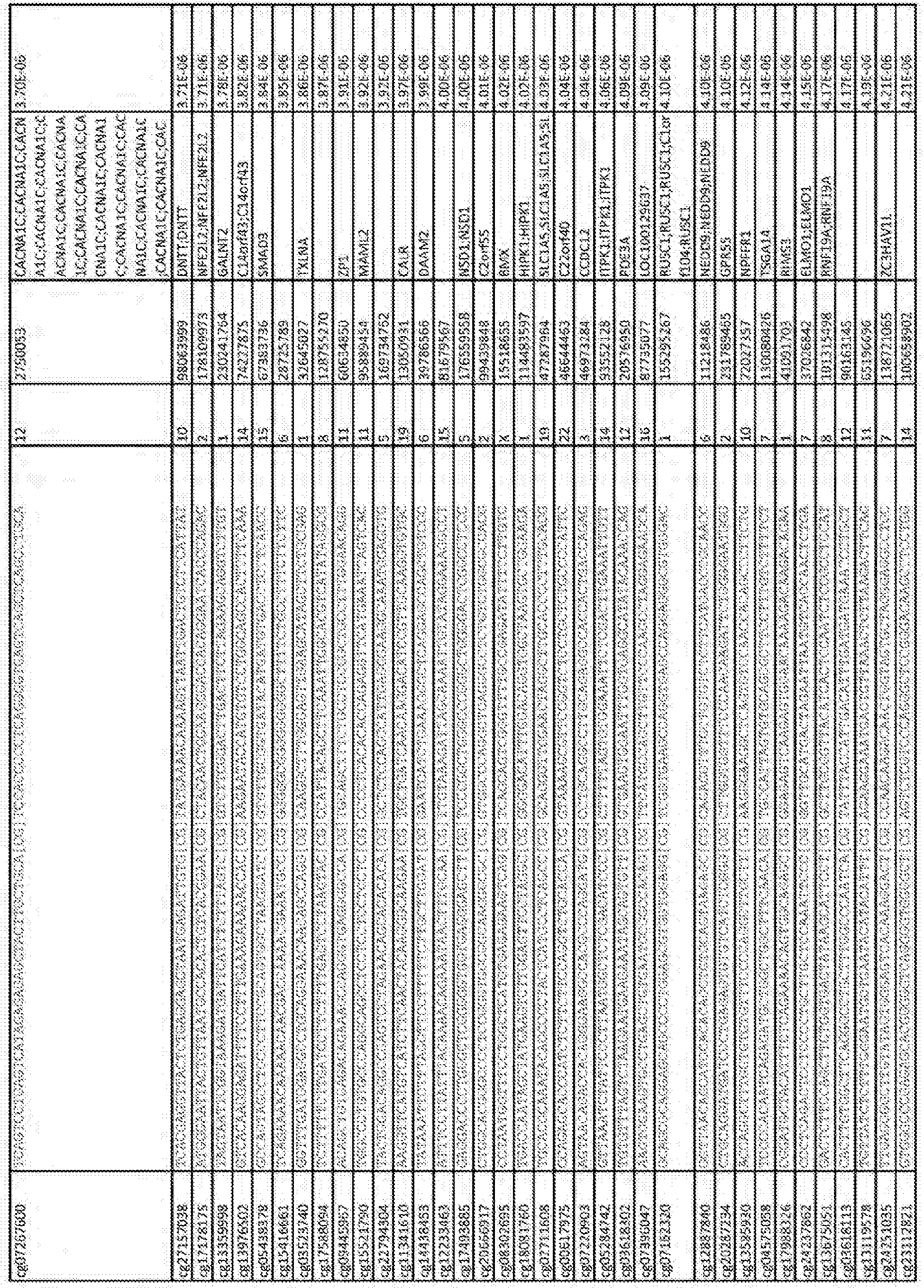

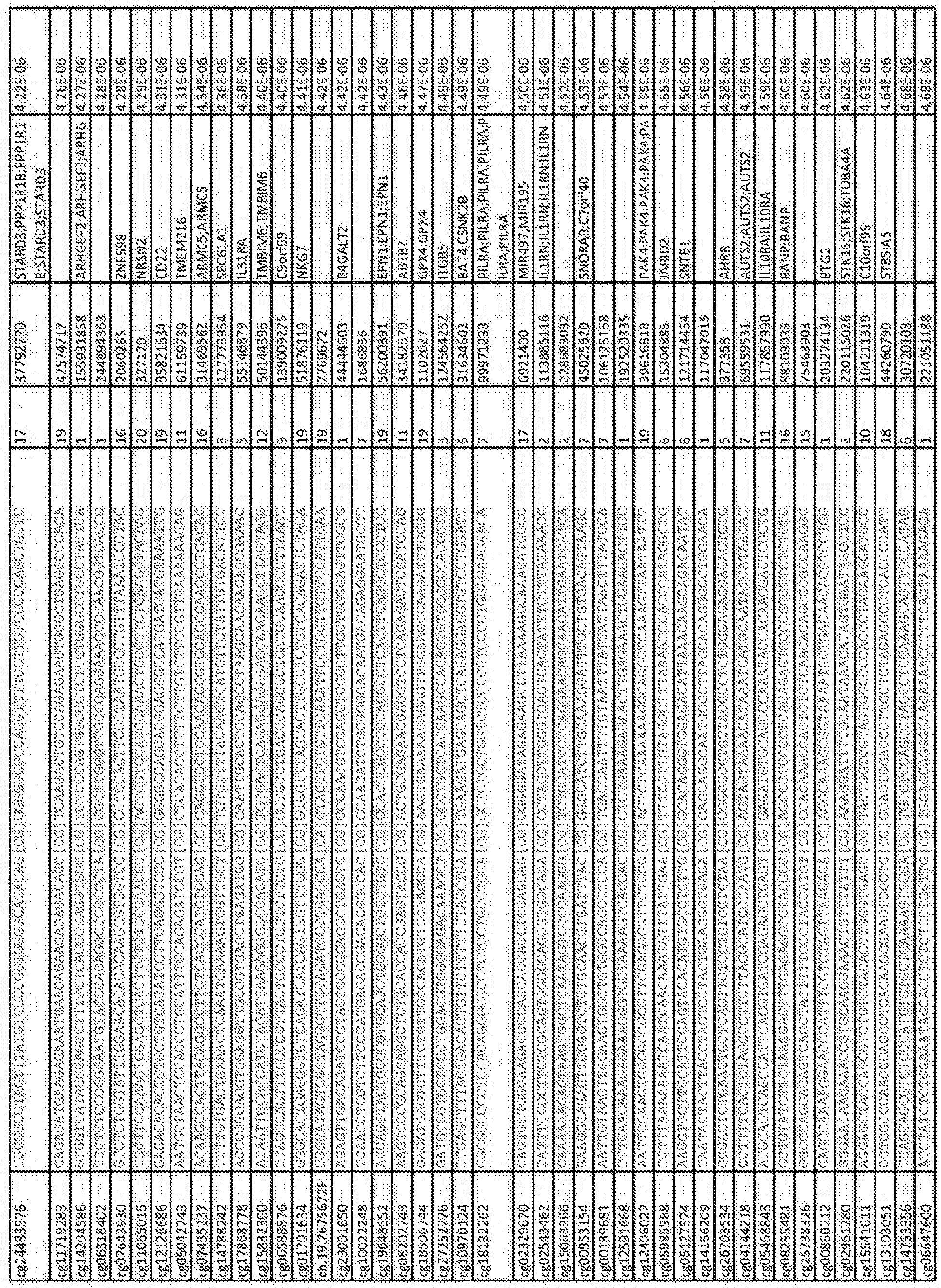

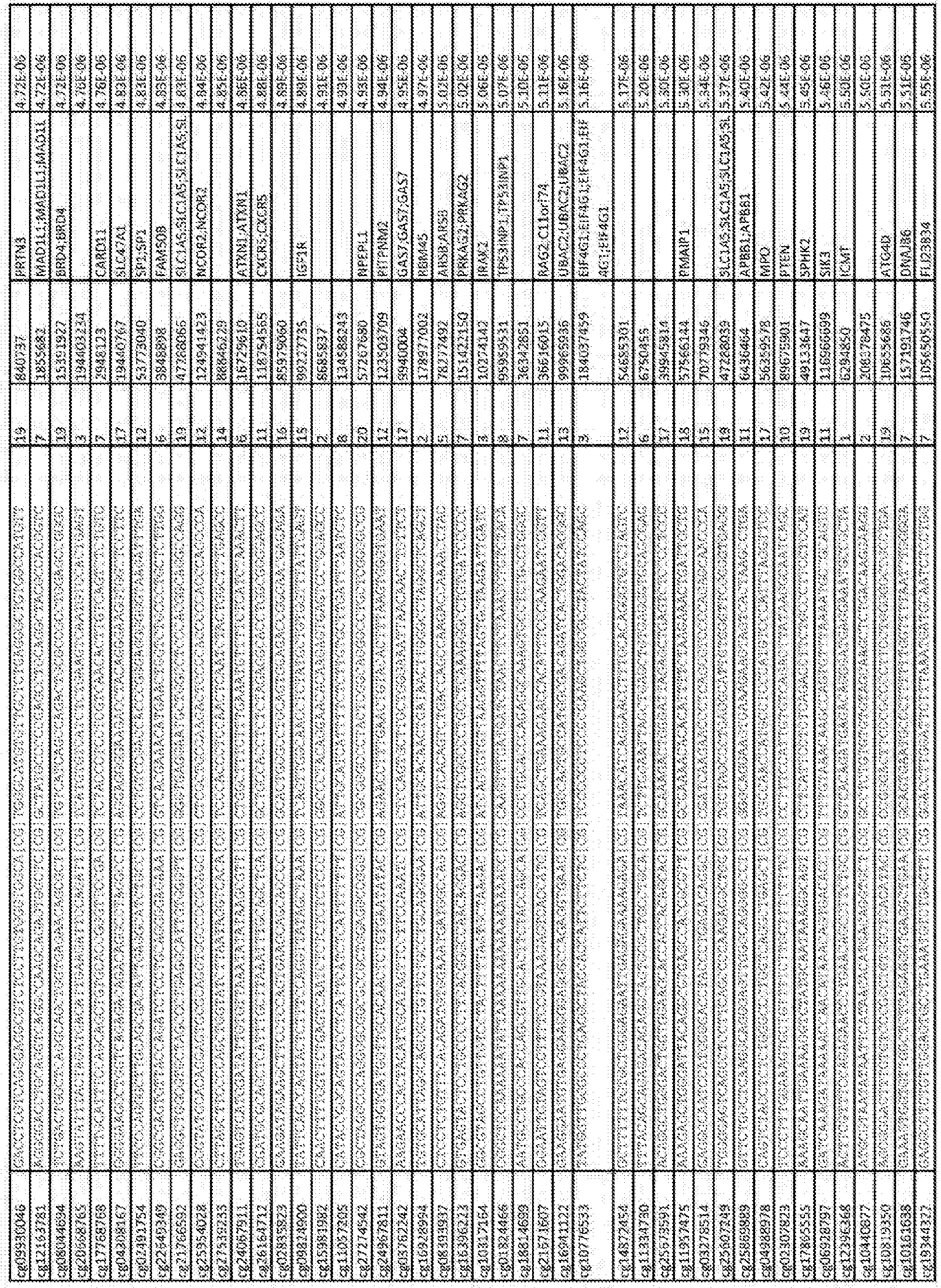

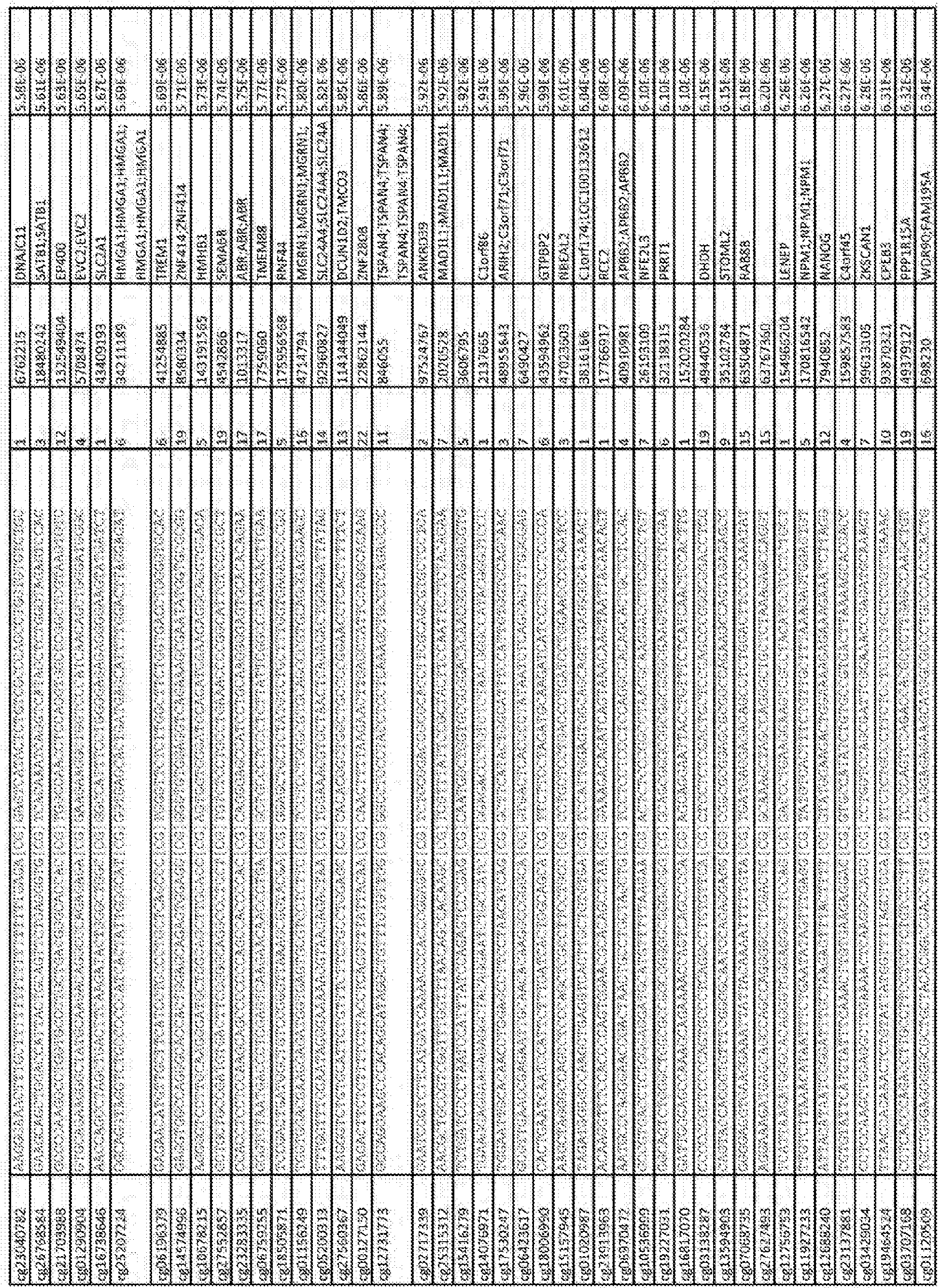

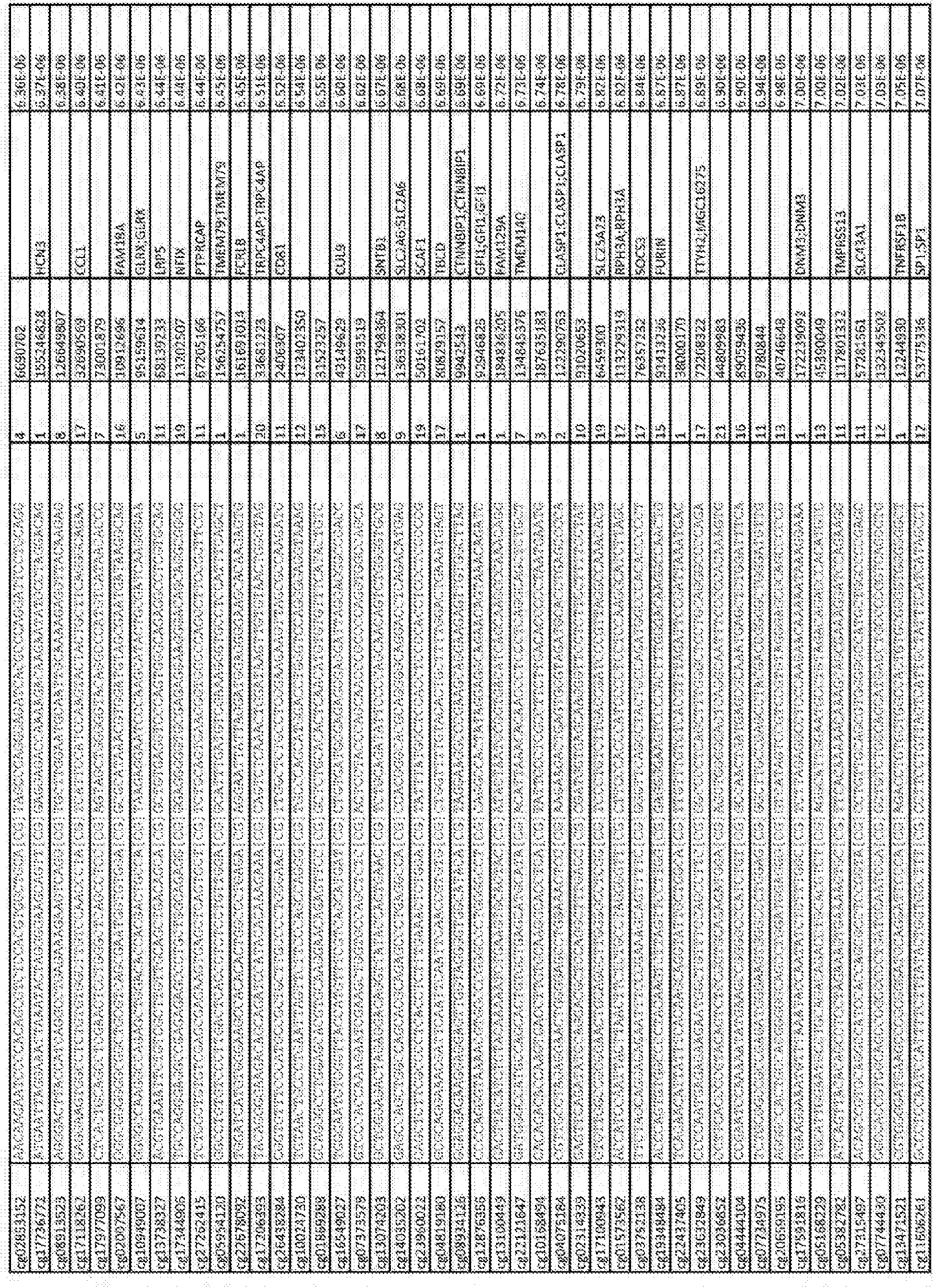

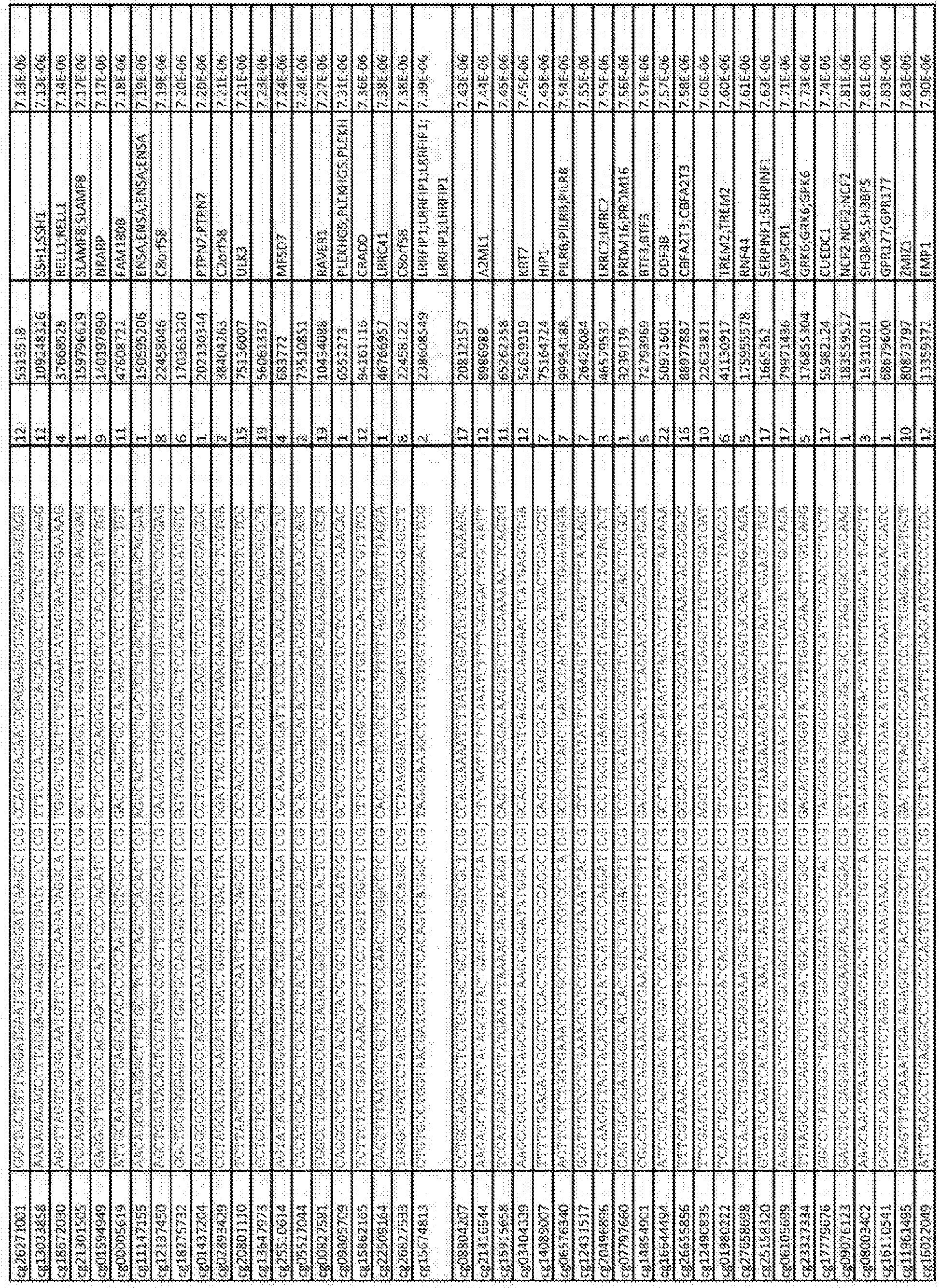

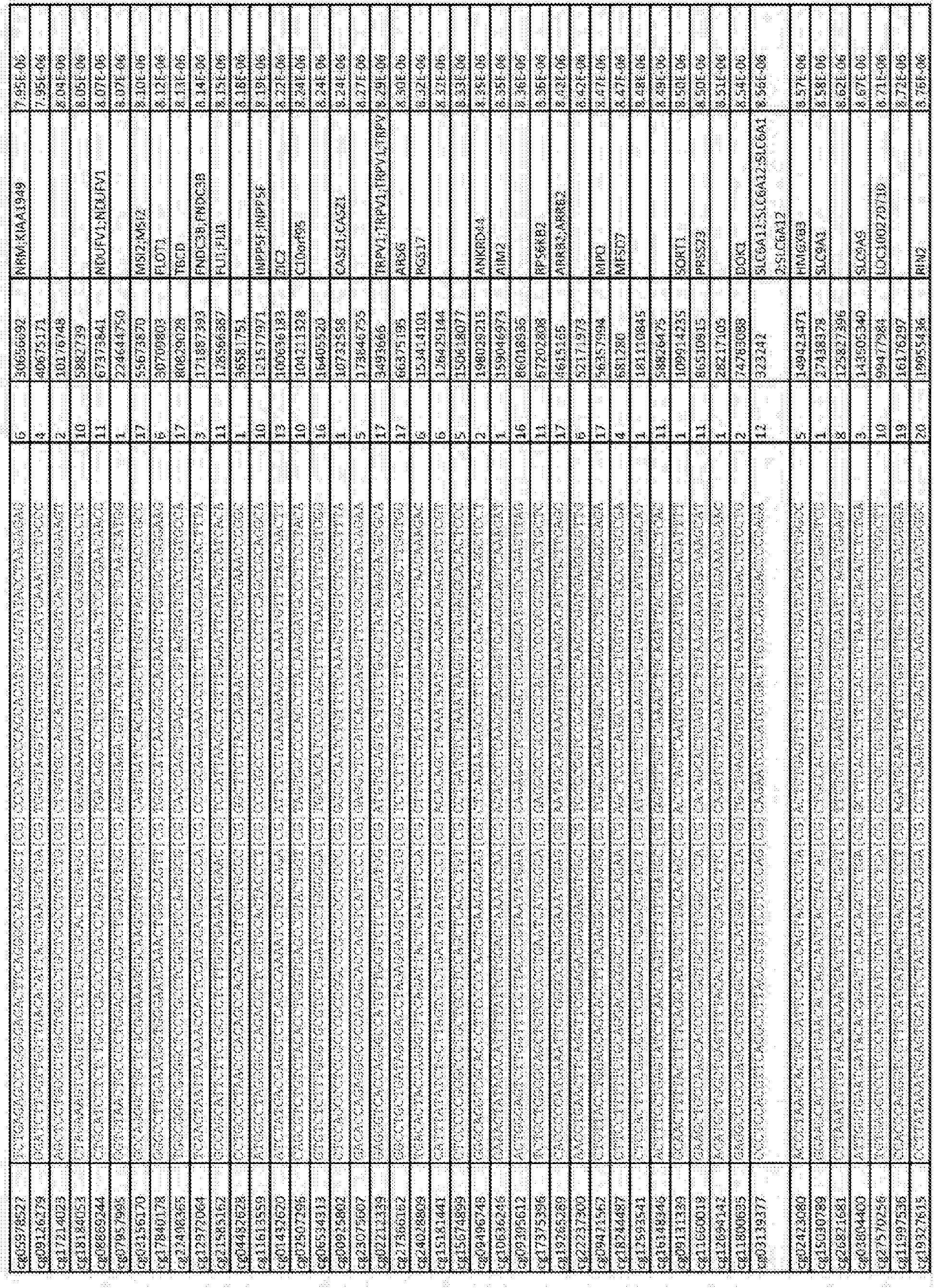

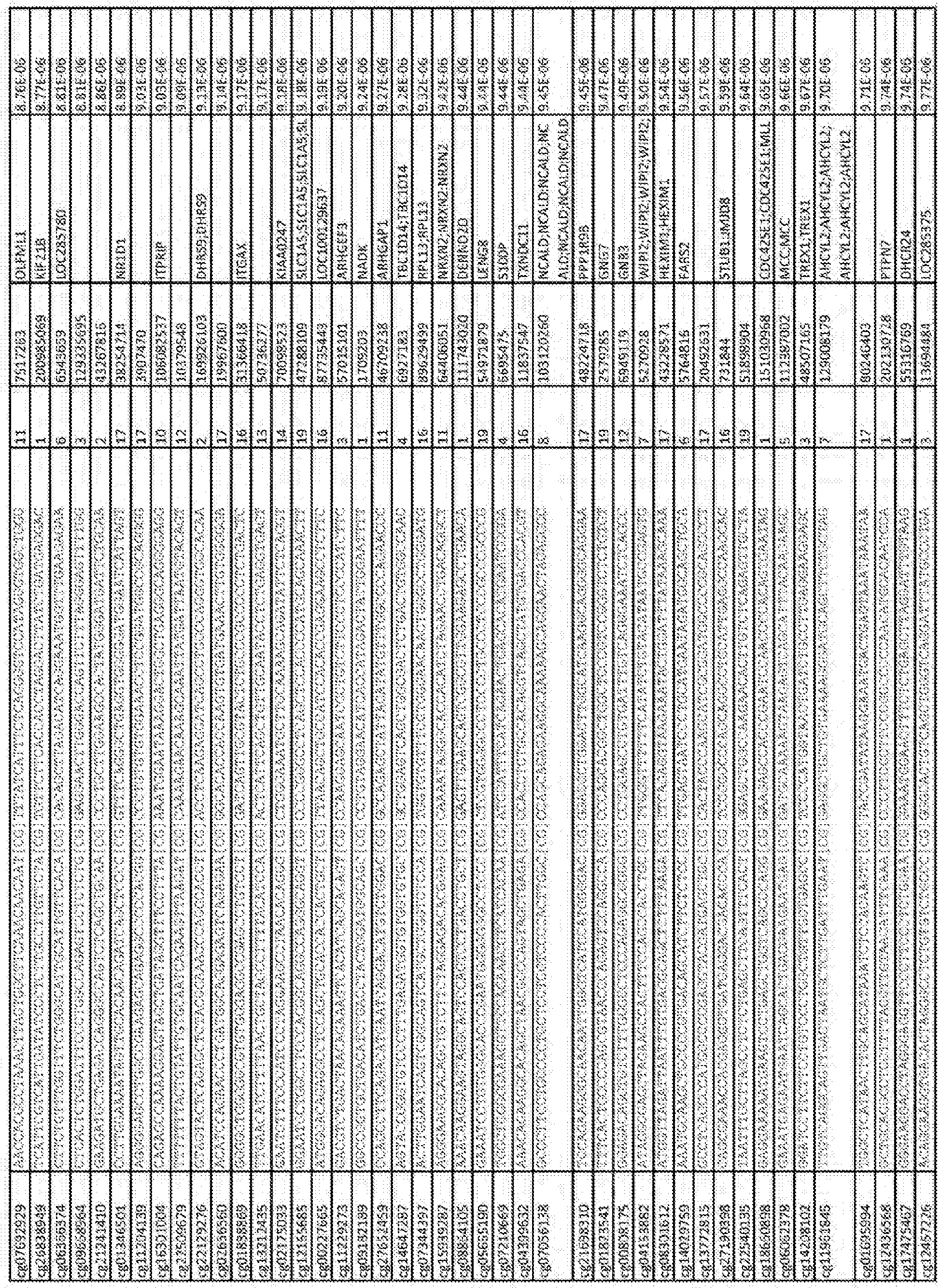

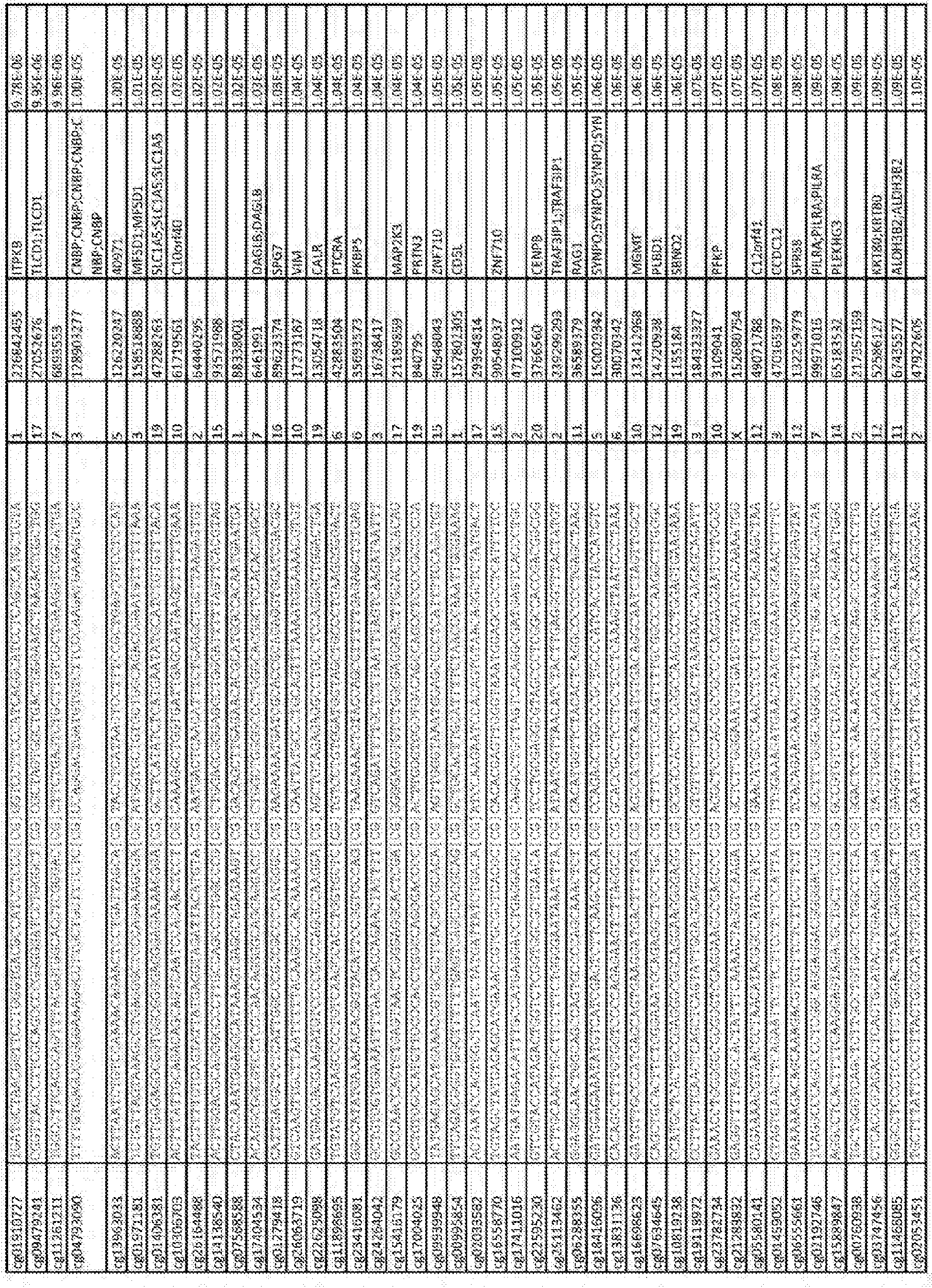

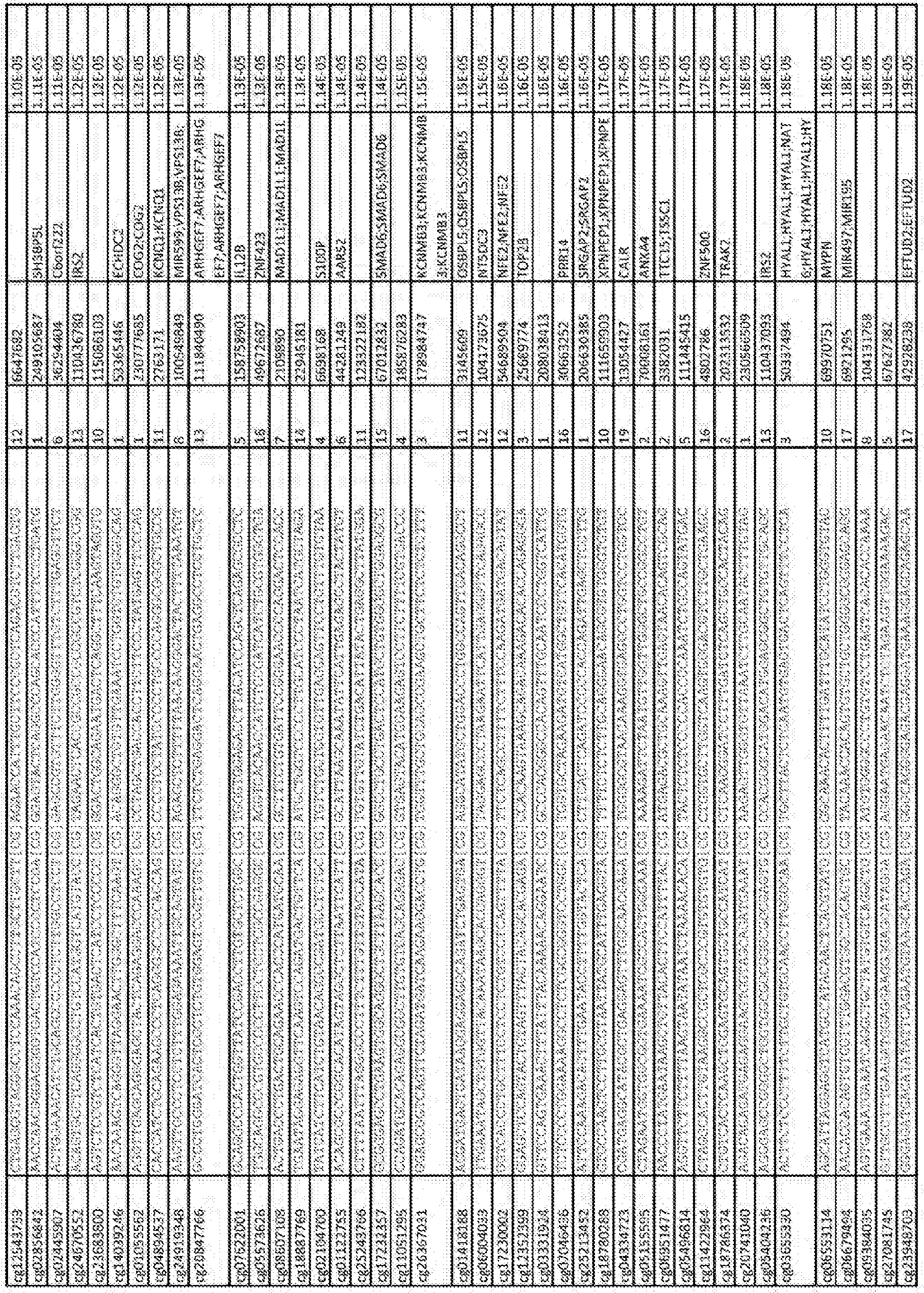

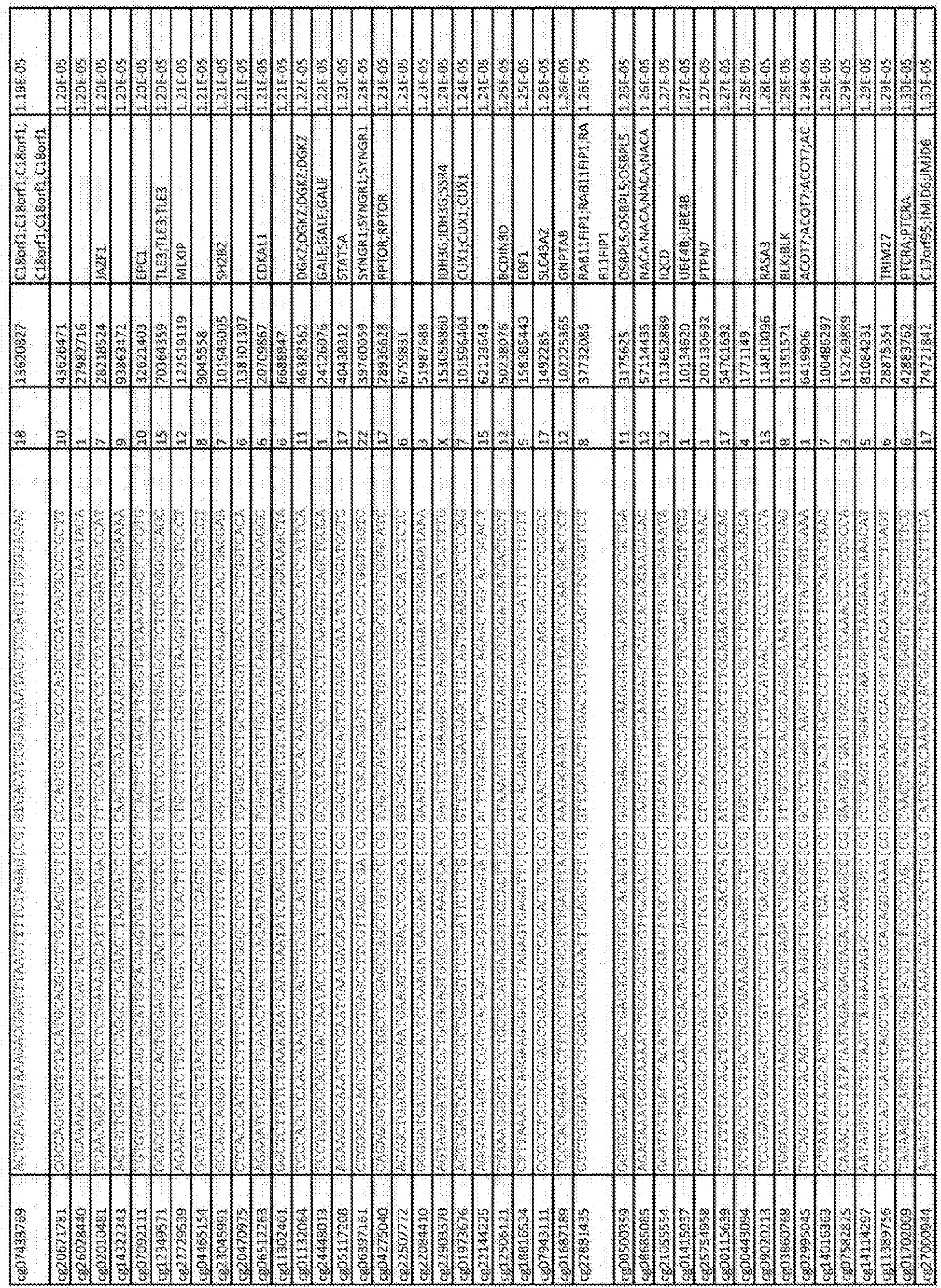

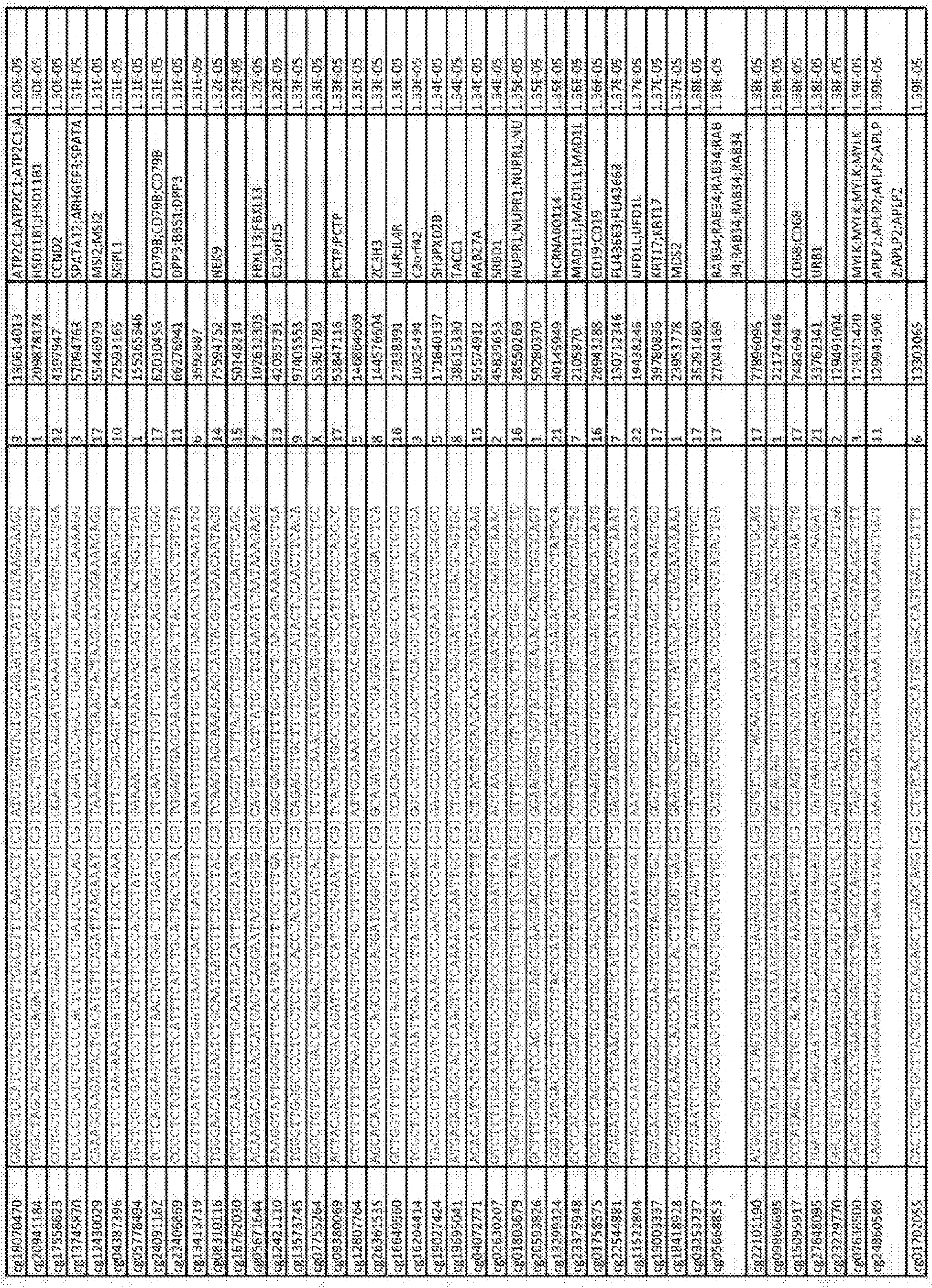

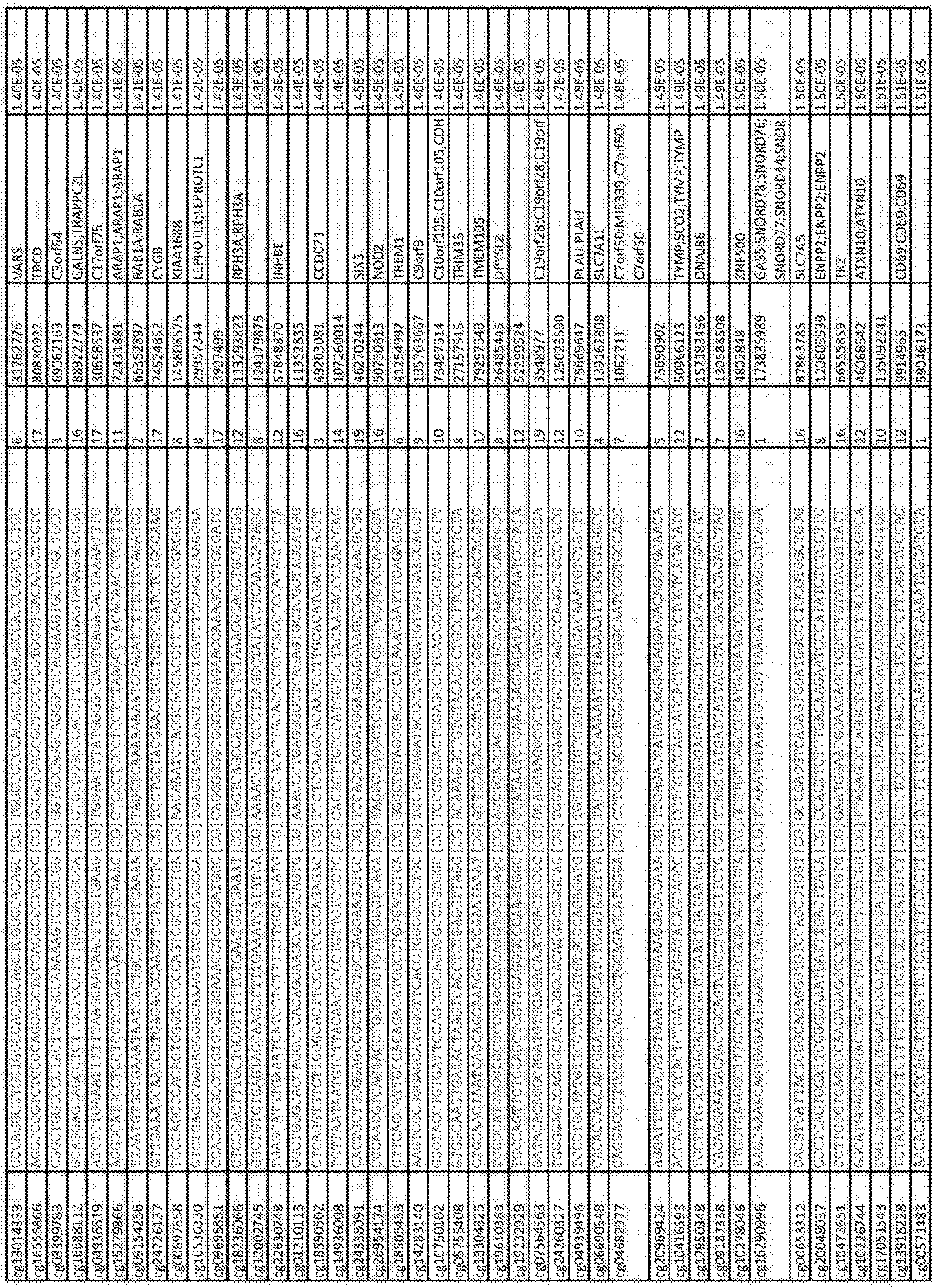

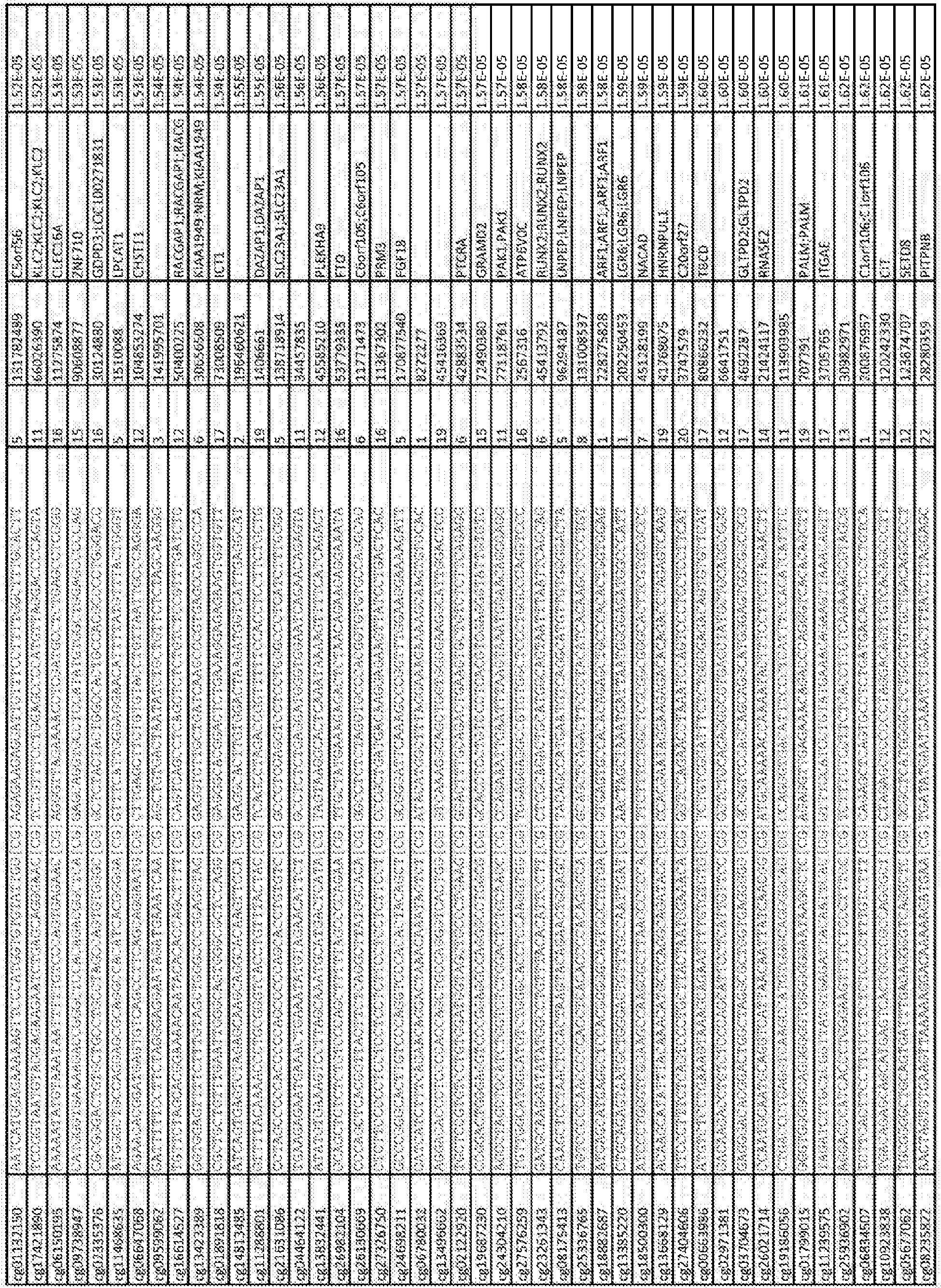

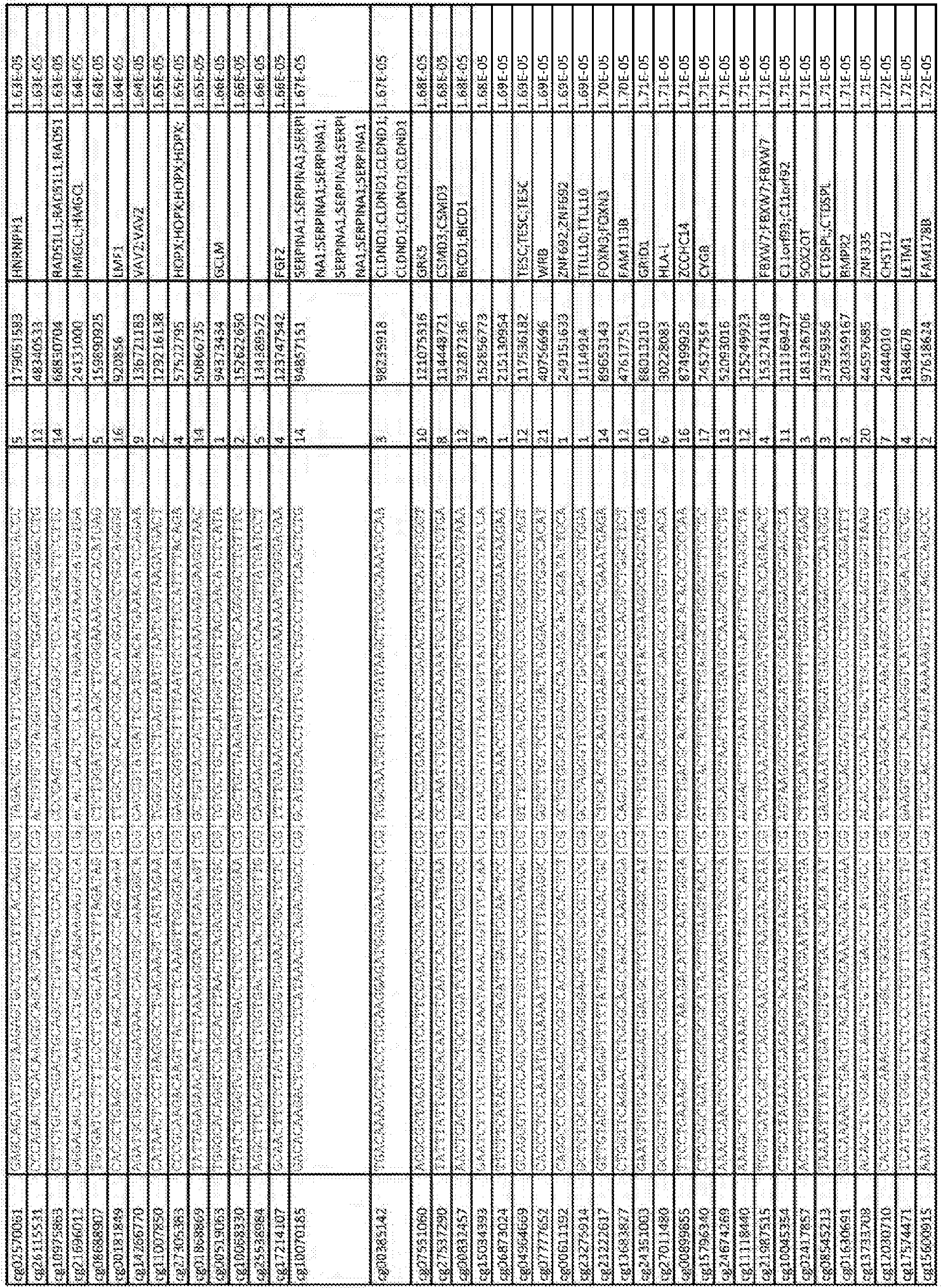

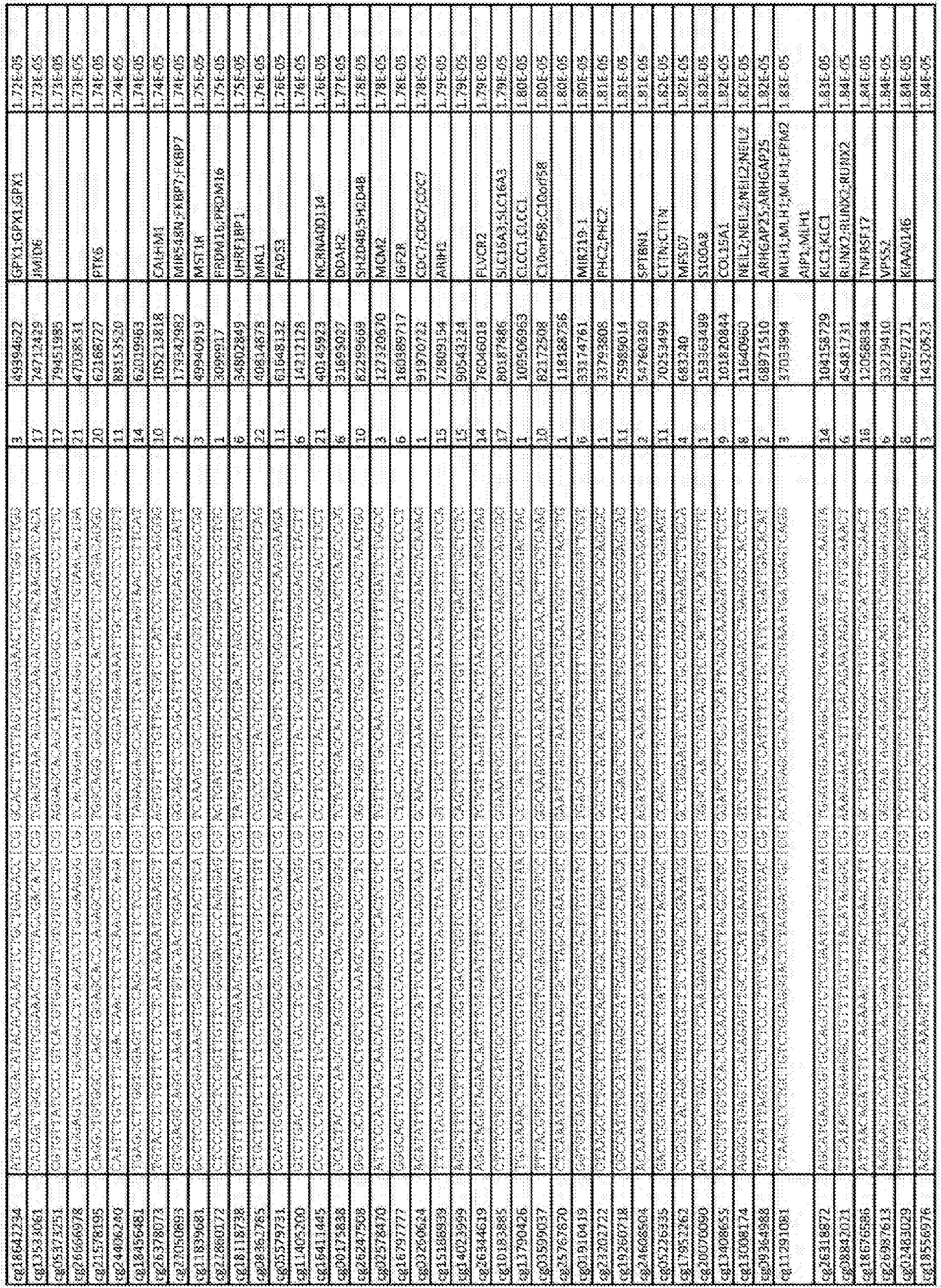

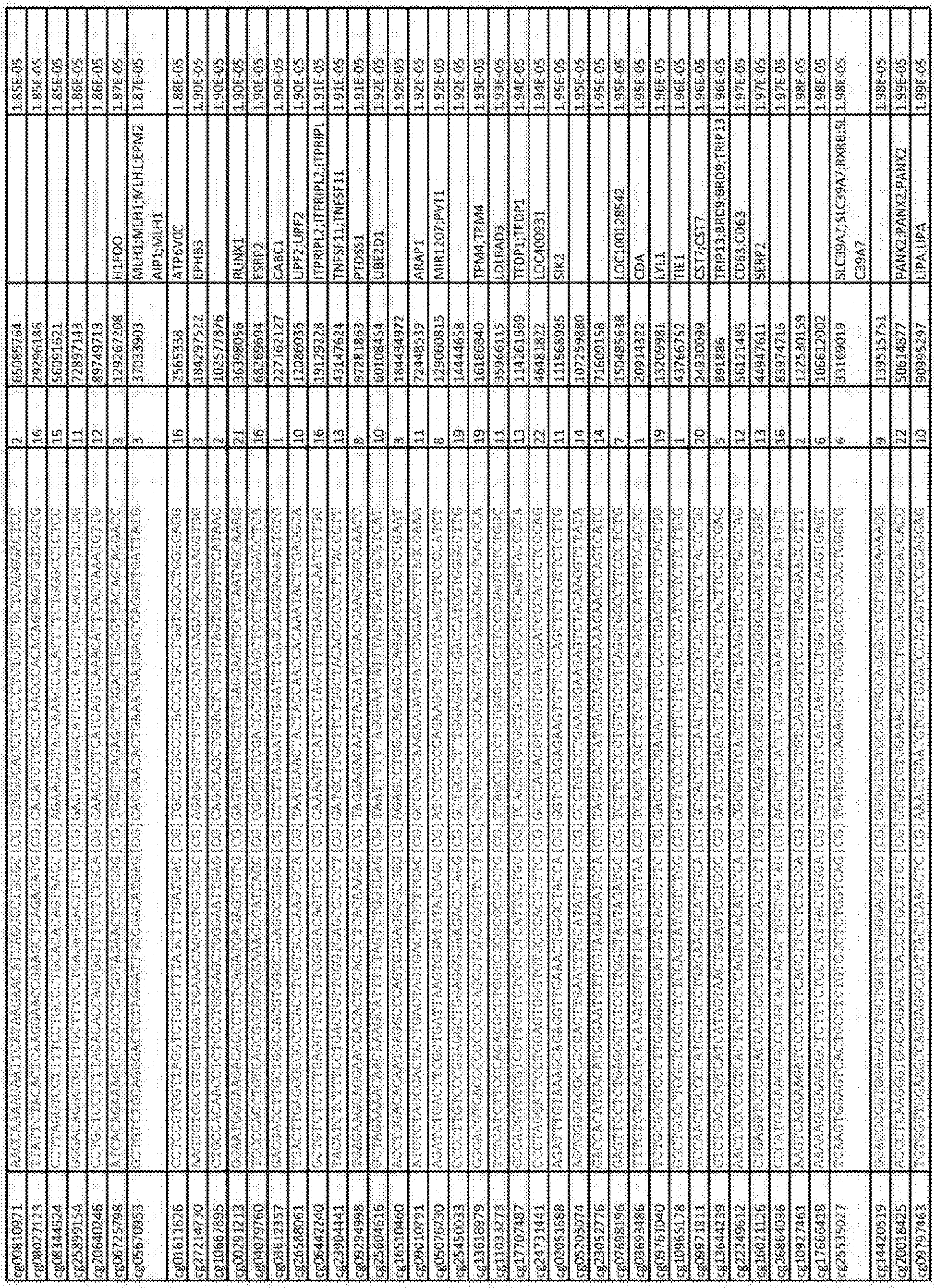

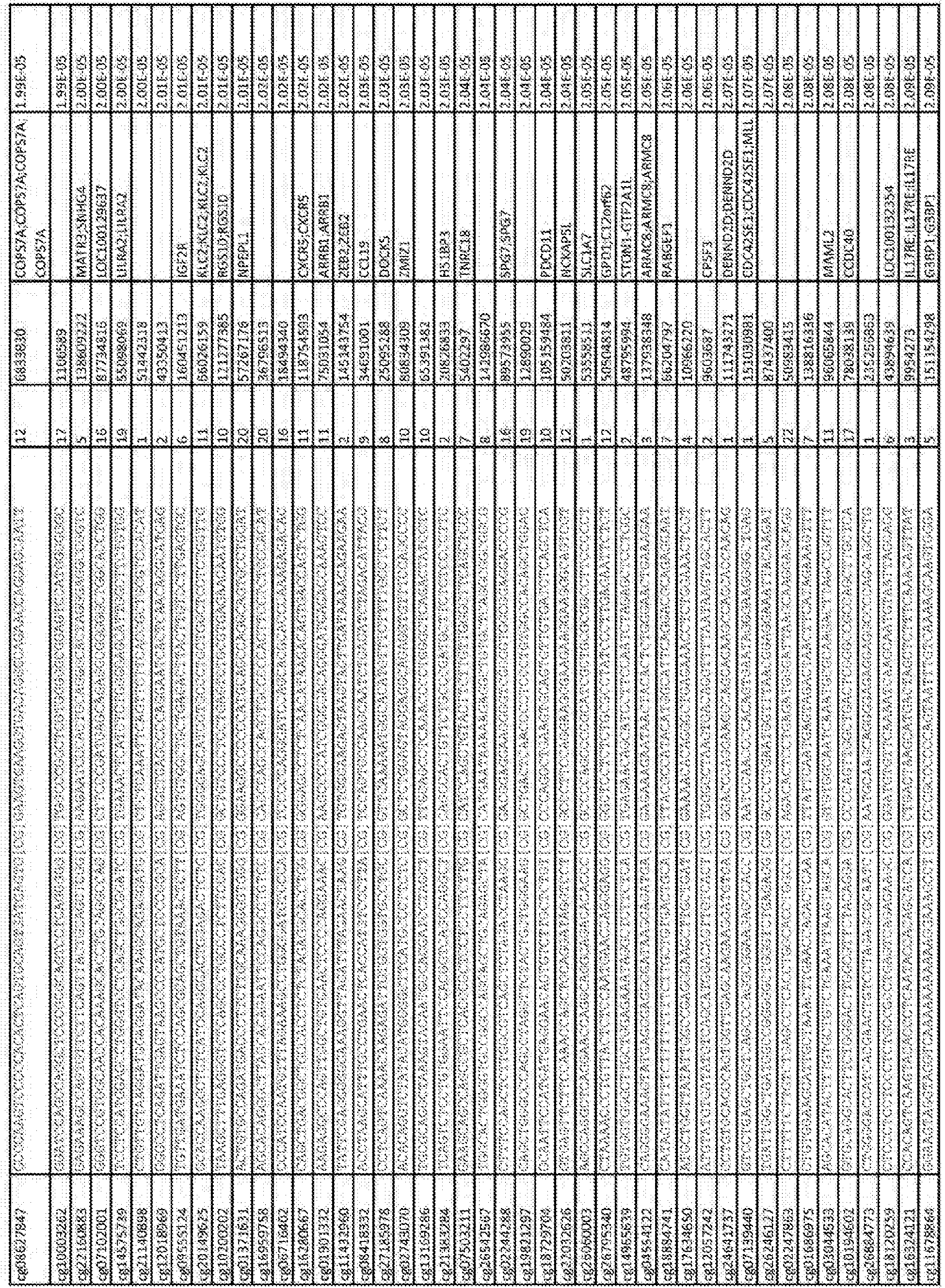

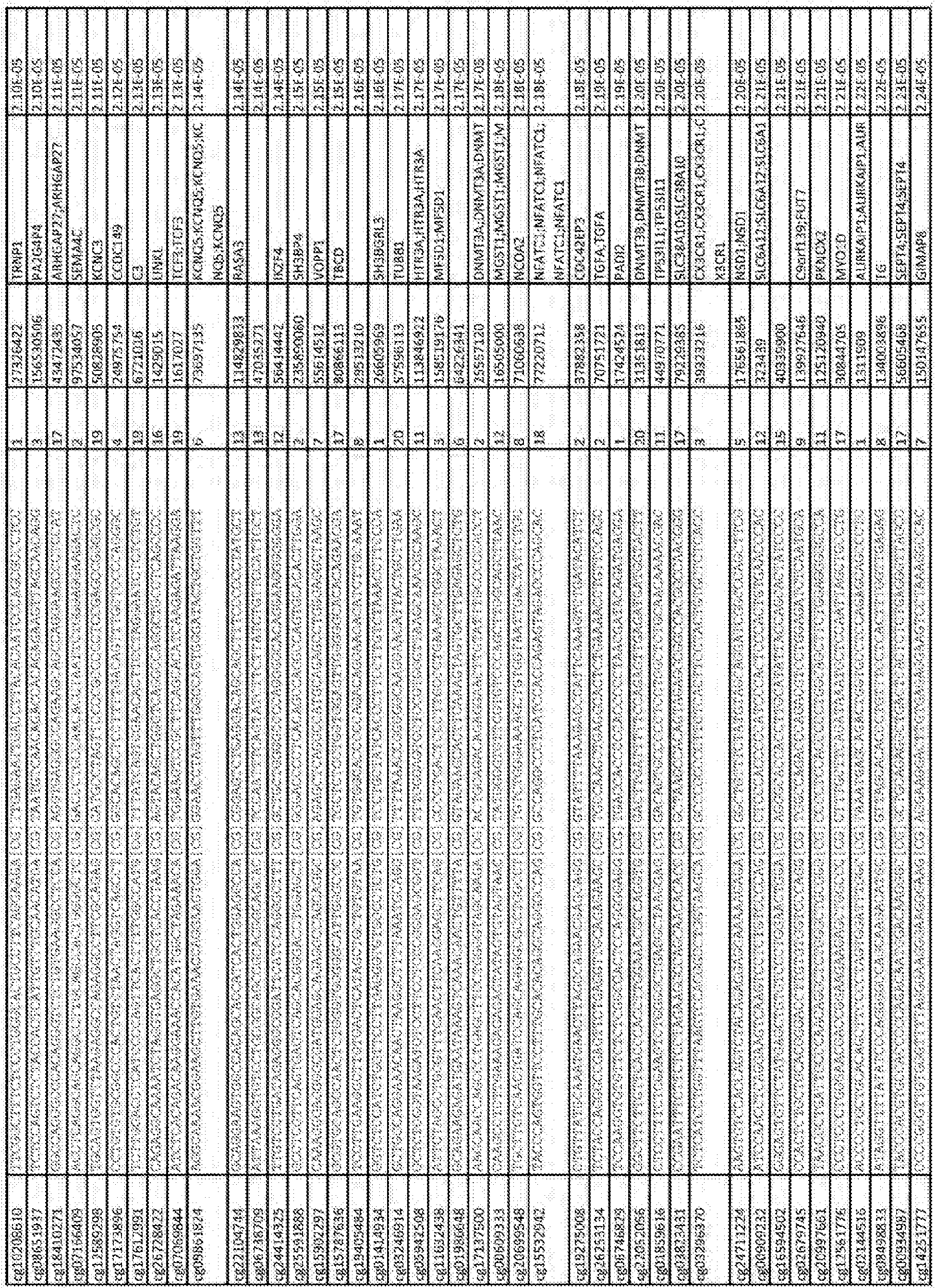

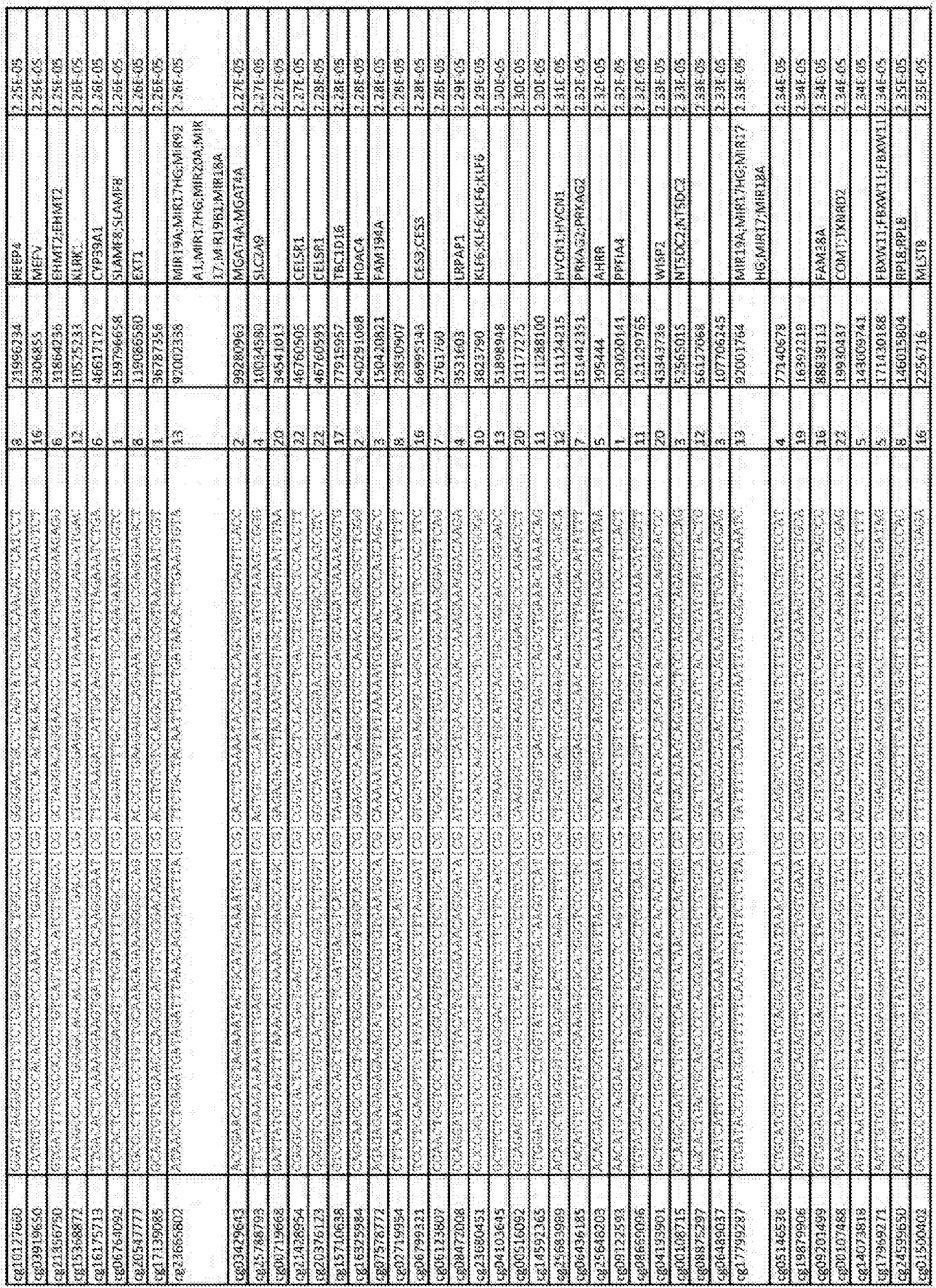

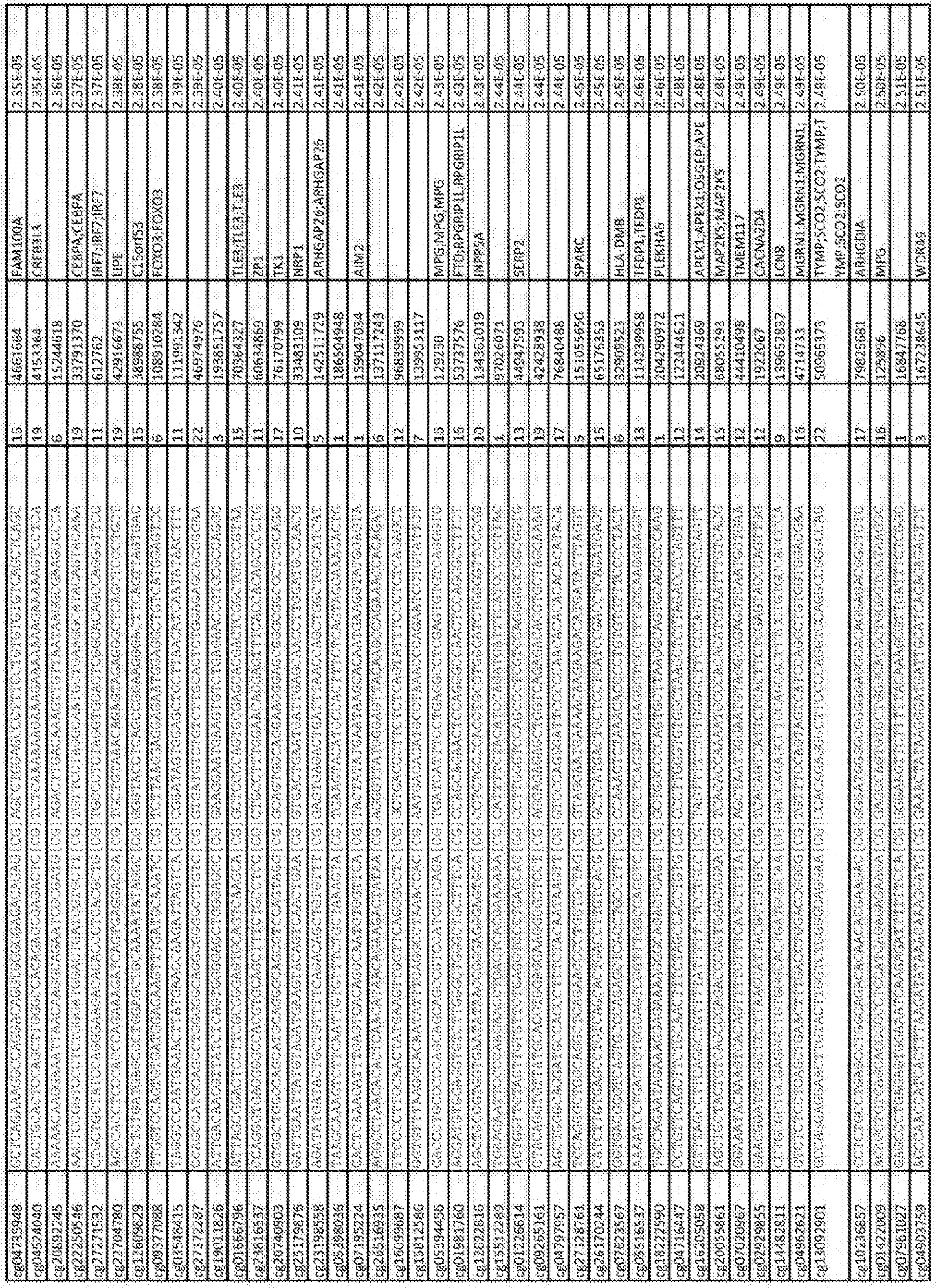

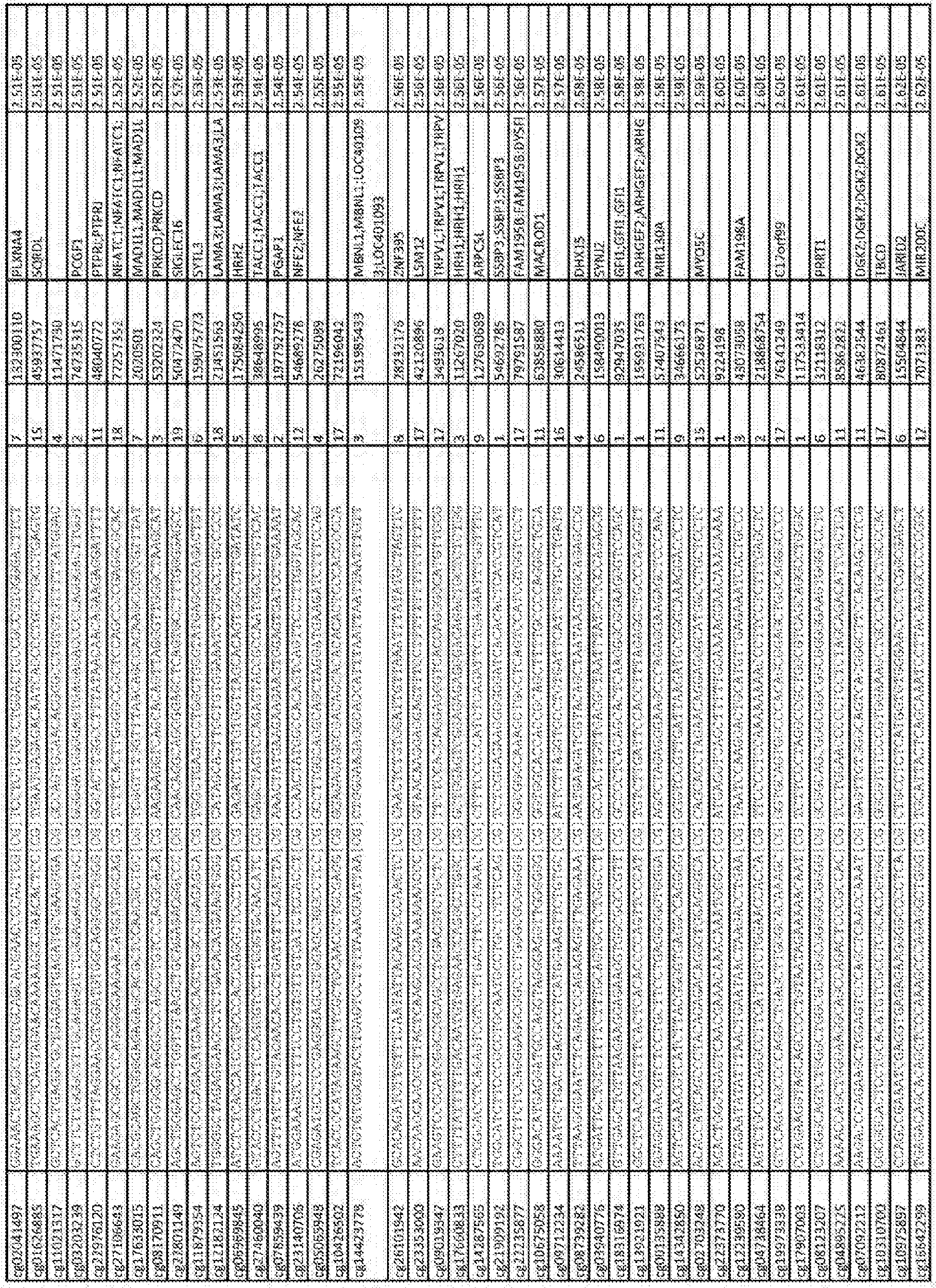

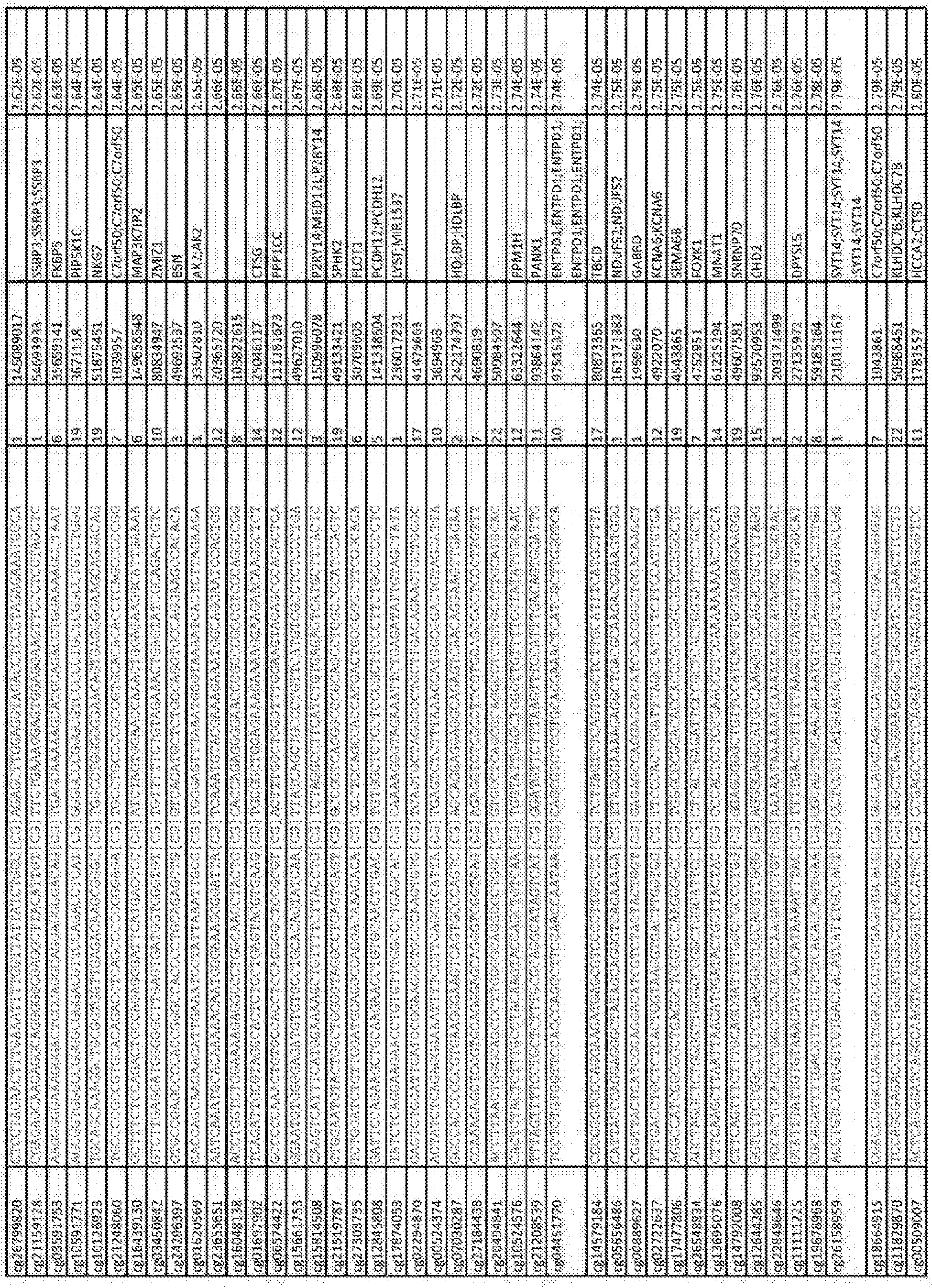

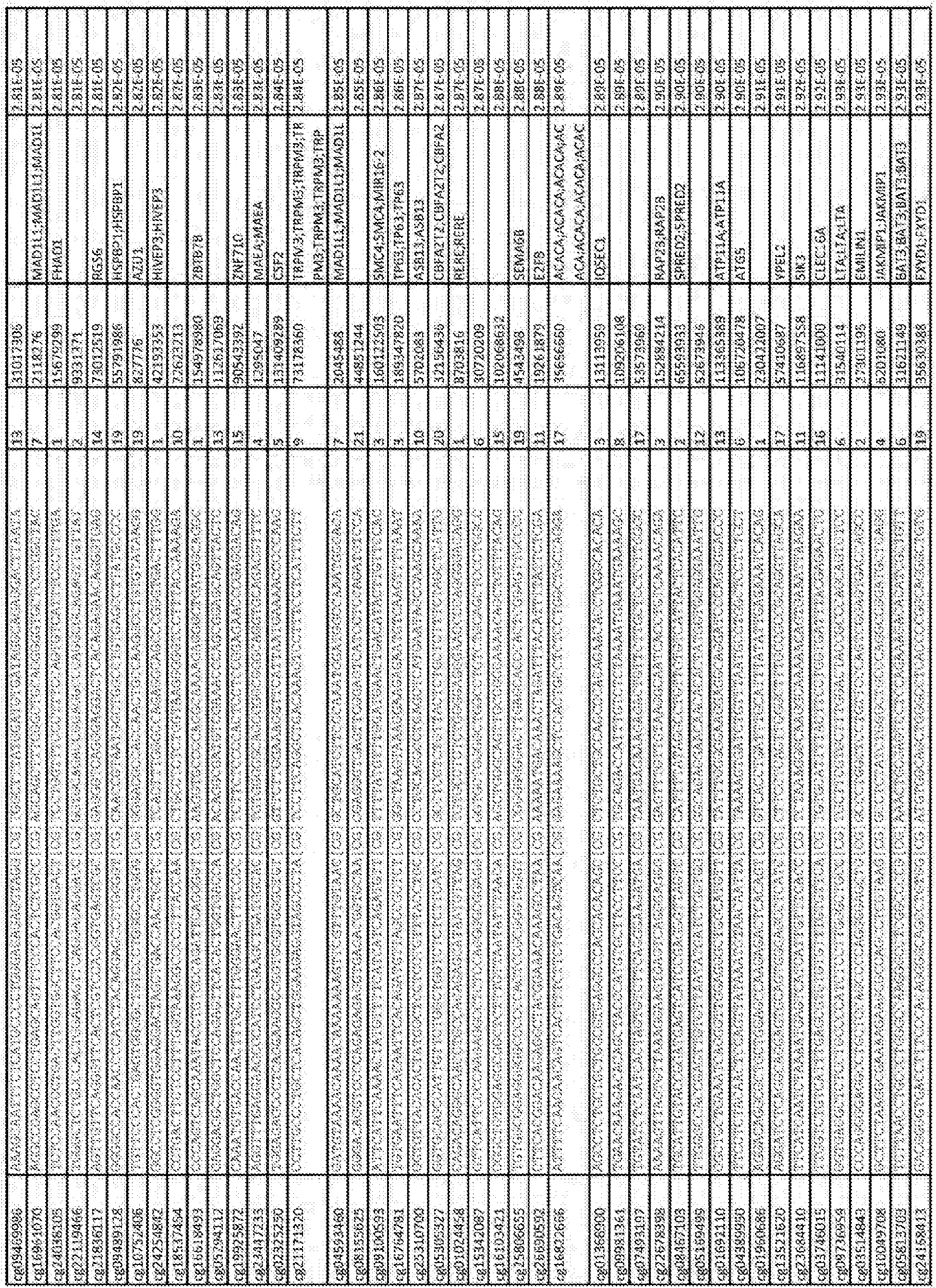

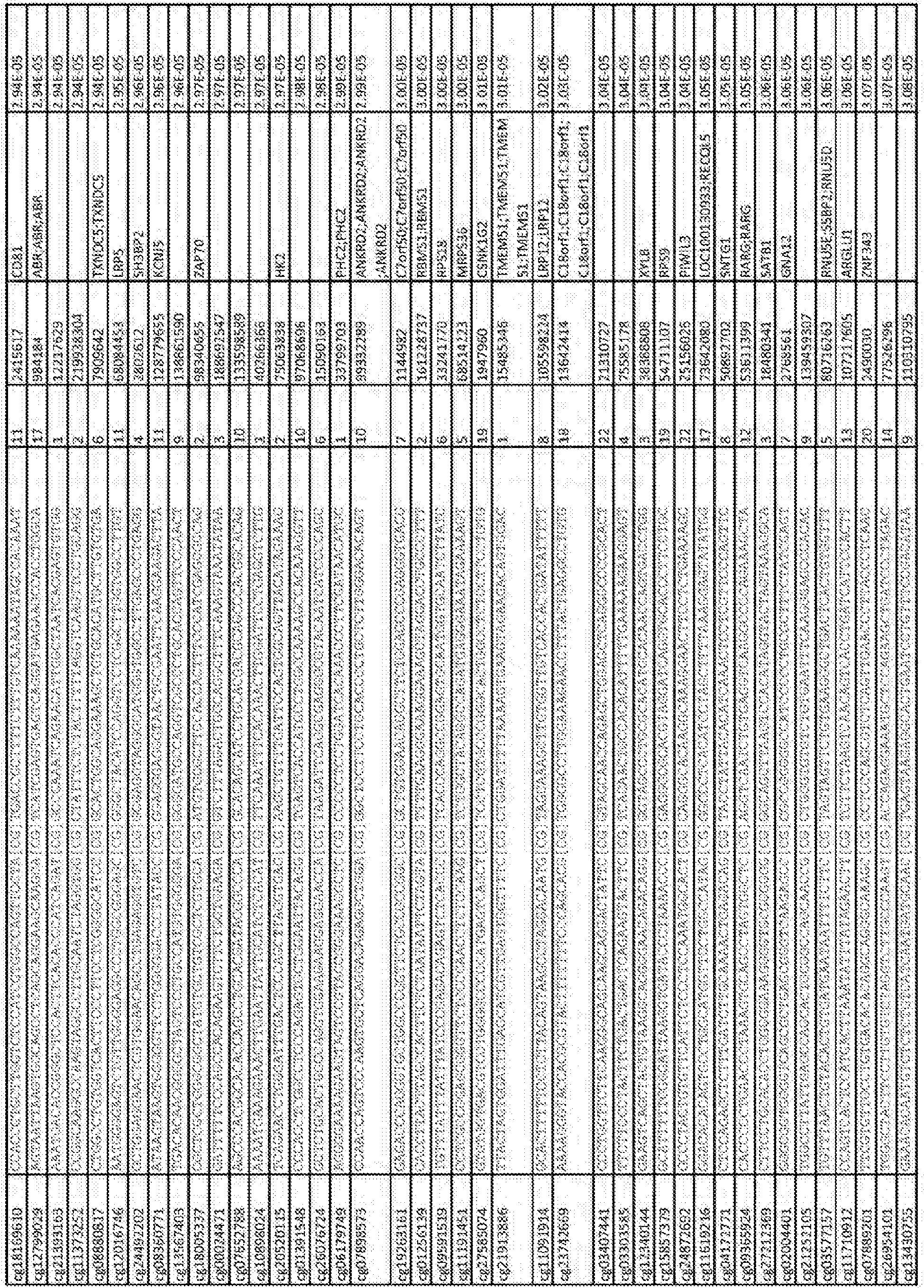

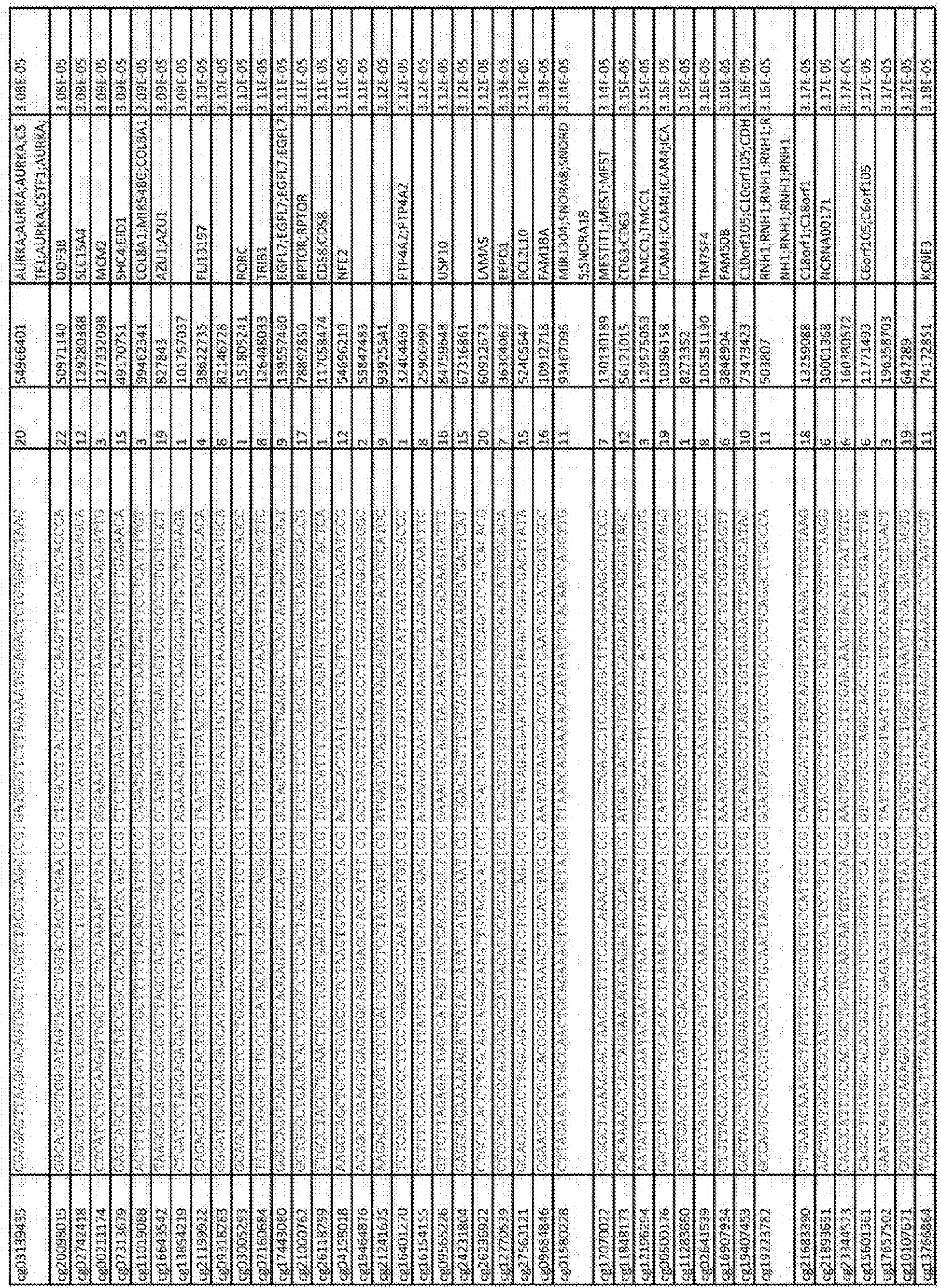

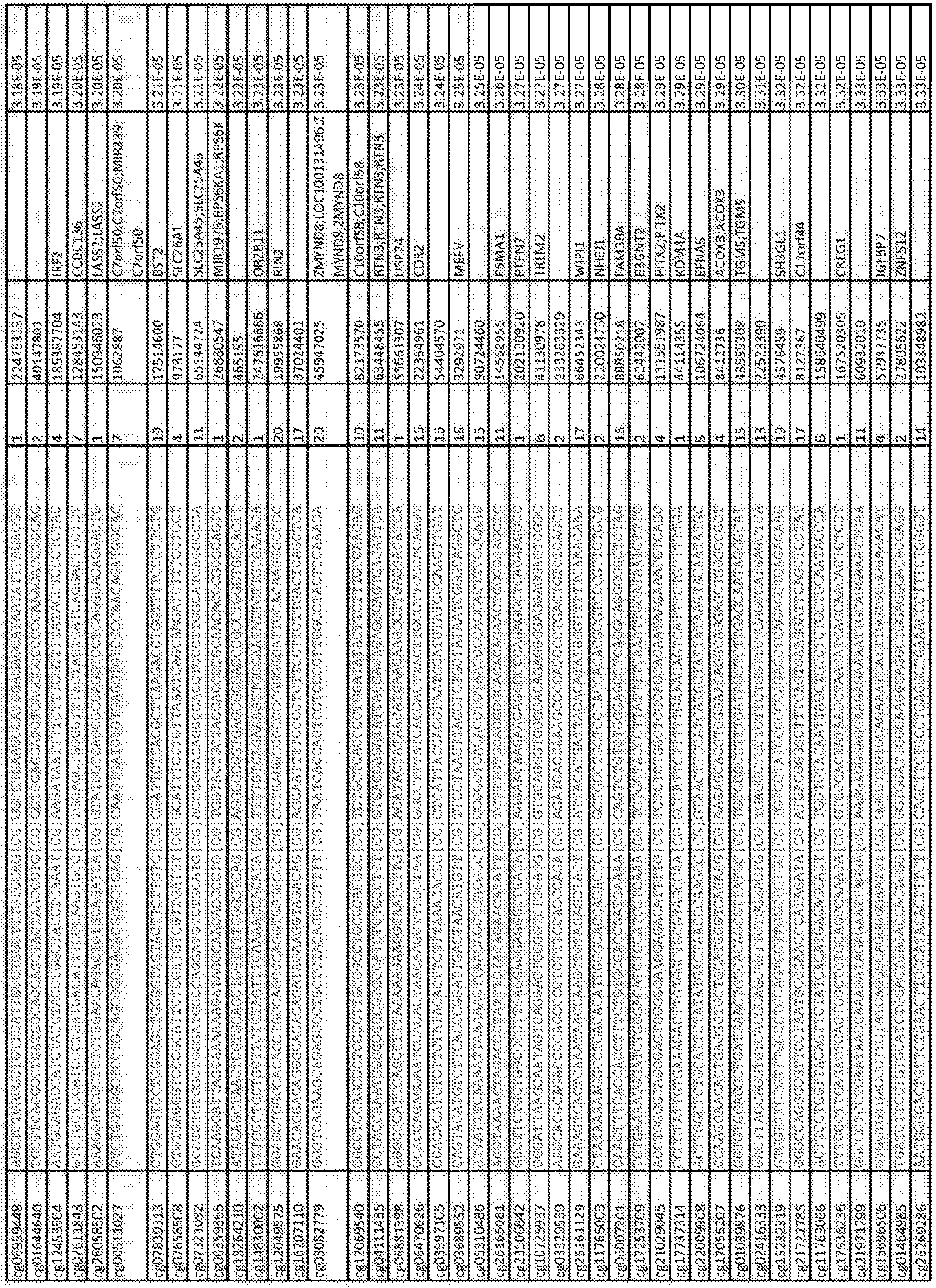

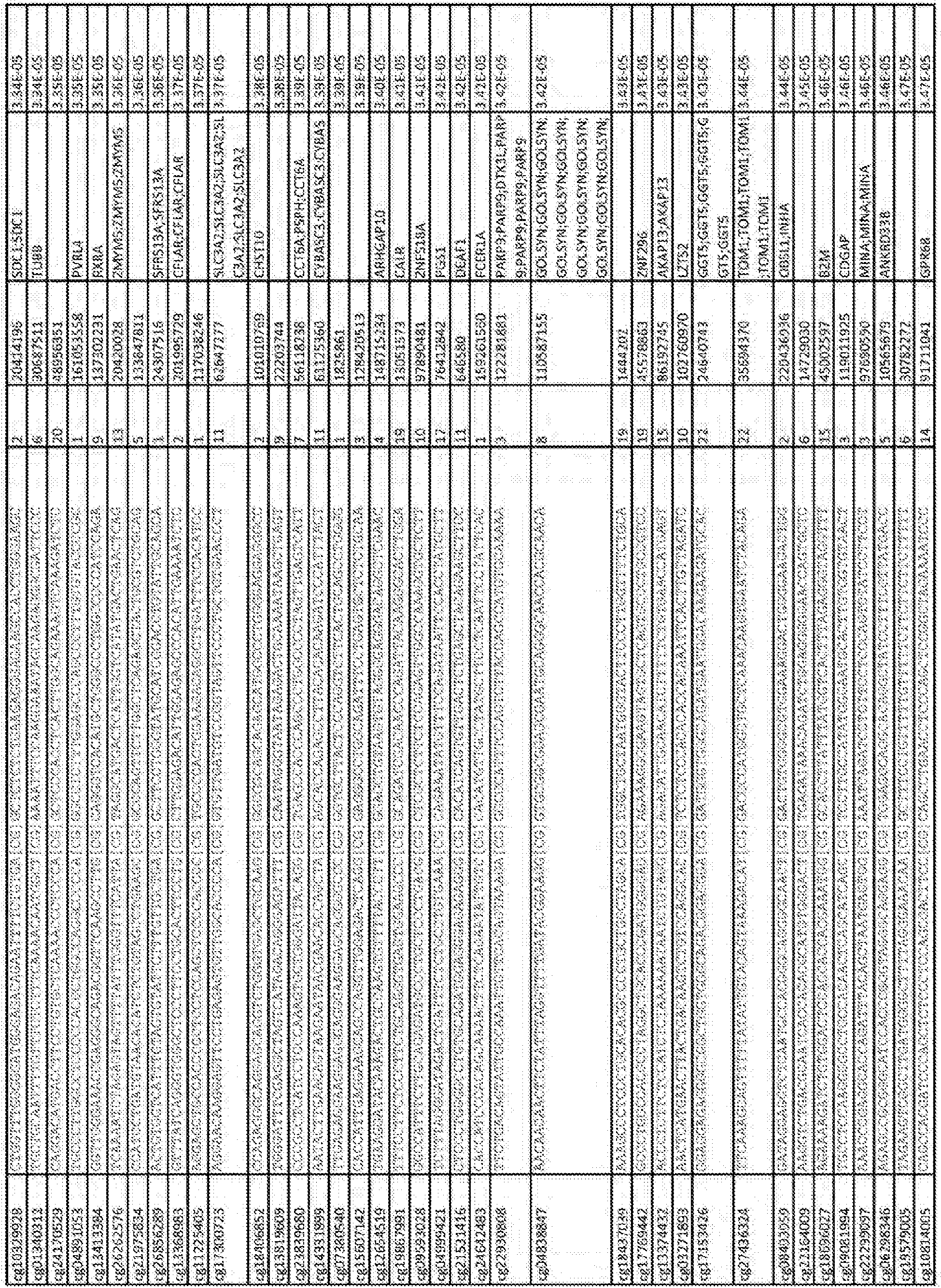

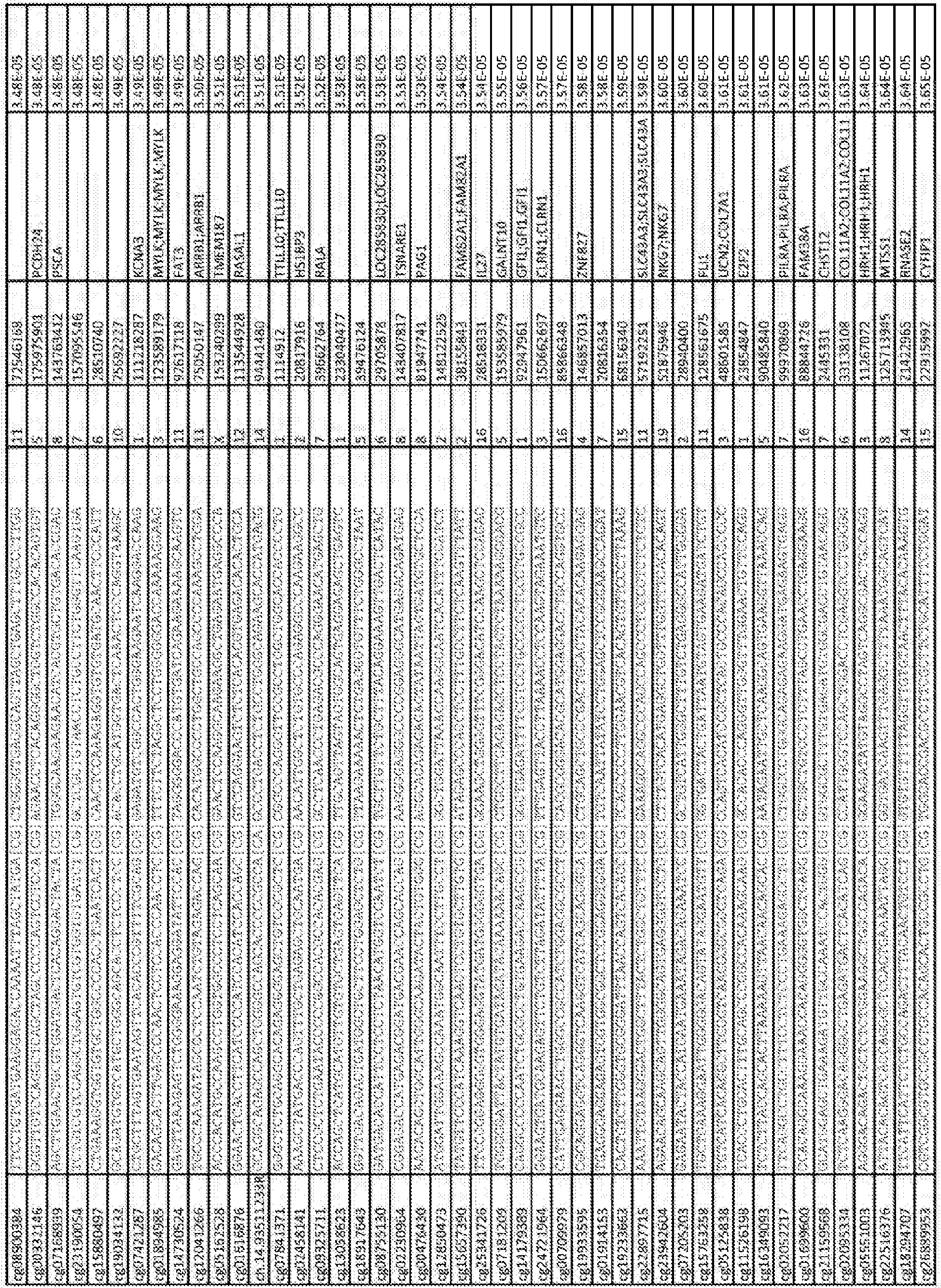

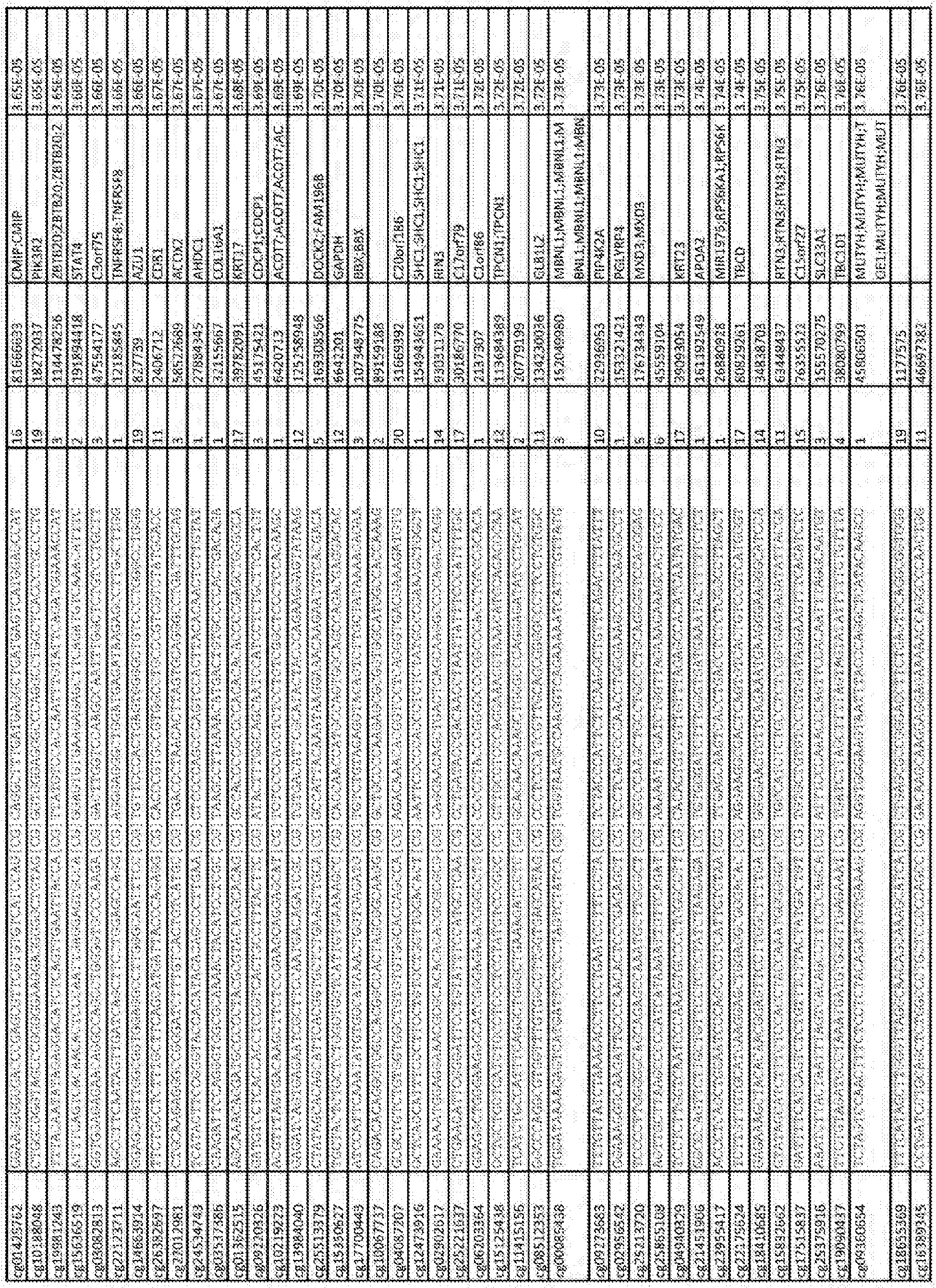

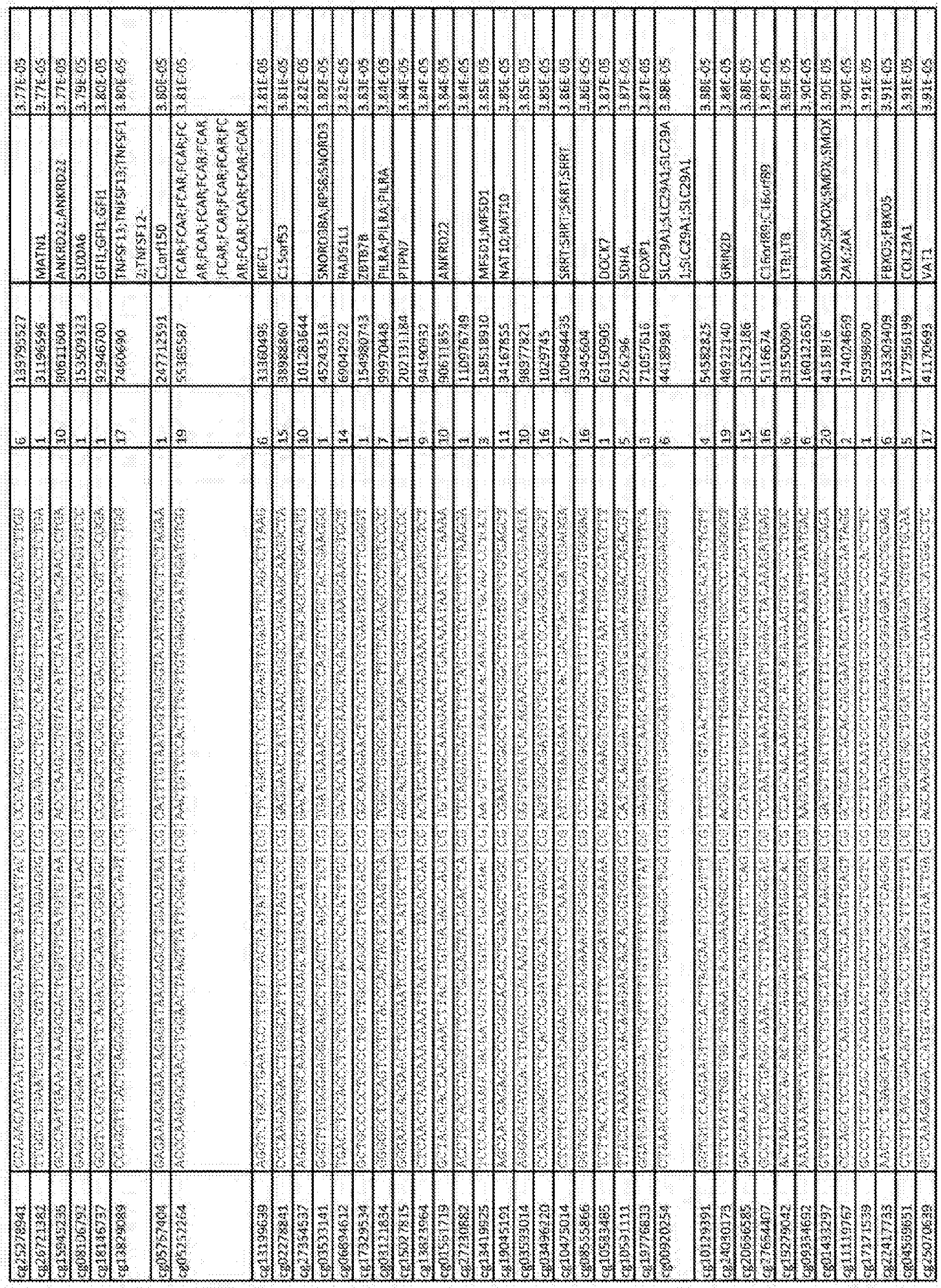

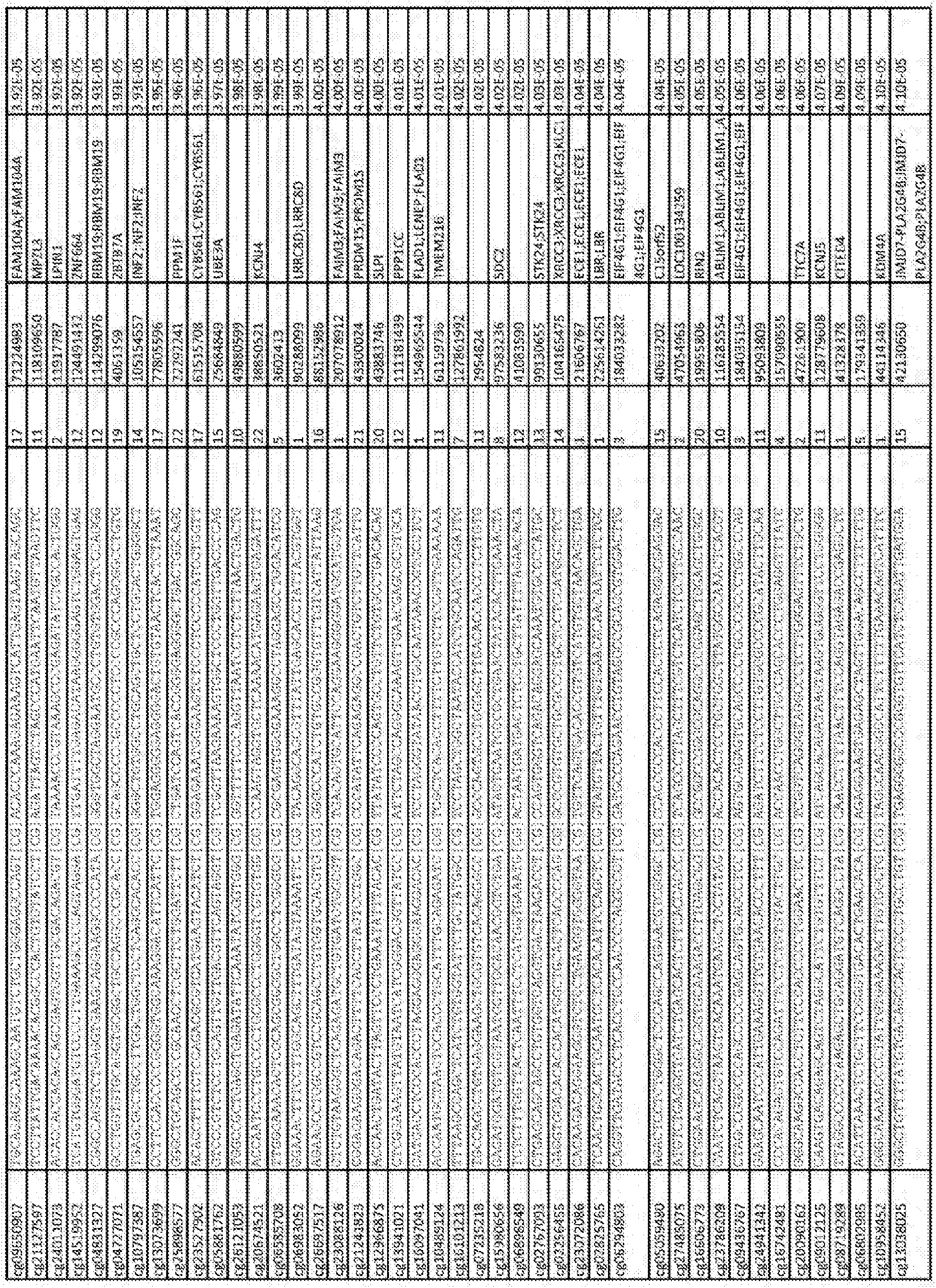

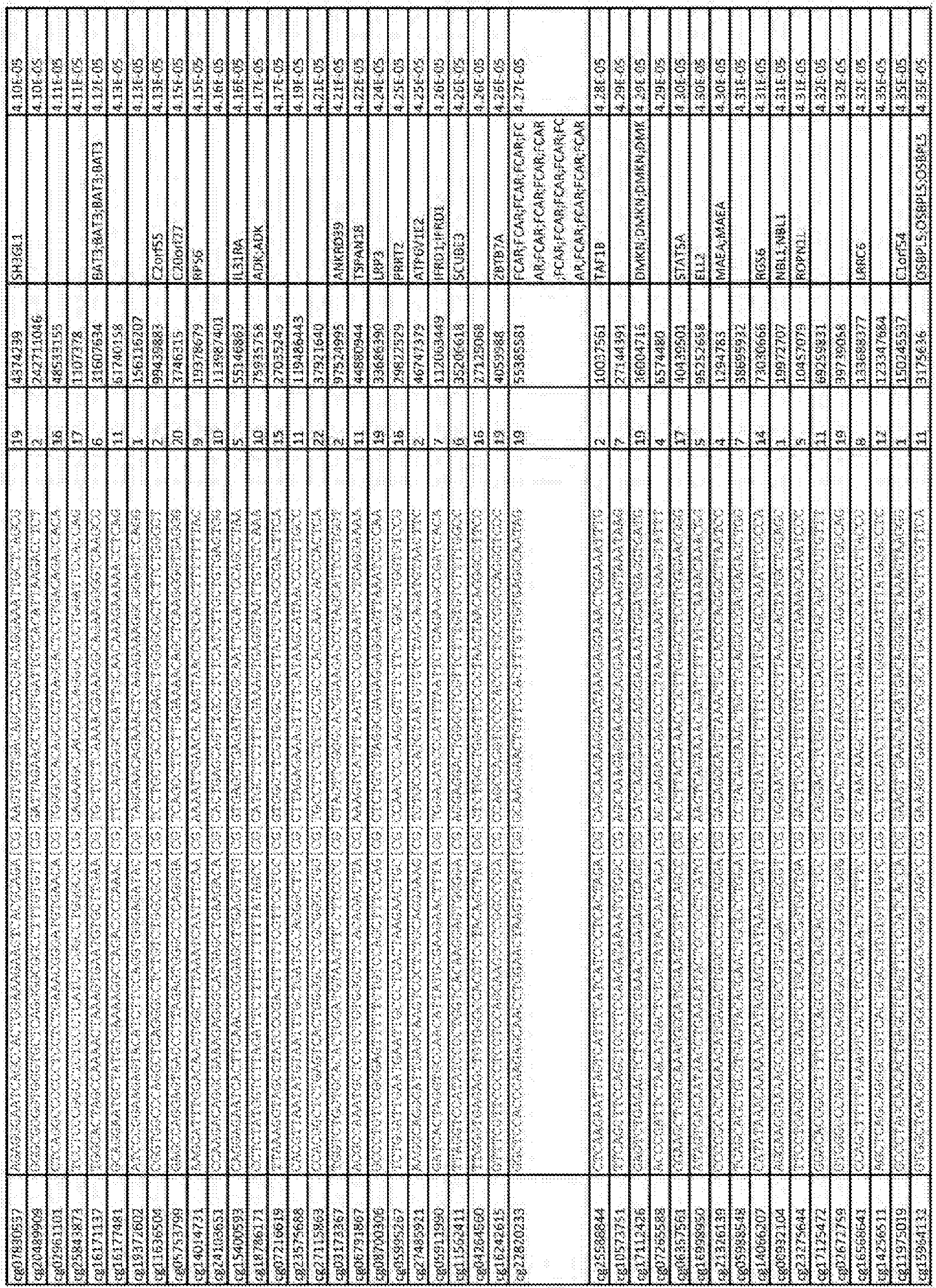

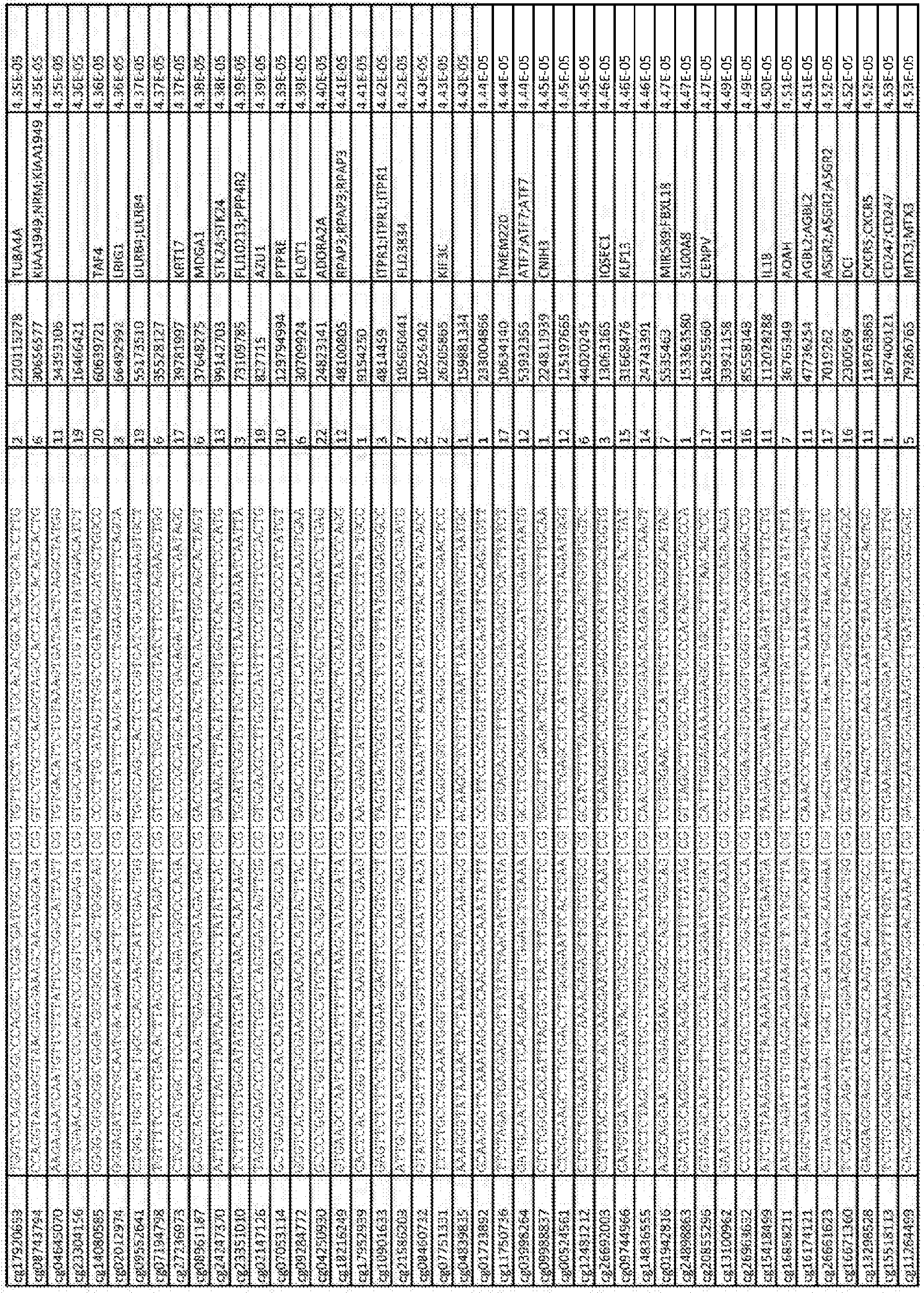

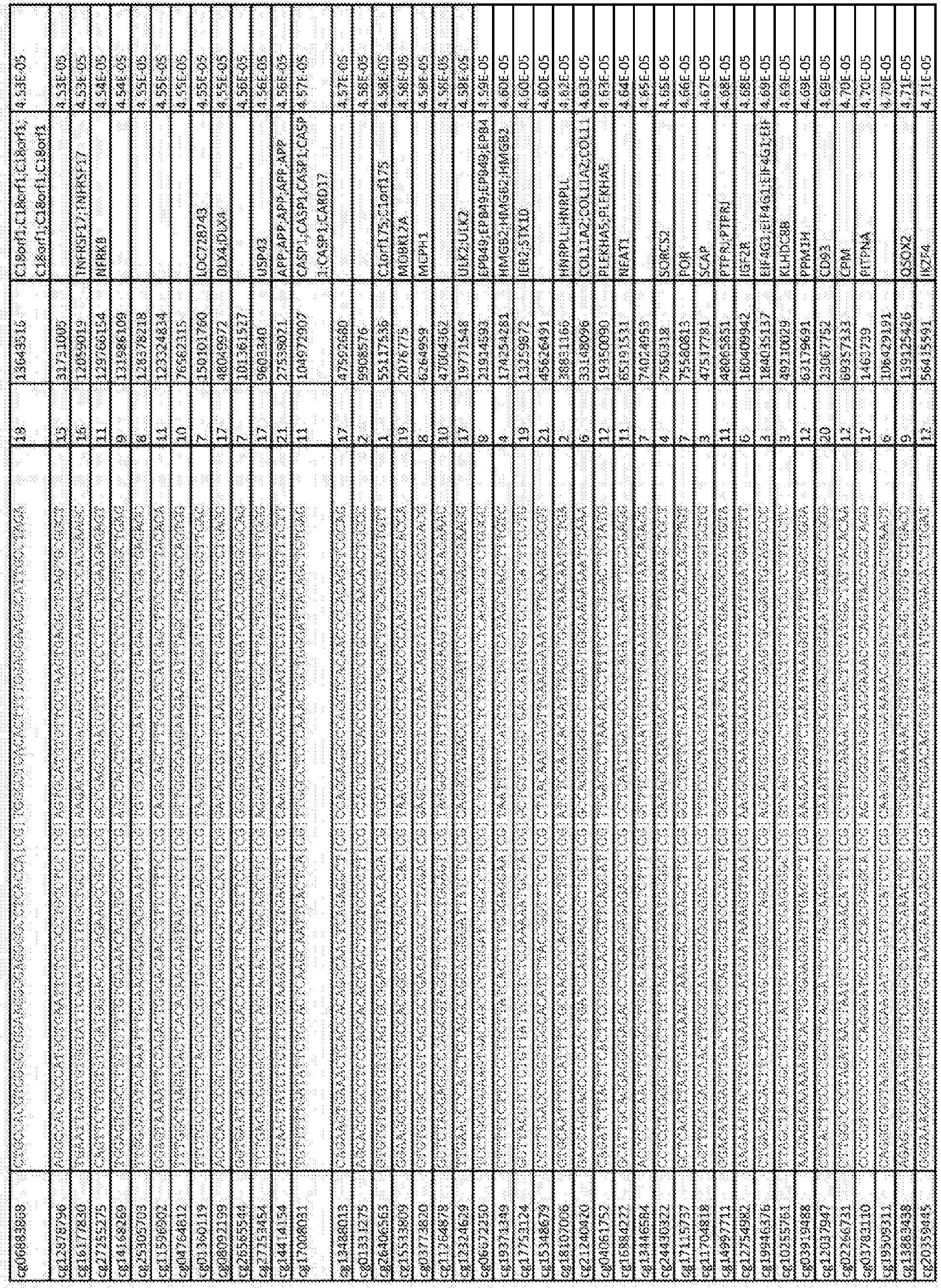

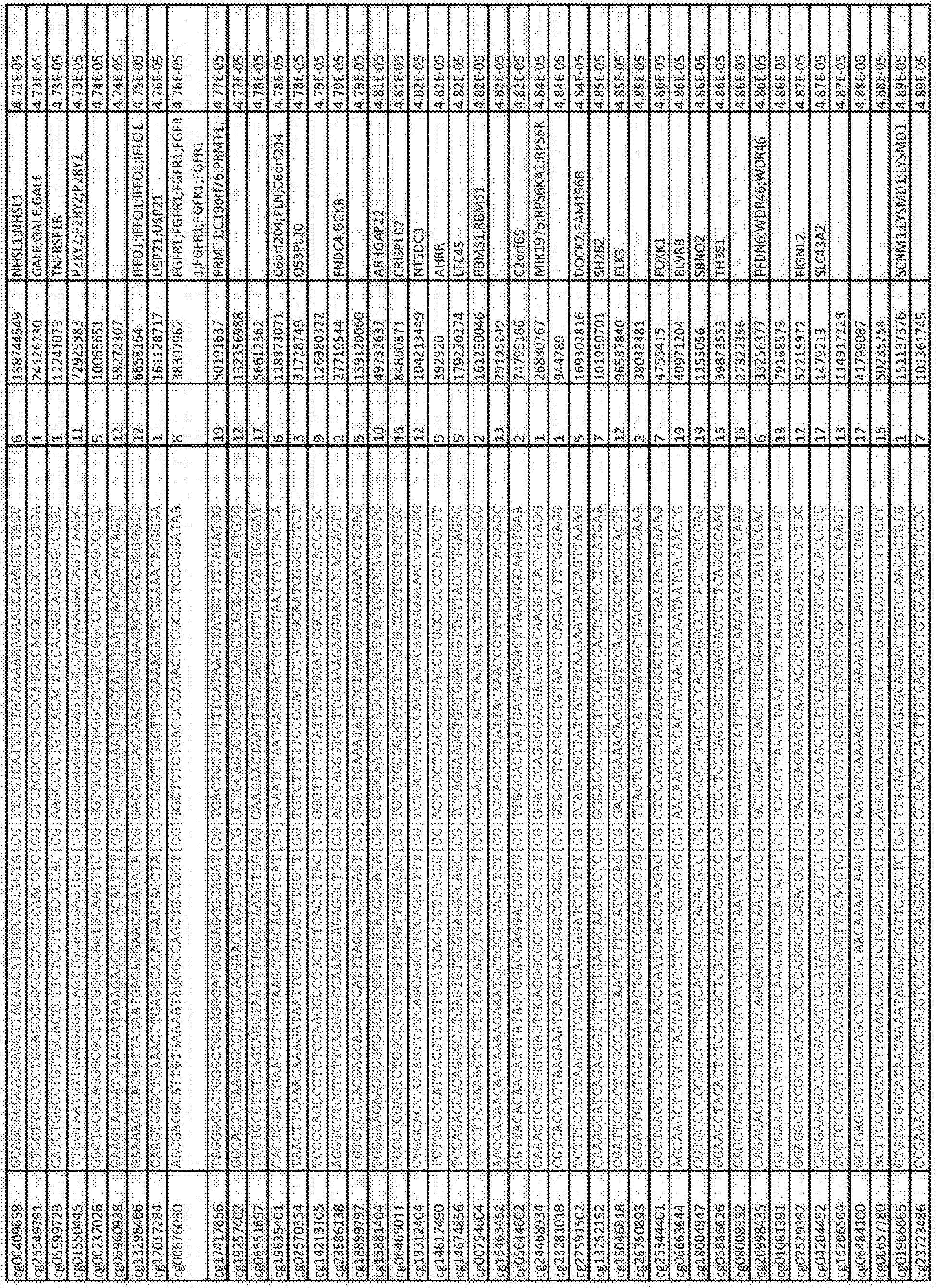

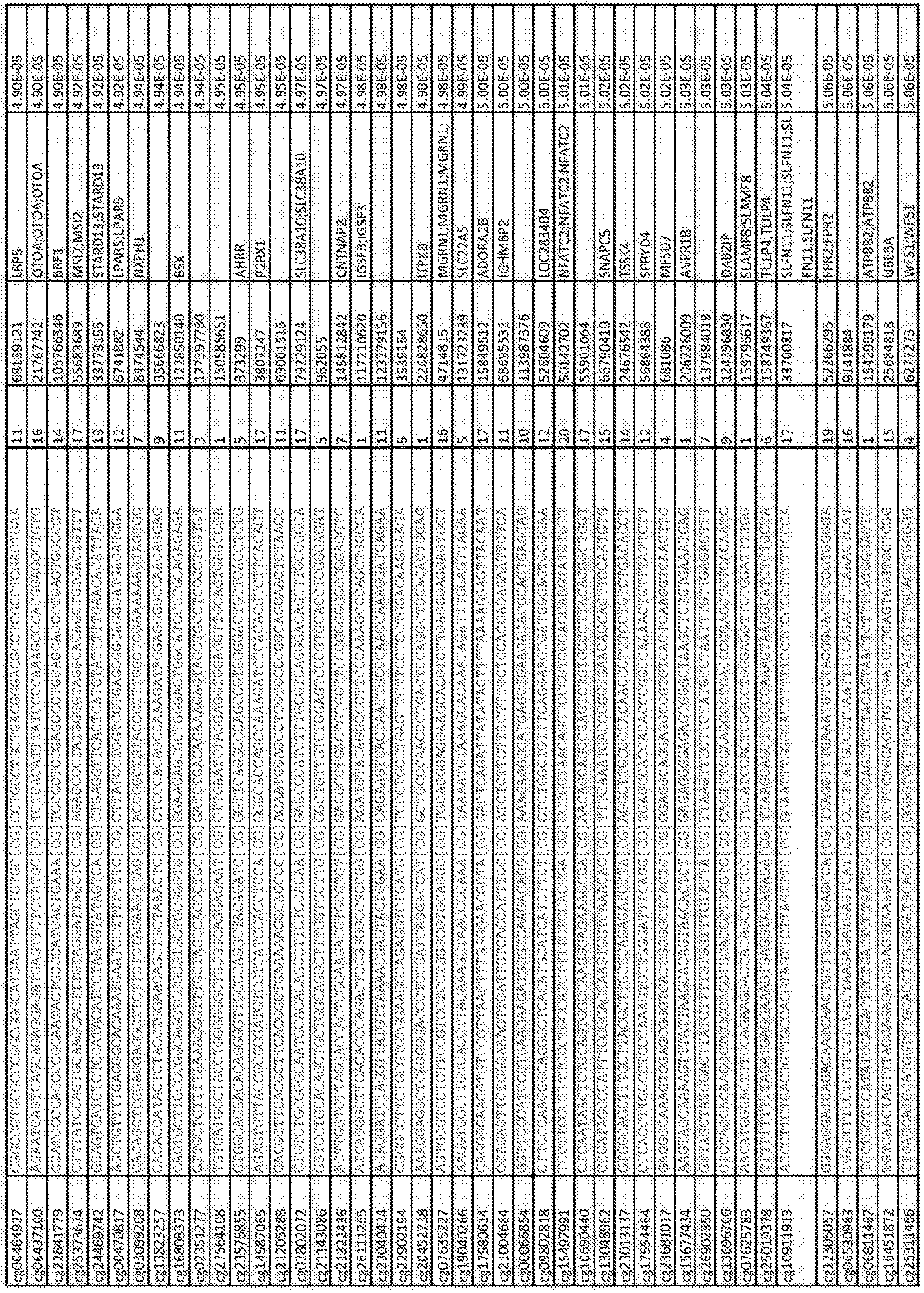

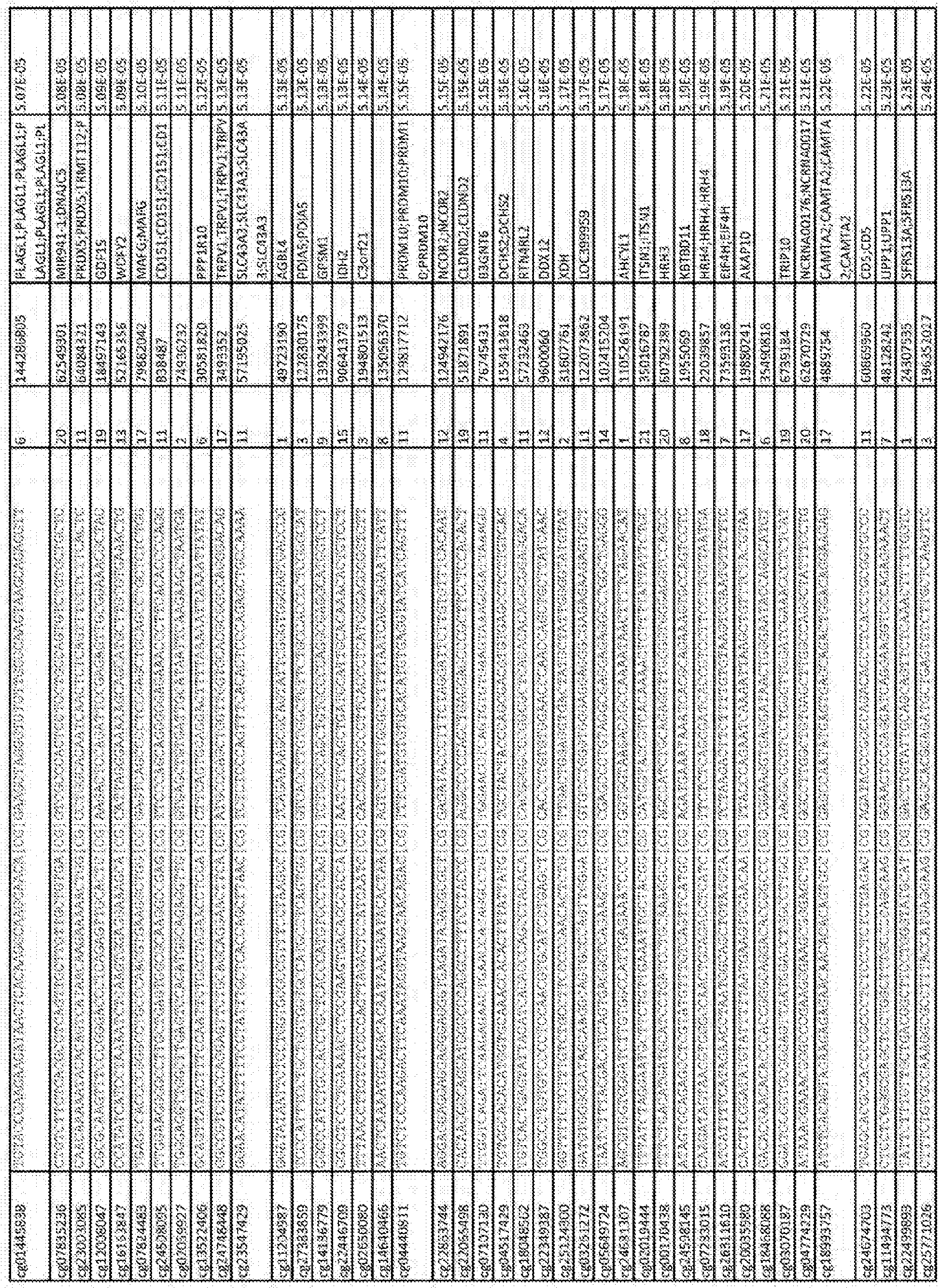

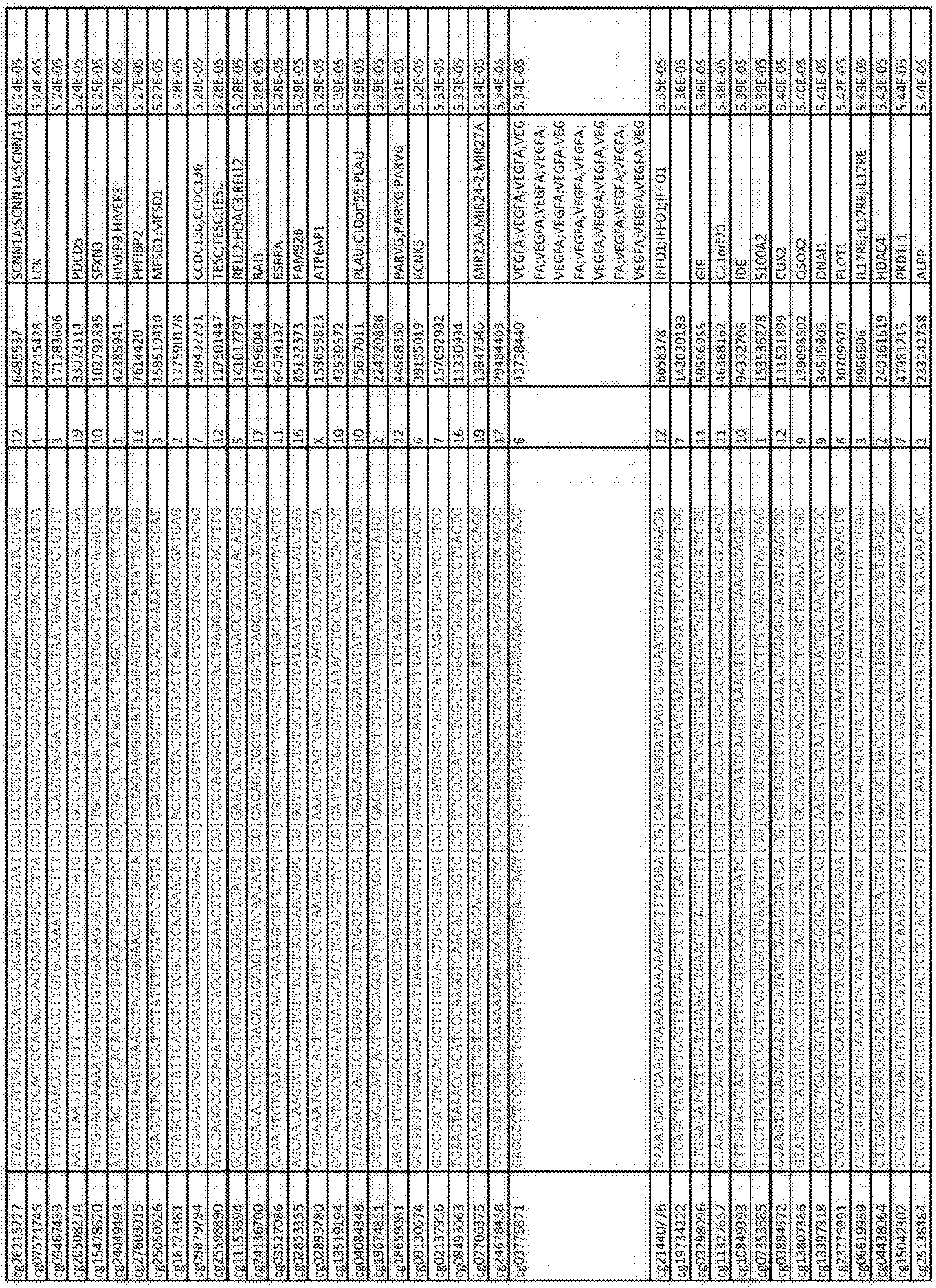

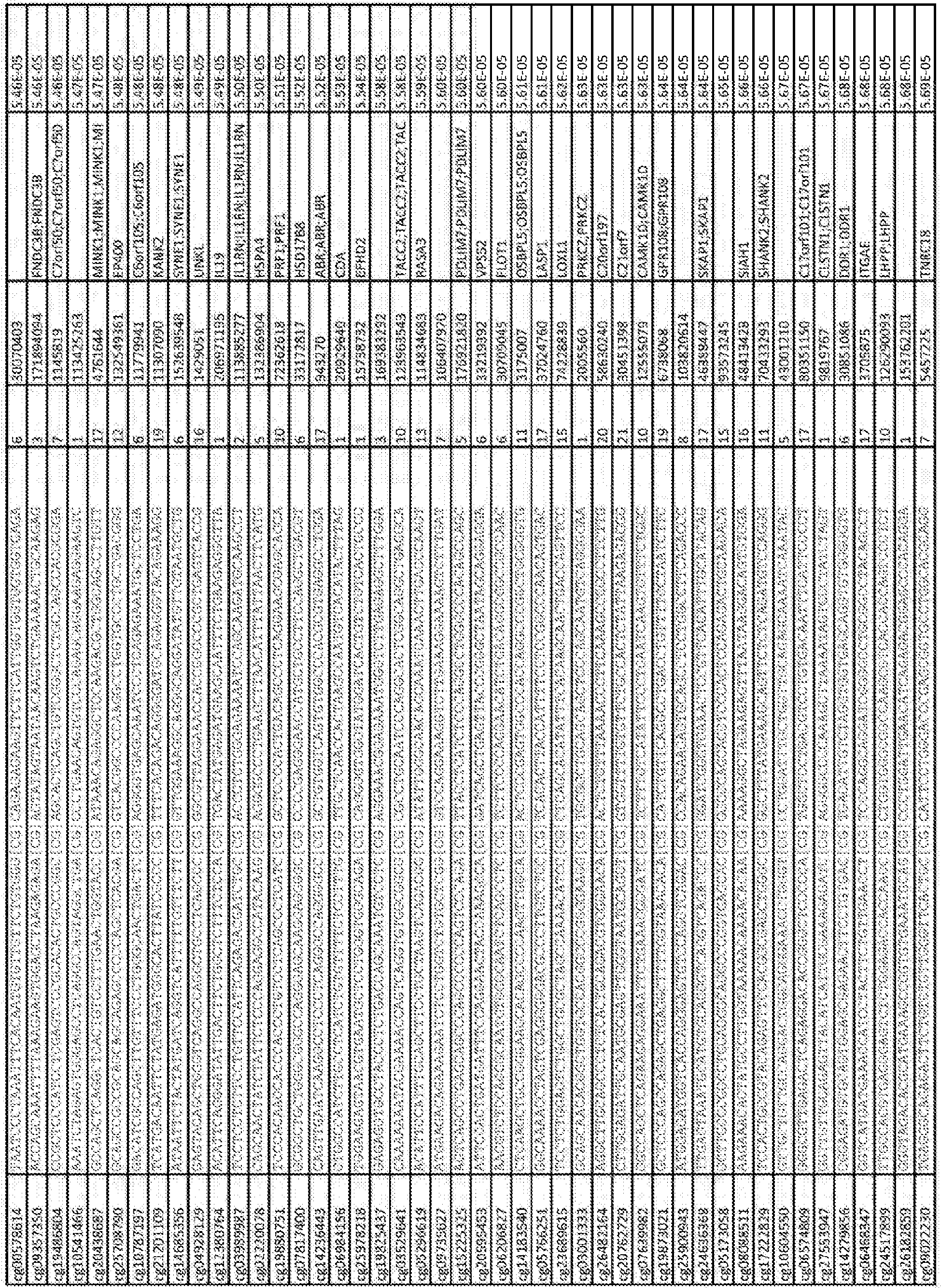

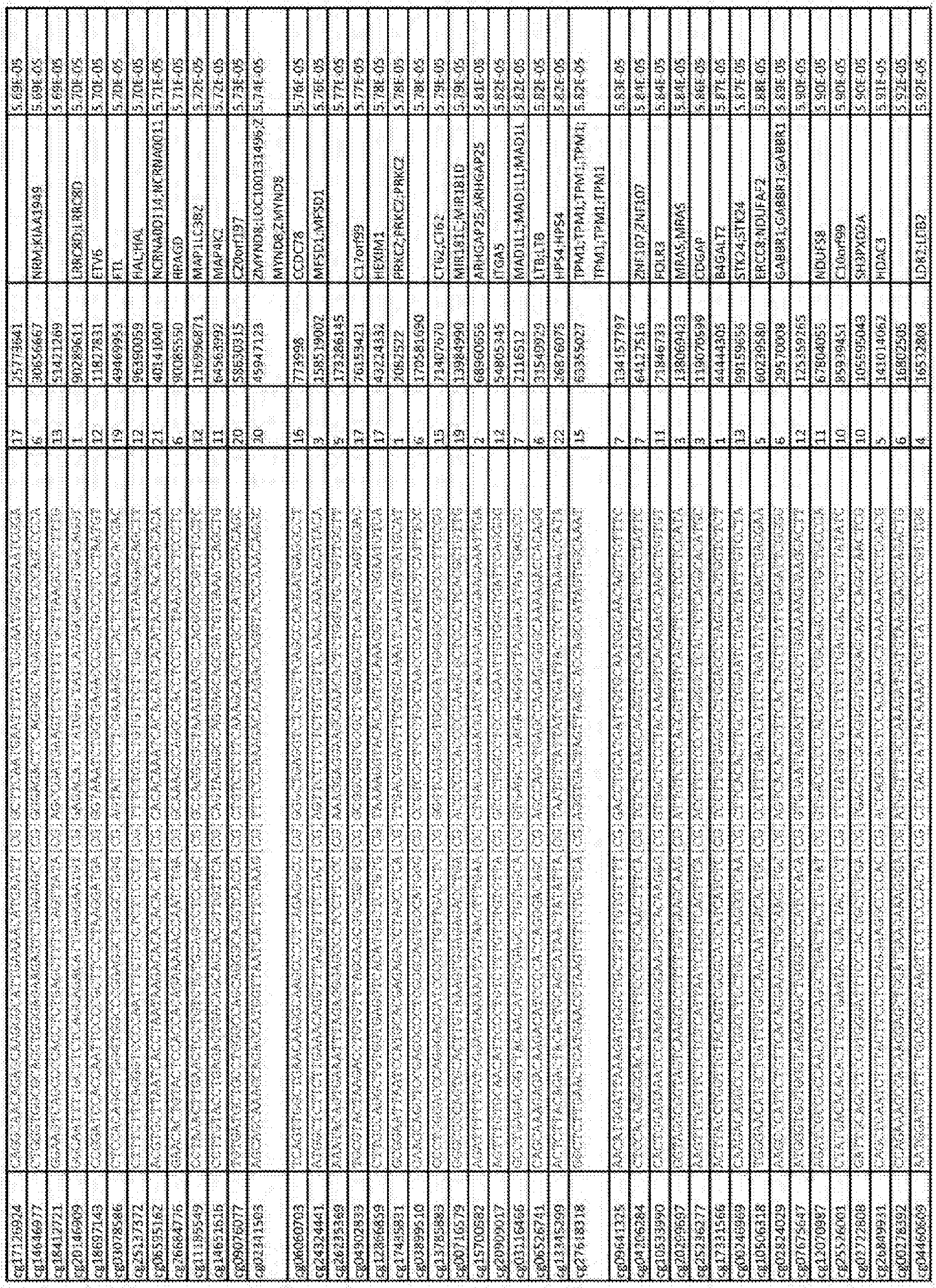

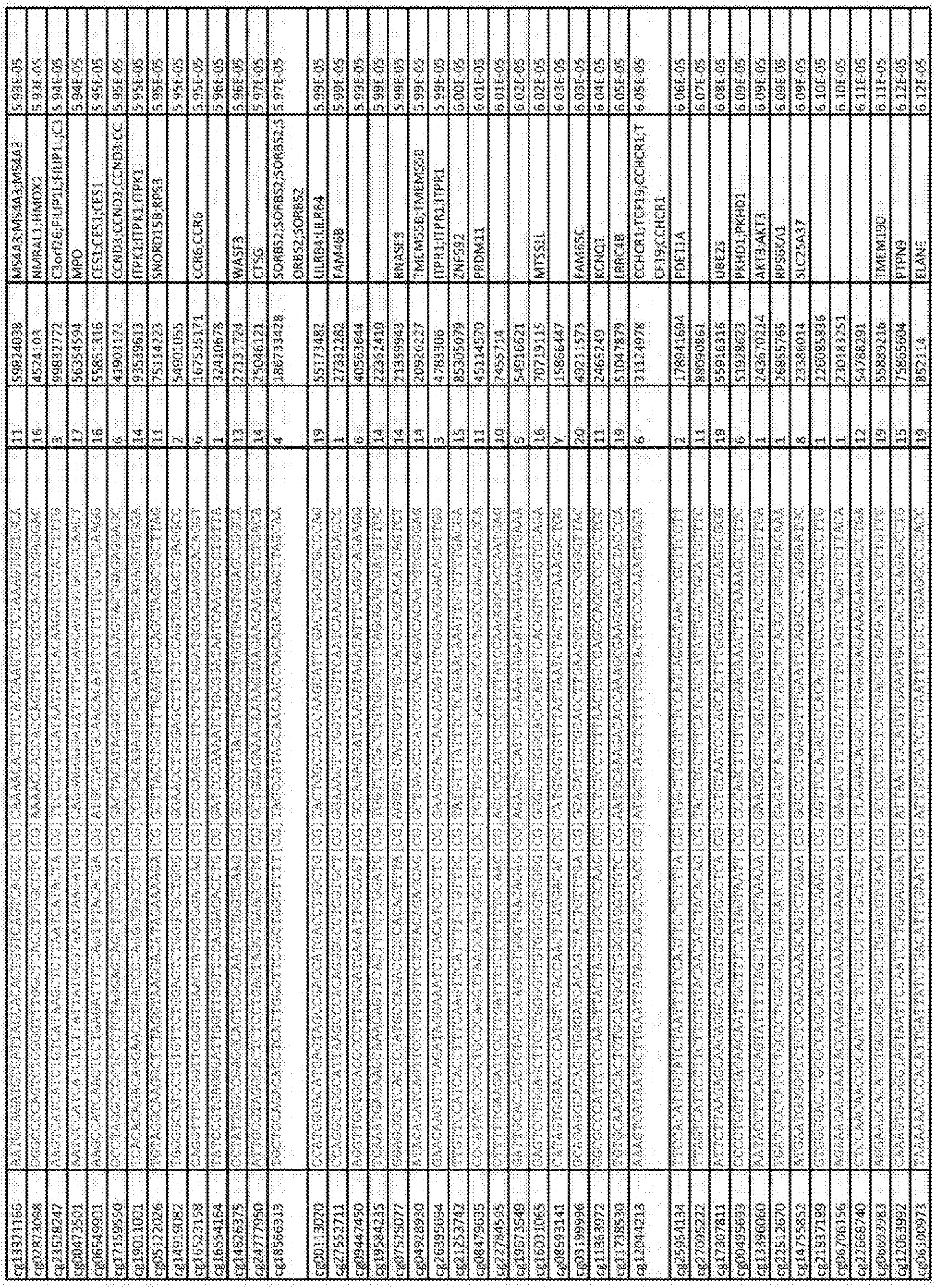

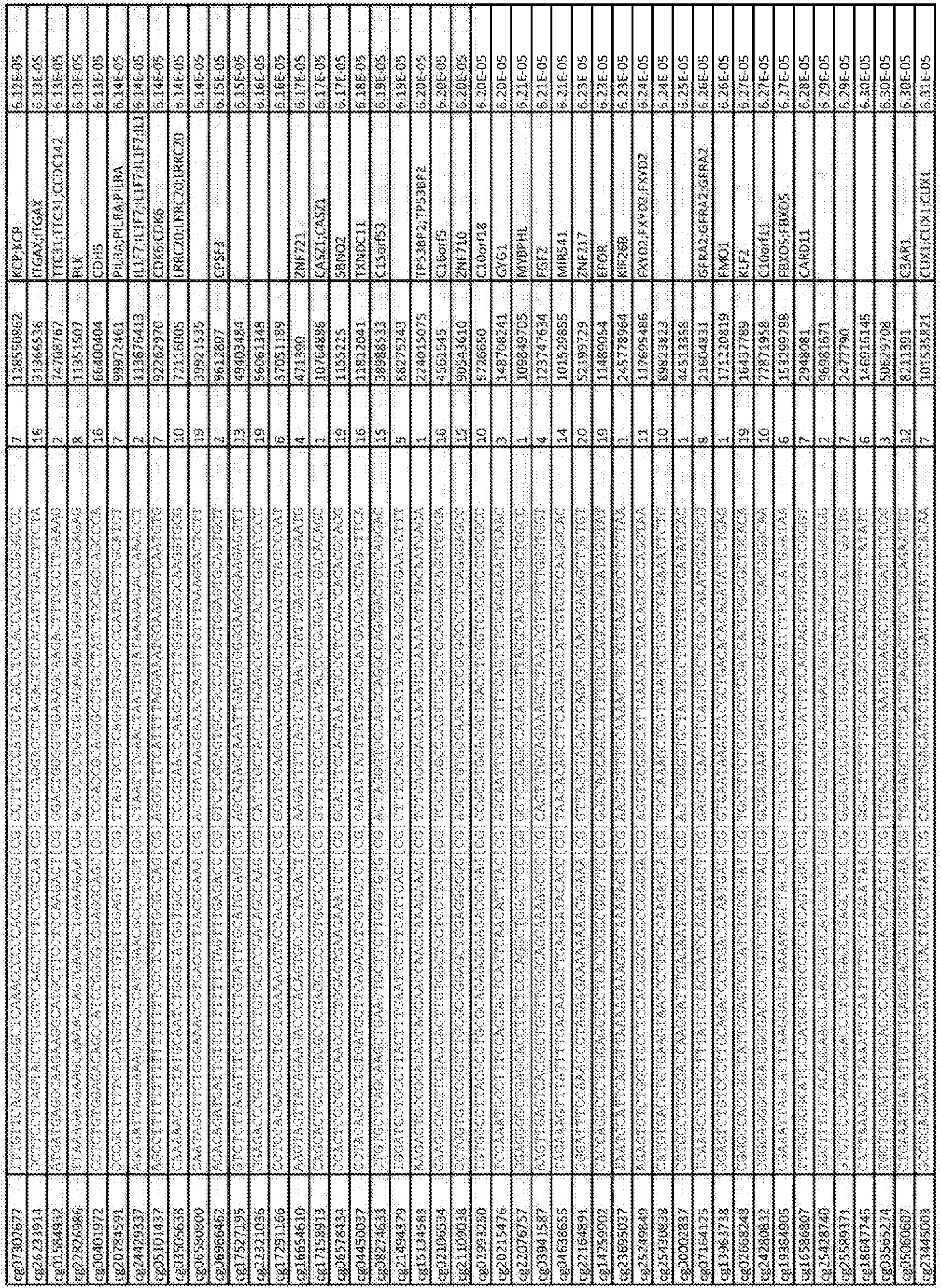

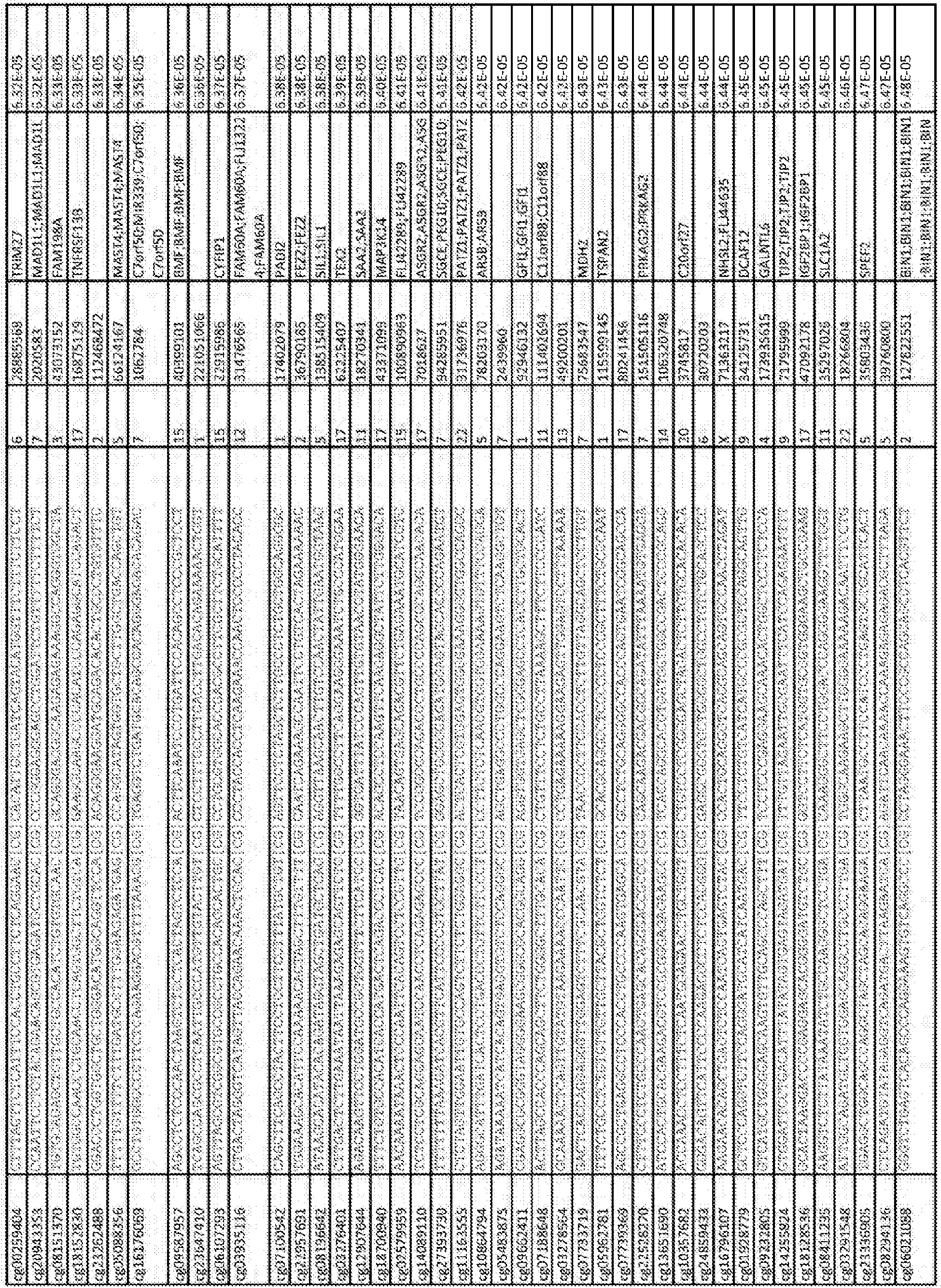

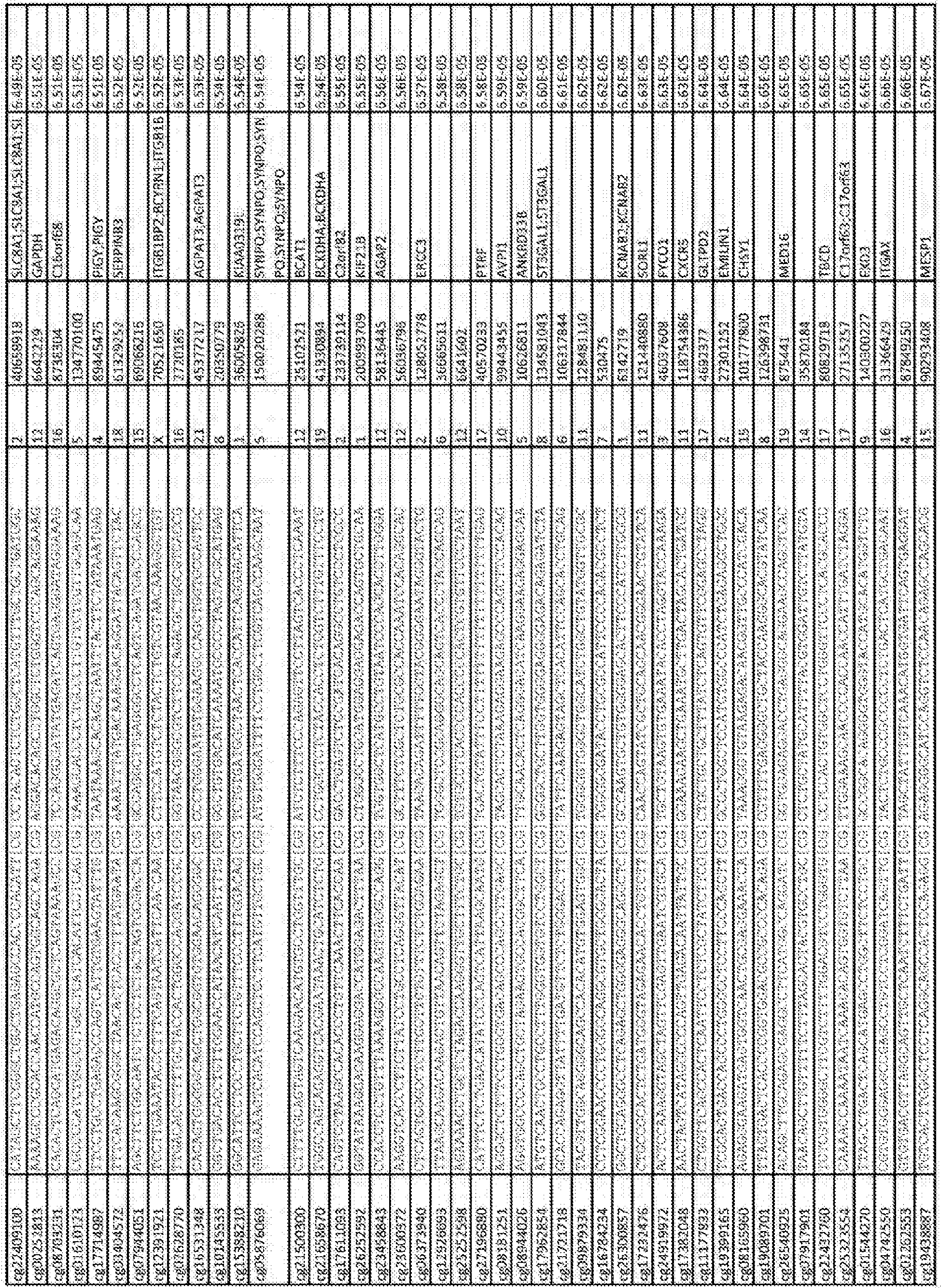

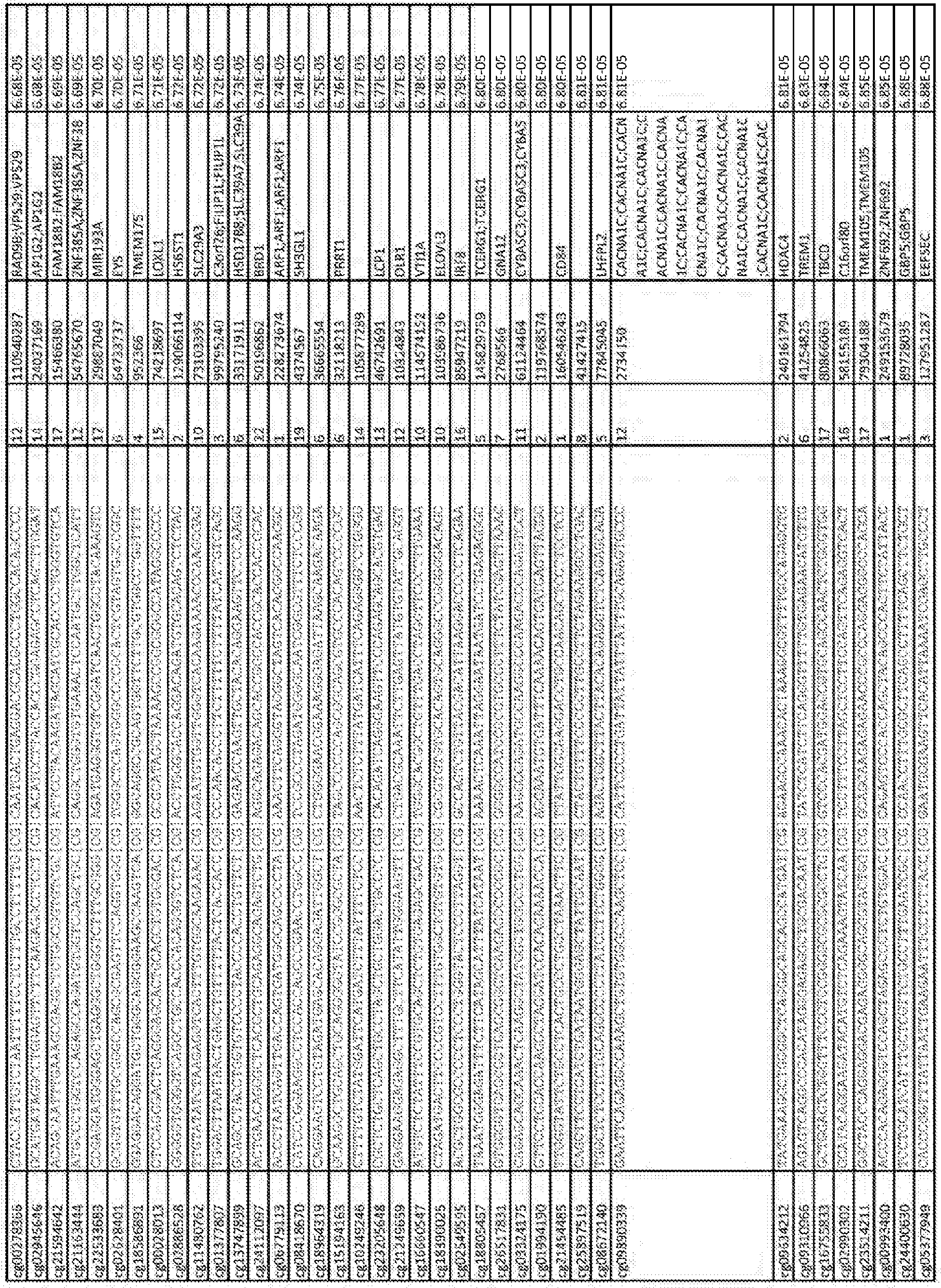

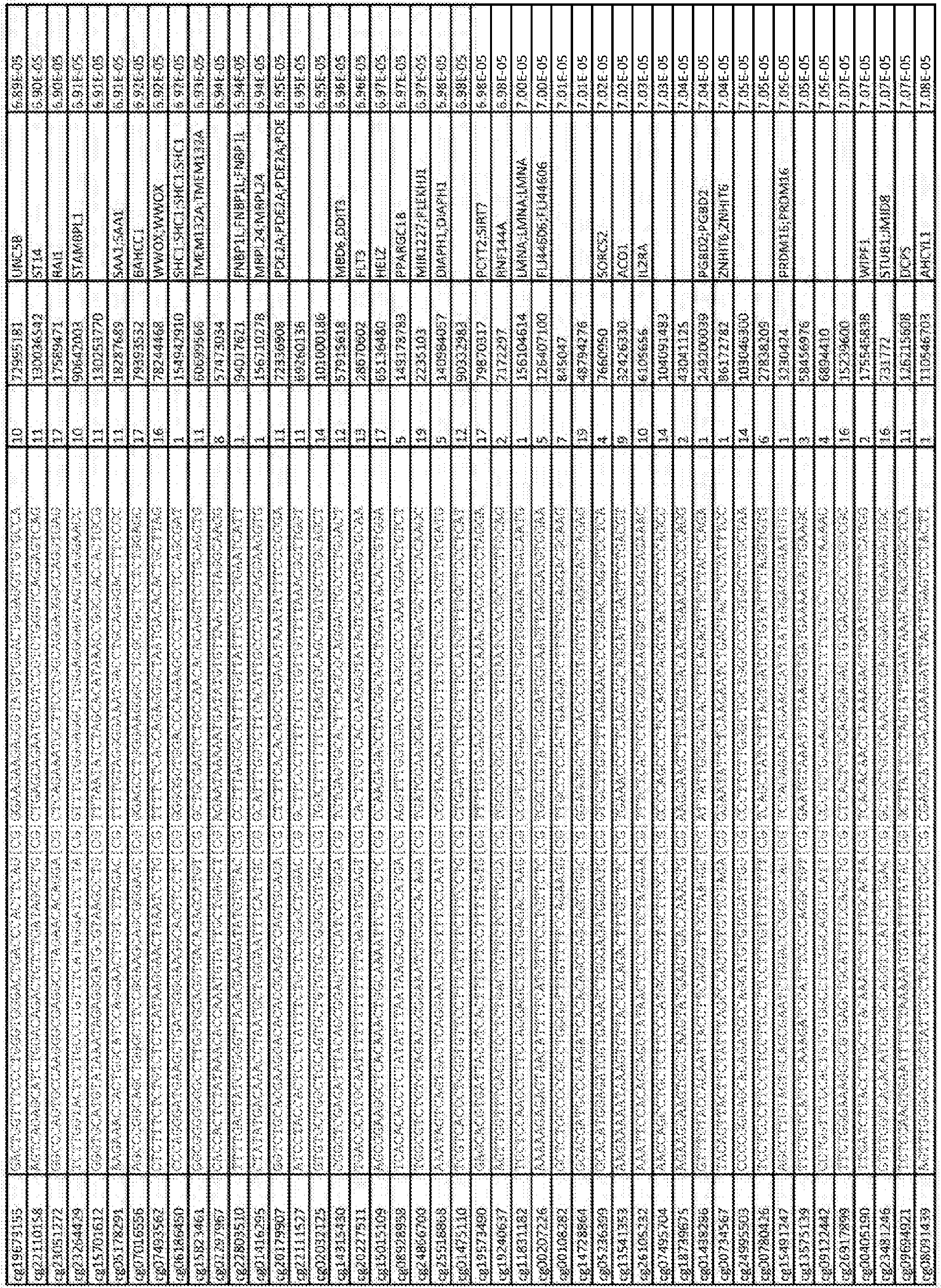

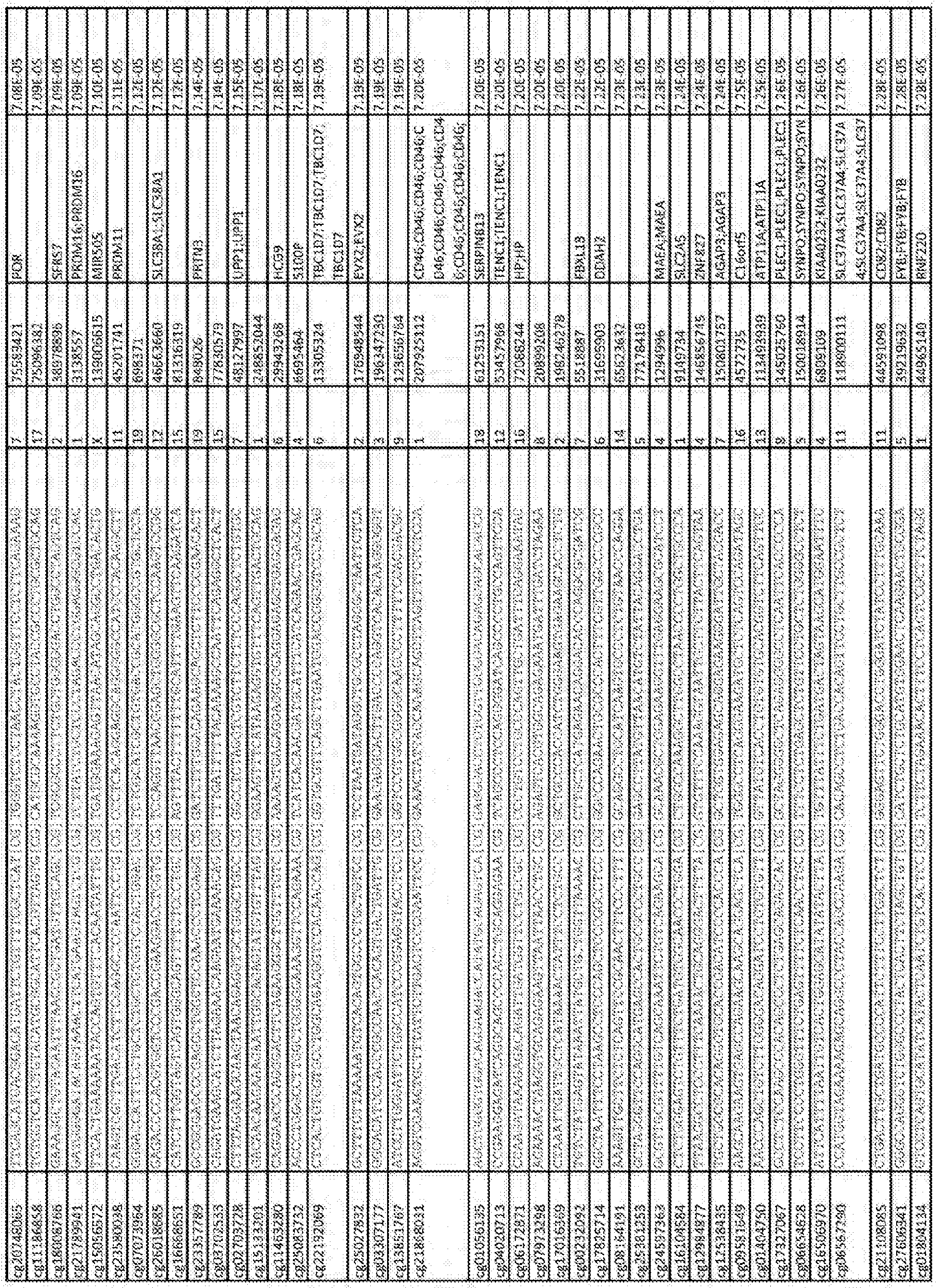

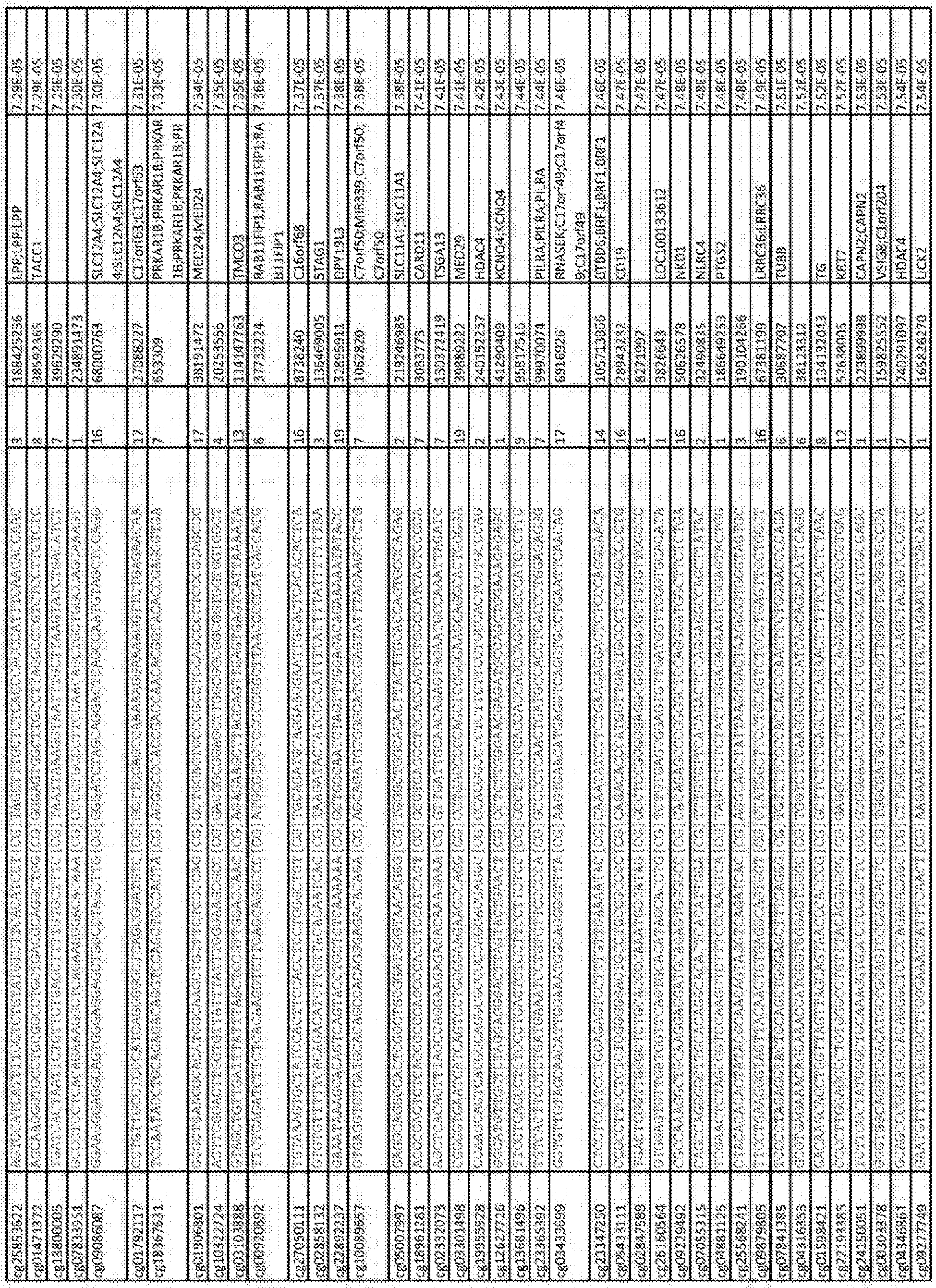

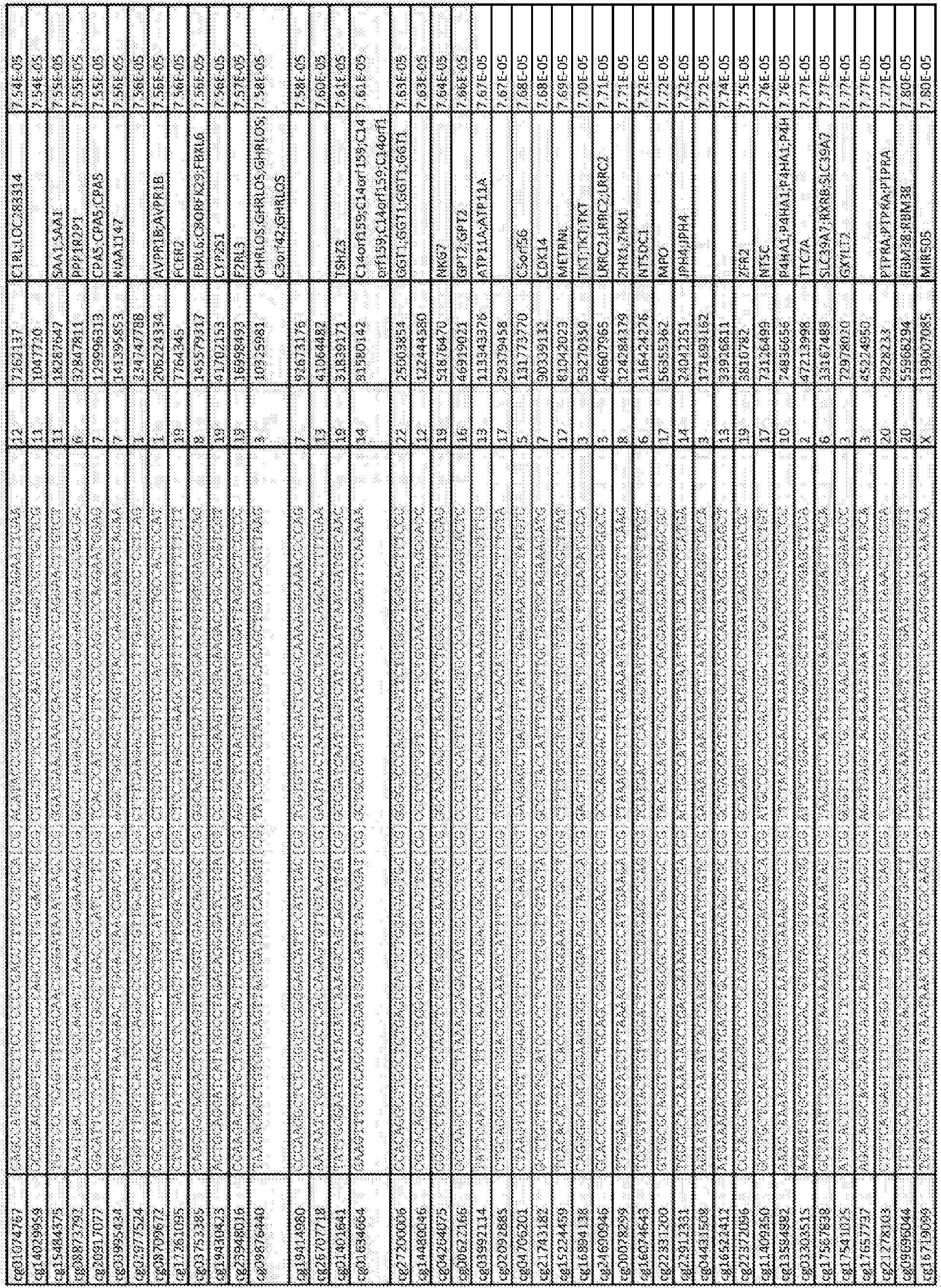

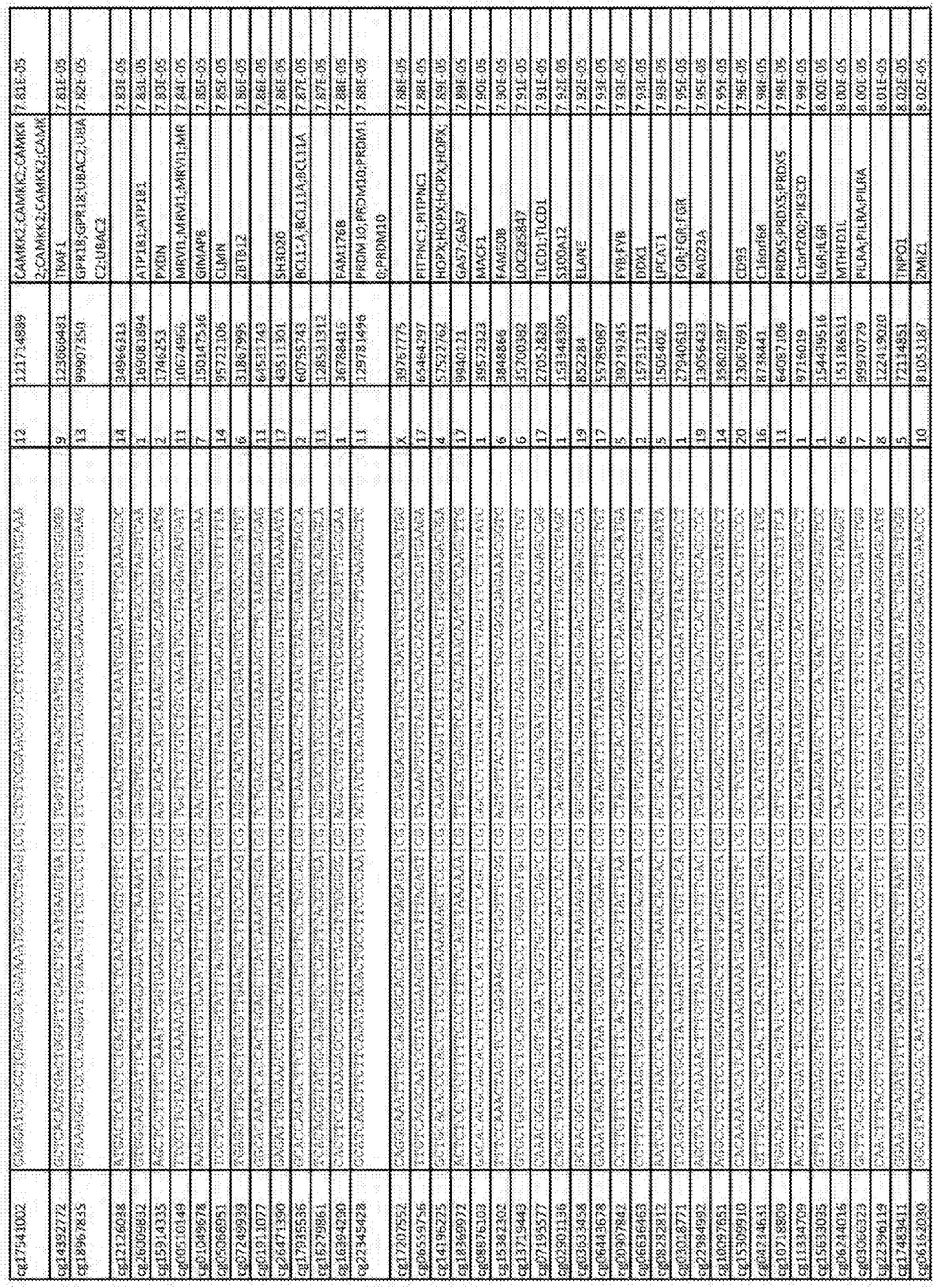

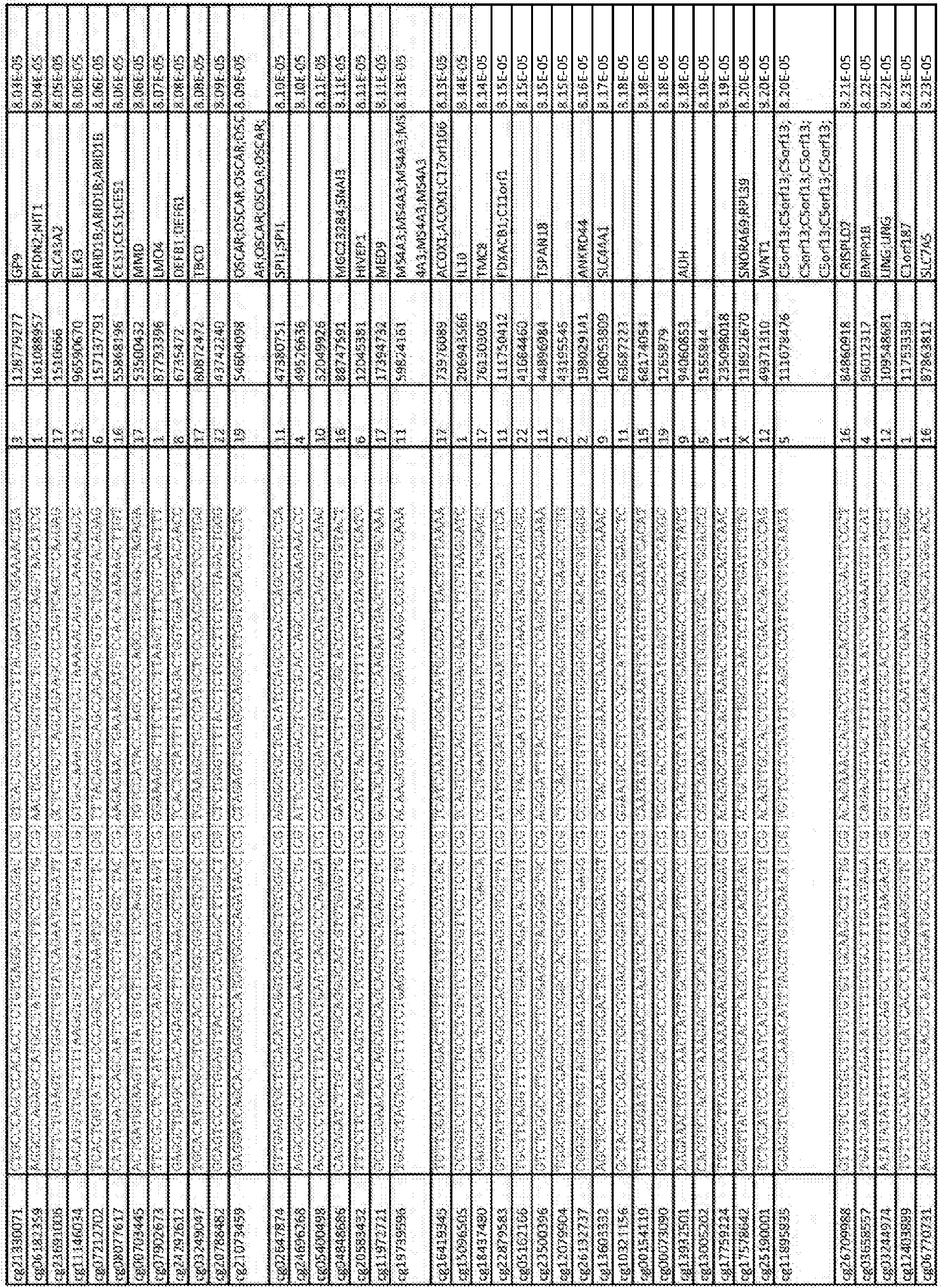

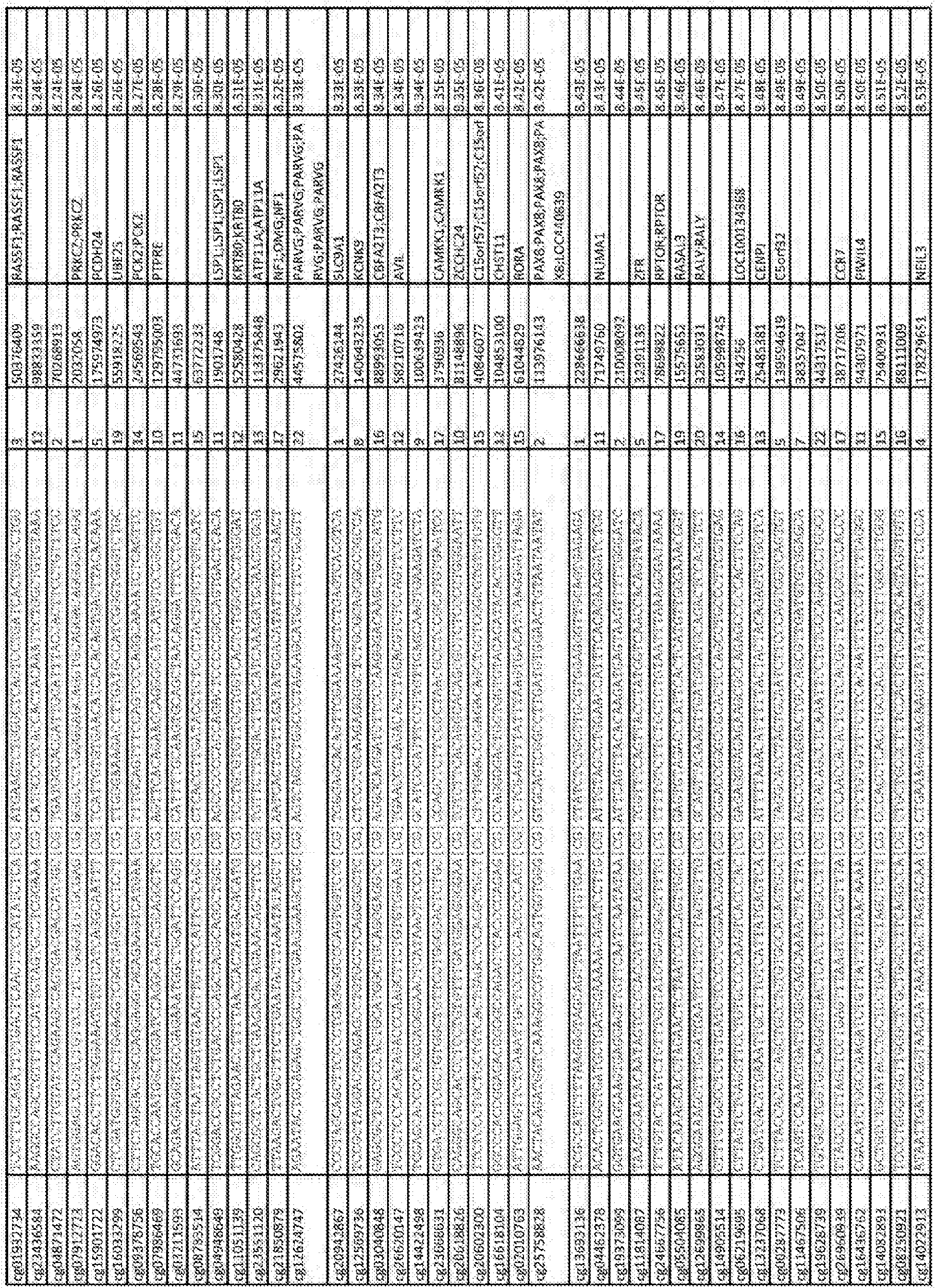

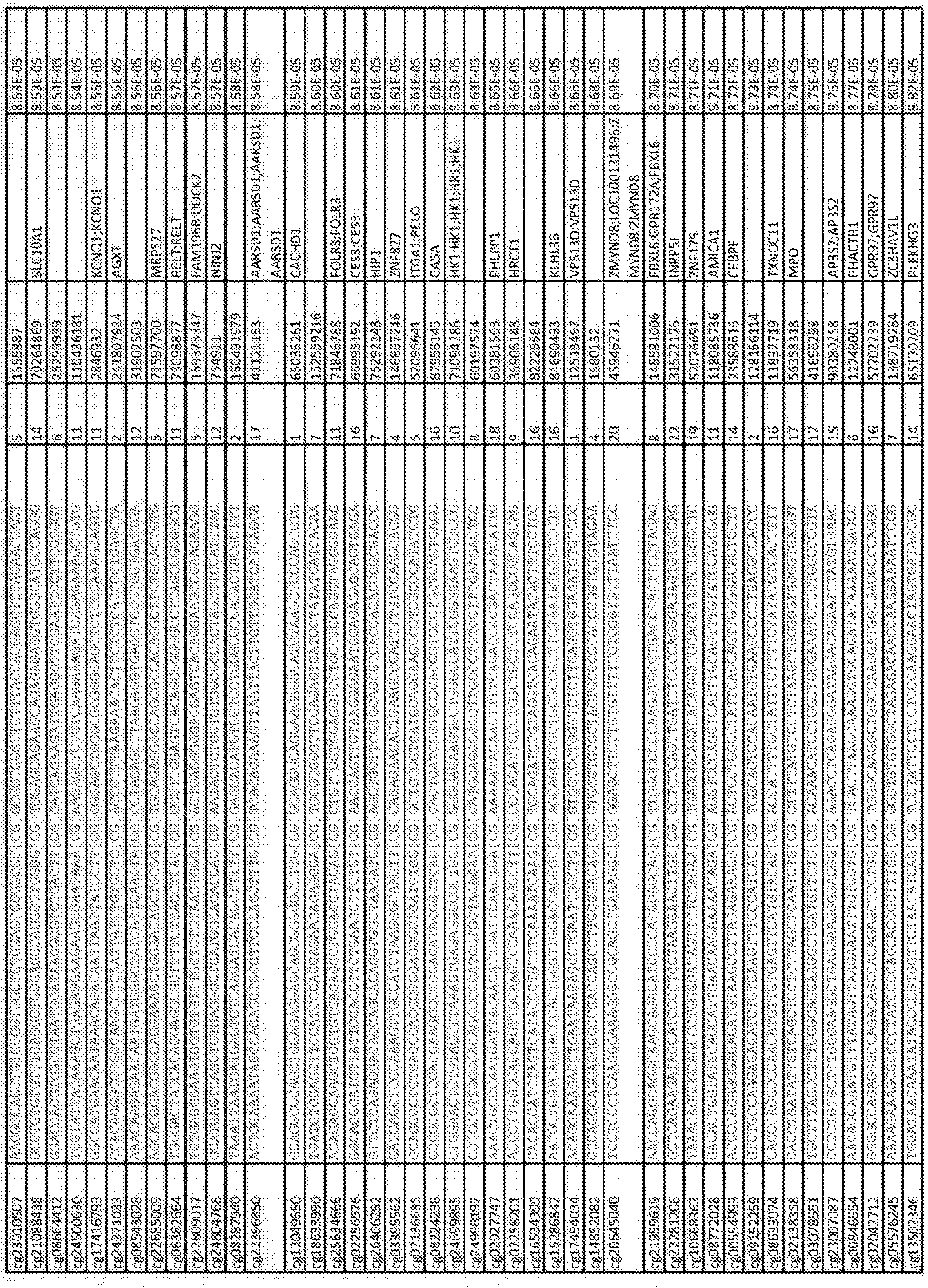

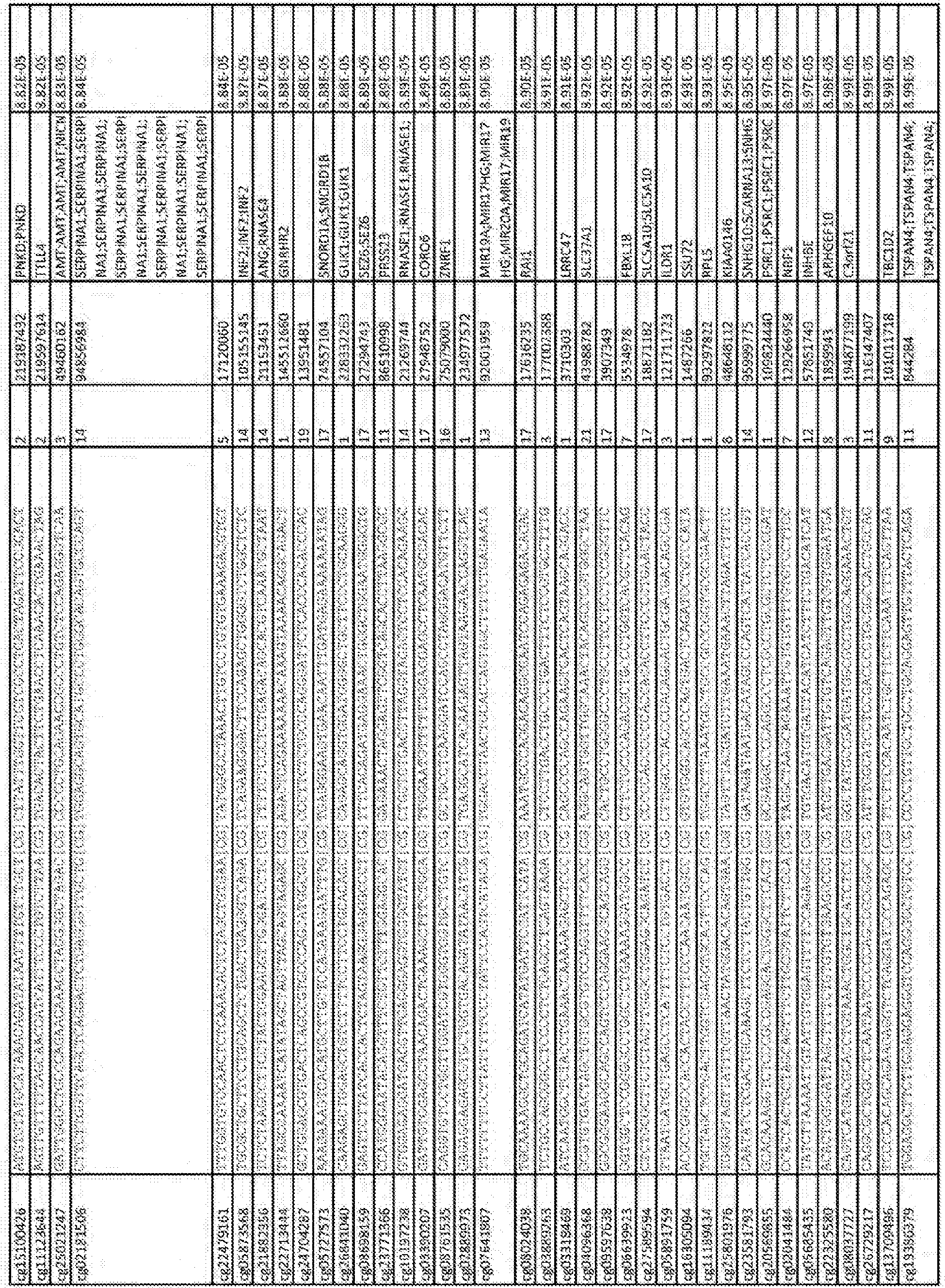

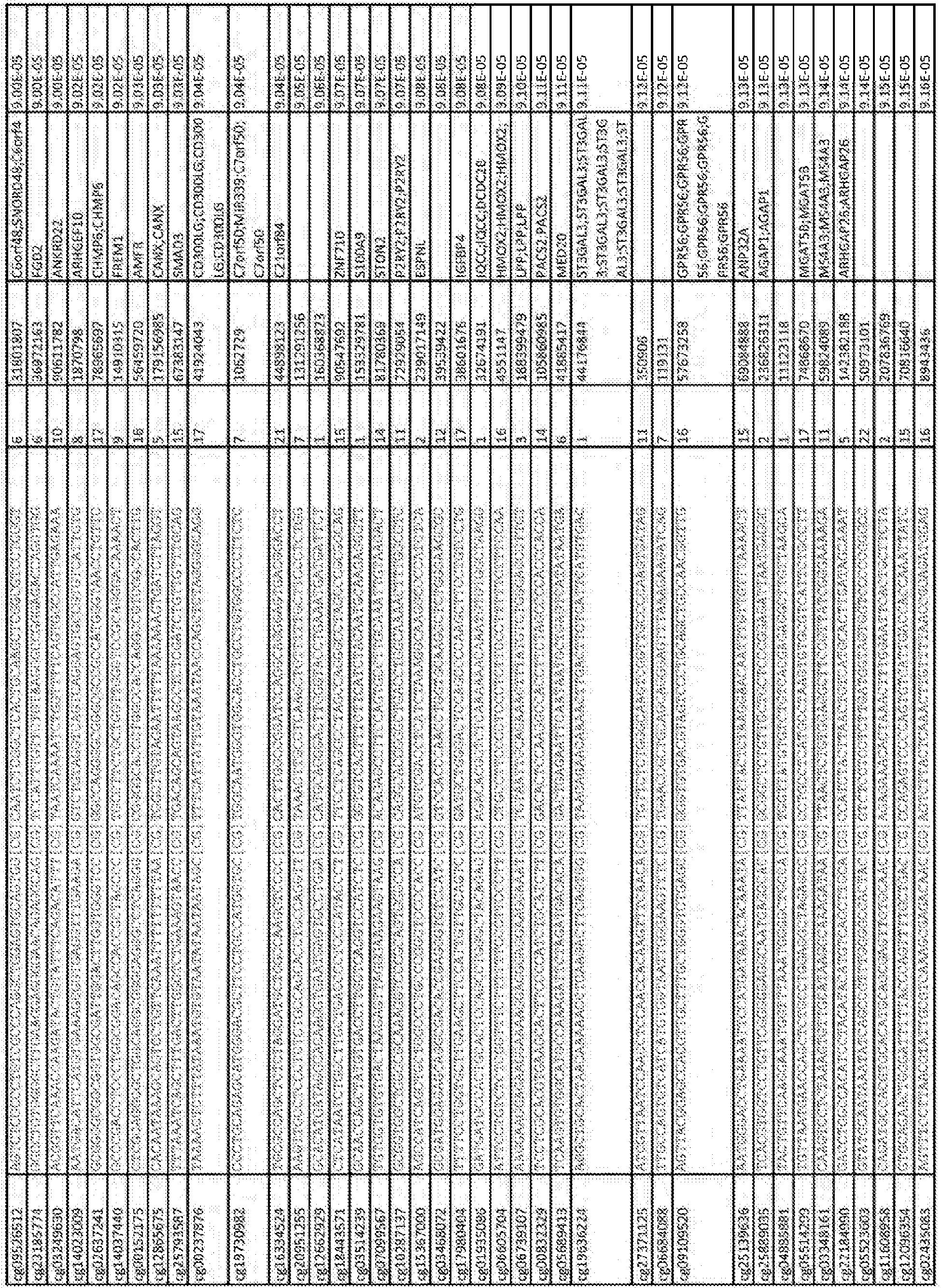

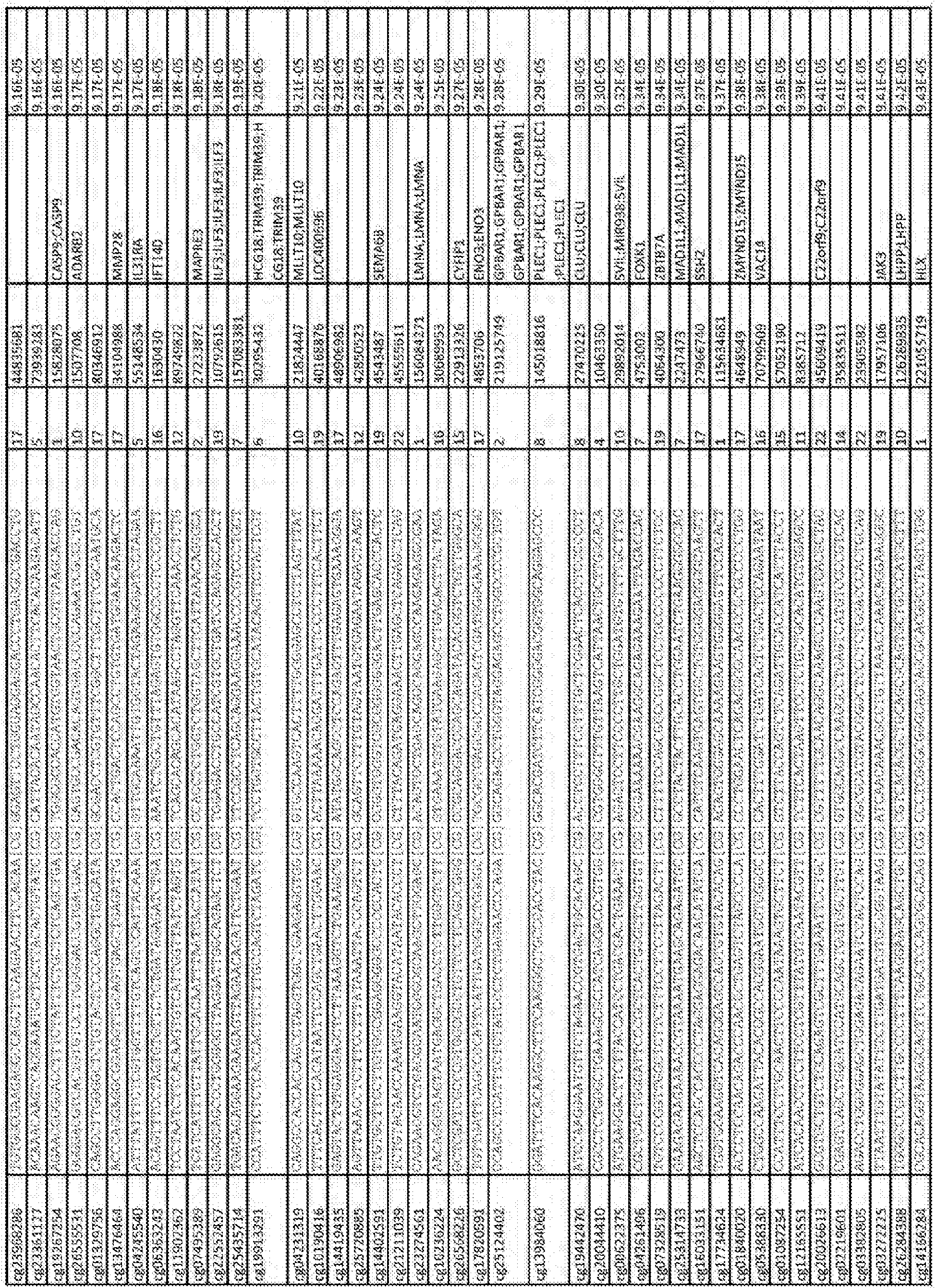

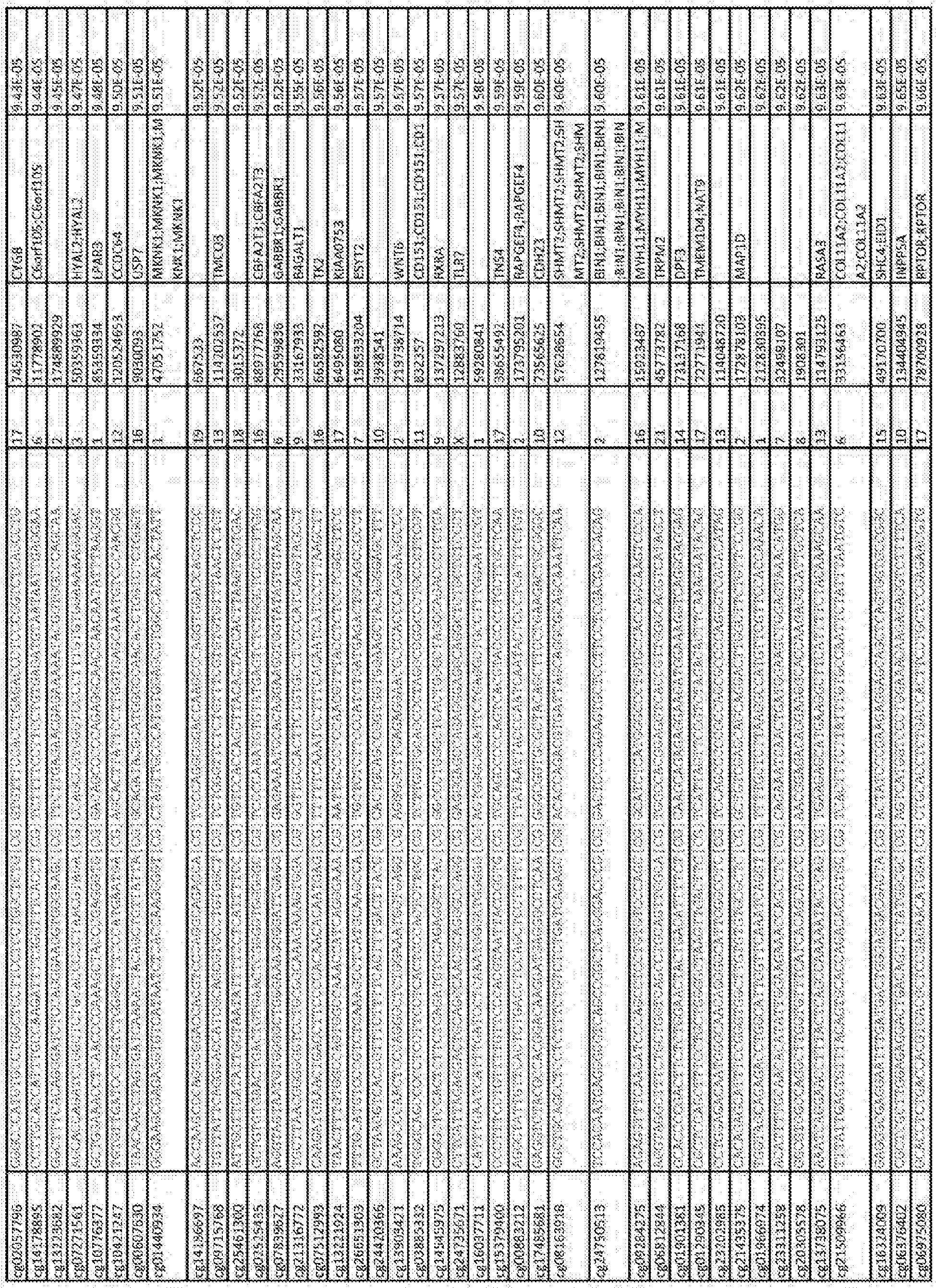

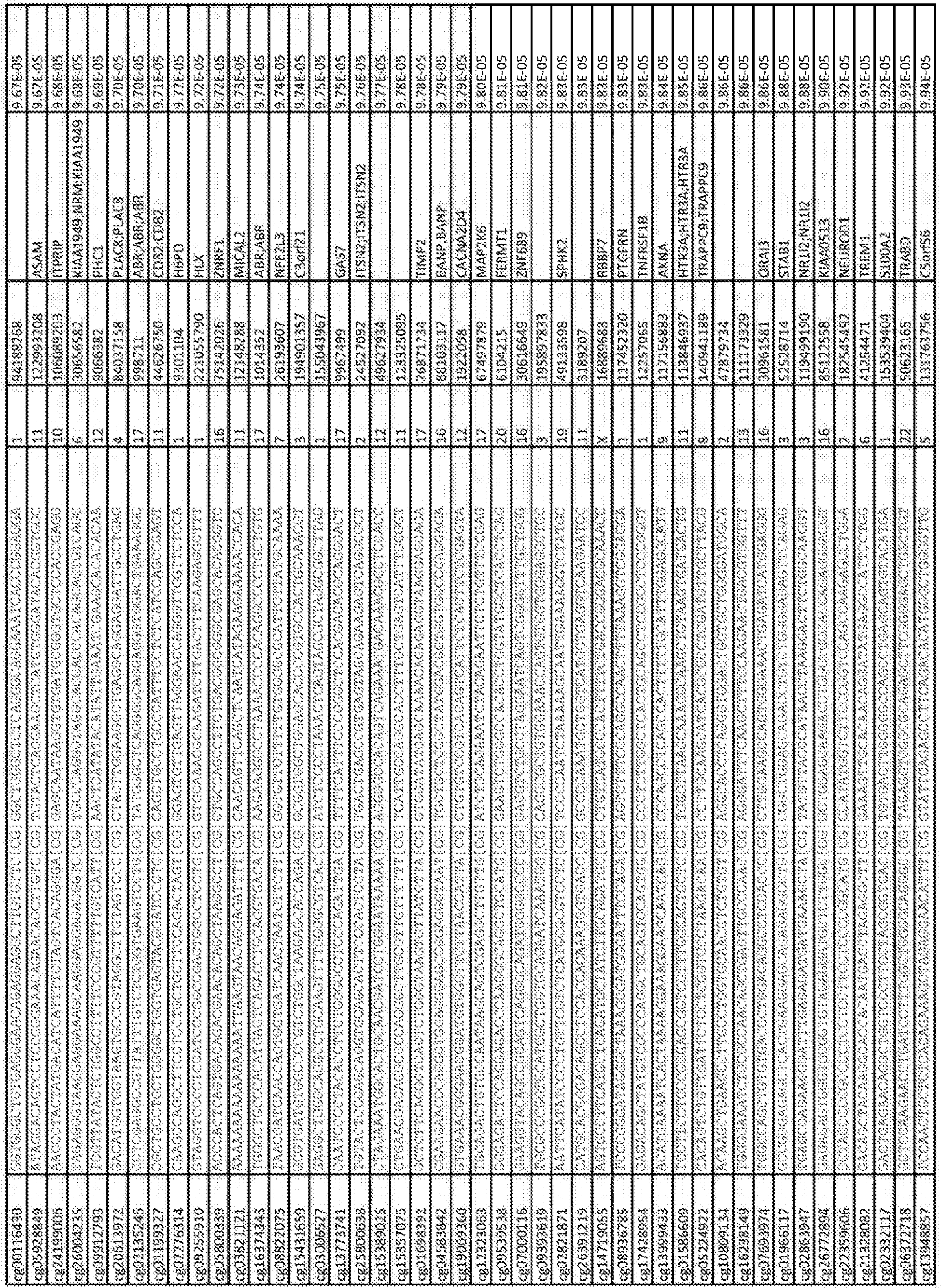

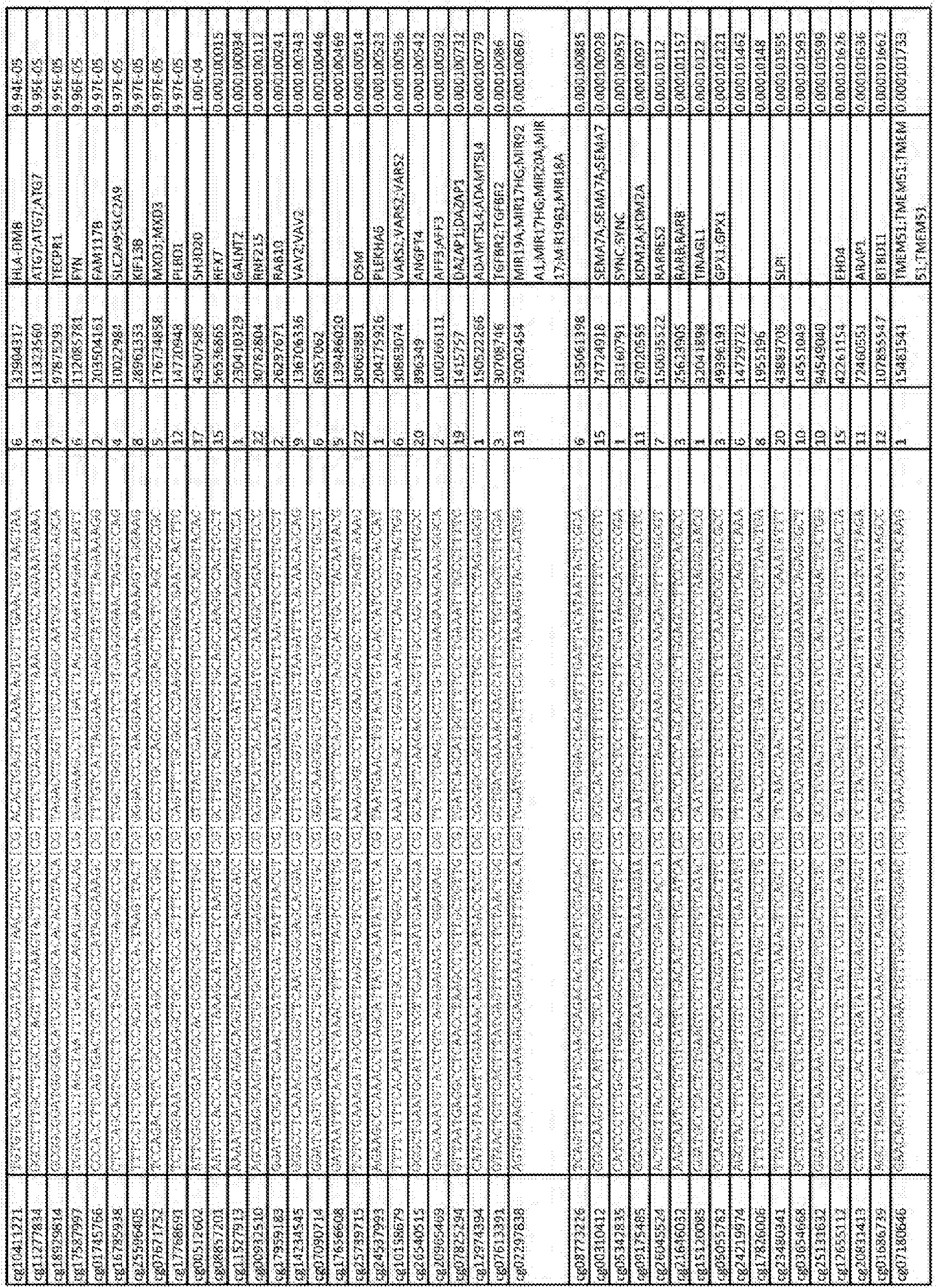

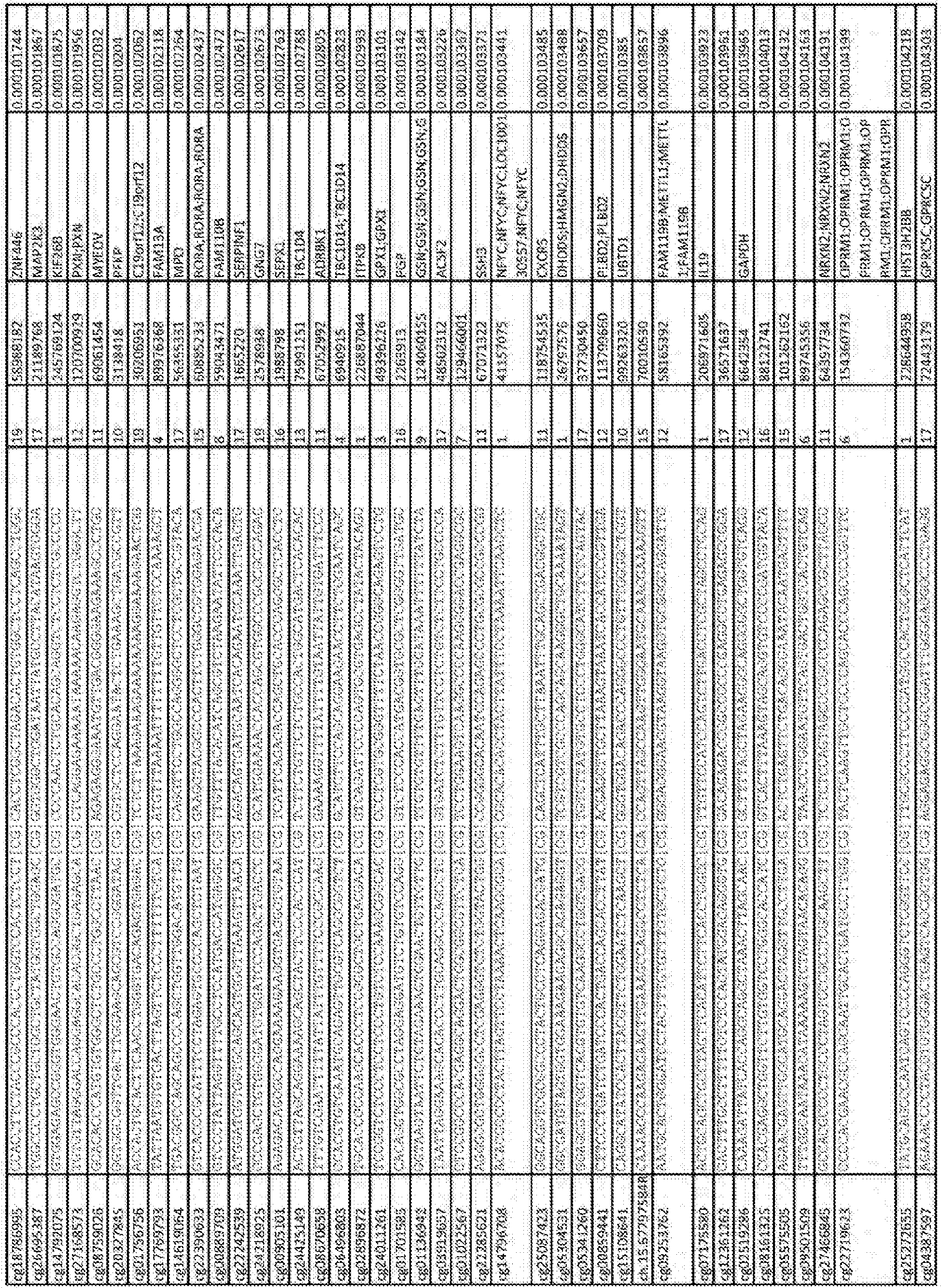

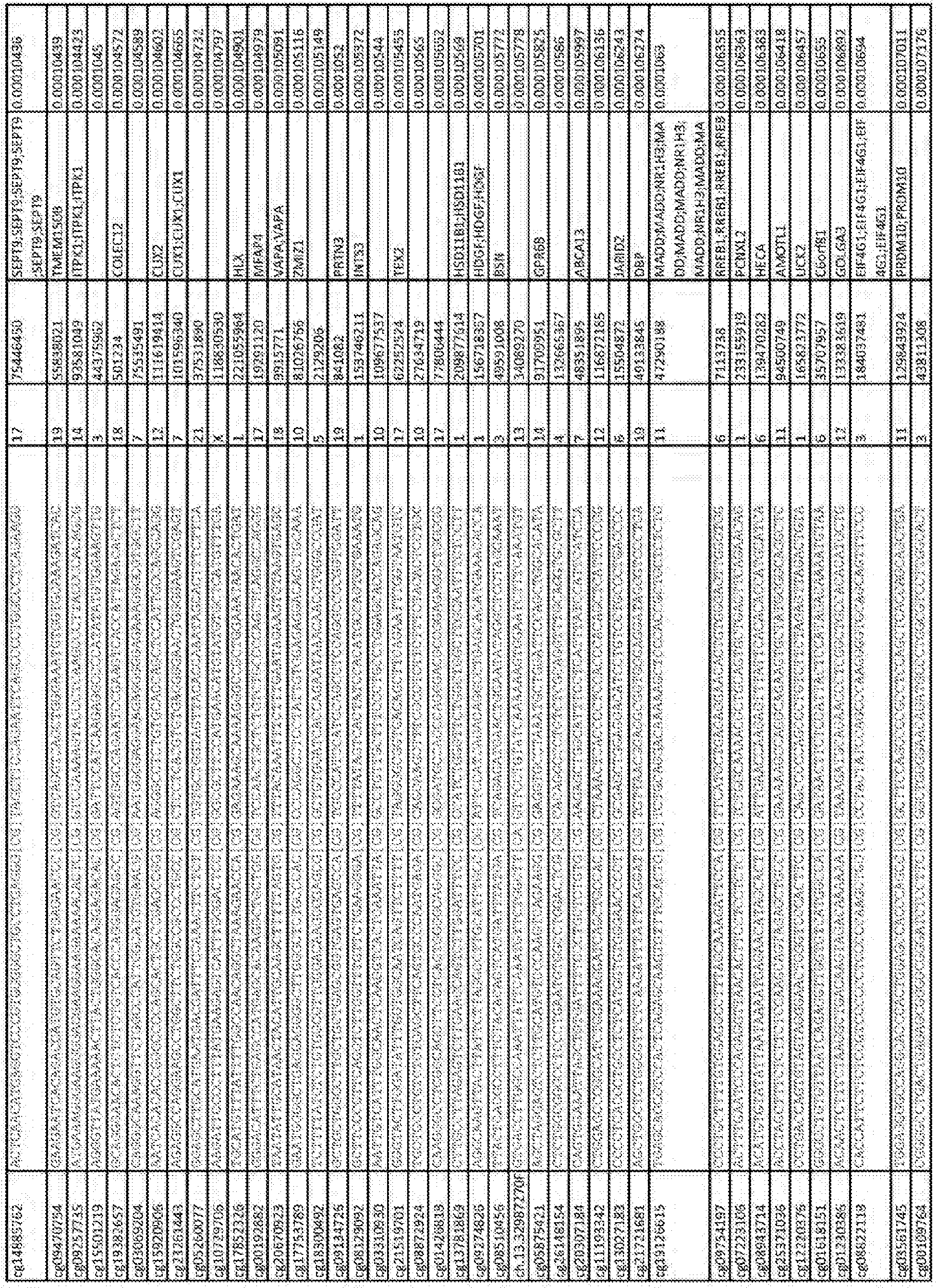

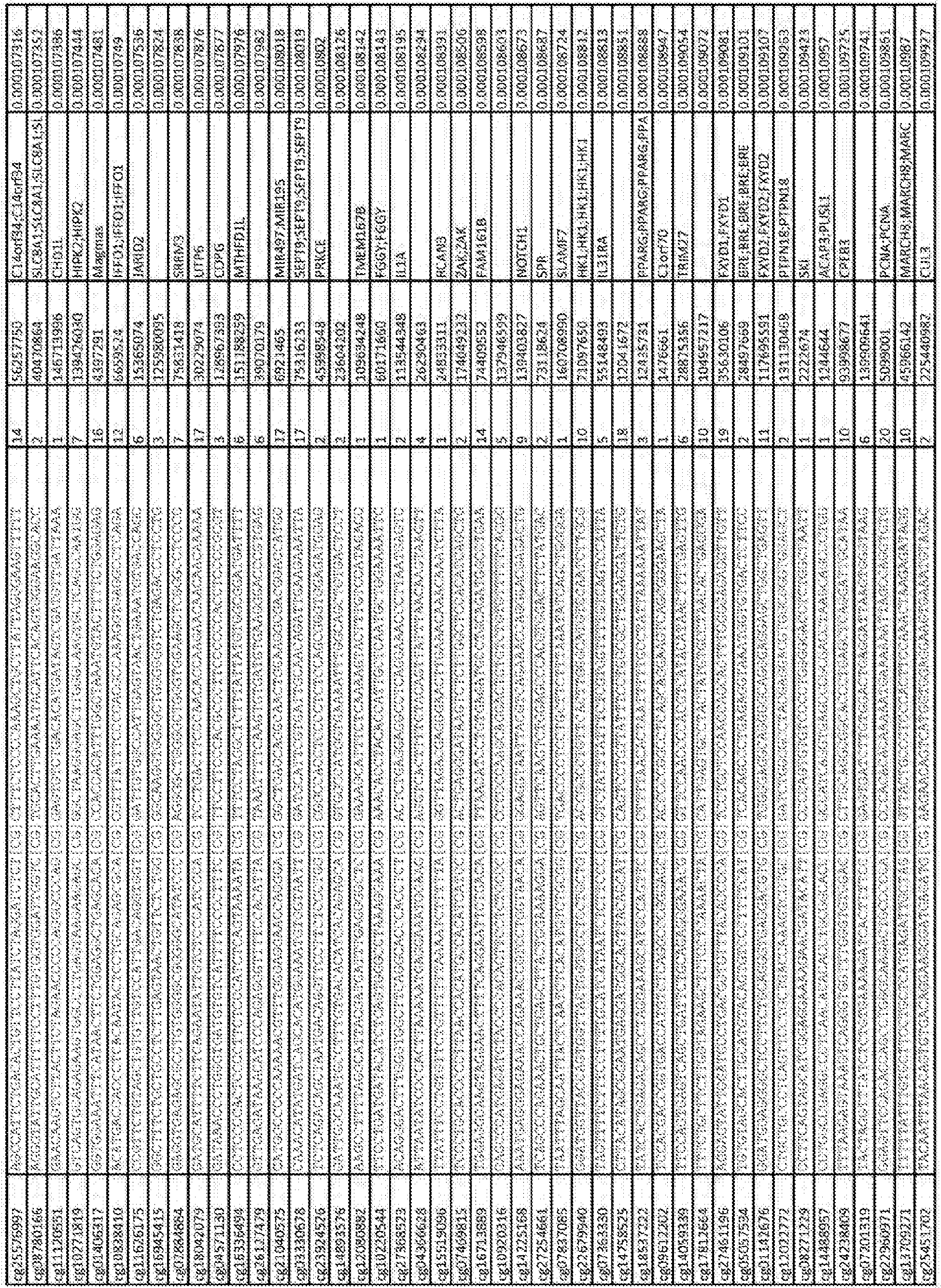

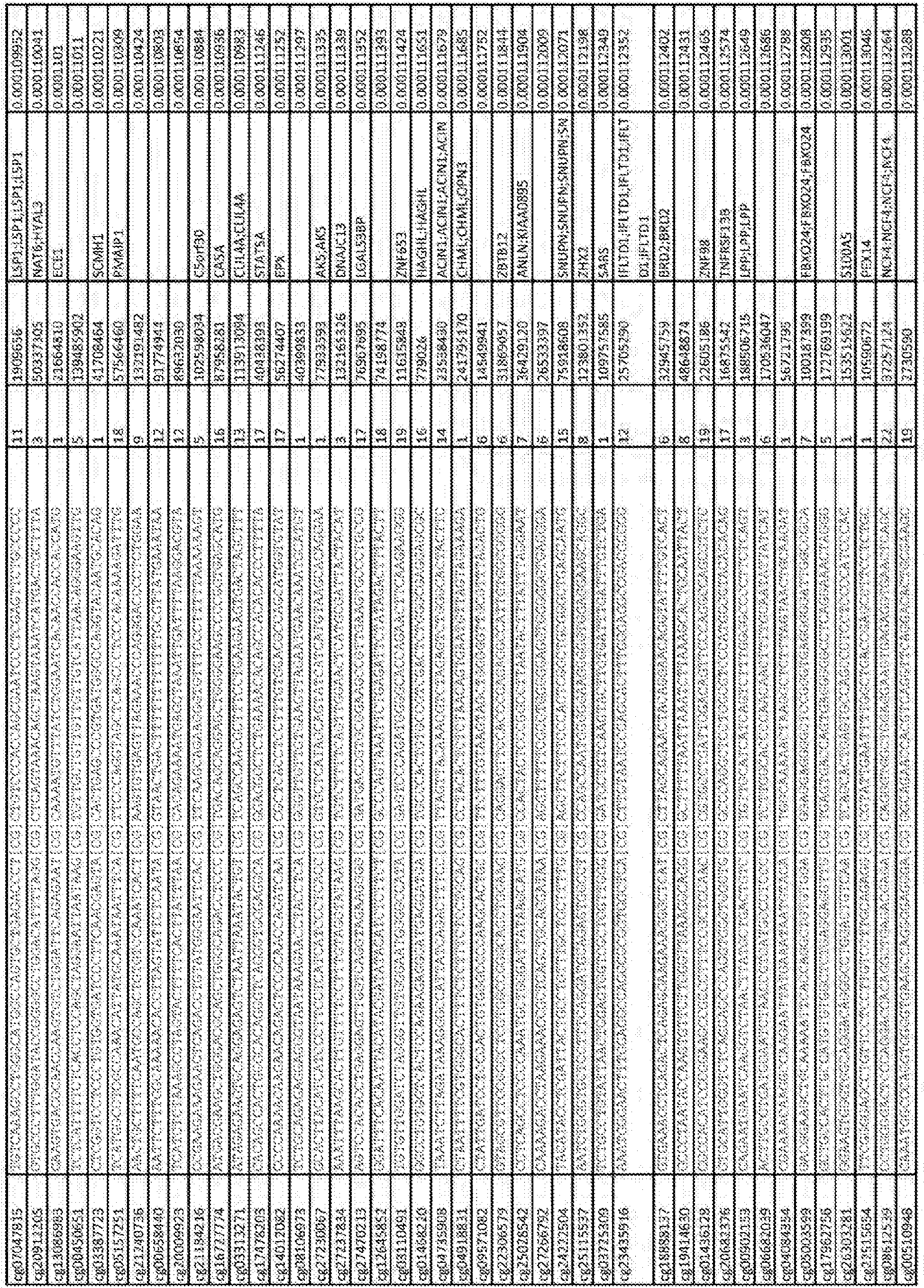

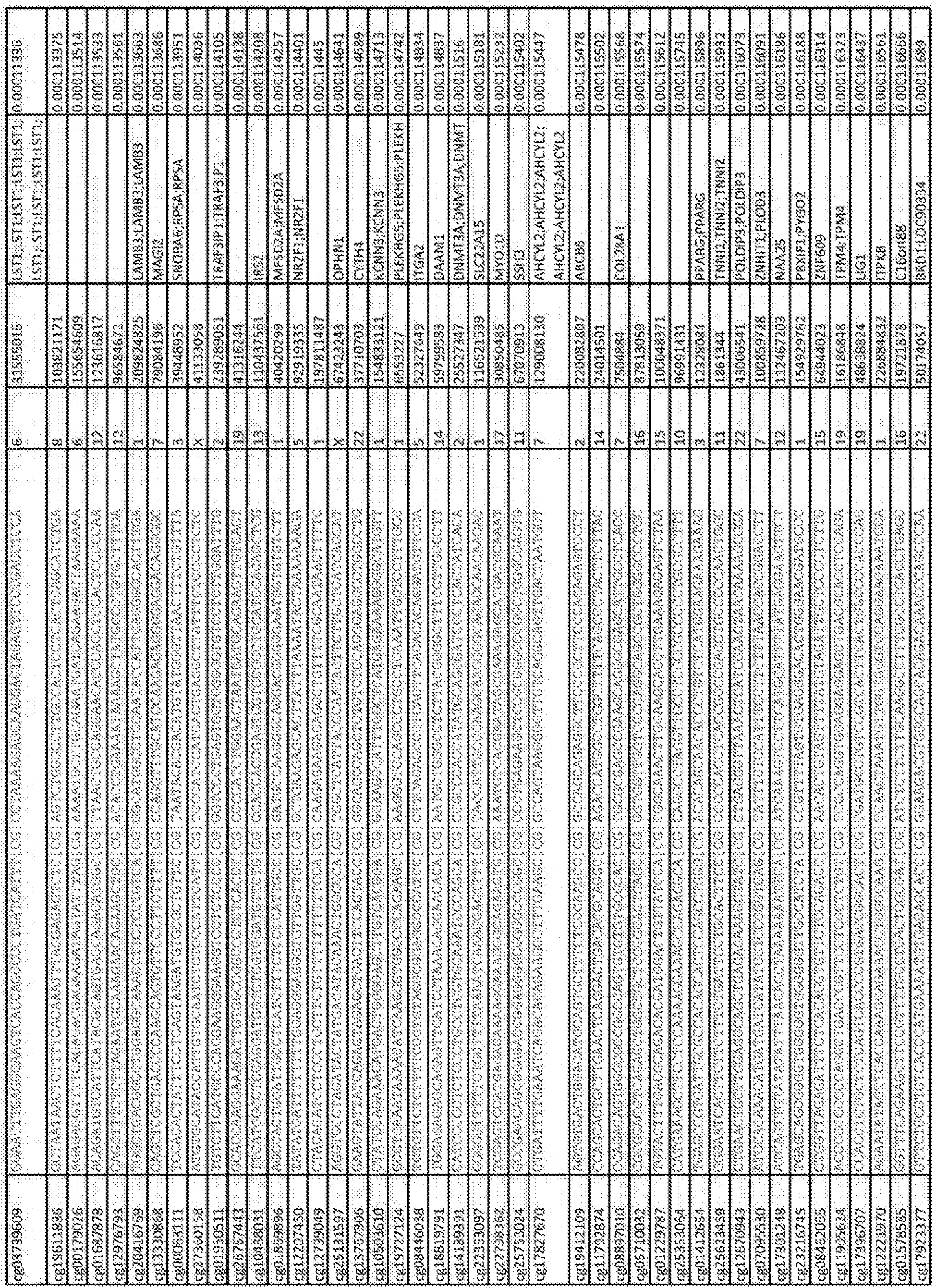

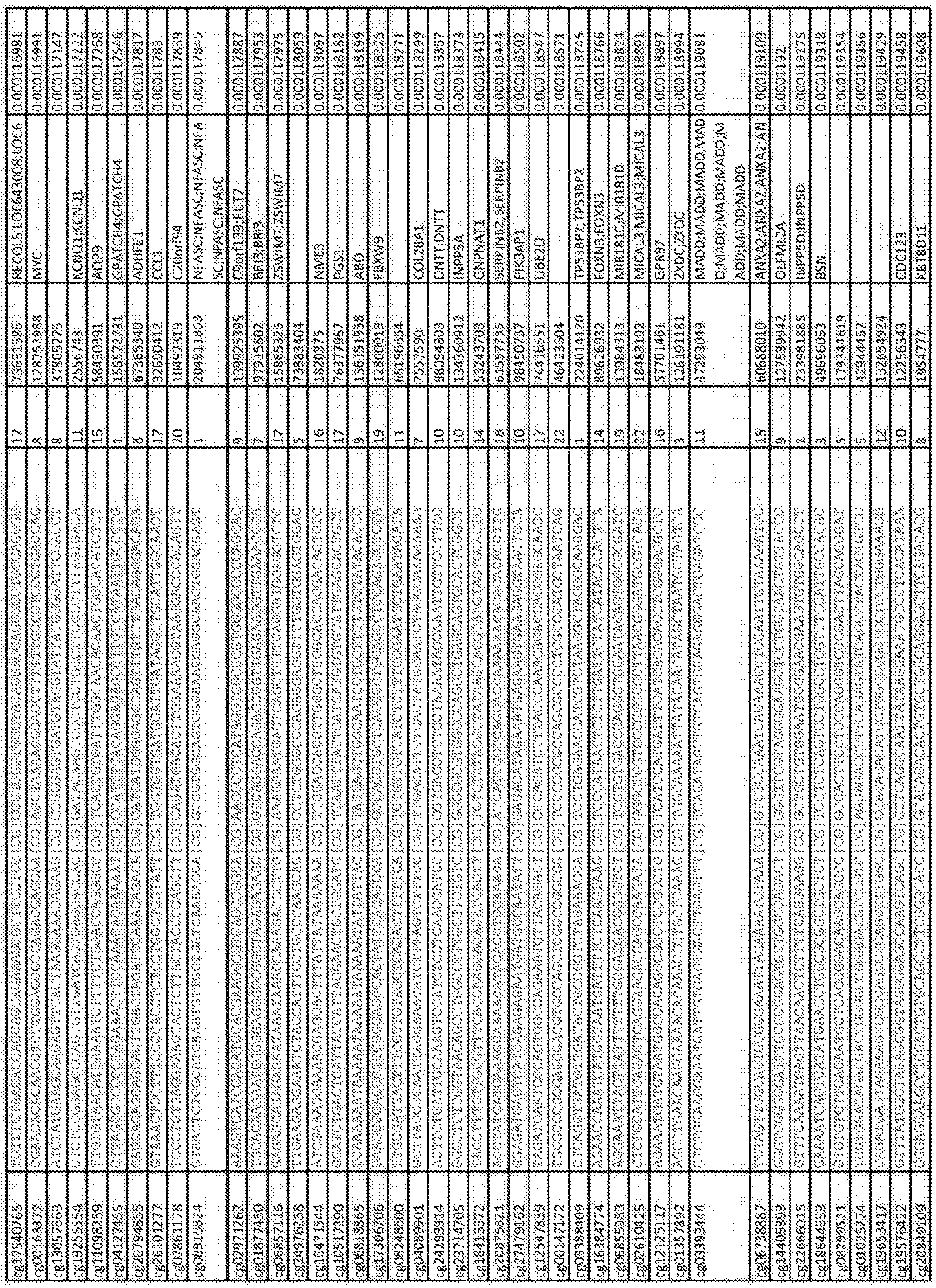

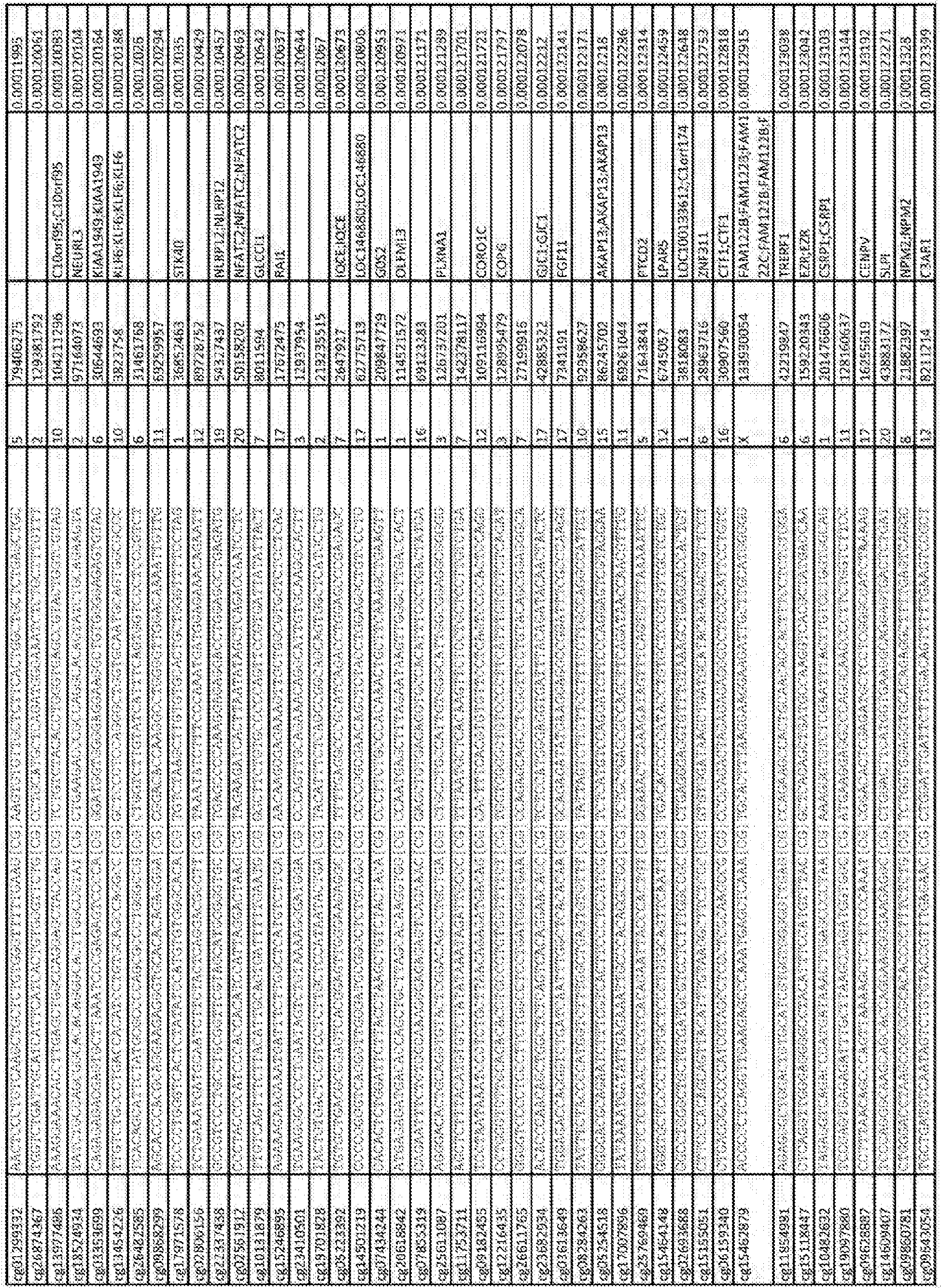

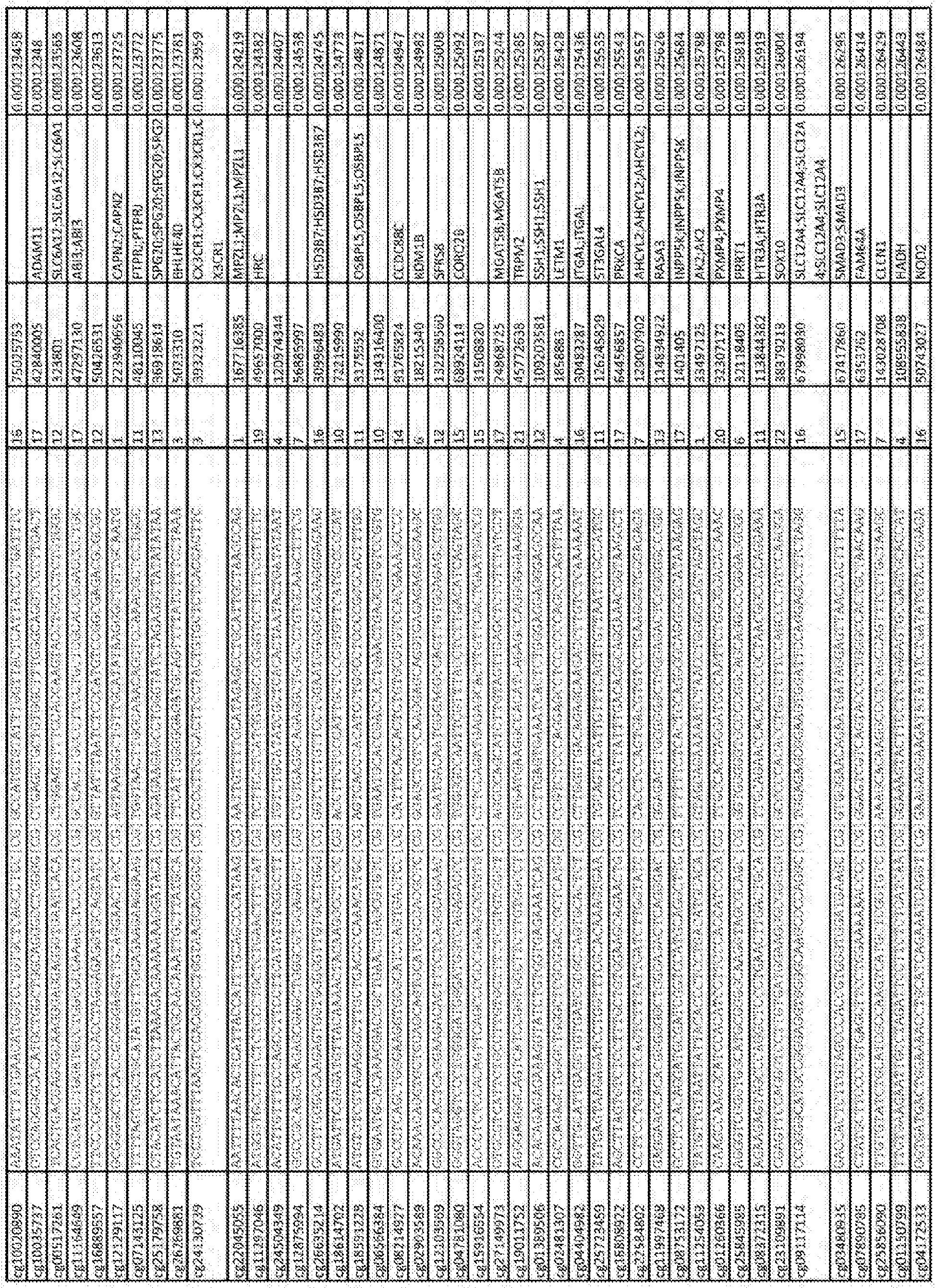

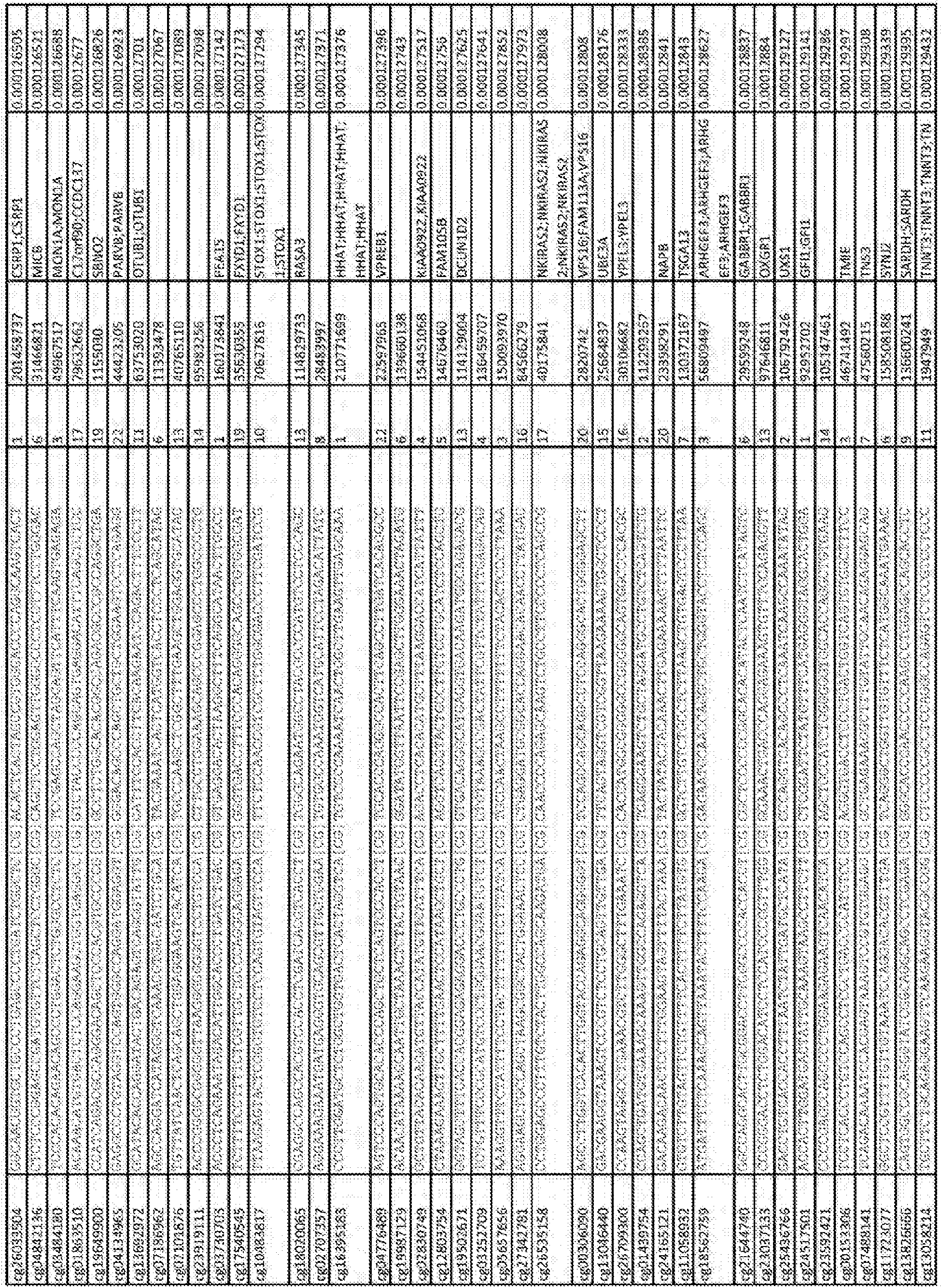

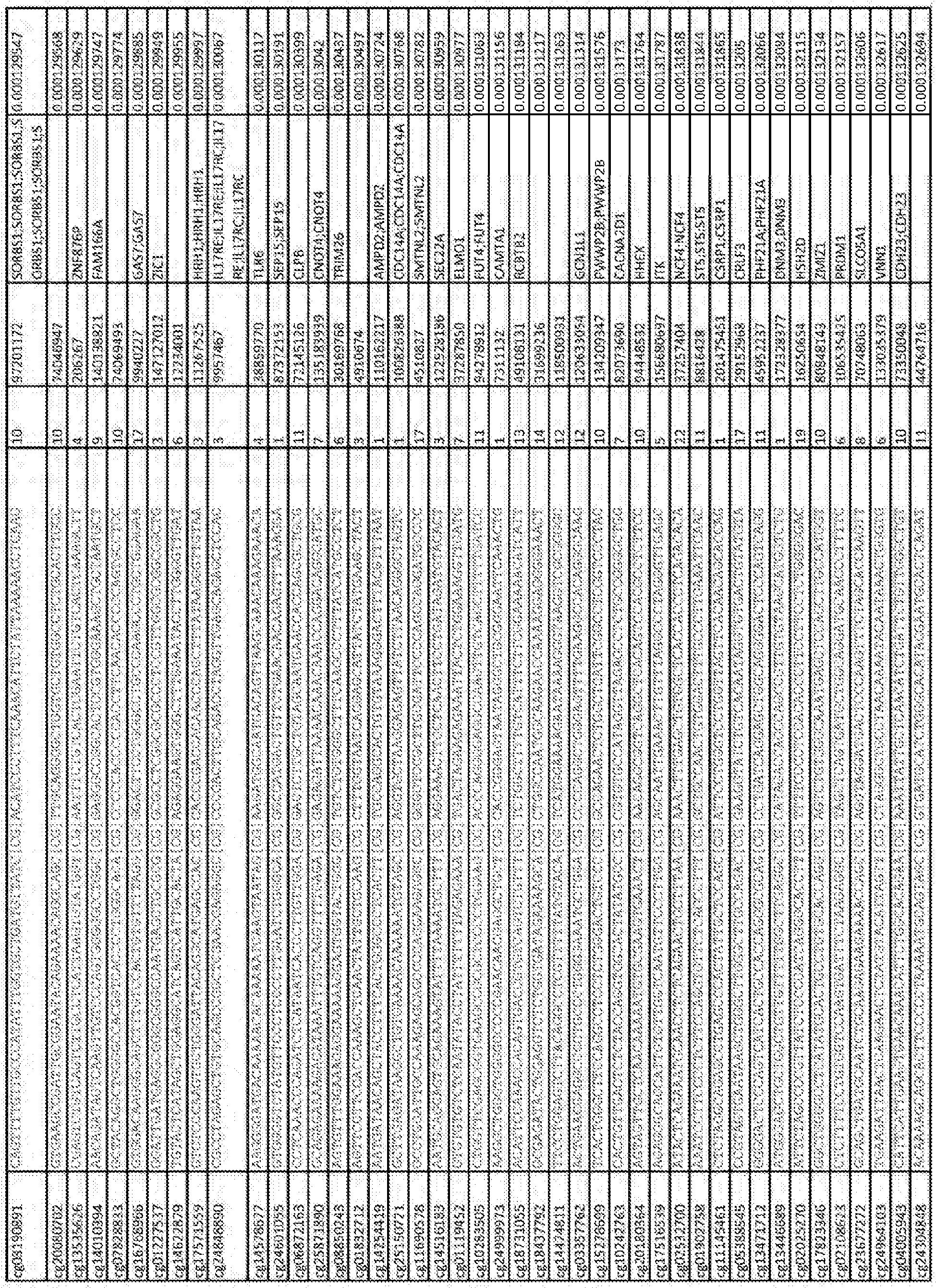

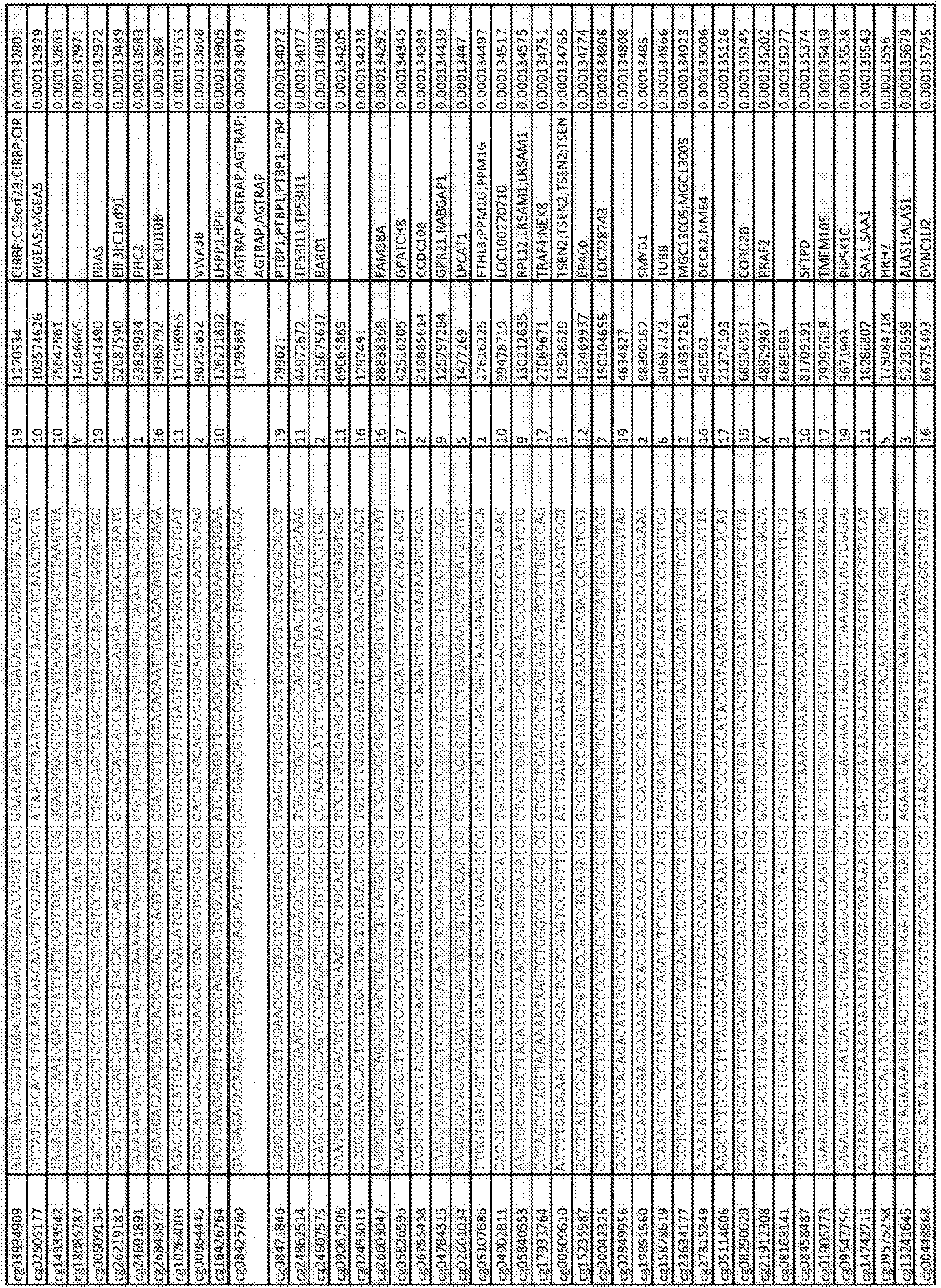

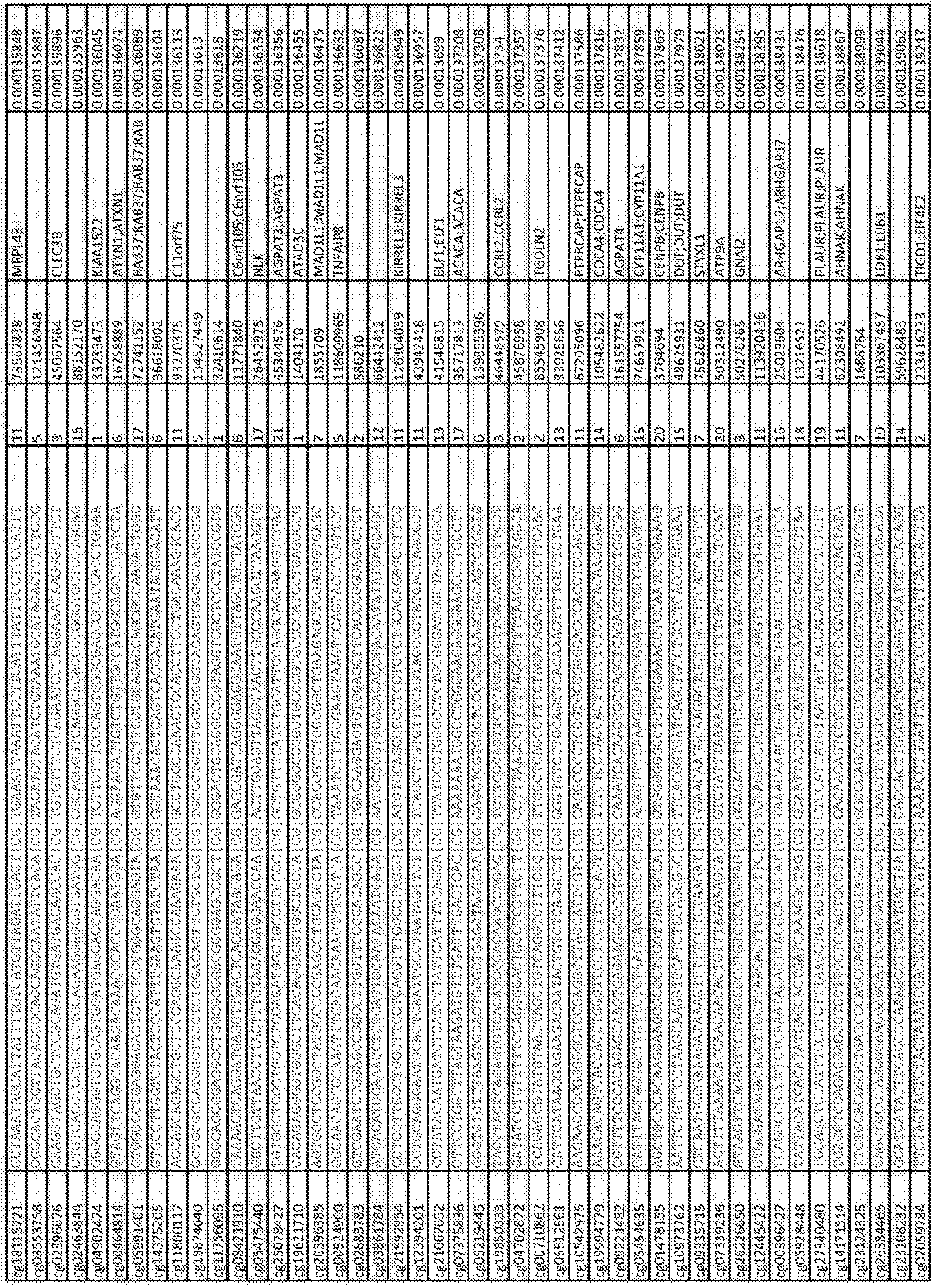

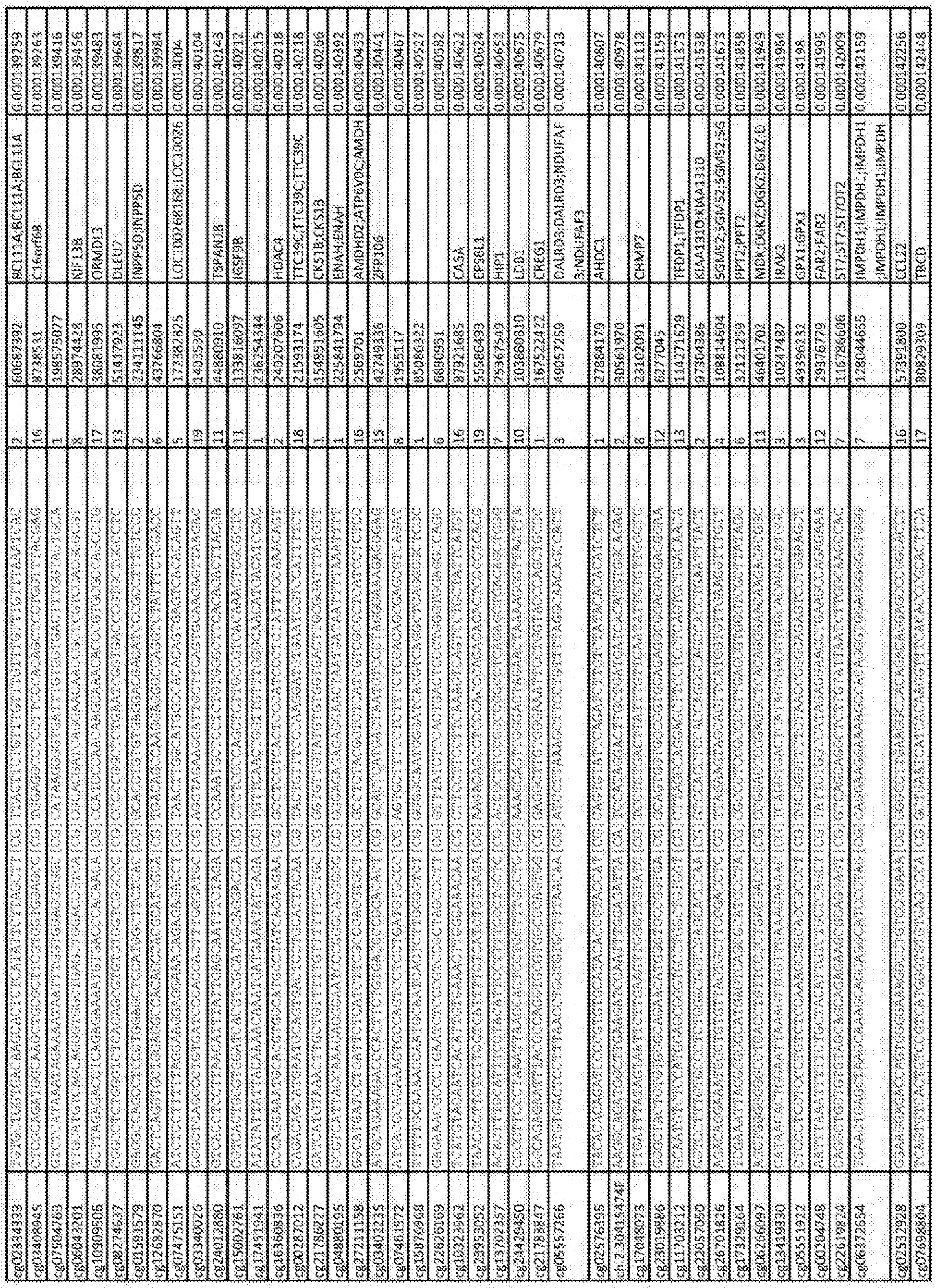

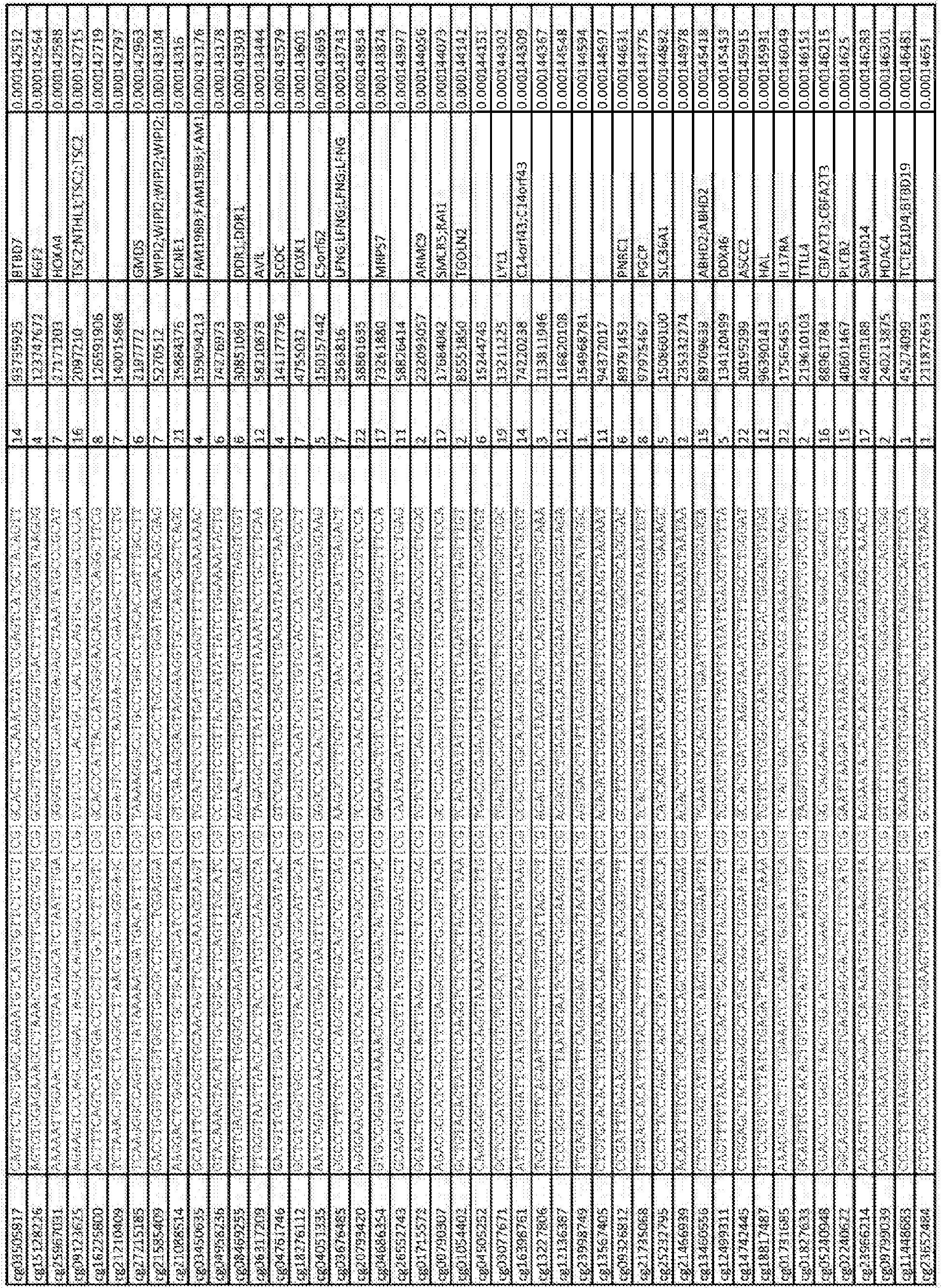

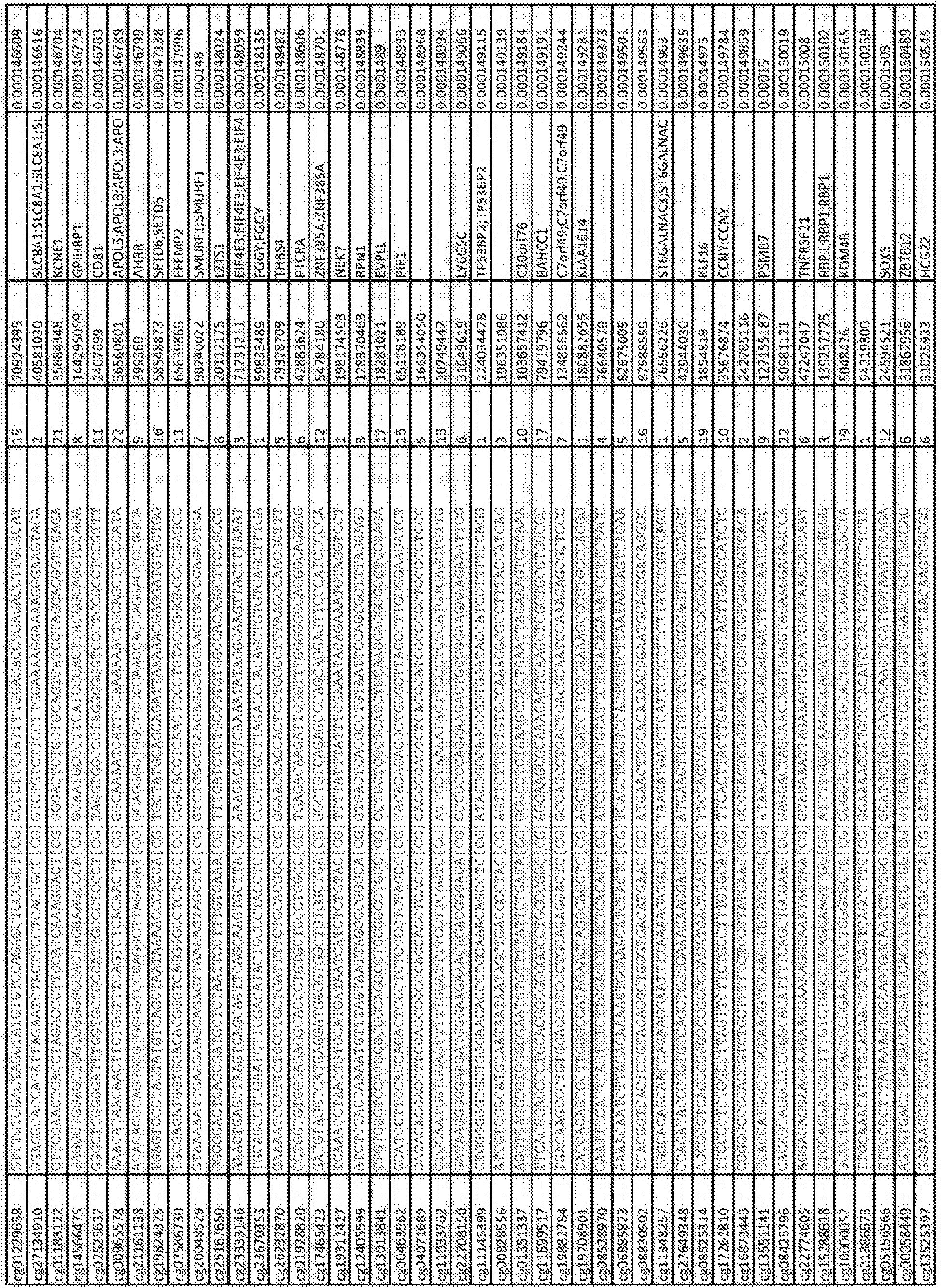

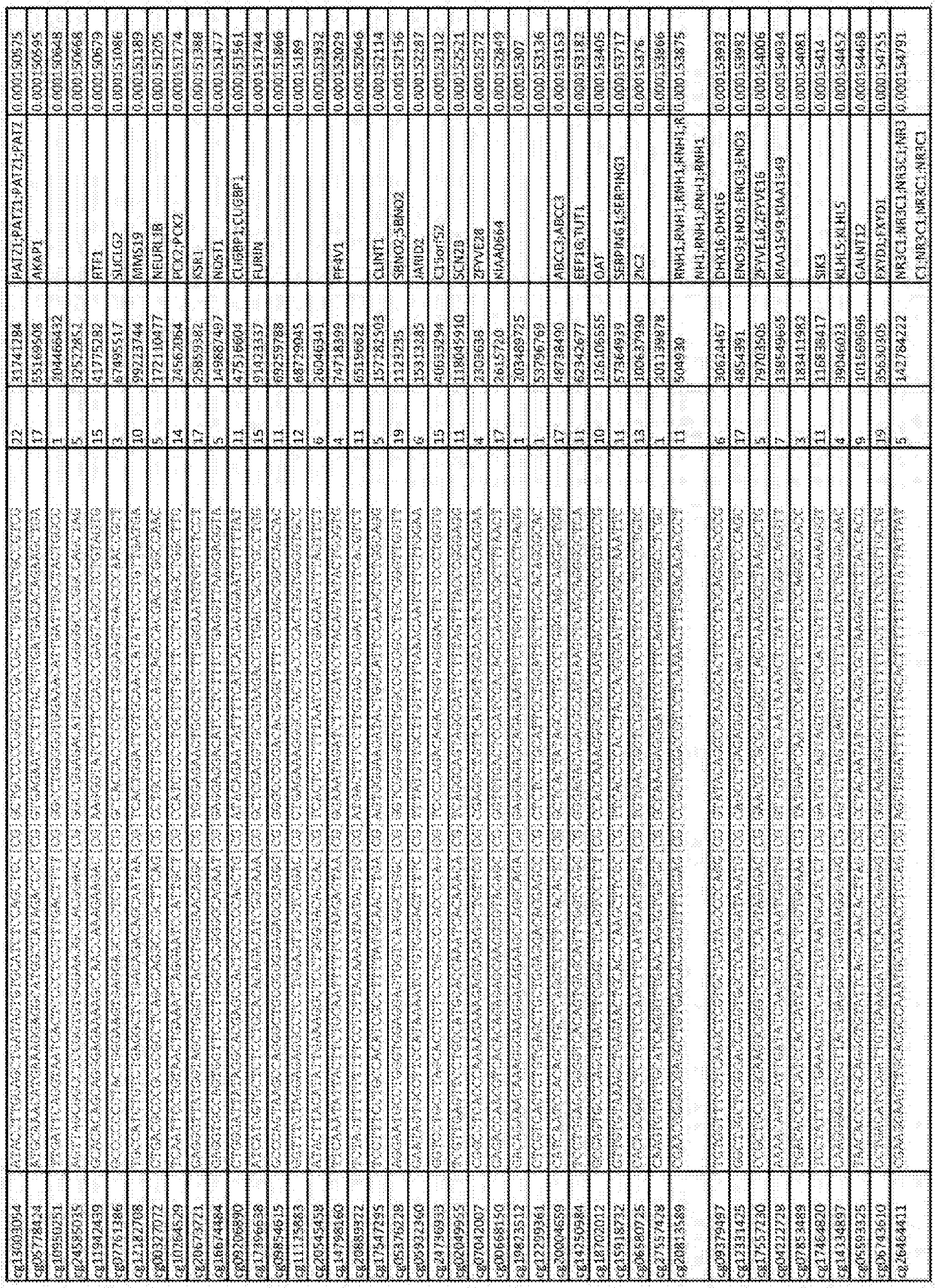

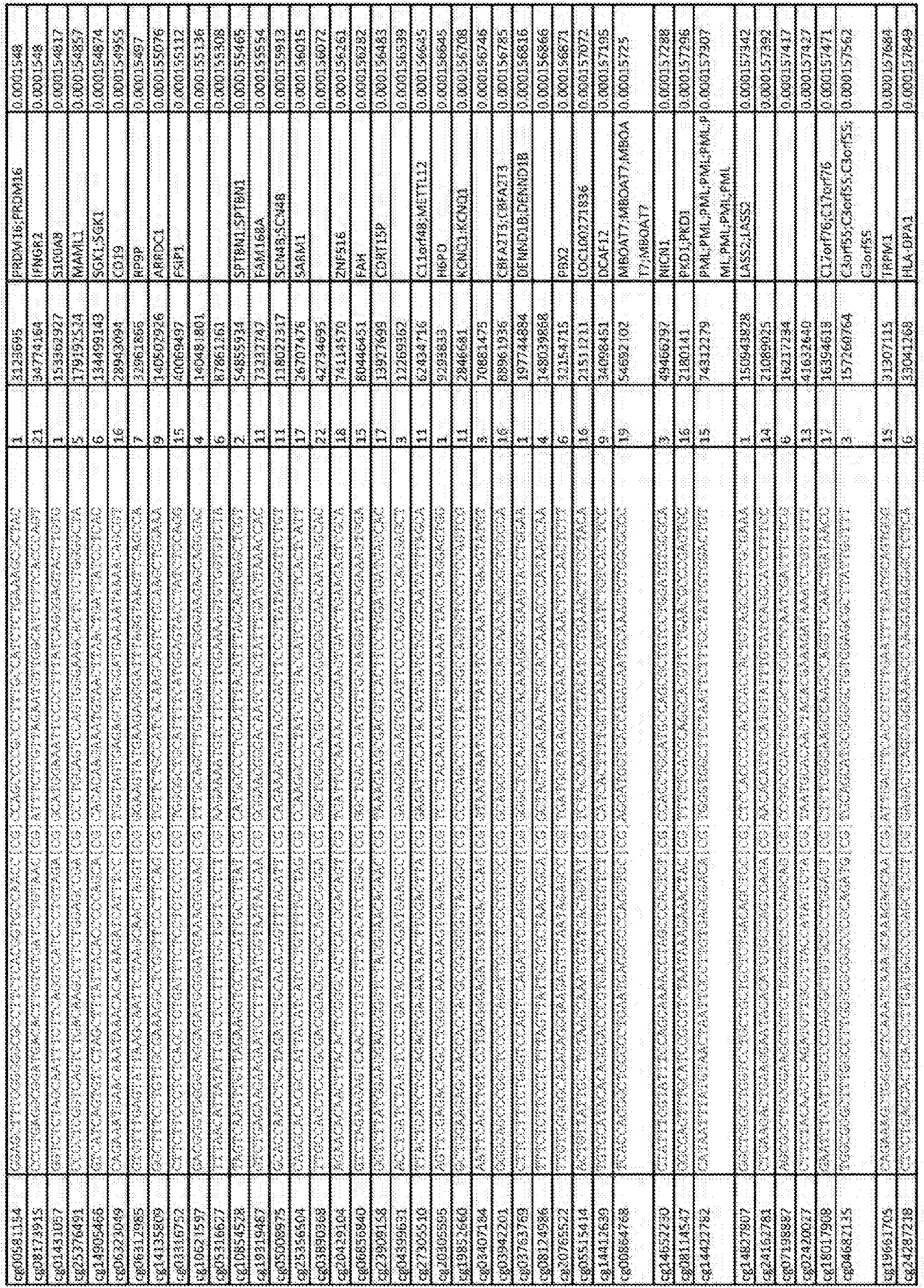

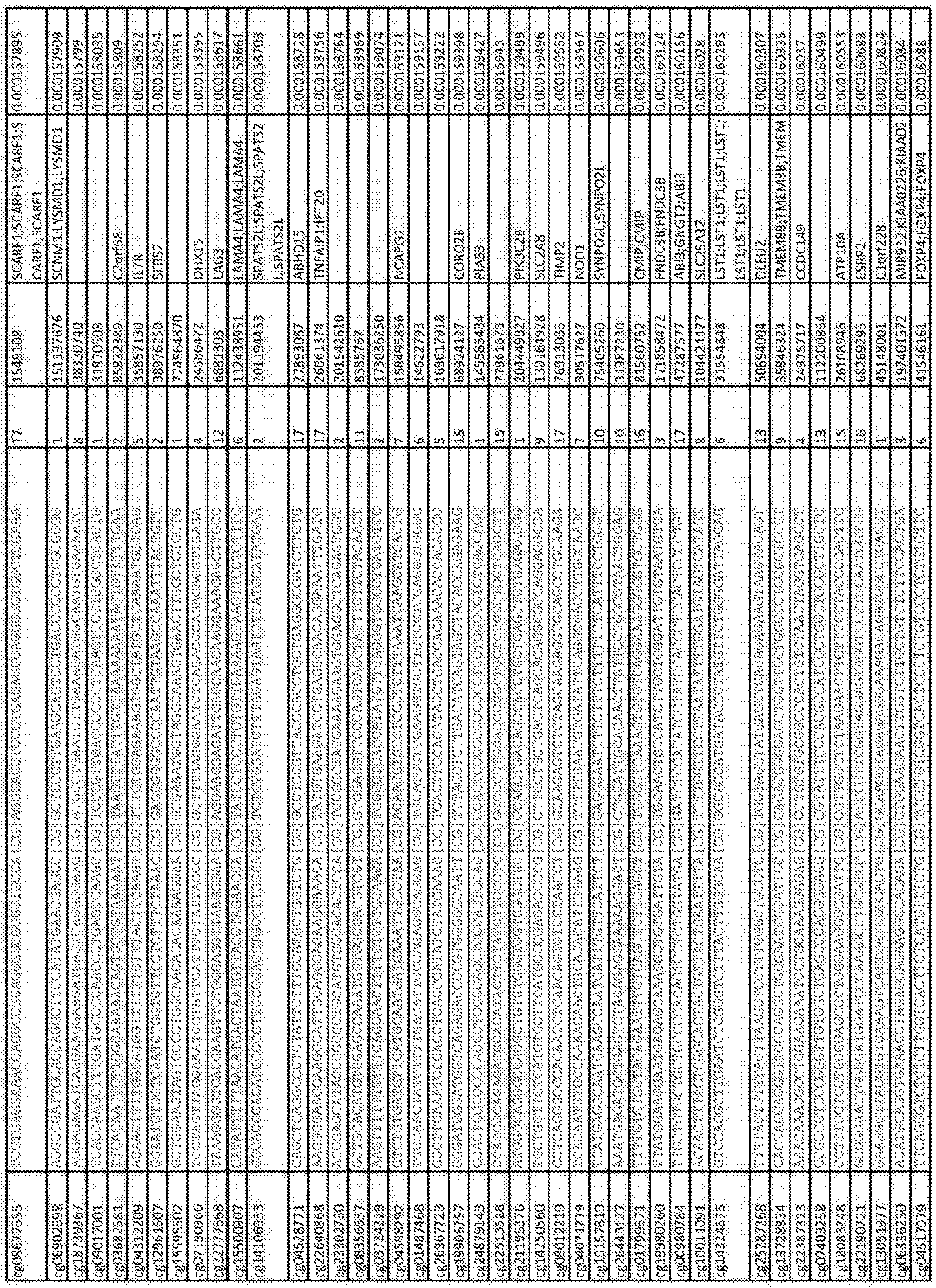

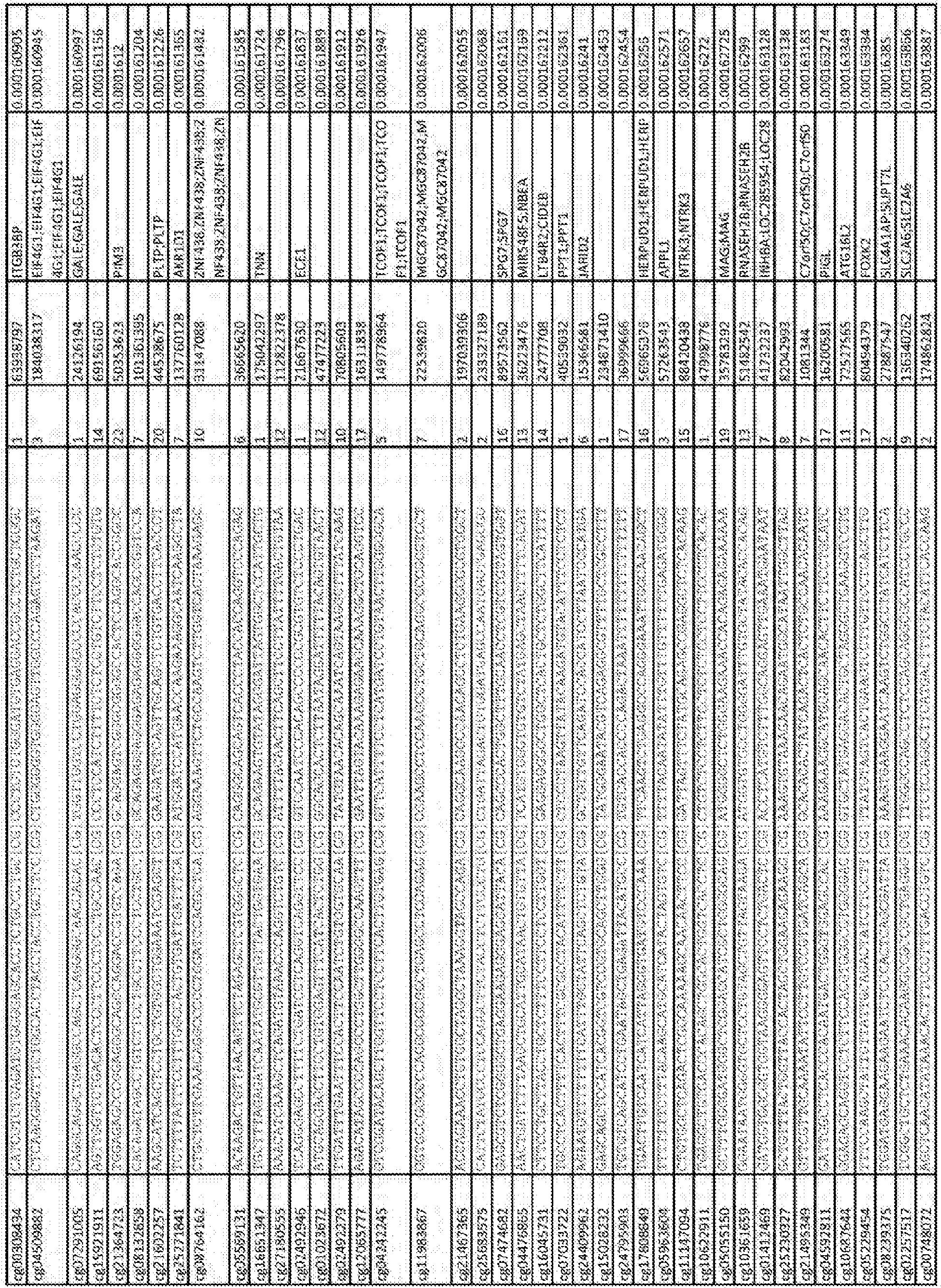

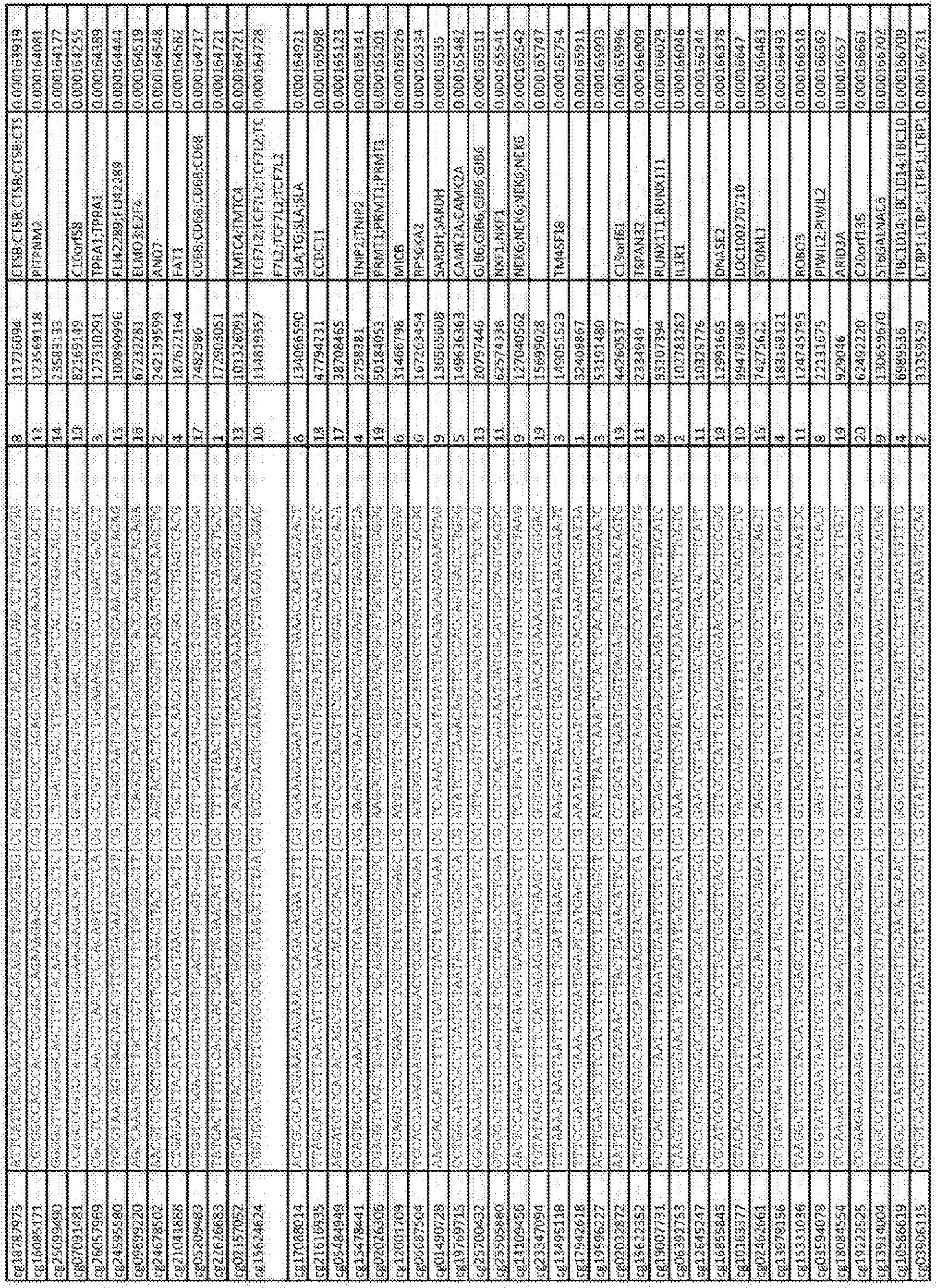

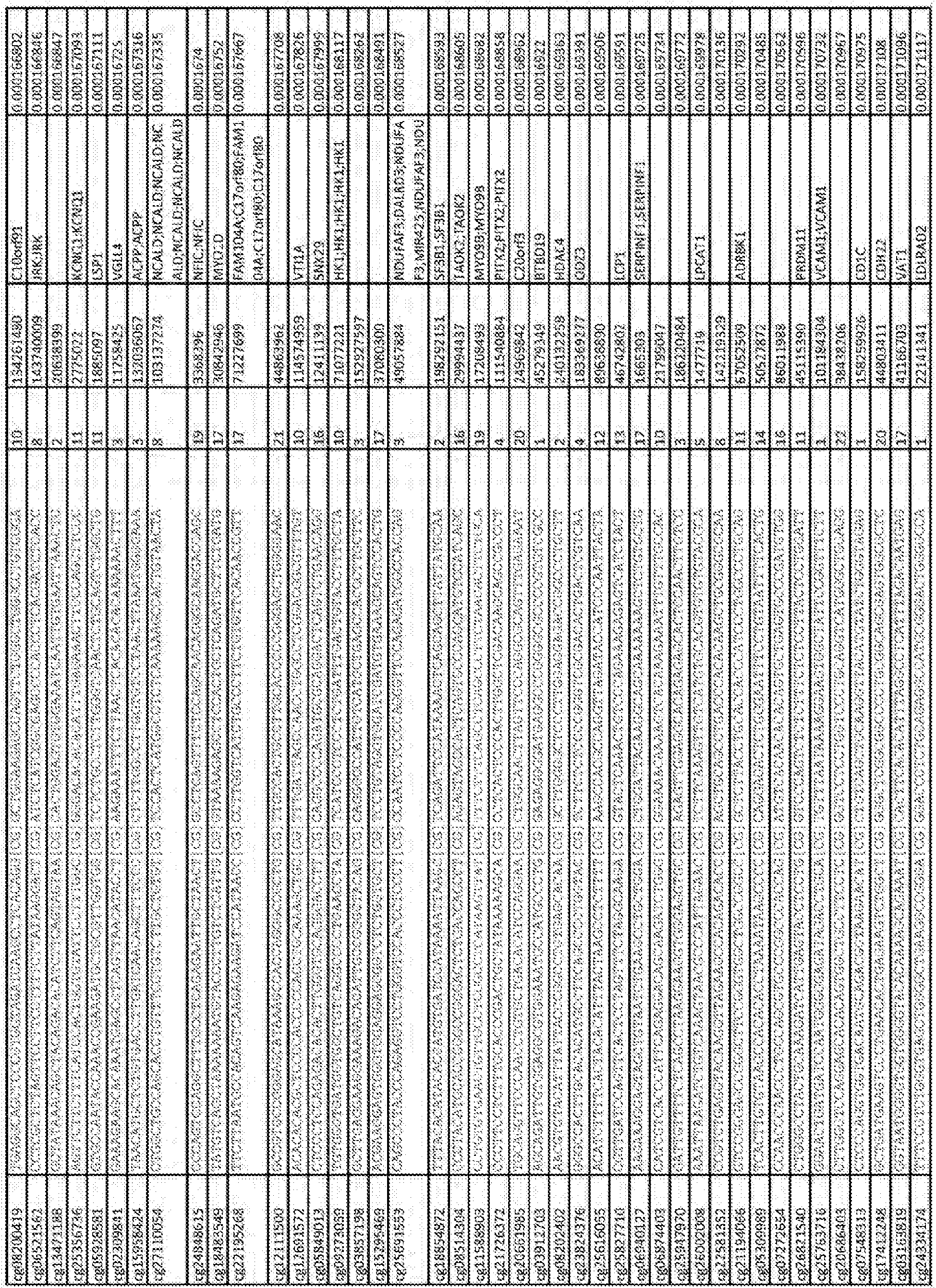

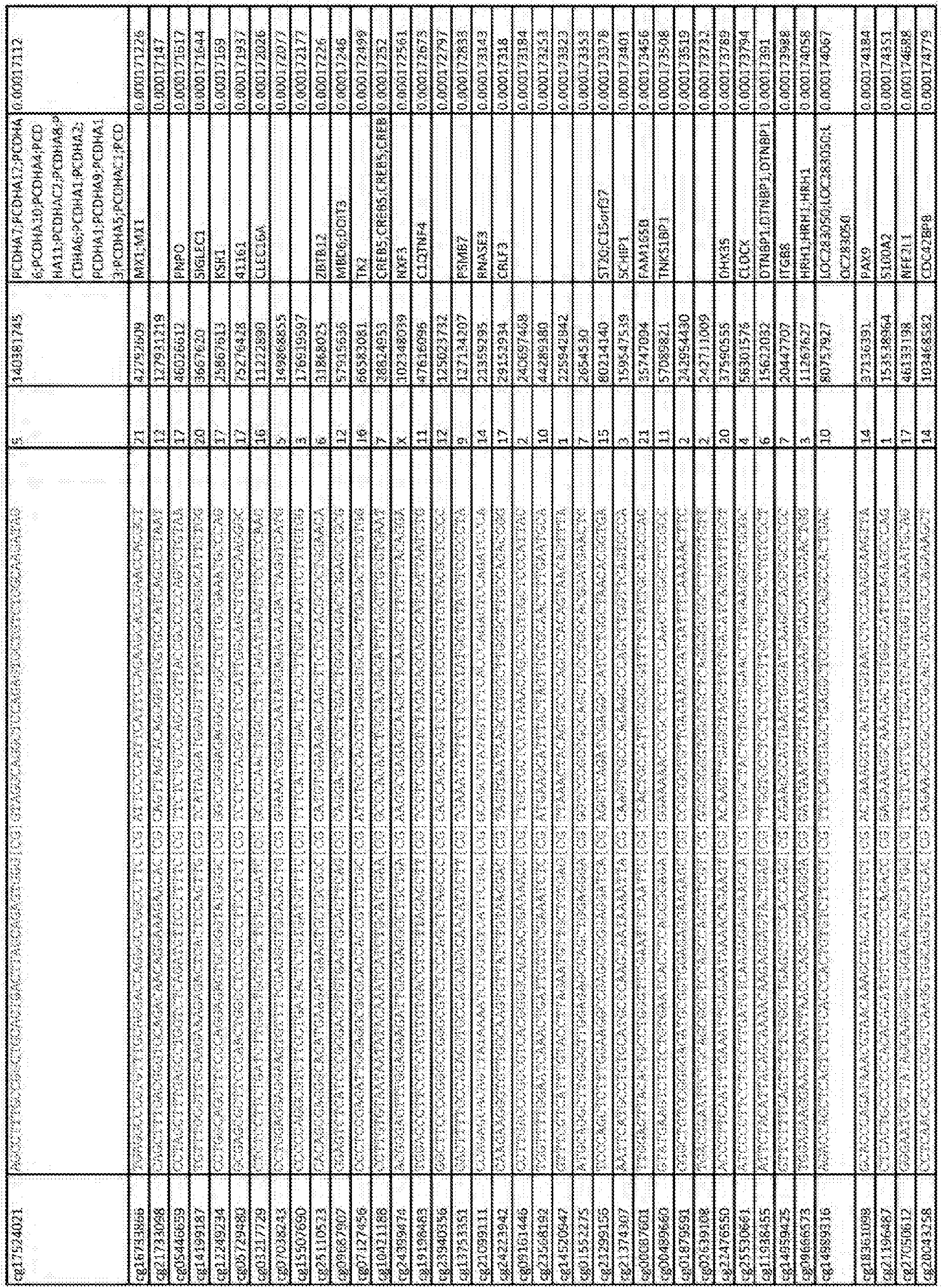

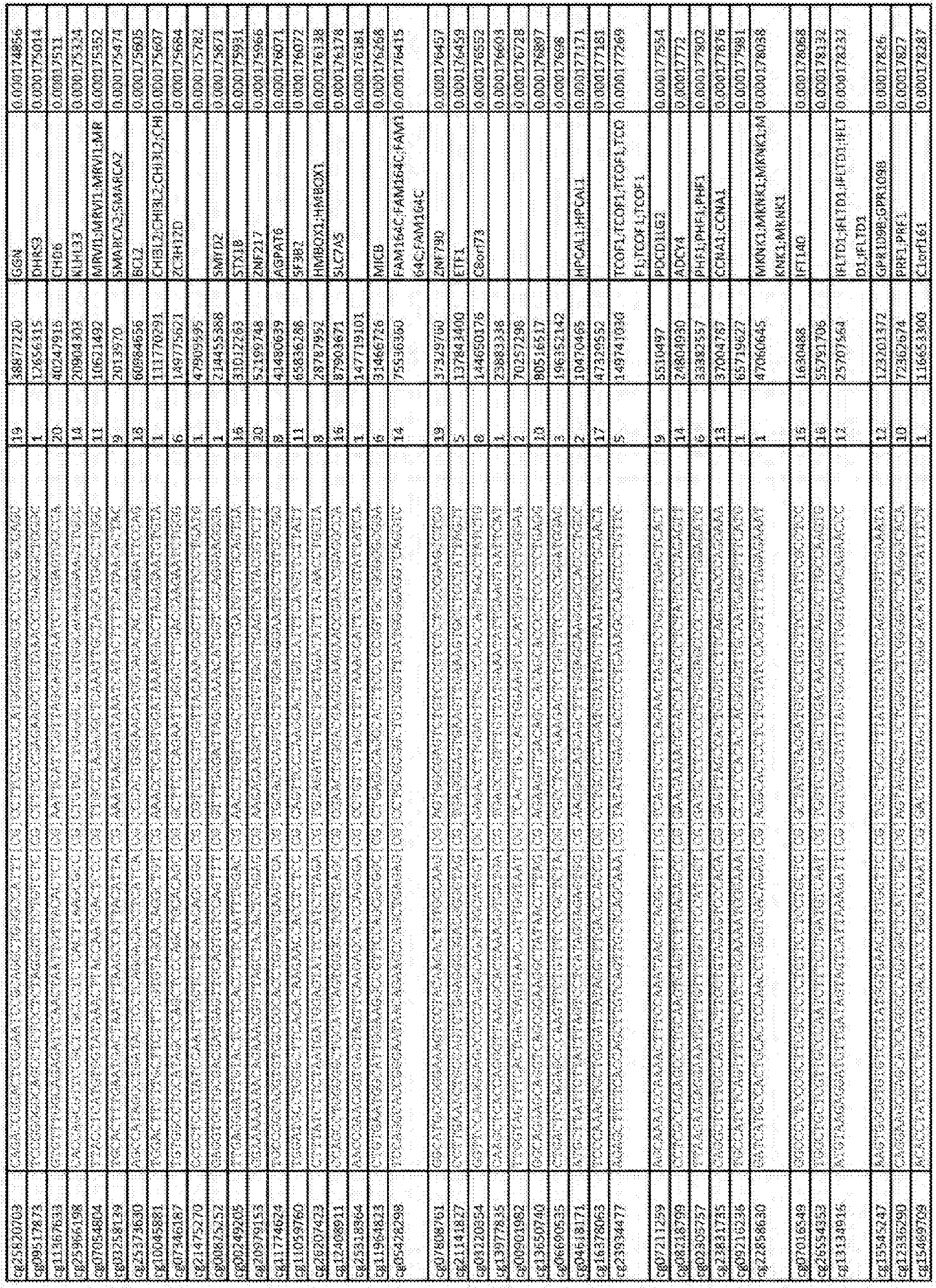

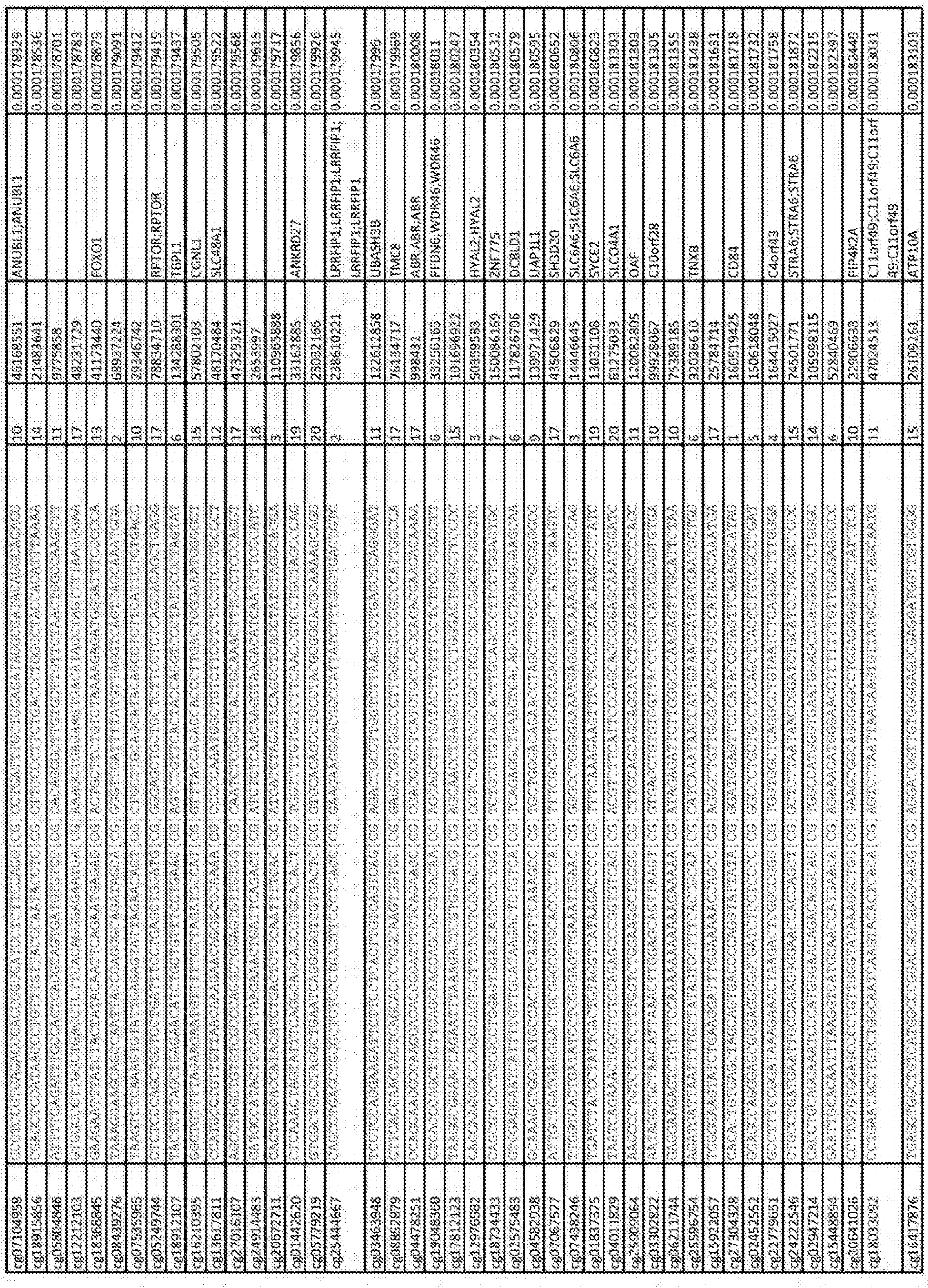

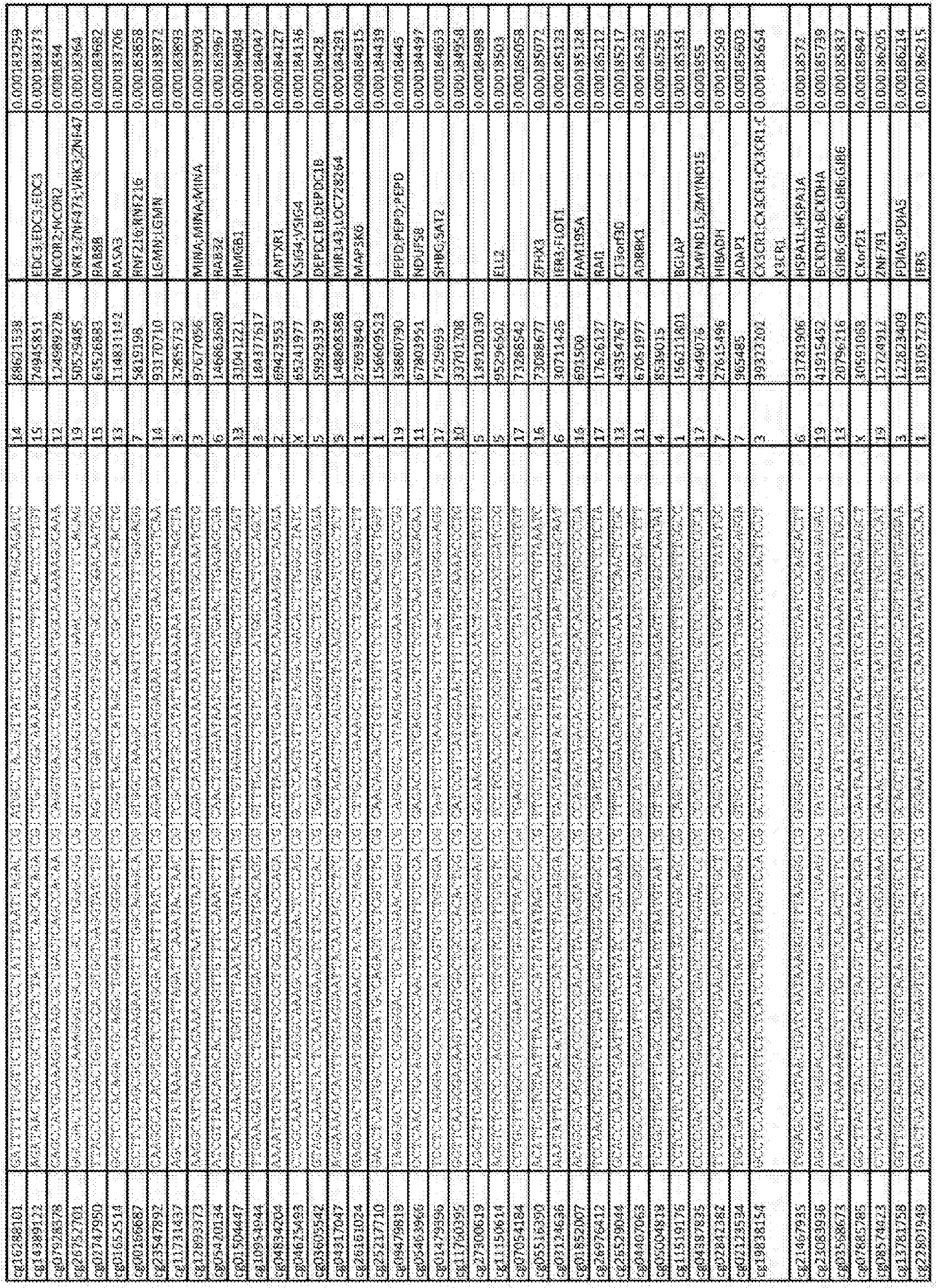

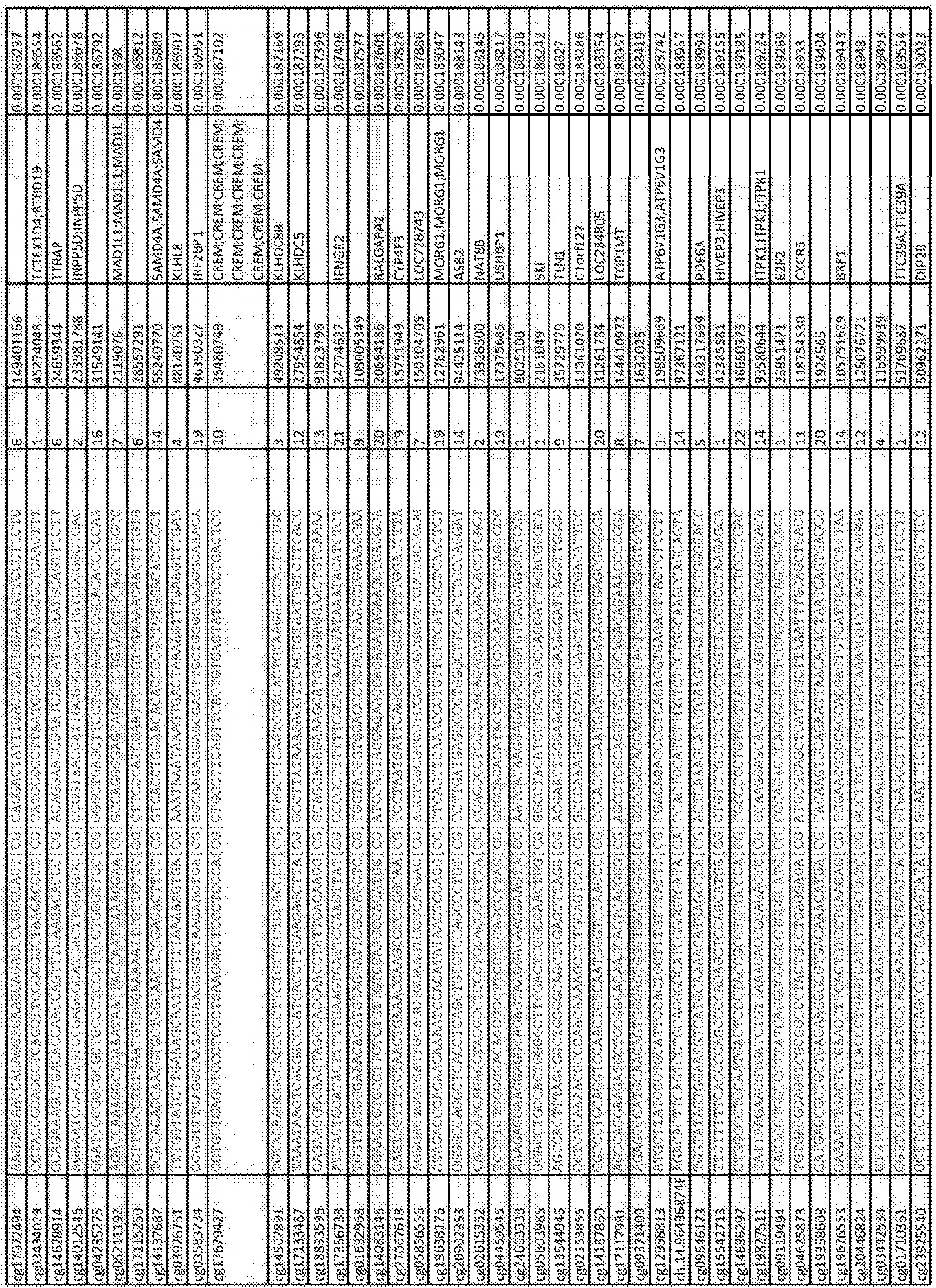

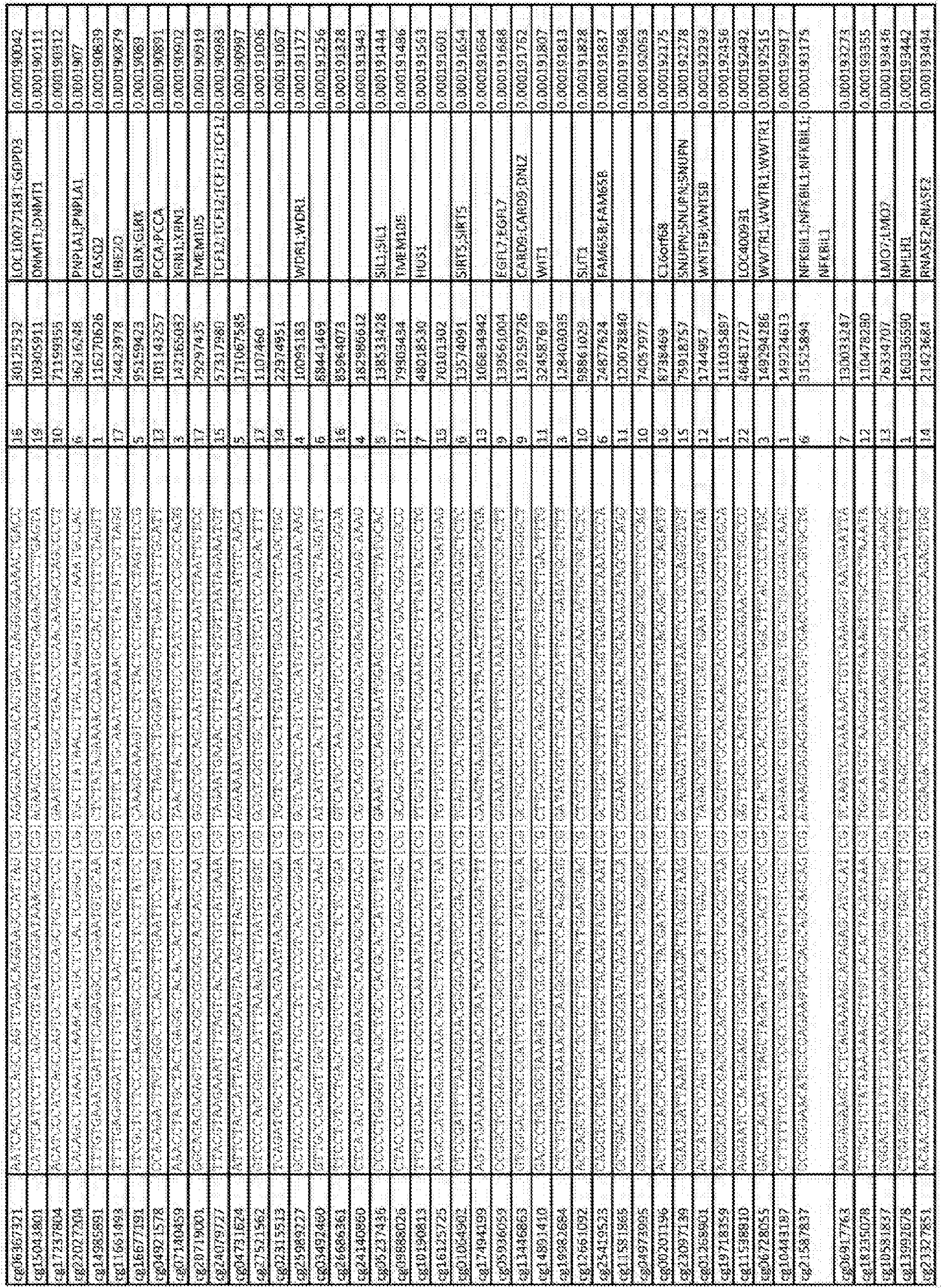

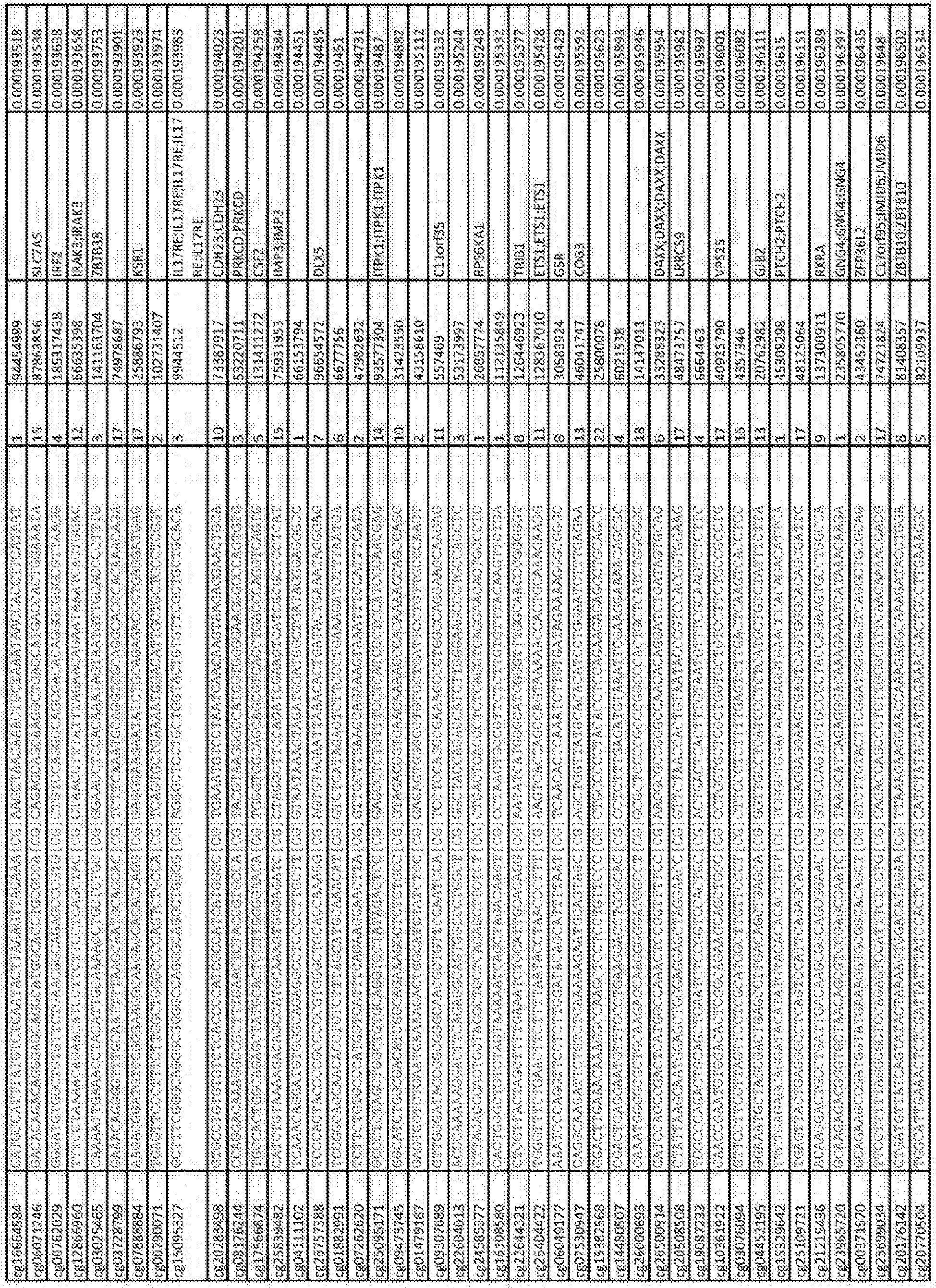

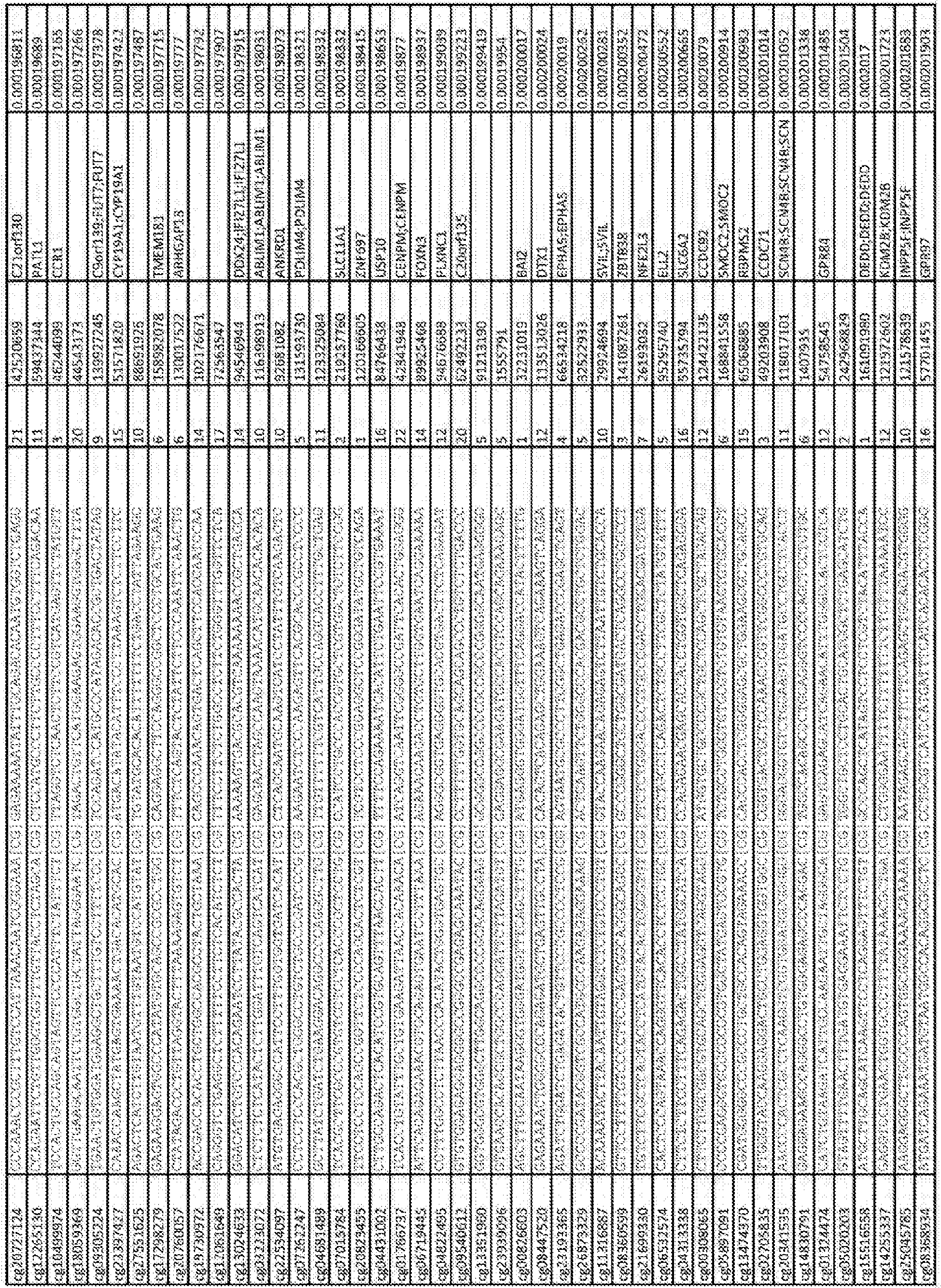

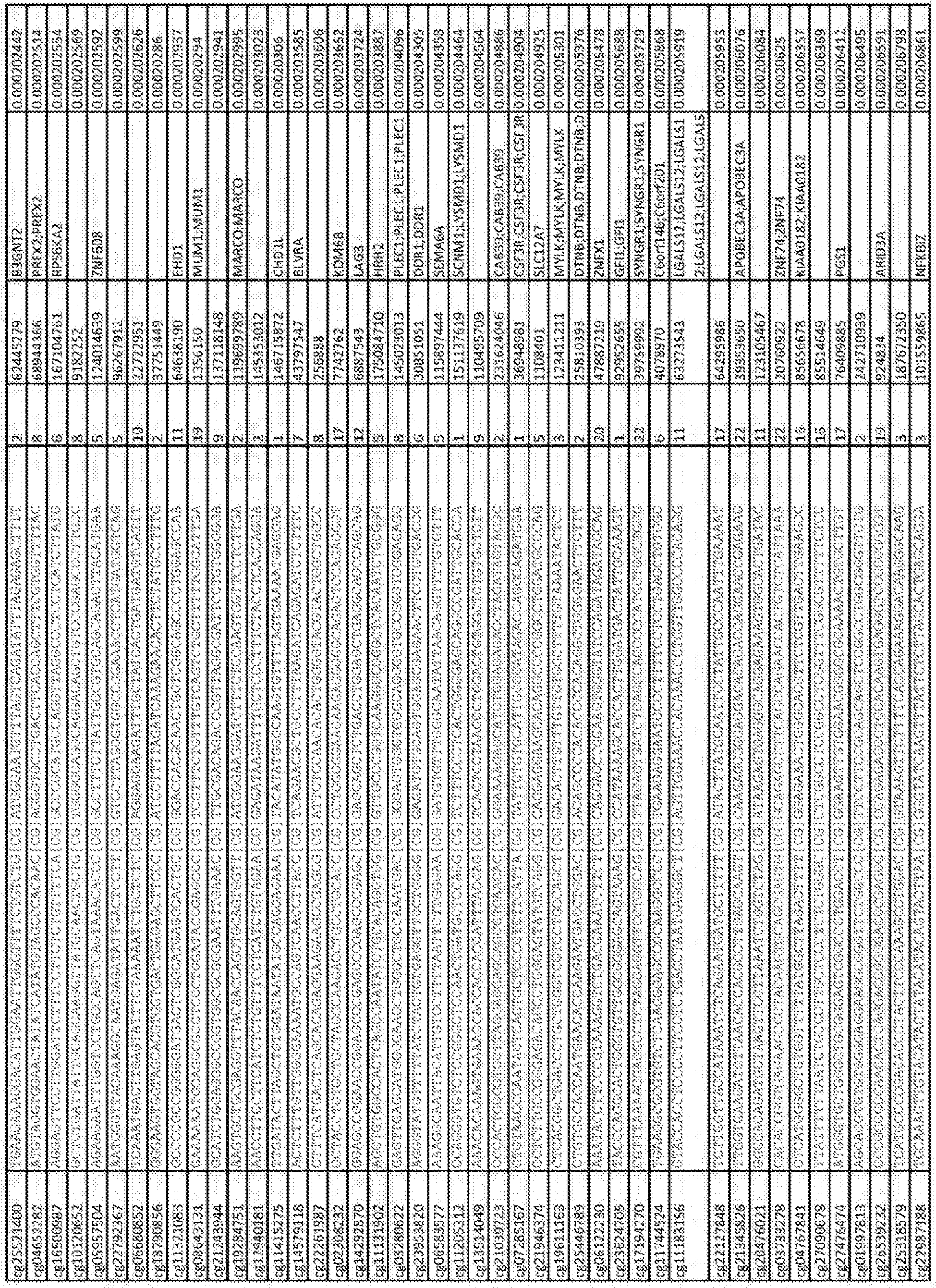

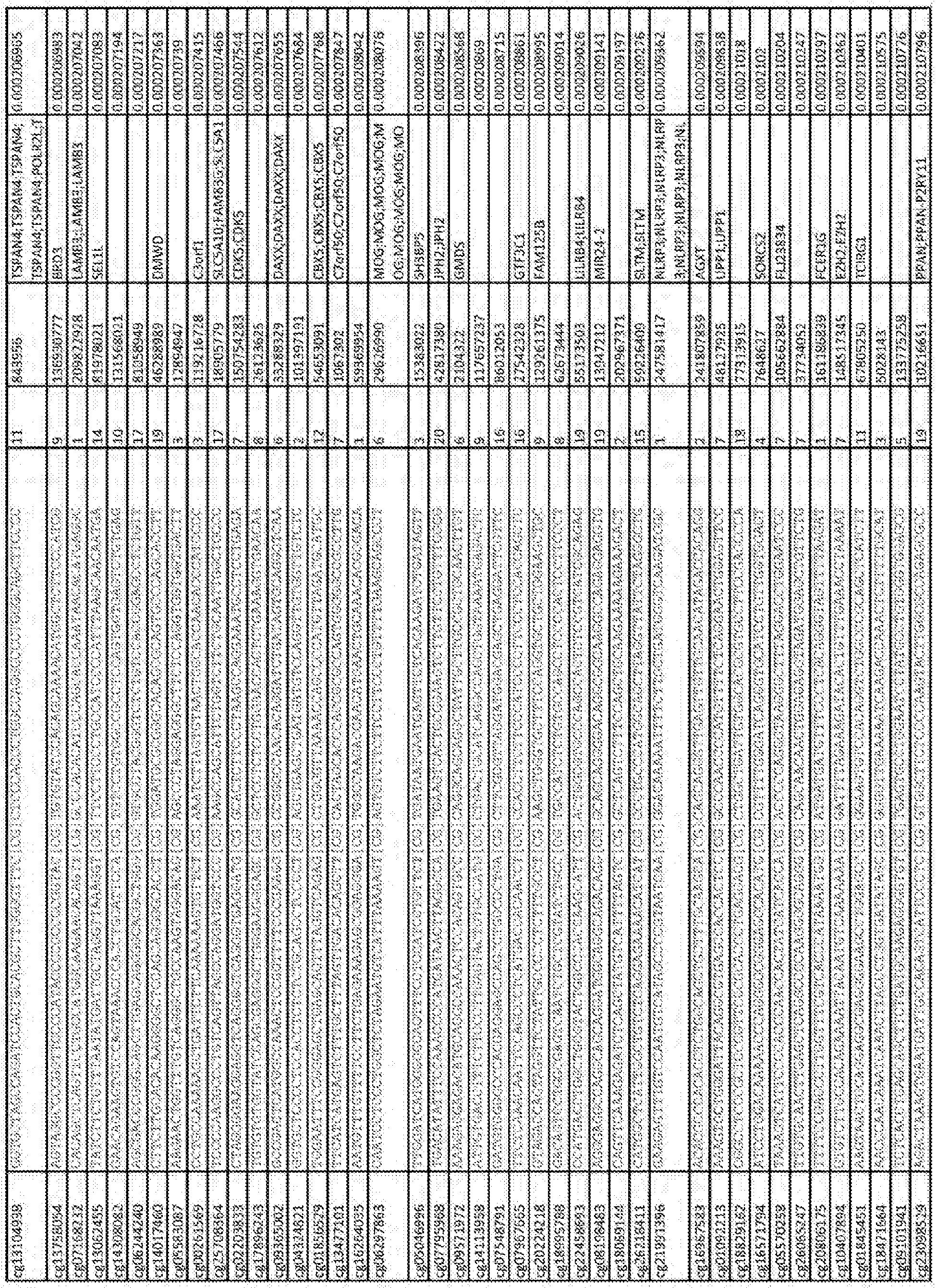

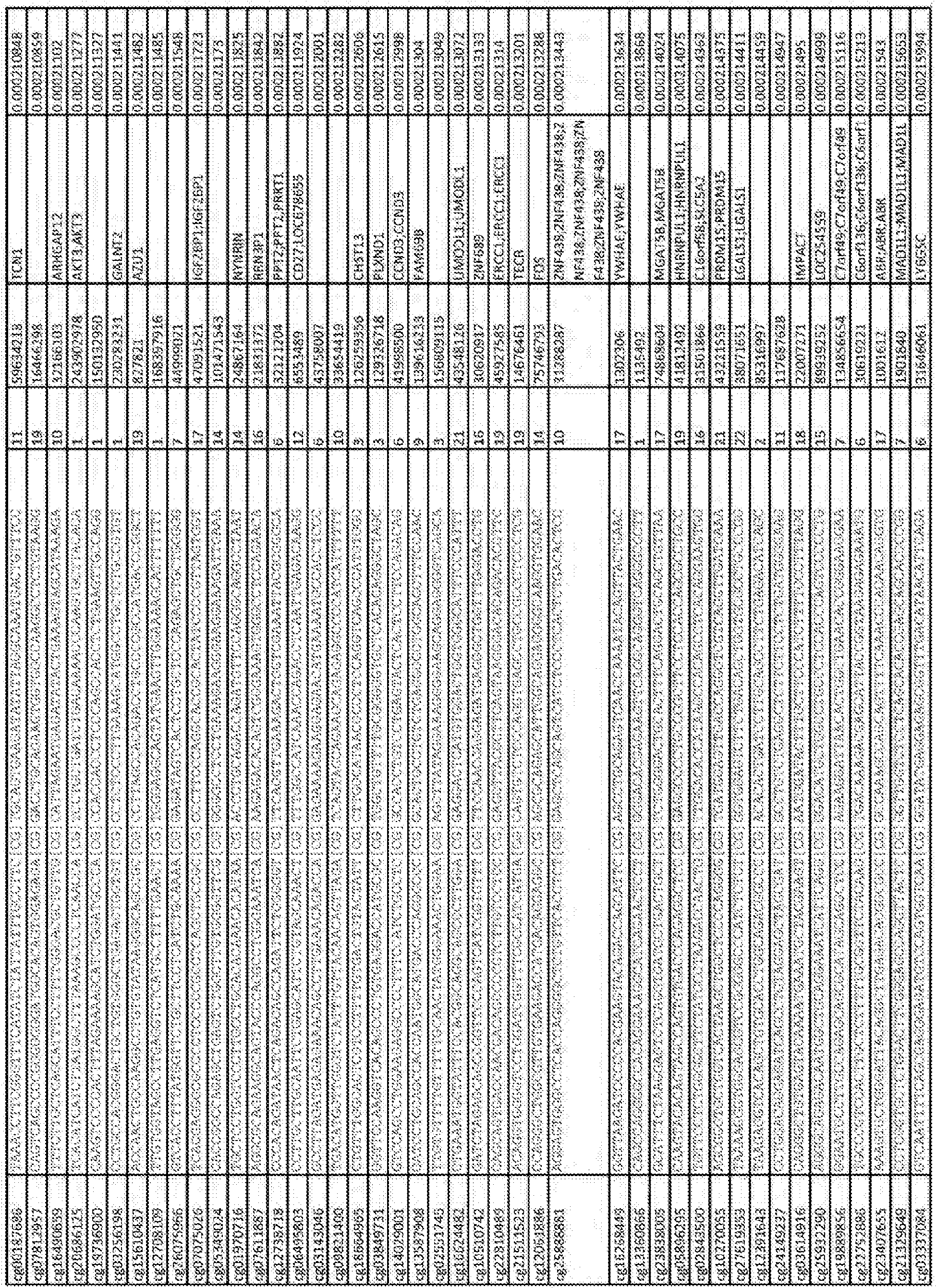

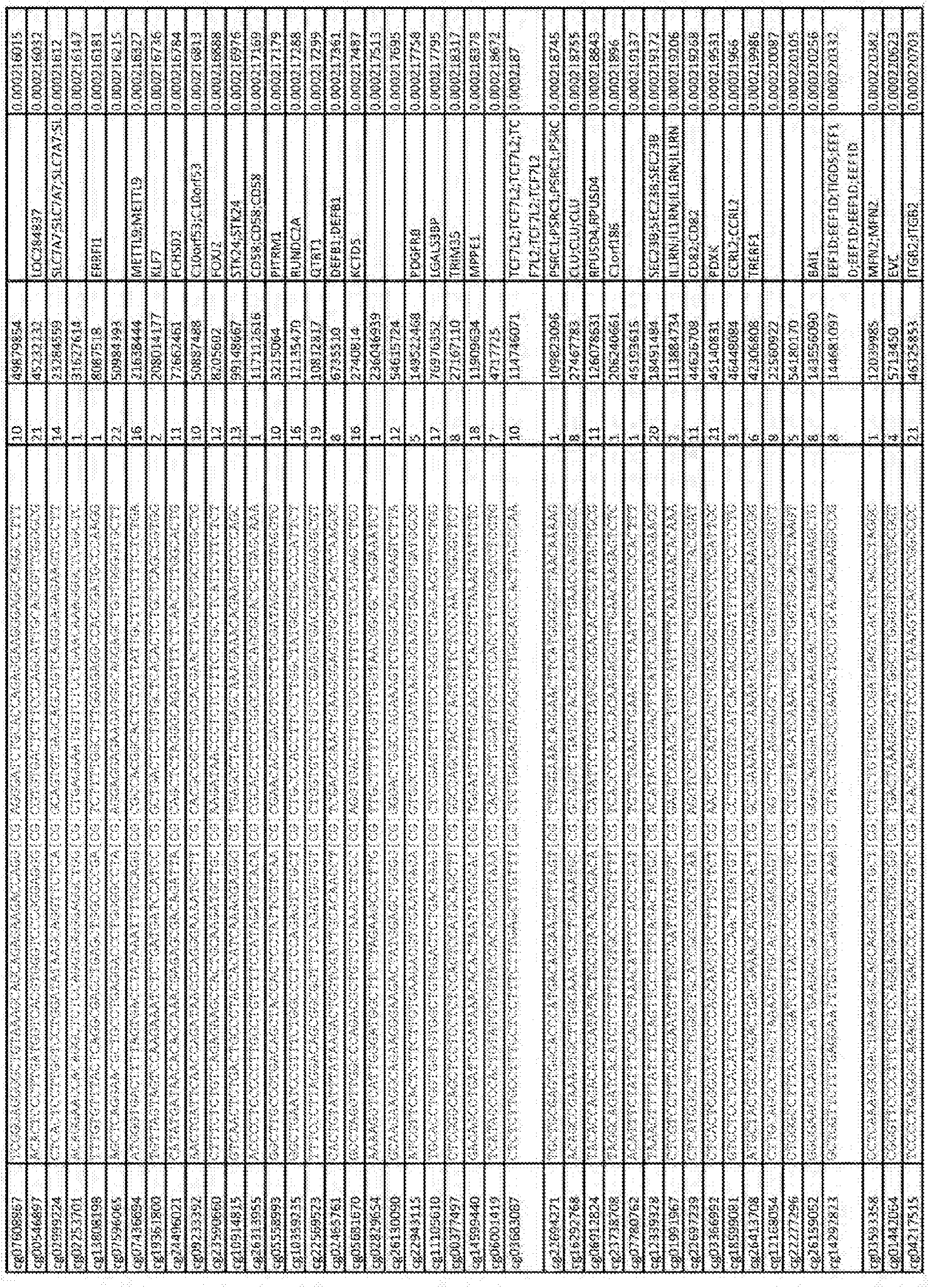

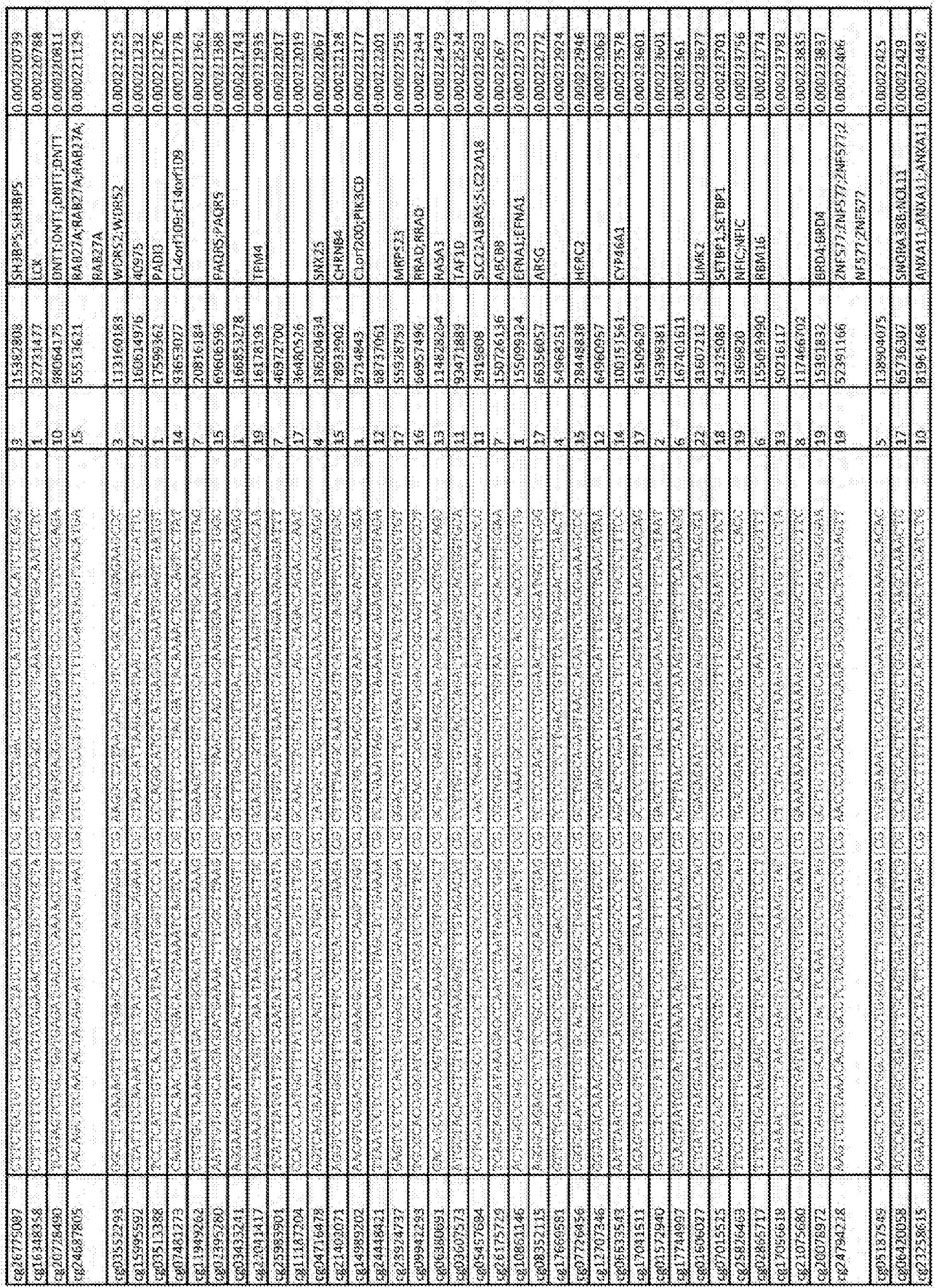

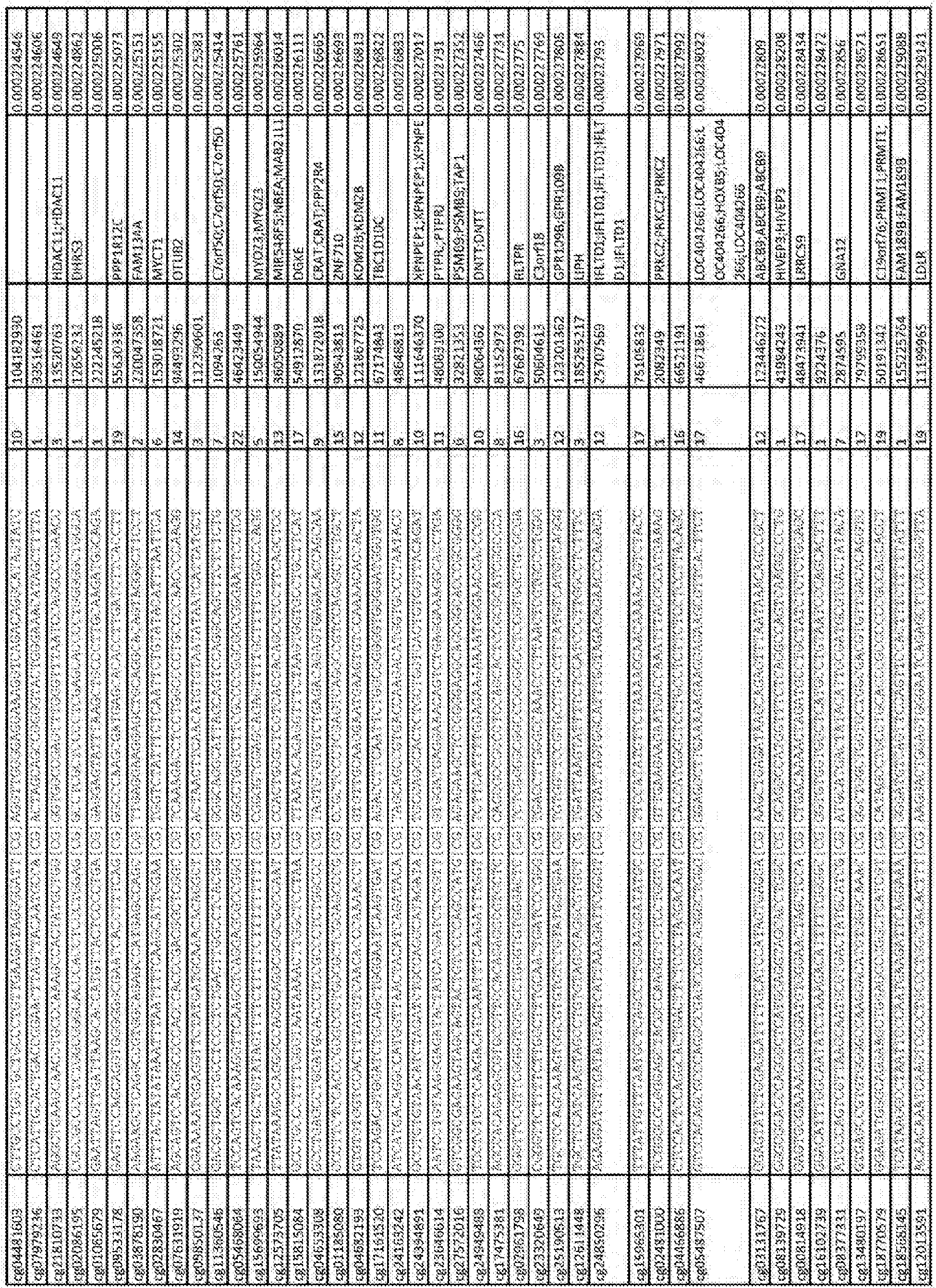

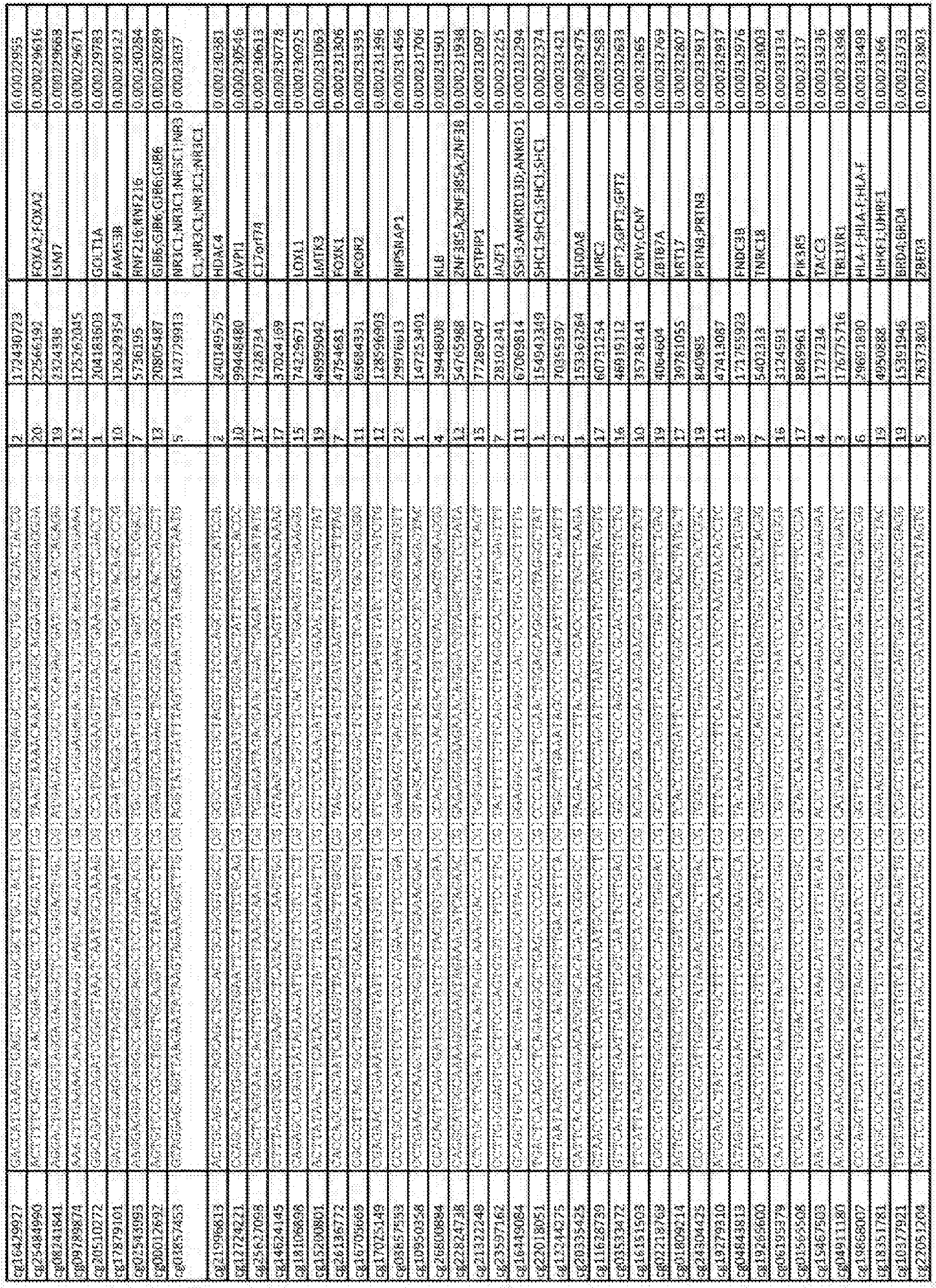

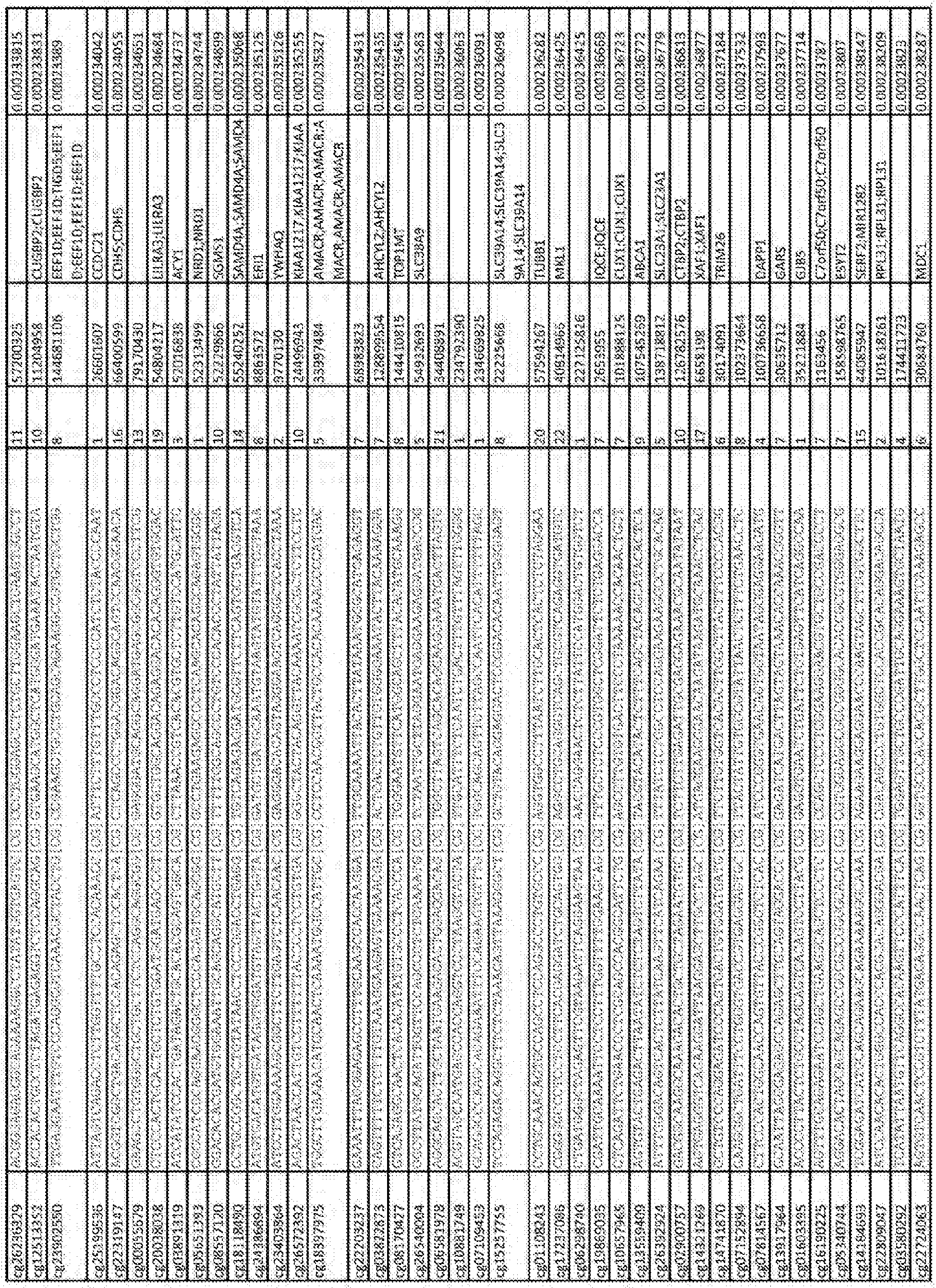

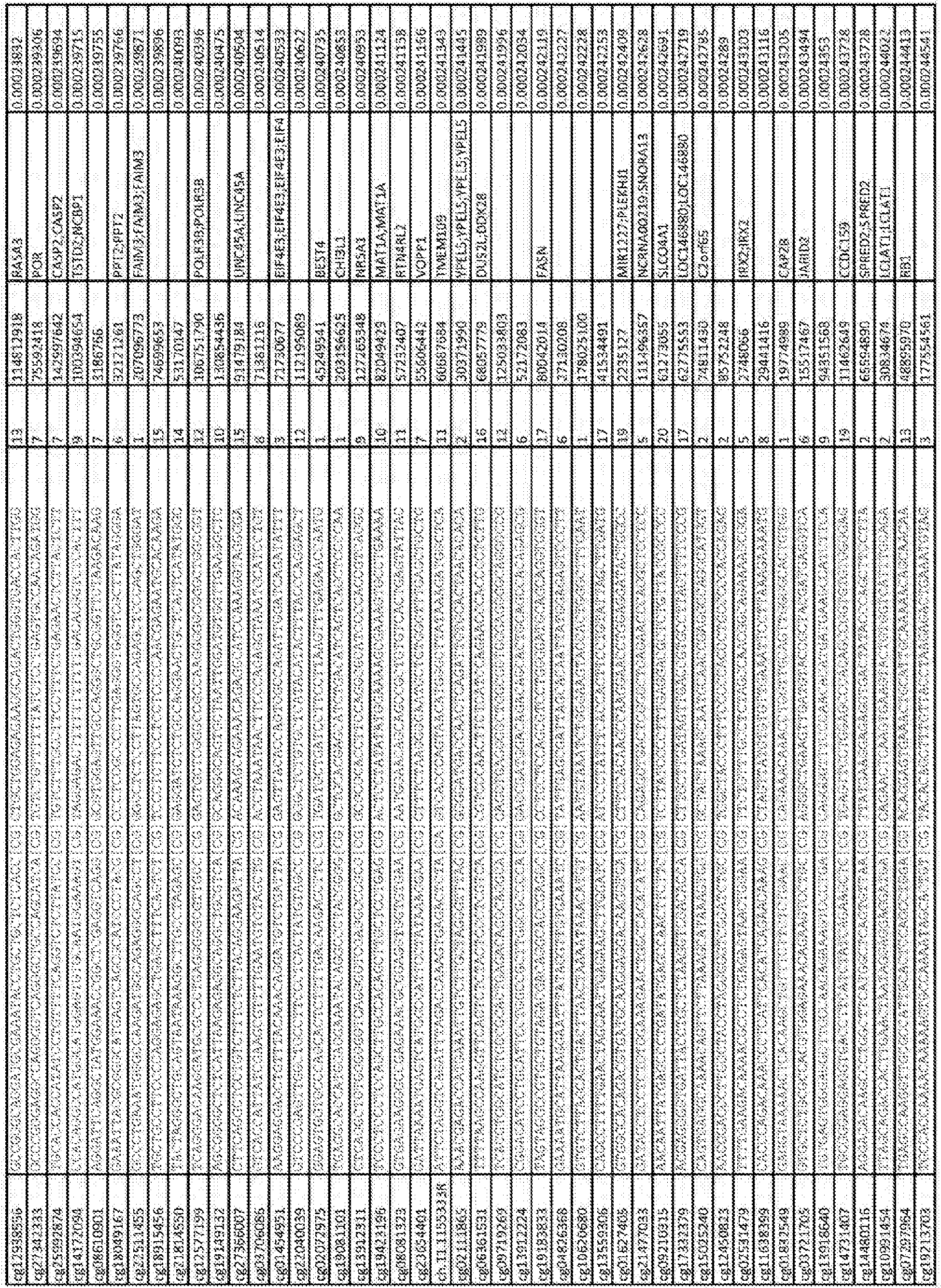

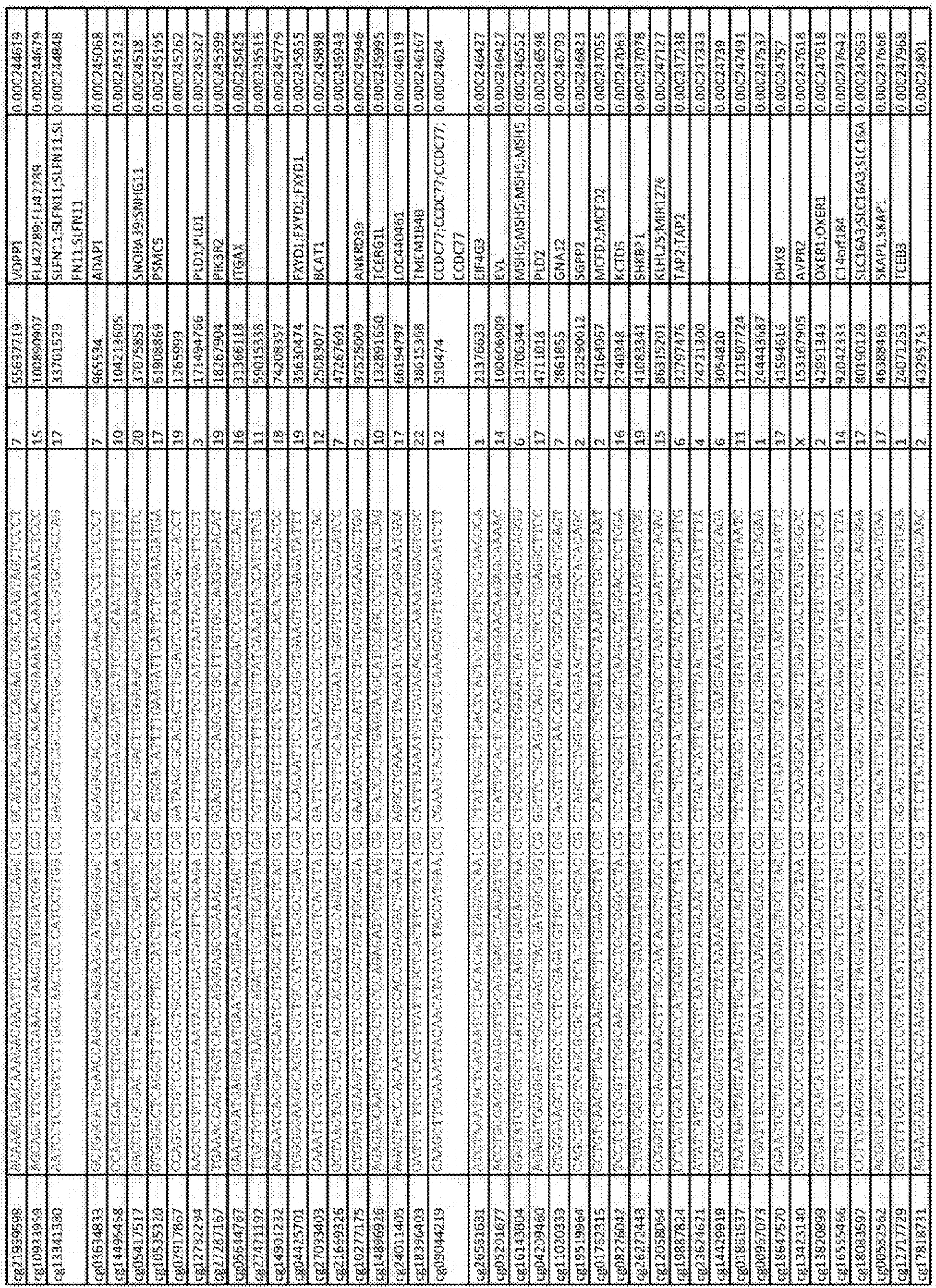

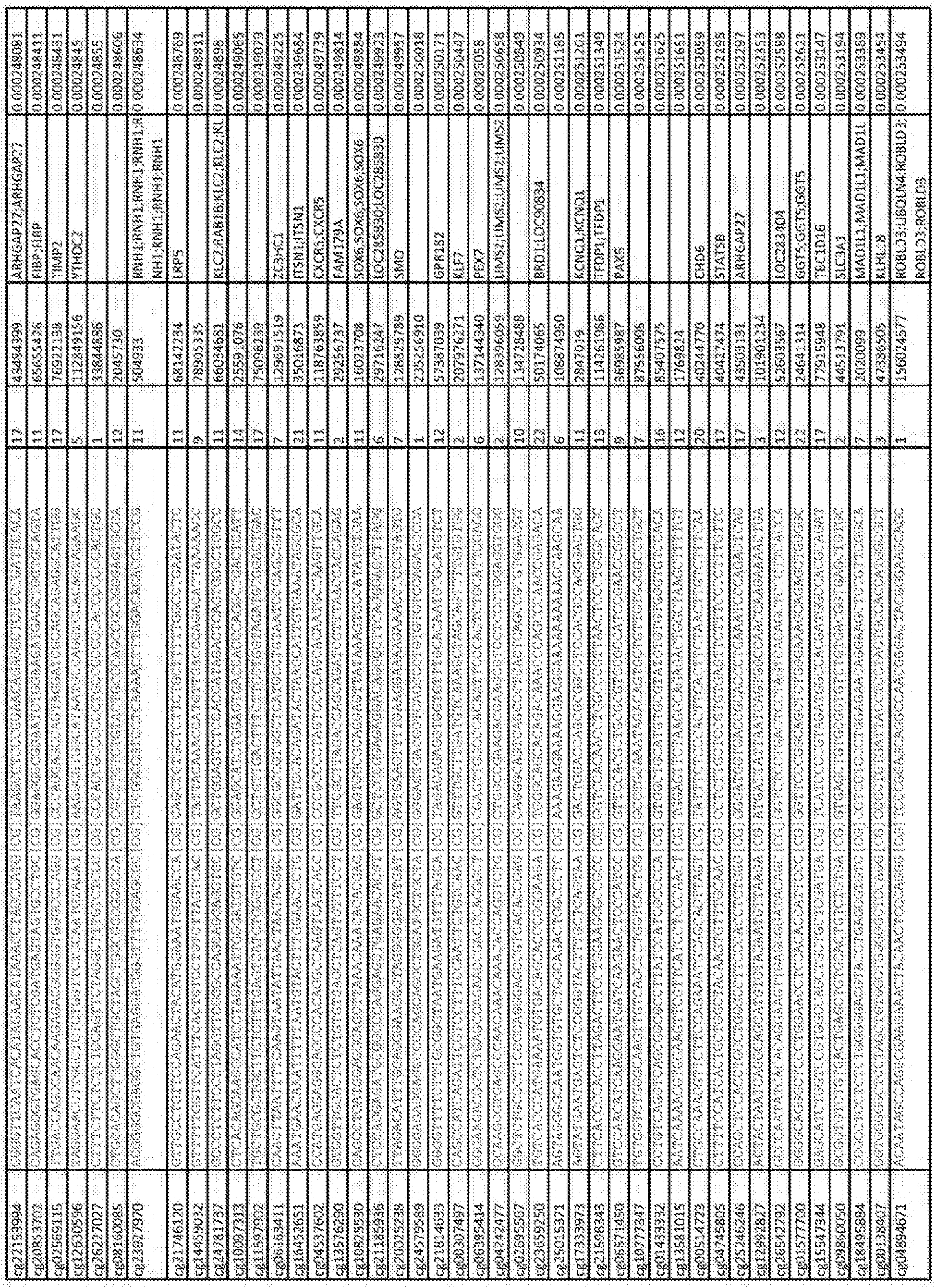

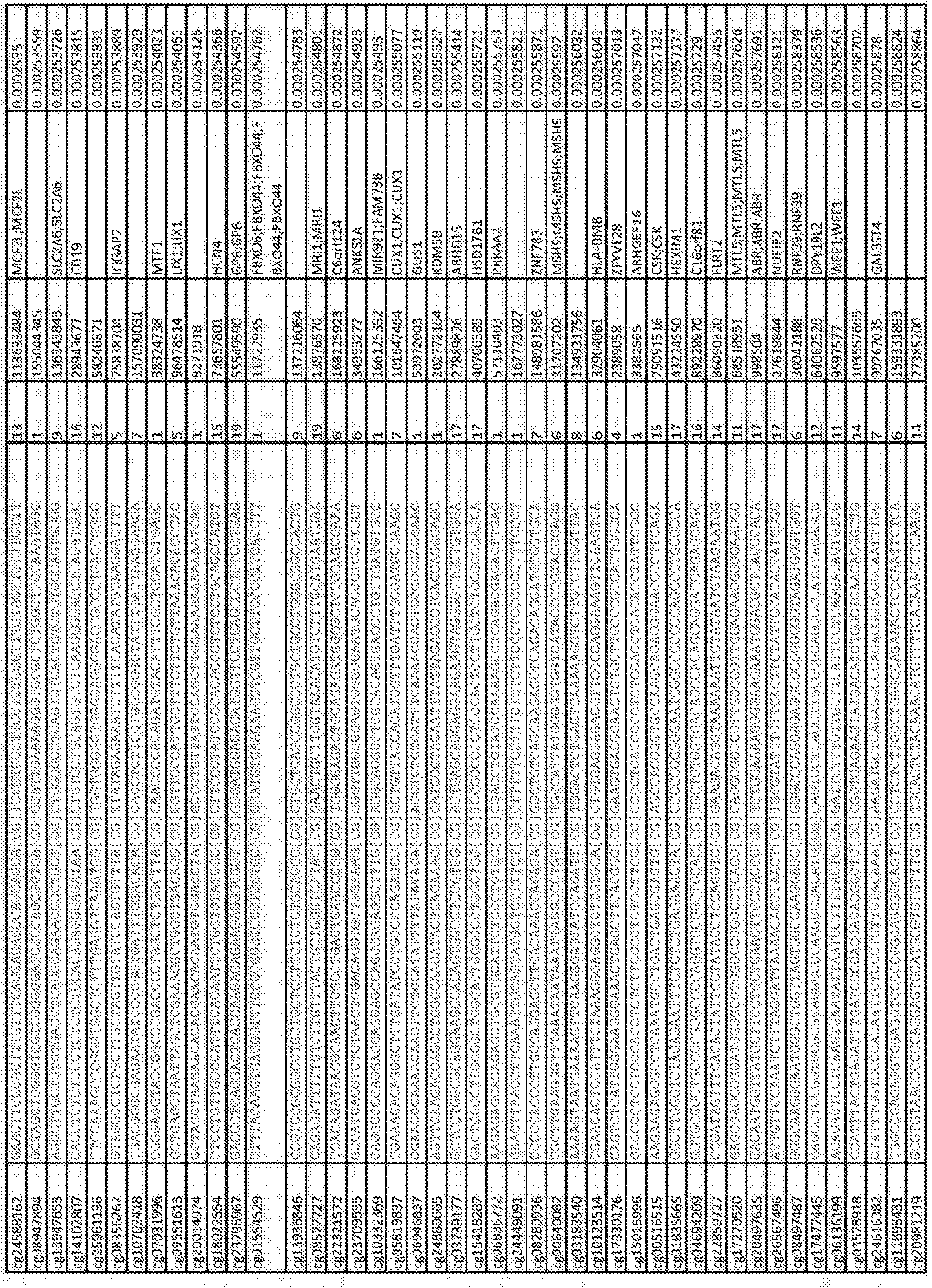

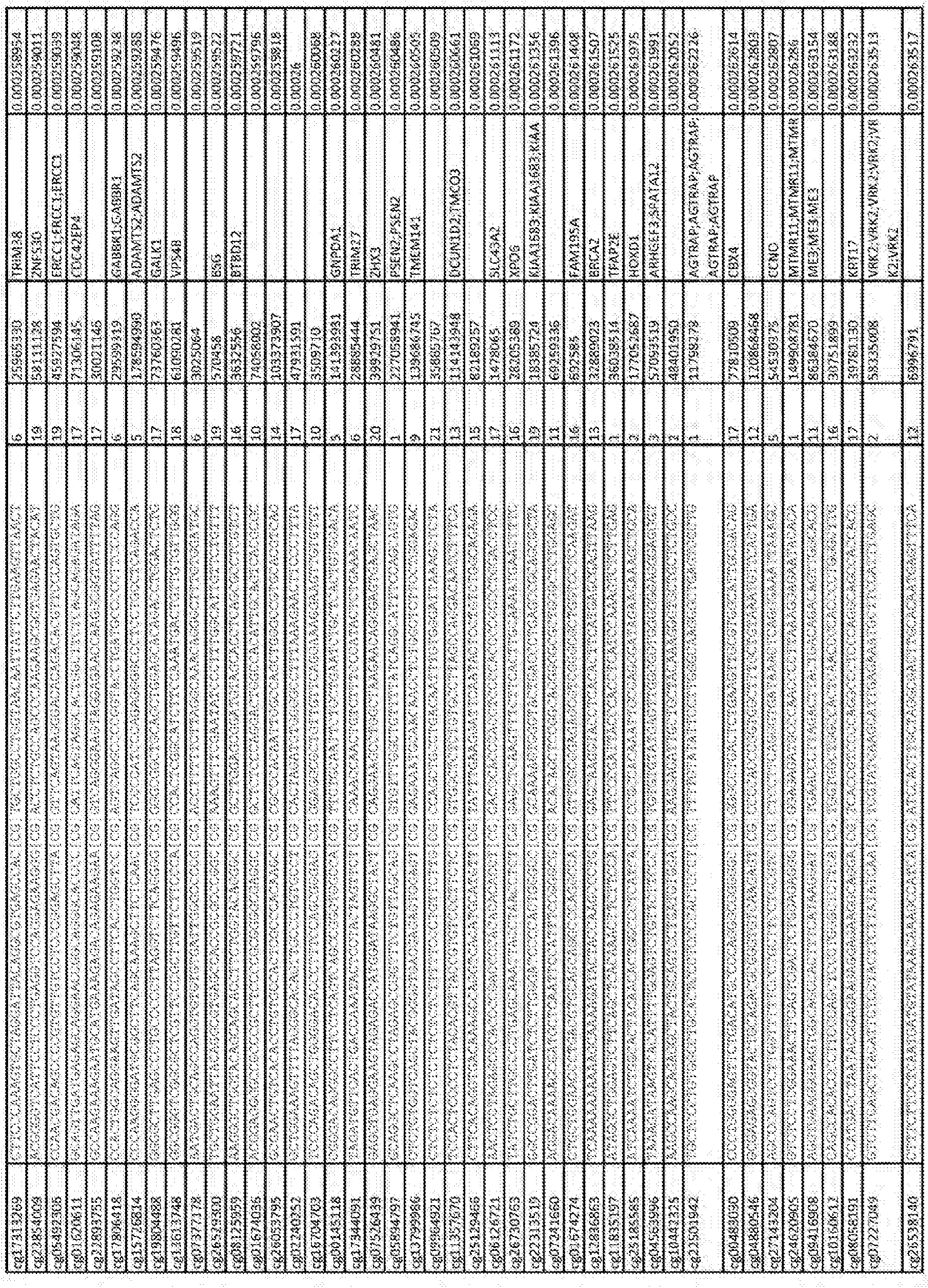

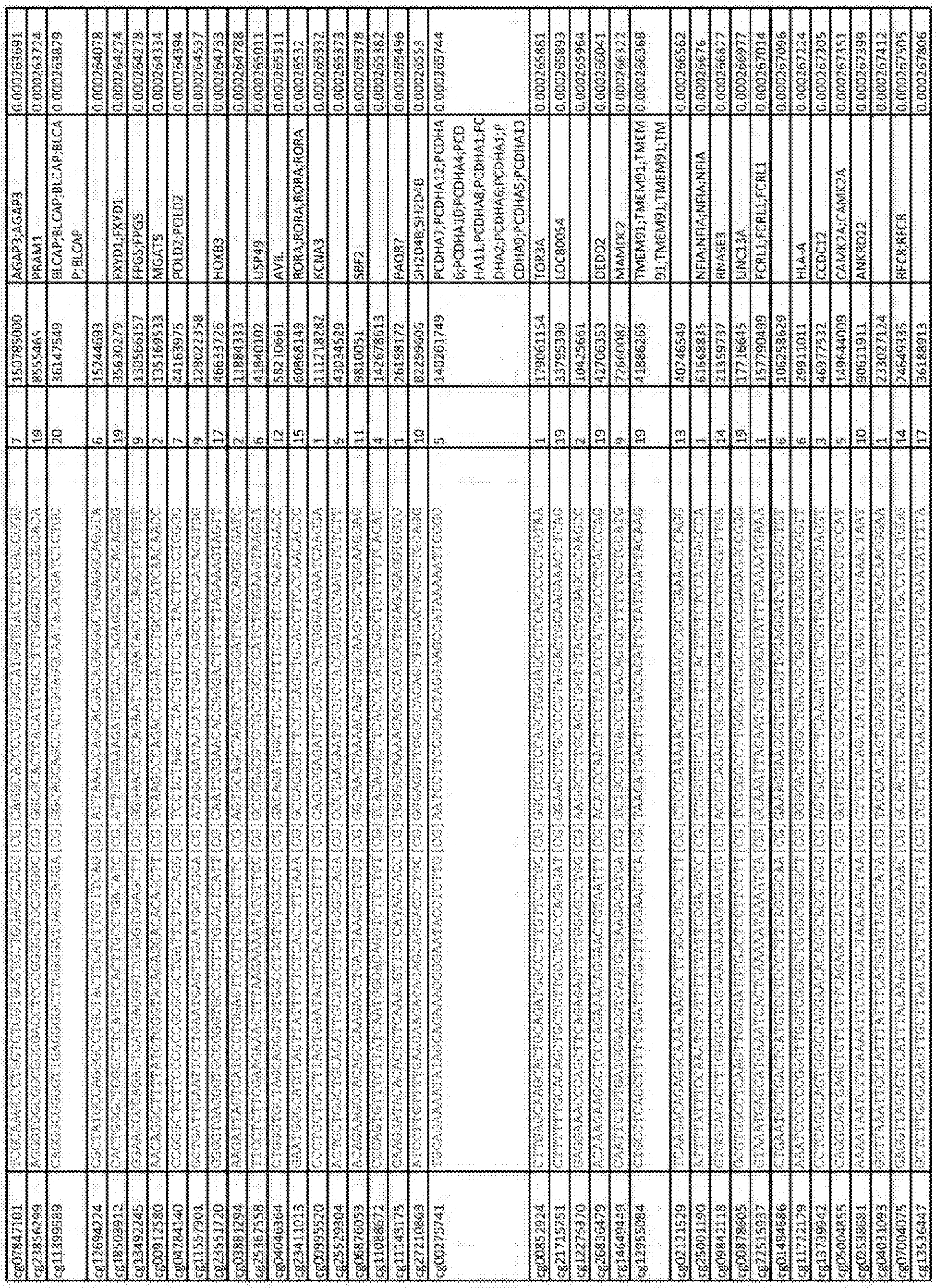

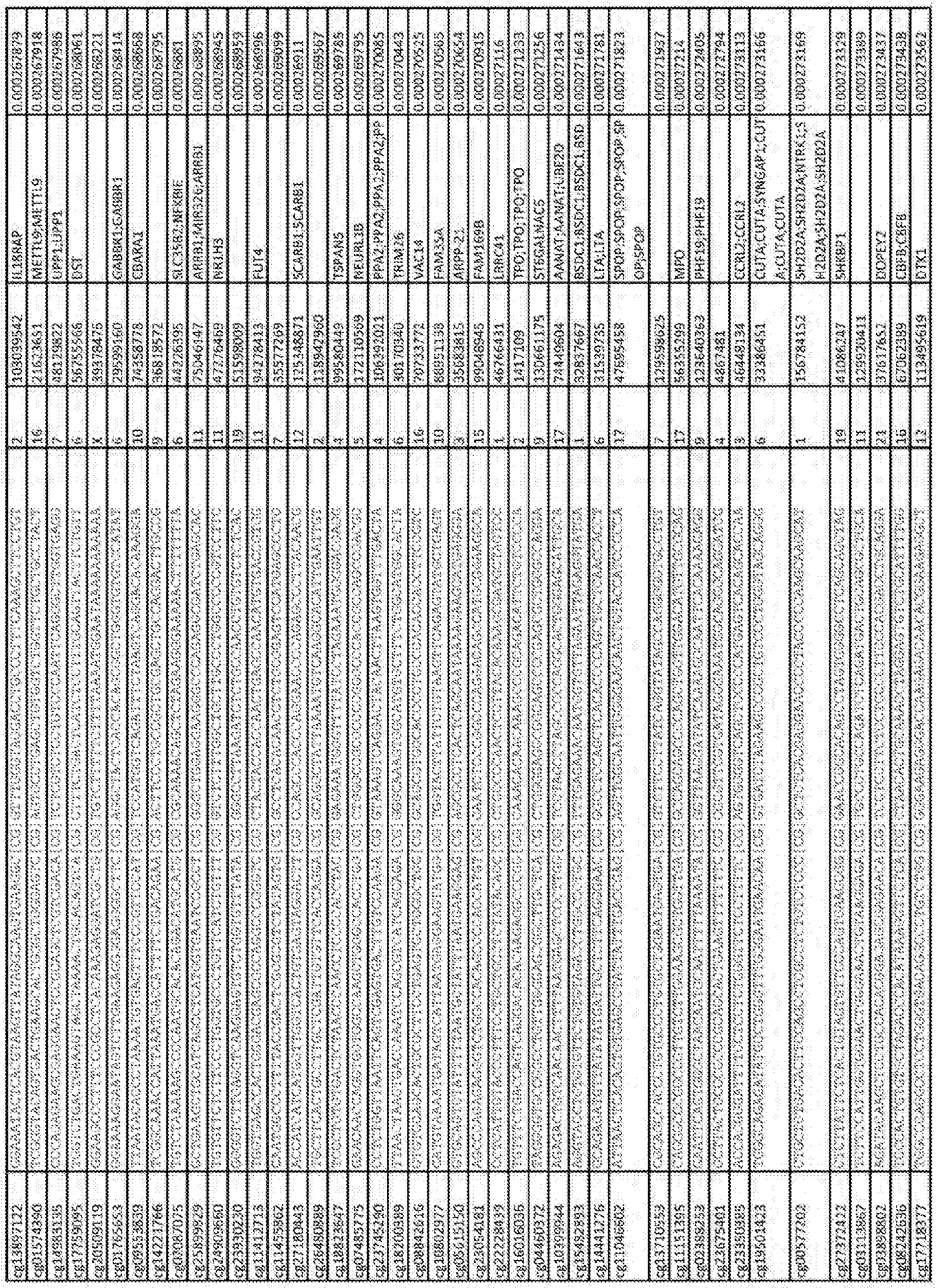

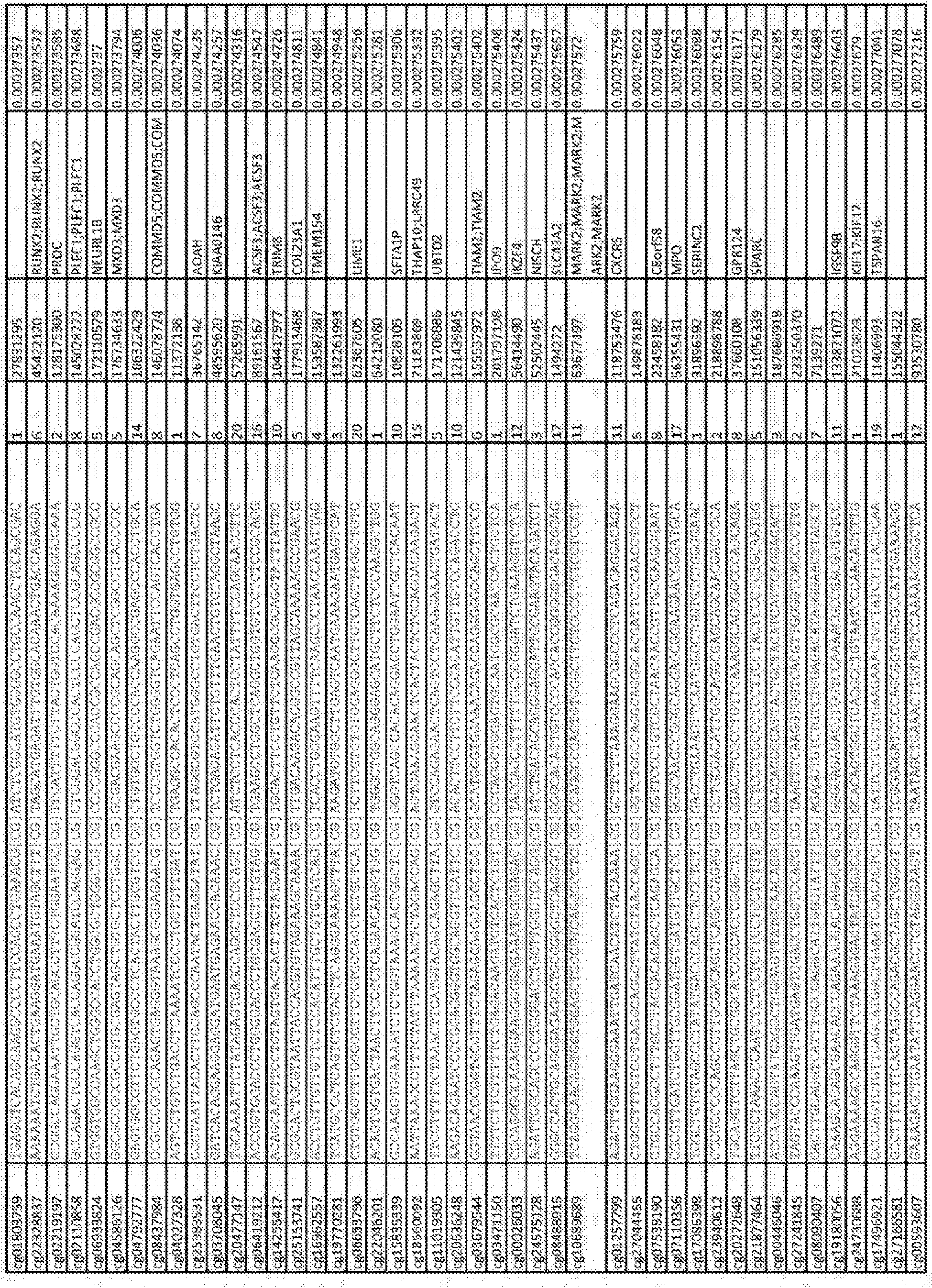

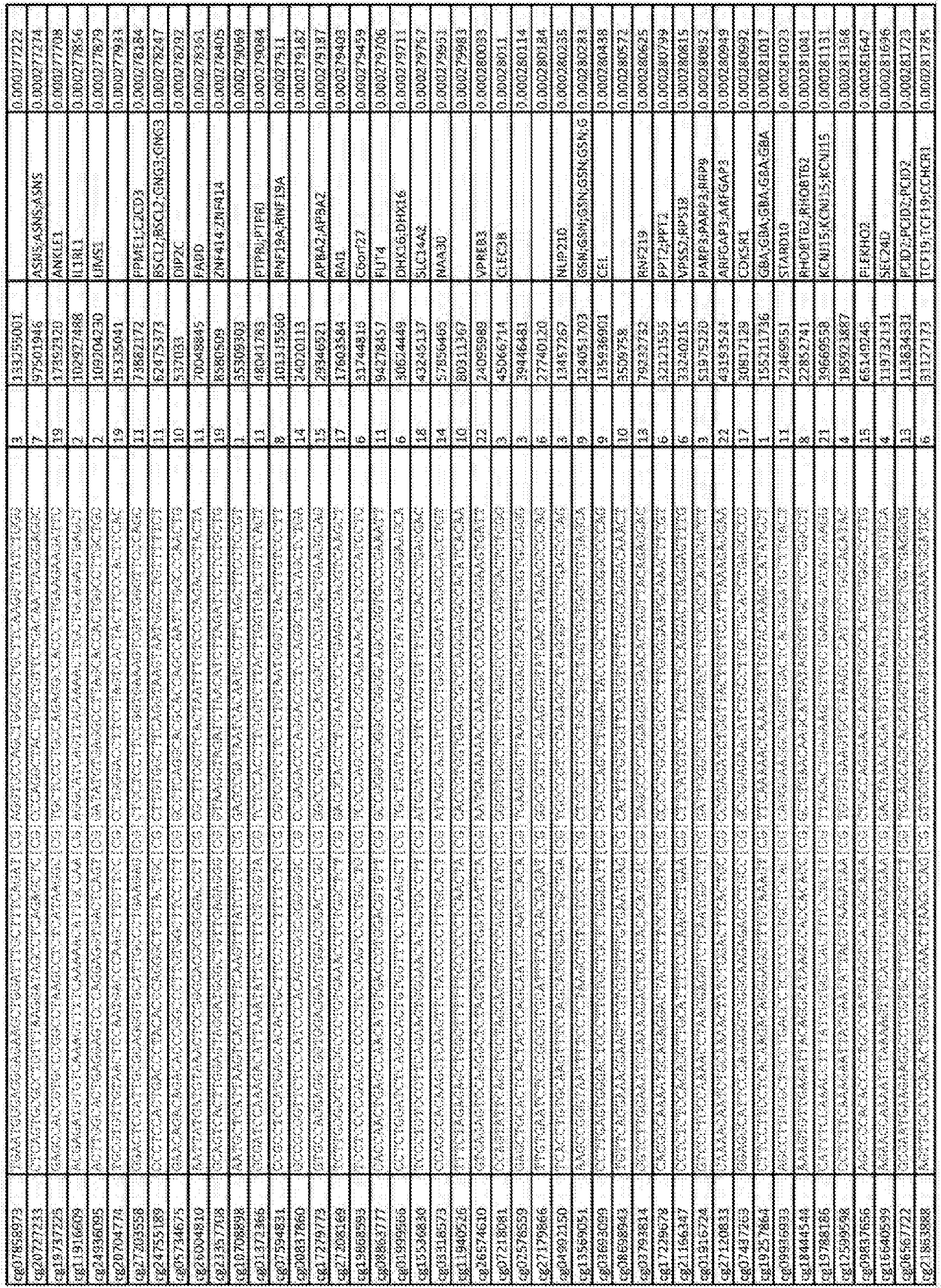

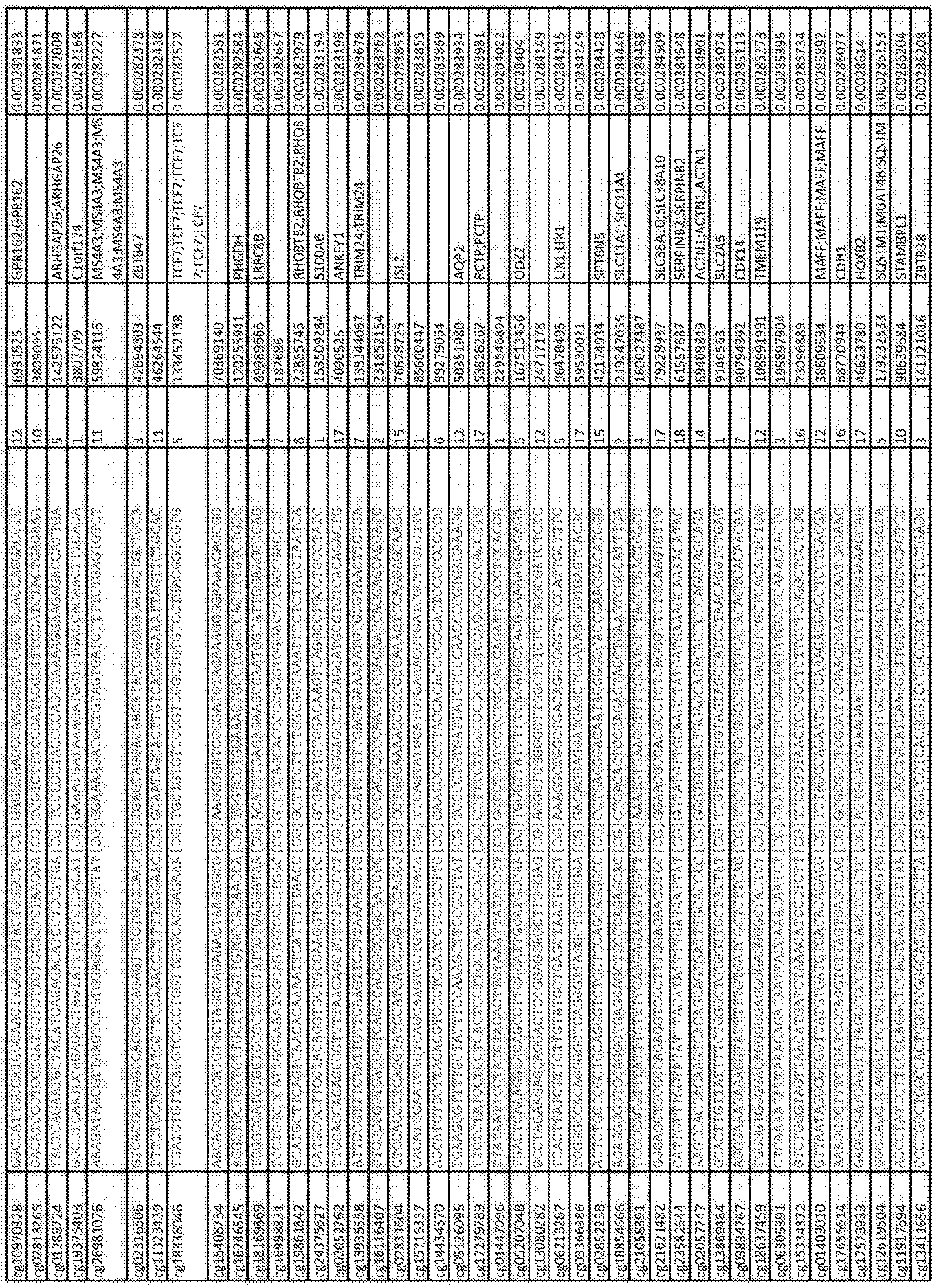

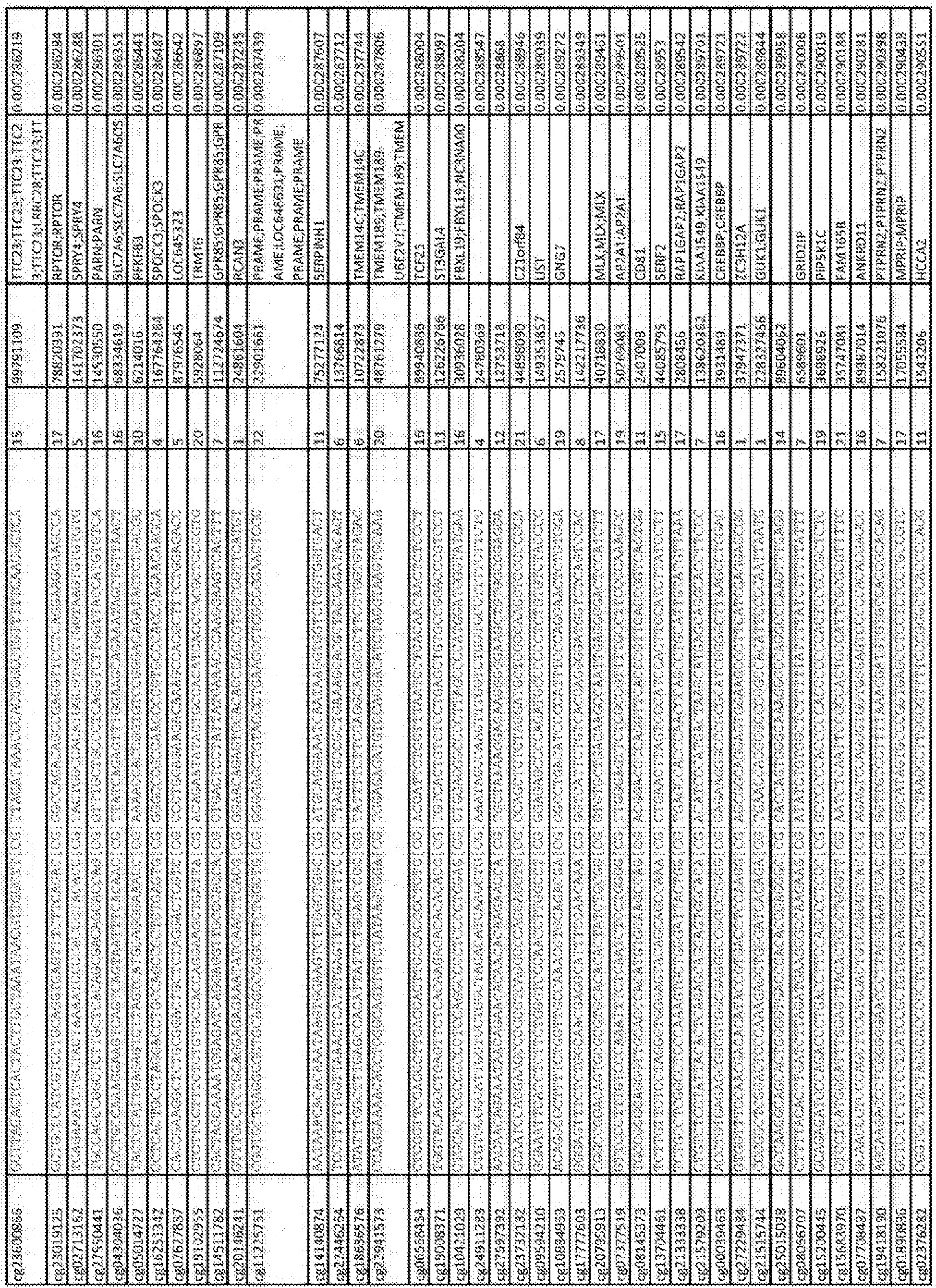

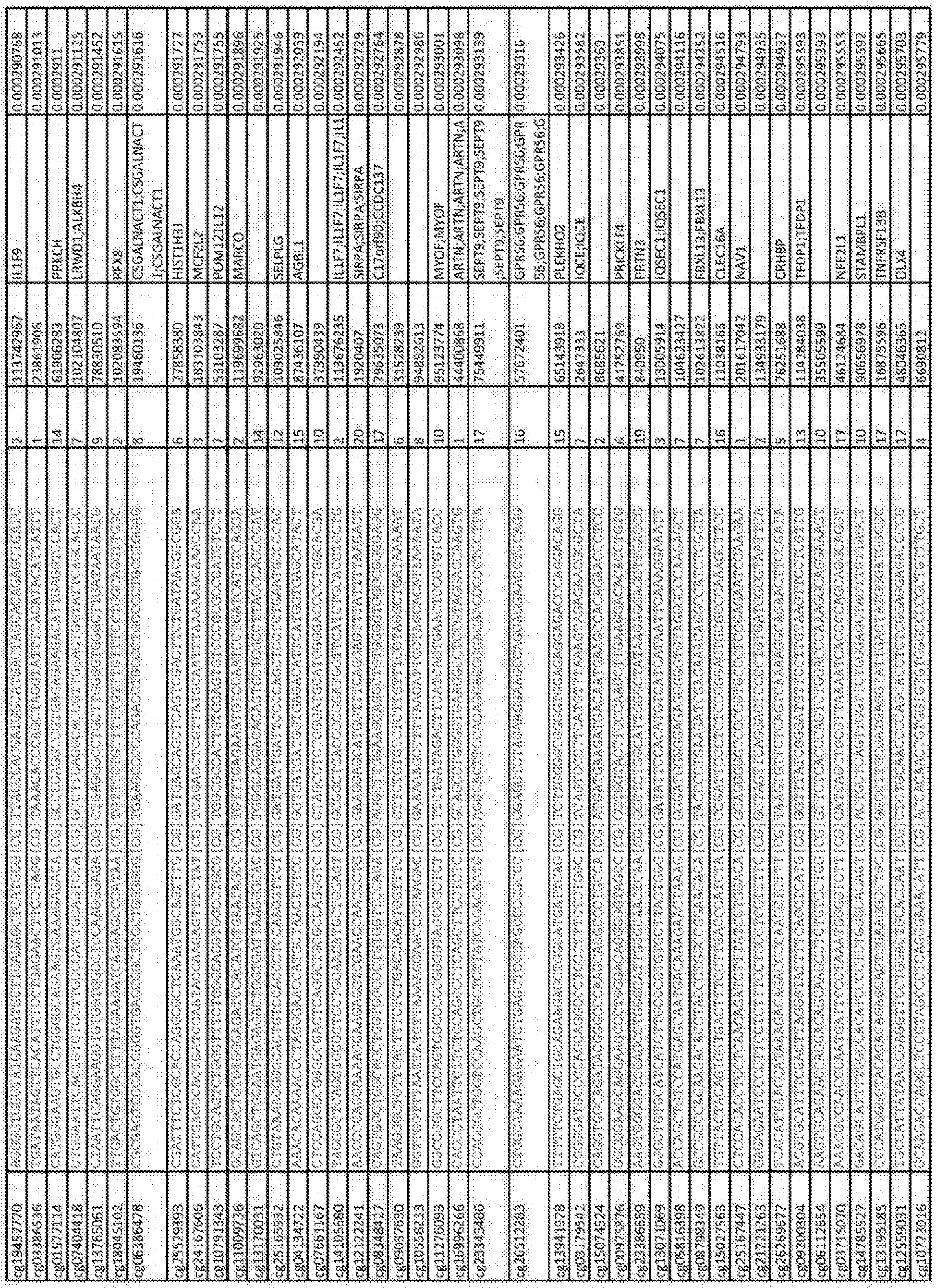

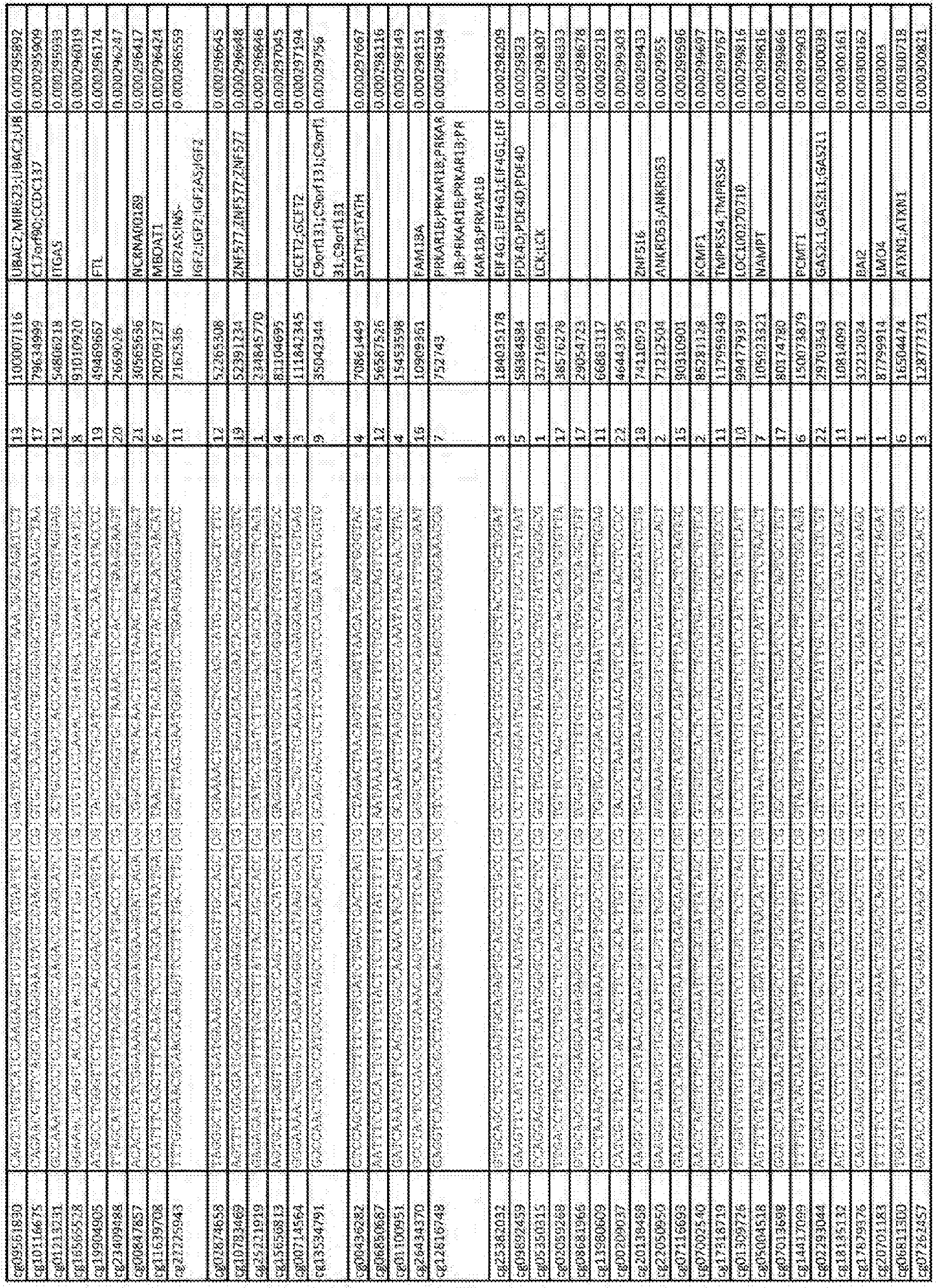

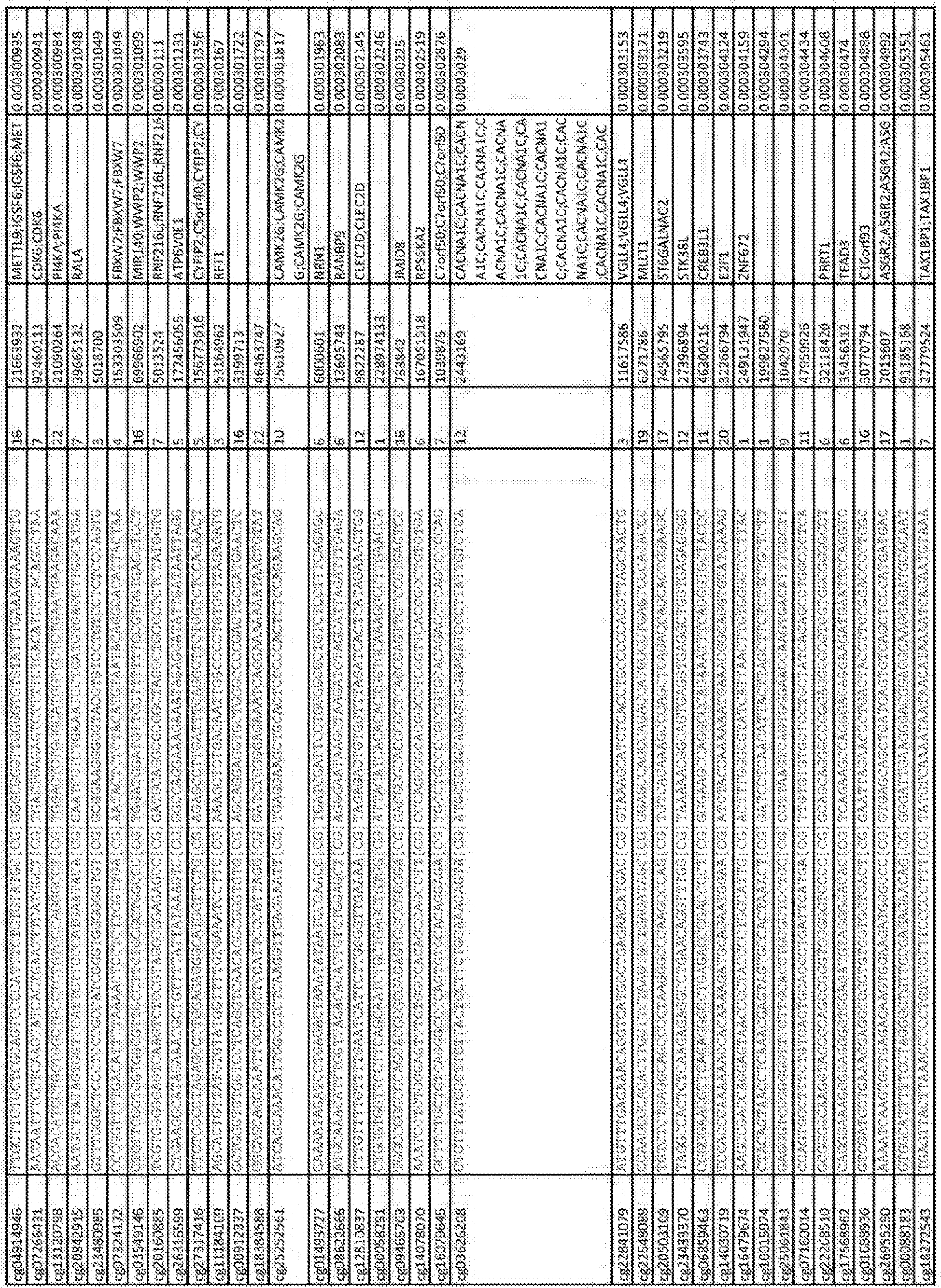

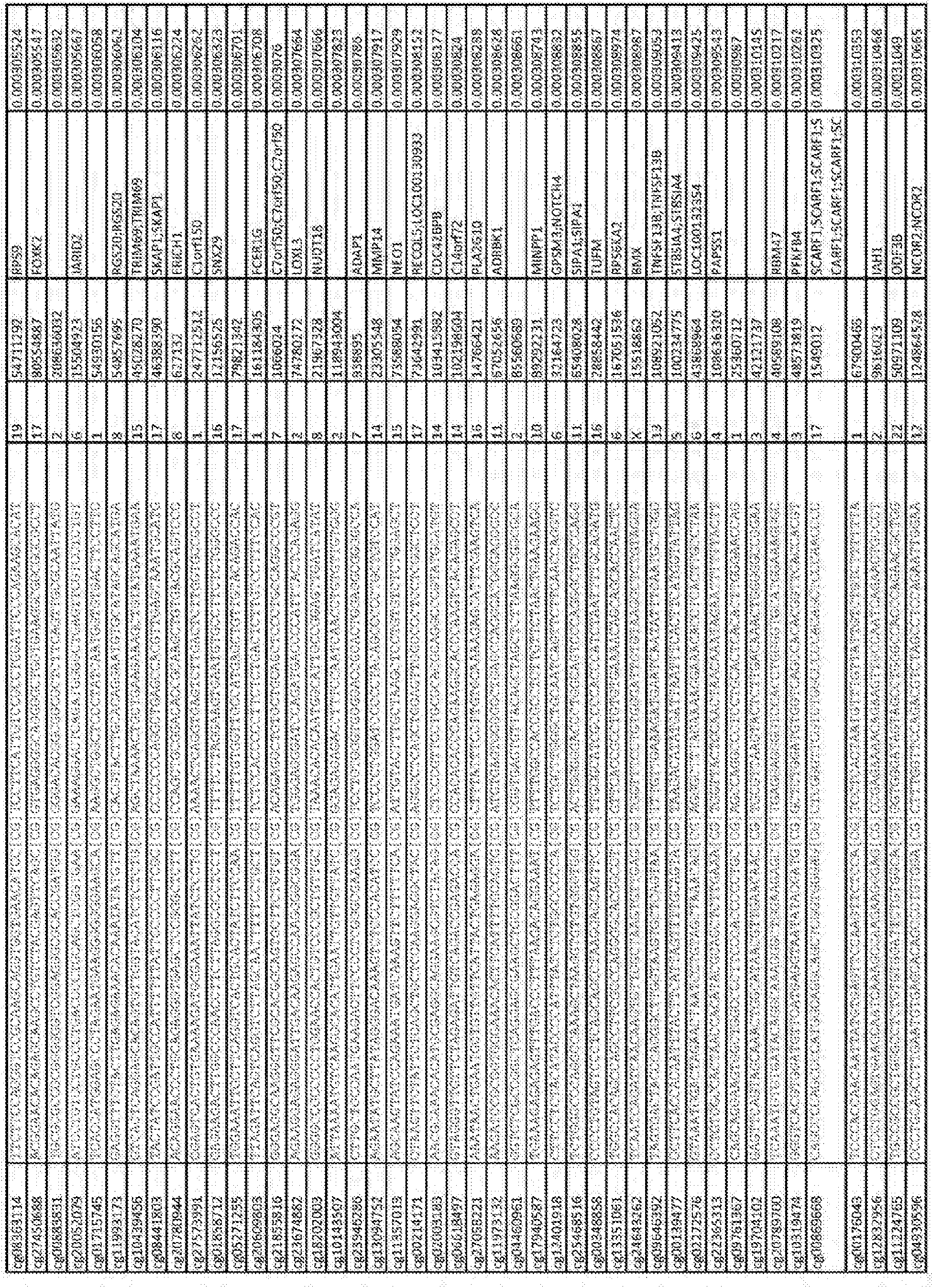

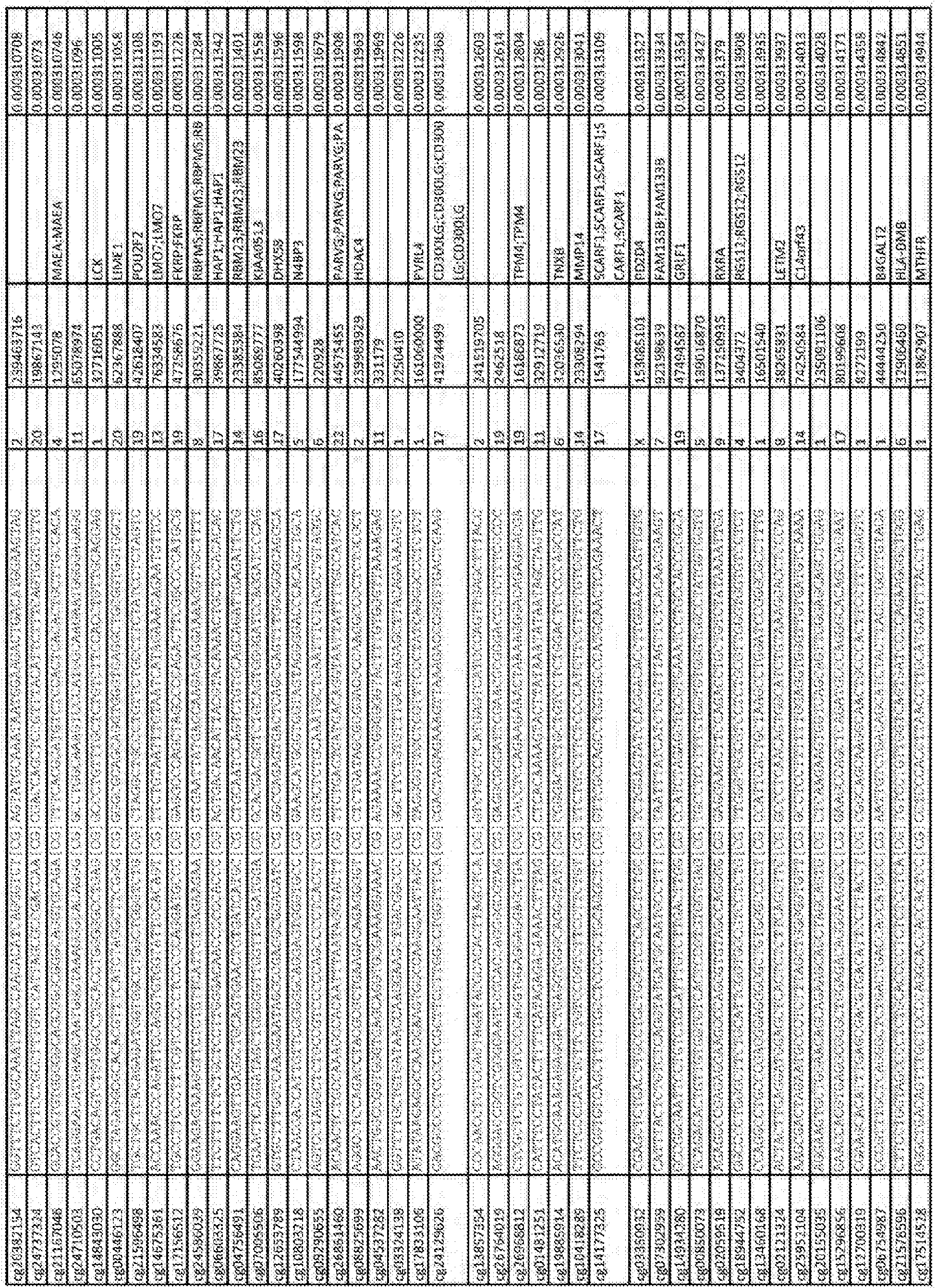

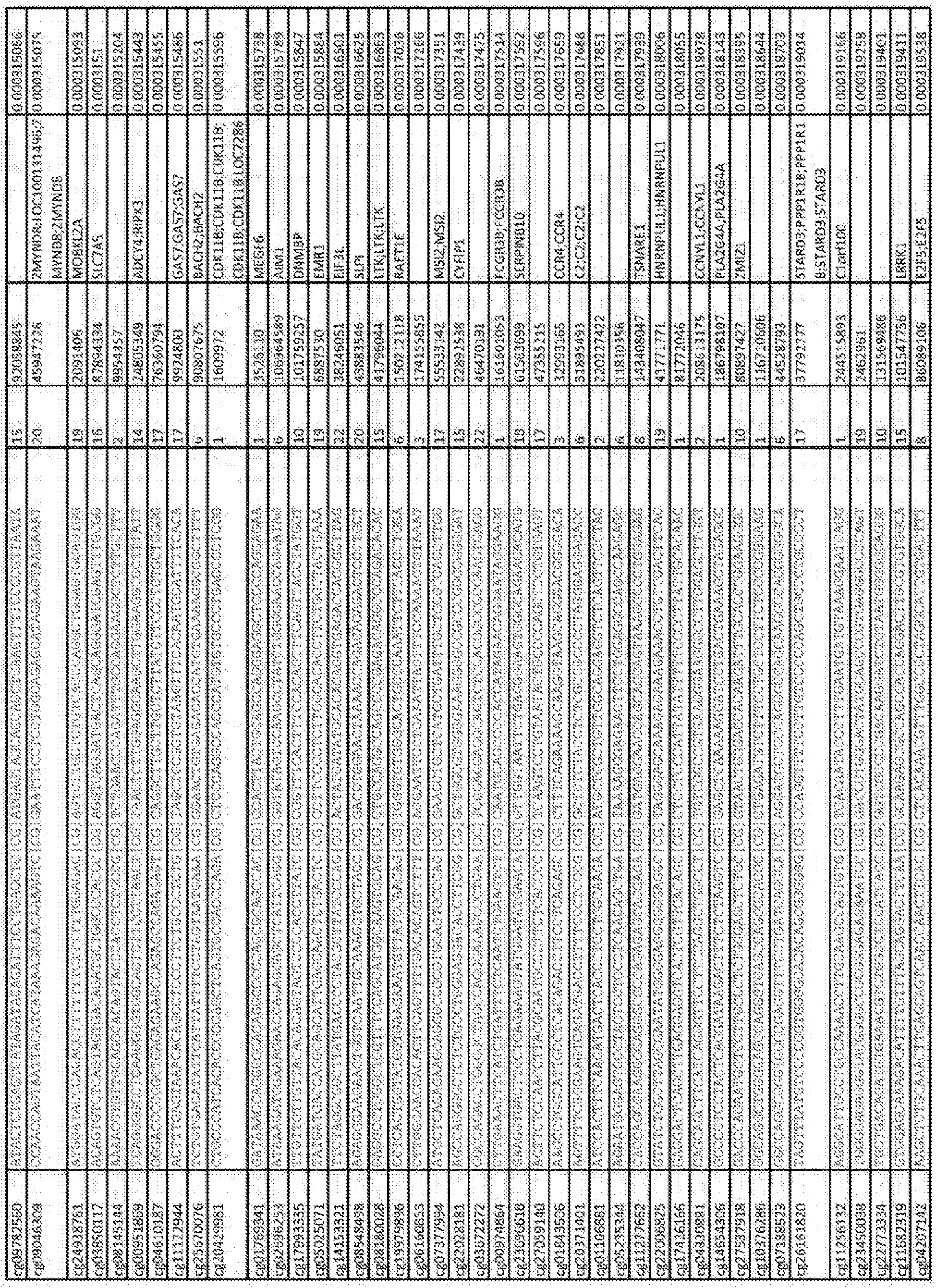

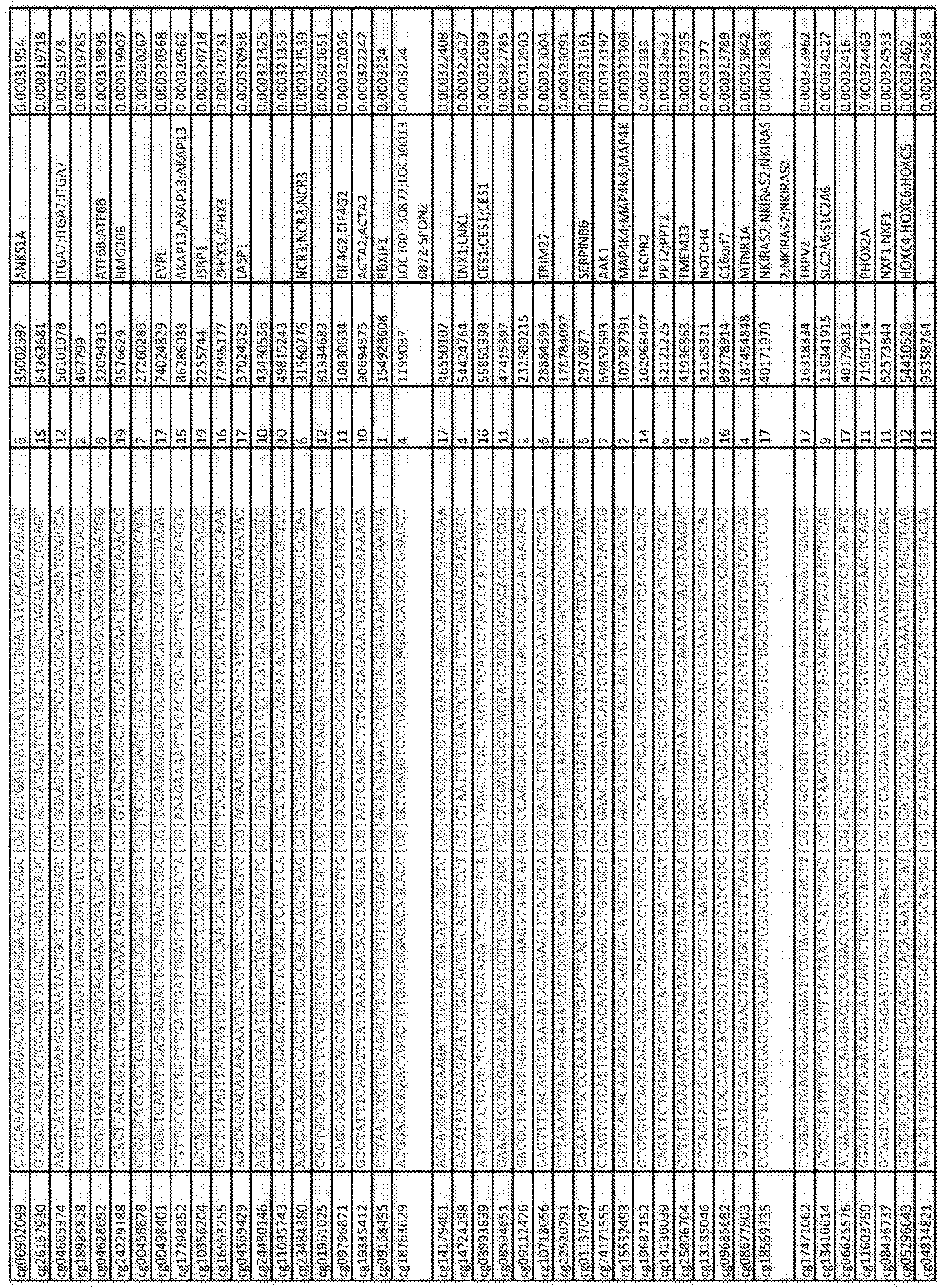

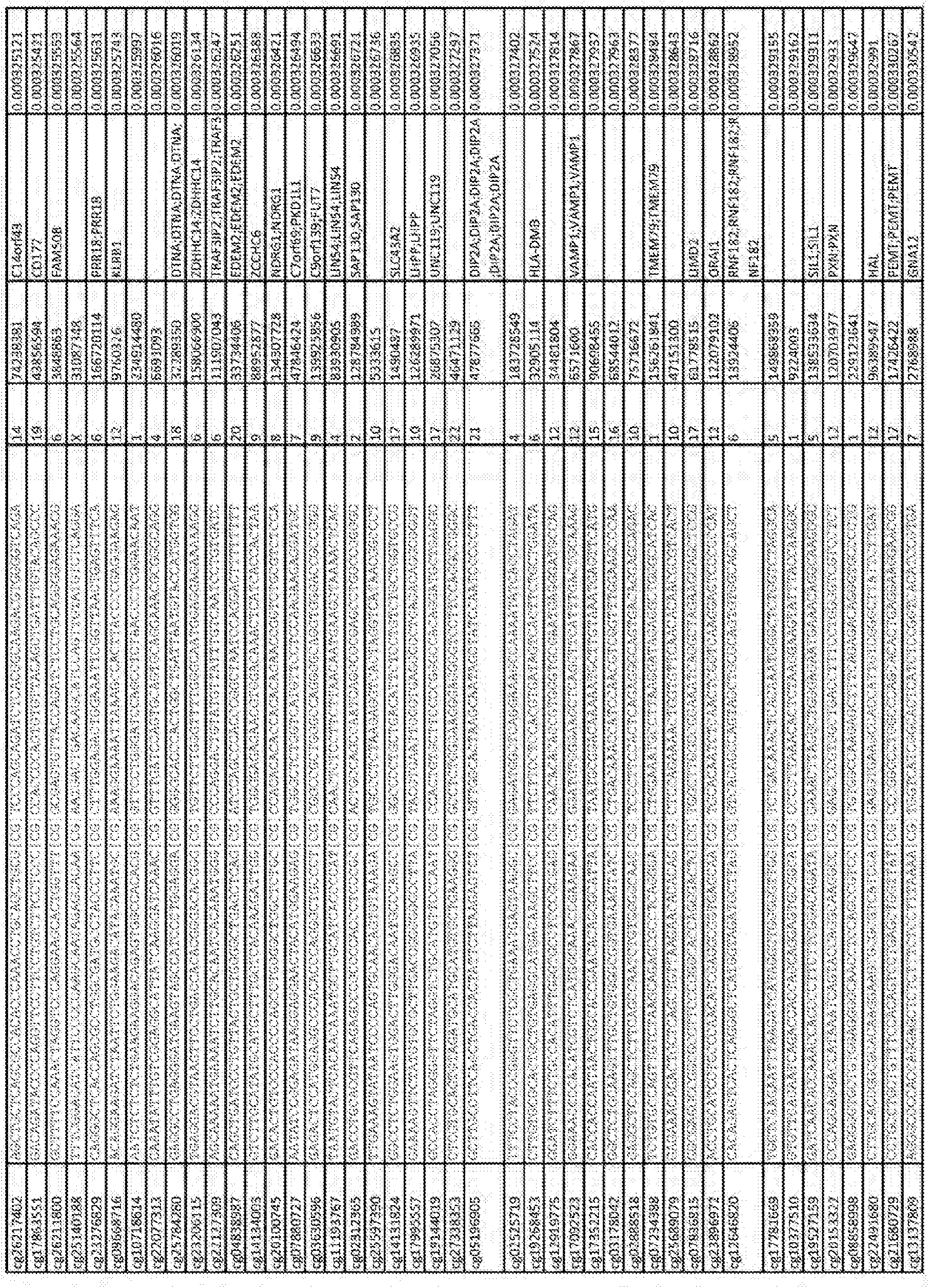

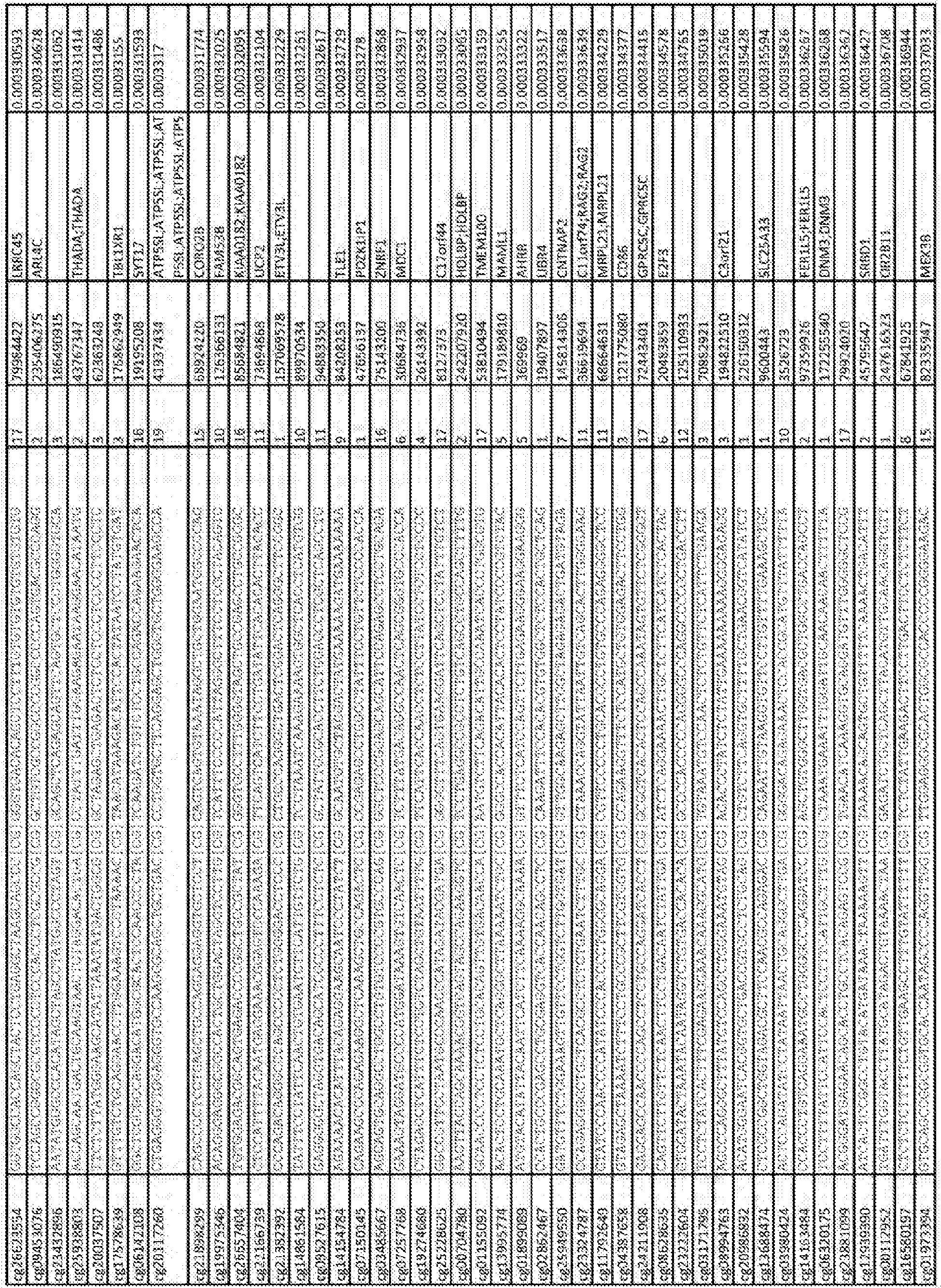

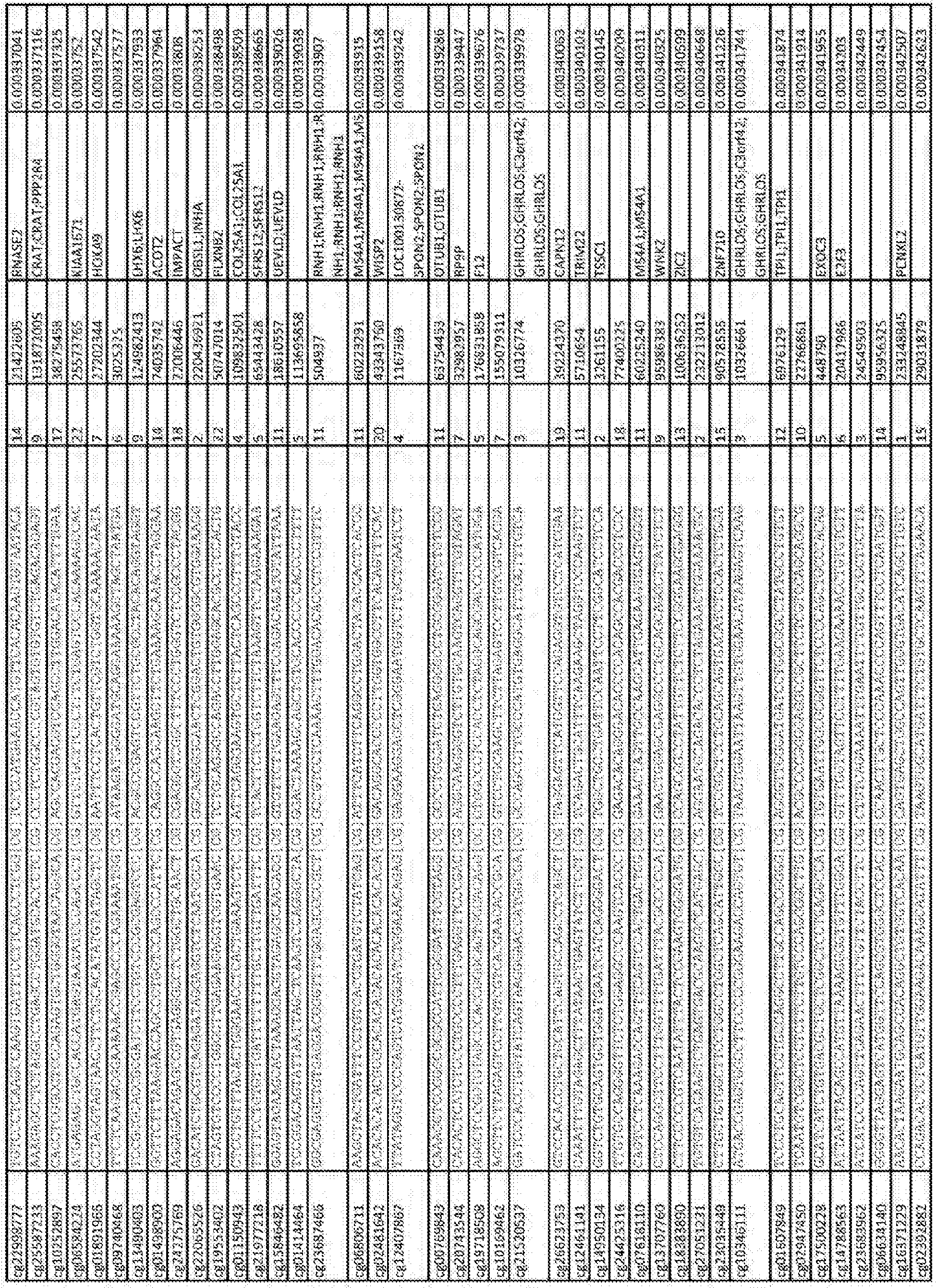

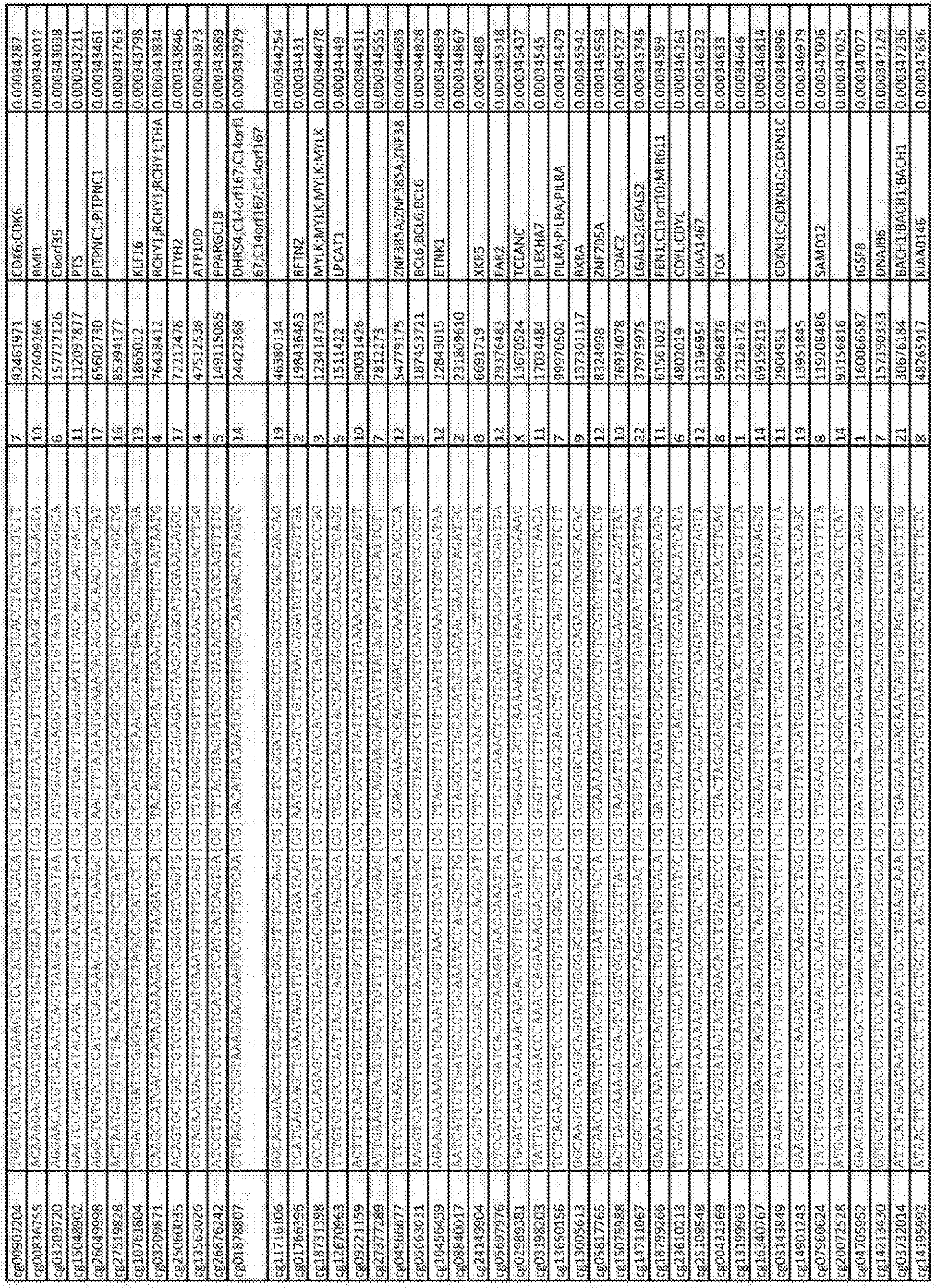

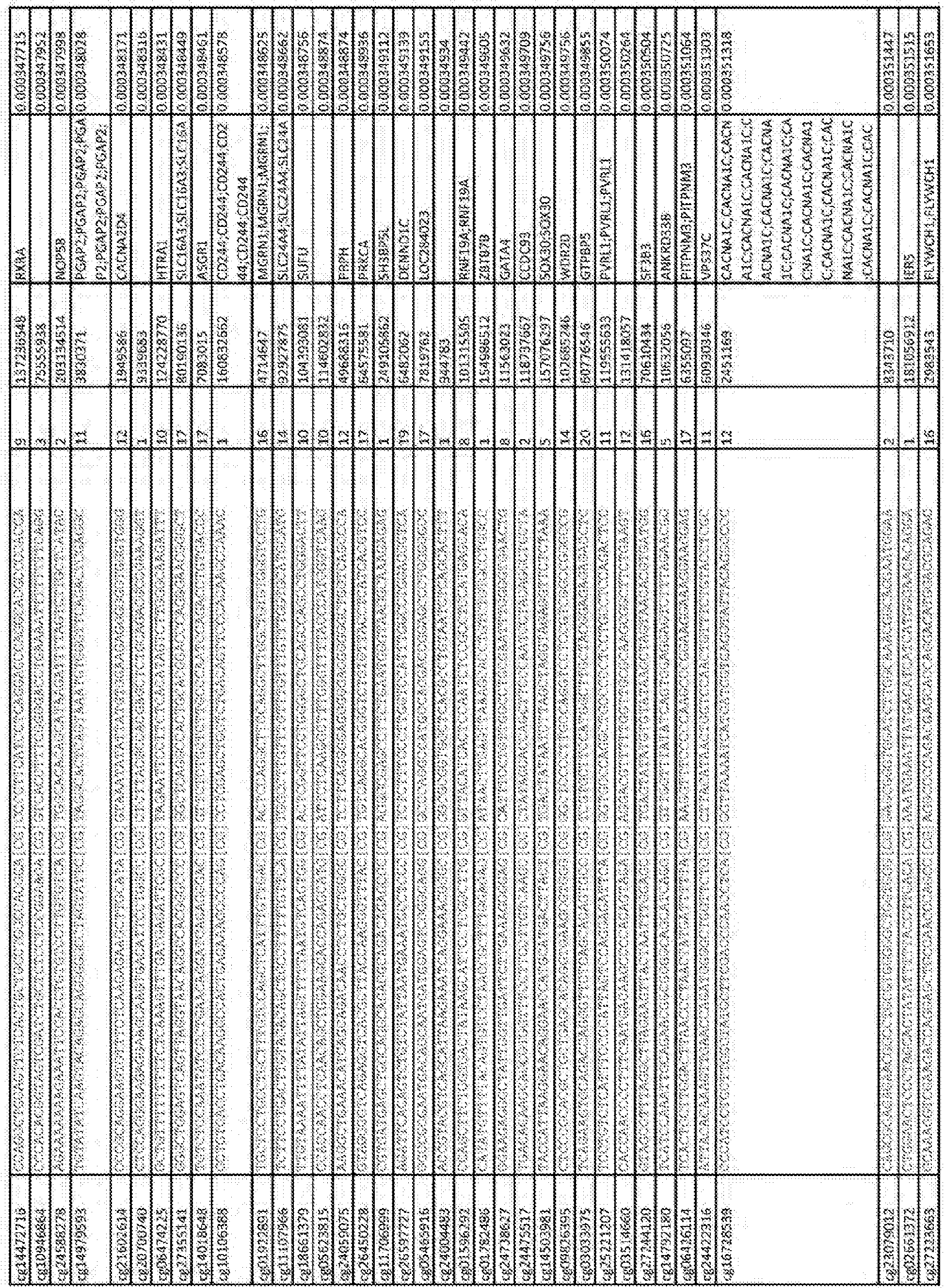

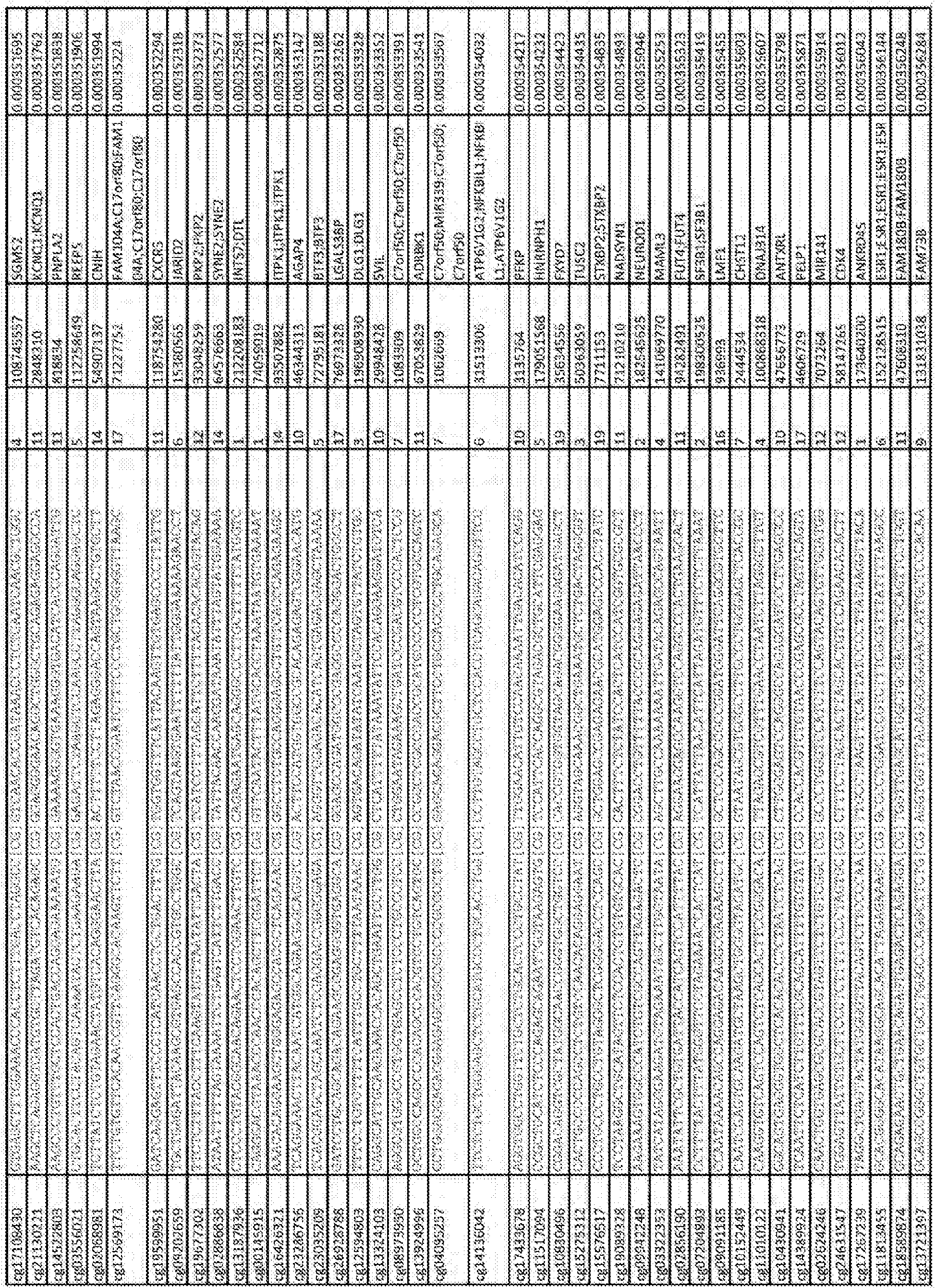

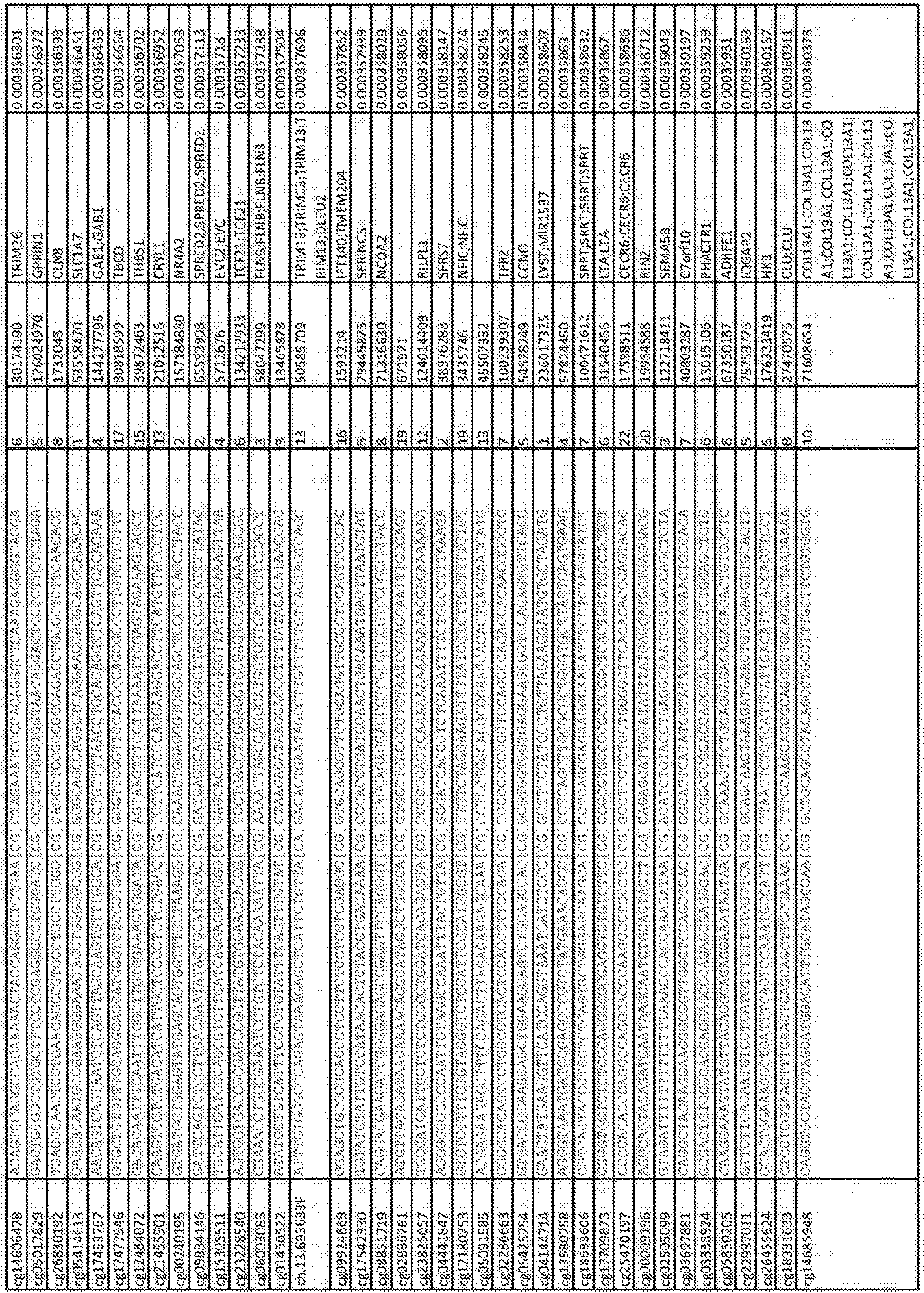

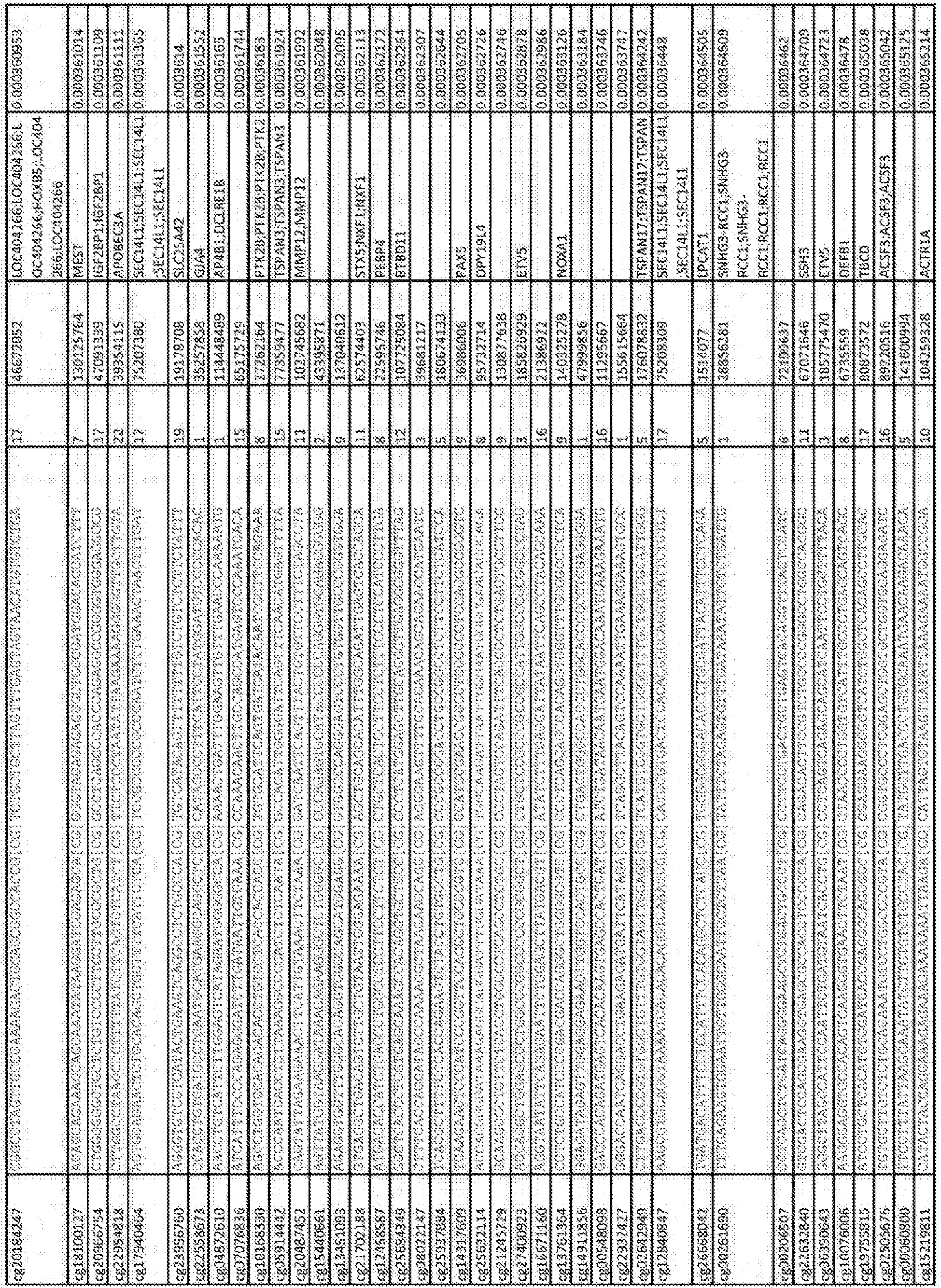

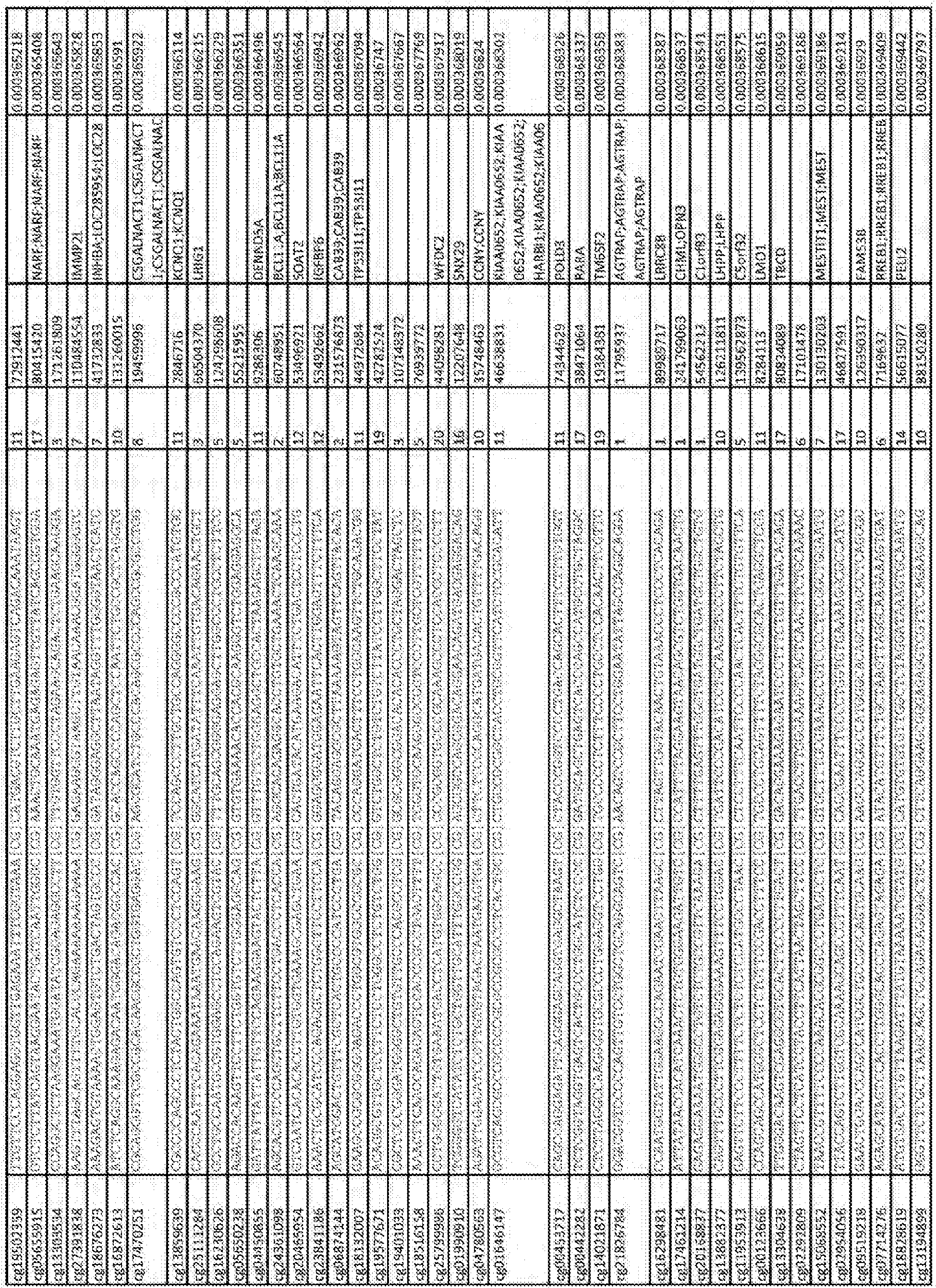

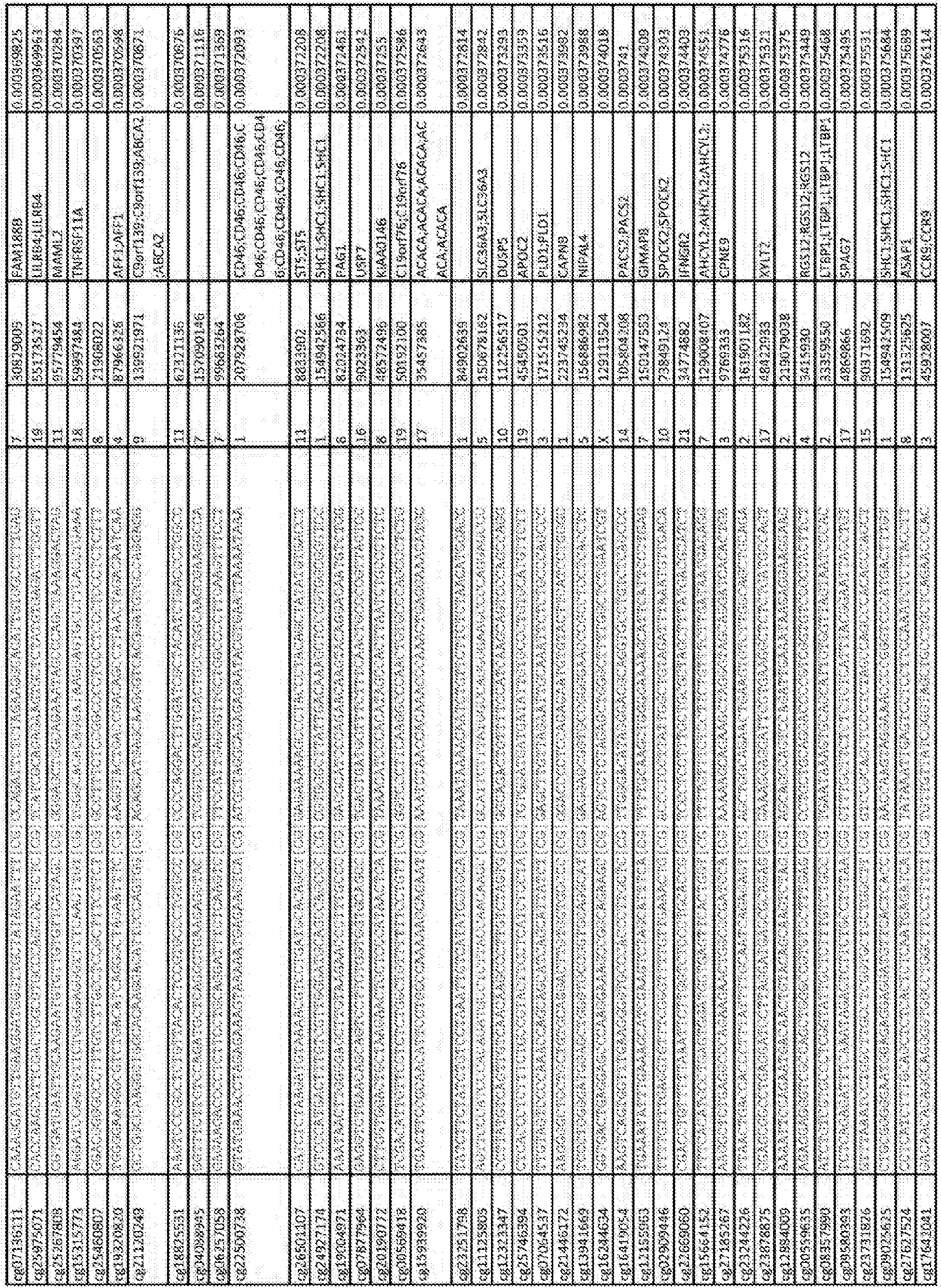

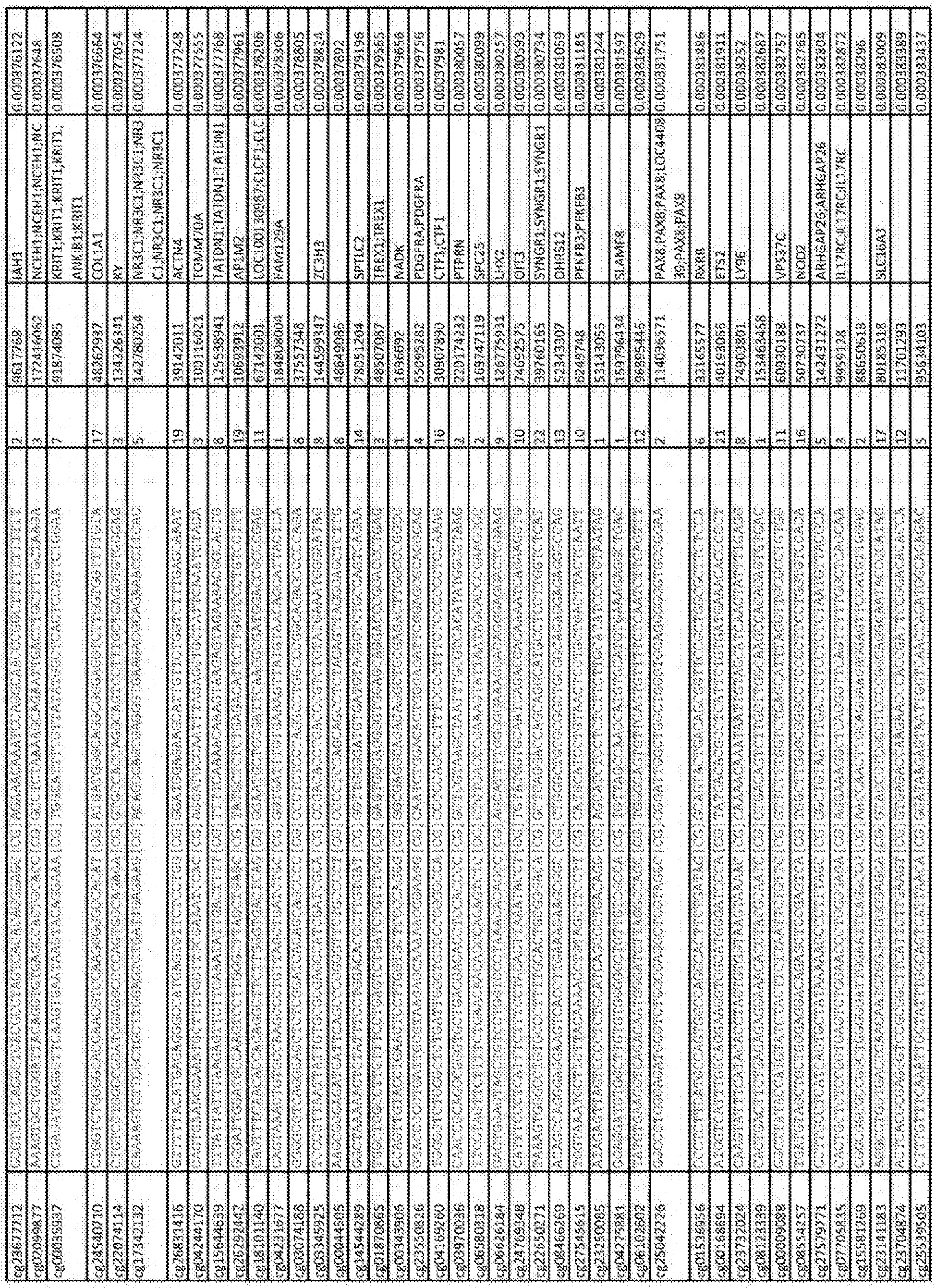

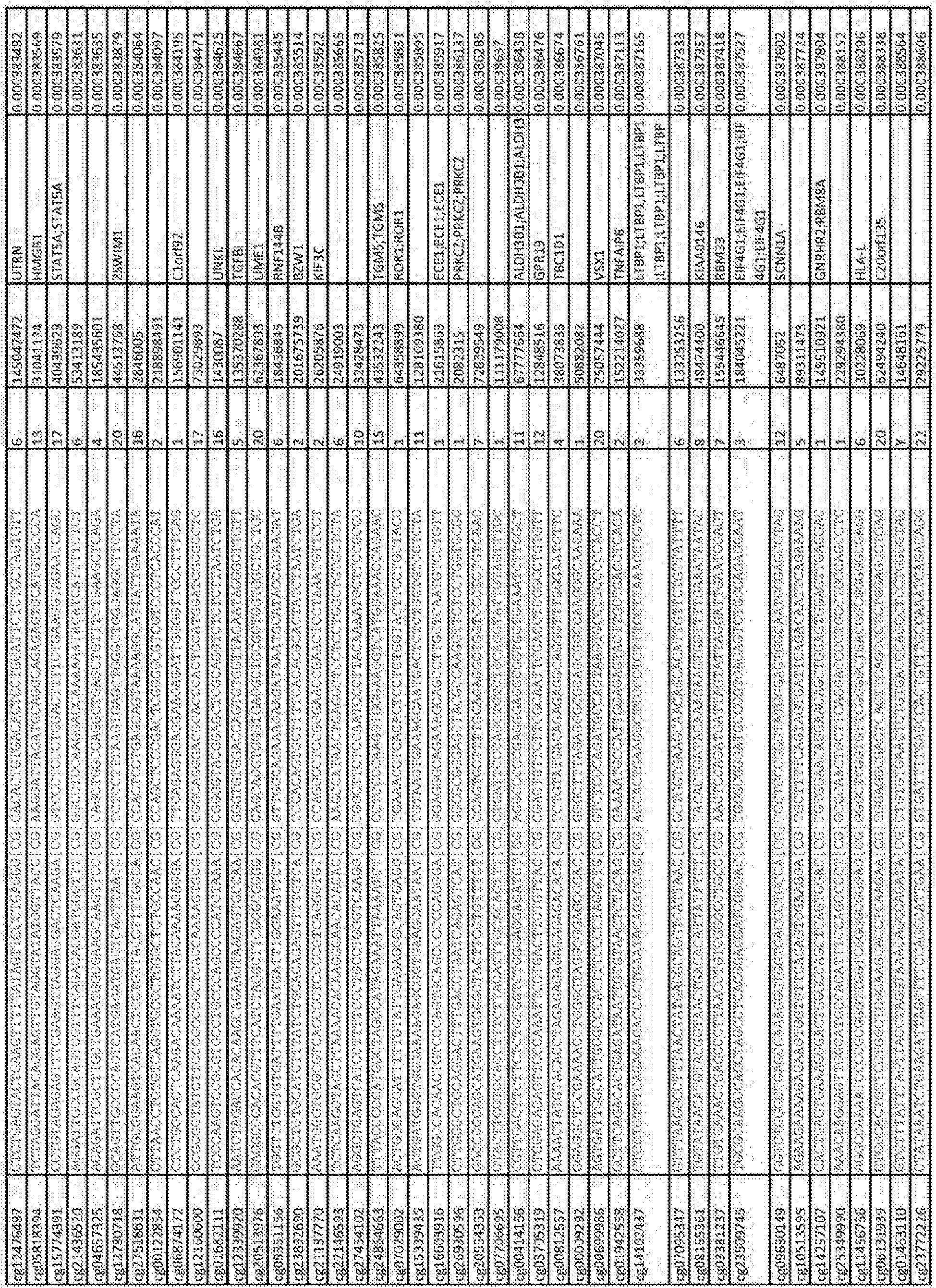

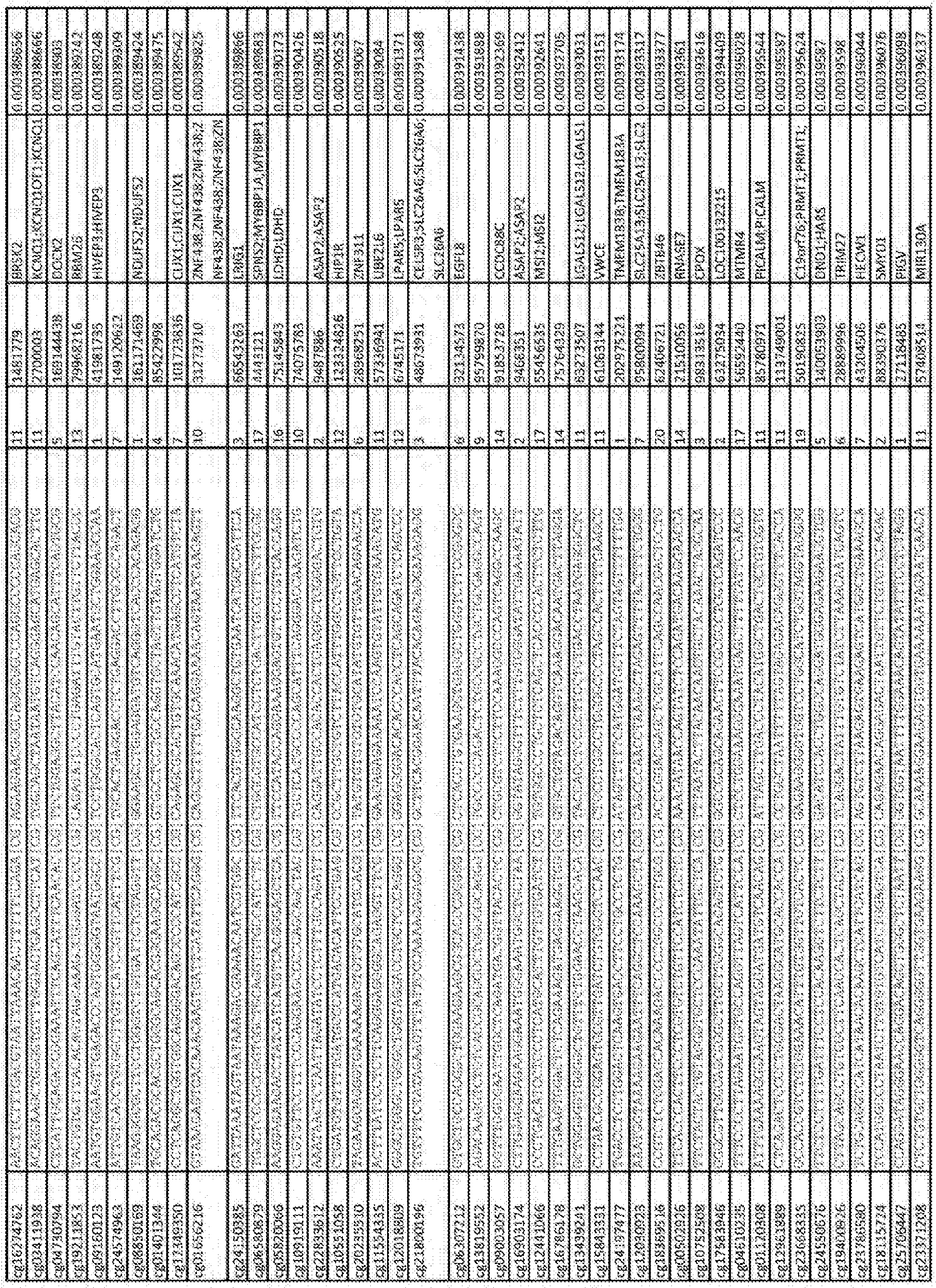

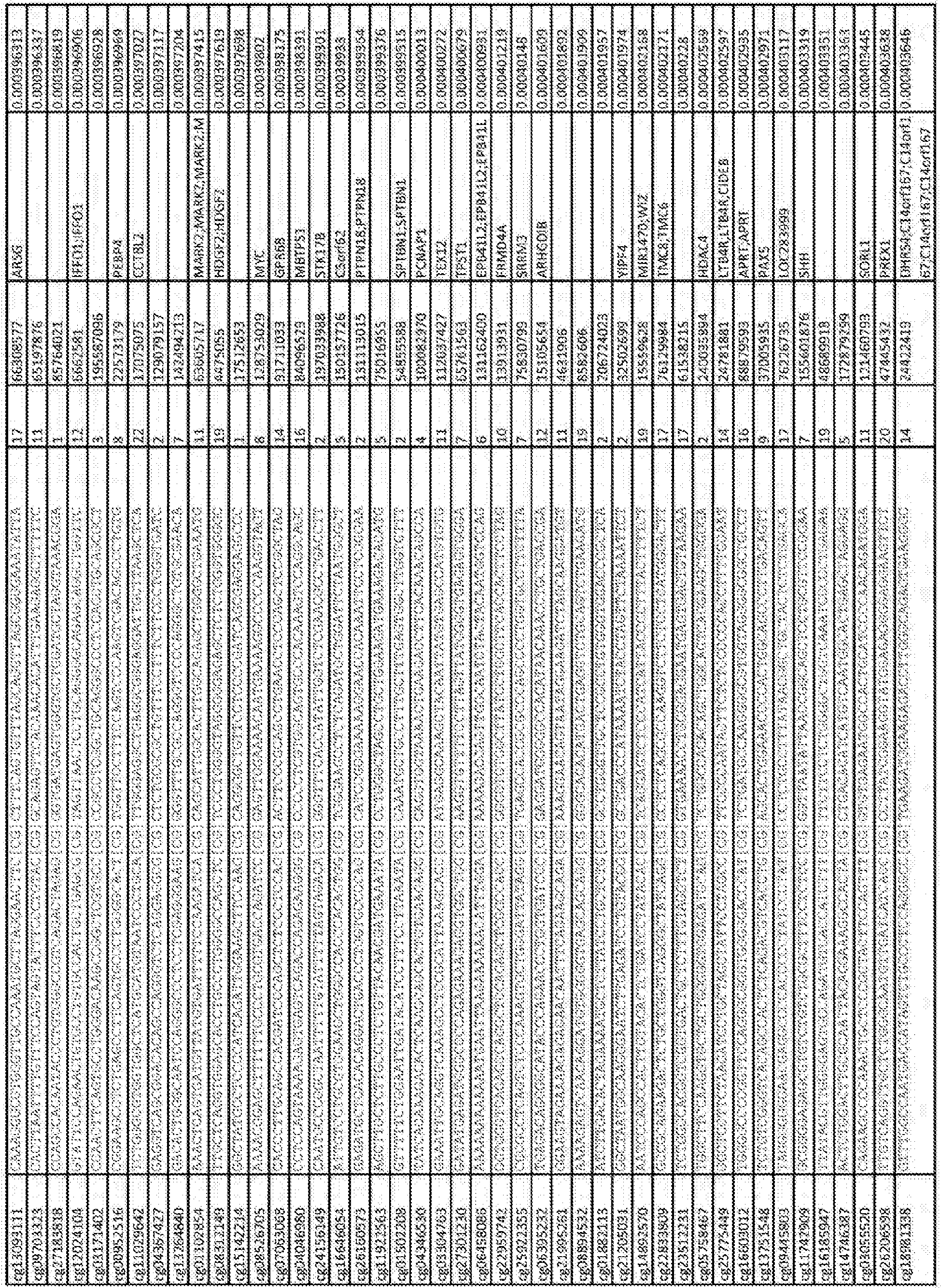

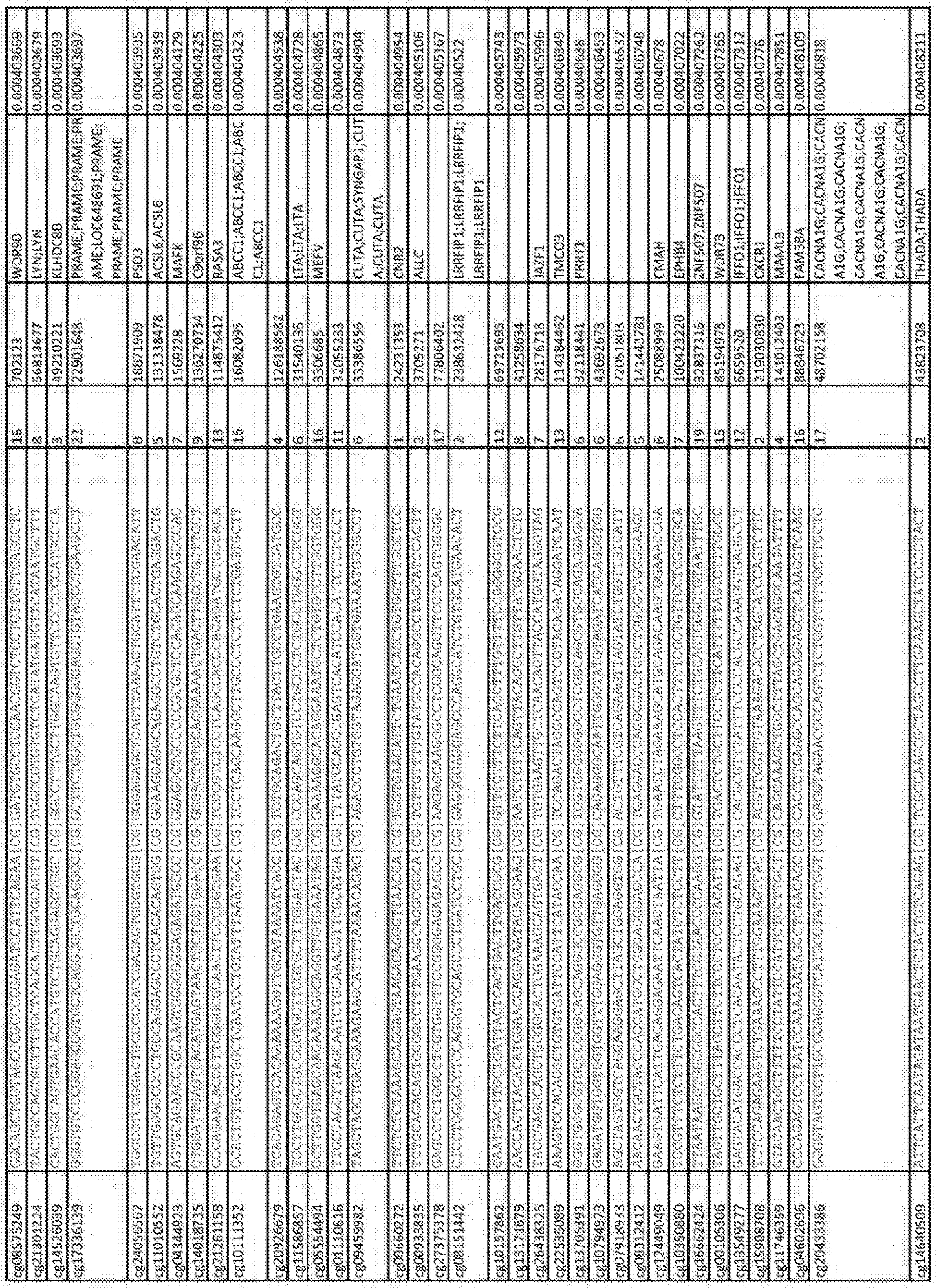

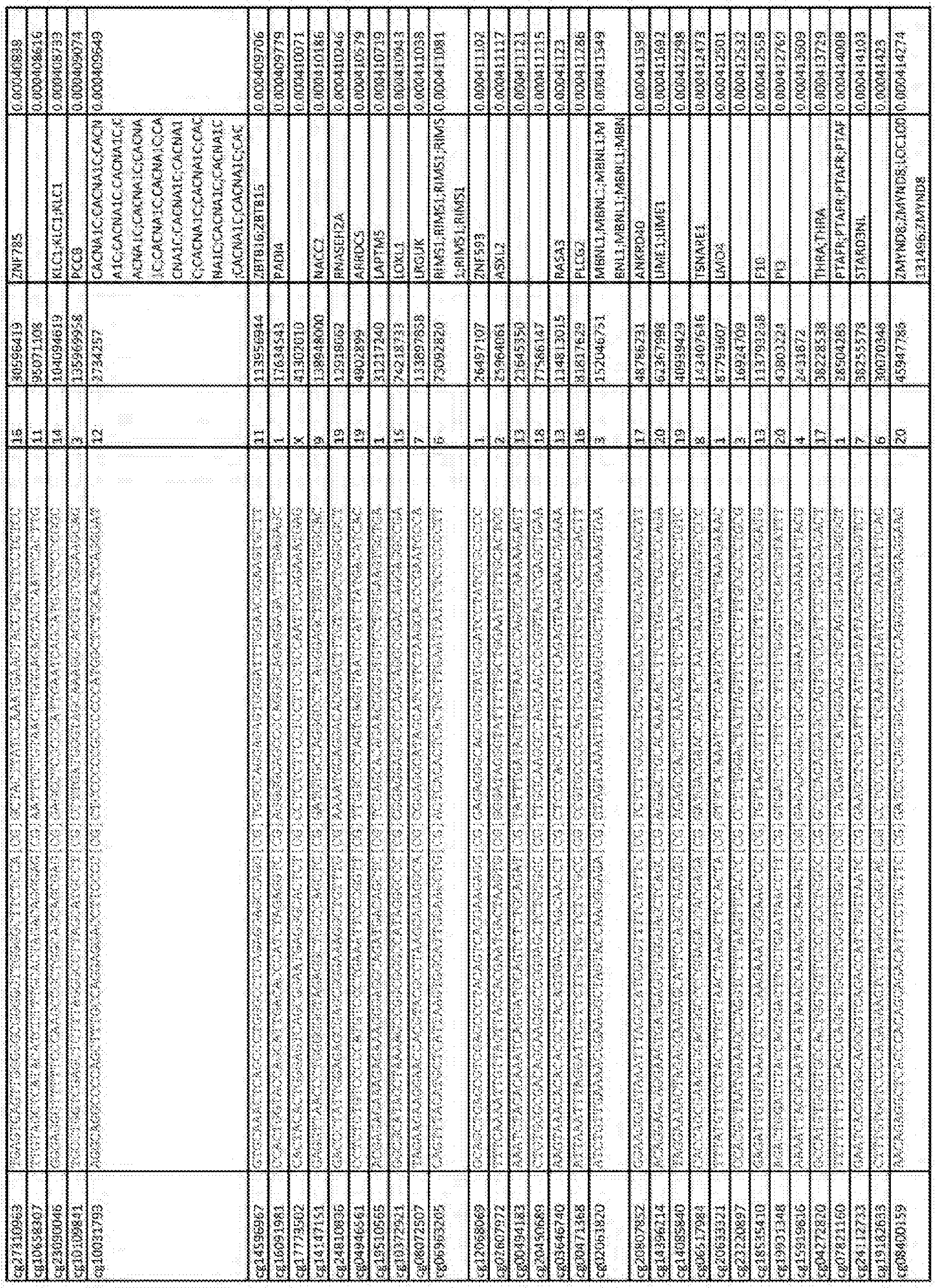

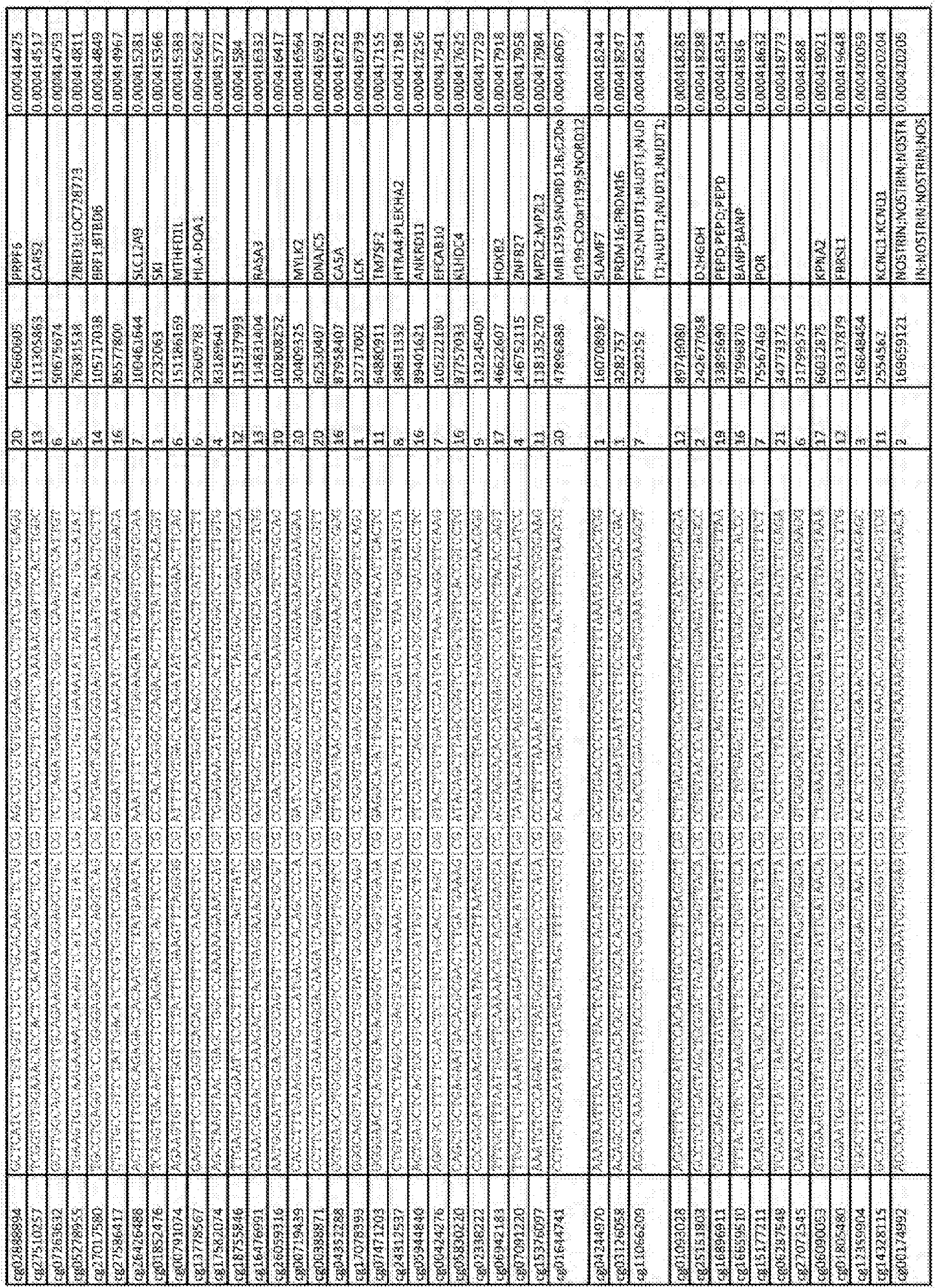

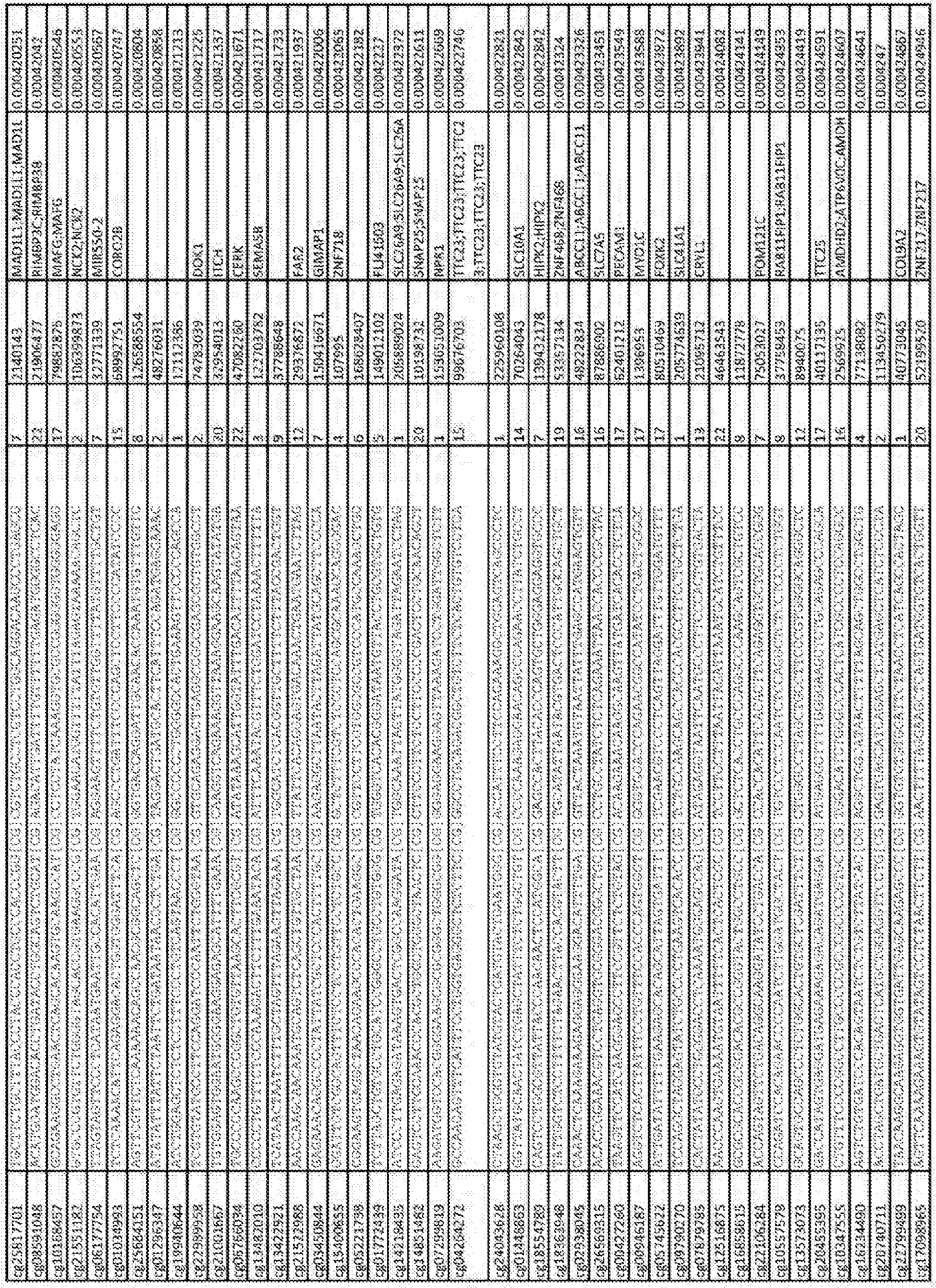

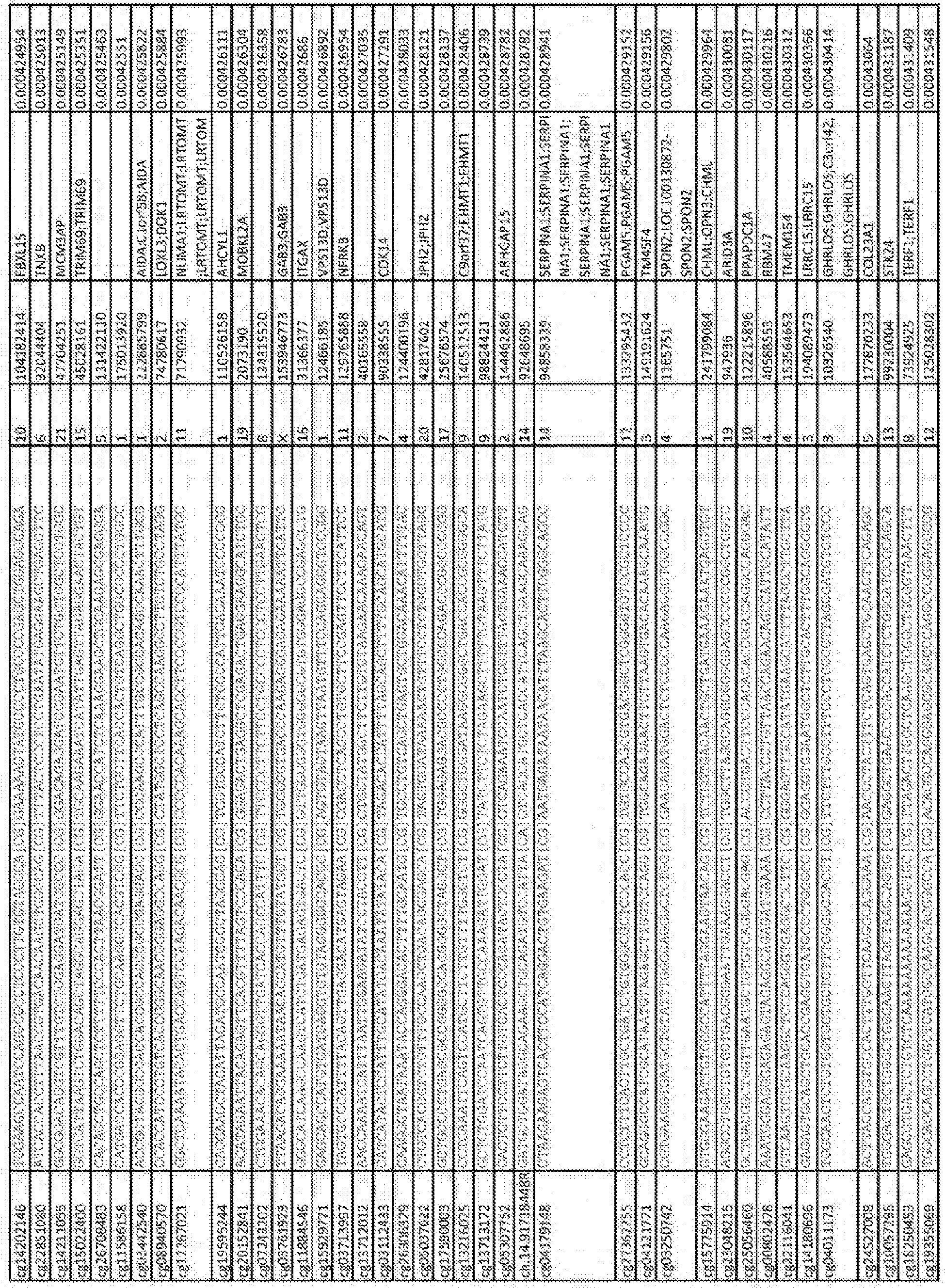

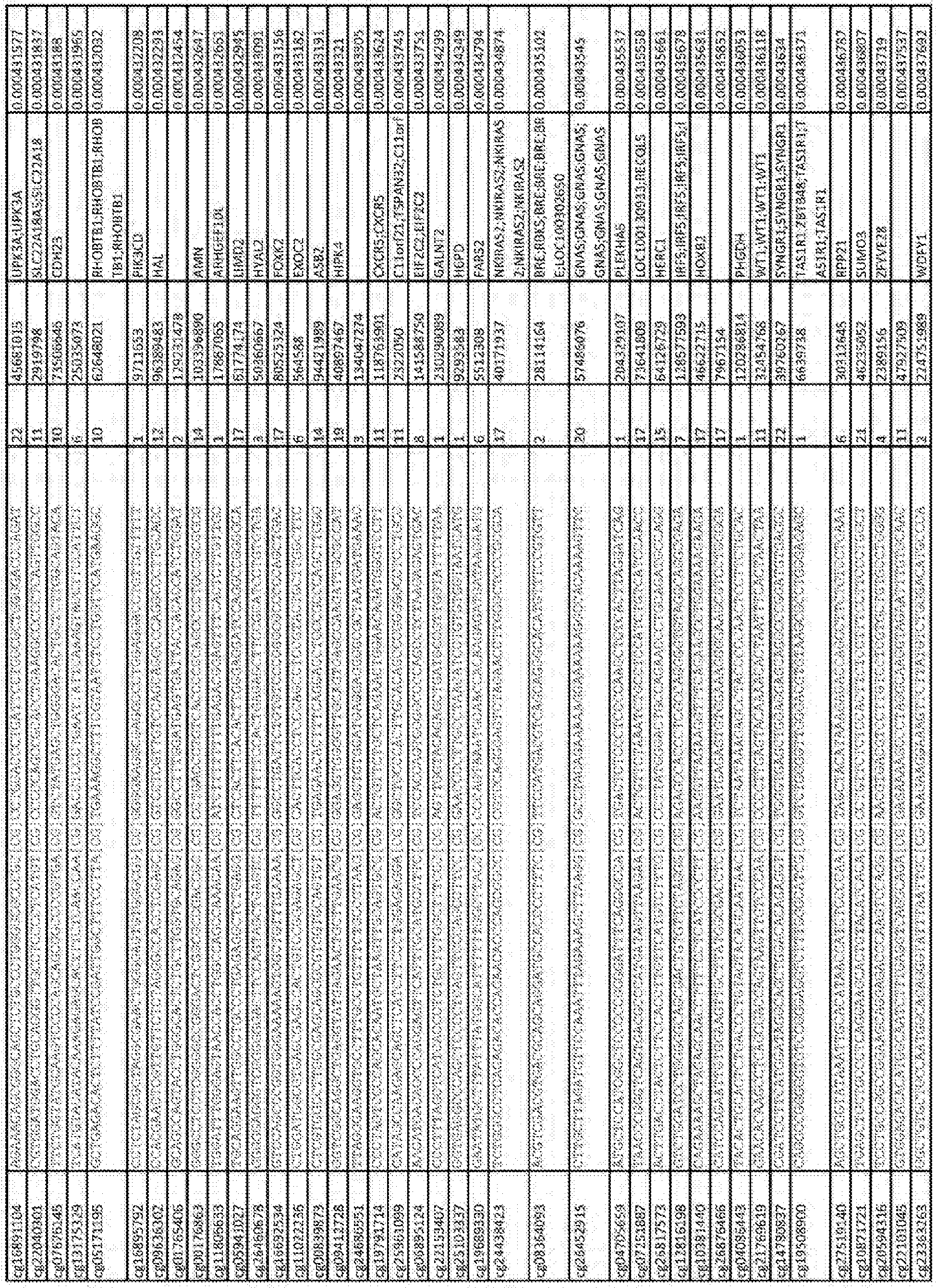

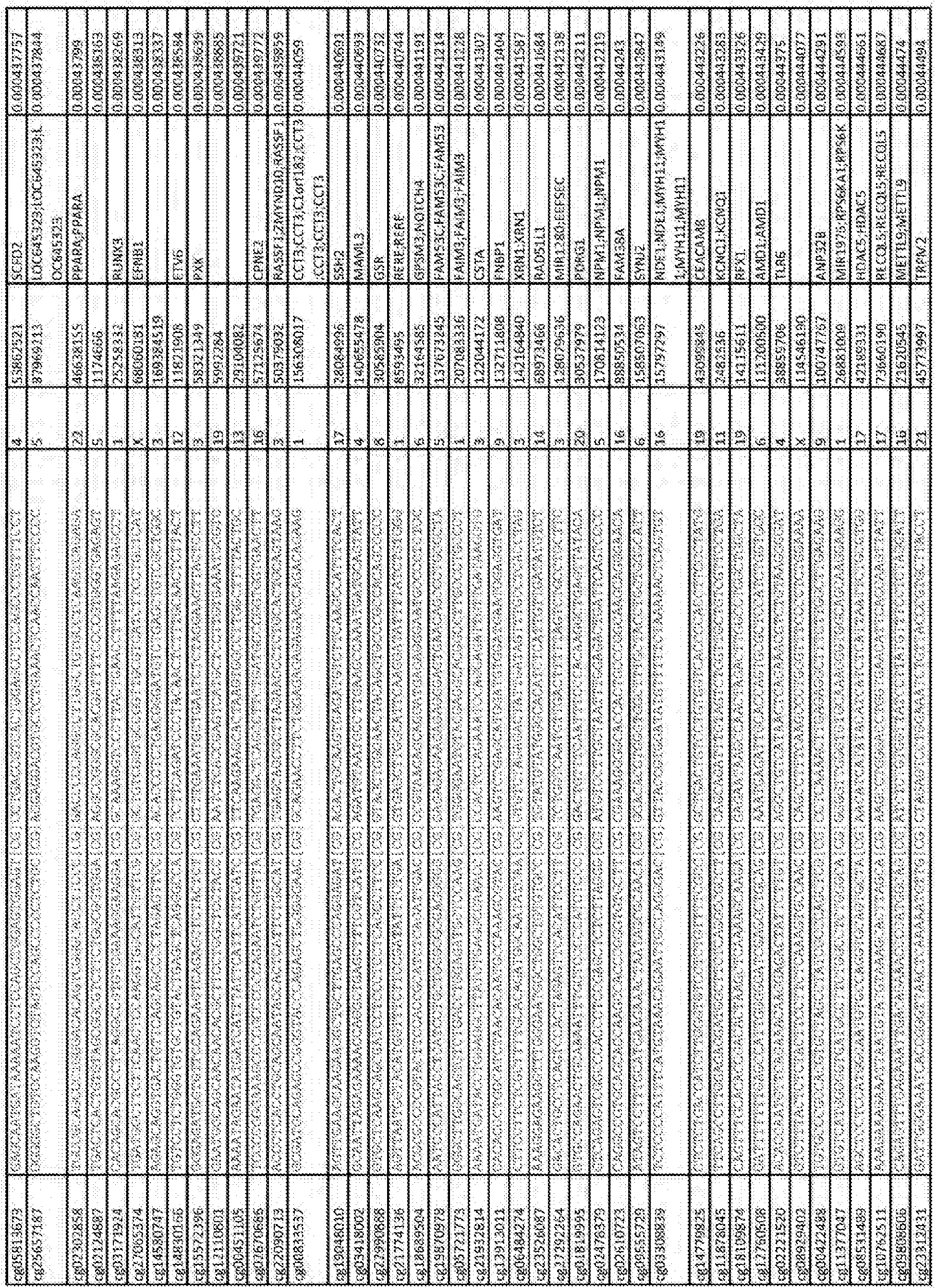

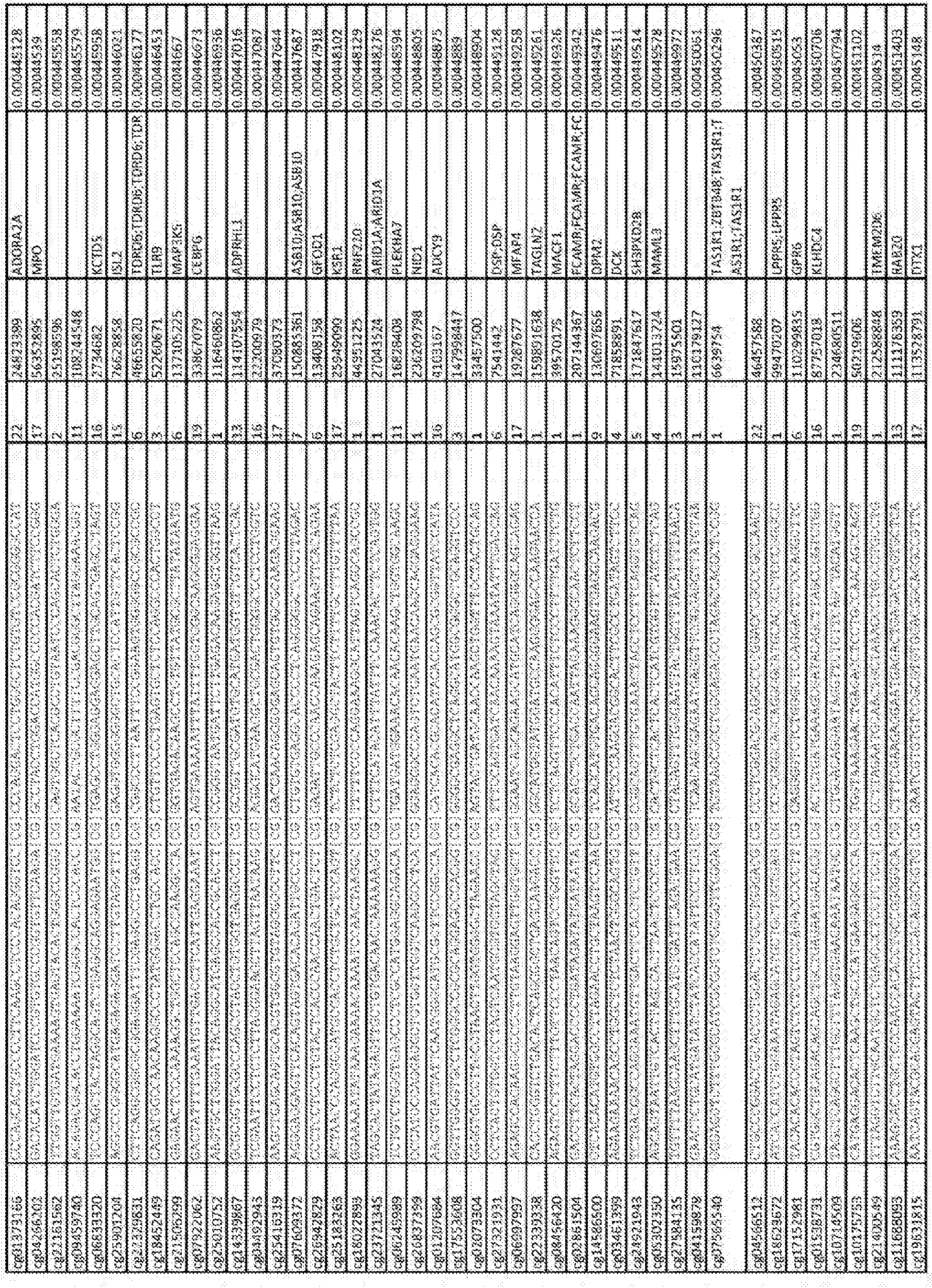

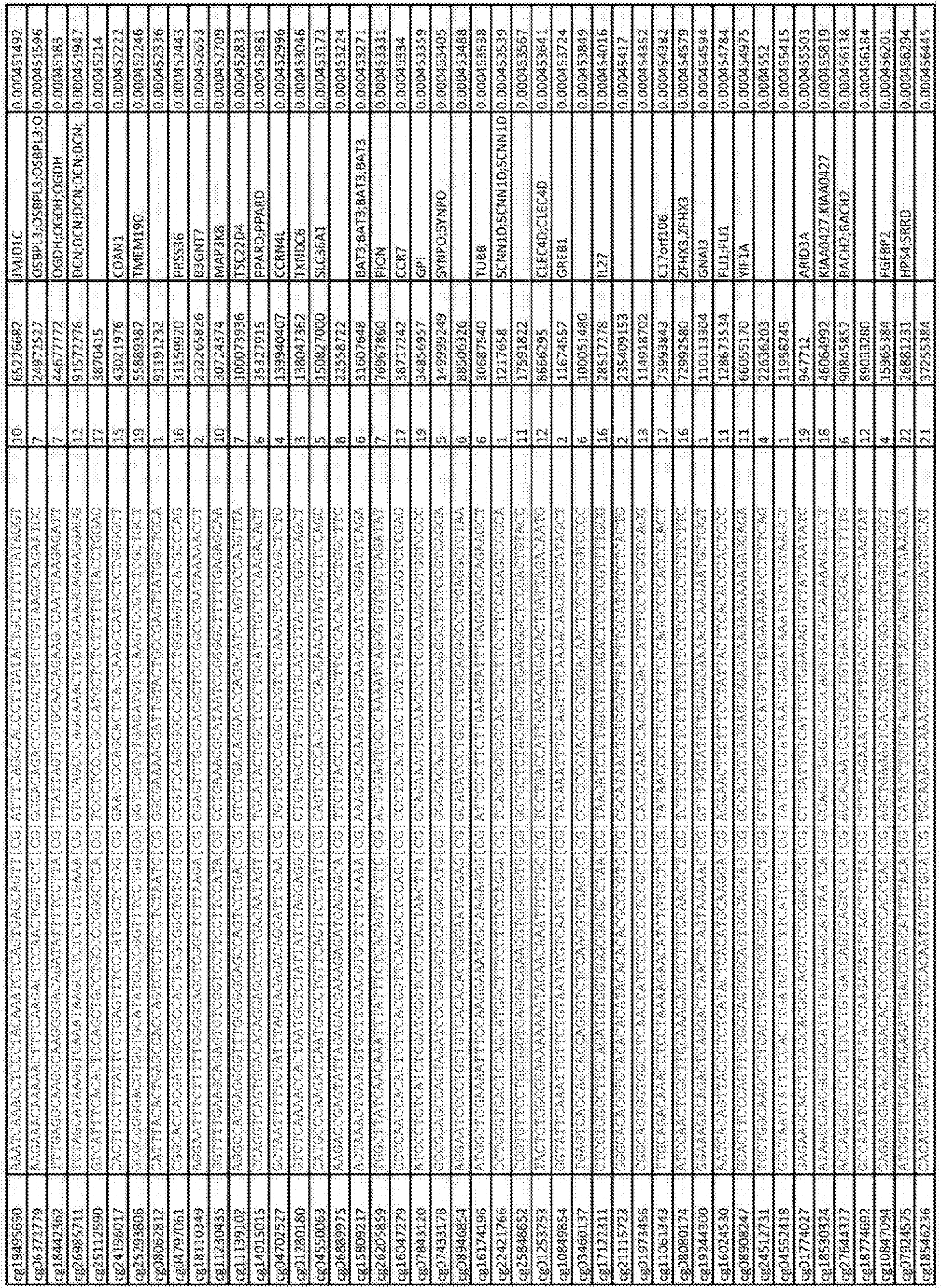

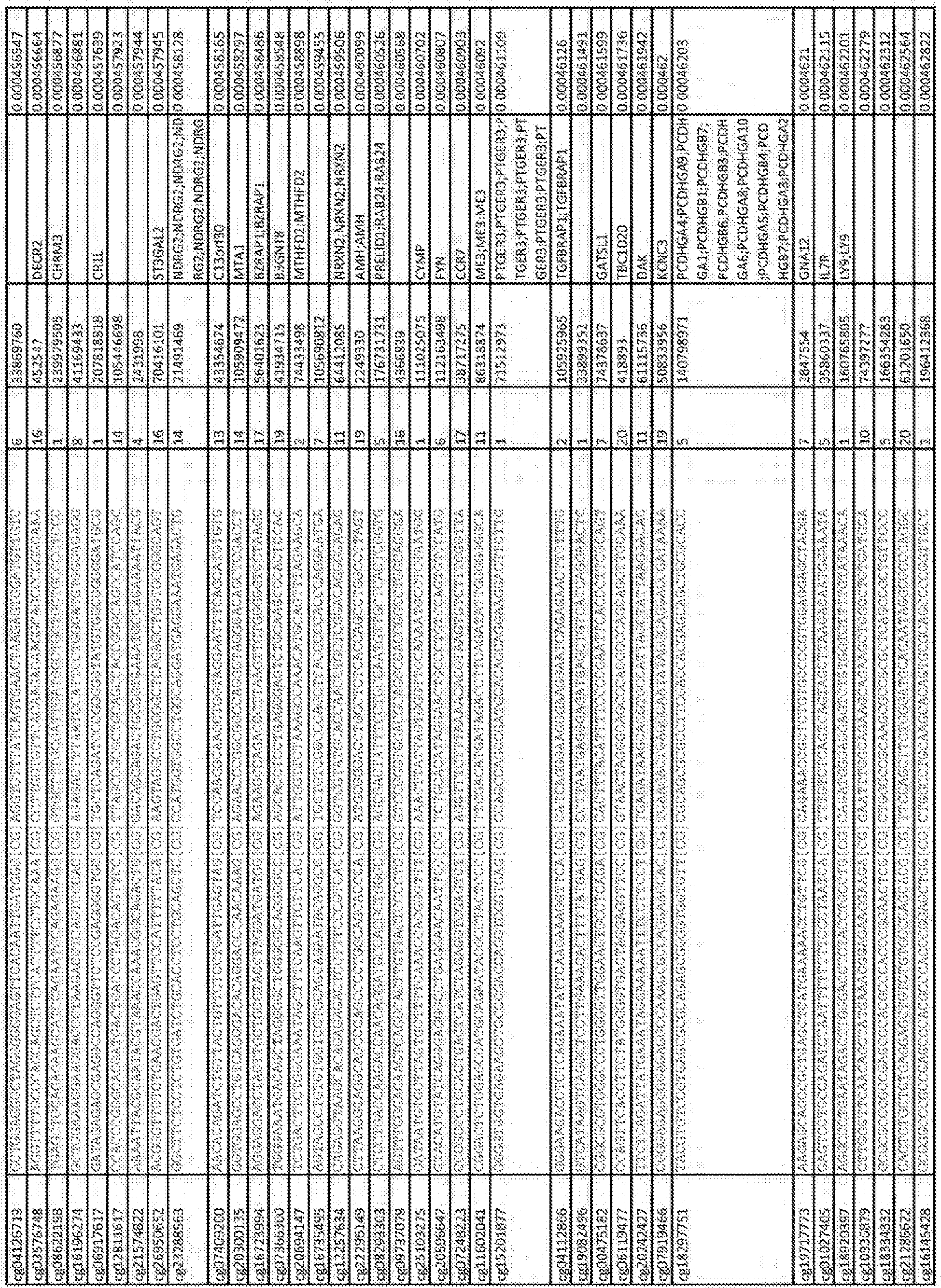

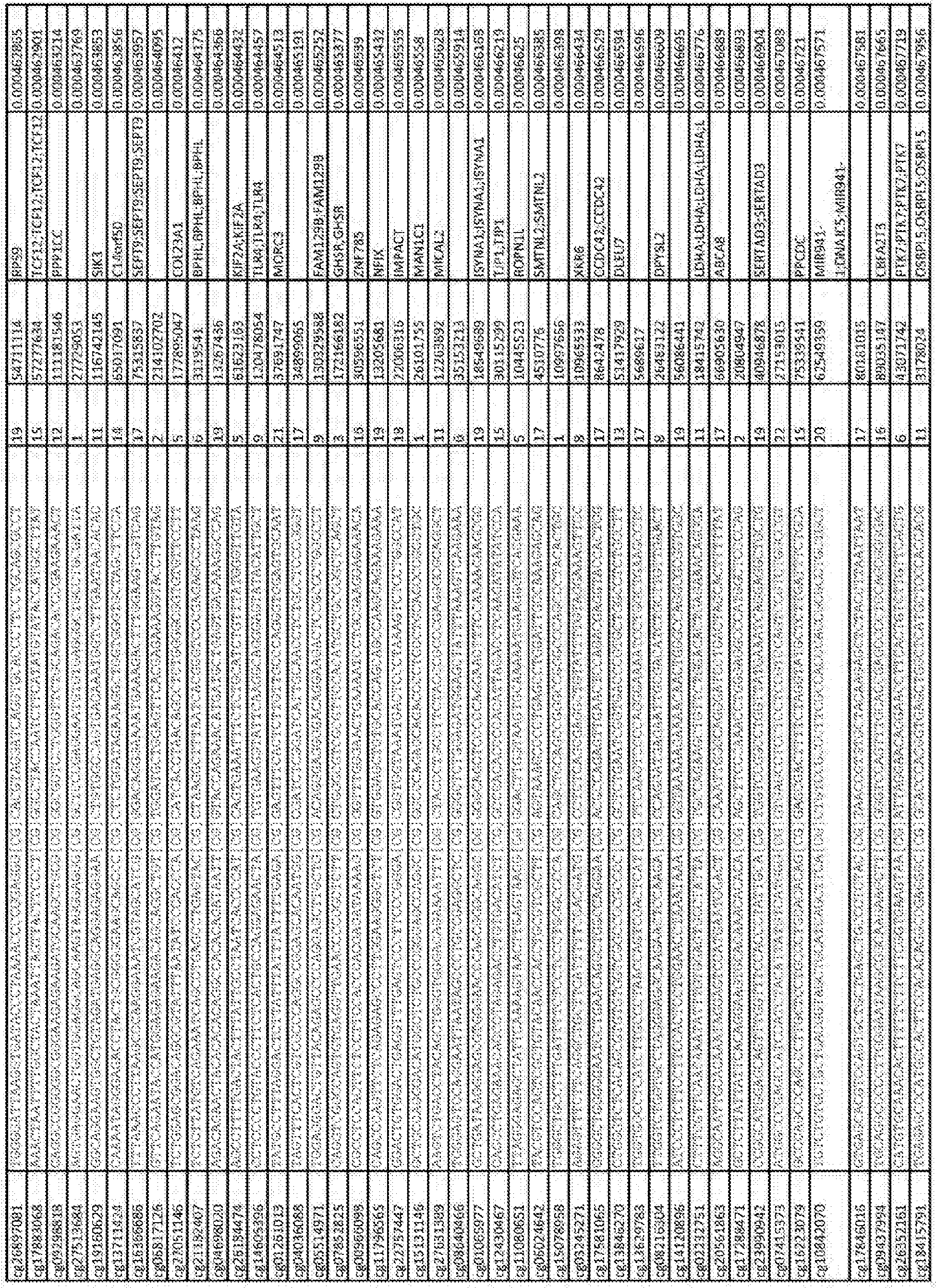

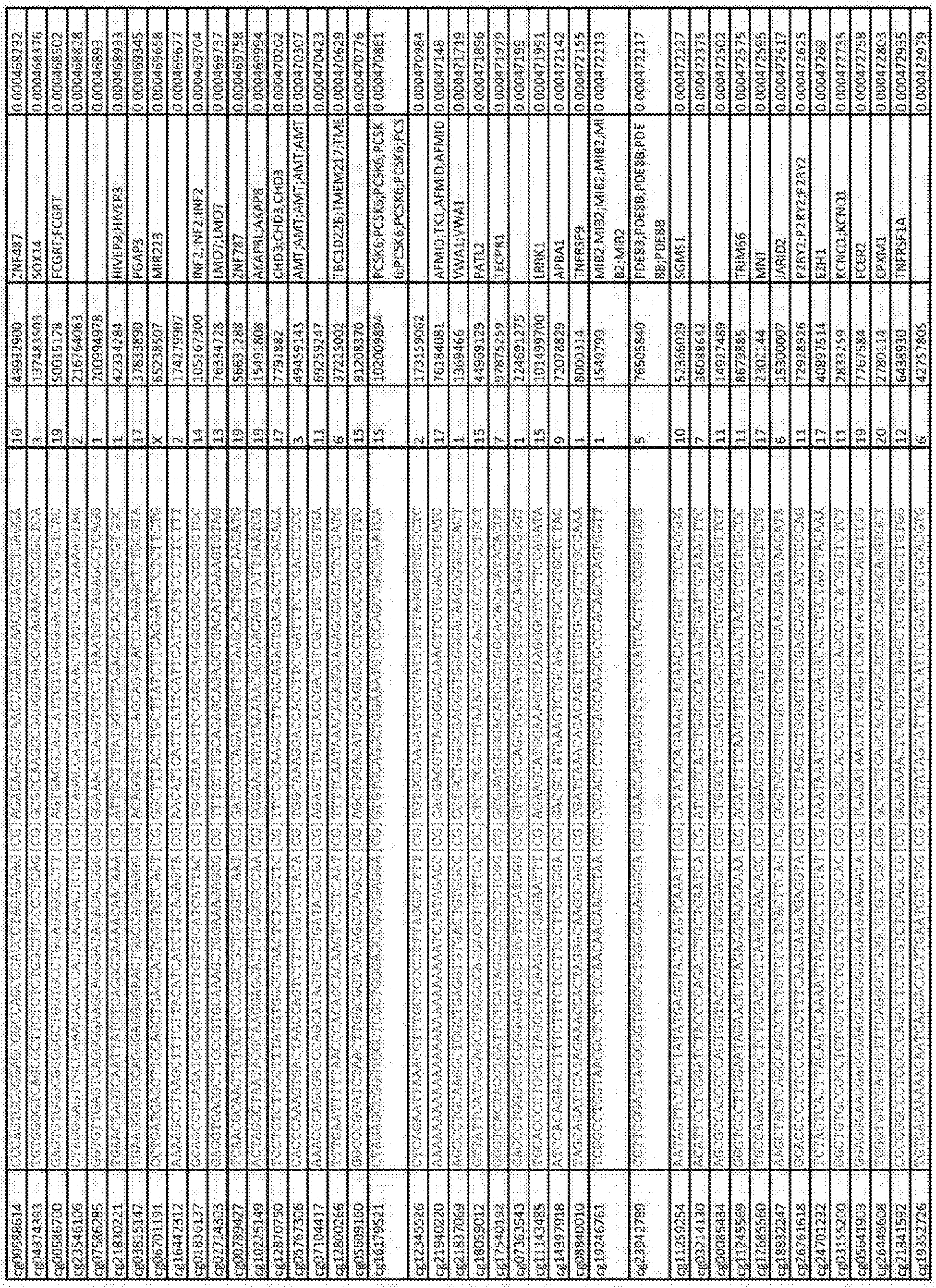

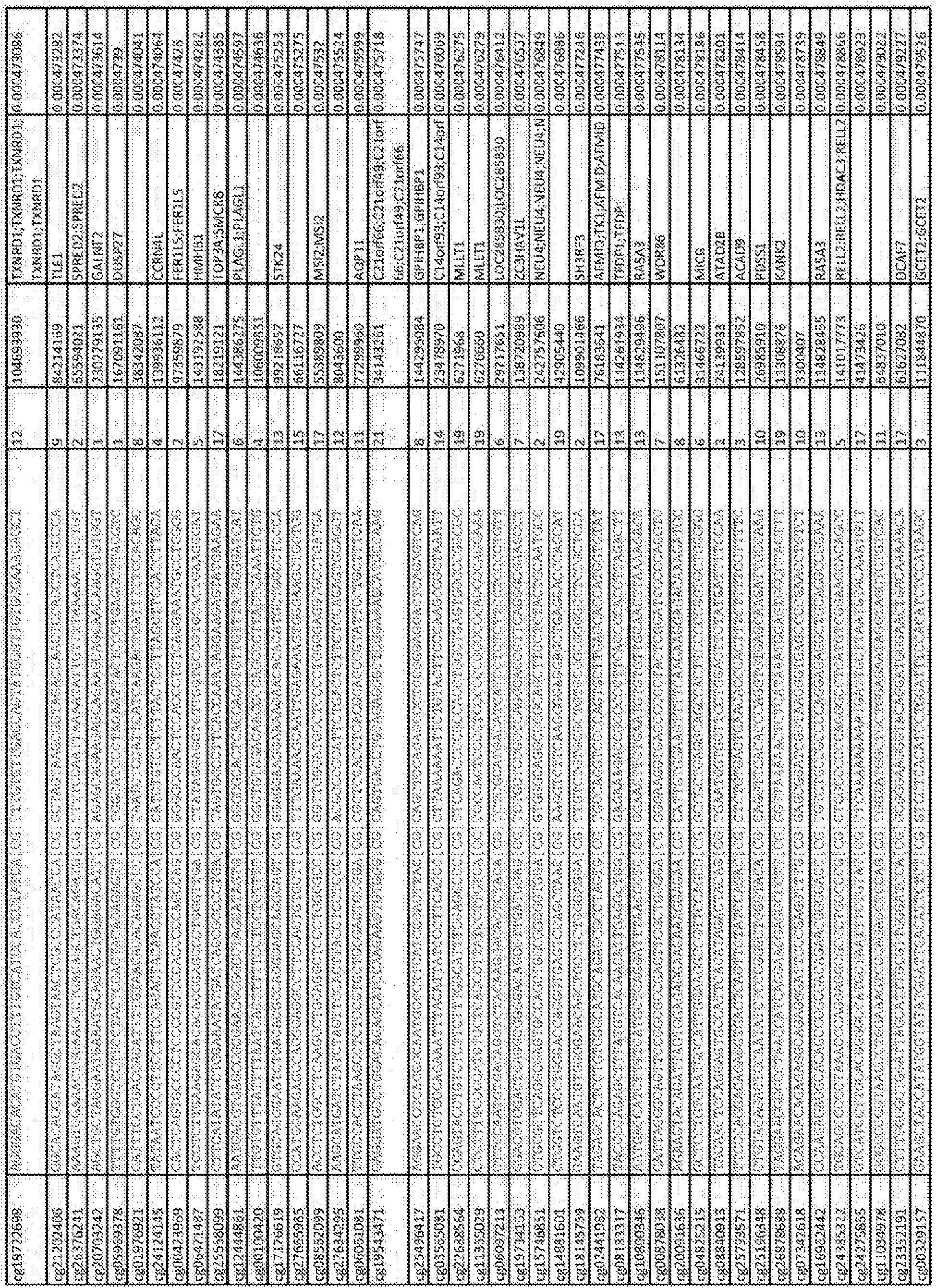

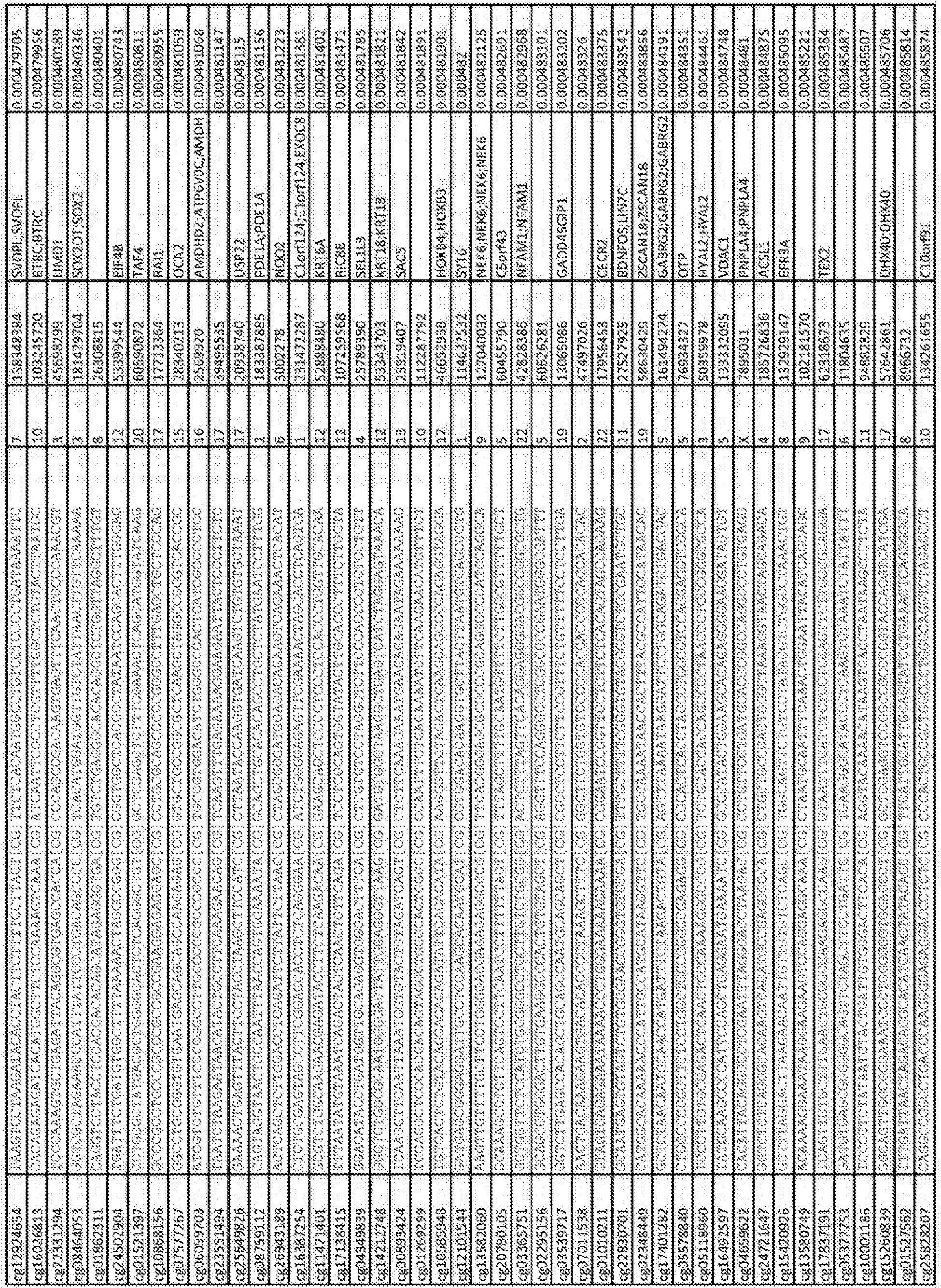

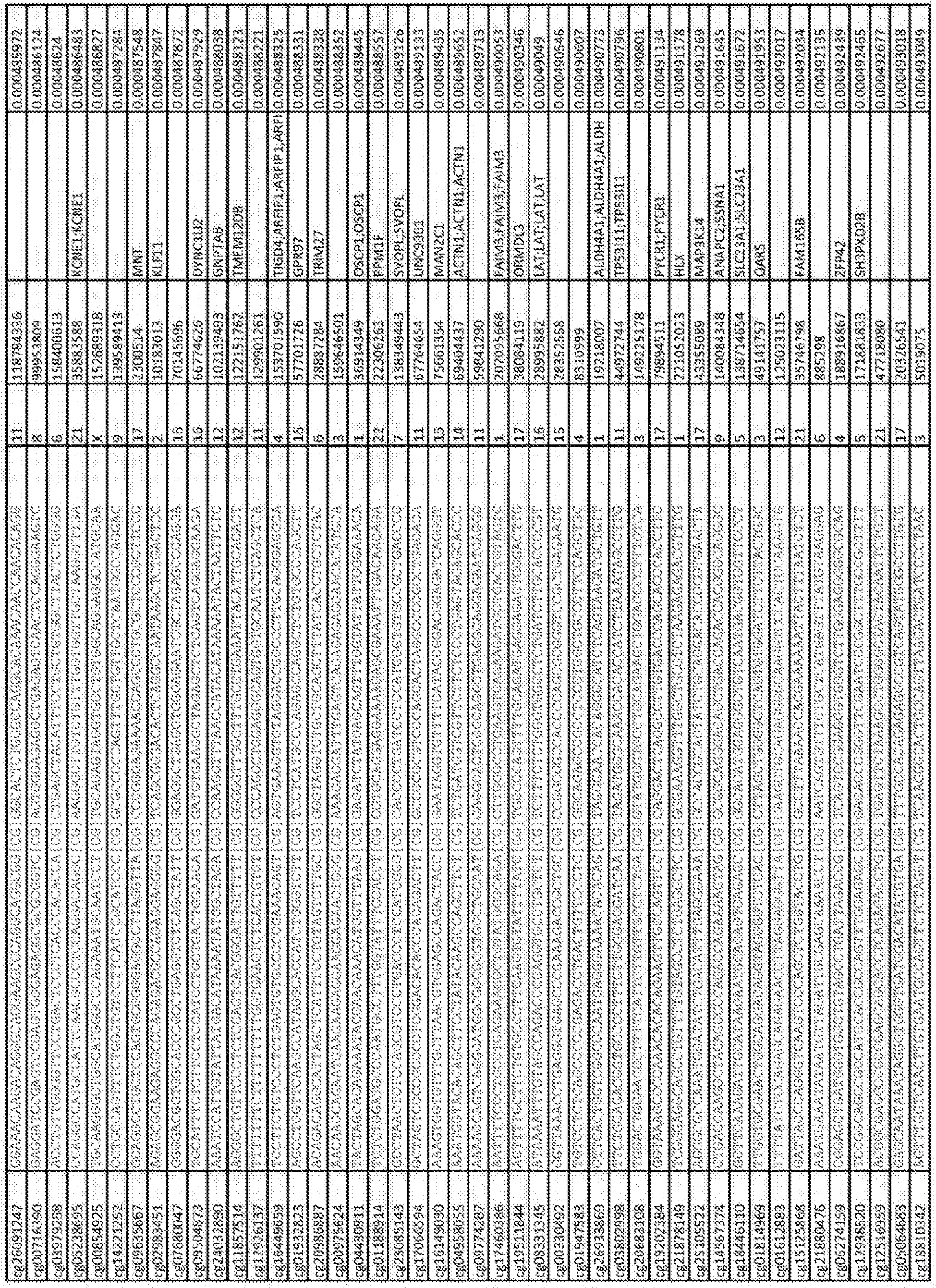

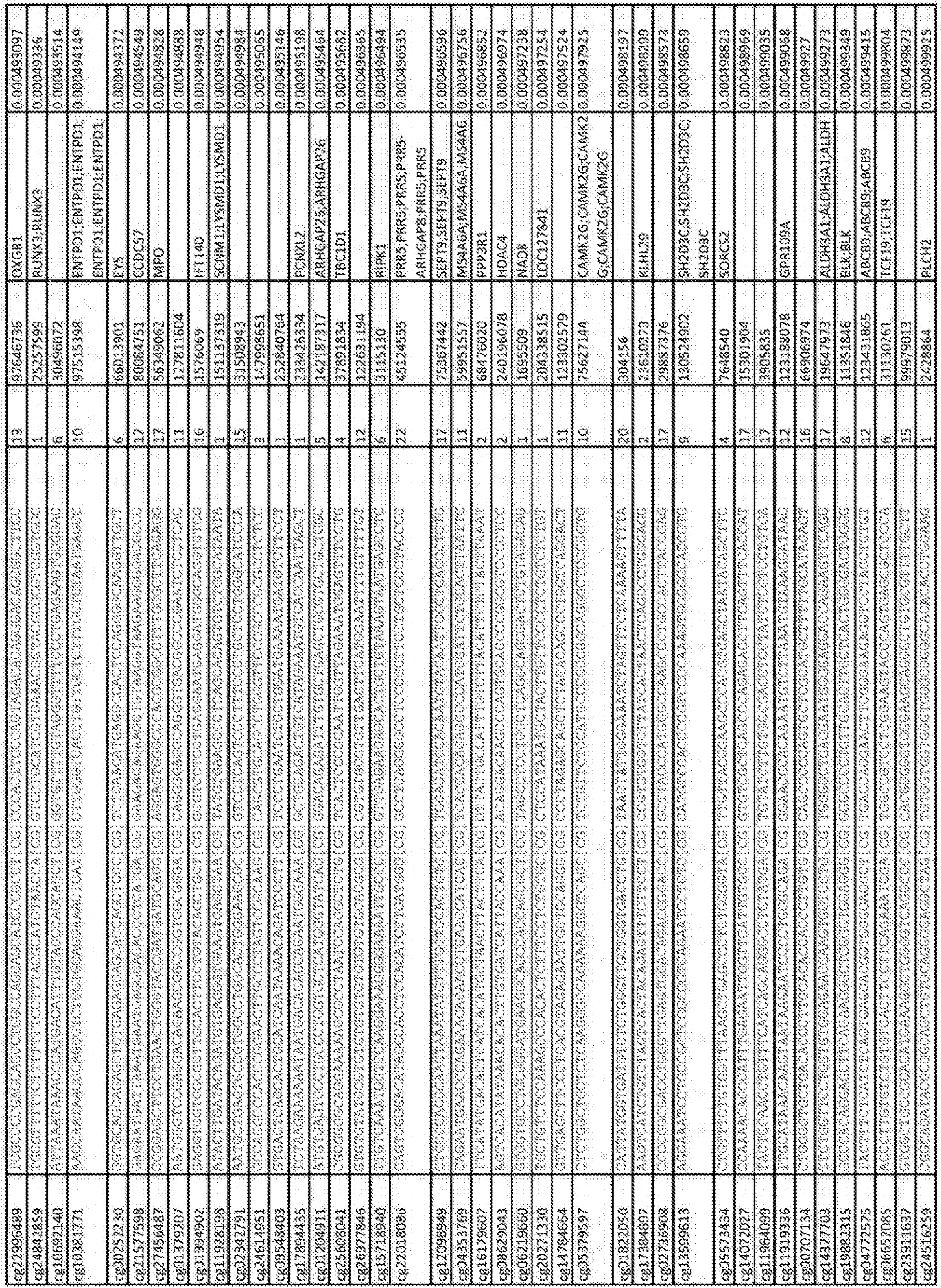

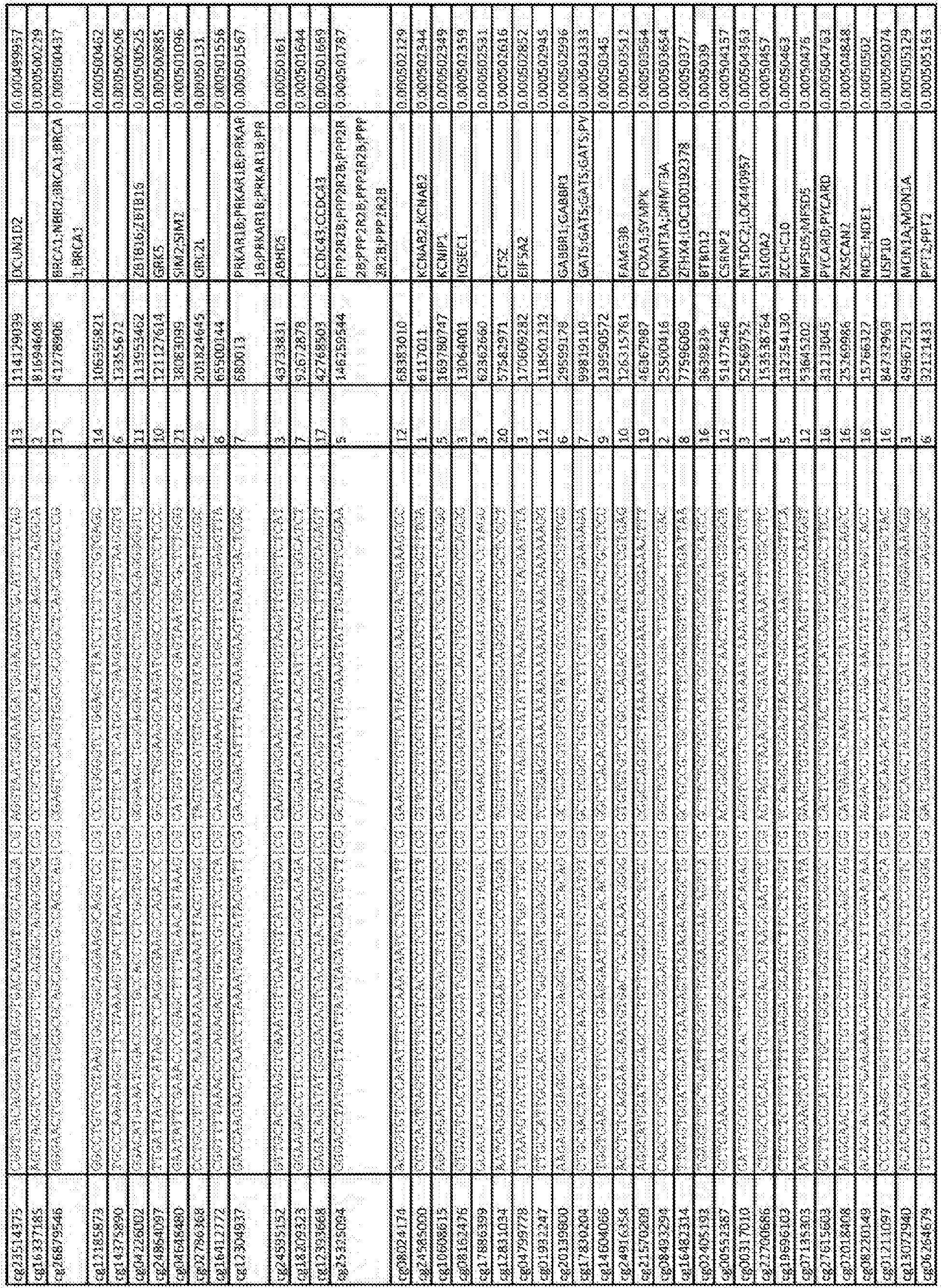

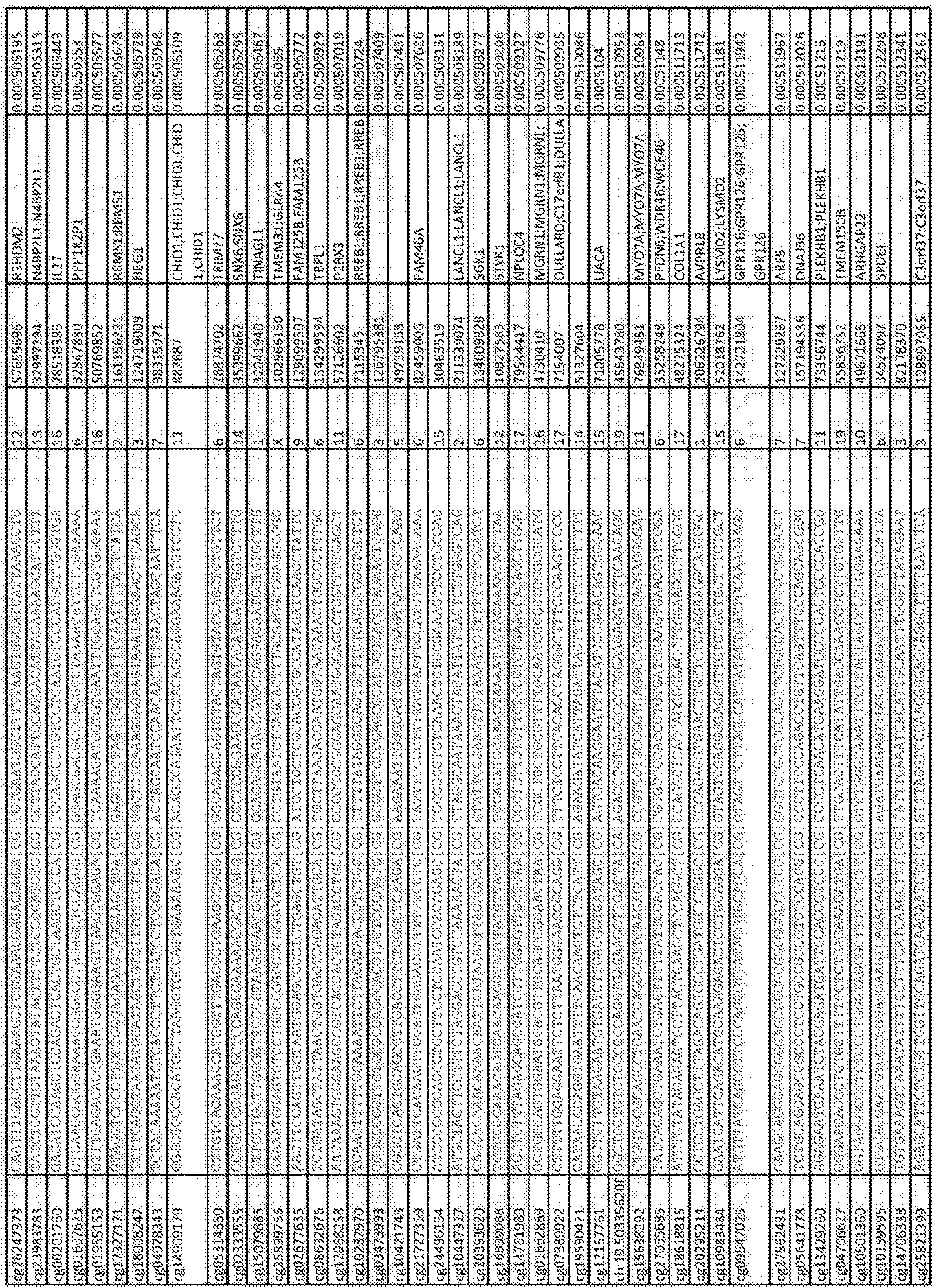

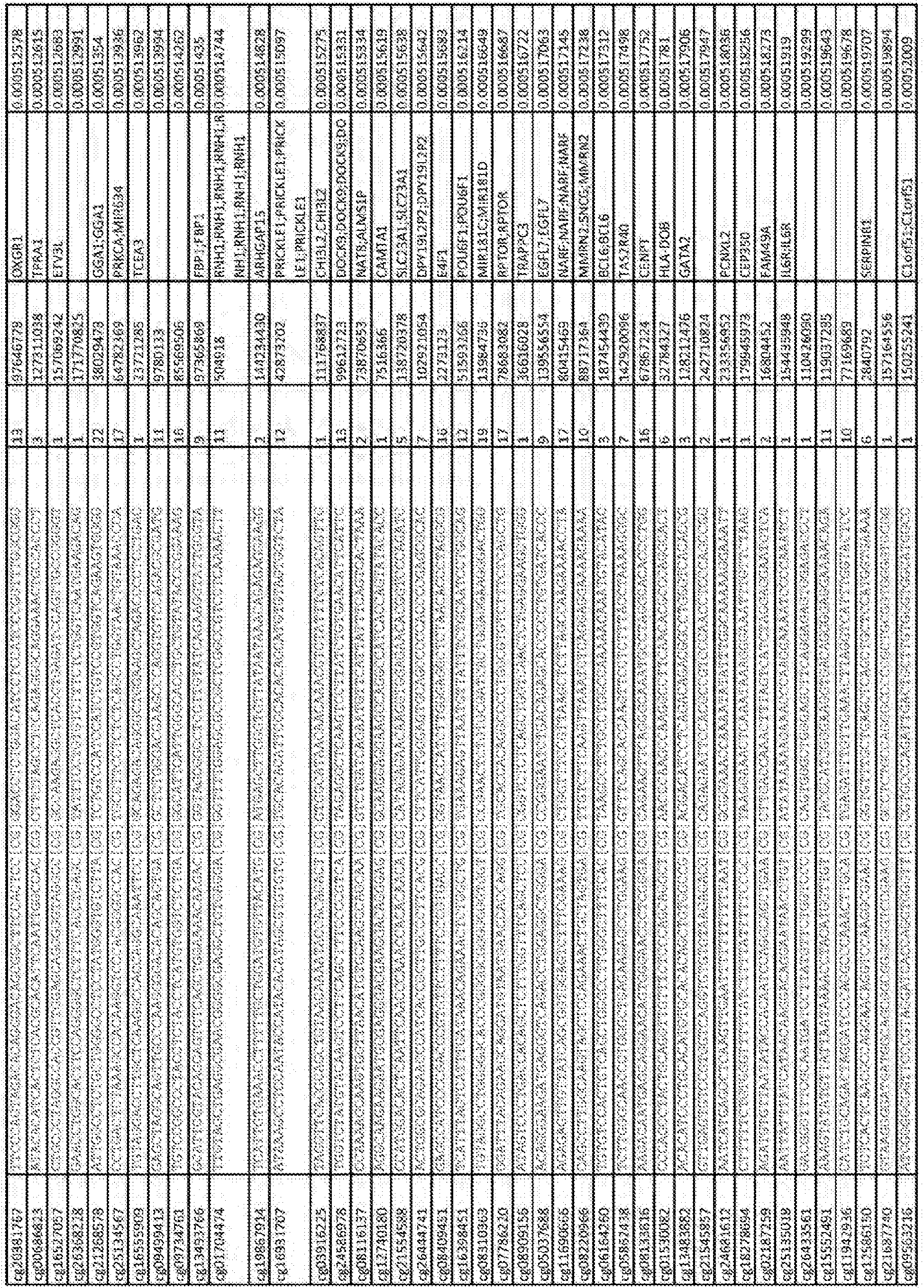

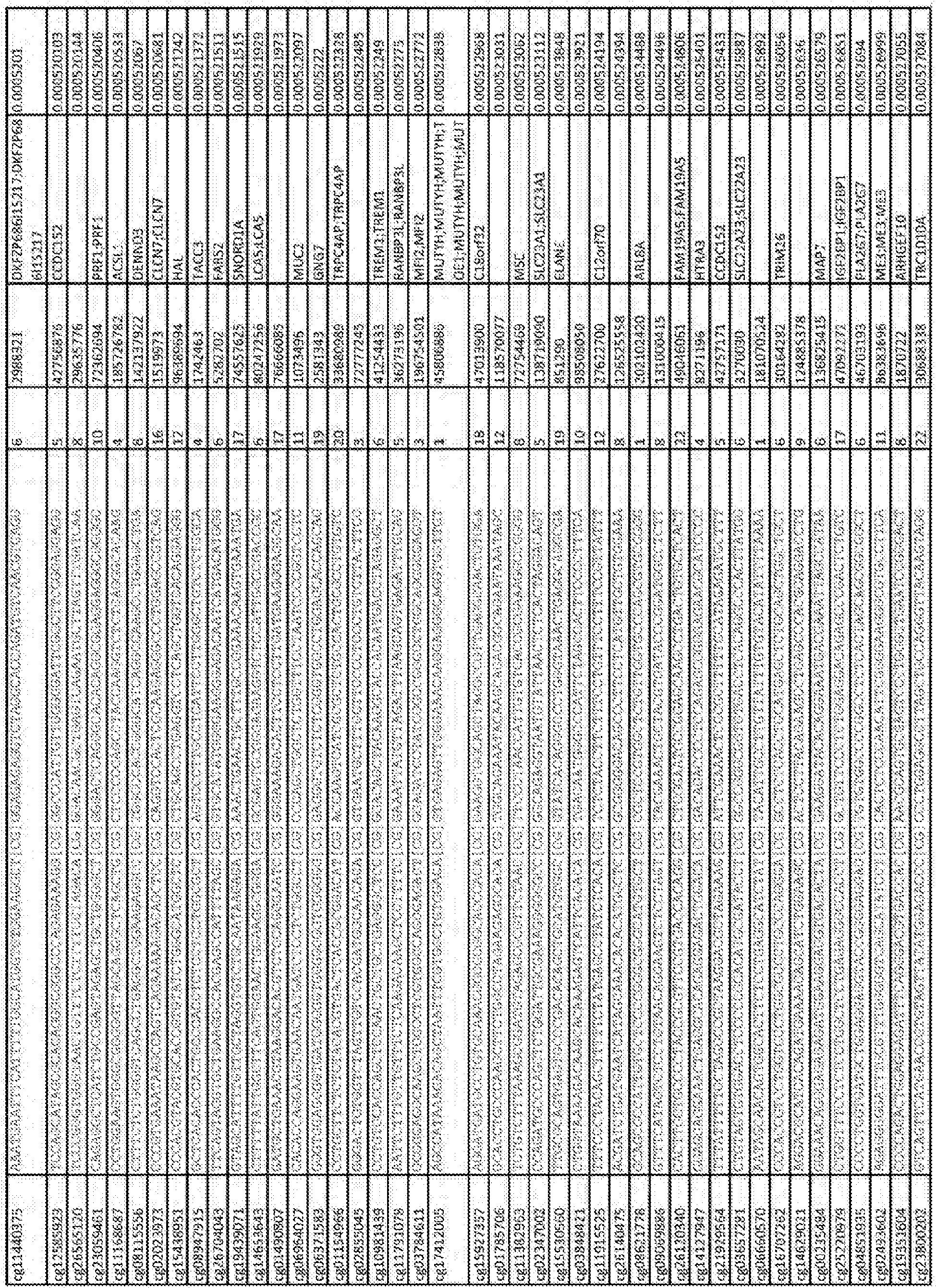

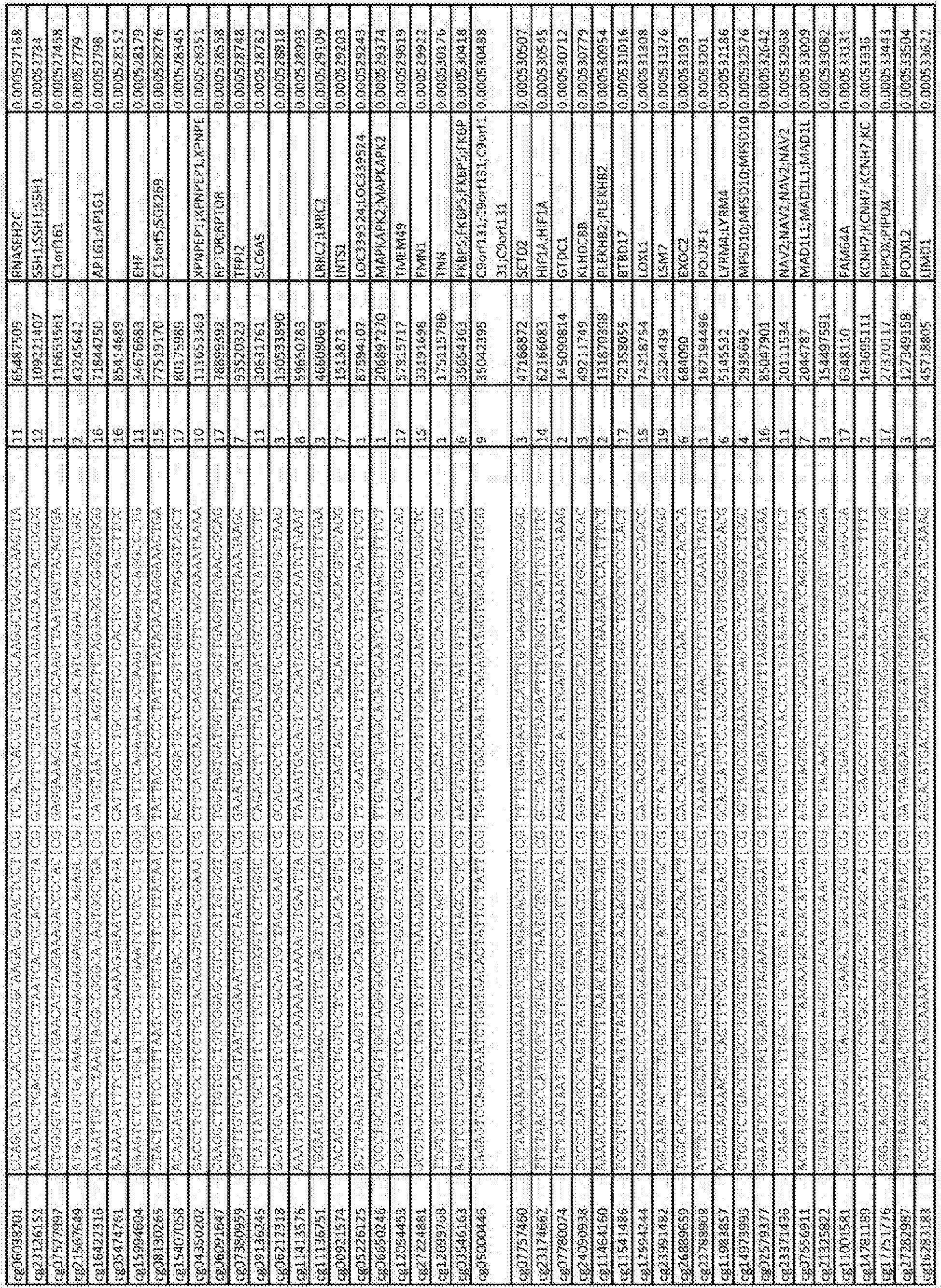

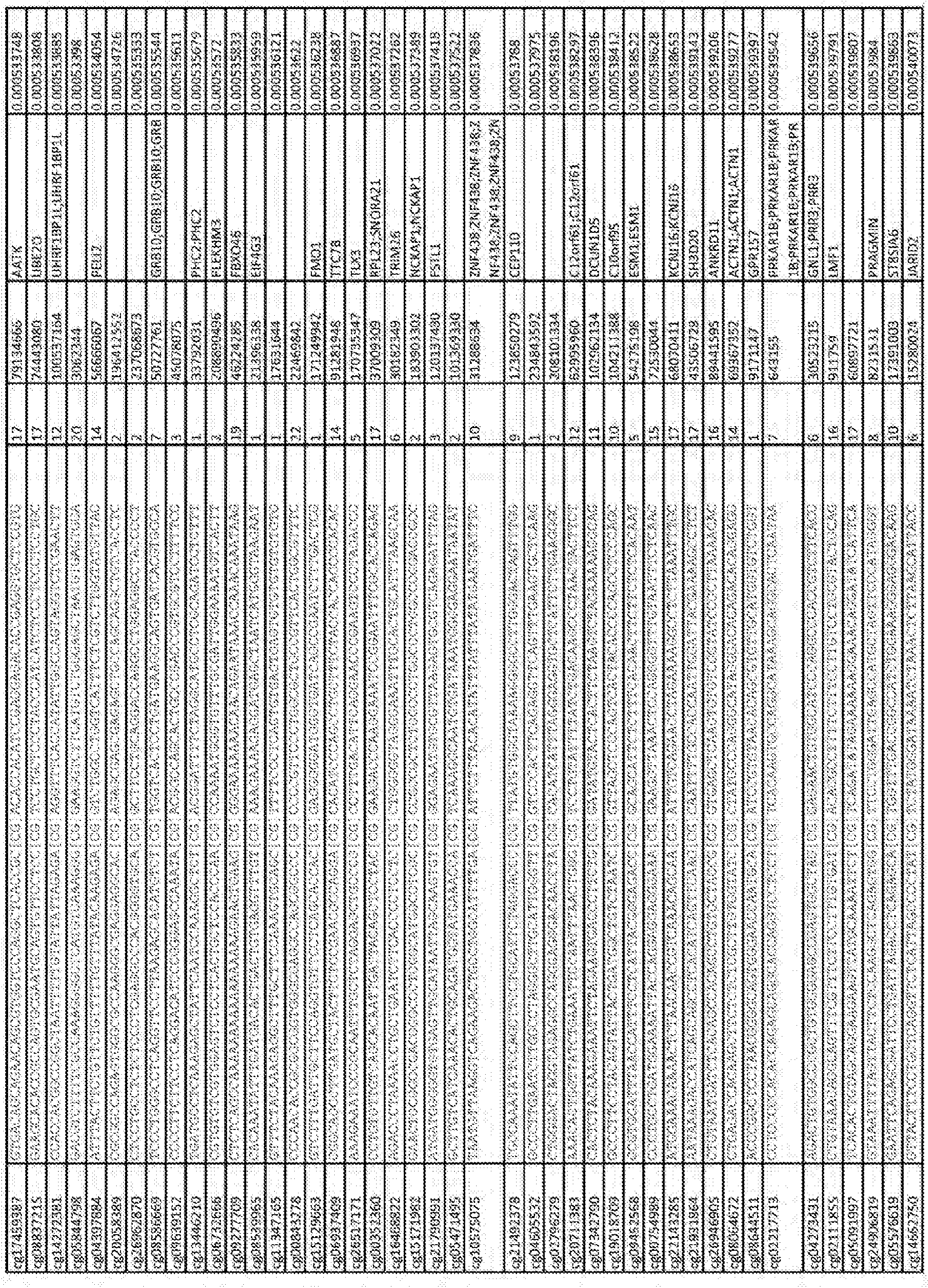

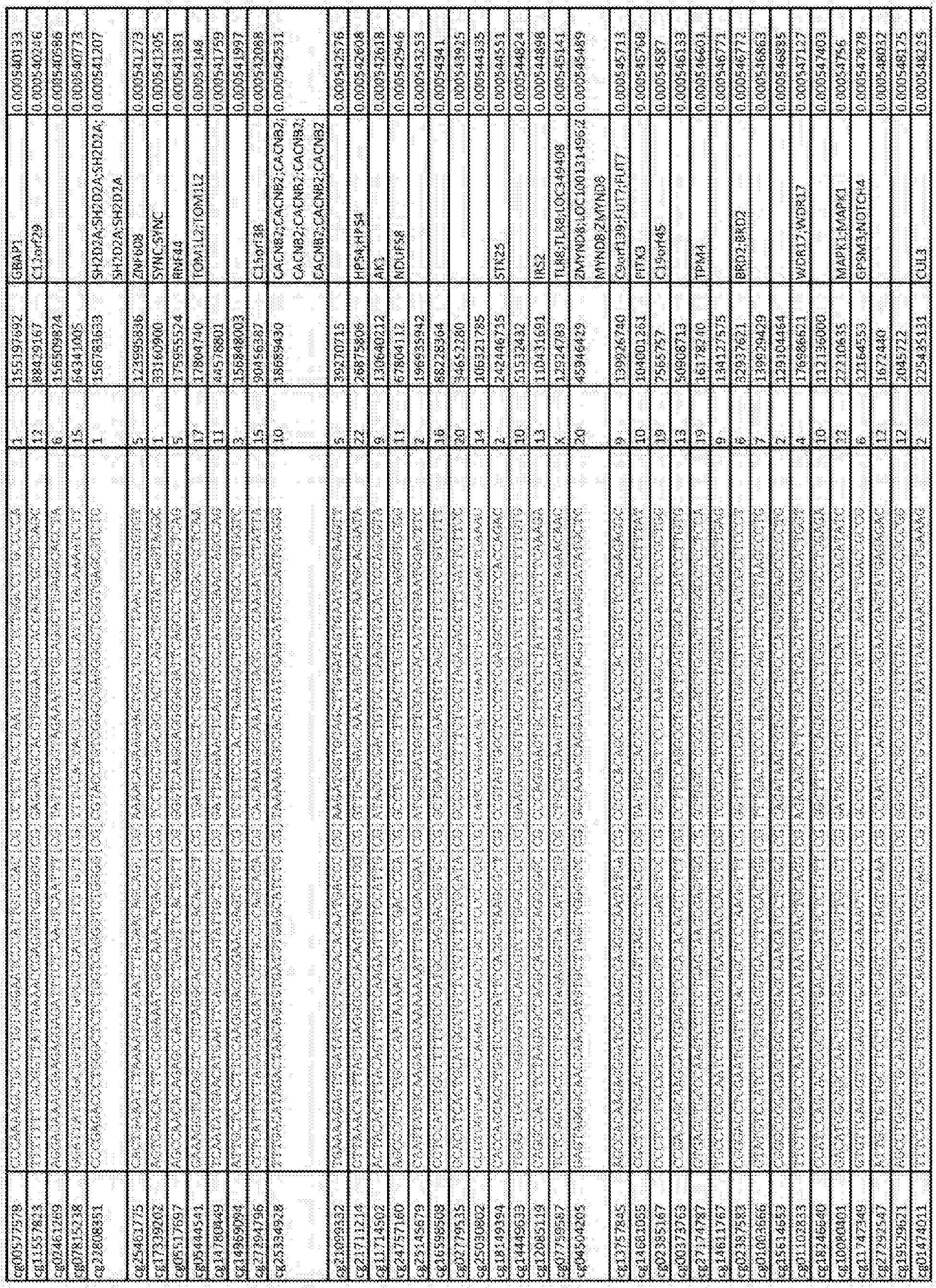

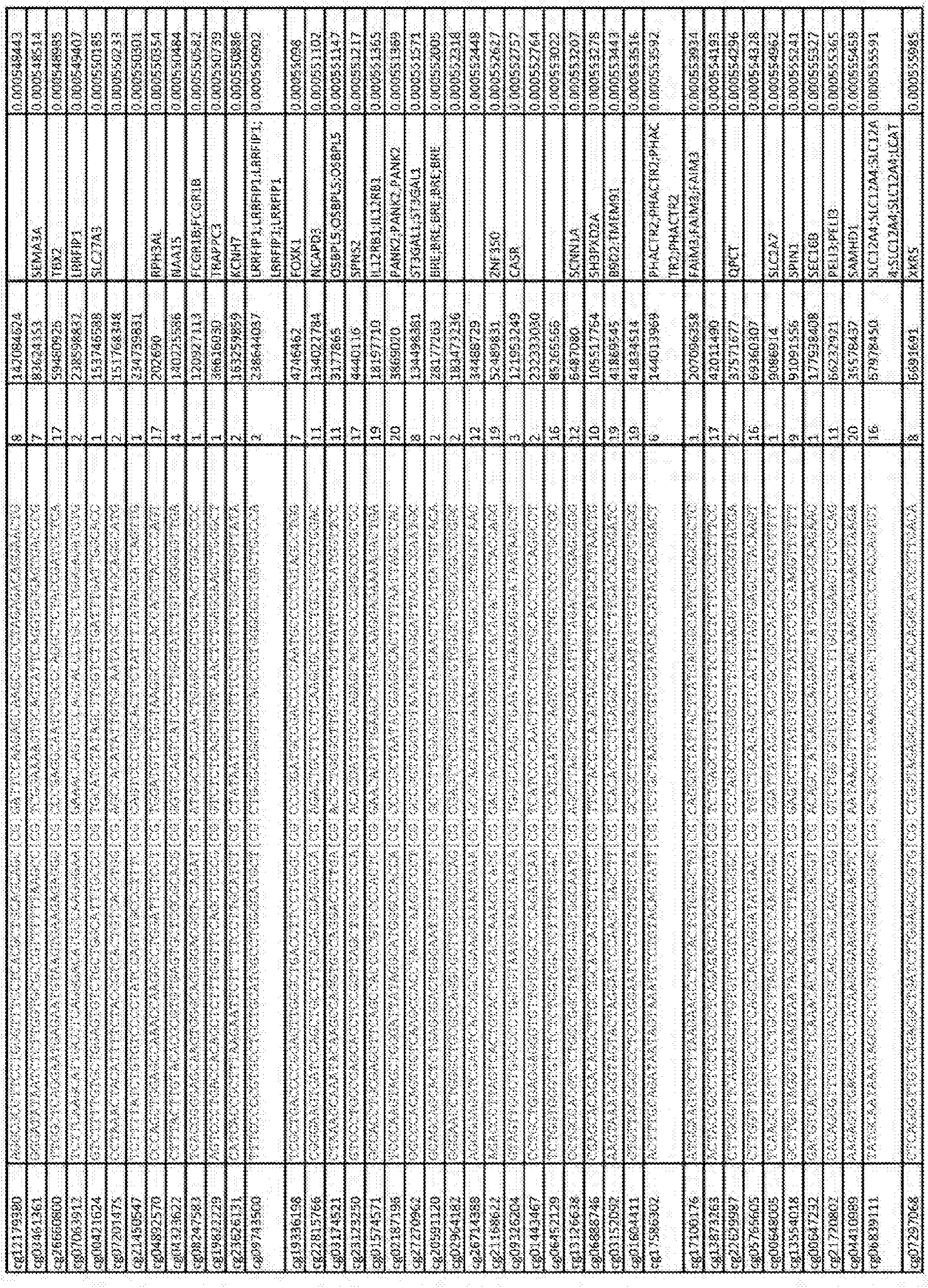

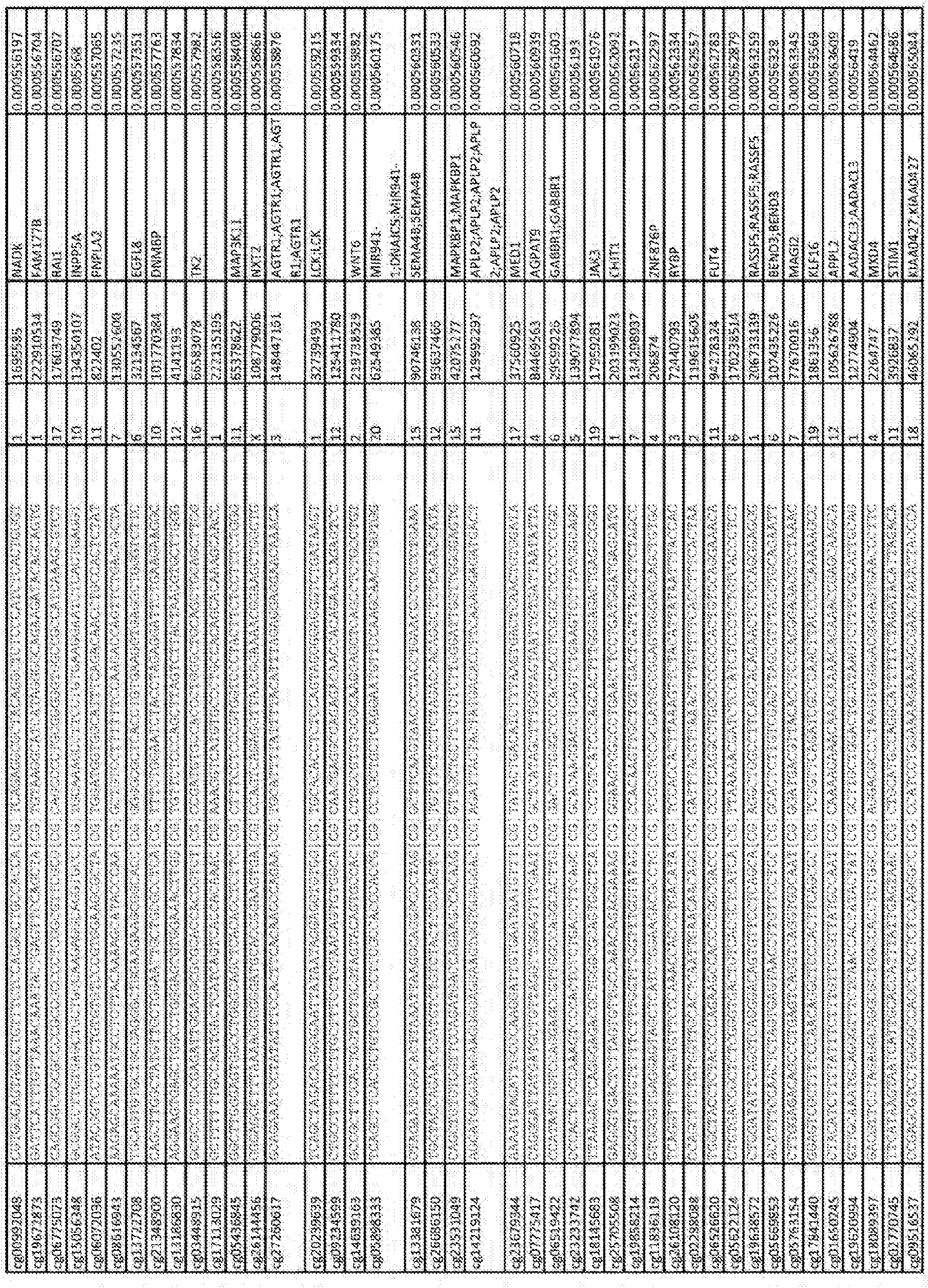

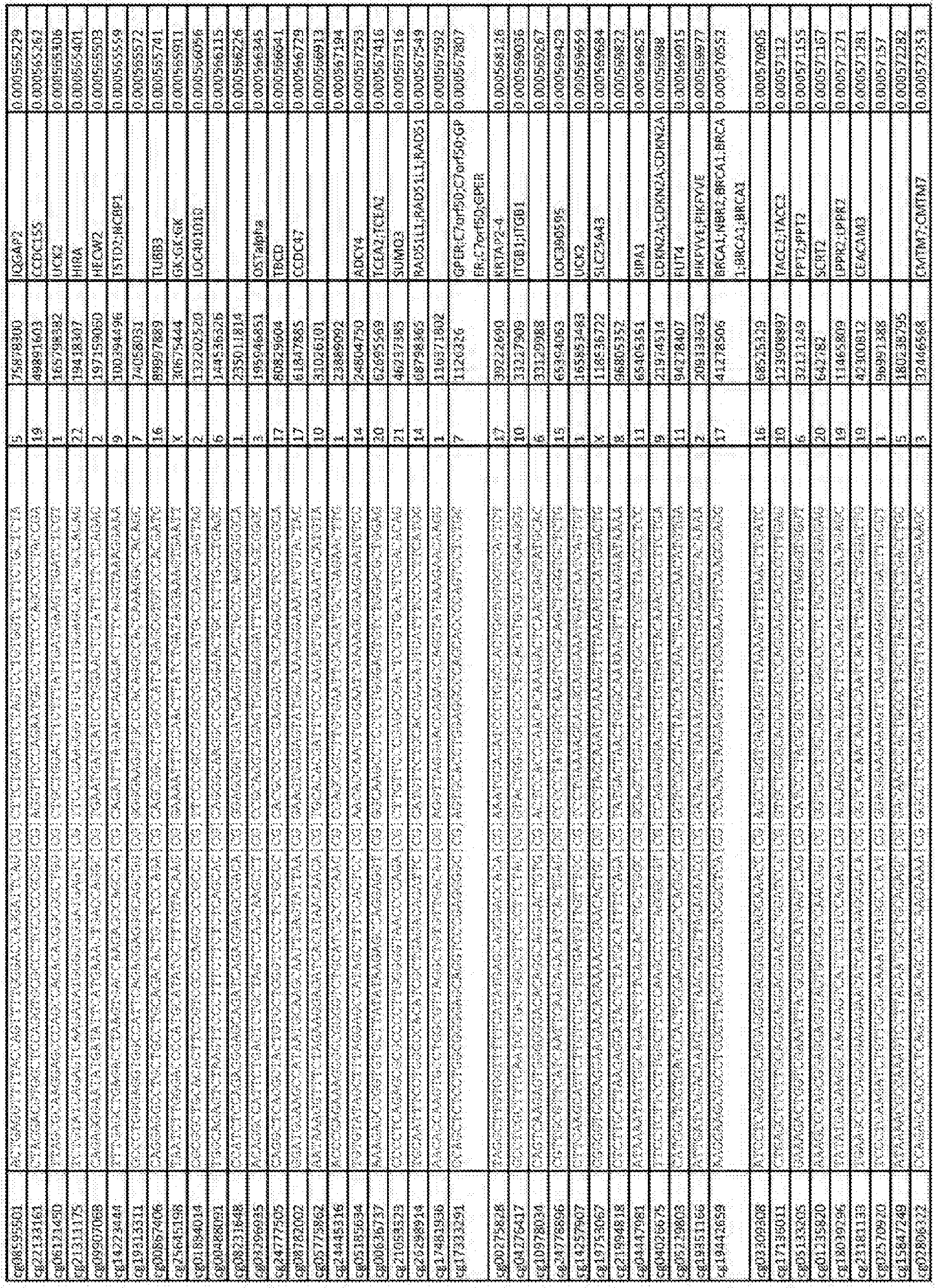

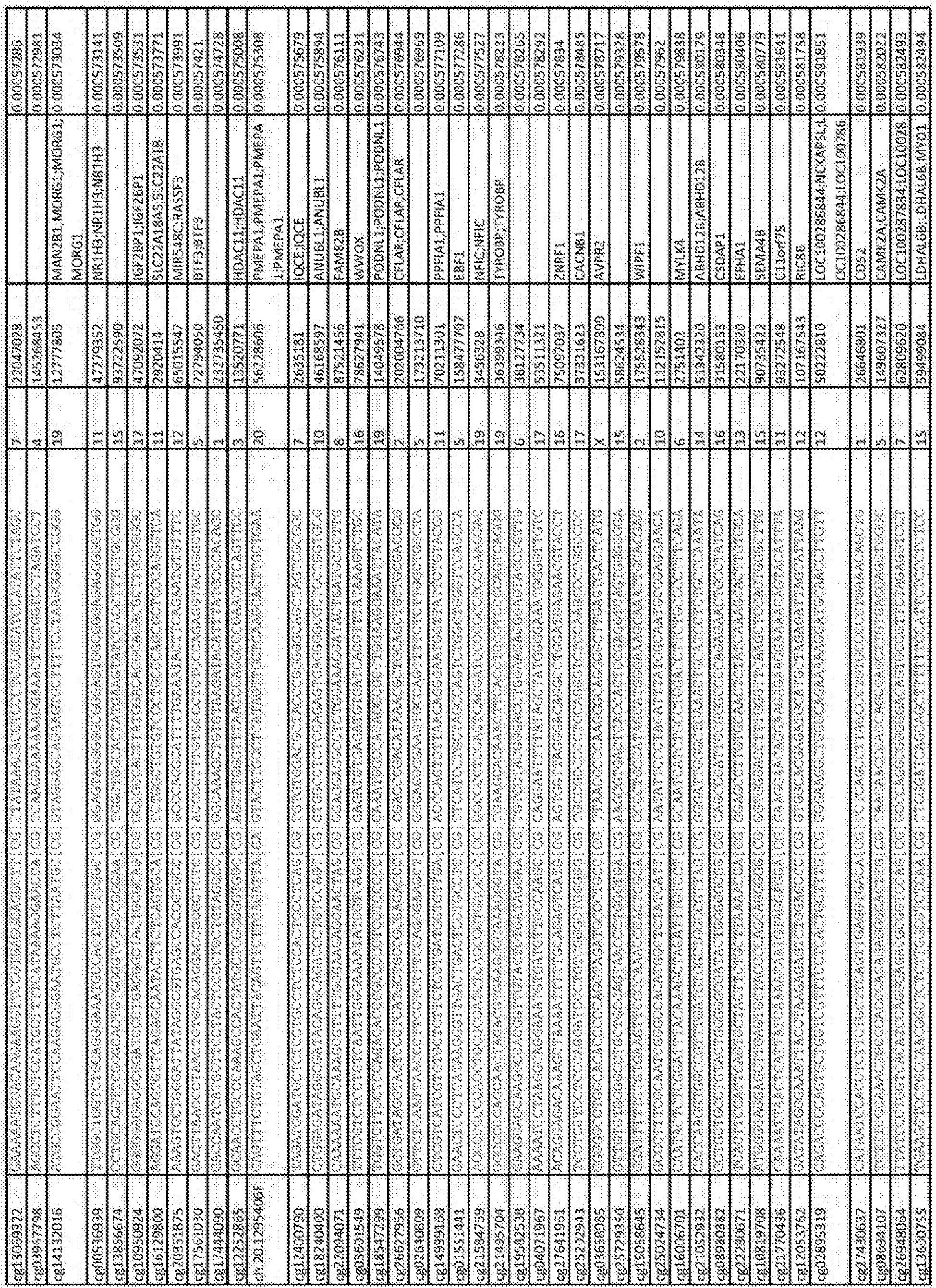

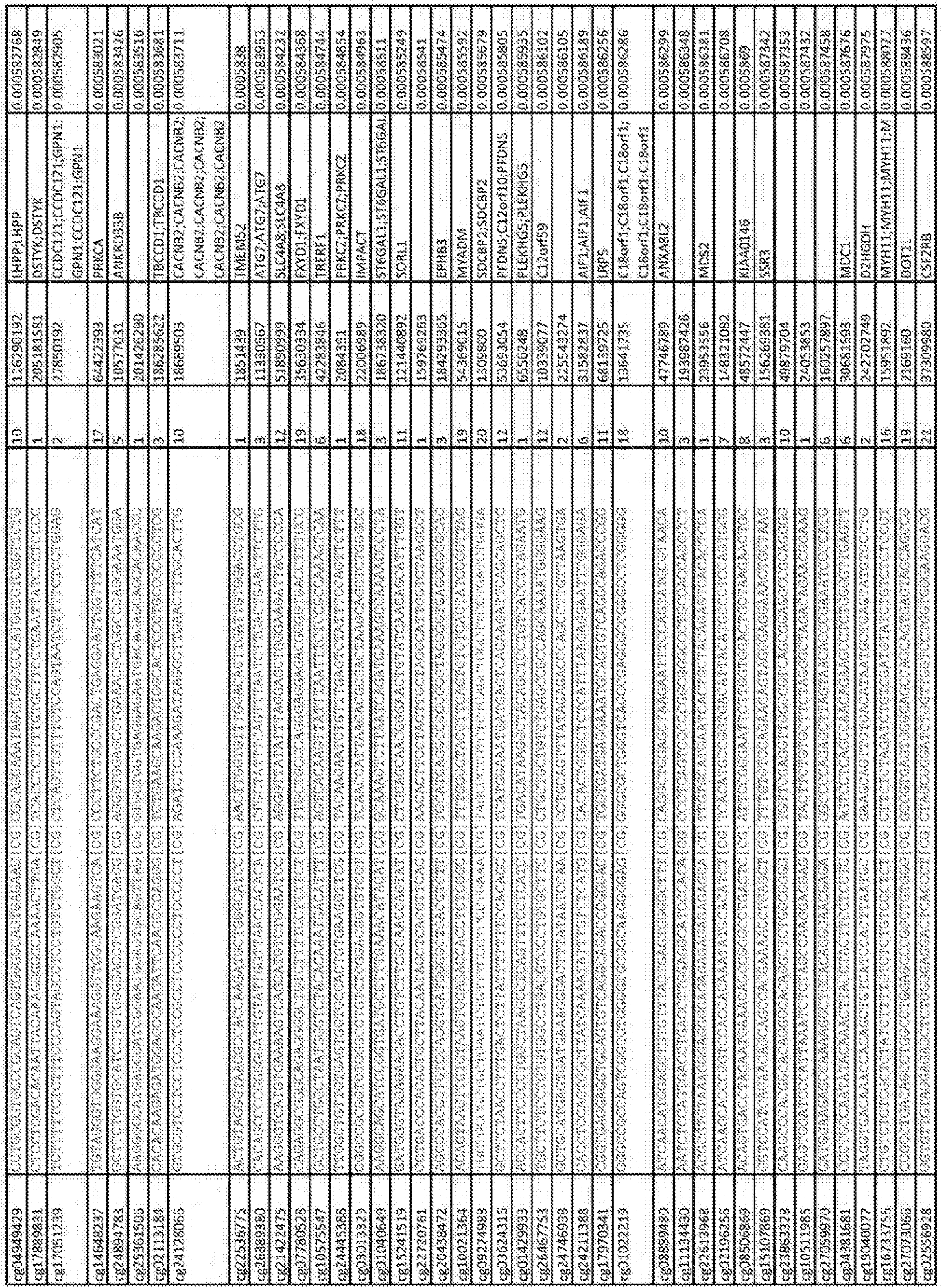

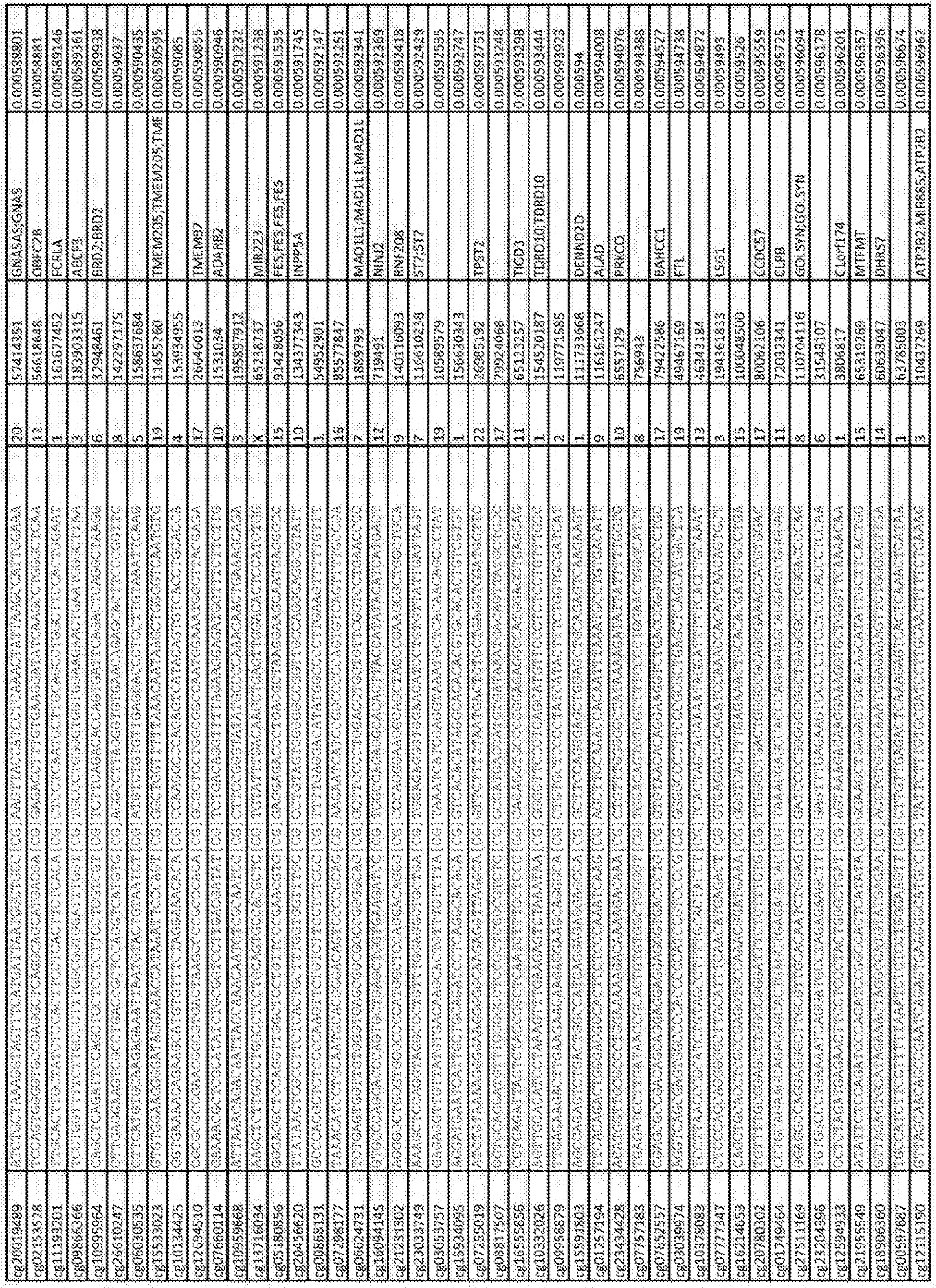

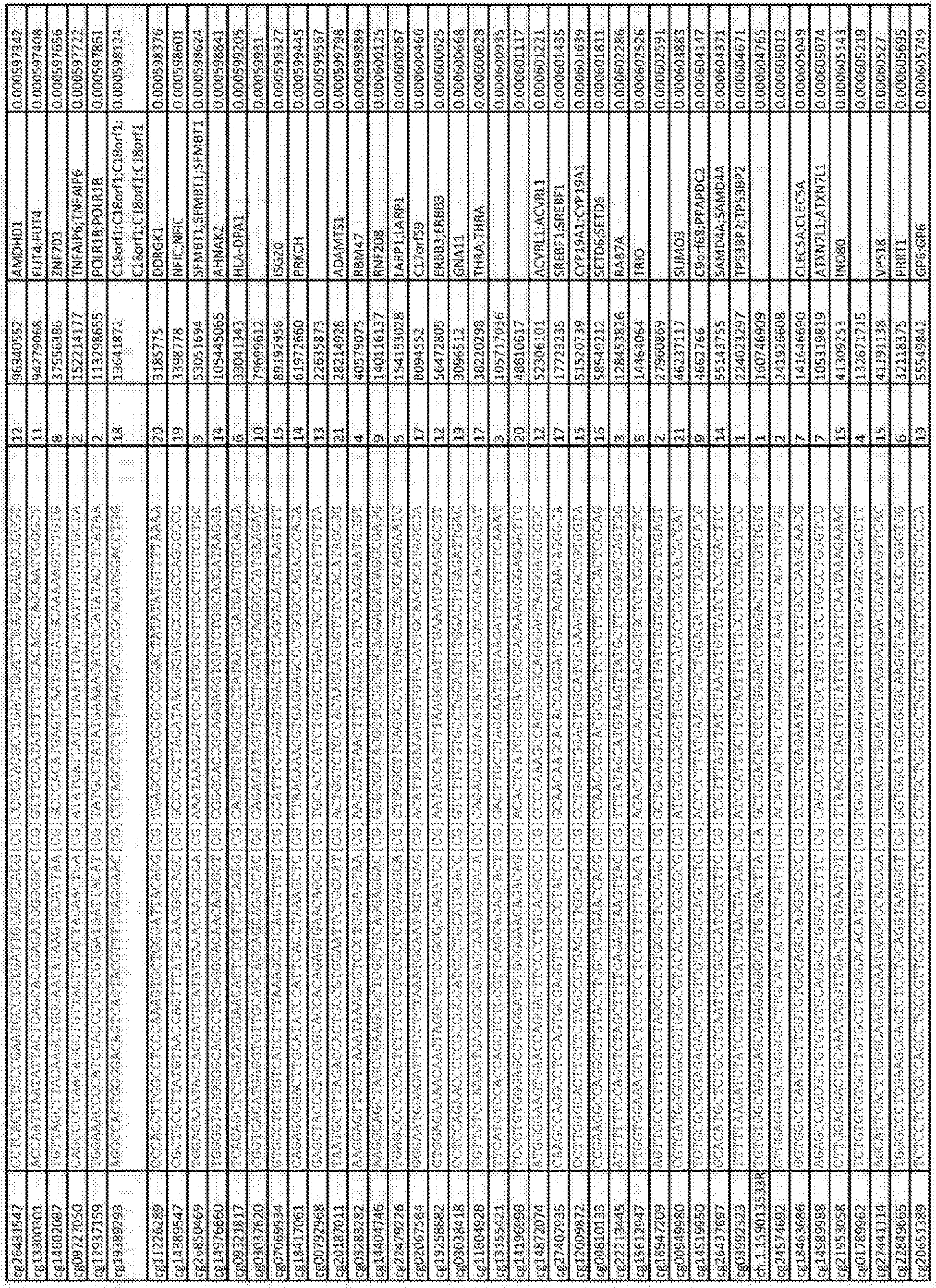

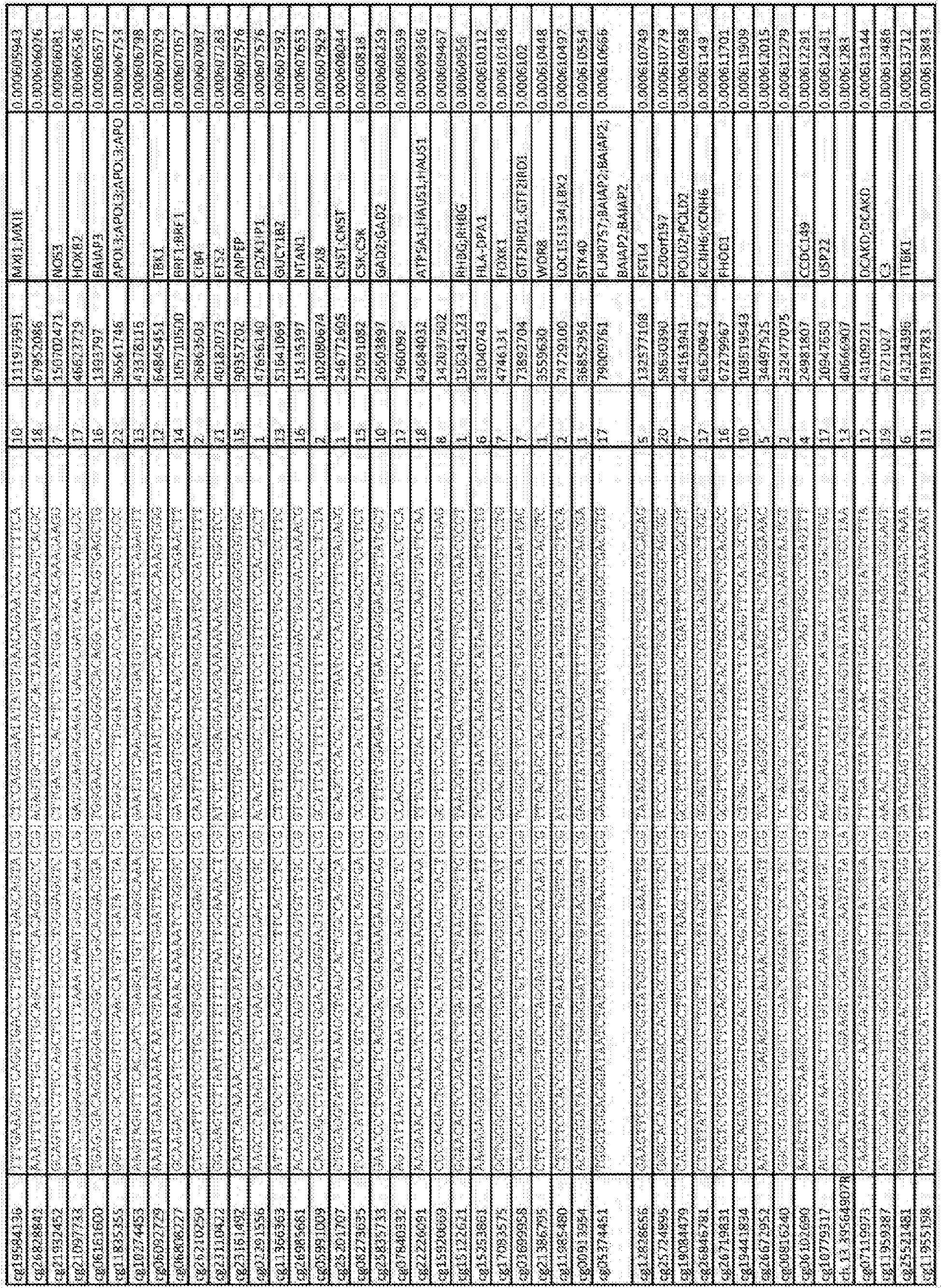

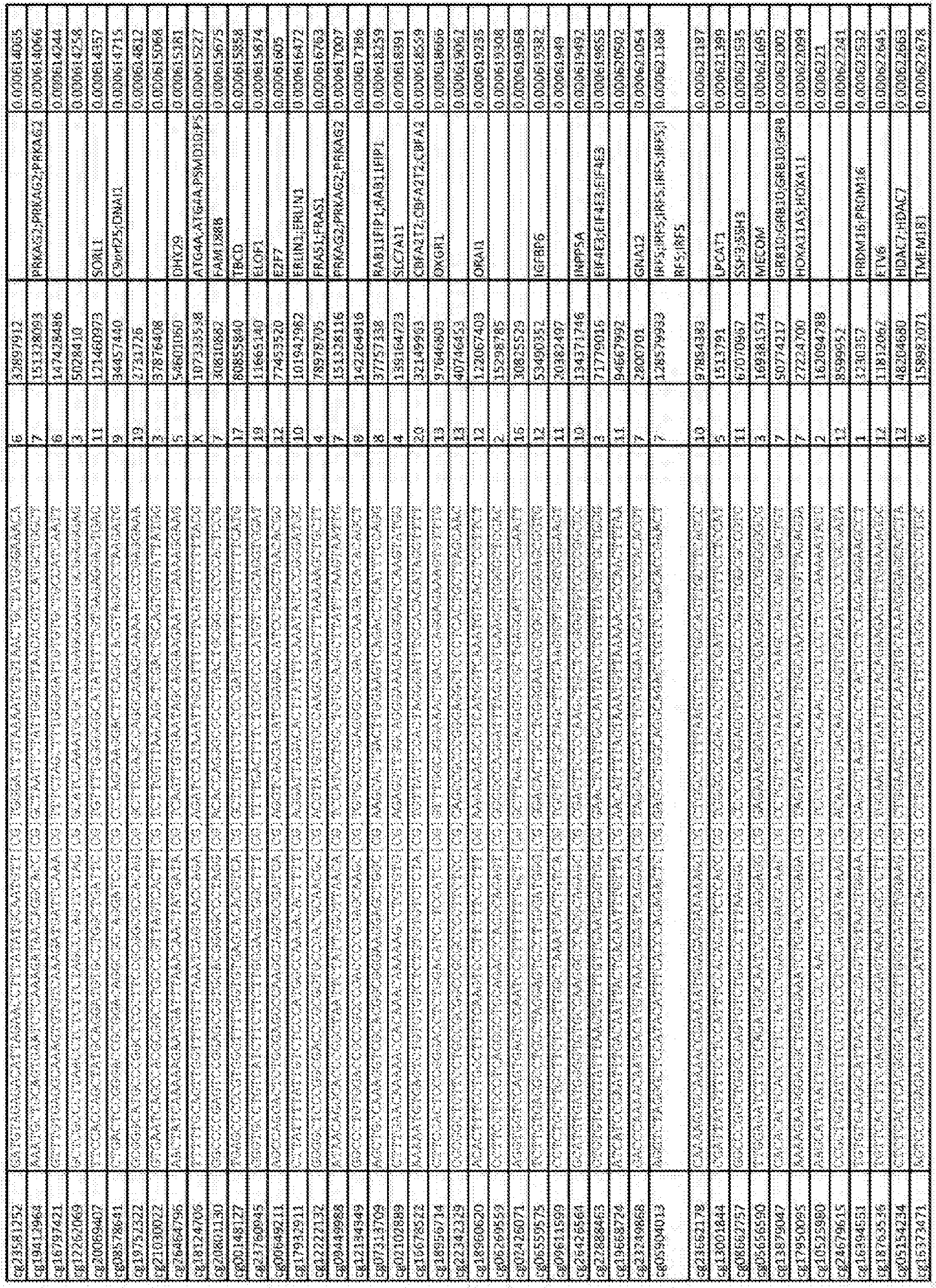

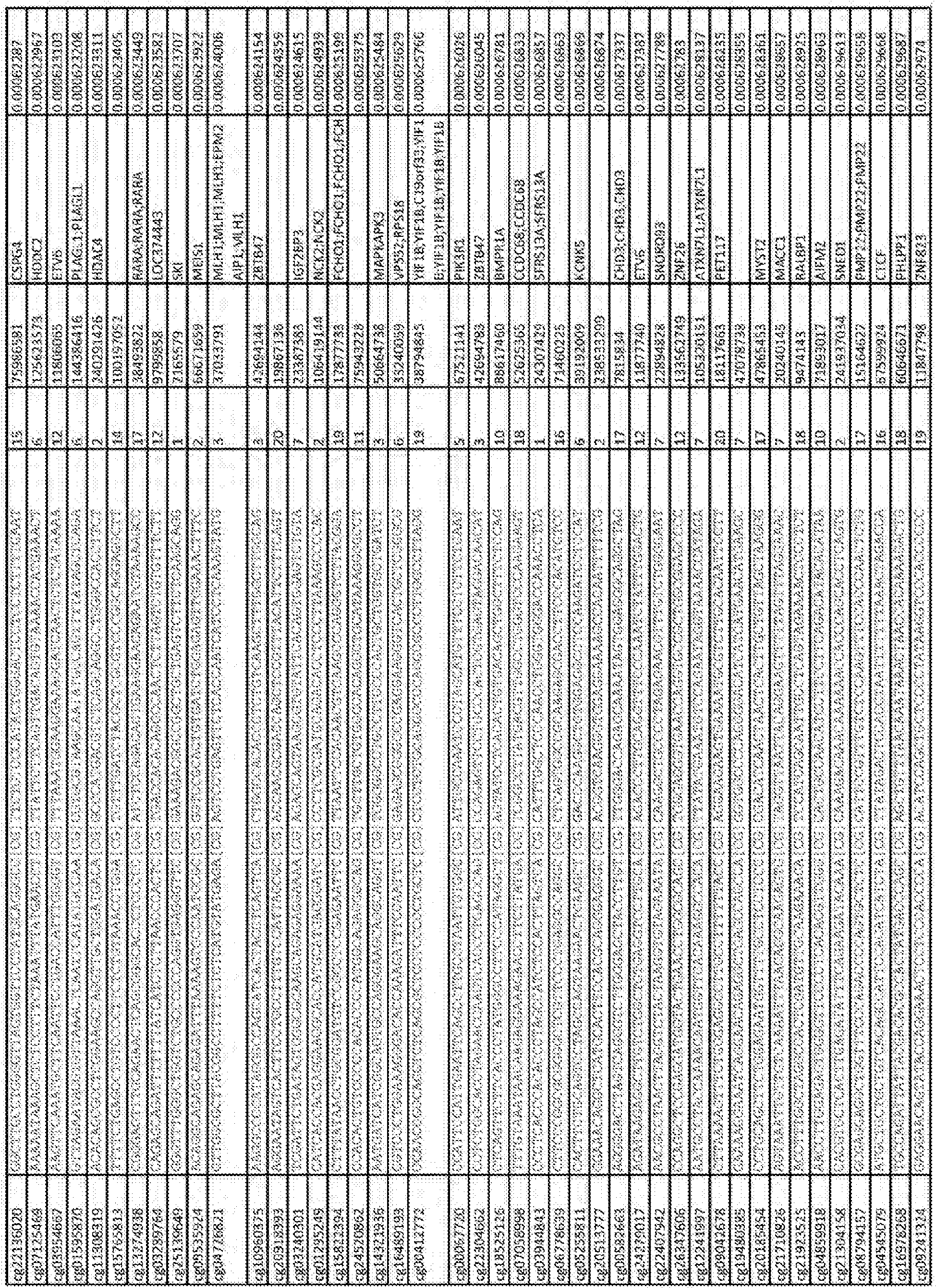

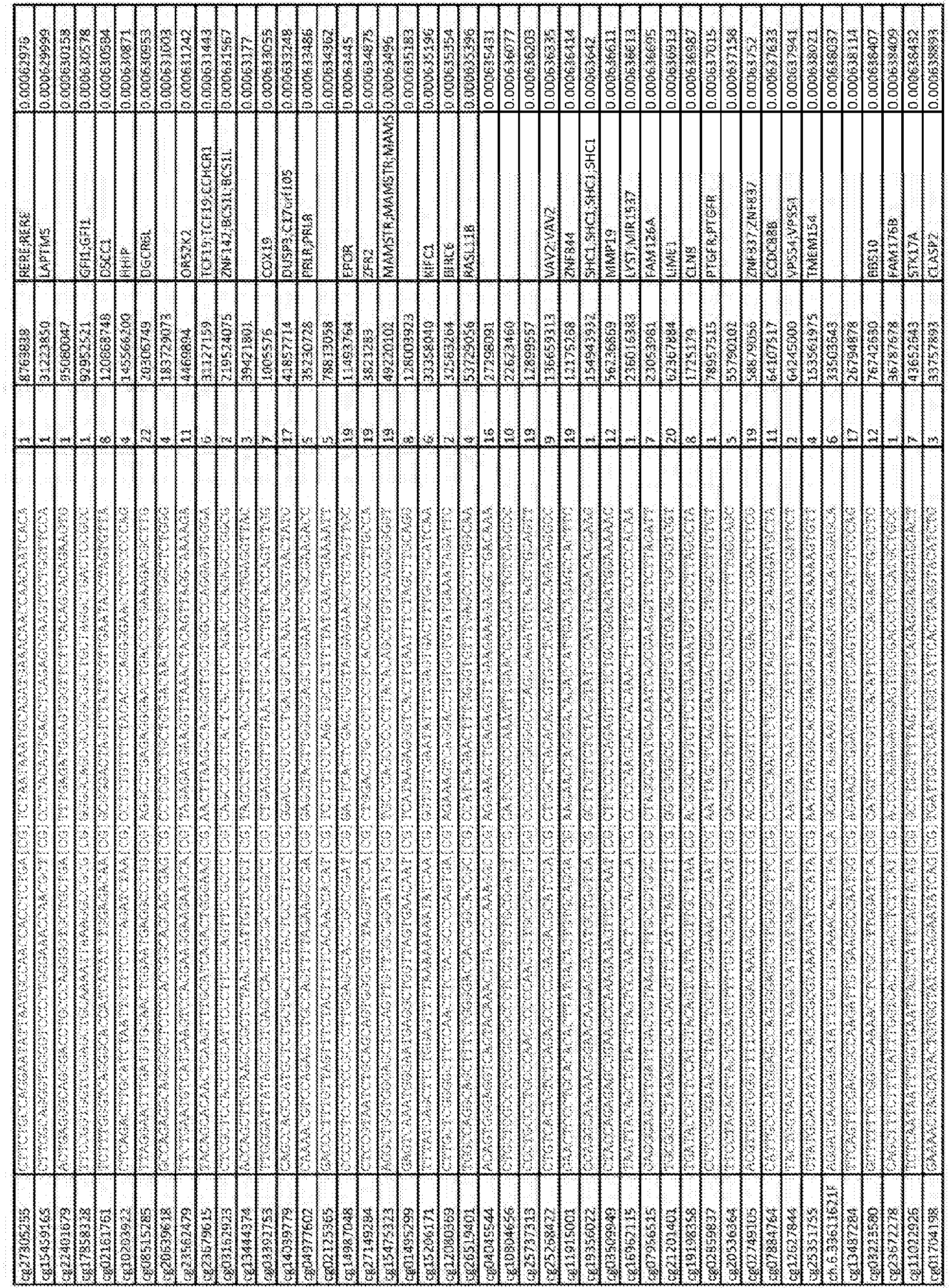

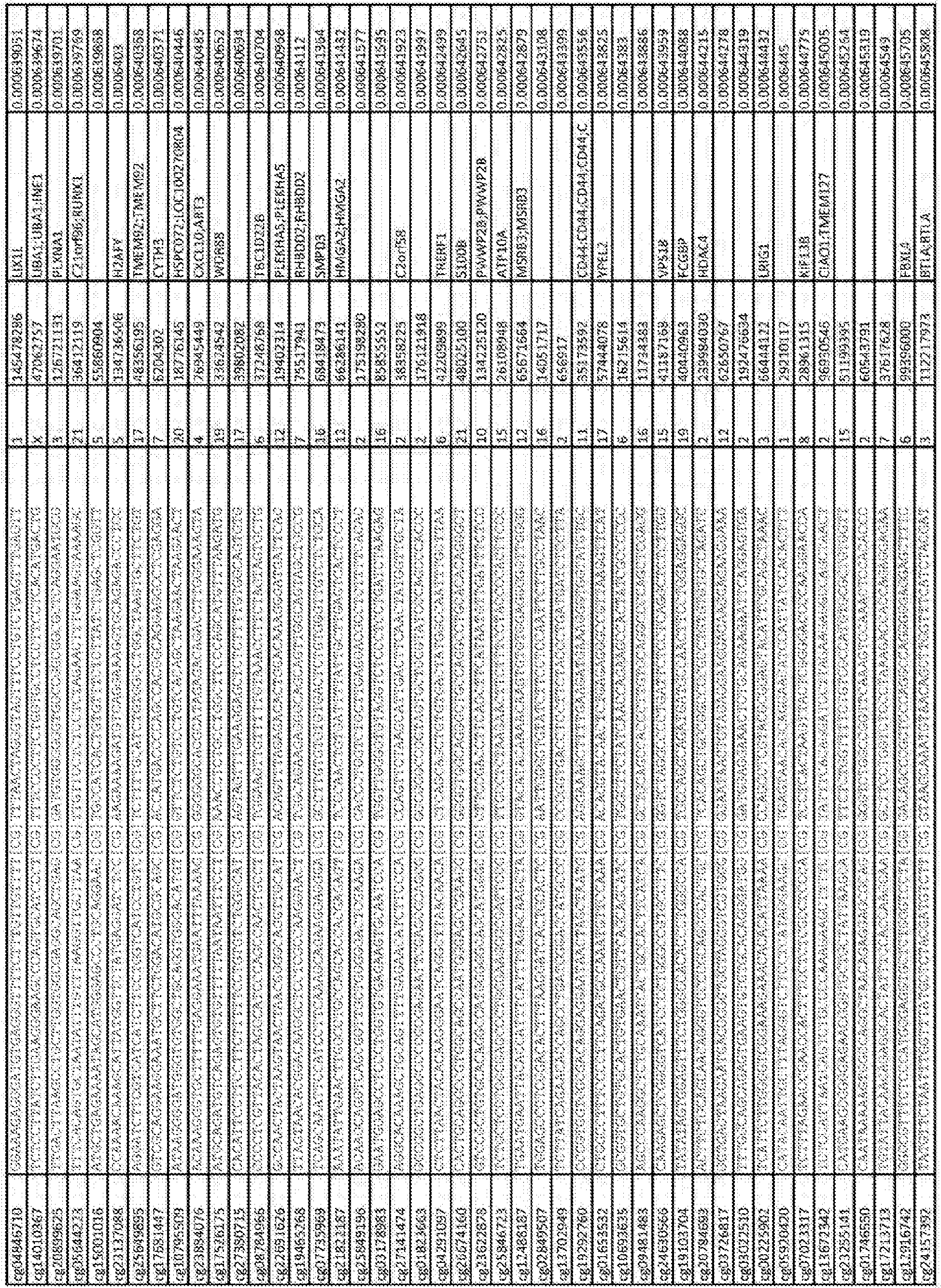

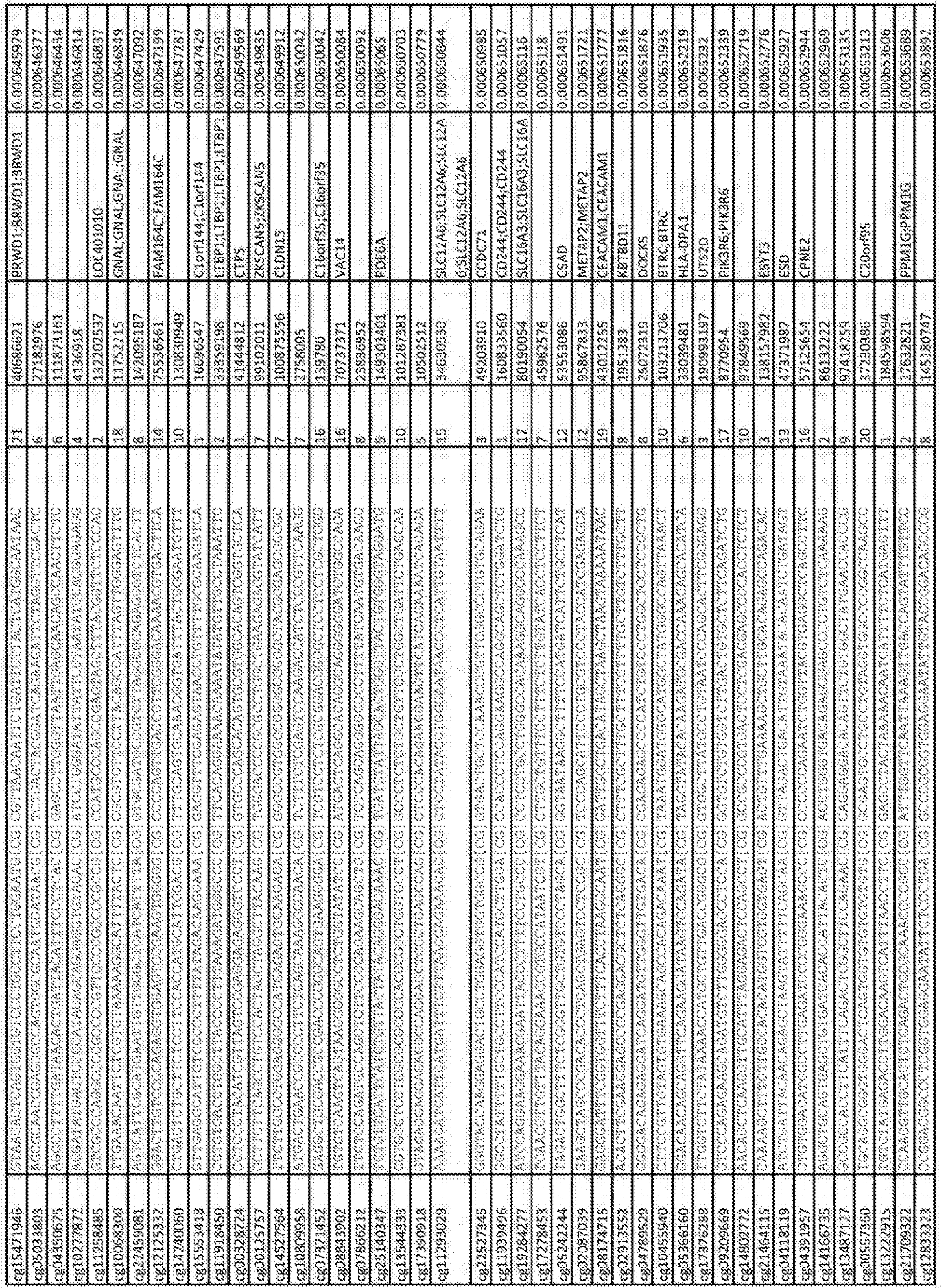

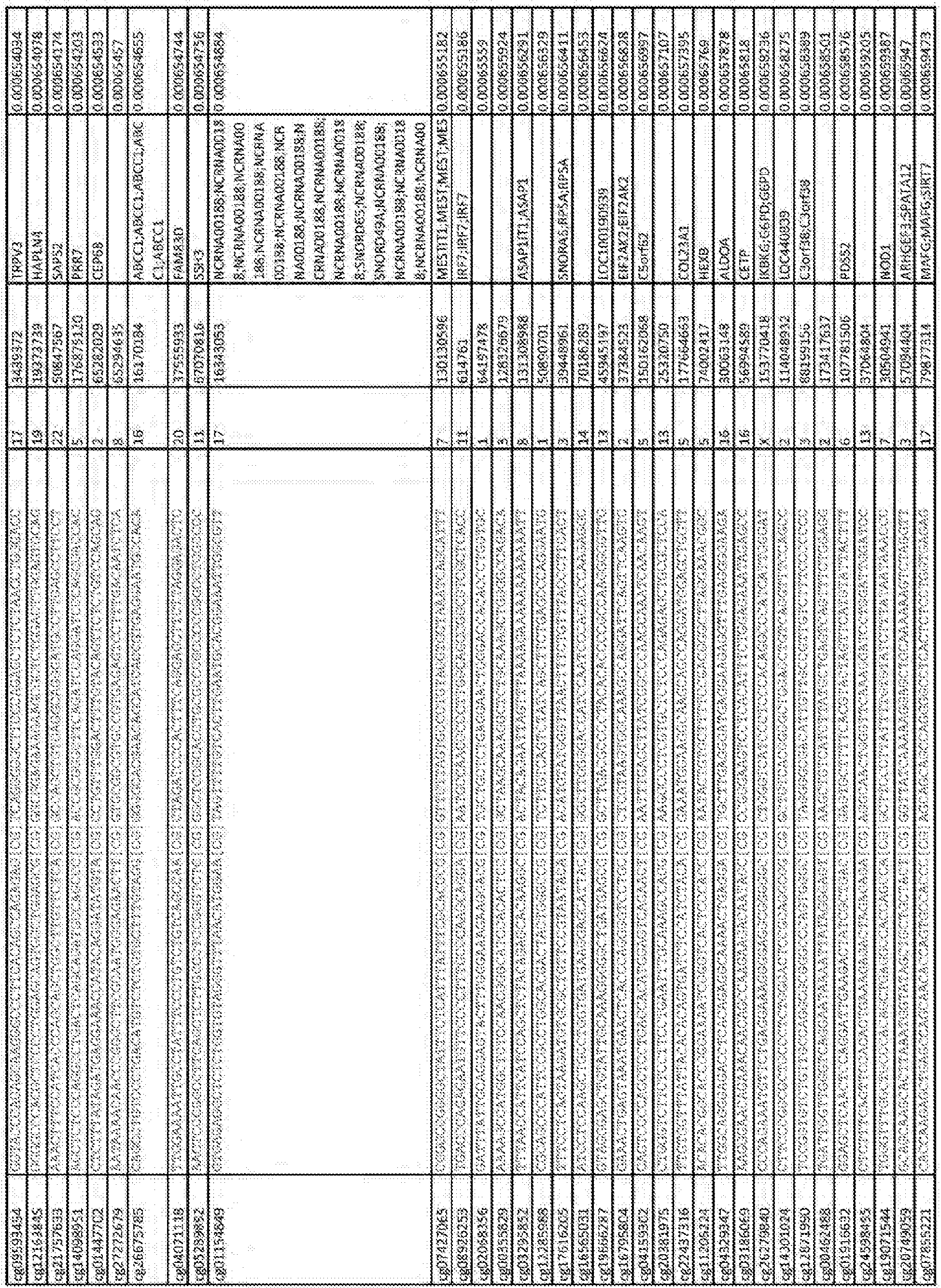

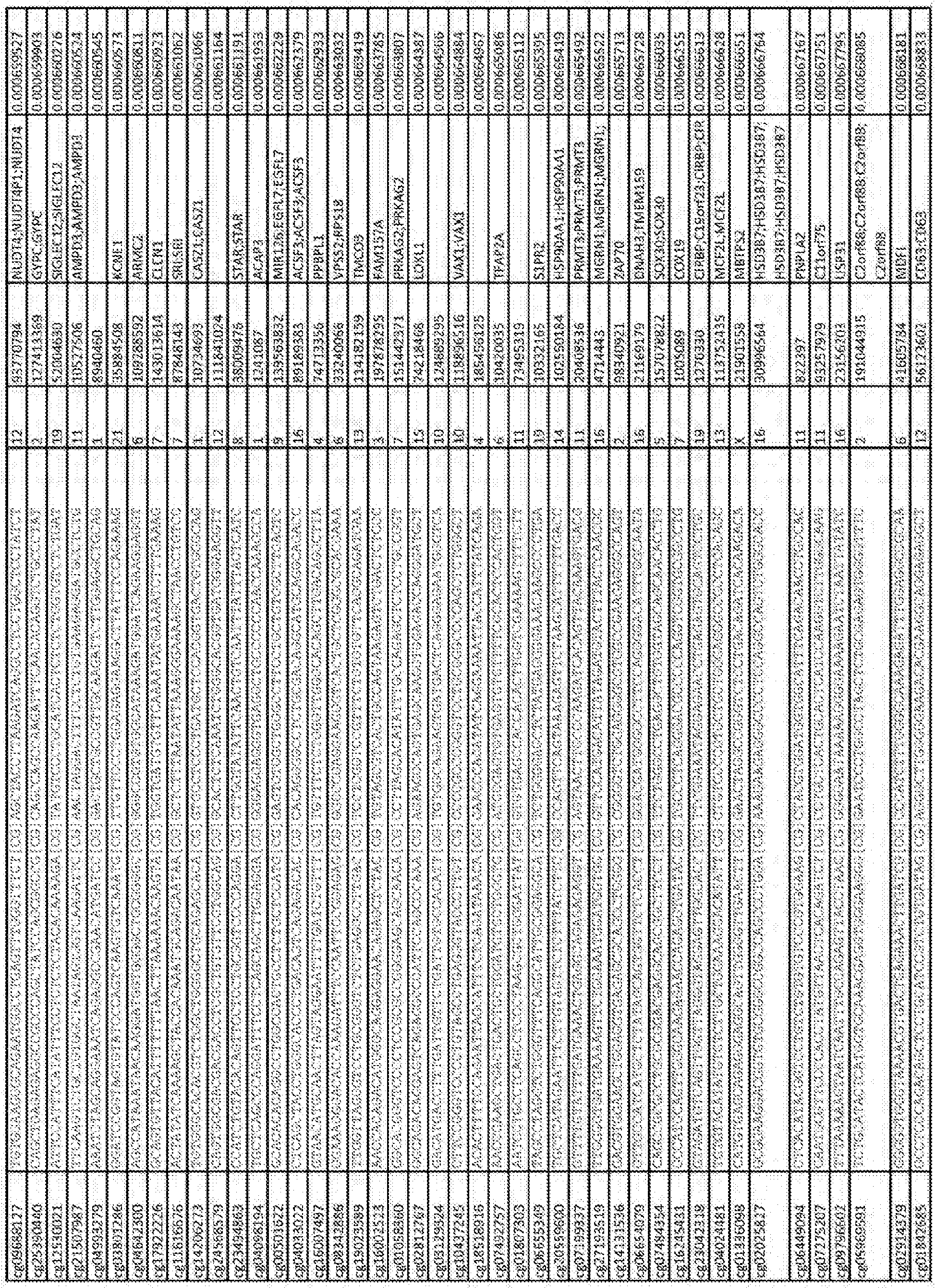

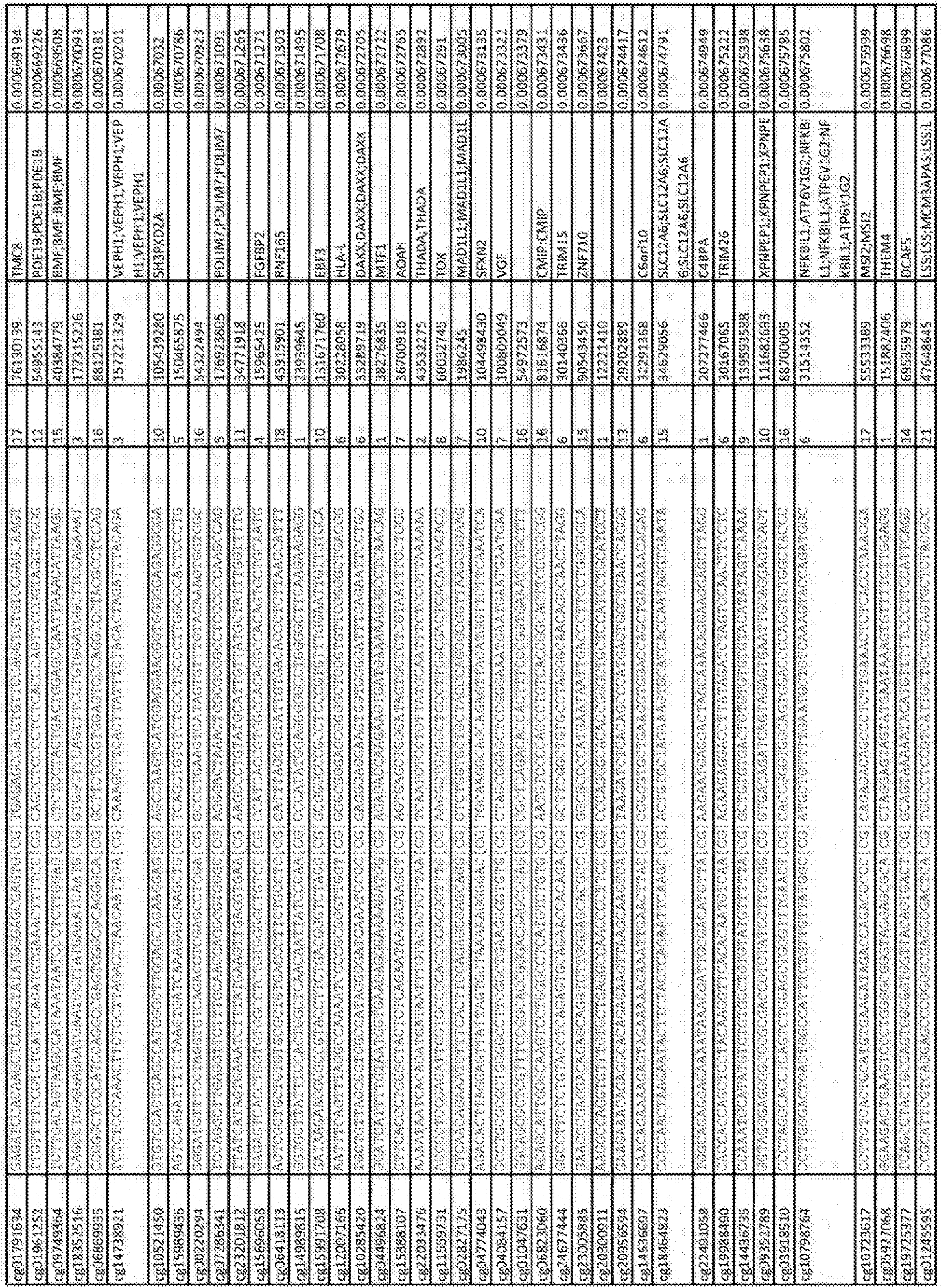

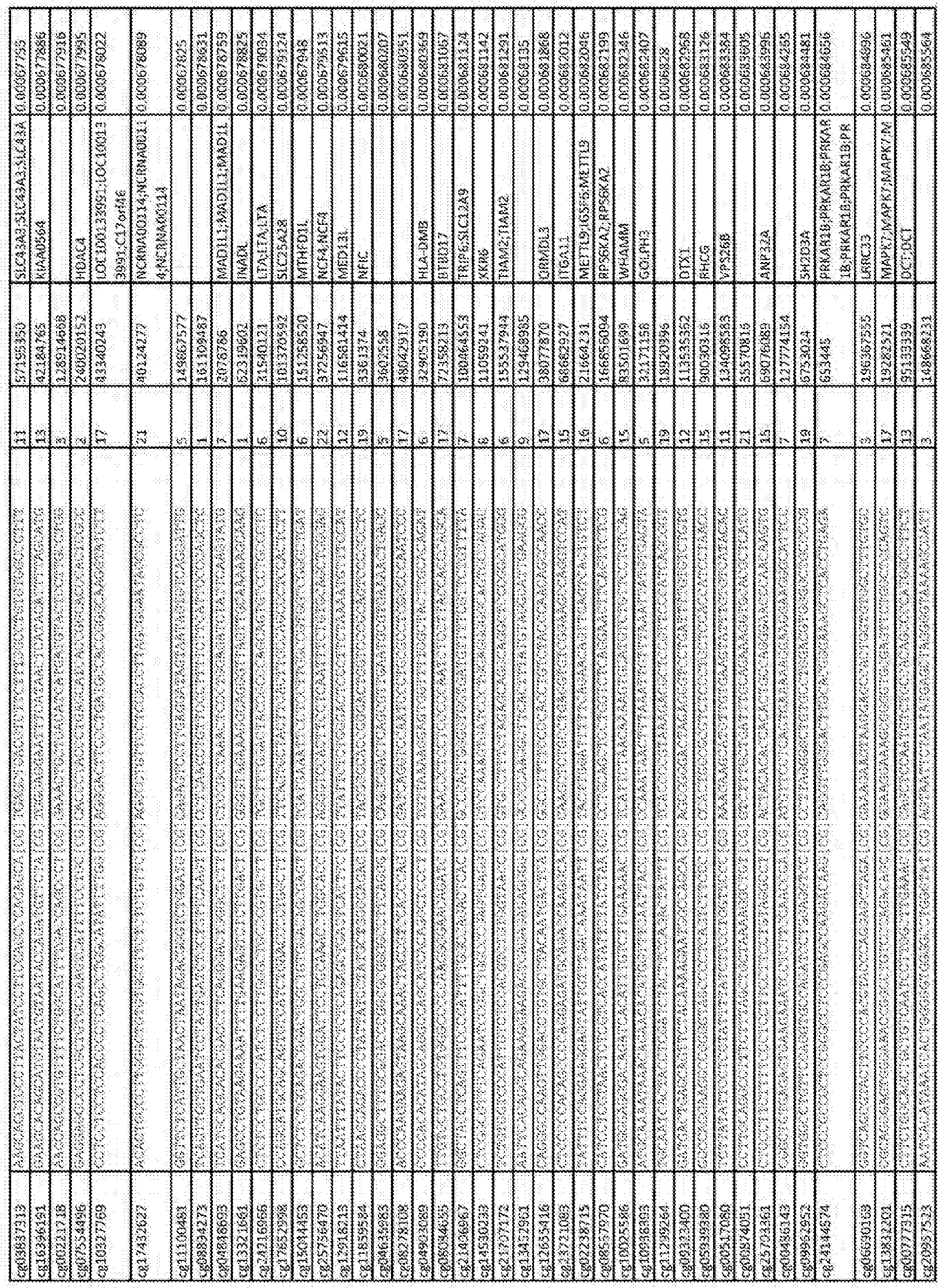

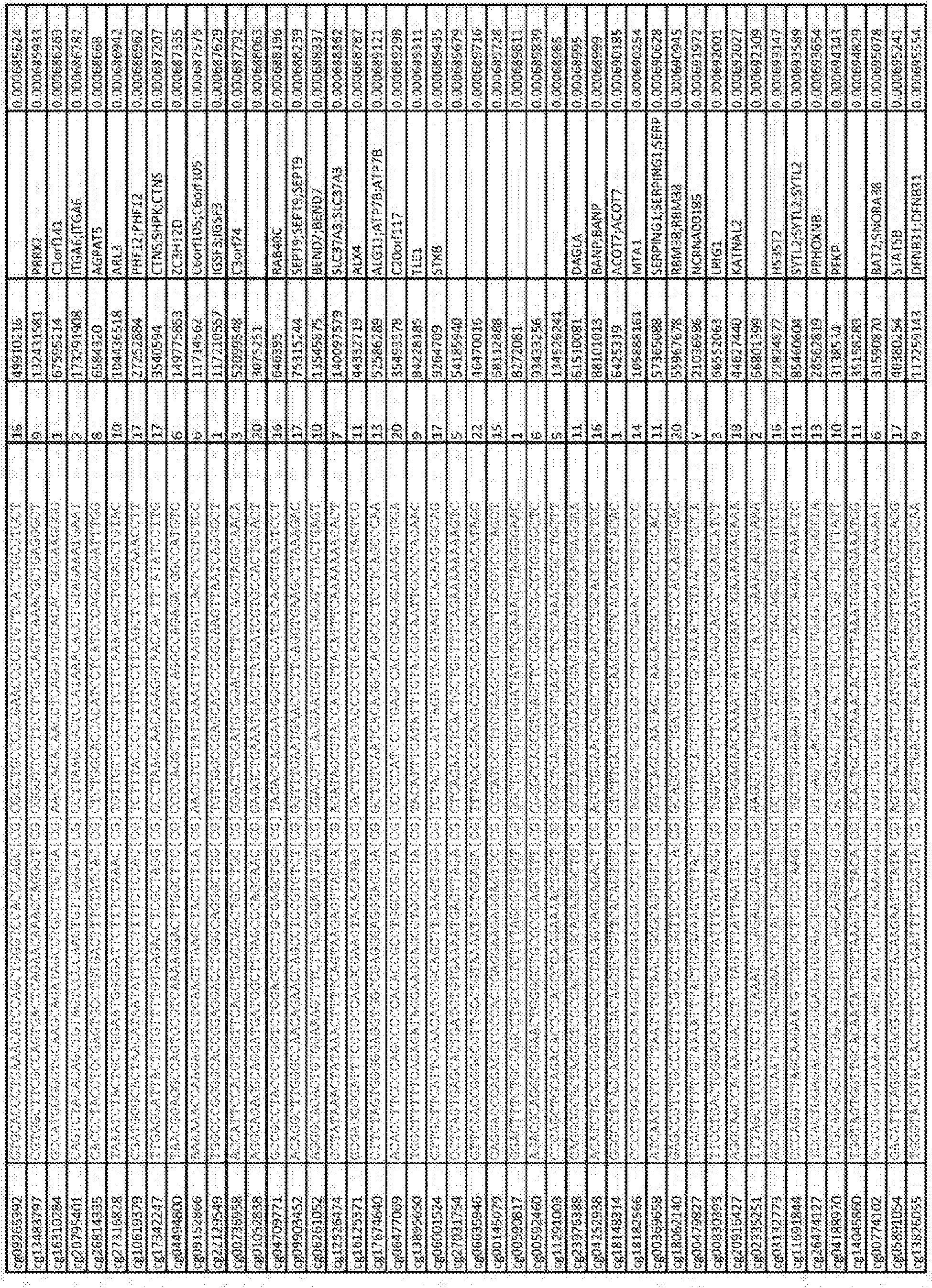

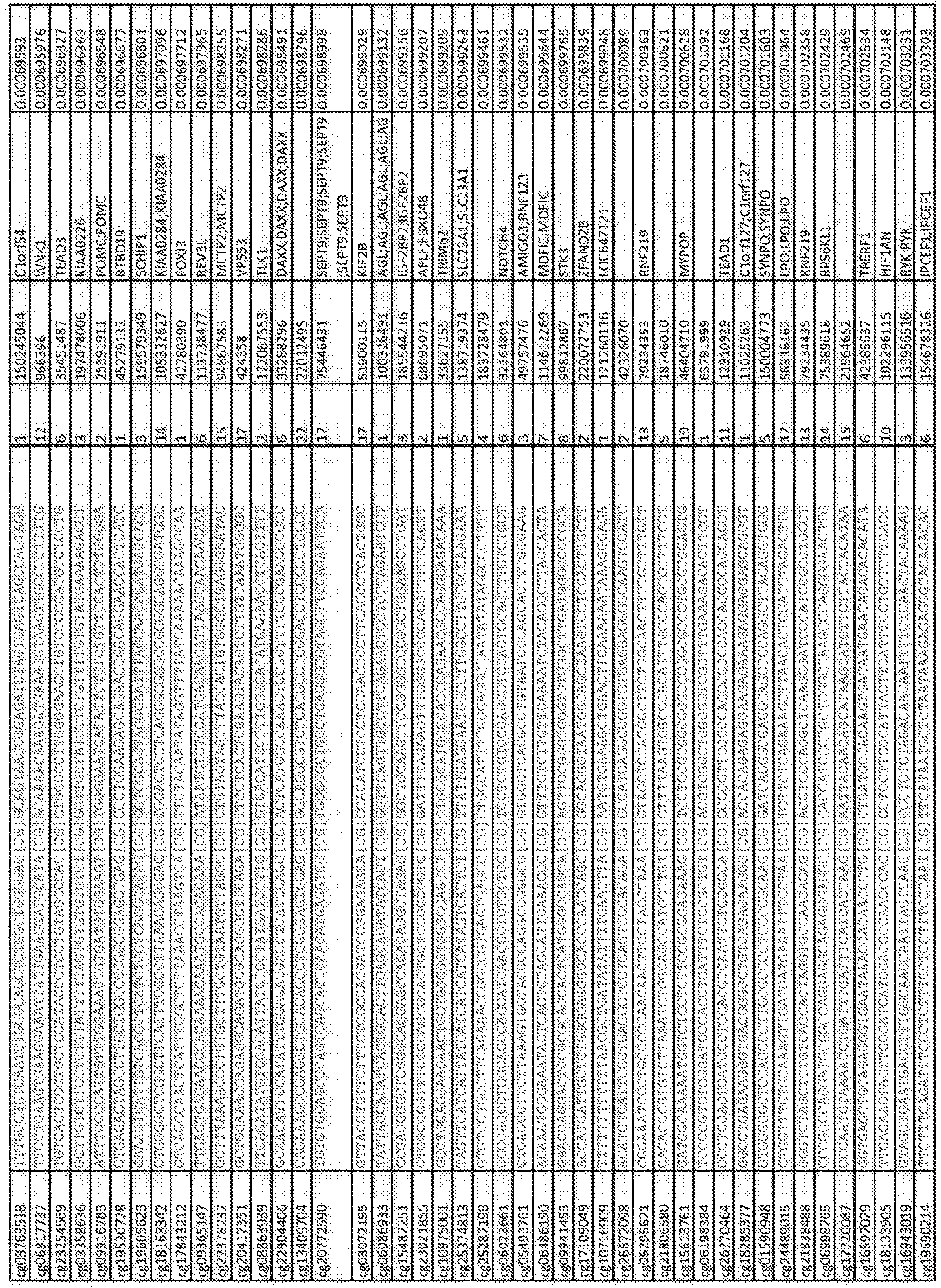

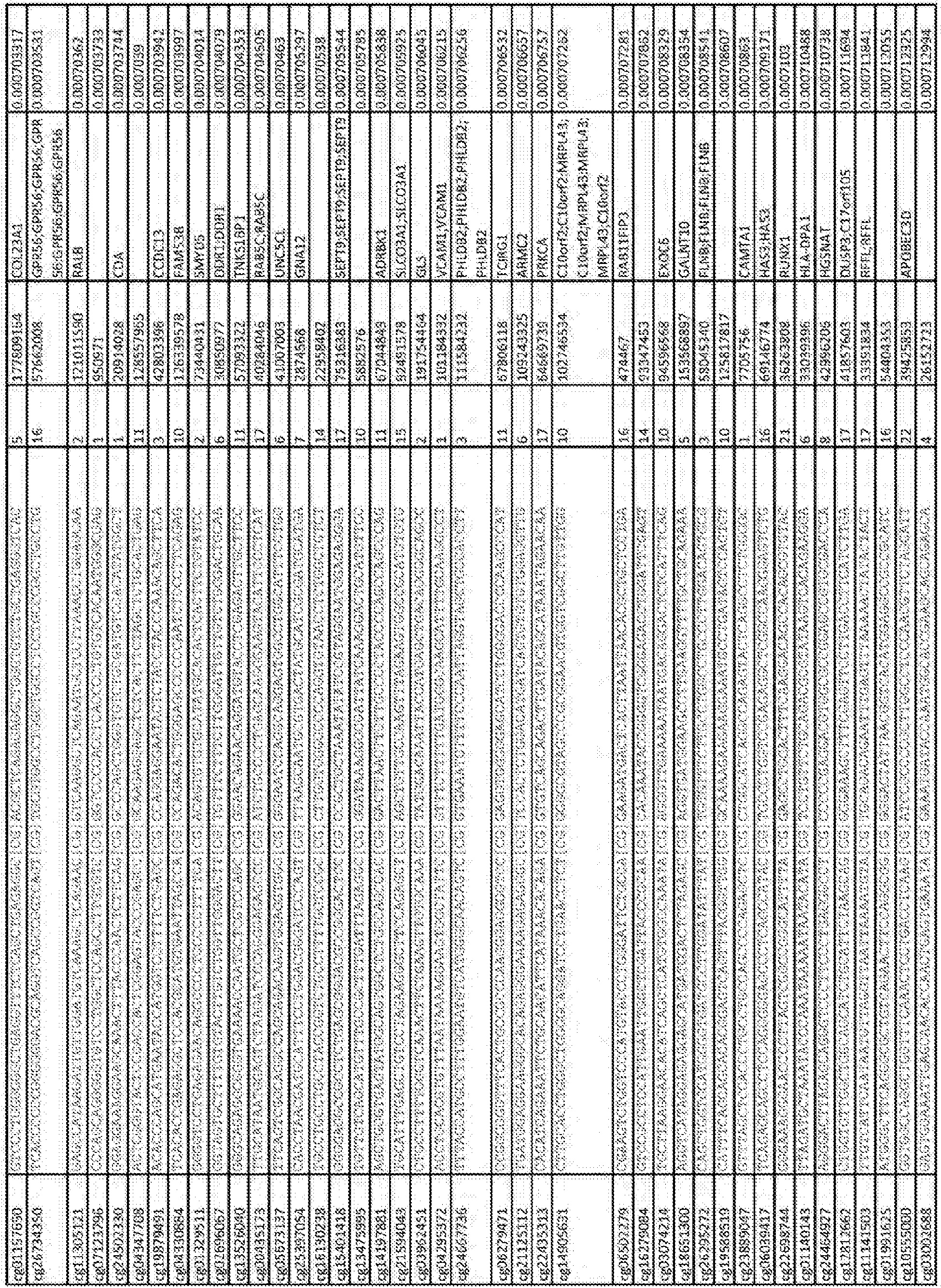

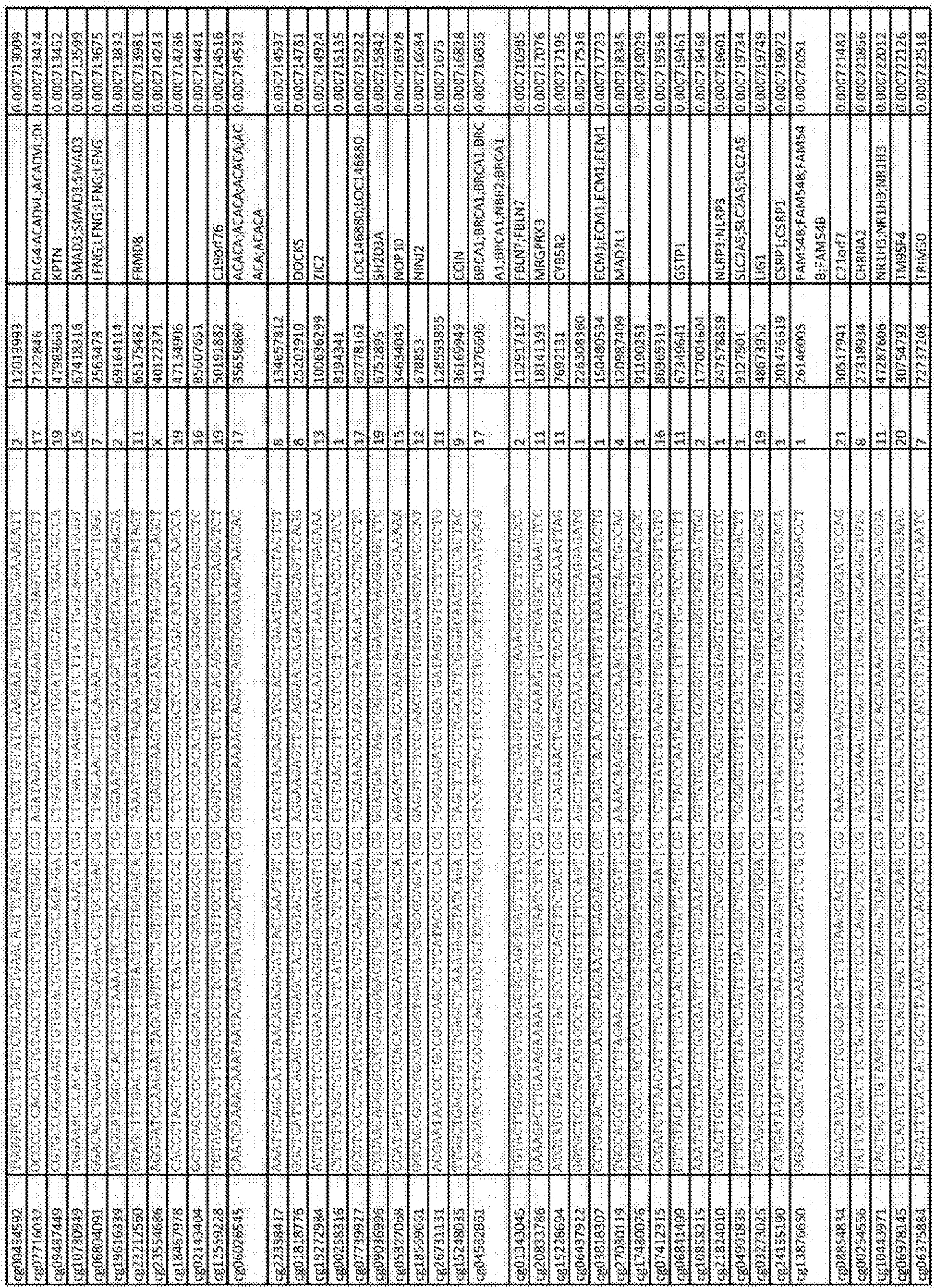

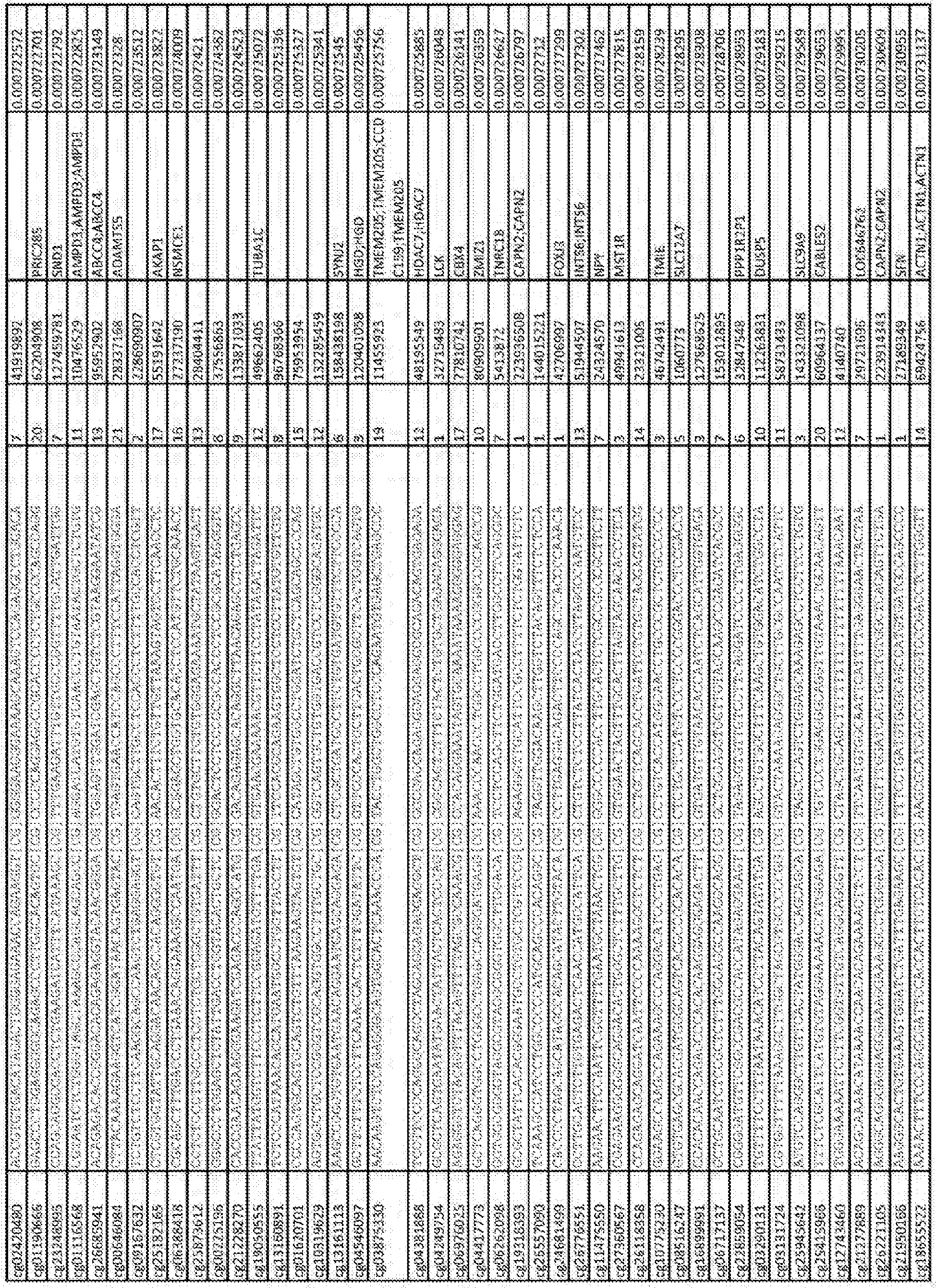

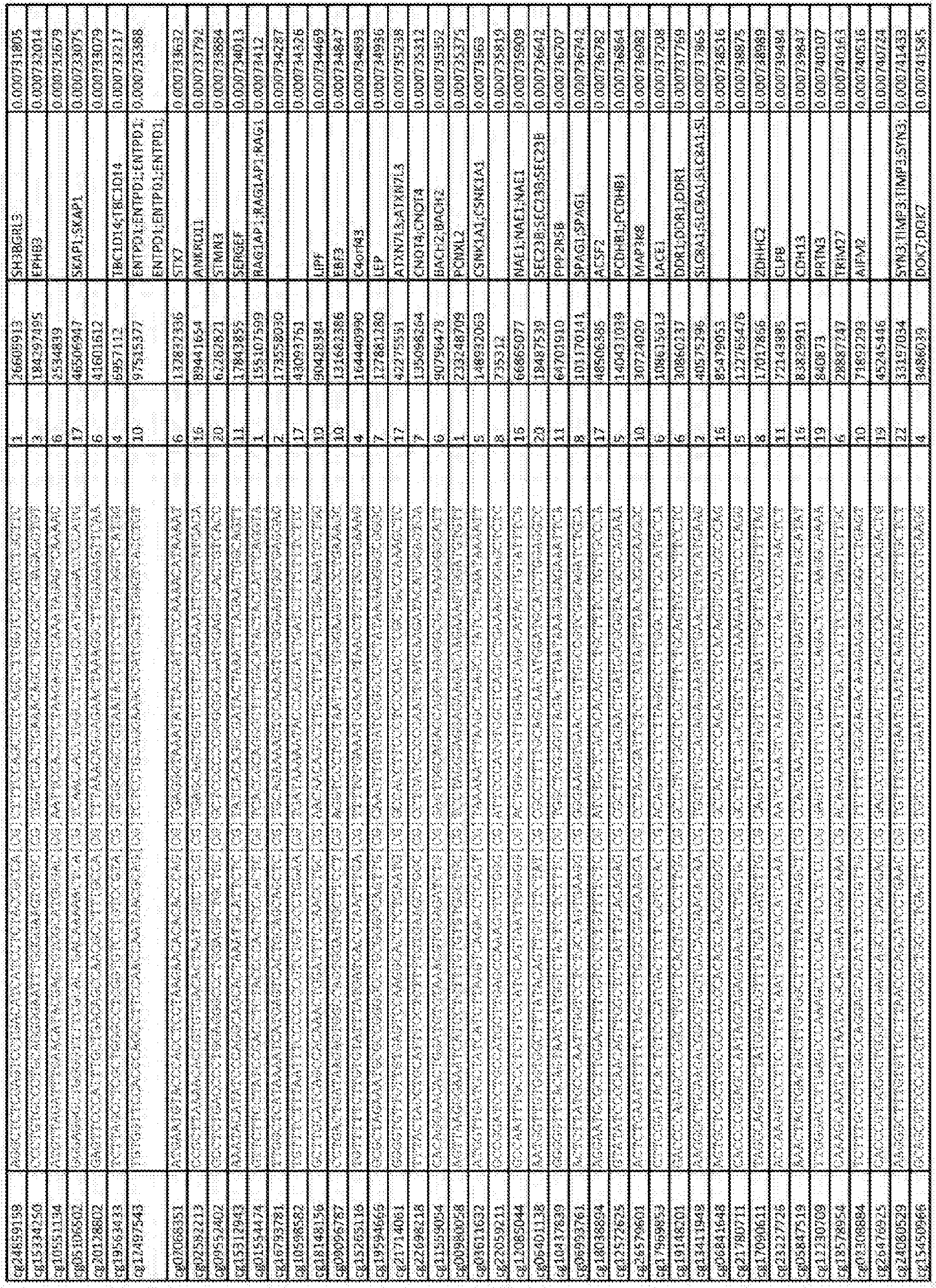

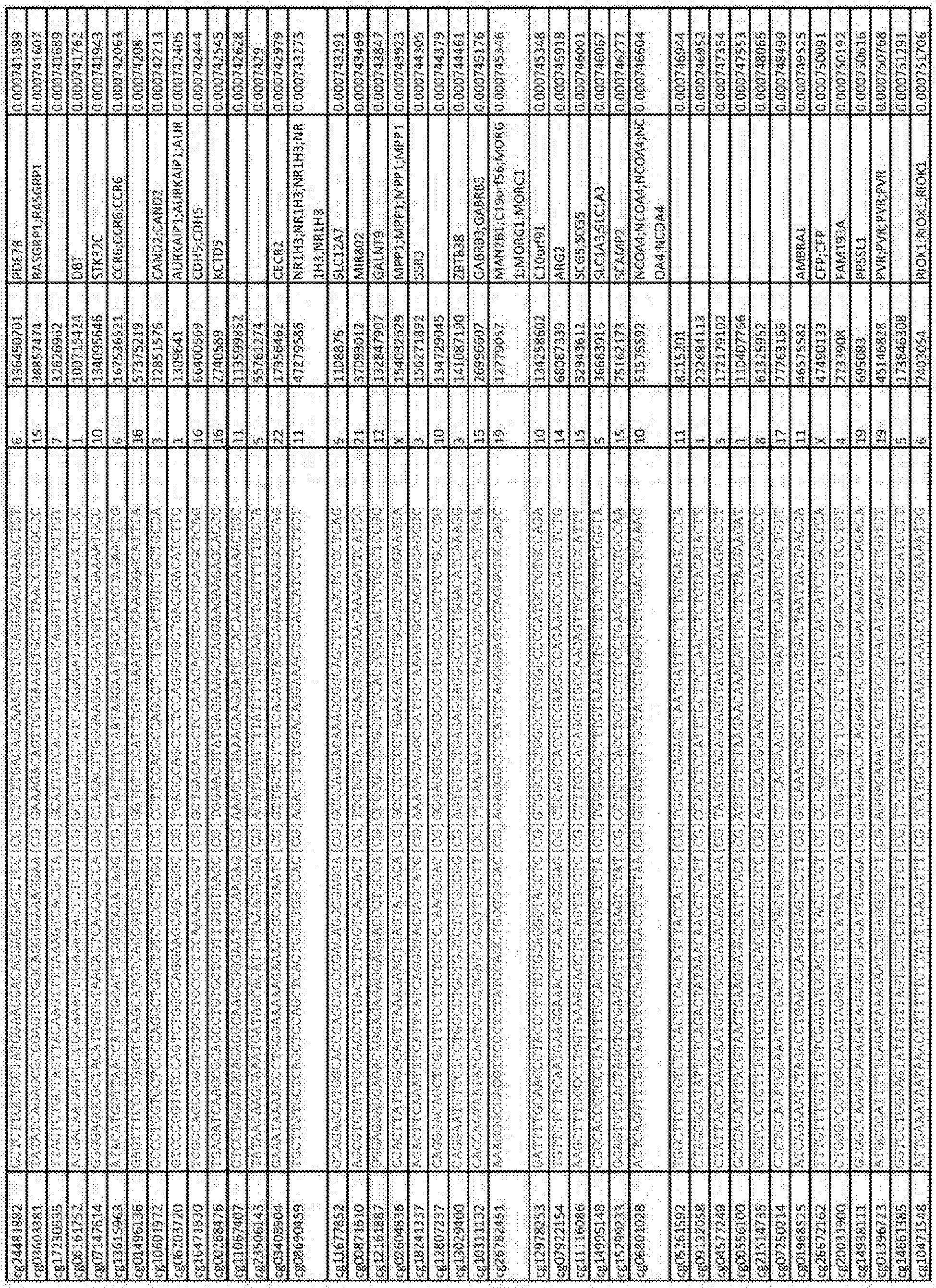

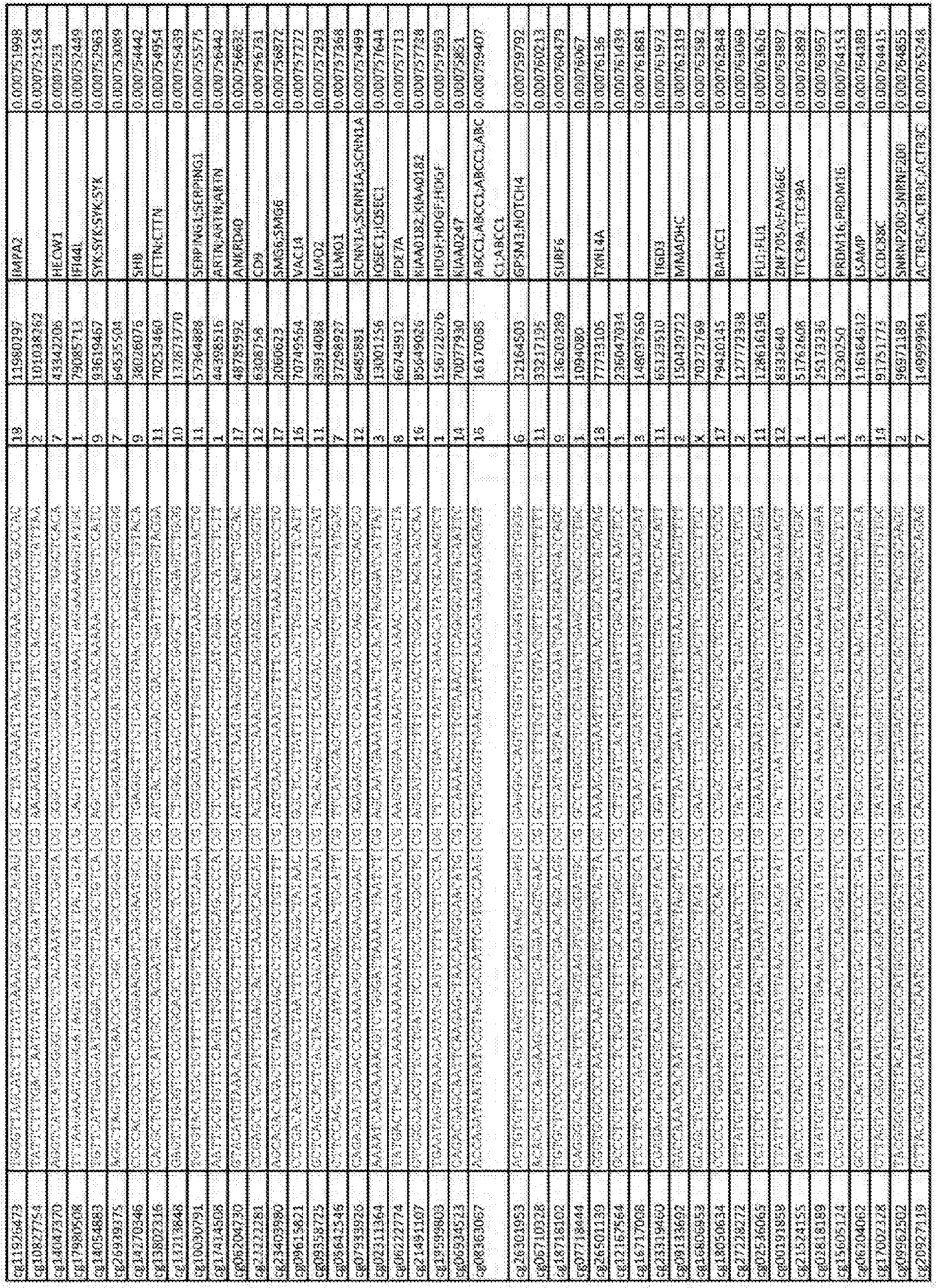

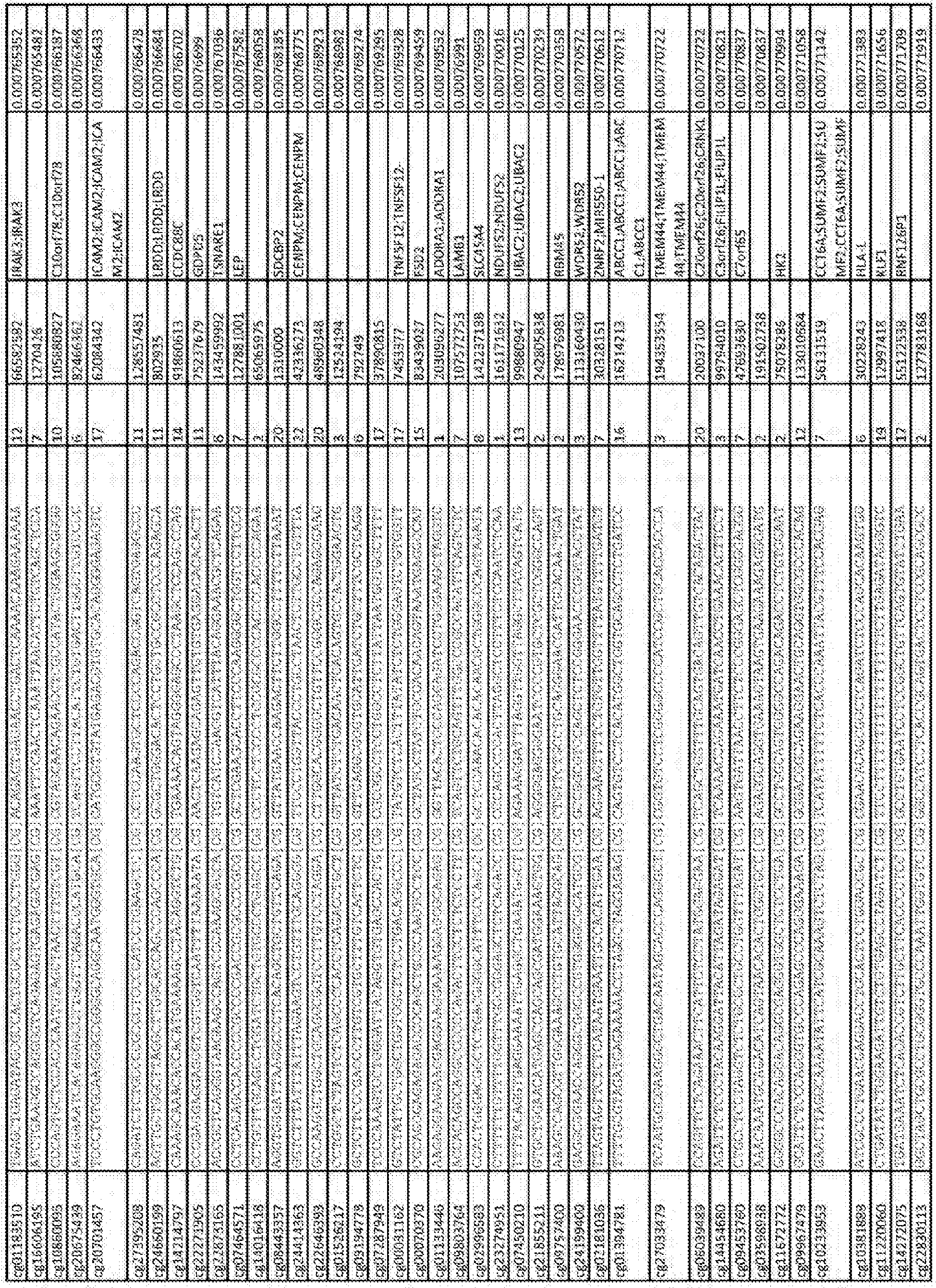

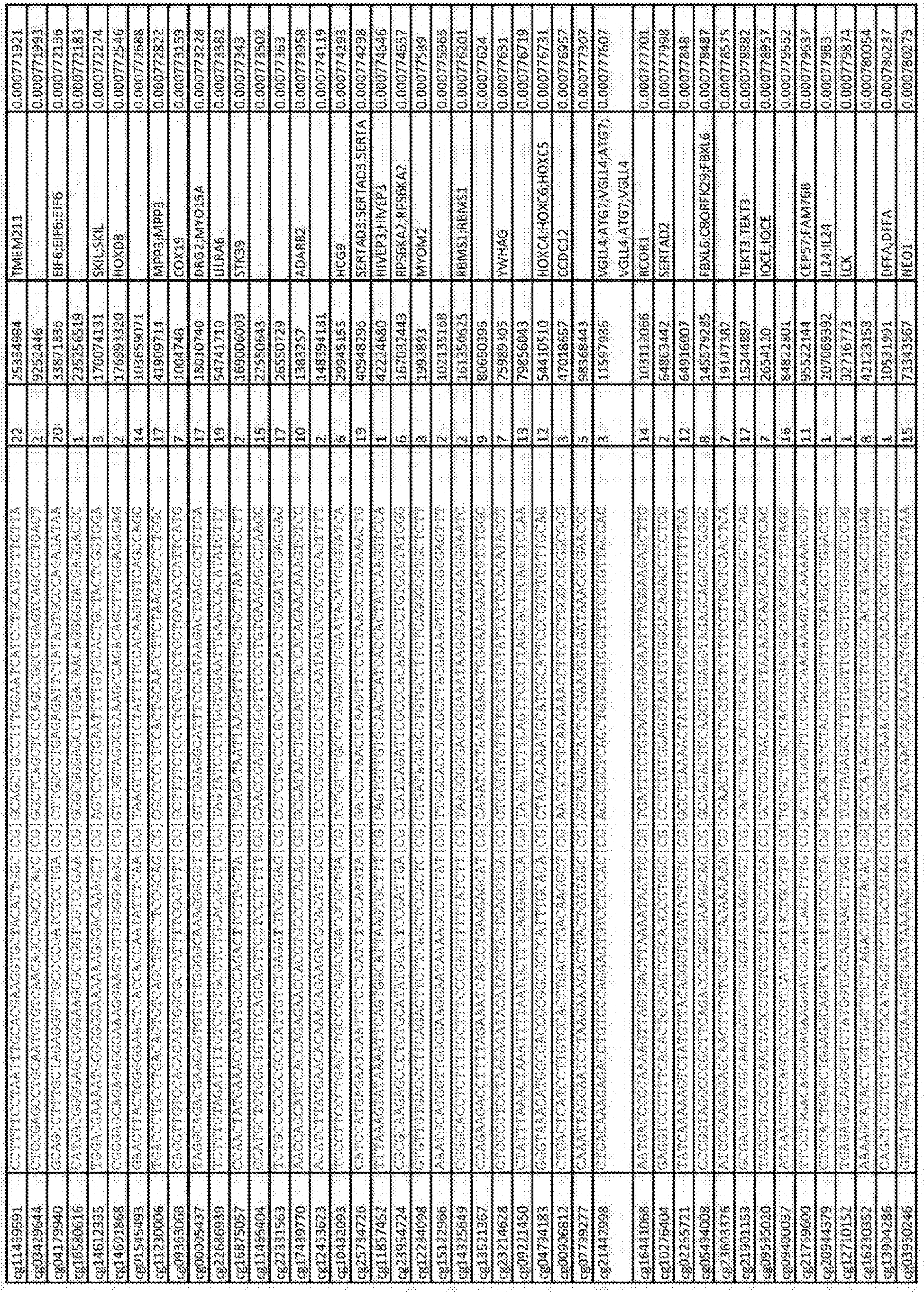

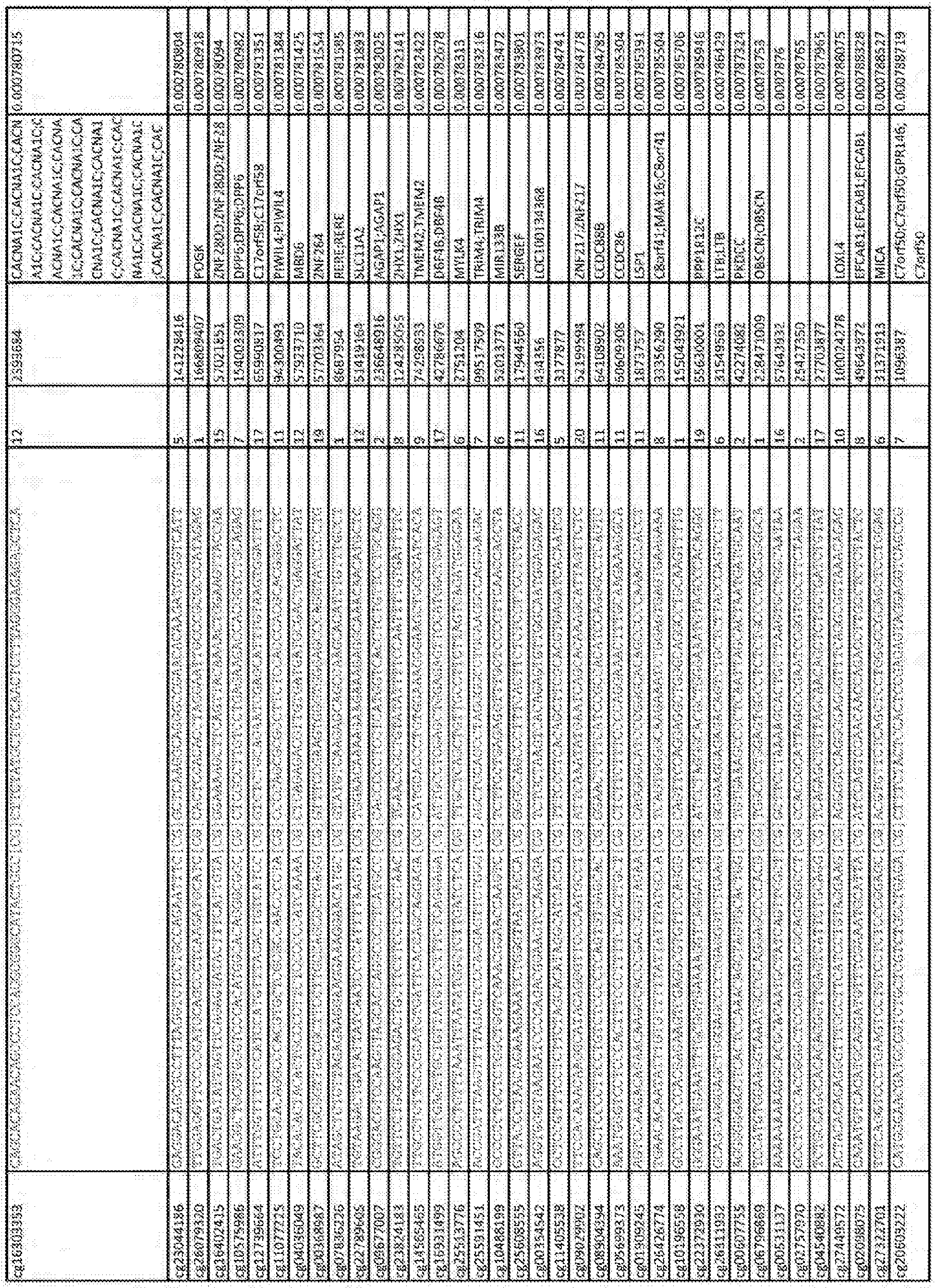

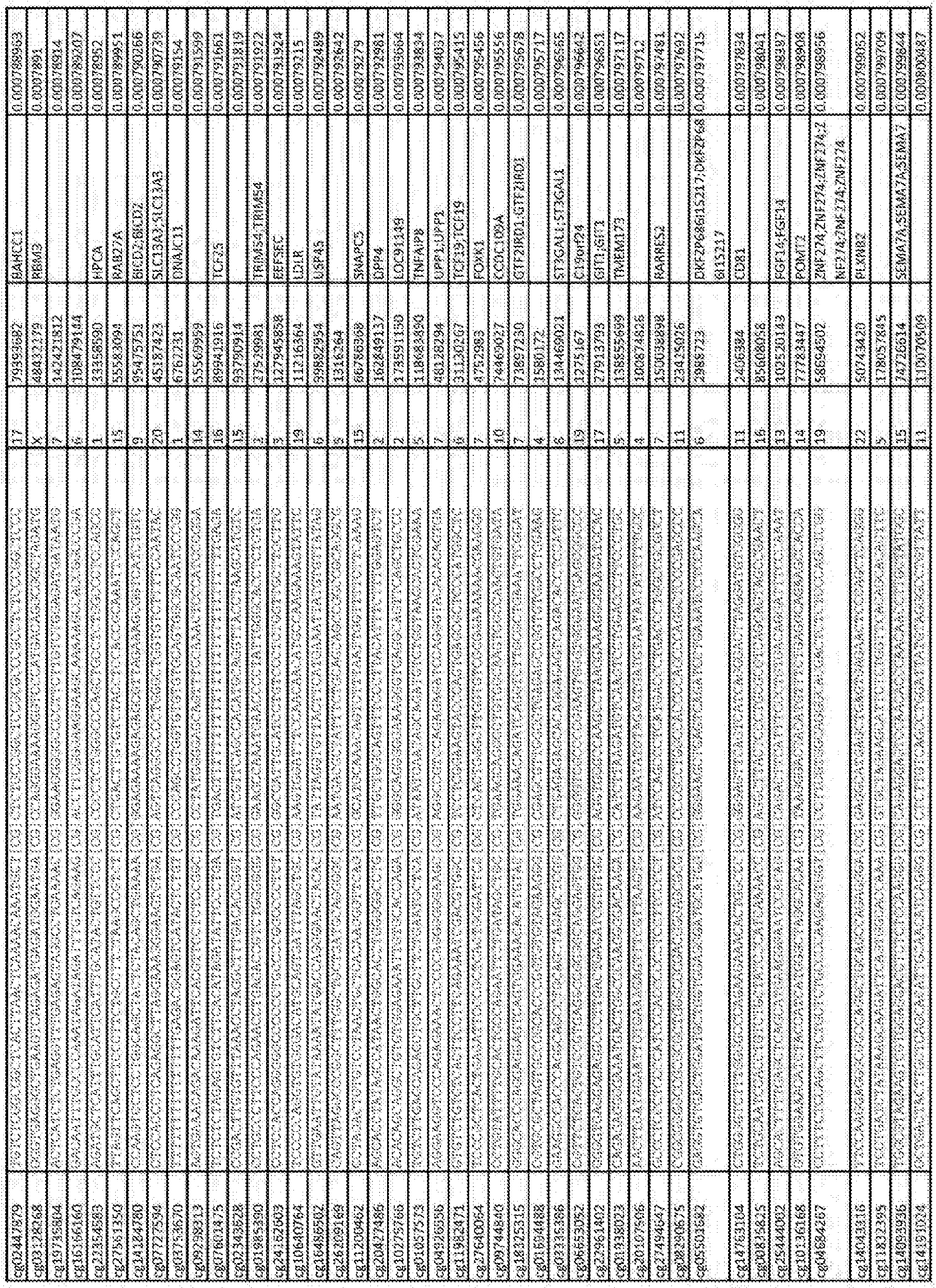

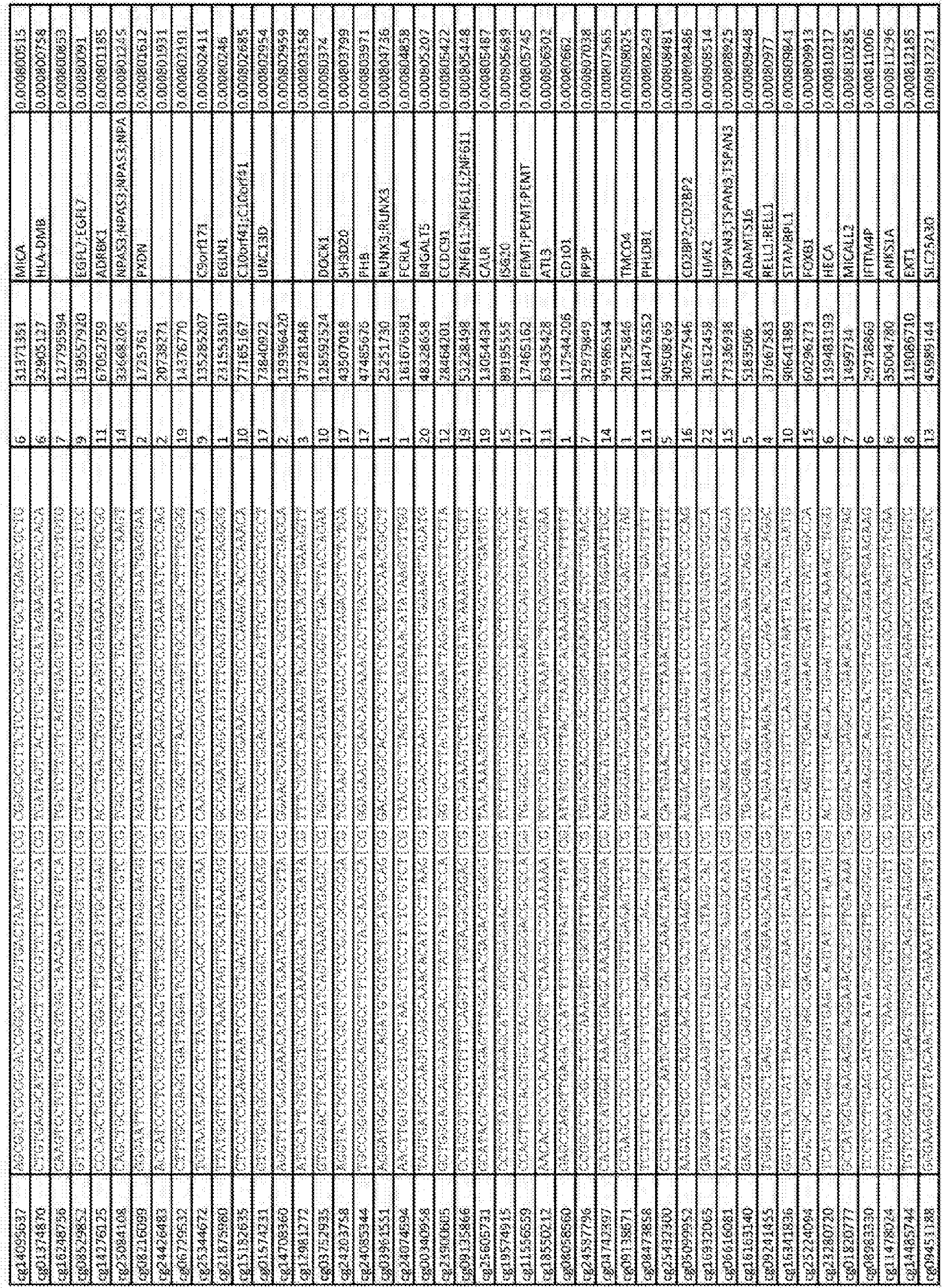

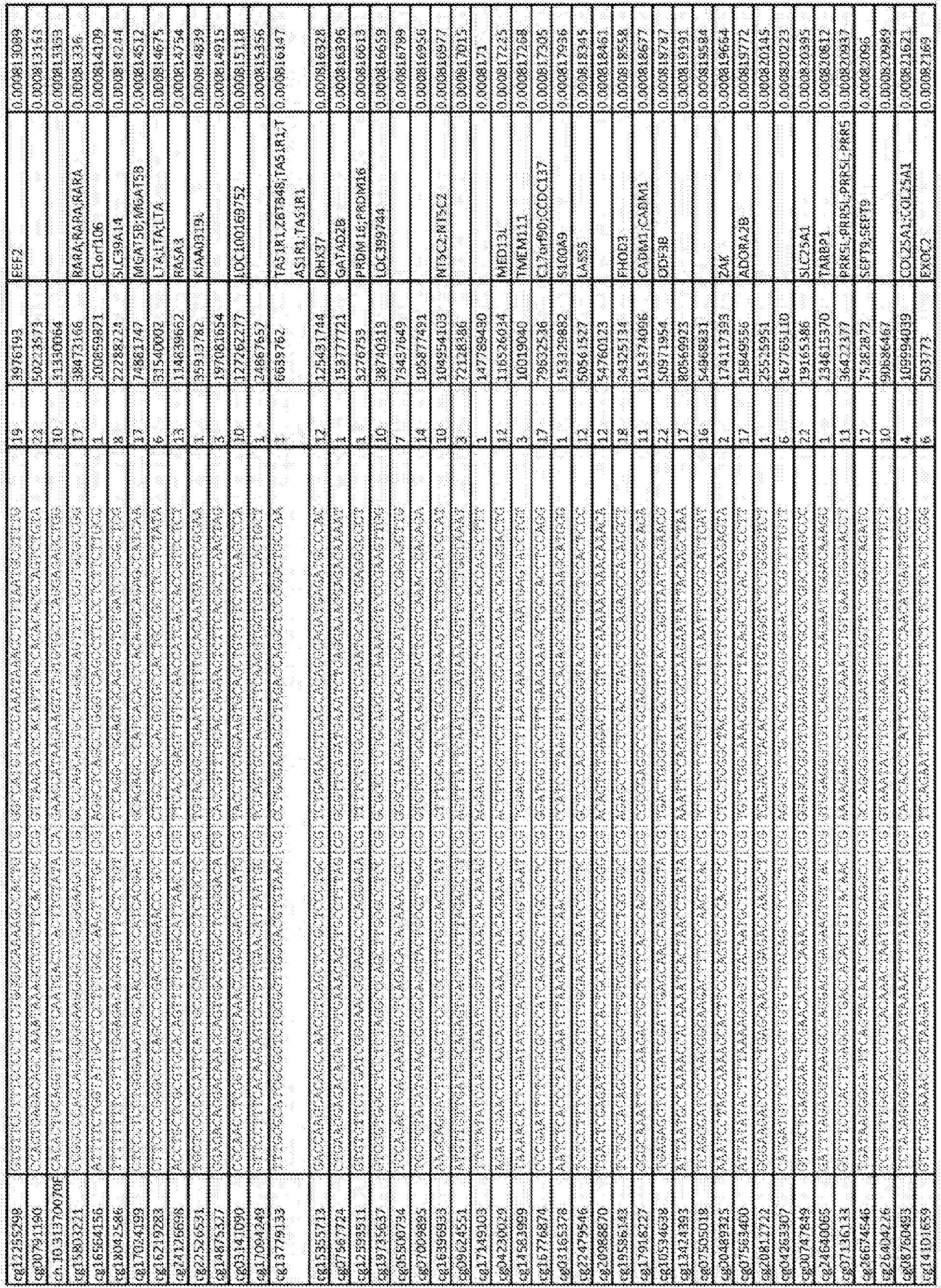

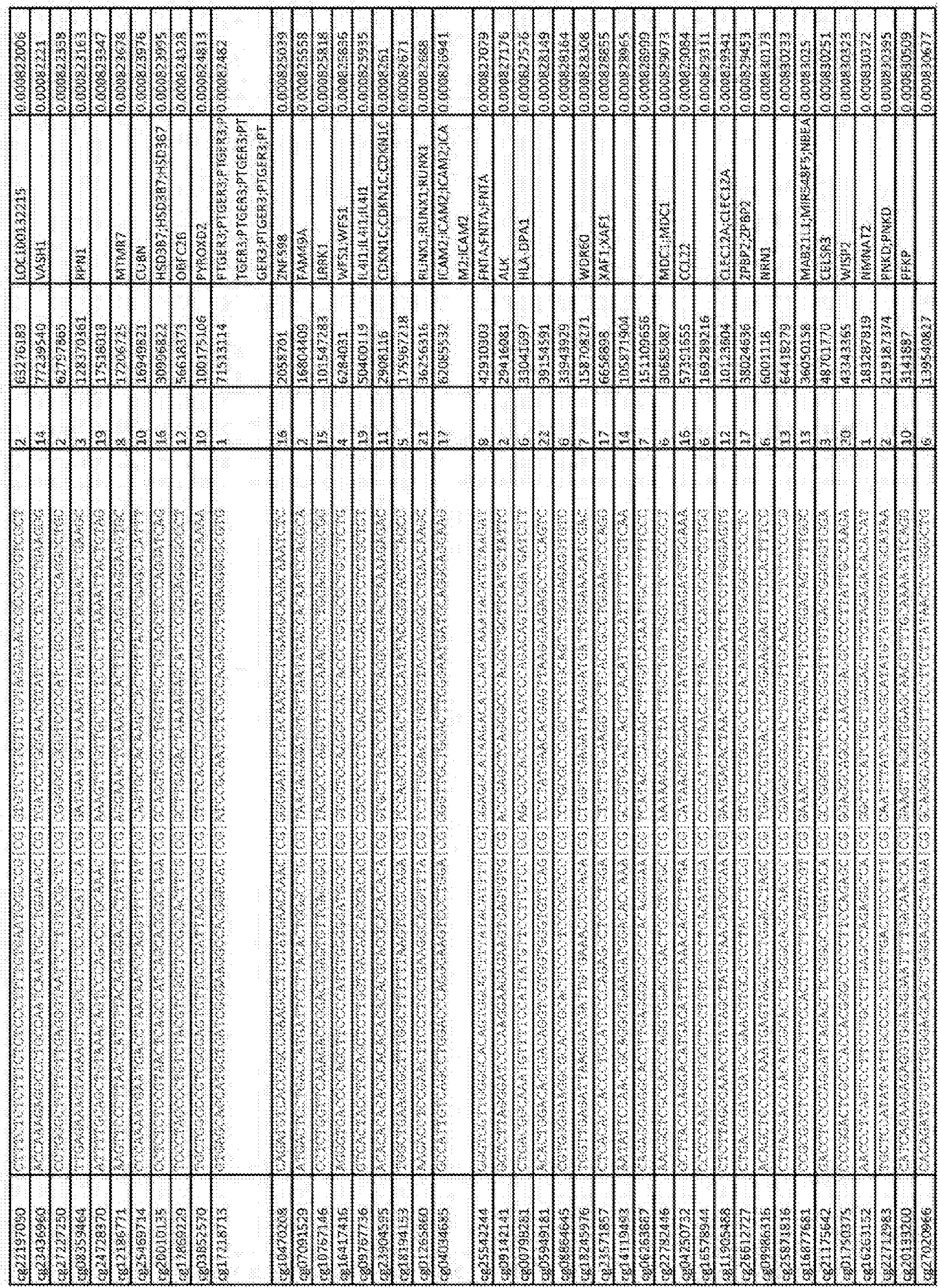

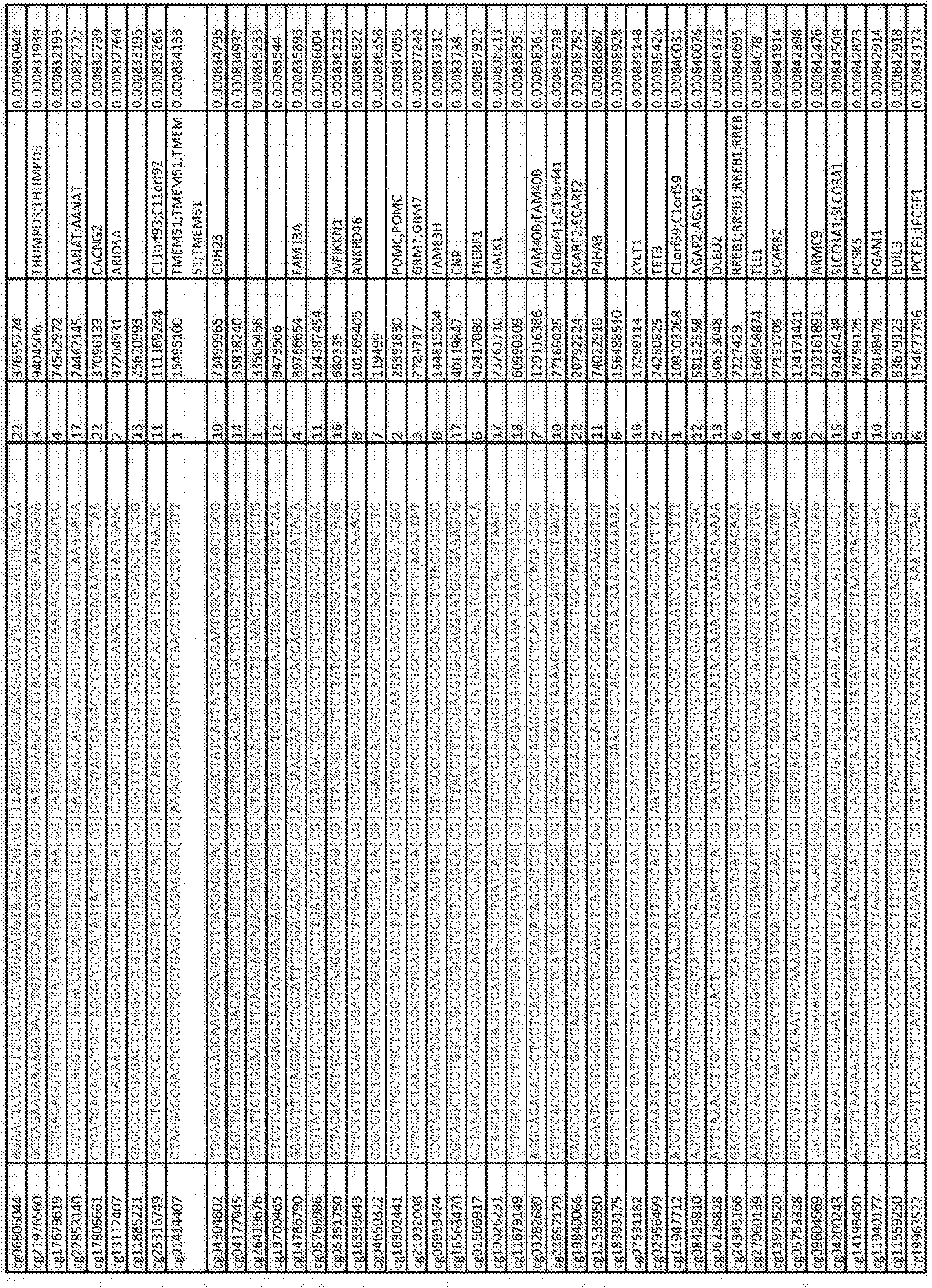

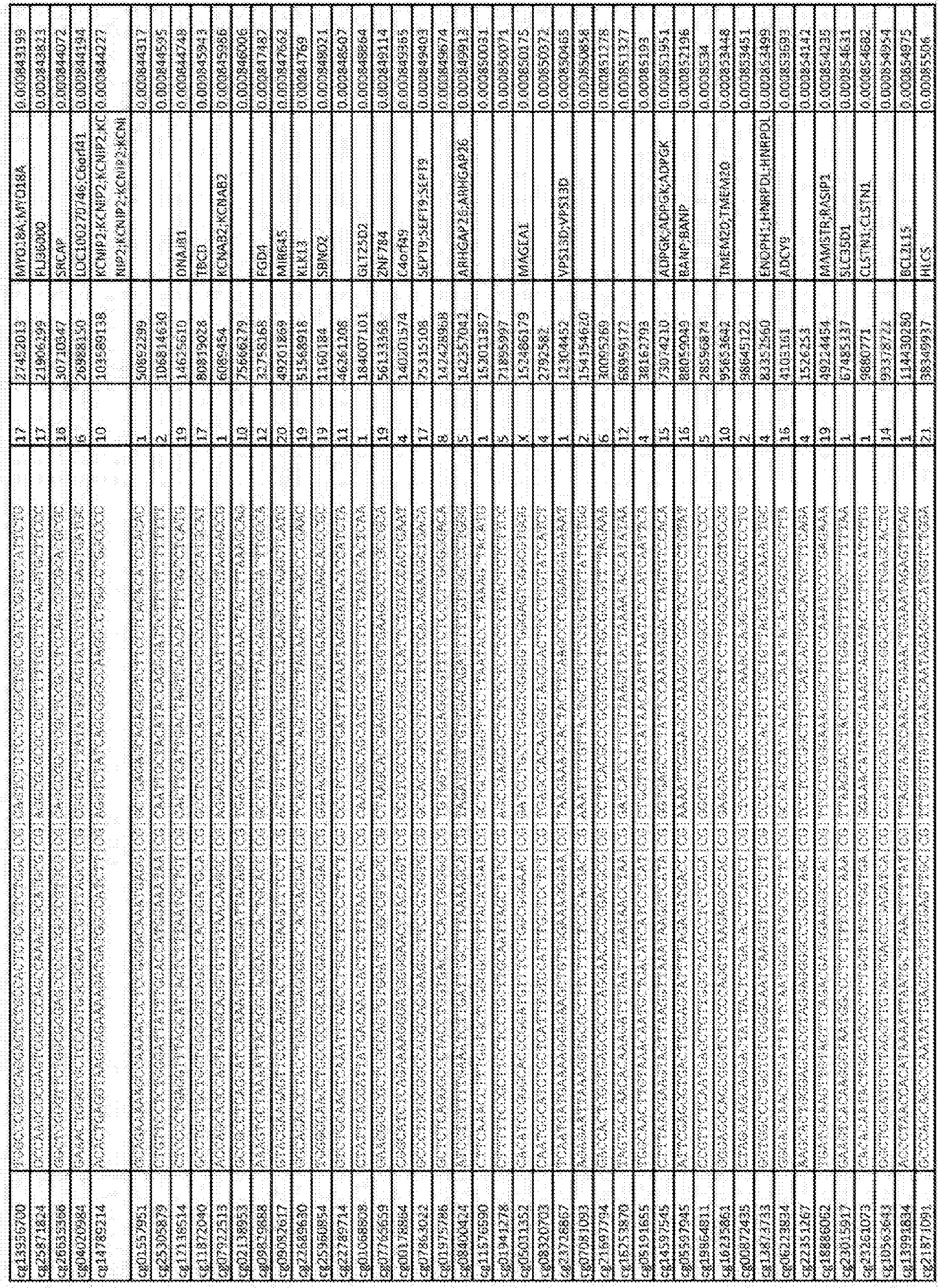

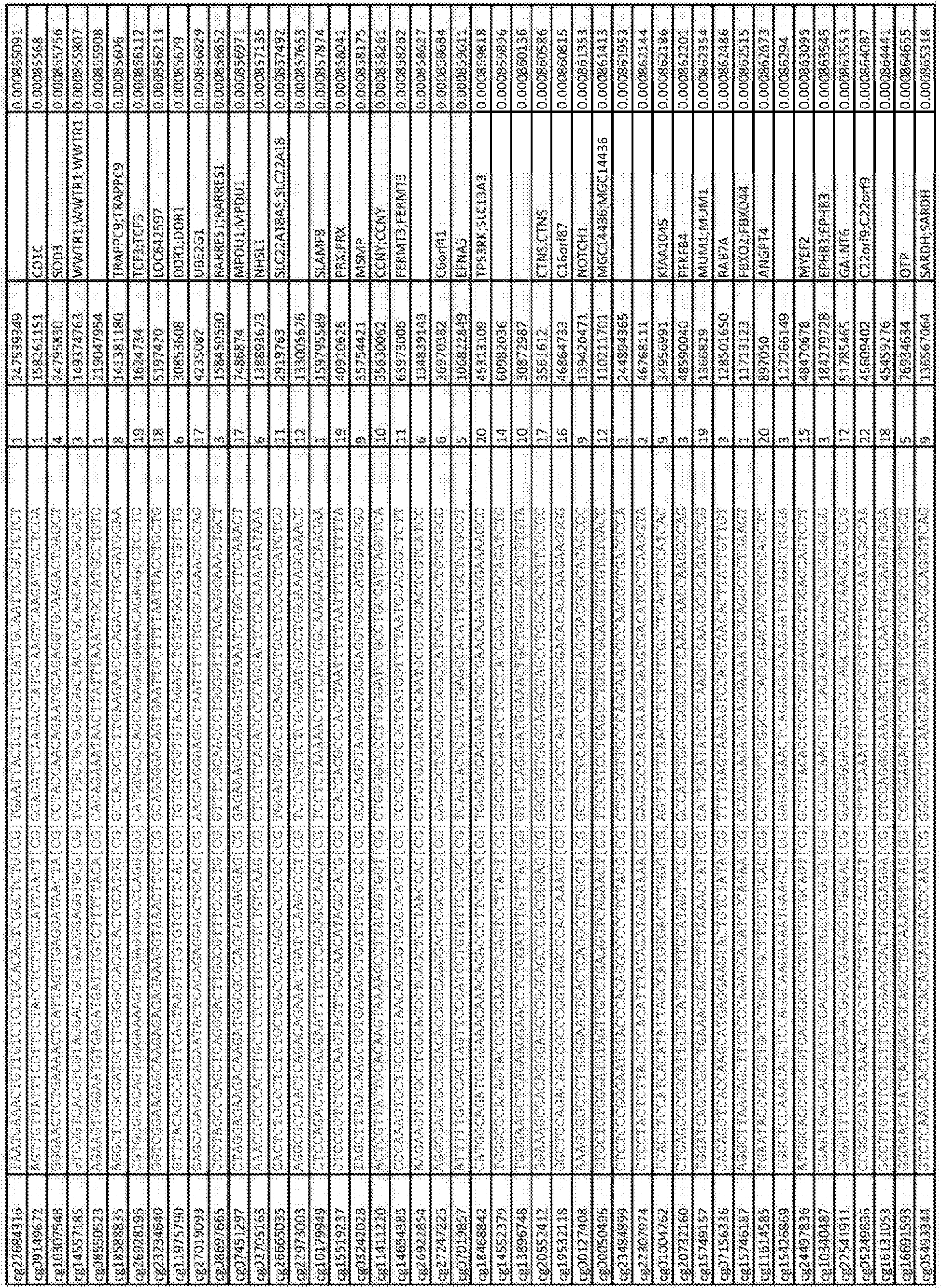

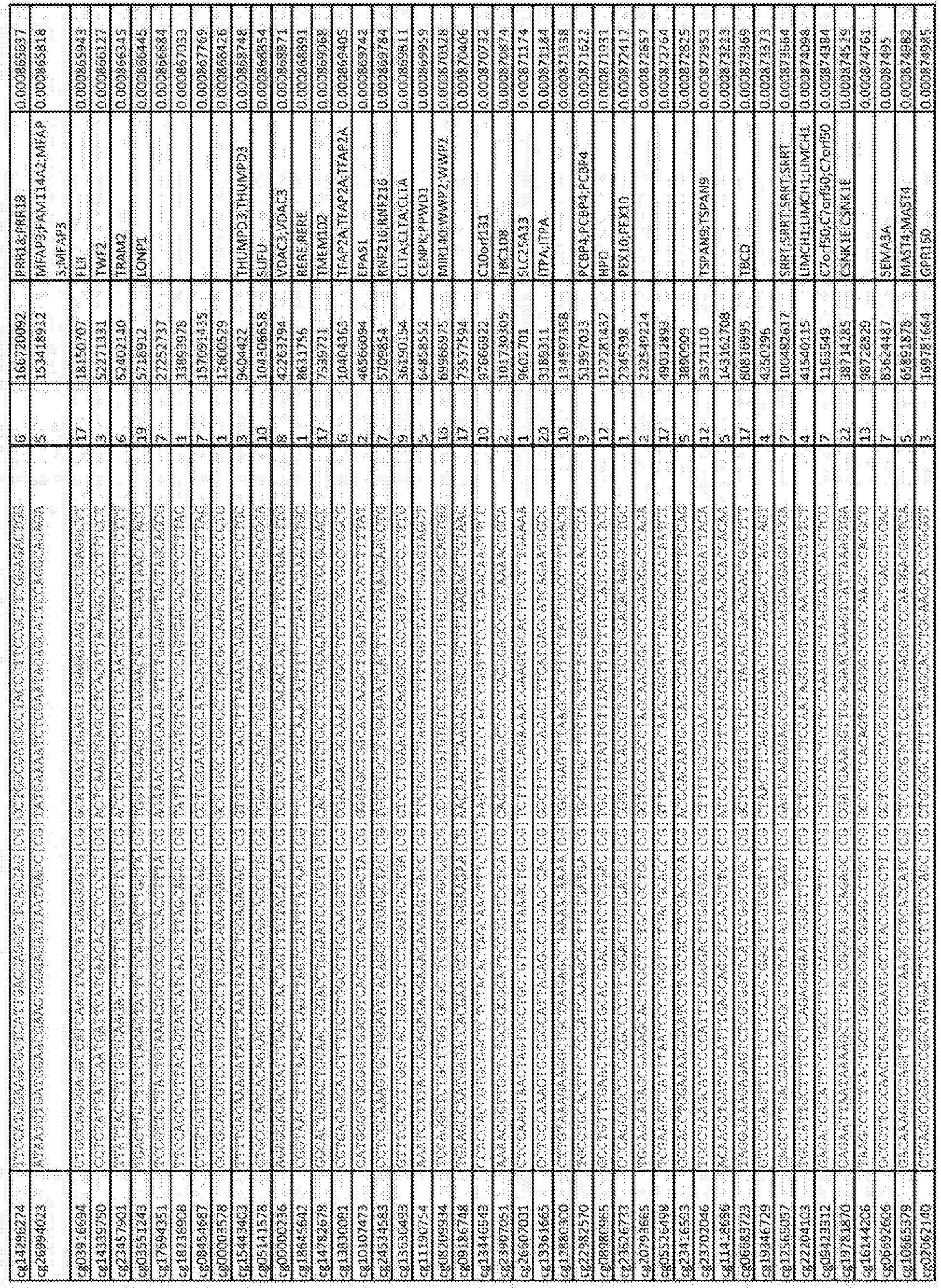

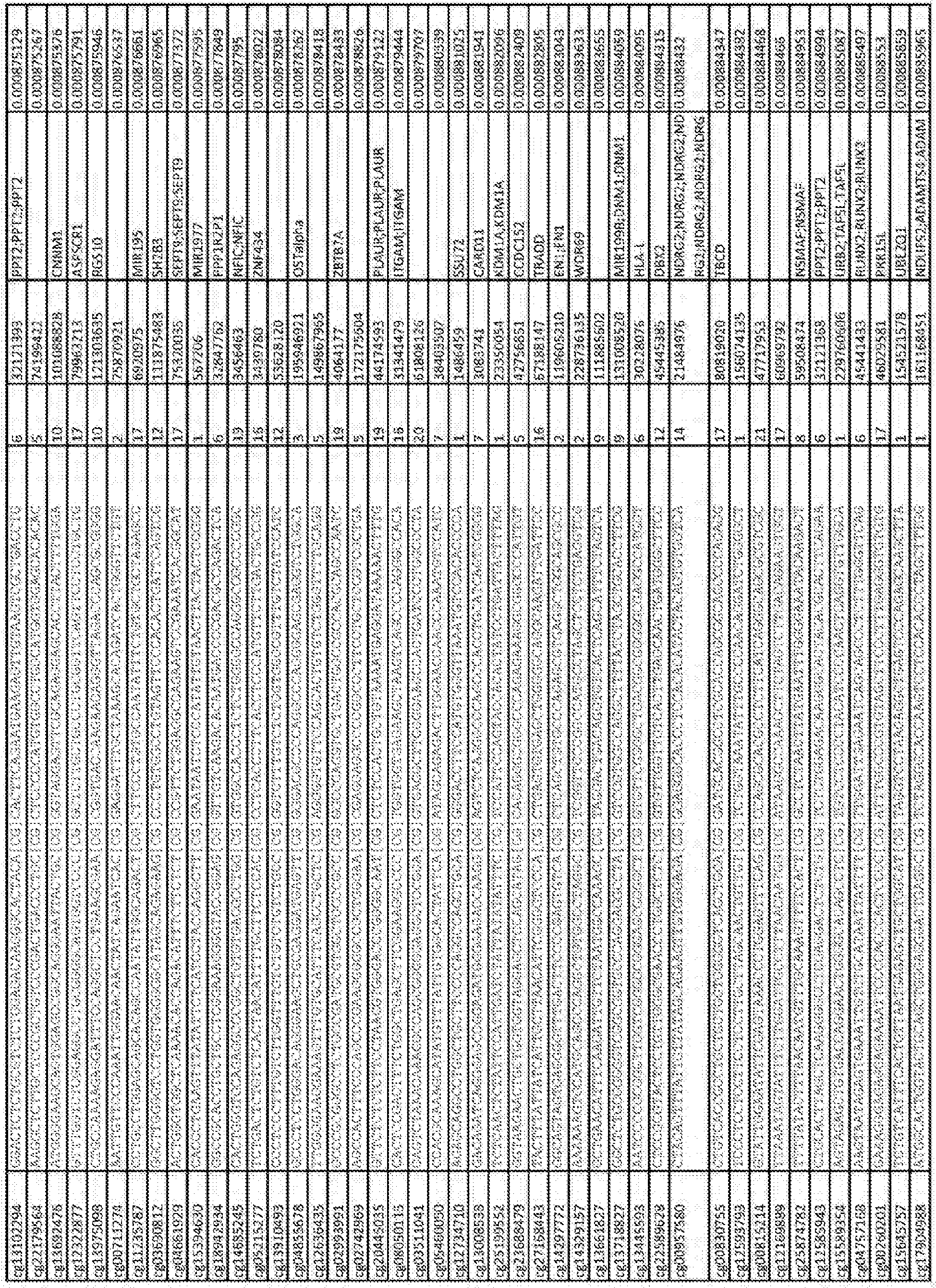

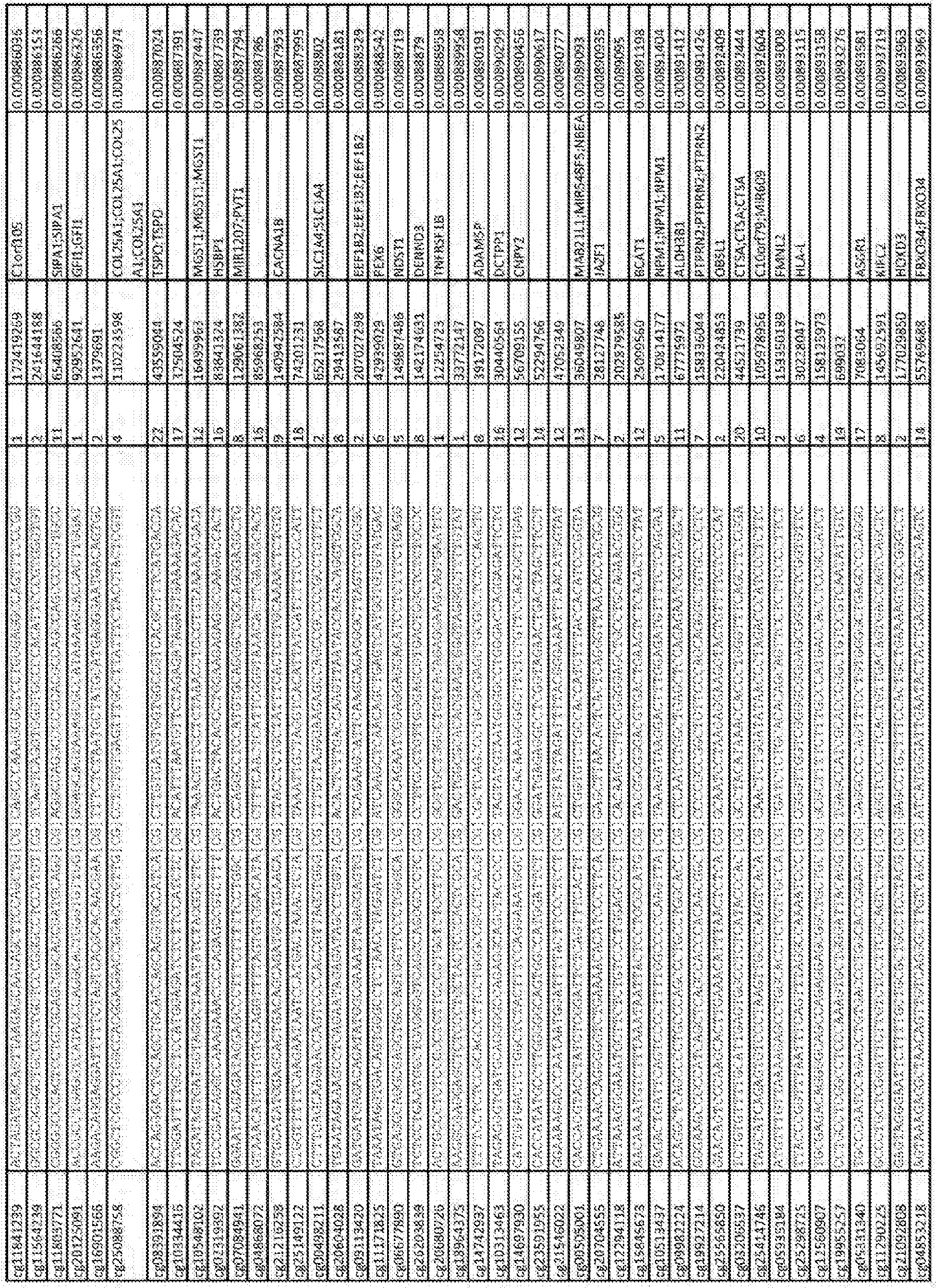

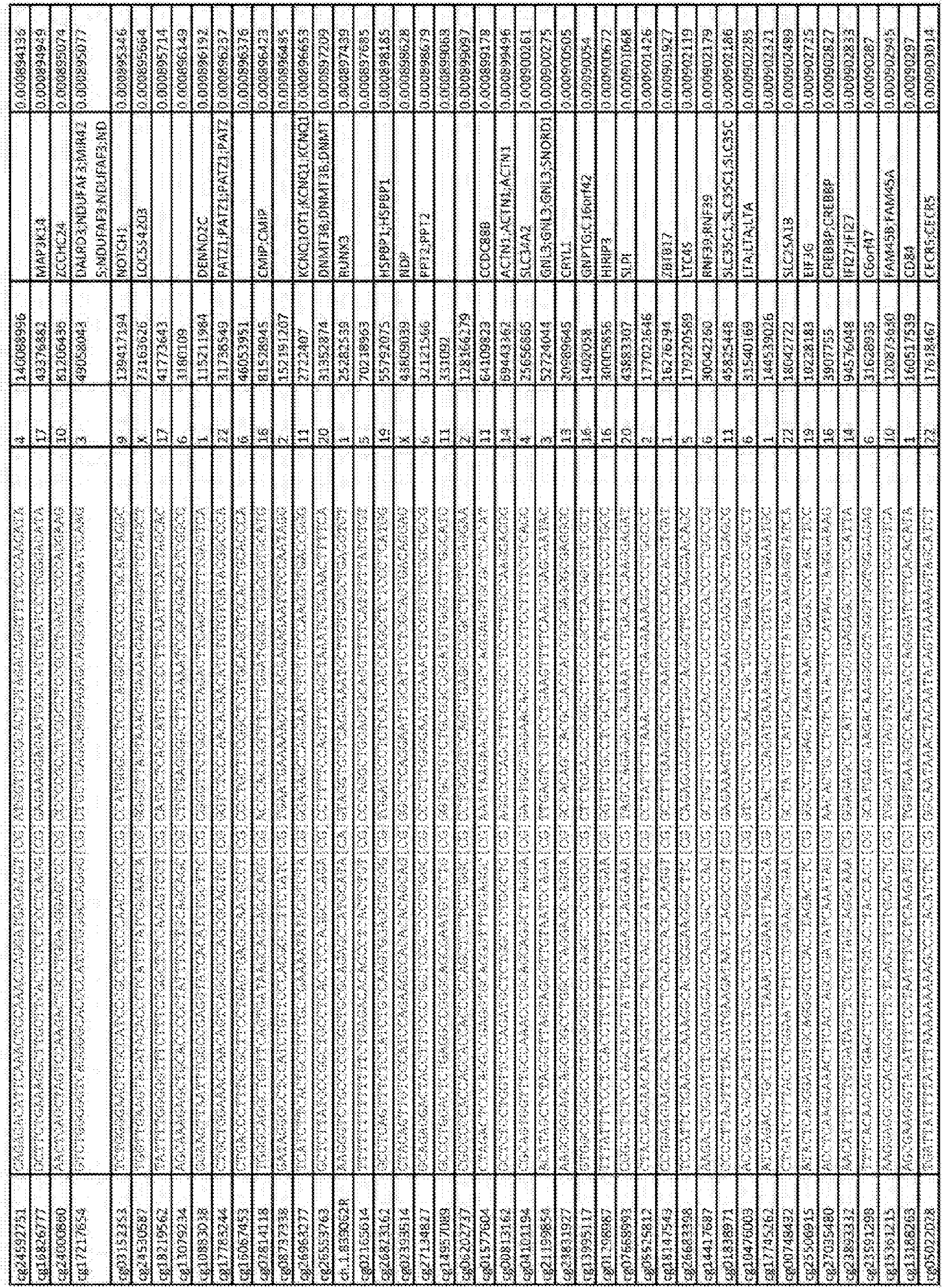

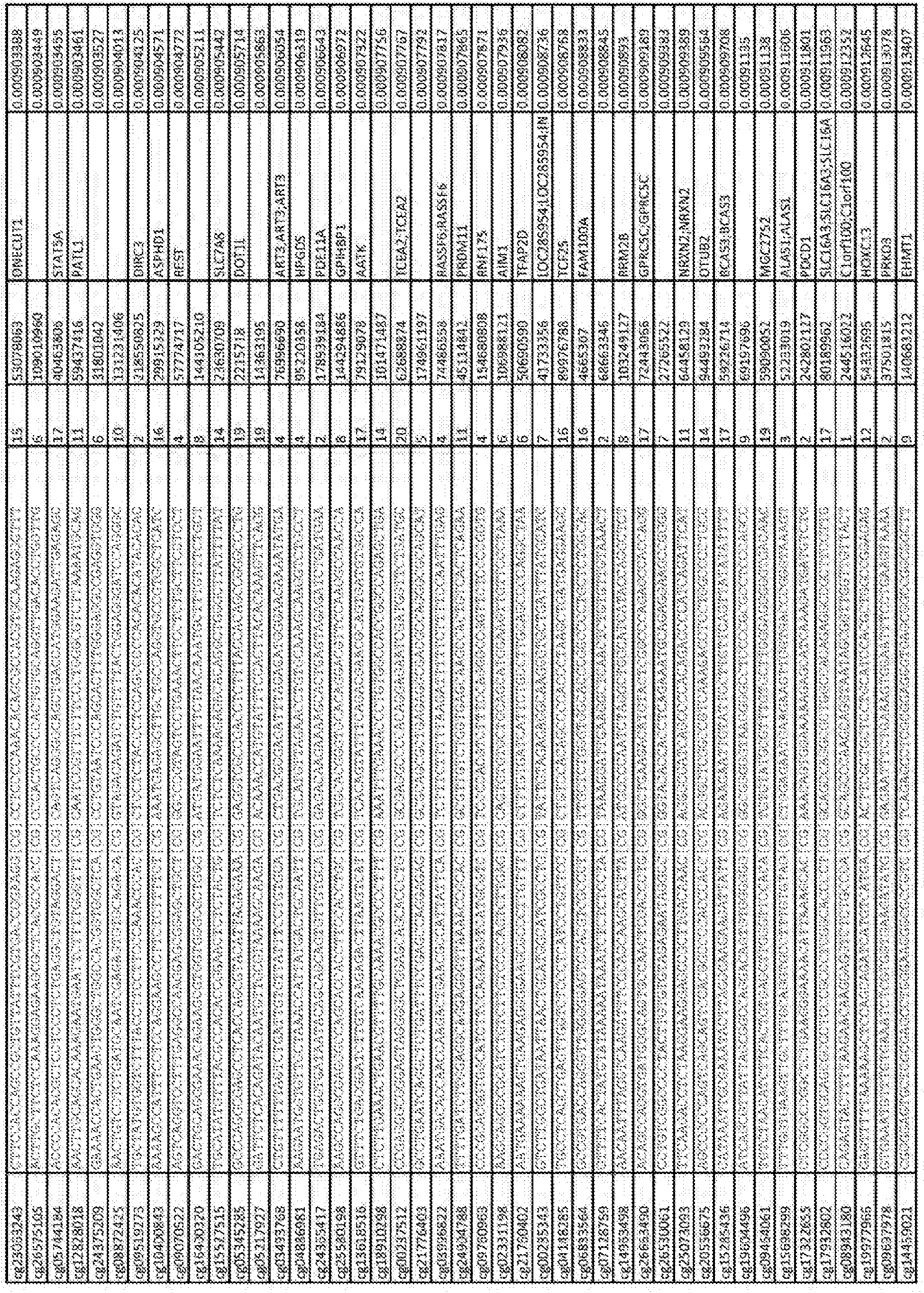

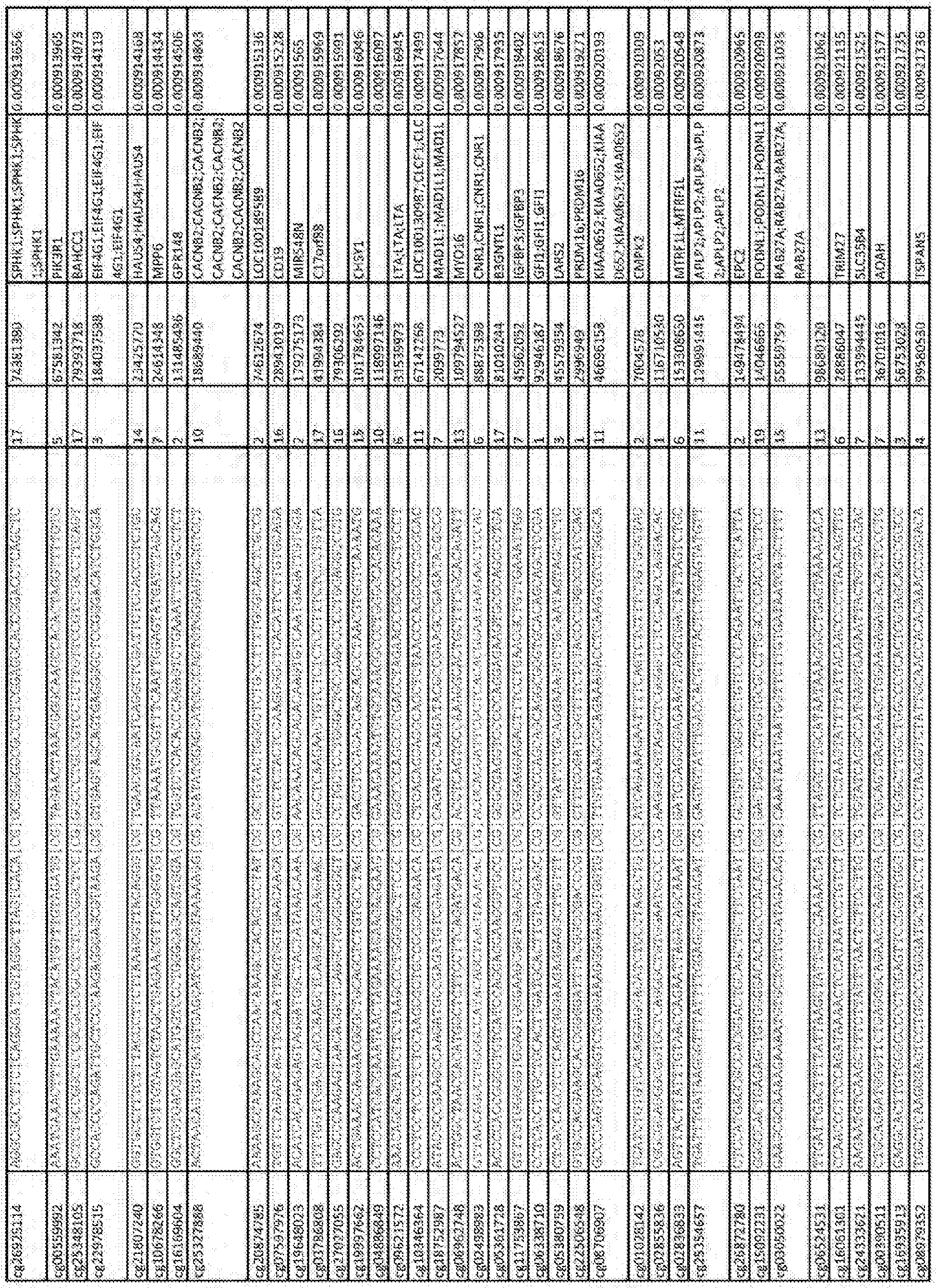

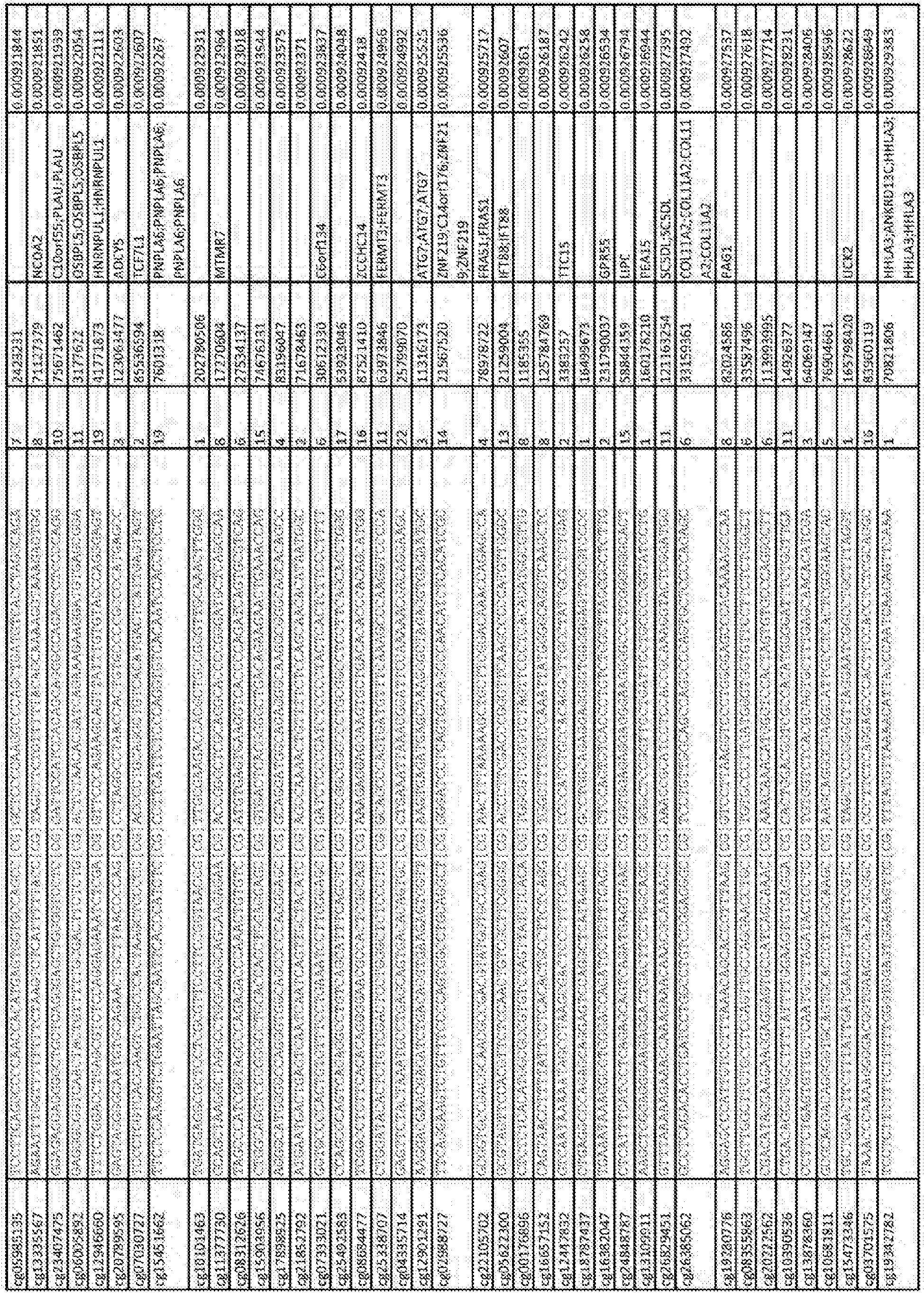

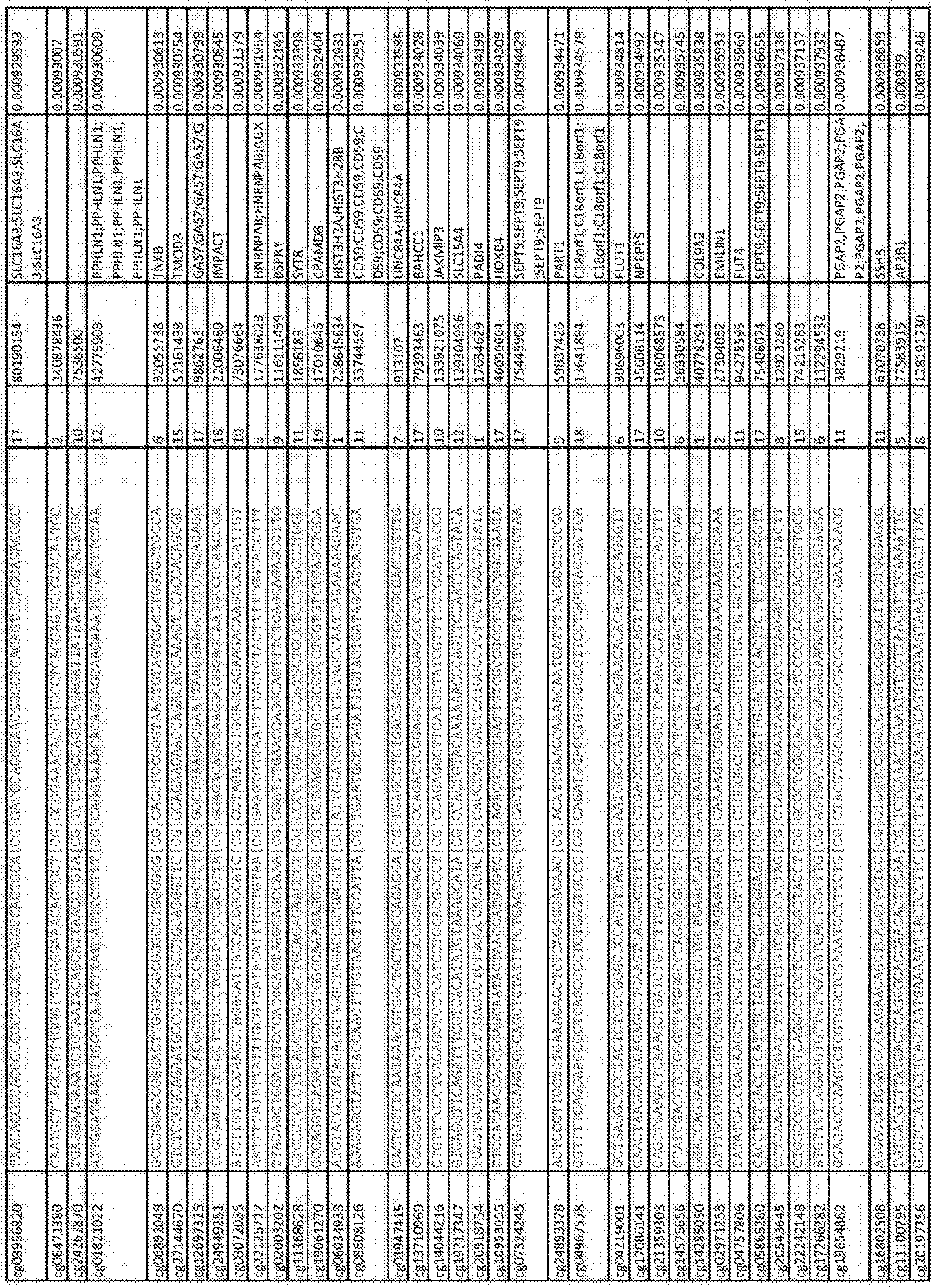

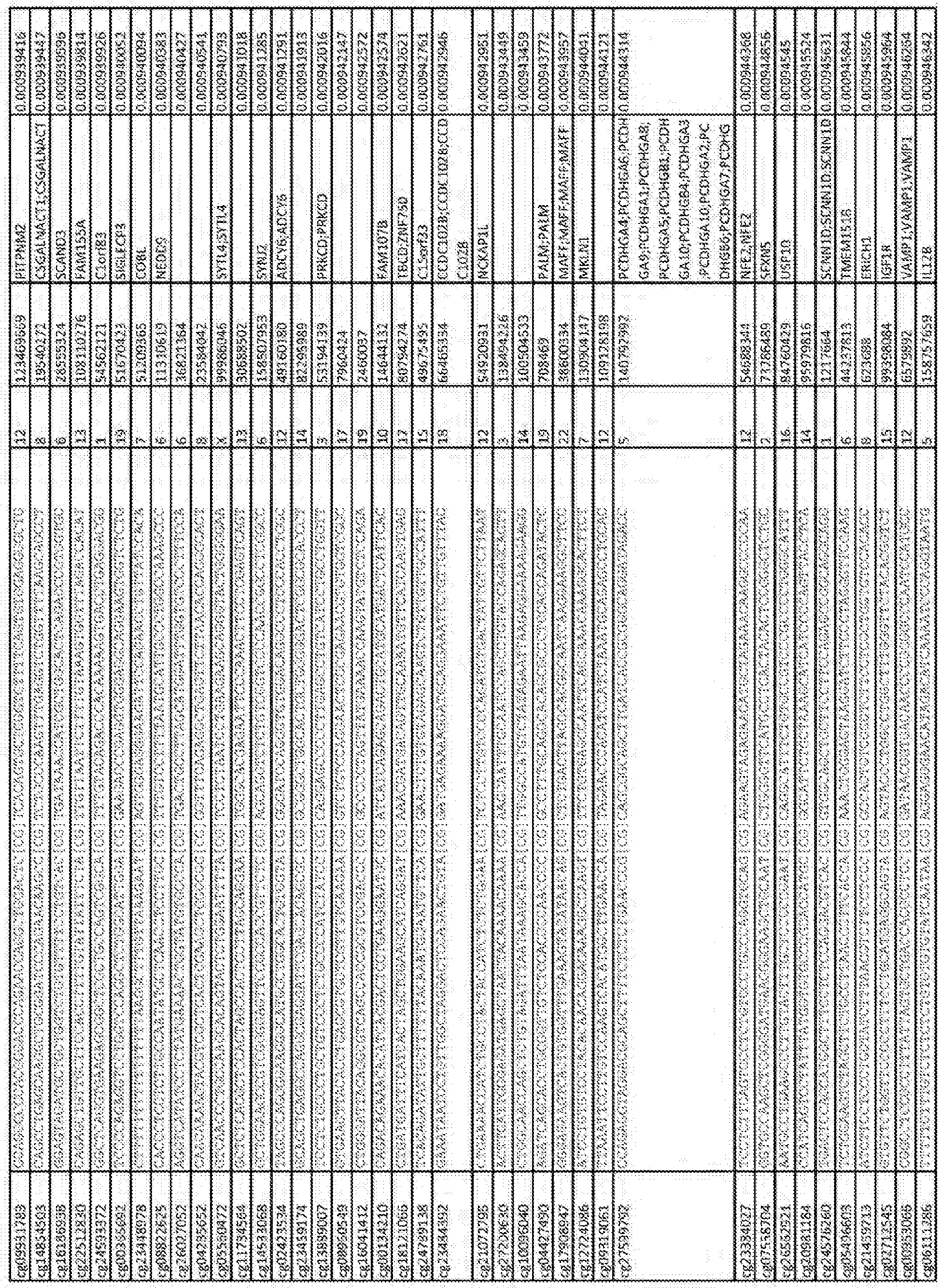

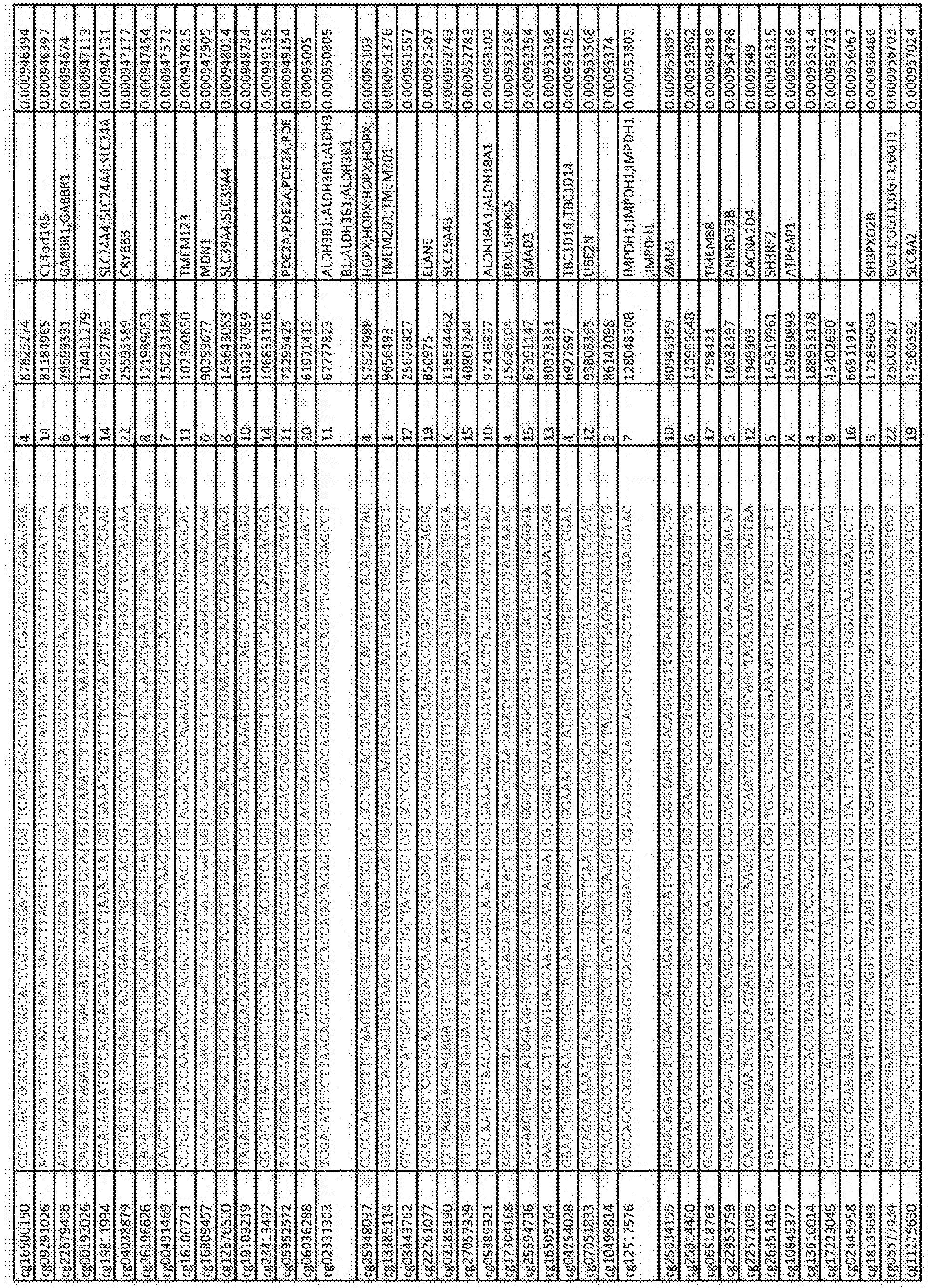

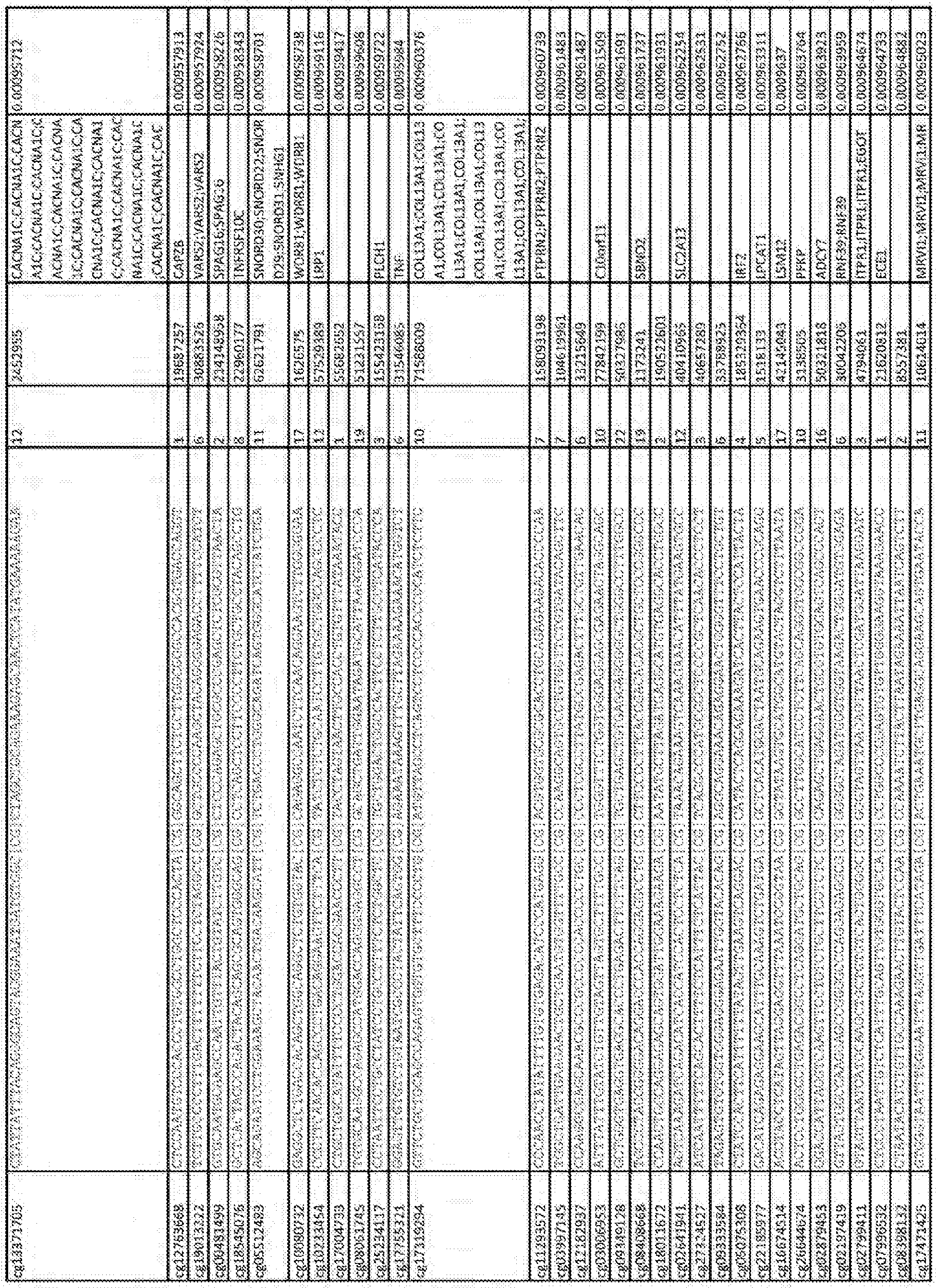

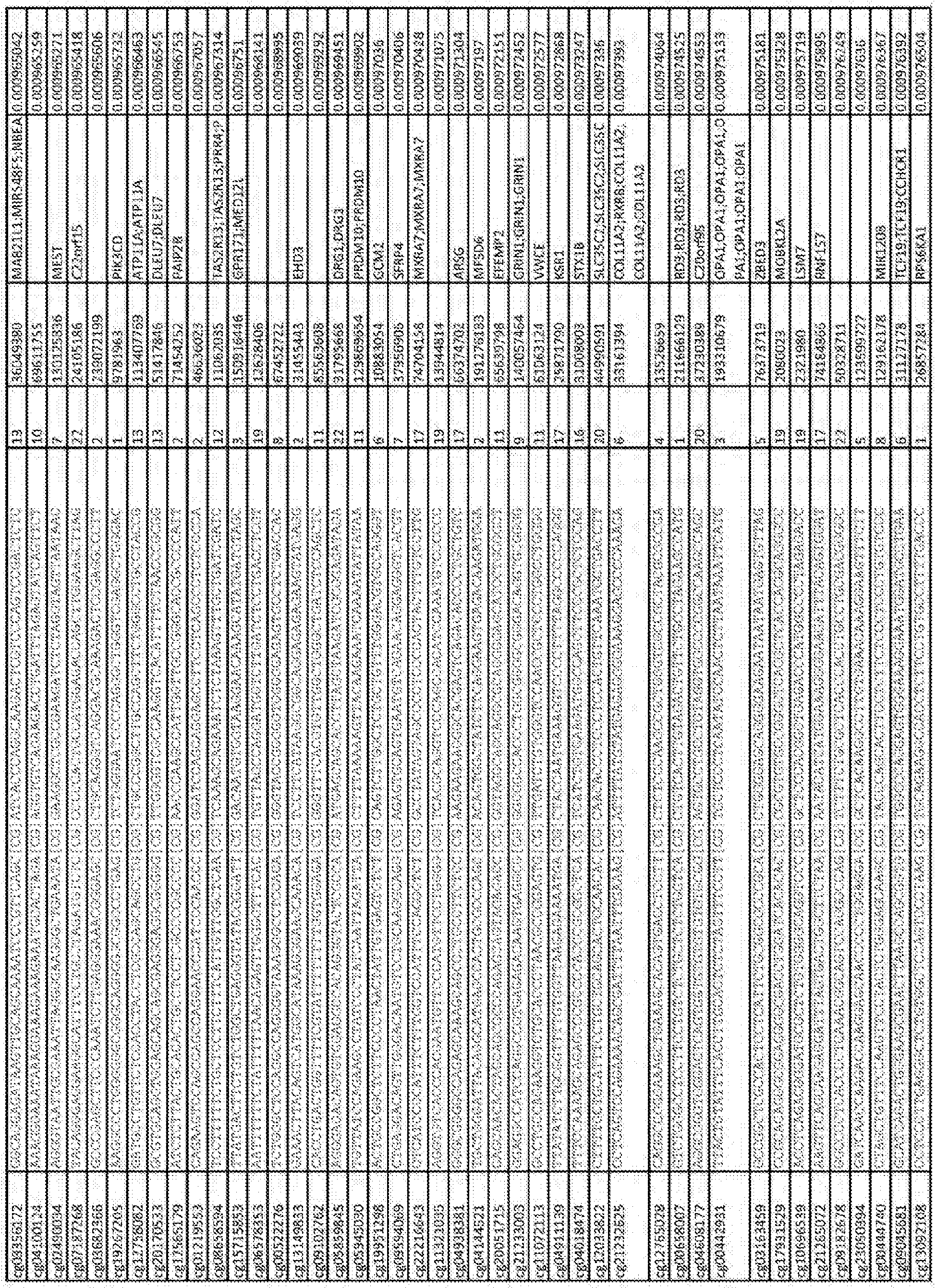

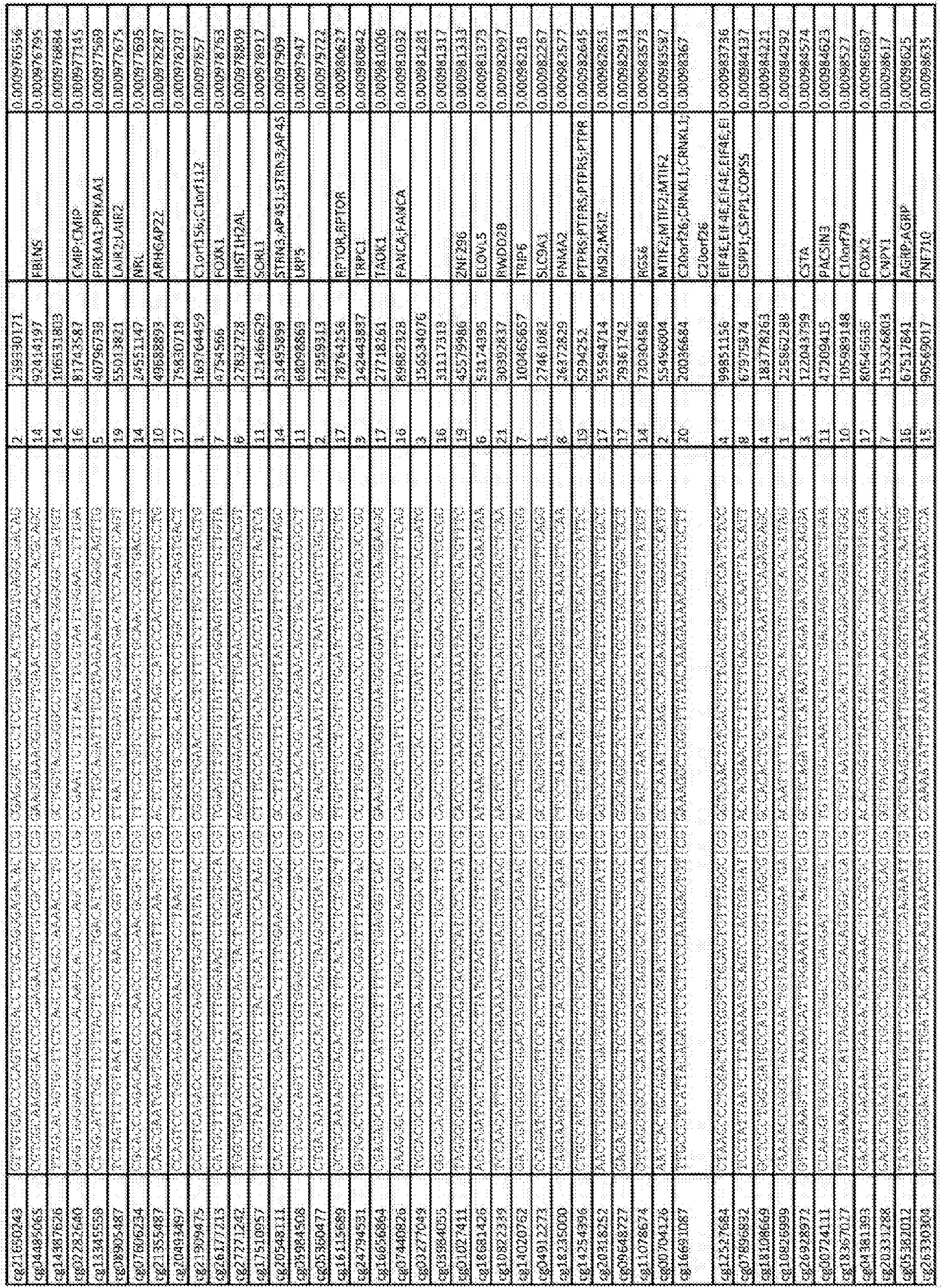

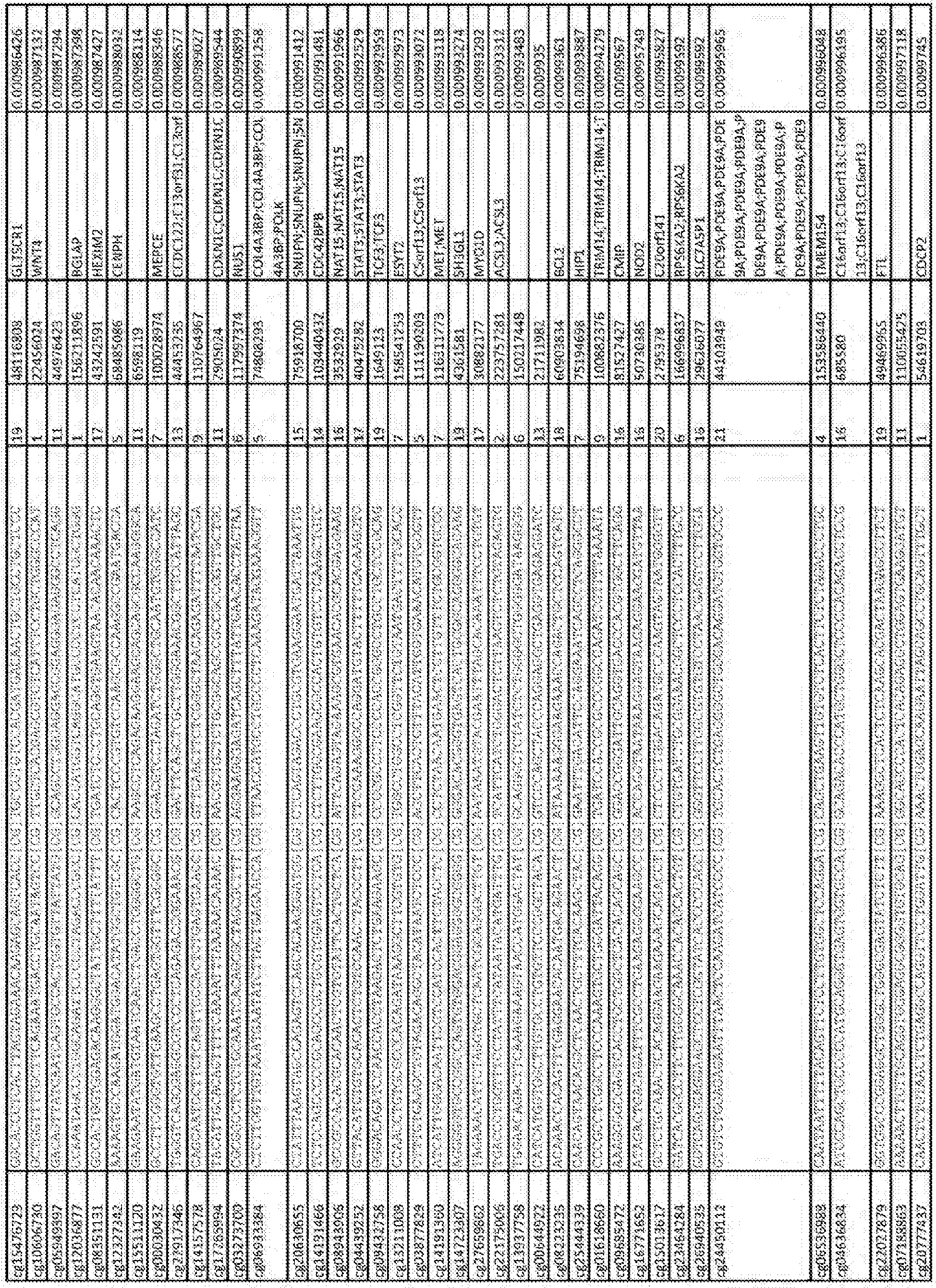

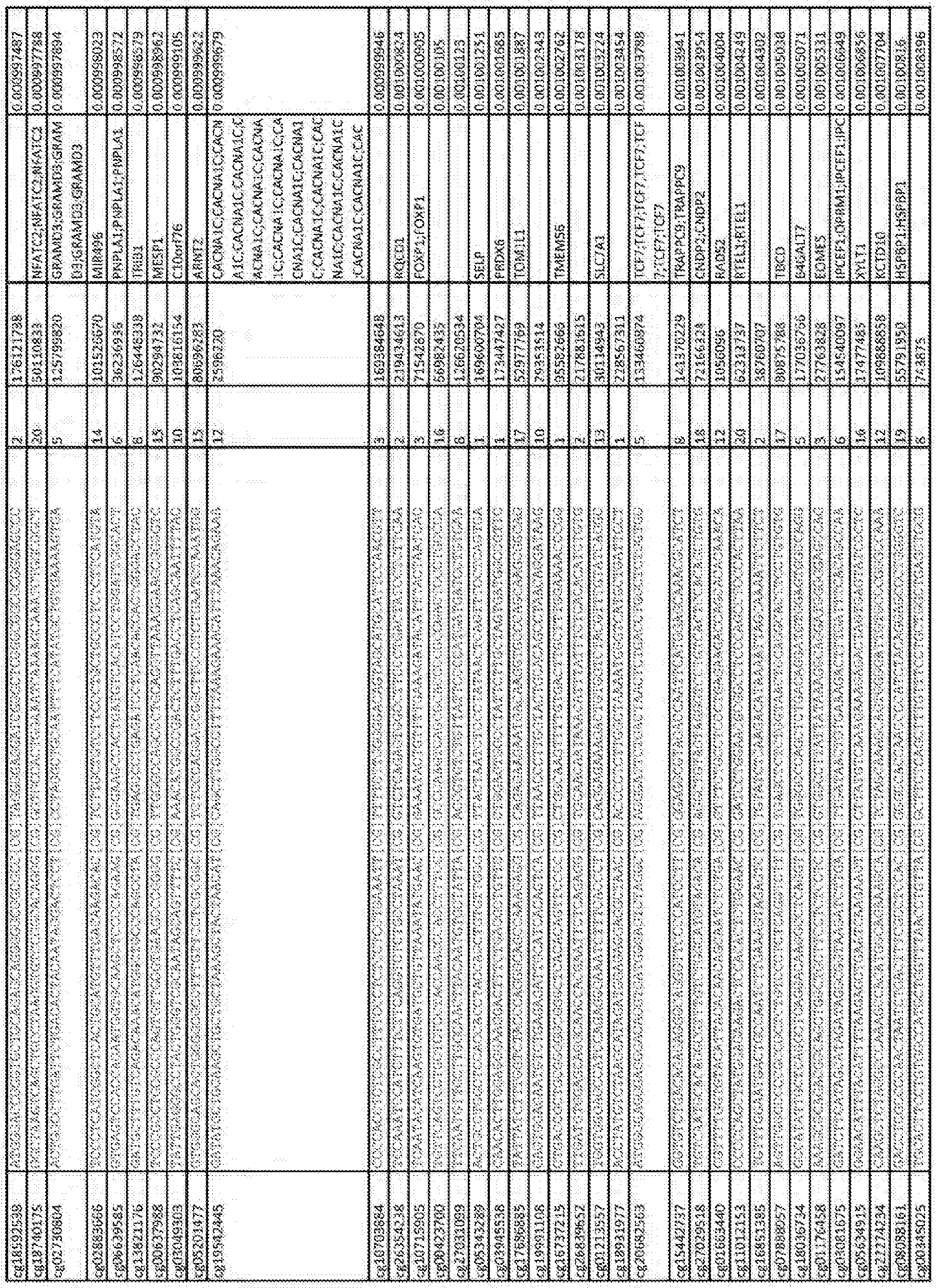

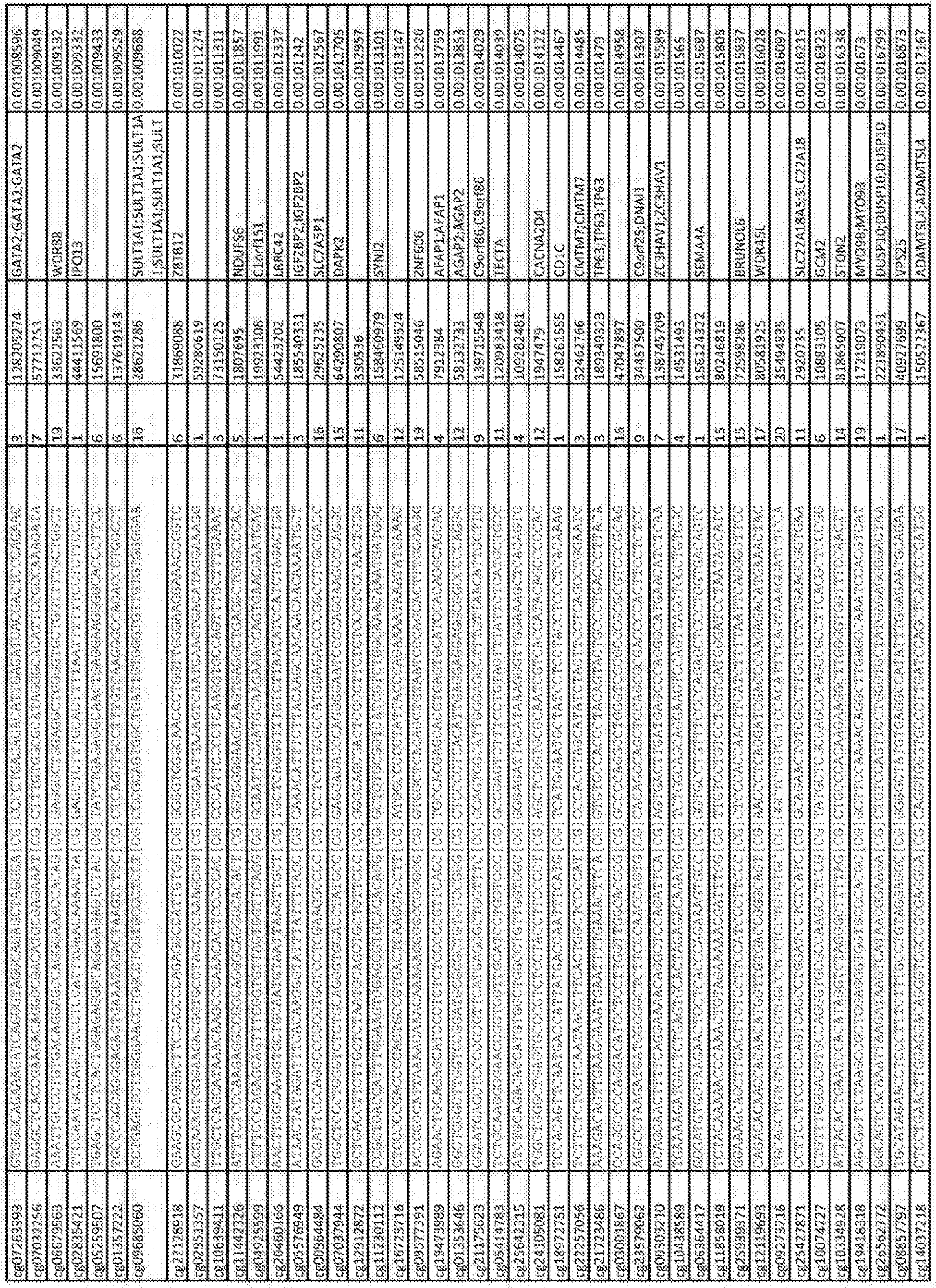

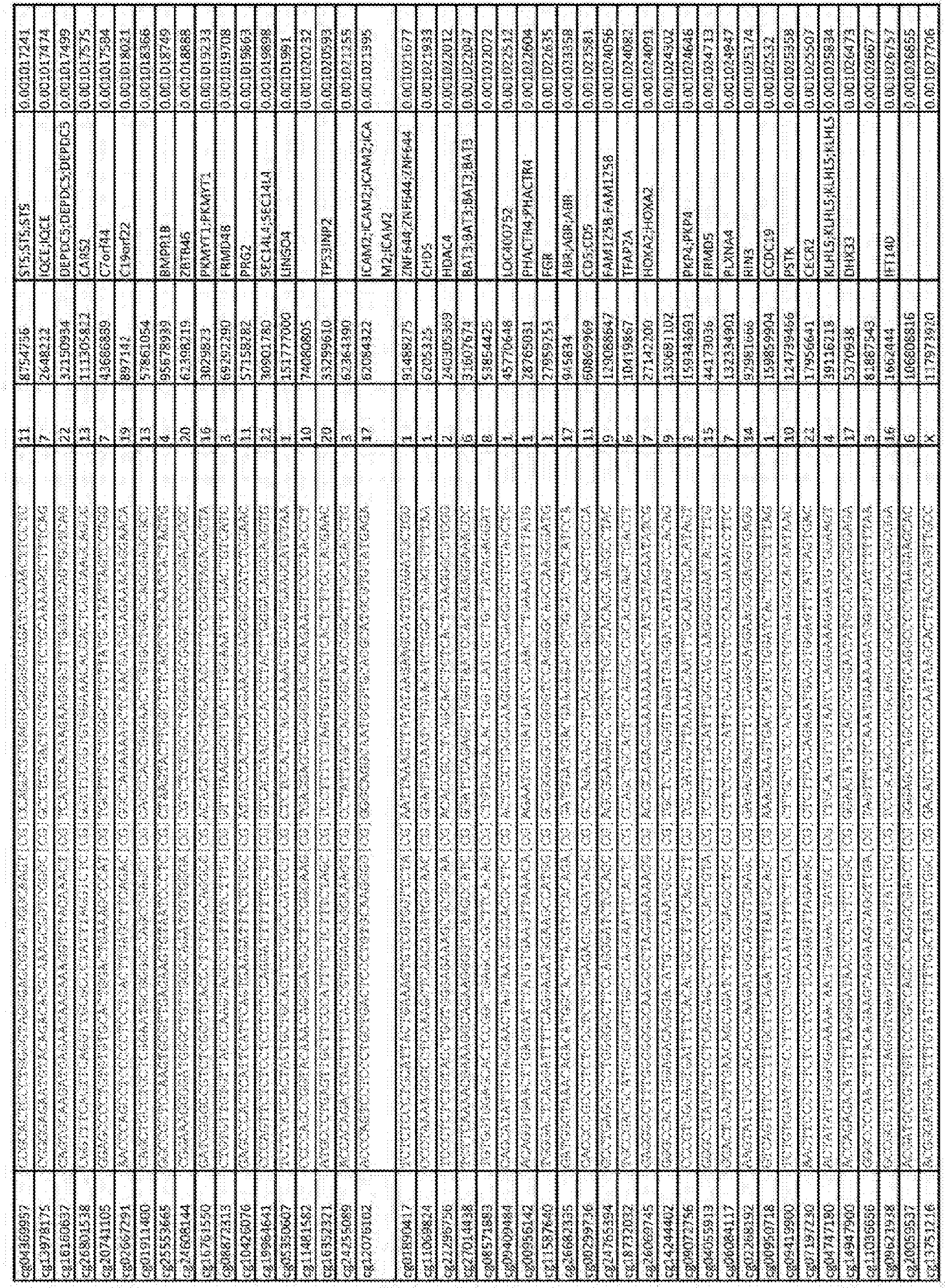

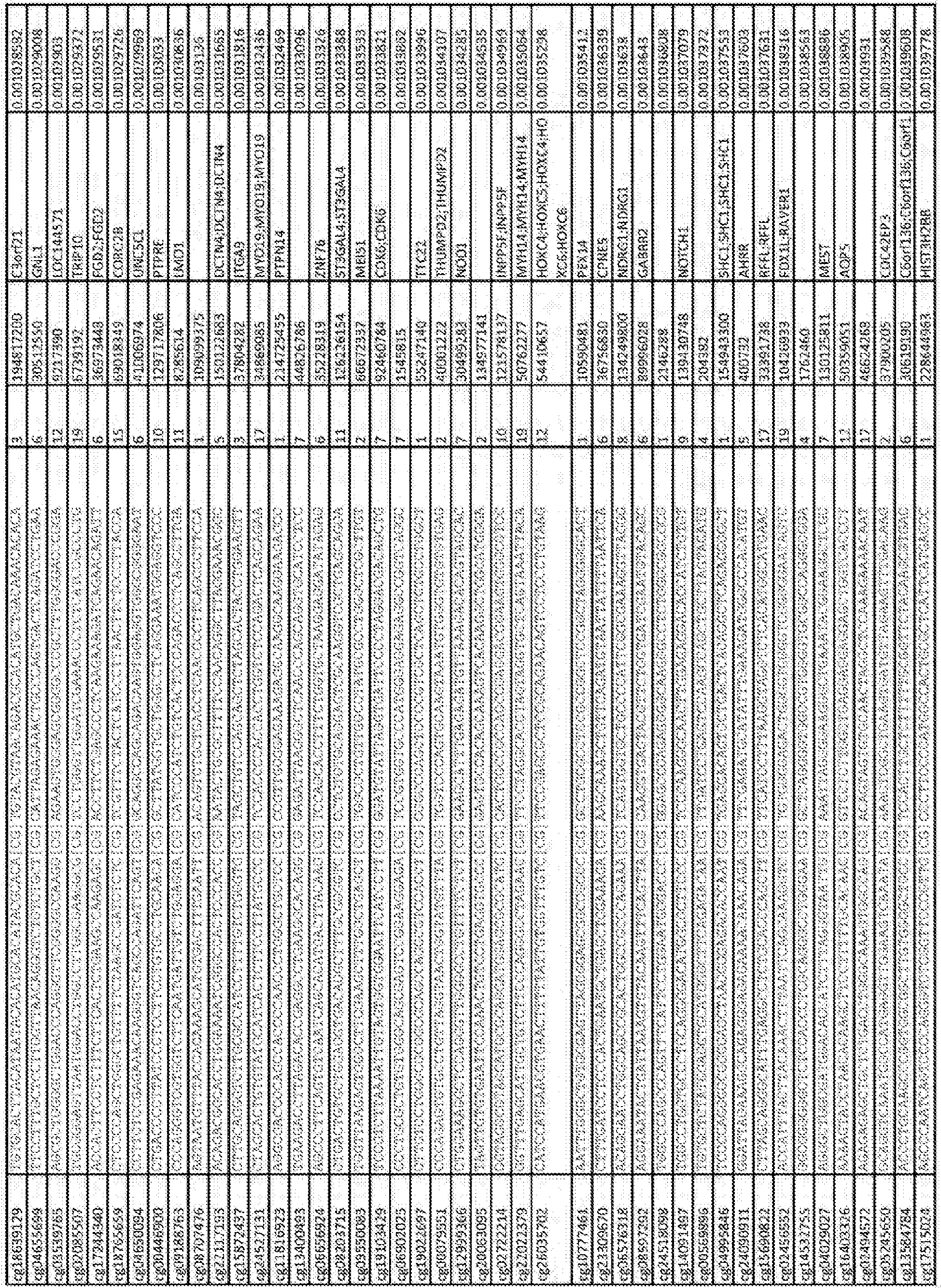

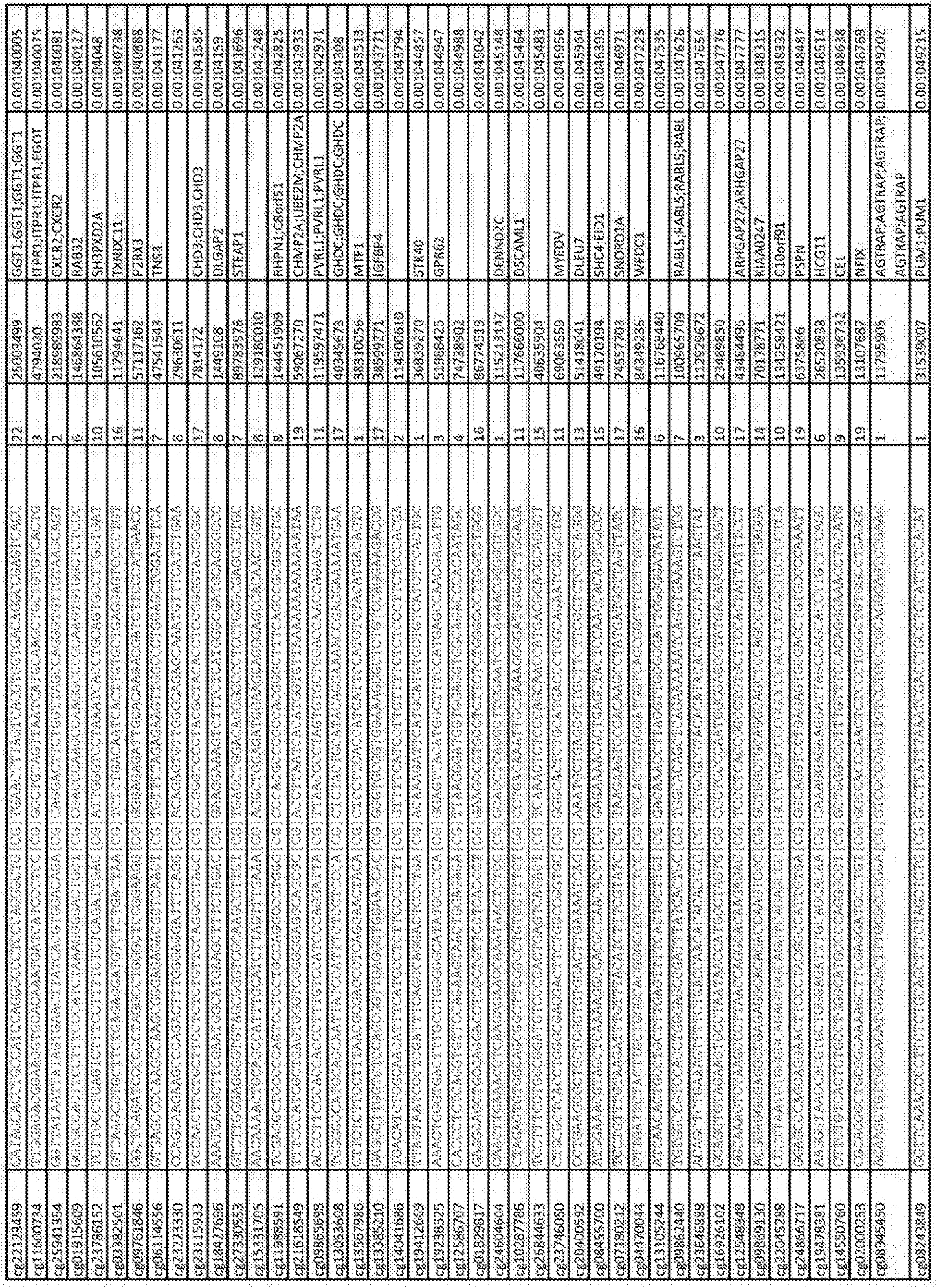

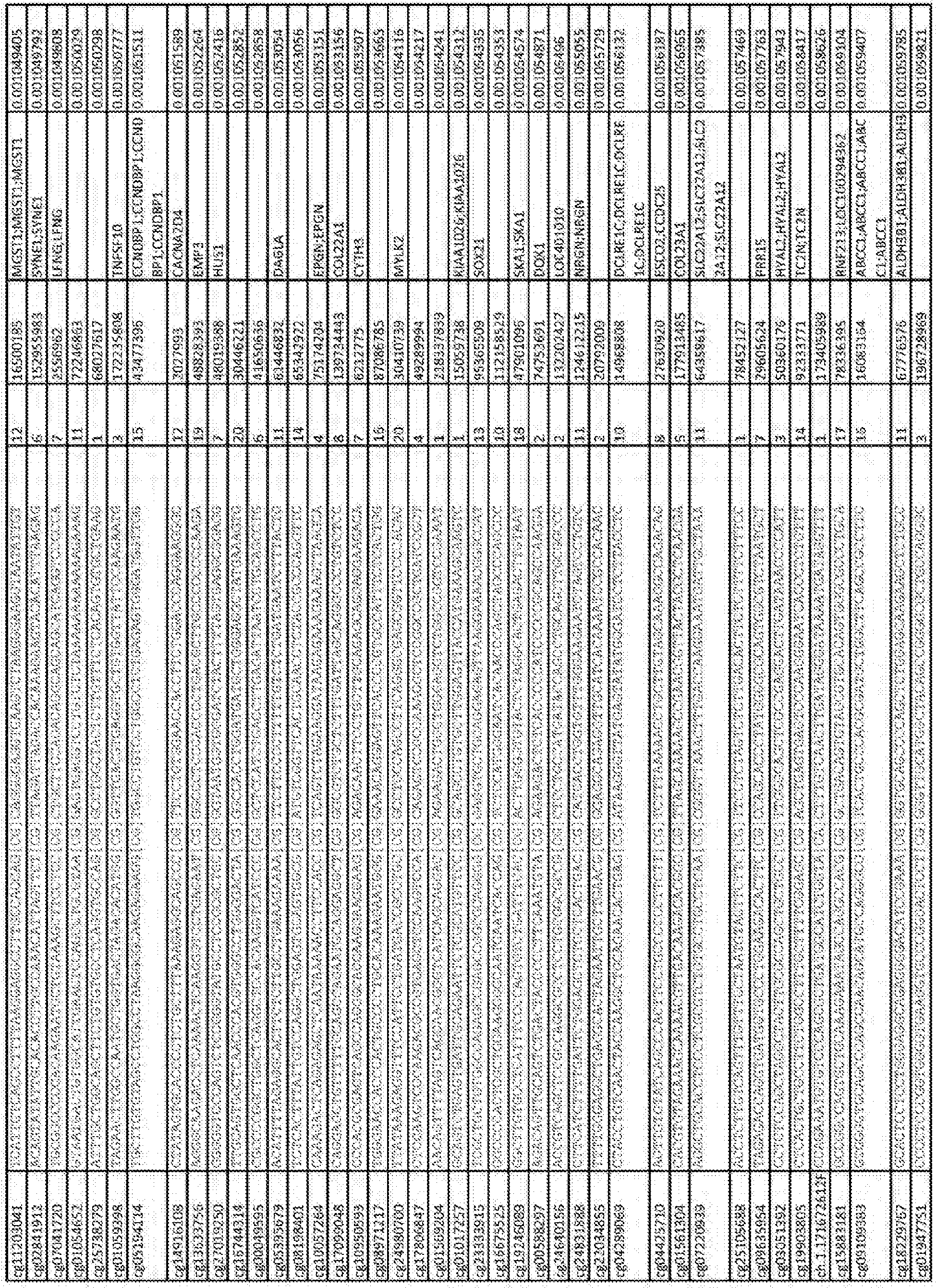

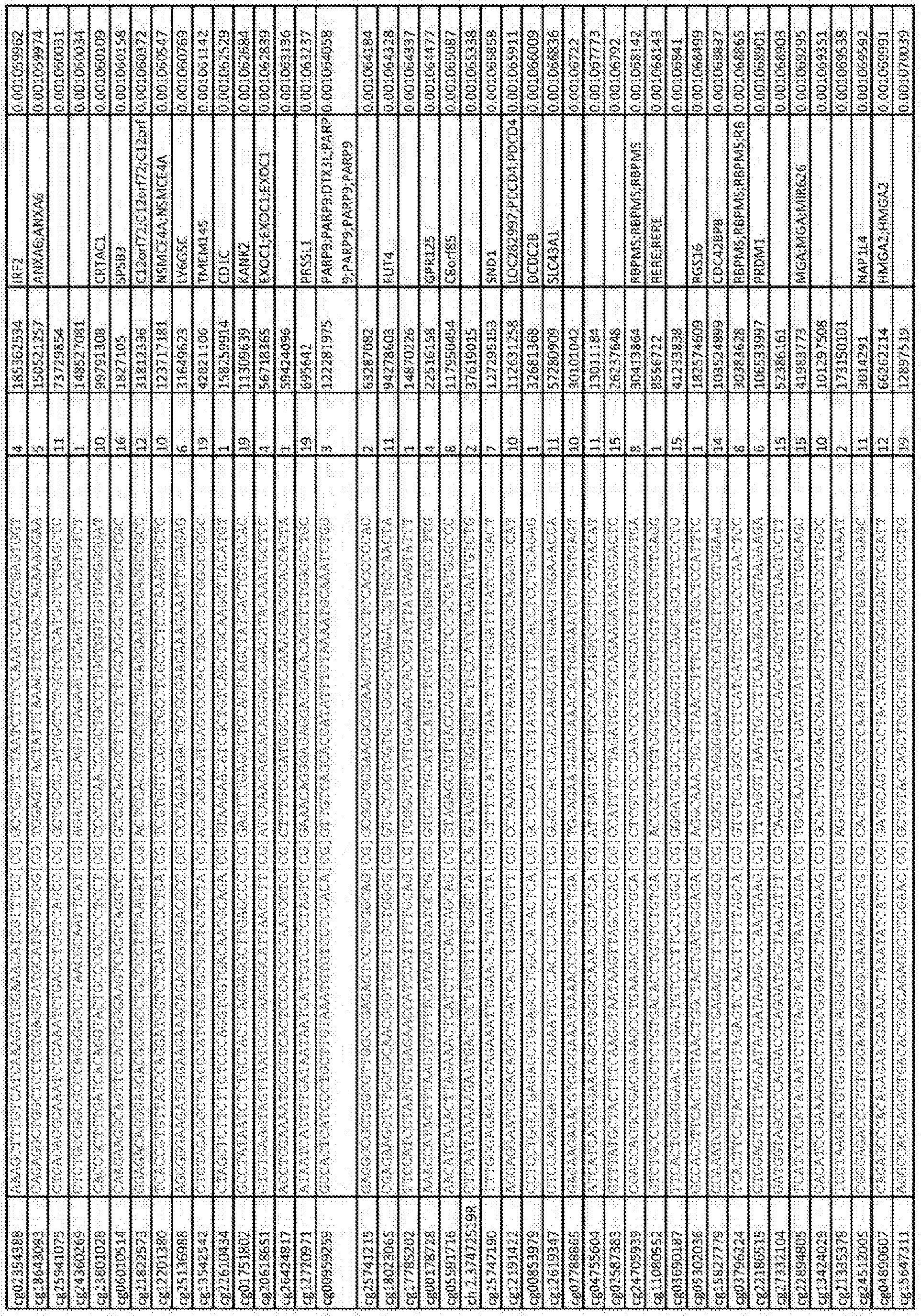

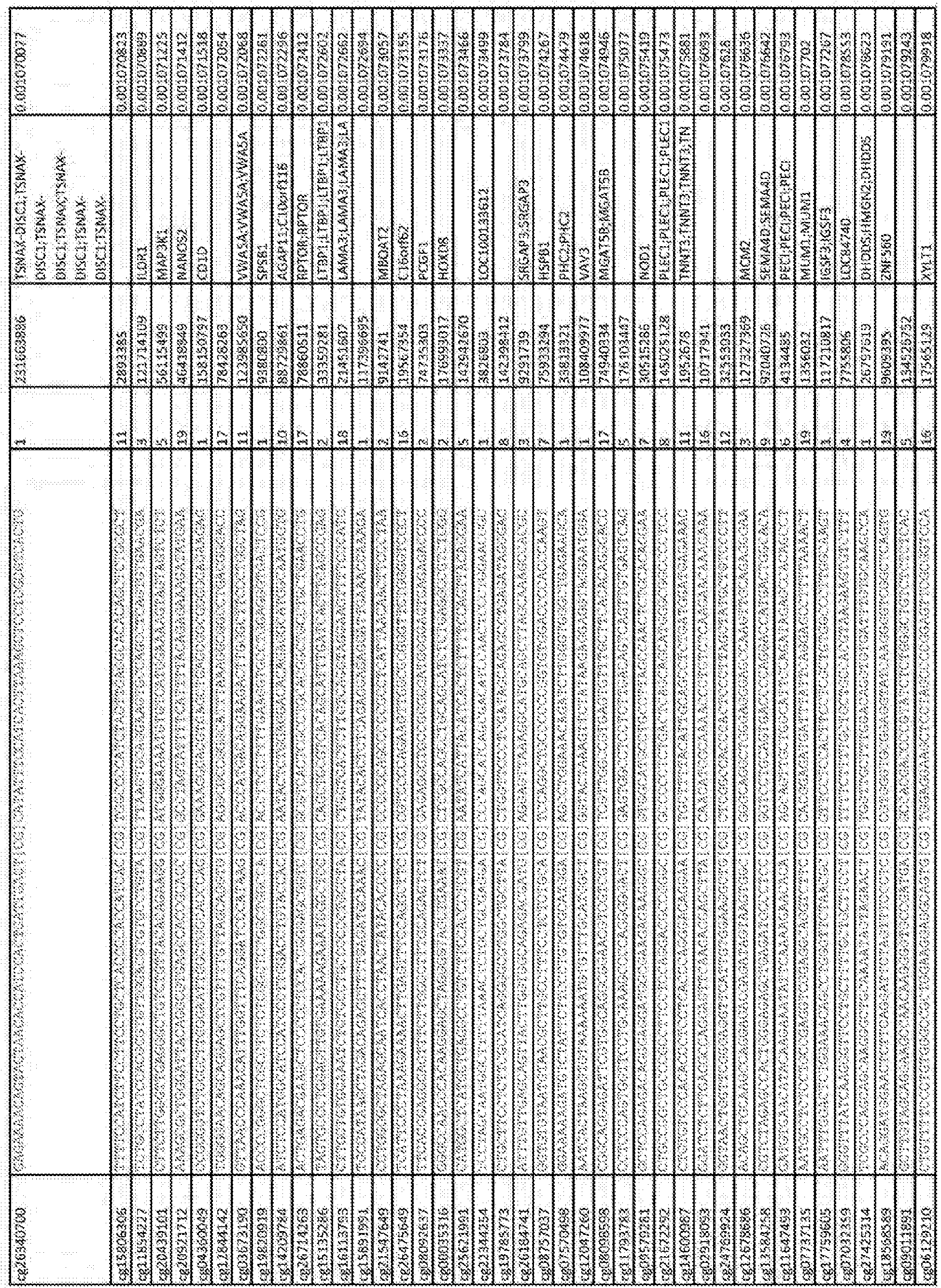

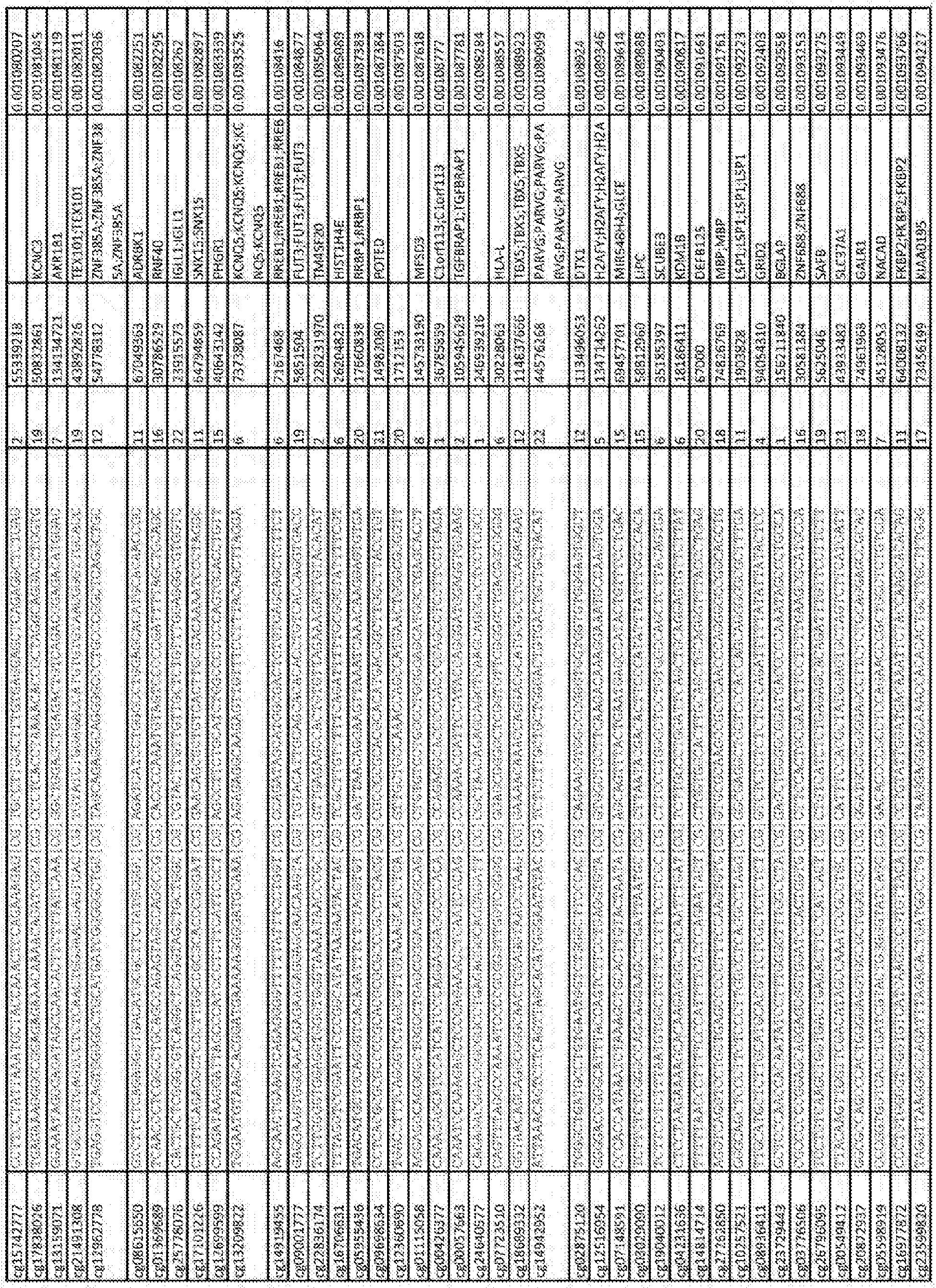

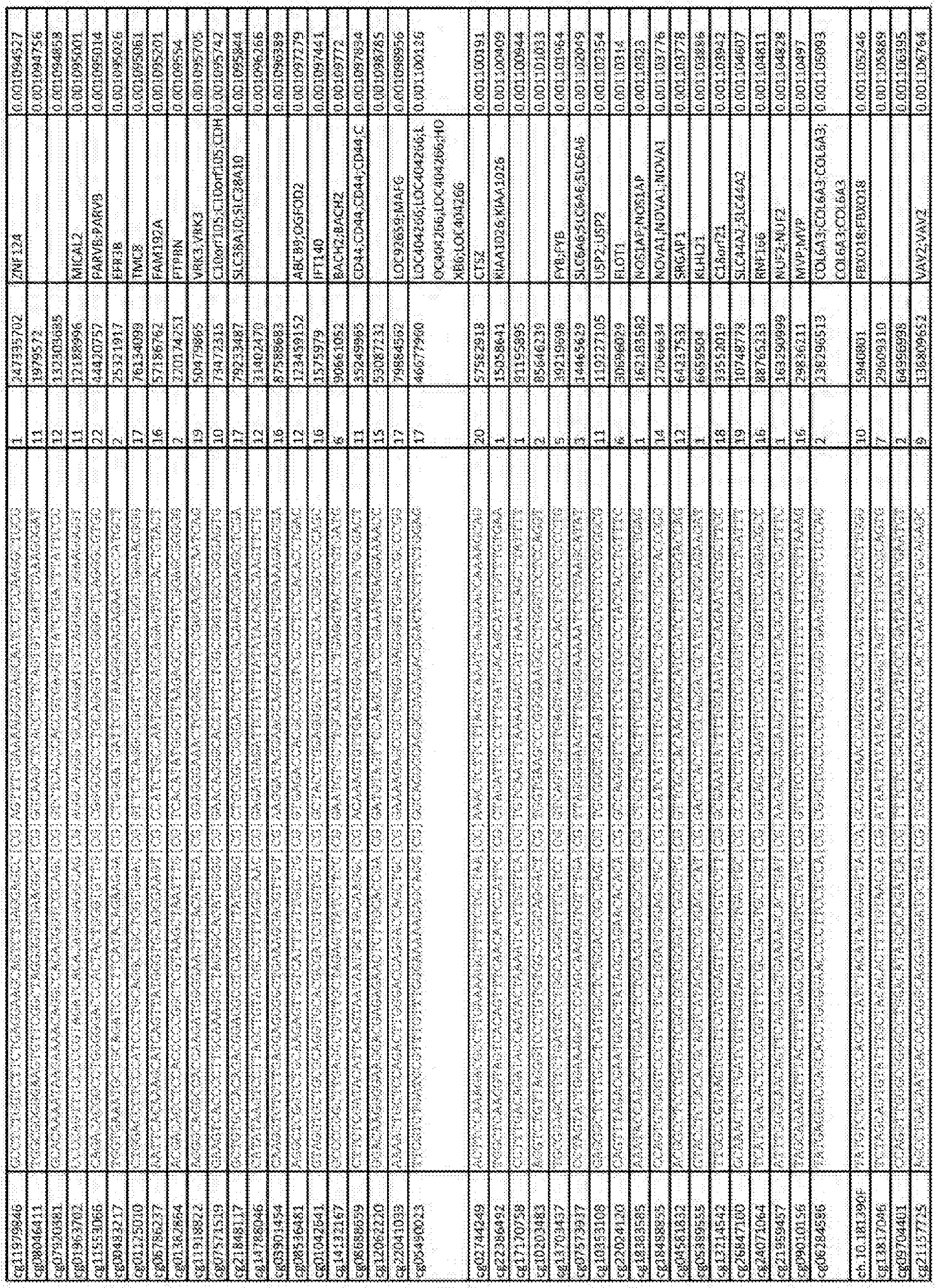

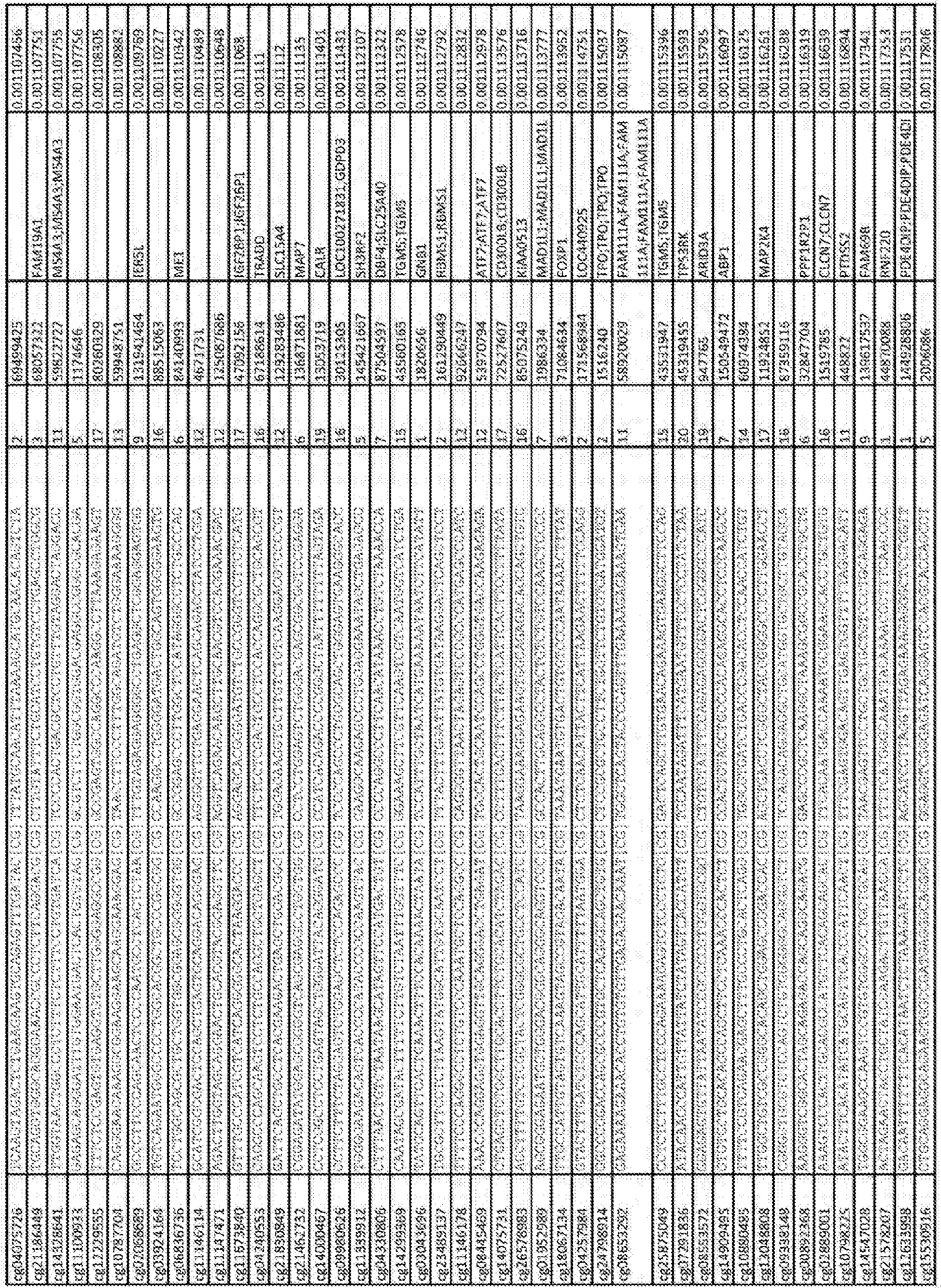

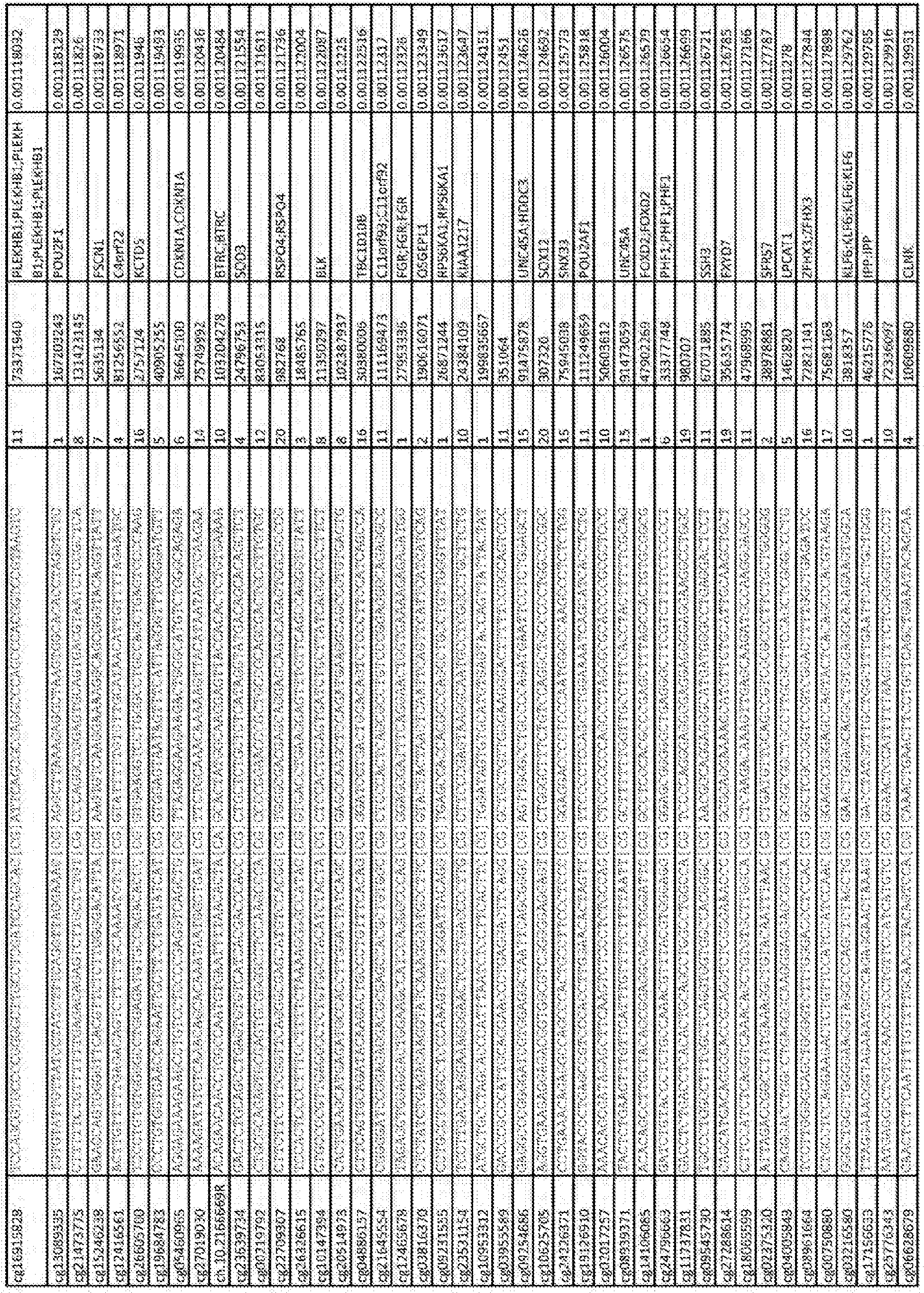

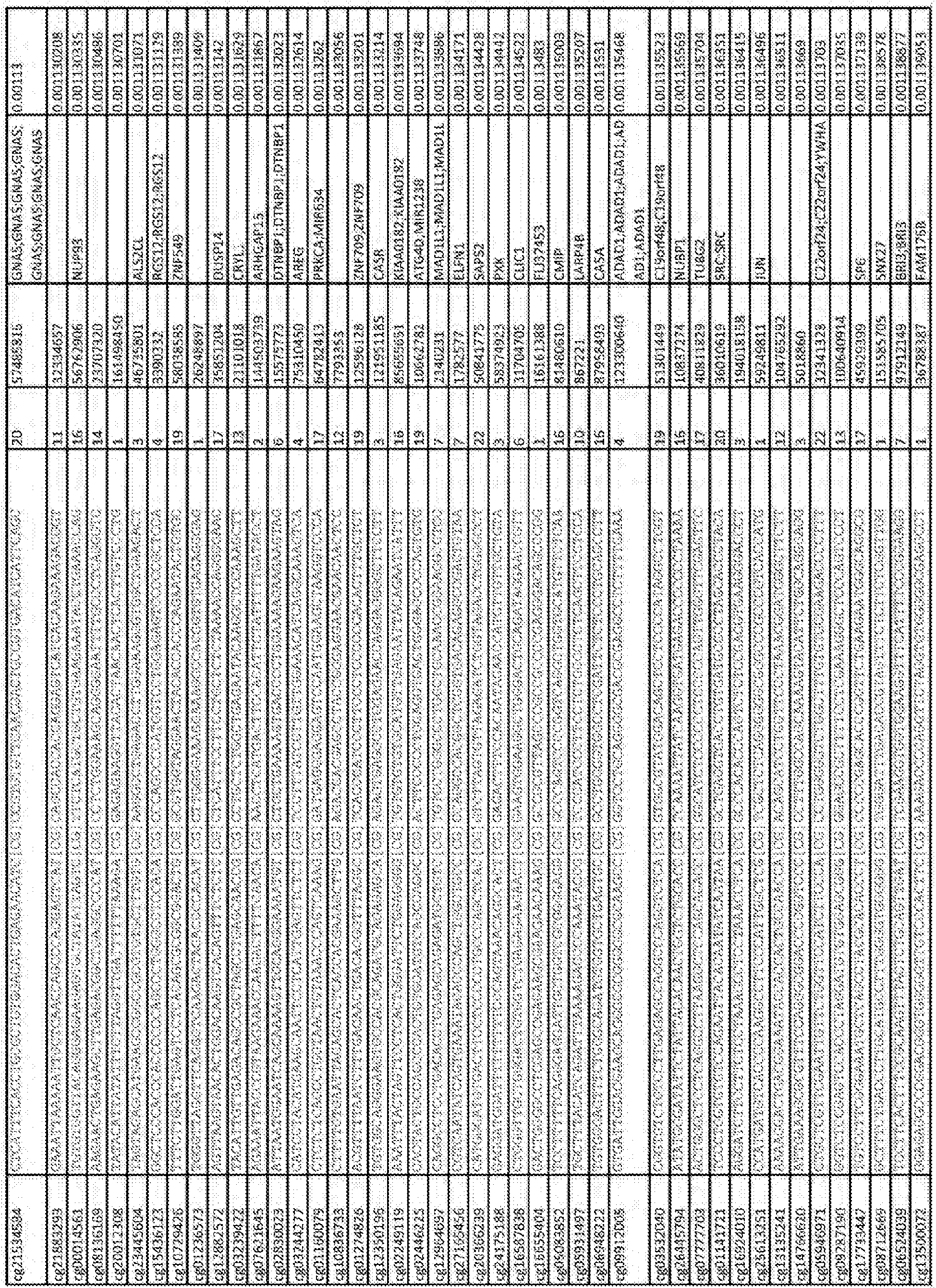

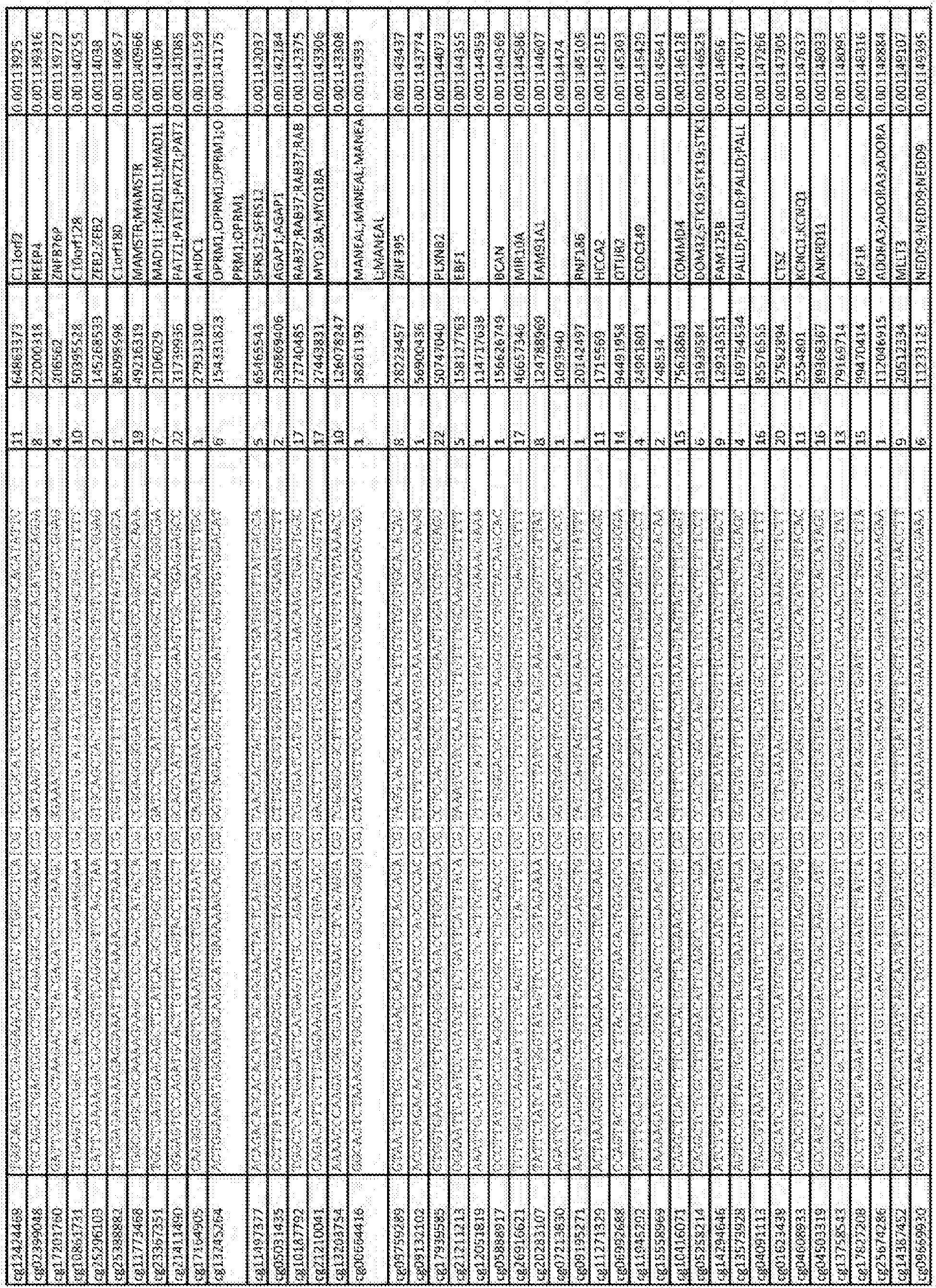

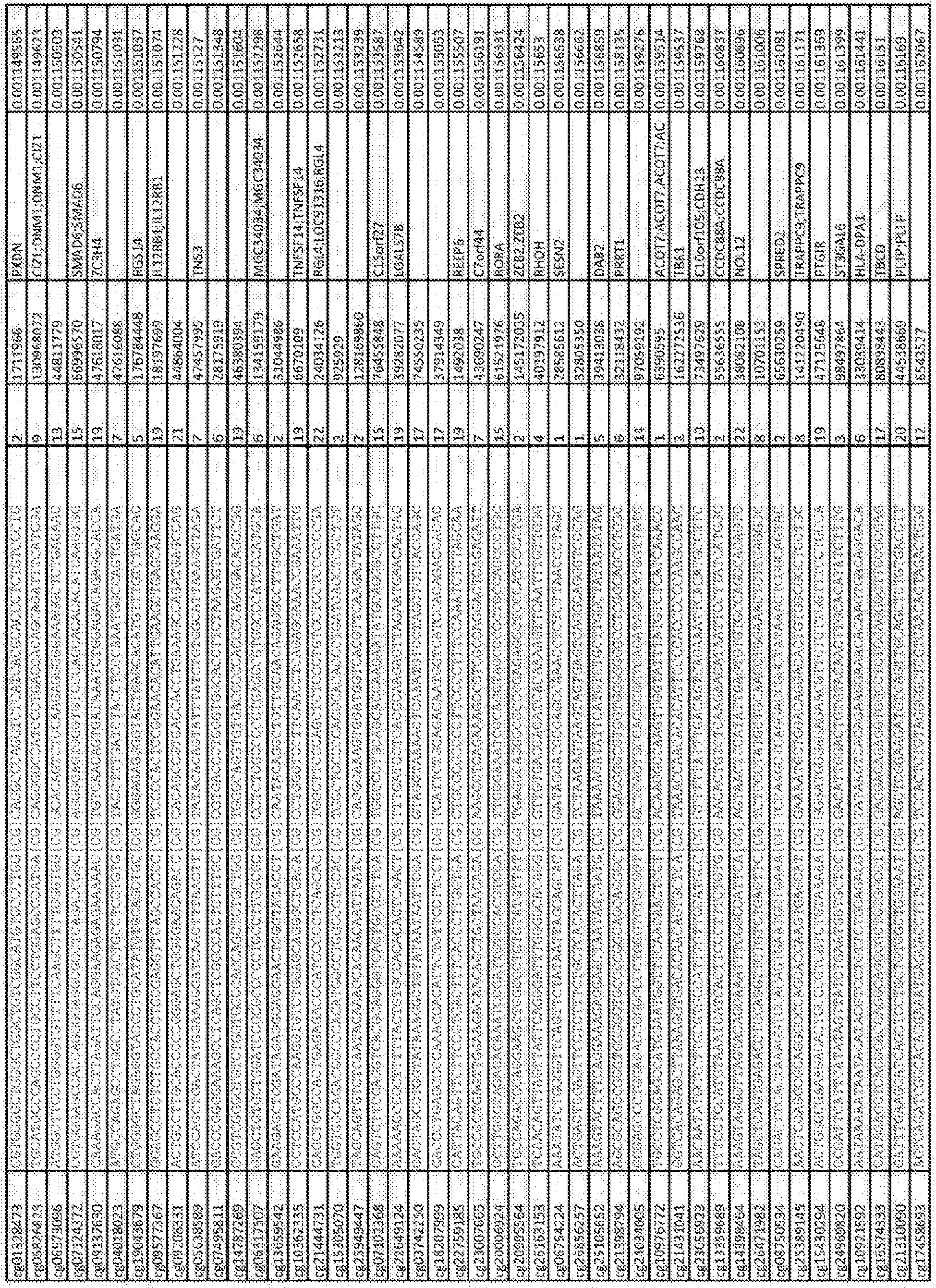

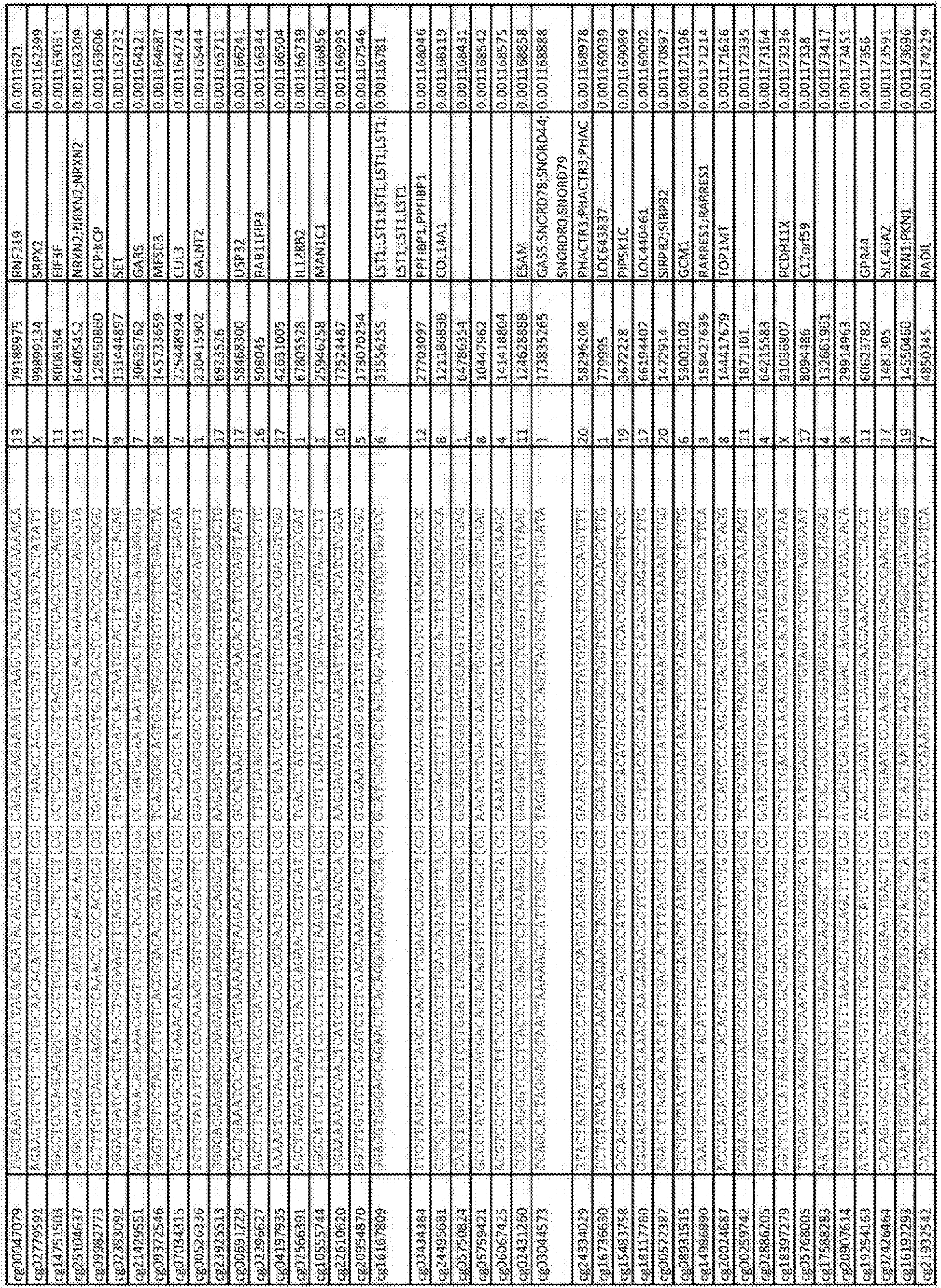

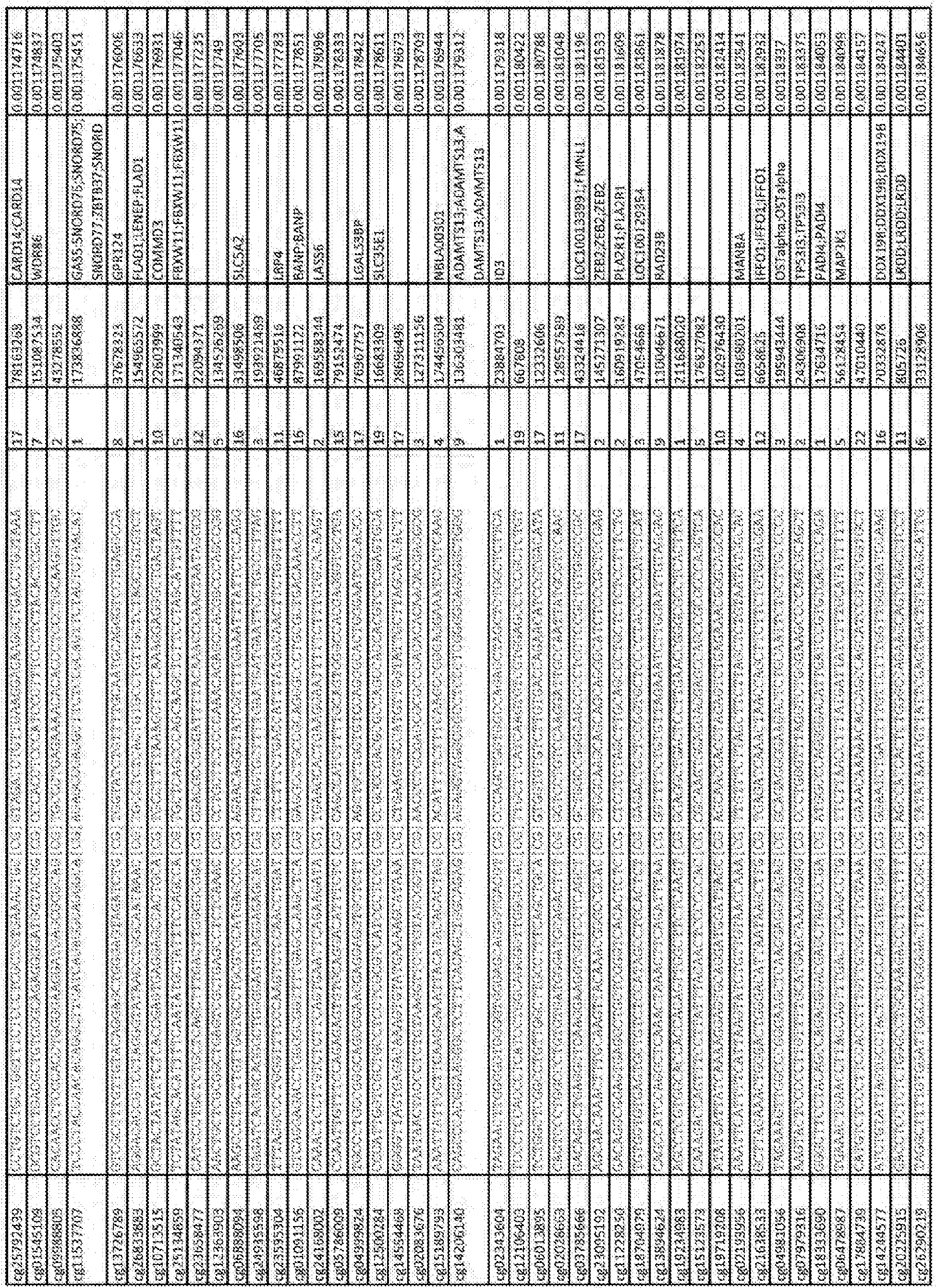

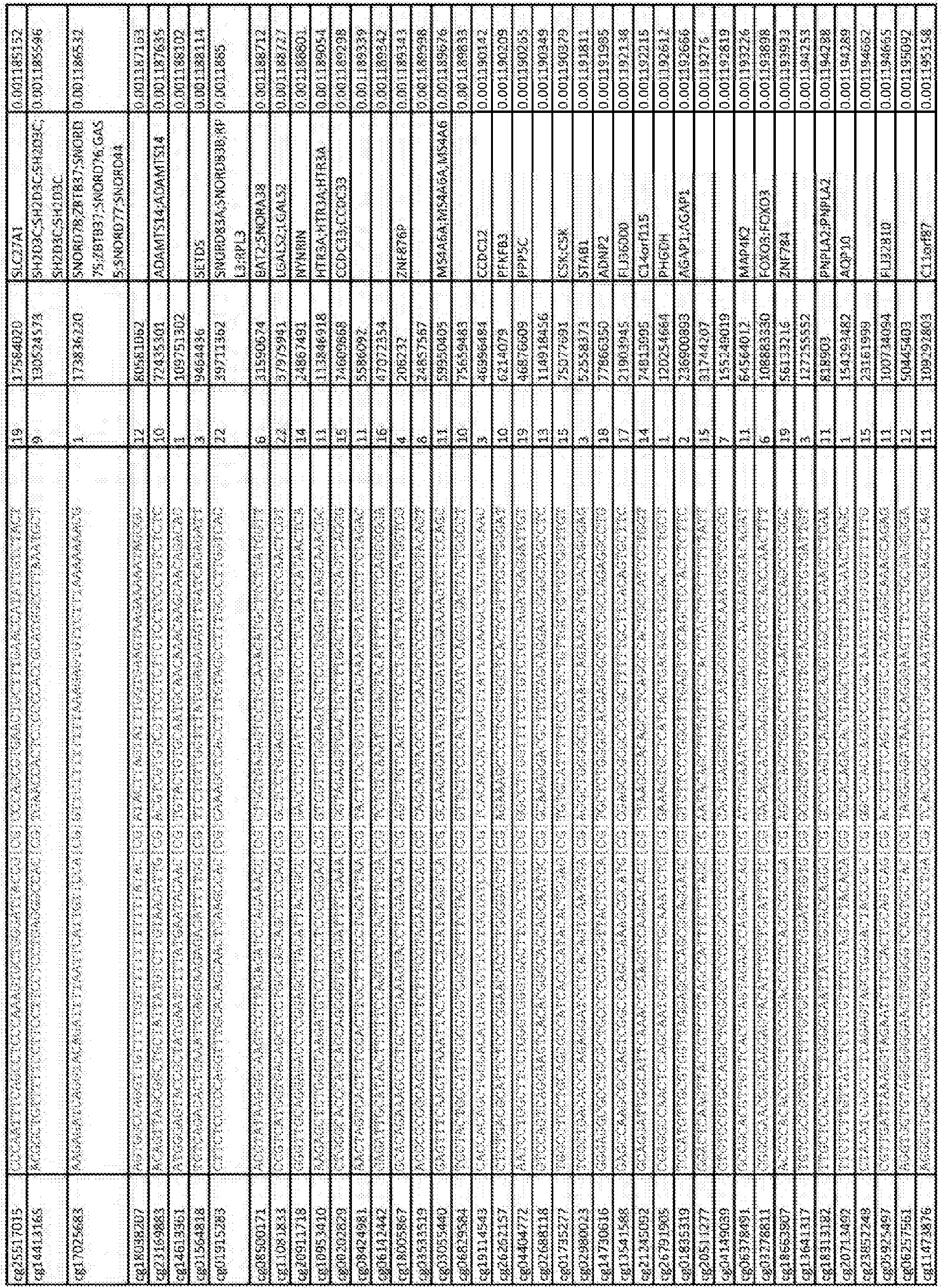

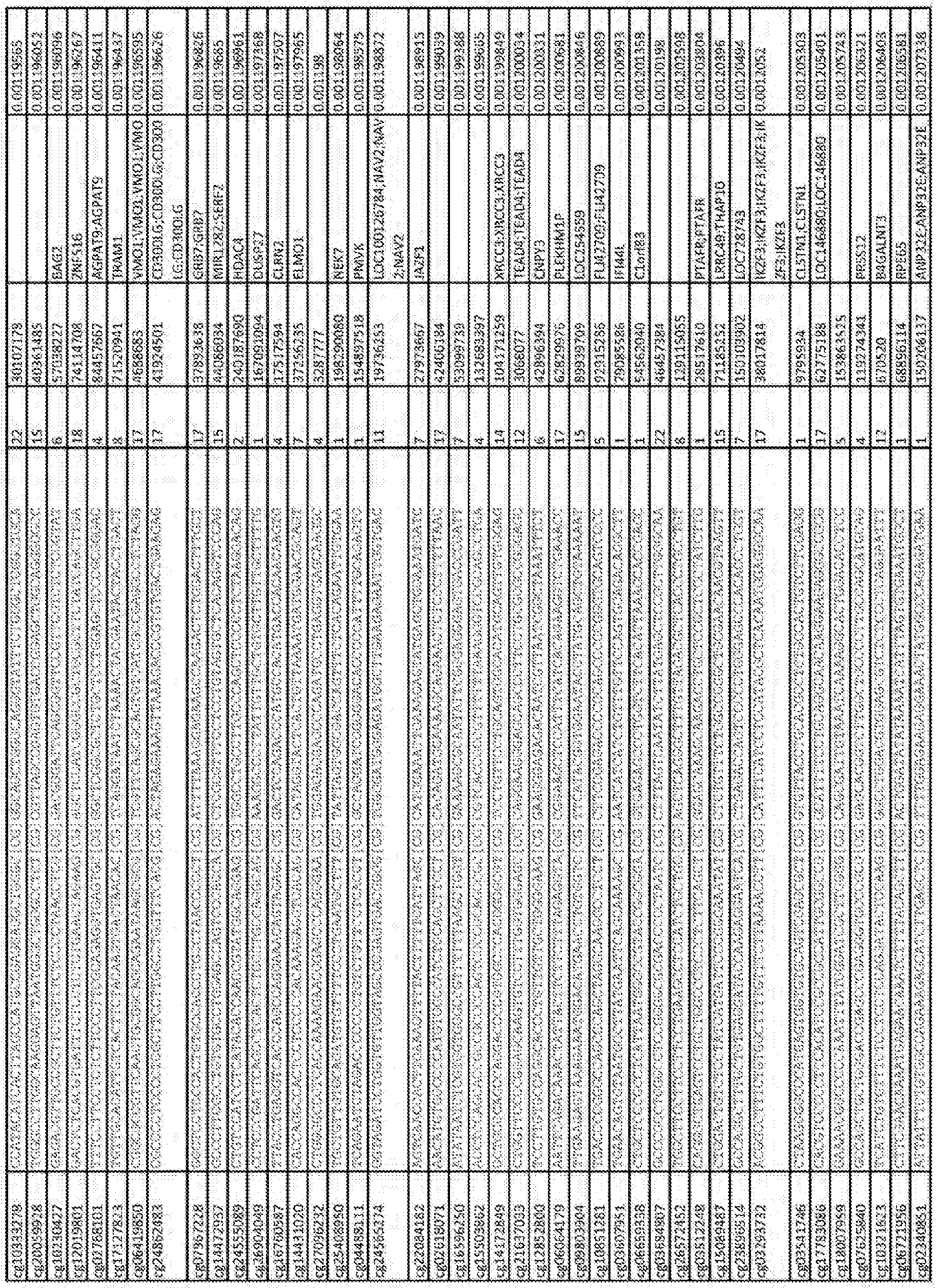

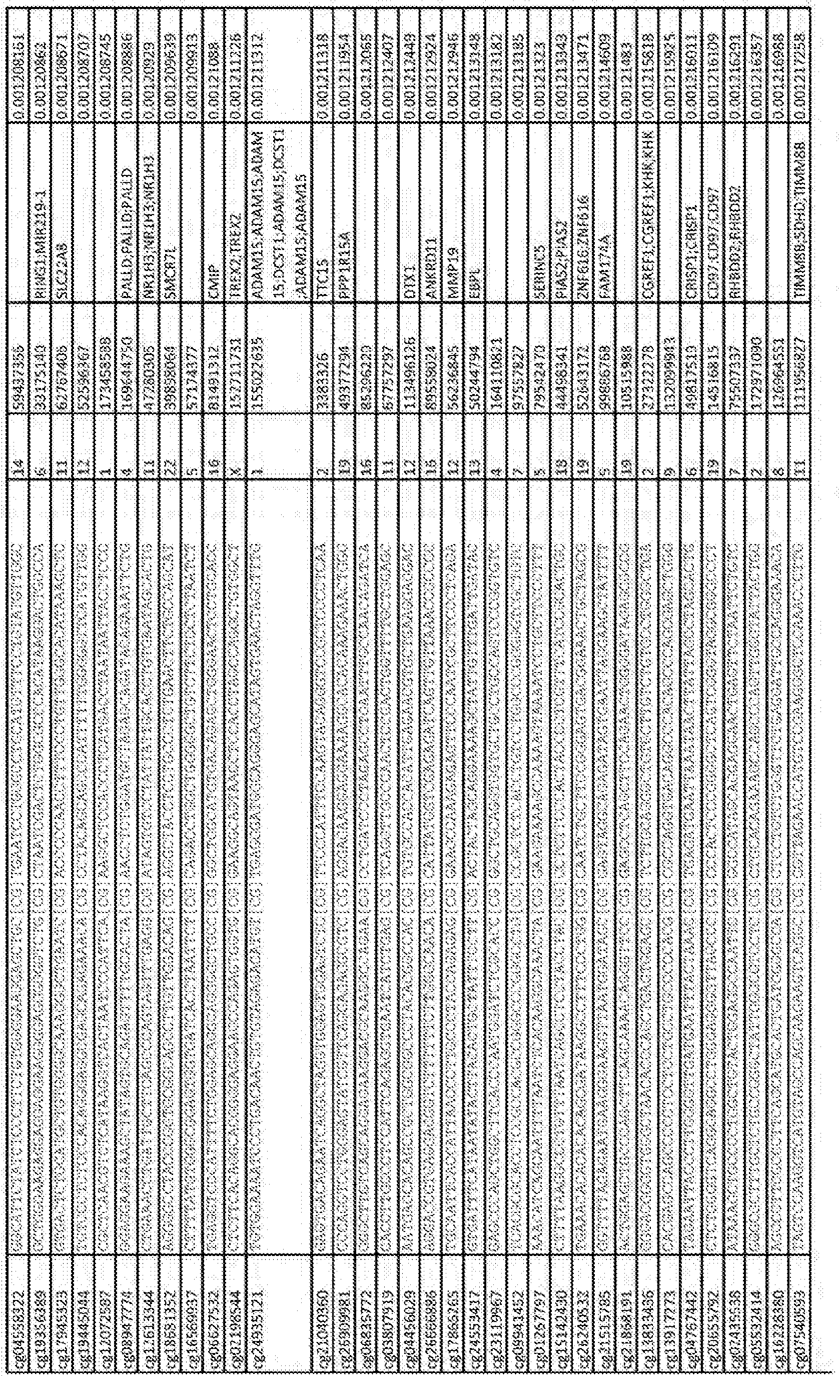

## Claims

1. An *in vitro* method of determining whether or not an individual uses alcohol, comprising the steps of:
determining the methylation status of at least one CpG dinucleotide in a biological sample from the individual,
wherein the at least one CpG dinucleotide comprises at least position 71389896 of chromosome 10; and
correlating the methylation status of the at least one CpG dinucleotide to determine whether or not the individual uses alcohol.

2. The method of claim 1, wherein demethylation at position 71389896 of chromosome 10 is indicative of alcohol use in the past 6 months, wherein optionally remethylation at position 71389896 of chromosome 10 is indicative of less or no alcohol use.

3. The method of claim 1, wherein the at least one CpG dinucleotide further comprises position 54677008 of chromosome 12, wherein optionally demethylation at position 54677008 of chromosome 12 is indicative of alcohol use in the past 6 months, wherein optionally remethylation at position 54677008 of chromosome 12 is indicative of less or no alcohol use.

4. The method of claim 1, wherein the at least one CpG dinucleotide further comprises position 75262522 of chromosome 8, wherein optionally demethylation at position 75262522 of chromosome 8 is indicative of previous or current alcohol use in the past 6 months, wherein optionally remethylation at position 75262522 of chromosome 8 is indicative of less or no alcohol use.

5. The method of claim 1, wherein the at least one CpG dinucleotide comprises position 92137791 of chromosome 9, wherein optionally demethylation at position 92137791 of chromosome 9 is indicative of alcohol use in the past 6 months, wherein optionally remethylation at position 92137791 of chromosome 9 is indicative of less or no alcohol use.

6. The method of claim 1, wherein the determining step comprises:
contacting DNA in the biological sample with bisulfite under alkaline conditions to produce bisulfite-treated DNA;
optionally, amplifying the bisulfite-treated DNA to produce amplified bisulfite-treated DNA;
contacting the bisulfite-treated DNA with at least one oligonucleotide that is complementary to a sequence comprising the at least one CpG dinucleotide; and
detecting the methylation status of the at least one CpG dinucleotide.

7. The method of claim 6, wherein the at least one oligonucleotide detects the CpG dinucleotide in the bisulfite-treated DNA in the unmethylated state or in the methylated state.

8. The method of claim 1, wherein the biological sample is selected from the group consisting of peripheral blood, lymphocytes, urine, saliva, and buccal cells, and/or wherein the methylation status of the at least one CpG dinucleotide is determined using bi-sulfite treated DNA.

9. The method of claim 1, further comprising obtaining self-report data from the individual regarding whether or not the individual is user of alcohol.

10. A computer implemented method for determining whether or not an individual uses alcohol, the method comprising:
obtaining measured data associated with the user, the measured data comprising one or more measured CpG methylation status,
wherein the one or more measured CpG methylation status comprises at least position 71389896 of chromosome 10;
generating a predictive score based on the obtained measured data; and
providing a likelihood of alcohol use by the user based on the predictive score.

11. The method of claim 10, wherein the CpG methylation status further comprises position 54677008 of chromosome 12, position 75262522 of chromosome 8, and/or position 92137791 of chromosome 9.

12. The method of claim 10, further comprising:
determining the CpG methylation status for the user, wherein a change in methylation status is an indicator of alcohol use.

13. The method of claim 10, further comprising:
outputting a predicted level of alcohol use based on the predictive score.

14. A kit for determining the methylation status of at least one CpG dinucleotide, the kit comprising:
at least one first nucleic acid primer at least 8 nucleotides in length that is complementary to a bisulfite-converted nucleic acid sequence that includes at least one CpG dinucleotide,
wherein the at least one first nucleic acid primer detects the methylated CpG dinucleotide or the unmethylated CpG dinucleotide at position 71389896 of chromosome 10; and
instructions for correlating a change in methylation status at one or more CpG positions with alcohol use.

15. The kit of claim 14, further comprising
a solid substrate to which the at least one first nucleic acid primer is bound, optionally wherein the solid substrate is selected from polymer, glass, semiconductor, paper, metal, gel and hydrogel.

## Patentansprüche

1. In-vitro-Verfahren zur Bestimmung, ob eine Person Alkohol konsumiert oder nicht, umfassend die folgenden Schritte:
Bestimmen des Methylierungsstatus von wenigstens einem CpG-Dinukleotid in einer biologischen Probe aus der Person,
wobei das wenigstens eine CpG-Dinukleotid wenigstens die Position 71389896 von Chromosom 10 umfasst; und
Korrelieren des Methylierungsstatus des wenigstens einen CpG-Dinukleotids, um zu bestimmen, ob die Person Alkohol konsumiert oder nicht.

2. Verfahren nach Anspruch 1, wobei die Demethylierung an Position 71389896 von Chromosom 10 ein Hinweis auf Alkoholkonsum in den letzten 6 Monaten ist, wobei gegebenenfalls die Remethylierung an Position 71389896 von Chromosom 10 ein Hinweis auf geringeren oder keinen Alkoholkonsum ist.

3. Verfahren nach Anspruch 1, wobei das wenigstens eine CpG-Dinukleotid ferner die Position 54677008 von Chromosom 12 umfasst, wobei gegebenenfalls die Demethylierung an Position 54677008 von Chromosom 12 ein Hinweis auf Alkoholkonsum in den letzten 6 Monaten ist, wobei gegebenenfalls die Remethylierung an Position 54677008 von Chromosom 12 ein Hinweis auf geringeren oder keinen Alkoholkonsum ist.

4. Verfahren nach Anspruch 1, wobei das wenigstens eine CpG-Dinukleotid ferner die Position 75262522 von Chromosom 8 umfasst, wobei gegebenenfalls die Demethylierung an Position 75262522 von Chromosom 8 ein Hinweis auf vorherigen oder gegenwärtigen Alkoholkonsum in den letzten 6 Monaten ist, wobei gegebenenfalls die Remethylierung bei Position 75262522 von Chromosom 8 ein Hinweis auf geringeren oder keinen Alkoholkonsum ist.

5. Verfahren nach Anspruch 1, wobei das wenigstens eine CpG-Dinukleotid die Position 92137791 von Chromosom 9 umfasst, wobei gegebenenfalls die Demethylierung an Position 92137791 von Chromosom 9 ein Hinweis auf Alkoholkonsum in den letzten 6 Monaten ist, wobei gegebenenfalls die Remethylierung an Position 92137791 von Chromosom 9 ein Hinweis auf geringeren oder keinen Alkoholkonsum ist.

6. Verfahren nach Anspruch 1, wobei der Bestimmungsschritt umfasst:
Inkontaktbringen von DNA in der biologischen Probe mit Bisulfit unter alkalischen Bedingungen, um Bisulfit-behandelte DNA herzustellen;
gegebenenfalls Amplifizieren der Bisulfit-behandelten DNA, um amplifizierte Bisulfit-behandelte DNA herzustellen;
Inkontaktbringen der Bisulfit-behandelten DNA mit wenigstens einem Oligonukleotid, das zu einer Sequenz komplementär ist, die das wenigstens eine CpG-Dinukleotid umfasst; und
Nachweisen des Methylierungsstatus des wenigstens einen CpG-Dinukleotids.

7. Verfahren nach Anspruch 6, wobei das wenigstens eine Oligonukleotid das CpG-Dinukleotid in der Bisulfit-behandelten DNA im unmethylierten Zustand oder im methylierten Zustand nachweist.

8. Verfahren nach Anspruch 1, wobei die biologische Probe ausgewählt ist aus der Gruppe bestehend aus peripherem Blut, Lymphozyten, Urin, Speichel und bukkalen Zellen und/oder wobei der Methylierungsstatus des wenigstens einen CpG-Dinukleotids unter Verwendung von Bisulfit-behandelter DNA bestimmt wird.

9. Verfahren nach Anspruch 1, ferner umfassend das Erhalten von Selbstberichtsdaten von der Person darüber, ob die Person Alkoholkonsument ist oder nicht.

10. Computerimplementiertes Verfahren zum Feststellen, ob eine Person Alkohol konsumiert oder nicht, wobei das Verfahren umfasst:
Erhalten von dem Konsumenten zugeordneten Messdaten, wobei die Messdaten einen oder mehrere gemessene(n) CpG-Methylierungsstatus umfassen;
wobei der eine oder die mehreren gemessene(n) CpG-Methylierungsstatus wenigstens die Position 71389896 von Chromosom 10 umfassen;
Erstellen einer prädiktiven Punktwertung basierend auf den erhaltenen Messdaten; und
Bereitstellen einer Wahrscheinlichkeit des Alkoholkonsums durch den Konsumenten basierend auf der prädiktiven Punktwertung.

11. Verfahren nach Anspruch 10, wobei der CpG-Methylierungsstatus ferner die Position 54677008 von Chromosom 12, die Position 75262522 von Chromosom 8 und/oder die Position 92137791 von Chromosom 9 umfasst.

12. Verfahren nach Anspruch 10, ferner umfassend:
Bestimmen des CpG-Methylierungsstatus für den Konsumenten, wobei eine Änderung des Methylierungsstatus ein Indikator für den Alkoholkonsum ist.

13. Verfahren nach Anspruch 10, ferner umfassend:
Ausgeben eines vorhergesagten Niveaus des Alkoholkonsums basierend auf der prädiktiven Punktwertung.

14. Kit zur Bestimmung des Methylierungsstatus von wenigstens einem CpG-Dinukleotid, wobei das Kit umfasst:
wenigstens einen ersten Nukleinsäureprimer mit einer Länge von wenigstens 8 Nukleotiden, der zu einer Bisulfit-konvertierten Nukleinsäuresequenz komplementär ist, die wenigstens ein CpG-Dinukleotid enthält;
wobei der wenigstens eine erste Nukleinsäureprimer das methylierte CpG-Dinukleotid oder das nicht methylierte CpG-Dinukleotid an Position 71389896 von Chromosom 10 nachweist; und
Anleitungen zum Korrelieren einer Änderung des Methylierungsstatus an einer oder mehreren CpG-Positionen mit dem Alkoholkonsum.

15. Kit nach Anspruch 14, ferner umfassend
ein festes Substrat, an das der wenigstens eine erste Nukleinsäureprimer gebunden ist, wobei das feste Substrat gegebenenfalls aus Polymer, Glas, Halbleiter, Papier, Metall, Gel und Hydrogel ausgewählt ist.

## Revendications

1. Procédé *in vitro* consistant à déterminer si un individu consomme de l'alcool ou non, comprenant les étapes consistant à :
déterminer l'état de méthylation d'au moins un dinucléotide CpG présent dans un échantillon biologique provenant de l'individu,
l'au moins un dinucléotide CpG comprenant au moins la position 71389896 du chromosome 10 ; et
corréler l'état de méthylation de l'au moins un dinucléotide CpG pour déterminer si l'individu consomme de l'alcool ou non.

2. Procédé selon la revendication 1, dans lequel la déméthylation en position 71389896 du chromosome 10 est révélatrice d'une consommation d'alcool dans les 6 derniers mois, éventuellement la reméthylation en position 71389896 du chromosome 10 étant révélatrice d'une moindre ou d'aucune consommation d'alcool.

3. Procédé selon la revendication 1, dans lequel l'au moins un dinucléotide CpG comprend en outre la position 54677008 du chromosome 12, éventuellement la déméthylation en position 54677008 du chromosome 12 étant révélatrice d'une consommation d'alcool dans les 6 derniers mois, éventuellement la reméthylation en position 54677008 du chromosome 12 étant révélatrice d'une moindre ou d'aucune consommation d'alcool.

4. Procédé selon la revendication 1, dans lequel l'au moins un dinucléotide CpG comprend en outre la position 75262522 du chromosome 8, éventuellement la déméthylation en position 75262522 du chromosome 8 étant révélatrice d'une consommation d'alcool antérieure ou actuelle dans les 6 derniers mois, éventuellement la reméthylation en position 75262522 du chromosome 8 étant révélatrice d'une moindre ou d'aucune consommation d'alcool.

5. Procédé selon la revendication 1, dans lequel l'au moins un dinucléotide CpG comprend la position 92137791 du chromosome 9, éventuellement la déméthylation en position 92137791 du chromosome 9 étant révélatrice d'une consommation d'alcool dans les 6 derniers mois, éventuellement la reméthylation en position 92137791 du chromosome 9 étant révélatrice d'une moindre ou d'aucune consommation d'alcool.

6. Procédé selon la revendication 1, dans lequel l'étape de détermination comprend :
la mise en contact d'ADN présent dans l'échantillon biologique avec du bisulfite dans des conditions alcalines pour produire de l'ADN traité au bisulfite ;
éventuellement, l'amplification de l'ADN traité au bisulfite pour produire de l'ADN traité au bisulfite amplifié ;
la mise en contact de l'ADN traité au bisulfite avec au moins un oligonucléotide qui est complémentaire à une séquence comprenant l'au moins un dinucléotide CpG ; et
la détection de l'état de méthylation de l'au moins un dinucléotide CpG.

7. Procédé selon la revendication 6, dans lequel l'au moins un oligonucléotide détecte le dinucléotide CpG présent dans l'ADN traité au bisulfite à l'état non méthylé ou à l'état méthylé.

8. Procédé selon la revendication 1, dans lequel l'échantillon biologique est choisi dans le groupe constitué par du sang périphérique, des lymphocytes, de l'urine, de la salive et des cellules buccales et/ou dans lequel l'état de méthylation de l'au moins un dinucléotide CpG est déterminé à l'aide d'ADN traité au bisulfite.

9. Procédé selon la revendication 1, comprenant en outre l'obtention de données auto-déclarées provenant de l'individu concernant le fait de savoir si l'individu est consommateur d'alcool ou non.

10. Procédé mis en œuvre par ordinateur permettant de déterminer si un individu consomme de l'alcool ou non, le procédé comprenant :
l'obtention de données mesurées associées à l'utilisateur, les données mesurées comprenant un ou plusieurs états de méthylation de CpG mesurés,
le ou les états de méthylation de CpG mesurés comprenant au moins la position 71389896 du chromosome 10 ;
l'élaboration d'un score prédictif sur la base des données mesurées obtenues ; et
la production d'une probabilité de consommation d'alcool par l'utilisateur sur la base du score prédictif.

11. Procédé selon la revendication 10, dans lequel l'état de méthylation de CpG comprend en outre la position 54677008 du chromosome 12, la position 75262522 du chromosome 8 et/ou la position 92137791 du chromosome 9.

12. Procédé selon la revendication 10, comprenant en outre :
la détermination de l'état de méthylation de CpG pour l'utilisateur, un changement d'état de méthylation étant un indicateur de consommation d'alcool.

13. Procédé selon la revendication 10, comprenant en outre :
la sortie d'un niveau prédit de consommation d'alcool sur la base du score prédictif.

14. Kit permettant de déterminer l'état de méthylation d'au moins un dinucléotide CpG, le kit comprenant :
au moins une première amorce acide nucléique ayant une longueur d'au moins 8 nucléotides qui est complémentaire à une séquence d'acide nucléique converti par du bisulfite qui comprend au moins un dinucléotide CpG,
l'au moins une première amorce acide nucléique détectant le dinucléotide CpG méthylé ou le dinucléotide CpG non méthylé en position 71389896 du chromosome 10 ; et
des instructions permettant de corréler un changement d'état de méthylation en une ou plusieurs positions de CpG avec la consommation d'alcool.

15. Kit selon la revendication 14, comprenant en outre
un substrat solide sur lequel l'au moins une première amorce acide nucléique est liée, éventuellement le substrat solide étant choisi entre un polymère, du verre, un semi-conducteur, du papier, du métal, du gel et de l'hydrogel.
